# EUROPEAN PATENT APPLICATION

(11) **EP 0 587 279 A1**
(43) Date of publication of application: **16.03.1994**
(21) Application number: 93305451.2
(22) Date of filing: 13.07.1993
(51) Int. Cl.: C12N 15/11, C12N 15/62, C12P 21/08, C07K 15/28, C12Q 1/68

(54) **Human nucleic acid fragments isolated from brain, adrenal tissue, placenta or bone narrow and their use**

(30) Priority: 13.07.1992 GB 9214857
(71) Applicant: MEDICAL RESEARCH COUNCIL, London W1N 4AL (GB)
(72) Inventor: Sibson, David Ross, Bucks, HP6 6HF (GB); Hadfield, Kathyrn Mary, Huntingdon, Cambs, PE17 4PQ (GB); Gross, Jacqueline, Kenton, Middlesex HA3 8LH (GB); Howells, David, Harfield, Herts, AL9 7LW (GB); Starkey, Michael, Abbots Langley, Herts. WO5 0HS (GB); Kelly, Maria, Ealing, London W5 3ST (GB); Shaw, Diana, 342 Glacier Hall, Calgary, Alberta T2N 1N4 (CA)
(74) Representative: Bizley, Richard Edward

(57) **Abstract**

This invention provides a nucleic acid fragment encoding a gene product or portion thereof and comprising any one of:-(a) a sequence selected from SEQ ID Nos 1 to 1193 from the attached sequence listings; (b) an allelic variation of a sequence as defined in (a); or (c) a sequence complementary to (a) or (b). The invention includes uses of such fragments, and gene products corresponding thereto.

## Description

This invention relates to new nucleic acid fragments encoding gene products or portions thereof, which fragments are obtainable from human nucleic acid populations, individual members of such populations being present in widely varying amounts.

Situations are increasingly arising in which it is necessary to study complex nucleic acid or polynucleotide populations. For example, it is now widely appreciated that an invaluable resource could be created if the entire sequence of the genomes of organisms such as man were determined and the information available. The magnitude of such a task should not, however, be underestimated. Thus, the human genome may contain as many as 100,000 genes [a very substantial proportion of which may be expressed in the human brain (Sutcliffe, Ann. Rev. Neurosci. 11:157 (1988))]. Only a very small percentage of the stock of human genes has presently been explored, and this largely in a piecemeal and usually specifically targeted fashion.

There has been much public debate about the best means of approaching human genome sequencing. Brenner has argued (CIBA Foundation Symposium 149:6 (1990)) that efforts should be concentrated on cDNAs produced from reverse transcribed mRNAs rather than on genomic DNA. This is primarily because most useful genetic information resides in the fraction of the genome which corresponds to mRNA, and this fraction is a very small part of the total (5% or less). Moreover, techniques for generating cDNAs are also well known. On the other hand, even supposing near perfect recovery of cDNAs corresponding to all expressed mRNAs, some potentially useful information will be lost by the cDNA approach, including sequences responsible for control and regulation of genes. Nonetheless, the cDNA approach at least substantially reduces the inherent inefficiencies resulting from analysis of repeated sequences or non-coding sequences in an approach which depends upon genomic DNA sequencing.

Recently, the results of a rapid method for identifying and characterising new cDNAs has been reported (Adams, M.D. et al., Science 252, 1991, pp 1651-1656). Essentially, a semi-automated sequence reader was used to produce a single read of sequence from one end of each of a number of cDNAs picked at random. It was shown, by comparing the nucleic acid sequences of the cDNAs (or the protein sequences produced by translating the nucleic acid sequence of the cDNAs) to each other and to known sequences in public databases, that each of the cDNAs picked at random, could be unambiguously classified. The cDNAs could be classified as being either entirely new or as corresponding, to a greater or lesser extent, to a previously known sequence. cDNAs identified in this way were further characterised and found to be useful in a variety of standard applications, including physical mapping. Unfortunately, such a process is insufficient. The longer the process is pursued with any given population of cDNAs the less efficient it becomes and the lower the rate of identification of new clones. In essence, as the number of cDNAs which have already been picked rises, the probability of picking a particular cDNA more than once increases. This difficulty is exacerbated by the wide range of abundancies at which different cDNAs can occur, which abundancies can vary by several orders of magnitude. Thus, whereas some sequences are exceedingly rare, a single cDNA type may comprise as much as 10% of the population of cDNAs produced from a particular tissue (Lewin, B. Gene Expression, Vol. 2: Eukaryotic Chromosomes, 2nd ed., pp. 708-719. New York: Wiley, 1980). The need to avoid missing rarer species in any given population presents a considerable problem.

Various approaches (so-called "normalisation" techniques) have been tried in addressing the problem of increasing the efficiency of examination of a mixed nucleotide population, for example, such a population as is to be examined in human genome sequencing.

Thus, a standard PCR protocol can be used to amplify selectively cDNAs which are present at extremely low levels, if there is information about the sequence of those cDNAs. If not, a primer specific to the desired cDNA cannot be constructed and the desired cDNA cannot be selectively amplified. The standard PCR method is therefore inadequate if it is desired to characterise a number of unknown genes.

A second approach involves hybridization of cDNA to genomic DNA. At saturation, the cDNAs recovered from genomic/cDNA hybrids will be present in the same abundance as the genes encoding them. This will provide a much more homogenous population than the original cDNA library, but does not entirely solve the problem. In order to reach saturation in respect of the very rare sequences, it will be necessary to use huge quantities of cDNA, which need to be allowed to anneal to large amounts of genomic DNA over a considerable periods of time. Furthermore, cDNAs which are homologous to genes which are present in multiple copies in the genome will be over-represented.

A third approach exploits the second order reassociation kinetics of cDNA annealing to itself. After a long period of annealing, the cDNAs which remain single stranded will have nearly the same abundance, and can be recovered by standard PCR (see Patanjali, S.R. et al., PNAS USA 88, 1991, pp. 1943-1947; Ko, M.S.H., NAR 19, no.18, 1991, pp 5705-5711). The methods disclosed in these two publications, however, suffer from notable disadvantages. They are entirely dependent on the stringent physical separation of single stranded and double stranded DNA, require an elevated number of manual manipulations in each reaction, and necessitate protracted hybridisation times (up to 288 hours in the method of Patanjali et al.)

Yet a further approach in "normalising" a nucleotide population is described in co-pending British Patent Application No 91 15407.0, filed 17th July, 1991 by MRC, and involves a PCR process in which a mixture comprising a heterogenous DNA population and appropriate oligonucleotide primers is first formed and the DNA denatured, but before effecting a conventional PCR protocol the conditions are altered to allow the denatured strands of the more common DNA species to reanneal together, whilst avoiding annealing of primers to the DNA strands. By this means, rarer species can subsequently be amplified in preference to the more common species.

This PCR normalisation method in general comprises the steps of:
(a) preparing a mixture comprising a heterogenous DNA population and oligonucleotide primers suitable for use in a PCR process, in which the DNA is denatured;
(b) altering the conditions to allow the denatured strands of the more common DNA species to reanneal, while preventing the annealing to the primers to the DNA strands;
(c) further altering the conditions of the mixture in order to allow the primers to anneal to the remaining single-stranded DNA comprising the rarer DNA species; and
(d) carrying out an extension synthesis in the mixture produced in step (c).

Advantageously, the method consists of a cyclic application of the above four steps.

It will be appreciated that the conditions may be altered by the alteration of the temperature of the reaction mixture. However, any conditions which affect the hybridisation of complementary DNA strands to one another may be varied to achieve the required result.

Because the reannealing efficiency of any given DNA species will depend on the product of its concentration and time, the more abundant the sequence the greater the extent to which it will reanneal in any given time period. Once a DNA species has reached a certain threshold concentration it will no longer be amplified exponentially, as a significant amount will have annealed to the double stranded form before the priming step. Thus, as each individual DNA species is amplified by the process to its threshold concentration, the rate of amplification of that species will start to tail off. Eventually, therefore, all DNA species will be present at the same concentration.

The length of the reannealing step will determine how much DNA is present at the threshold concentration. Preferably, therefore, the duration of the reannealing step will be determined empirically for each DNA population.

In the PCR normalisation process in general, the DNA primers may be adapted to prime selectively a sample of the total DNA population. By using primers which will only prime a sample of the population, only that sample will be amplified and normalised. The total quantity of DNA generated will thereby be reduced, which means that the cycling times can be kept low. This ensures that the method is applicable to complex DNA populations such as cDNA populations. In addition, a first primer can be used which is adapted selectively to prime a sample of the total cDNA population, and a second primer which is a general primer. Advantageously, the general primer is oligo dT (each primed cDNA will then be replicated in its entirely, as the oligo dT primer will anneal to the poly-A tail at the end of the cDNA).

In co-pending British Patent Application No 92 14873.3, filed by MRC 13th July, 1992, a new process is described which allows the study and identification of the individual members of a mixed or heterogenous population of nucleotide sequences perhaps of varying abundance. In preferred embodiments of the said process, the starting nucleic acid population is treated by:
(a) subjecting the nucleic acid to the action of a reagent, preferably an endonuclease which has its cleavage and recognition sites separated, which reagent cleaves the nucleic acid so as to produce double stranded cleavage products the individual strands of which overlap at cleaved ends to leave a single strand extending to a known extent;
(b) ligating the cleavage products from (a) with a population of adaptor molecules to generate adaptored cleavage products, each of which adaptor molecules has a cleavage product end recognition sequence and the population thereof encompassing a range of adaptor molecules having recognition sequences complementary to a predetermined subset of the sequences of the cleavage-generated extending single strands; and
(c) selecting and separating only those adaptored cleavage products resulting from (b) which carry an adaptor of predetermined recognition sequence.

A preferred endonuclease for use in step (a) of the above process is Fok 1.

An important feature of this process is the use of adaptor molecules. The adaptors used must have "overhanging" fragment recognition sequences which reflect or are complementary to the extending cleavage-derived sequences which the adaptors are designed to react with. It is also preferred that the adaptors used should end with a 5' hydroxyl group. The avoidance of a 5' phosphate group removes the risk of inappropriate ligation involving the adaptors.

Adaptor molecules may also contain a portion permitting specific sequence selection and separation (as in step (c) of the process) when a sequence is attached to the adaptor. For example, an adaptor can carry biotin, thereby permitting advantage to be taken of the biotin/avidin reaction in selecting and separating desired adaptored molecules. Additionally, adaptors preferably comprise a known and selected sequence such that specifically isolated adaptored molecules can be amplified by known techniques (such as PCR) using a primer complementary to the core sequence.

Preferably the adaptors are short double-stranded oligonucleotides which can be joined to the ends of cleavage products. They will have been chemically synthesised so that their sequence can be predetermined and so that large concentrations can be easily produced. They may also be chemically modified in a way which allows them to be easily purified during the process. As mentioned above, ideally their 5' ends will be unphosphorylated so that once joined to fragments the adaptored end of the latter will no longer be able to participate in further ligation reactions.

It is preferred that the adaptor cleavage product end recognition sequences are on the 5' end of the longest oligonucleotide strand making up the preferred adaptor molecules, are at least 3 nucleotides in length and with totally random bases at the single-stranded position(s) two nucleotides in from the 5' end. This then allows selection to be performed both during the joining reaction and during subsequent priming reactions. Then, because the final degree of selection is a result of the product of the degrees of selection achieved at these two stages, maximum selection can be achieved per adaptor/primer available.

Adaptor strand extensions on the 5' end of the longest oligonucleotide also facilitate the use of modified oligonucleotides for separation purposes. Preferably, the short oligonucleotide will be modified at its 5' end. This has the double benefit of requiring just one modified oligonucleotide for all possible single-stranded extensions that are used, and also placing the modification at a position where it cannot interfere with ligation or subsequent priming reactions.

Although only one type of adaptor is required per ligation reaction, it is preferred that adaptors covering all possible reactions in a chosen subset of sequences be present, because then the opportunity for fragments in the chosen subset to ligate to each other is minimised. It is also preferred that the chosen specific adaptor, carrying a predetermined recognition sequence, should not only be different from the other adaptors in its single-stranded extension, but also different in the rest of its sequence since this allows orientation to be introduced which is useful in subsequent steps. It is therefore also preferred that this adaptor has a modified oligonucleotide to facilitate its separation with the cleaving products to which it joins.

The above "adaptoring" process can be used to generate categories or subsets of sequences by making some of the adaptors specific in some way, and selecting and separating as in step (c). In this way subsets of sequences can be provided depending upon the specific adaptor chosen, e.g. for use in subsequent nucleotide sequencing. This facilitates, for example, the identification of a large population of sequences by permitting a rational approach to splitting such populations into subsets, each of which subsets can be examined in turn.

In the light of these developments, the present invention now provides a nucleic acid fragment encoding a gene product or portion thereof and comprising any one of:-
(a) a sequence selected from SEQ ID Nos 1 to 1193;
(b) an allelic variation of a sequence as defined in (a); or
(c) a sequence complementary to (a) or (b).

In another aspect, the invention provides a nucleic acid sequence as set out in any one of SEQ ID Nos 1 to 1193, or a complement or allelic variation thereof. Preferred sequences exhibit no more than 90% homology to a human sequence known per se.

In a further aspect, the invention provides a nucleic acid fragment comprising a portion of a sequence as defined above of sufficient size such that a probe of the same size and exhibiting complementarity to said portion can hybridise to said sequence. Preferably, such portions are at least 15 bases in length. It will be appreciated that minor mismatches in the aforesaid "complementarity" are not excluded provided hybridisation can still occur. In general, hybridisation conditions are within the choice of the skilled person, but reference can be made, for example, to the following: Melting temperature of hybrids - Bolton, E. T. and McCarthy, B. J. Proc. Natl. Acad. Sci, 48 p1390 (1962). Effect of formamide on lowering melting temperature - Casey, J. and Davidson, N., Nucleic Acids Res. 4, p1539 (1977). Effect of imperfect homology - Bonner, T. I. et al., J. Mol. Biol. 81, p123 (1973). General - Meinkoth, J. and Wahl, G. Anal. Biochem. 138, p267 (1984). Oligo hybridization and washing - Lathe, R. J. Mol. Biol. 183, P1 (1985).

The present invention also envisages DNA constructs comprising fragments or sequences as referred to above with a control or regulatory sequence.

The invention includes such DNA constructs using a gene system known in the art ligated to a sequence or fragment of the invention so as to enable, upon expression, the provision of a fusion polypeptide. Preferably, an endopeptidase recognition site is provided such that when the sequence or fragment is expressed it is expressed in frame with a known protein with the boundary being a cleavage site for an endopeptidase with a rare cutting site. The known protein can then be affinity purified, and the peptide corresponding to the fragment or sequence in accordance with the invention may be released by the endopeptidase. Alternatively, the whole protein can be used to raise antibodies which can then be screened for those directed at polypeptide corresponding to the fragment or sequence of the invention.

Since the present fragments and sequences can be used to produce, inter alia, corresponding genes, whether by isolating them, by synthesis or otherwise, such use and the resulting DNA fragments comprising genes are further aspects of the invention.

Yet another aspect of the invention is an expression vector comprising a fragment, sequence, gene-comprising DNA fragment, or DNA construct, as above, positioned such that that nucleic acid sequence which encodes the polypeptide corresponding to said fragment, sequence or DNA fragment is in operable reading frame with a control or regulatory sequence.

Other aspects of the invention are host cells incorporating a sequence, or fragment, or gene-comprising DNA fragment, or DNA construct, as above, as a heterologous part of the expressible genetic information of the cell. The production of such modified host cells can be achieved using methods known in the art. Such modified host cells can be used to express corresponding proteins, and these materials lend themselves in turn to the preparation of corresponding monoclonal or polyclonal antibodies using standard techniques.

Also included in this invention are such antibodies. Reference can be made, inter alia, to the following literature: Monoclonal antibodies, Cambell, A. M. Laboratory Techniques in Biochemistry, and Molecular Biology Ed. Burdon, R. H. and van Knippenberg, P. H. vol 13. Elsevier Amsterdam 1984. Goding, J. W., Monoclonal antibodies: Principles and Practice, 2nd Edition, academic Press, London 1986. Kipps, T. J. and Herzenberg, L. A., Handbook of Experimental Immunology : Applications of immunological methods in biomedical sciences, 4th edition Ed. Weir, D. M. et al., p108 Blackwell scientific Publications, Oxford. Harlow, E and Lane, D. Antibodies, A Laboratory Manual, Cold spring harbor Laboratory, Cold Spring, New York.

Expression in an appropriate higher eucaryotic host may be important to ensure correct protein folding and also activity. Expression to avoid copurification of toxic products can sometimes be better performed in organisms approved for human consumption, eg prokaryotic Bacillus subtilis, eurkaryotic yeast, mammalian cows milk vectors, and other methods known in the art.

The invention also includes novel gene products or portions thereof encoded by a fragment, sequence or gene-comprising DNA fragment of the invention.

It will be appreciated that the sequences of the present invention collectively have utility based, inter alia, upon their common origin, and hence they can effectively be considered together rather than as separate entities. It is convenient to represent them as separate sequences, because this is how they were produced and serves as "punctuation" between the different functional entities which each sequence represents. However, the sequences could just as easily have been presented as a continuous sequence derived by placing them end to end in the order in which they were produced, with a separate indication of where the beginnings and ends of the component sequences are.

In contrast to investigations hitherto, where gene fragments (sequence fragments) could only be identified through some known characteristic [for example: their homology to a fragment which largely encodes amino acids identified by sequencing a previously isolated peptide or is the antisense of that coding sequence; or them having at least partial homology to previously characterised nucleic acids; or them having ability to encode expressed proteins which could later be detected by functional assays of the cells expressing those proteins or by using antibodies which had been previously raised against the proteins to detect their expression, Sambrook J., et al., Molecular Cloning CSH Press 1989], the sequences and fragments described by the present invention are entirely underivable and unpredictable from the prior art, but are nonetheless clearly of great value for various purposes.

Thus, such sequences, by comparing them to sequence databases, can be used as a means for determining the existence of new members of existing gene families, new human genes when previously only non-human genes were known, and new genes when previously no genes were known (Karlin, S. and Altschul, S. F. Proc. Natl. Acad. Sci. 87 p2264-2268 (1980)). In all cases, this allows the isolation of the corresponding genes and their products, and hence enables the manufacture of molecules of potential biological interest by recombinant means. Screening libraries of known materials or hitherto unexplored source materials for biological efficacy is now an important industrial activity in the search for new therapies and therapeutics. When new sequences have already been found to have counterparts in gene families or in non-human genes then knowledge about biological efficacy may already be apparant. For example, new receptors or receptor agonists/antagonists may exhibit differences to known instances of these molecules, and such differences could make them more suitable as therapeutics by, for example, exhibiting binding characteristics which are more in keeping with avoidance of toxicity. Reference can be made, for example, to polymorphic dopamine receptors and the implications for mental health (Iversen, L. Nature 358, p109 (1992), and Van Tol, H. M. M. et al., Nature 358, p149-152 (1992)). Where absolutely required, realisation of full length cDNAs for expression can be achieved by using the sequences to screen (by hybridisation) suitable cDNA libraries containing full length clones (D'Alession, J. M., et al., Focus (Gibco B.R.L) 9 pl (1987)). Alternatively, the sequences can be used to design primers suitable for obtaining the missing sequences by PCR or other amplification methods (Frohman, M. A., Dush, M. K. and Martin, G. R., Proc. Natl. Acad. Sci. 85 p8998-9002 (1988)).

Appropriate use of the sequence fragments in antisense or triple helix (Griffin et al., Science 245 p967-971 (1989)) applications will be useful for identifying manipulable targets related to disease. For example, viruses have been inhibited by antisense RNA to their mRNAs (Chang, L-J., and Stoltfuz, C. M. J. Virol. p921-974 (1987)). A similar effect could be achieved by targetting the expression of cellular proteins which are essential for growth or maintenance of the virus.

Partial or full length cDNAs have great utility once expressed. The manner of expression can be selected by one skilled in the art to suit the intended application. Expression of full length cDNAs is typically required for biological activity. Procaryotic, and lower or higher eucaryotic hosts may be selected as the host for expression and higher eucaryotes may be preferred to ensure correct modifications, for example, glycosylation in vivo, when this proves to be important. Expression can be ensured by situating the cDNA appropriately to signals for expression (Amann, E. and Brosius, J. Gene 40 p183 1985), Shimuzu, Y et al., Gene 65, p141 (1988), Straus, D. and Gilbert, W. Proc. Natl. Acad. Sci. 82, p2014 (1985)). Such signals may include a promoter for transcription, which may itself be regulatable.

The proteins thus-expressed can be screened for activities of therapeutic or commercial value. It may be that the proteins have to be first isolated for this purpose or can be assayed in situ. It may be desirable that some means of stabilising the expressed protein is employed. This can be achieved, for example, (and as indicated earlier) by expressing in frame as part of a fusion polypeptide (Smith, D. B., et al., Proc. Natl. Acad. Sci. 83 p8073 (1986)).

Useful antibodies can be raised against the expressed proteins. It is commonly not an absolute requirement that full length proteins are produced, although this may influence the quality of the antibodies produced. Peptides as short as 8 or 9 amino-acids in length can be used as antigens (Germain R., N. Nature 353 pp605-607 (1991), Rudensky, A., Y., et al., Nature 353 p622-627 (1991)). Immunogenic peptides could simply be synthesised using the amino-acid sequence translated from a sequence or fragment of this invention. It is desirable, although not absolutely required, that some means of producing purified antibodies is adopted. When fusion polypeptides are used to raise antibodies, an affinity matrix specific for the generic part of the protein allows the fusion polypeptide to be immobilised (Smith, D. B., et al., Proc. Natl. Acad. Sci. 83 p8073 (1986)). The immobilised polypeptide can then be used to affinity purify the antibodies. Antibodies to both the generic part of the fusion polypeptide and the part of interest are produced. When these need to be discriminated between, a different affinity column can be used to remove only those antibodies specific for the generic part of the polypeptide. Alternatively, and as mentioned earlier, it can be arranged that the boundary between the two separate protein components of the fusion polypeptide has the recognition sequence for an endopeptidase with a rare cutting site. The peptide of interest can then be released from the affinity purified polypeptide by the action of the endopeptidase (Nagai, K., and Thogersen, H., C. Methods Enzmol. 153 p461-481 (1987). Another alternative is raise monoclonal antibodies against the purified protein.

The antibodies can be used for localising in situ, or quantifying in samples through, for example, ELISA or RIA assays, peptides against which they were raised. These uses are particularly beneficial when the results of the assays can be correlated to a disease condition, eg cancer. For example tumour markers may be found and used to target therapeutic agents. The antibodies can also be used to detect or monitor markers of undifferentiated growth, infection, cardiovascular or immune disease or a therapeutic response. When the antibodies recognise cell surface proteins they can be used in isolation or in combination to isolate particular populations of cells. These in turn can be used to isolate yet more cDNAs which will be enriched for yet more of such surface markers for the population, which, if similarly screened, will permit yet further subdivision of the population. Ultimately, panels of antibodies which can describe particular disease states will accrue. Such antibodies could be tailored for forensic applications as well as diagnostic purposes and disease monitoring.

The sequences or fragments can also be used for genetic analysis and mapping, for example, to diagnose the likelihood that a given individual is predisposed towards a given genetic disease. In the event of a sequence co-locating, genetically, with a disease gene, it can be used for the derivation of new disease therapies bases upon precise genetic knowledge. Such therapies can include, for example, the techniques of so-called "gene therapy" (Dusty Miller, A. Nature 357 p455-460 (1992)).

Antibodies can be produced against the protein of a genetic disease with sufficient discriminating power to discriminate between diseased and non-diseased states (Caskey, T. Genome Sequencing Conference, Hilton Head, S. Carolina (1991)). This would be useful for reducing the dependence of such tests on nucleic acid-based screens. Such antibodies also have the advantage of allowing detection of faulty expression of the protein, for example levels of expression which may be important for development of the disease in slow onset conditions.

Also very important is that not all cDNAs are likely to be found by conventional means, whereas the present sequences are, in one sense, "comprehensive". The use of the class of cDNAs which corresponds of necessity to truncated clones increases the chances that part of a cDNA will be cloned free of any sequences that could otherwise compromise it from being cloned. Sequence obtained can then be used to generate PCR primers from which the remainder can be obtained without having to clone.

This invention will now be further described and illustrated by means of the following Examples.

All oligonucleotides used in these Examples were synthesised Trityl on using an ABI 380B DNA Synthesizer according to the manufacturers instructions. Purification was by reverse phase HPLC (see, for example, Becker, C., R., et al., J. Chromatography 326, p293-299 (1985)).

### Example 1

Human brain and adrenal tissues were obtained from a mixture of 12 to 15 week menstrual age foetuses and then snap frozen in liquid nitrogen before storing in bijou bottles in a -80°C freezer. The two types of tissue were used separately, directly from the freezer, to prepare cDNA from which restriction fragments were generated for sorting into subsets. 1g portions of each of the separate tissues were homogenised, using an Ultra-Turrax T25 Disperser (Janke and Kunkel, IKA-Labortechnik), on ice in the presence of 4M guanidinium isothiocyanate to solubilise macromolecules. RNA was isolated from each homogenate by using centrifugation to sediment it through caesium trifluoroacetate. This was performed using the Pharmacia kit according to the manufacturer's instructions, except that centrifugation was performed for 36 hours and the RNA obtained was finally desalted and concentrated by performing two ethanol precipitations in succession with two 70% ethanol washes after each precipitation. In each case, polyA⁺ (mRNA) was isolated from 200 to 400 µg of the total RNA by binding it to magnetic oligo-dT coated beads (Dynal). Solution containing unbound material was removed from the beads, which were washed, and then mRNA eluted directly for use. mRNA isolation was performed in accordance with the manufacturer's instructions. Yields of RNA from the beads were between 1 and 3% of the total RNA. 2 to 4 µg of the eluted RNA were used for cDNA synthesis. cDNA synthesis was performed according to the method of Gubler, U and Hoffman, (B. J. Gene 25 p263 (1983) using a Pharmacia kit according to the manufacturer's instructions. OligodT was used to prime the first strand cDNA synthesis reaction. The cDNA was purified by extracting twice with phenol/chloroform and then low molecular weight solutes including nucleic acids below ca. 300 bases were removed by passing the cDNA reaction mixture through a Pharmacia S400 spun column used according to the manufacturer's instructions. Running buffer for the column comprised 10 mM TrisHCl, 1 mM EDTA, 50 mM NaCl @ pH 7.5.

The column eluate was adjusted to 10 mM Mg**²⁺** and then the purified cDNA was restricted by the action of 1 unit per 10 µl of the endonuclease Fok I at 37°C for 1 hour, so that it would be able to accept adaptors to enable fragment sorting.

The cDNA fragments were purified by two successive phenol/chloroform extractions followed by passing them through S400 spun columns as described above.

The adaptors used were oligonucleotides 5' N**₄**N**₄**N**₄**N**₄**TCCTTCTCCTGCGACAGACA (SEQ ID: 1194) with the complementary strand 5' TGTCTGTCGCAGGAGAAGGA (SEQ ID: 1195) and 5' AAN**₄**N**₄**TCTCGGACAGTGCTCCGAGAAC (SEQ ID: 1196) or 5' TTN**₄**N**₄**TCTCGGACAGTGCTCCGAGAAC (SEQ ID: 1197) each with the complementary 5' biotinylated strand GTTCTCGGAGCACTGTCCGAGA (SEQ ID: 1198). These were added to 25% of the eluted material by incubating together 200 pmoles of the mixture of double-stranded adaptors in the elution buffer to which had been added MgCl**₂** to 10mM, ATP to 10mM and 0.025 units/µl of T4 DNA ligase. The oligonucleotide 5' biotinylated GTTCTCGGAGCACTGTCCGAGA, (SEQ ID: 1198) and whichever of the complementary oligonucleotides with which it was used, each comprised 1/32 of the molar proportion total adaptors. The final reaction volume was 90 µl which was heated to 65°C for 3 minutes and then cooled to room temperature before the ligase was added. Ligation was performed for 16 hours at 12°C.

Two successive phenol/chloroform extractions were performed to remove the ligase. The final aqueous phase was passed through an S400 spun column (Pharmacia) as described above except that the column was used with 10 mM Tris pH 8.3/50 mM NaCl.

The column eluate was adjusted to 25mM Mg2+, 0.5mM dNTPs in a final volume of 200 µl. The mixture was placed in a thermocycler (Techne MW2) and heated to 78°C for 5 minutes. At this point 10 units of cloned Taq DNA polymerase (AmpliTaq, Perkin Elmer) were added. This was followed by an incubation at 72°C for 10 minutes to fill in the unligated strand of the adaptor. After the second incubation 200 µl of streptavidin coated magnetic beads (Dynal) prepared according to the manufacturers instructions were added to bind cDNA ligated to that of the oligonucleotides which was complementary to the 5' GTTCTCGGAGCACTGTCCGAGA (SEQ ID: 1198) biotinylated adaptor. Bead binding was allowed to proceed at 28°C for 30 minutes with mixing every 10 minutes.

Un-biotinylated cDNAs were washed from the beads with 400µl each of 2M NaCl twice, fresh 0.15 mM NaOH four times at 28°C for 5 minutes each, water twice and finally a buffer comprising 20 mM Tris pH 8.3, 50 mM NaCl, and 25mM Mg**²⁺**. The beads were then resuspended in 240 µl of the final buffer including additionally 0.5 mM dNTPs and divided into 4x60 µl.

Four of the 60 µl aliquots, two from each tissue, were processed further specifically to prime and copy a subset of the immobilised, adaptored fragments. 2 pmoles of the primer 5' CTGTCTGTCGCAGGAGAAGGAA (SEQ ID: 1201) were added to each of two aliquots, one from each tissue. 2 pmoles of the primer 5' CTGTCTGTCGCAGGAGAAGGAG (SEQ ID: 1202) were added to each of the other two aliquots. 2.5 units of Taq DNA polymerase were added to each reaction and 16 cycles of alternate denaturation at 95°C for 30 seconds, annealing at 63°C for 2 minutes and polymerisation at 72°C for 3 minutes was performed to accumulate the selected single-strands in solution.

On completion of the DNA synthesis reactions a further 30 µl of resuspended beads were added to each reaction to remove the biotinylated fragments. The reaction was incubated at 28°C for 30 minutes mixing every 10 minutes to ensure that the biotinylated strands were bead bound. Each aqueous phase containing the newly synthesised strands was then removed and extracted with phenol/chloroform twice to remove the enzyme before being further purified by passing through an S400 spun column equilibrated with 10 mM Tris pH 8.3/50 mM NaCl as described above.

Rounds of PCR amplification of subsets of the selected fragments were performed by using the original primer in each case, together with one of the primers 5' GTTCTCGGAGCACTGTCCGAGAG (SEQ ID: 1199) or 5' GTTCTCGGAGCACTGTCCGAGAC SEQ ID: 1200). This simultaneously rendered the fragments double-stranded and increased the amounts of available material. It was not known how many cycles of amplification would be required at this stage, since each primer pair would be expected to behave differently. It was therefore necessary directly to determine a suitable number empirically by using standard agarose gel electrophoresis to examine the reaction products after a given number of cycles. In some cases, to avoid the accumulation of non-specific products, it was necessary to perform an initial 5 cycles of amplification with both of the primers present at 2 pmoles each. All reactions were performed using 8 µl or 12.5 % whichever was the larger but not exceeding 12 µl of the column effluent above. Reaction conditions were adjusted to 20 mM Tris pH 8.3, 50 mM NaCl, 25mM Mg**²⁺**, 0.5mM dNTPs and 2.5 units of Taq DNA polymerase in a final volume of 40 µl. Apart from when an initial amplification with 2 pmoles of each primer was performed, 20 pmoles of each primer were used. Cycles of amplification were performed at 95°C for 30 seconds, 65°C for 1 minute and 72°C for 3 minutes.

For the purposes of cloning, selected cDNA was amplified as described immediately above, except that the reaction was not monitored. Instead, the number of cycles which had previously been shown to just give rise to all observable products plus another 4 cycles were performed. In addition, an extra 72°C for 10 minutes incubation was performed after the last cycle.

The products of the reaction were then prepared for directional cloning. Water was added to adjust the final reaction volume to 60 µl. Enzyme was removed by two successive phenol/chloroform extractions. The final aqueous mixture was passed through an S400 column as described above, except that it had been equilibrated with 10 mM Tris HCl pH 7.5, 50mM NaCl.

For directional cloning, advantage was taken of the different known sequences introduced at each end of the selected cDNAs by the adaptors in a modification of the method of Aslandis, C. and de Jong, P. J. (Nucl. Acids Res. 18, p6156 (1990)). Different cohesive ends were produced on each end by using the exonuclease activity of T4 DNA polymerase to resect from the 3' end, to the first T in each case. To 75 µl or 75 % of the column eluate, whichever was least, were added 9.5 µl of 100mM TrisHCl pH7.4, 100 mM MgCl2, and 9.5 µl of 0.5 mM dTTP. 16 units of T4 DNA polymerase were added and the reaction incubated in a water bath at 37°C for 30 minutes. The enzyme was removed by extracting with phenol/chloroform, twice successively. The salt of the final aqueous phase was adjusted by passing it through an S300 column (Pharmacia) equilibrated with 10 mM TrisHCl pH 7.4, 1 mM EDTA as described above.

The E.coli plasmid cloning vector pBluescript KS+ (Alting-Meese, M. A. and Short J. M., Nucl. Acids Res. 17 p9494) was prepared for accepting the resected cDNA by restriction cleavage at the BamHI and HindIII sites and then adaptoring the resultant cohesive ends using the specific adaptors produced by the oligonucleotide 5' AGCTCGGCTCGAGTCTG (SEQ ID: 1203) with its partially complementary oligonucleotide 5' GCGACAGACAGCAGACTCGAGCCG (SEQ ID: 1204) and the oligonucleotide 5' GATCCGGCTCGAGT (SEQ ID: 1205) with its partially complementary oligonucleotide 5' CCGAGAACACTCGAGCCG (SEQ ID: 1206). Preparation of the vector and adaptoring were performed according to standard procedures. Insertion of the cDNA was performed between the BamHI and HindIII restriction sites. Recombinant vectors were transformed into the host XL1-Blue (Bullock, W. O. et al Biotechniques 5 p376-378 (1987)) by the method of Hannahan, D. J. (Mol. Biol. 166 p577-580 (1983)). Suitable standard controls for the ligations and transformations were also included.

Post transformation procedures were as described in "Molecular Cloning", 2nd Edition (Sambrook J., Fritsch, E. F., and Maniatis, T. CSH Press (1989)). Colonies were produced by plating onto X-gal/IPTG L-agar plates containing 50µg/ml ampicillin and 10µg/ml tetracyclin. Clear colonies were picked, each into a separate well of a microtitre plate, containing 100µl of L-broth and 50µg/ml ampicillin. Growth was allowed to occur for 16 hours at 37°C. 100µl of 50% or 30% glycerol was added to plates which were archived at -20°C or -80°C, respectively.

Bacteria corresponding to those archived were used for preparing templates for sequencing by the dideoxy method (Sanger, F. Milklen, S. and Coulson, A. R. Proc. Natl. Acad. Sci. 74 p5463-5467 (1977)). Bacteria for this purpose were either grown on L-agar plates containing 50µg/ml of ampicillin, prepared at the same time as they had been grown in liquid culture, or after plating out from the archive. Alternatively, fresh liquid cultures were inoculated from the archive. In all cases, cDNA inserts were amplified for sequencing by PCR (Saiki, R. K. et al Science 239 p487-491 (1988)). PCR was either performed using bacteria directly added to the reaction, by a toothpick, or PCR was performed using 1/50th of the plasmid isolated by preparative methods (Holmes, D. S. and Quigley, M. Anal. Biochem. 114 p193 (1981)) from the bacteria in the liquid cultures or from the plates.

20 pmoles of each of the PCR primers 5' biotinylated GTAAAACGACGGCCAGT (SEQ ID: 1207) and 5' CGAGGTCGACGGTATCG (SEQ ID: 1208) were used in 40µl reactions containing 2.5mM Mg**²⁺**, 50 mM KCl, Tris-HCl pH 8.3 and 0.25 units of Amplitaq (Cetus). Reactions were performed at 95°C, for 1 minute, followed by 35 cycles at 95°C for 30 seconds, 60°C for 30 seconds and 72°C for 40 seconds. After the cycles, a final incubation at 72°C for 5 minutes was performed.

After PCR, standard agarose gel electrophoresis was used to determine which reactions had been successful. The biotinylated strands of successful reactions were then recovered for single-stranded sequencing by binding them to steptravidin coated beads (Dynal) and then washing, all according to the manufacturers instructions, except that the washing steps were either performed manually or performed automatically in the 96 well microtitre plate format using a Biomek robotic work-station attached to a side-arm loader (Beckman).

Dideoxy chain termination sequencing reactions were performed using the immobilised, biotinylated strands as templates and 2 pmoles of the oligonucleotide 5' CGAGGTCGACGGTATCG (SEQ ID: 1209) as primer. Reactions were performed using fluorescently-labelled terminators (Du Pont) or a fluoroscein-labelled primer (Pharmacia) according to the manufacturers instructions. Reactions were analysed using automated DNA sequencers. A Genesis 2000 was used for the "Du Pont" reactions and an A.L.F. for the "Pharmacia" reactions. Bases were assigned for the Genesis 2000 reads using the manufacturers Base Caller software. Files of called bases were then transferred to a SUN Network from an Apple Macintosh computer which had been used for base calling. Raw data from the A.L.F. reads was directly transferred to a SUN network where bases were called using the public domain "trace editor software" (TED). In both cases, files of called bases were entered into a Sybase^{**TM**} database. Entering data entailed automatically removing vector and adaptor or linker sequences, but not editing ambiguous bases. After removal of the unwanted bases, files were automatically compared to other sequences in the cDNA database and the latest versions of the publically available databases, GENBANK and SWISSPROT. Searches were performed with the "basic local alignment search tool" (BLAST) (Karlin, S. and Altschul, S. F. Proc. Natl. Acad. Sci. 87 p2264-2268 (1990)).

Sequences SEQ ID Nos 1 to 610, given hereinafter, were obtained by the above procedure.

### Example 2

A second method of preparing cDNA libraries for obtaining gene fragments of the invention took advantage of the PCR normalisation process described above. Standard procedures were used to prepare mRNA from RNA that had been isolated by standard caesium chloride bouyant density gradient methods from a full term human placenta. The oligonucleotide LNotdt, sequence 5' TACGTTCGACAAGCTTGAATTCGCGGCCGC(T)_{**26**,} (SEQ ID: 1210) was used at 1 µM with AMV reverse transcriptase, to prime first strand cDNA synthesis under standard conditions from 0.5 µg of the placental mRNA.

Temperatures above 65°C were used to inactivate the reverse transcriptase and then the volume of the reaction made up to 100 µl with water.

PCRs were then performed in reactions containing 1 µl of the diluted cDNA, 10 mM Tris-HCl pH 8.3, 40 mM KCl, 1.5 mM MgCl**₂**, 0.01% gelatin, 200 µM dNTPs, 10 uCi a**³²**P dCTP, 1 µM each of the primers 11AD1, sequence 5' GCC(TA)(GC)CGCCGA (SEQ ID: 1211), and LNotdT and Taq DNA polymerase. An initial denaturation period of 95°c for 90 seconds was followed either by 35 cycles of standard PCR, comprising 95°C for 30 seconds, 45°C for 30 seconds and 72°C for 30 seconds or alternatively 3 cycles of the standard PCR already described followed by 27 cycles of Cot PCR during which an additional step of 72°C for 16 minutes was placed between all of the 95°C and 45°C steps of the standard PCR. The standard PCR was followed by a single 72°C for 3 minutes step while the Cot PCR was followed by one standard PCR cycle except that the 72°C incubation was performed for 3 minutes.

Products of the PCR reaction were end repaired by adding 5 units of T4 DNA polymerase to the reaction and then incubating at 37°C for 10 minutes. Enzymes were removed by phenol extraction. The cDNA was precipitated by 70% ethanol, dried and then resuspended in NotI buffer. 20 units of NotI were used to digest the cDNA under standard conditions. cDNA was again phenol extracted and ethanol precipitated. 10% of the purified NotI cut DNA were ligated to the vector pBluescript lting-Meese, M. A. and Short J. M. Nucl. Acid Res. 17 p9494 which had been prepared as standard to receive this DNA by restricting with the enzymes NotI and EcoRV. Transformation and processing of clear colonies was performed as described above except that the host E.coli strain DH5a was used in place of XL-1 Blue.

Preparation of clones for sequencing, sequencing and sequence analysis of cDNAs in clones thus-produced were performed as described in Example 1.

Sequences SEQ ID Nos 611 to 772, given hereinafter, were obtained by the above procedure.

### Example 3

cDNA libraries corresponding to adult brain cortex (Clontech Laboratories, Inc., Cat No. HL10036) and adult bone marrow (Clontech Laboratories, Inc., Cat No. HL10586) prepared in lambda gt11 phage were transfected into E.coli Y1090 and plated out for colour selection of recombinant plaques ("Molecular Cloning", 2nd Edition Sambrook J., Fritsch, E. F., and Maniatis, T. CSH Press (1989)). 192 lambda Zap clones, corresponding to rhabdomyocarcoma cDNAs and a gift from C. Cooper, ICR, Sutton, were similarly plated except that the host XL-1 Blue was used.

Clear plaques from each library were resuspended in 5 µl of Tris-HCl pH 8, 1 mM EDTA. 2 µl of the resultant phage suspensions were added directly to PCRs for the purposes of amplifying the cDNA inserts for sequencing. PCR was performed as described in Example 1, except that the oligonucleotides used as primers for the lambda gt11 clones were 5' GGTGGCGACGACTCCTGGAGCCCG (SEQ ID: 1212) and 5' TTGACACCAGACCAACTGGTAATG (SEQ ID: 1213). Whichever of the oligonucleotides was to be used to prime the strand which would serve as the sequencing template was used in biotinylated form.

Preparation of clones for sequencing, sequencing and sequence analysis of cDNAs in clones thus-produced was performed as described in Example 1, except that 2 pmoles of the primers that were unbiotinylated in the PCR were used as sequencing primers.

Sequences SEQ ID Nos 773 to 1193, given hereinafter, were obtained by the above procedure.

The following are the SEQUENCE LISTINGS which comprise sequences SEQ ID Nos 1 to 1213 referred to hereinbefore. Certain of these sequences are preferred, and are listed as such after the main SEQUENCE LISTINGS.

(1) GENERAL INFORMATION

(i) APPLICANT

(A) NAME: MEDICAL RESEARCH COUNCIL

(B) STREET: 20 PARK CRESCENT

(C) CITY: LONDON

(E) COUNTRY: ENGLAND

(F) POSTAL CODE: W1N 4AL

(ii) TITLE OF INVENTION: HUMAN GENOME SEQUENCES

(iii) NUMBER OF SEQUENCES: 1213

(iv) COMPUTER READABLE FORM:

(A) MEDIUM TYPE: DISKETTE

(B) COMPUTER: IBM PC COMPATIBLE

(C) OPERATING SYSTEM: MS-DOS

(D) SOFTWARE: EXTRACT

(2) INFORMATION FOR SEQ ID :1:

(i) SEQUENCE CHARACTERISTICS:

(A) LENGTH: 264 base pairs

(B) TYPE: nucleic acid

(C) STRANDEDNESS: double

(D) TOPOLOGY: linear

(xi) SEQUENCE DESCRIPTION: SEQ ID :1:

GCCGATTCGT GACCAAGAAG GCTCTGTGCA TTCGGGTTTT CCAGGAGACT 50

CAAAAGCTGA AGAAGCGAAG AAGAGCCTTA AAGGCTGCAG CAGCAGCTCA 100

ATAAACAAGC AAAGCGGAGG AACCCAGACA GCCCTTGTCC AAAGCCATAC 150

CAATATGATC TATCTTCTAA TGTATCCATG TTGTAATTAT ATATGTGTCT 200

GTGTGTGTCG AAATCTCTAG ACATACAGAT ATATATTCAT ATATCATATA 250

TATATATATA CACA 264

(2) INFORMATION FOR SEQ ID :2:

(i) SEQUENCE CHARACTERISTICS:
(A) LENGTH: 124 base pairs
(B) TYPE: nucleic acid
(C) STRANDEDNESS: double
(D) TOPOLOGY: linear

(xi) SEQUENCE DESCRIPTION: SEQ ID :2:

AGGAACATGT GTTTATTCAT CCAGCAGTGT TGCTCAGCTC CTACCTCTGT 50

GCCAGGGCAG CATTTTCATA TCCAAGATCA ATTCCCTCTC TCAGCACAGC 100

CTGGGGAGGG GGTCATTGTT CTCT 124

(2) INFORMATION FOR SEQ ID :3:

(i) SEQUENCE CHARACTERISTICS:
(A) LENGTH: 333 base pairs
(B) TYPE: nucleic acid
(C) STRANDEDNESS: double

(D) TOPOLOGY: linear

(xi) SEQUENCE DESCRIPTION: SEQ ID :3:

ACAGAGCCGA TTCTGCATCC ACTGTGGTCA ACATTTAGGA AGTTTTAAGC 50

TAAGATTTGC CAAATTGTAG CCTACTGGAT TCCGGTTCTC TTGACATCTC 100

TTTCTAGTAG CCATGTCTTG CACTTCCCGA GTATAAACGA ACTGAGATGC 150

AAATTAAAAA AGGGAGGATT TAGAATAATG AAAAGAGAAA AGTCAAGAAA 200

GCACAATCAC TAGTGTAGAG ATAACAGAAT TTCTGAATTC CCTGAAAGCA 250

ATCTATATAA ATGCATGTGA AATAATACAC CAGCATCTGT GGCCCATACG 300

TCACATATTA GGAACTGATA ACATAAGGTA AAC 333

(2) INFORMATION FOR SEQ ID :4:

(i) SEQUENCE CHARACTERISTICS:
(A) LENGTH: 200 base pairs
(B) TYPE: nucleic acid
(C) STRANDEDNESS: double
(D) TOPOLOGY: linear

(xi) SEQUENCE DESCRIPTION: SEQ ID :4:

AGGGAGAAAG GAACATCCGC GAACAGCCAA AGAAATCTCA GAAGAGTCCC 50

GGAGCTCAAG GATCAGAGTA ACACAATTTT CACTTTTTCT GTCTTTATGT 100

AAGAAGAAAC TGCCTAGATG ACGGGGCCTC CTTCTTCAAA CAGGAATTTC 150

TGTTAGCAAT ATGTTAGCAA GAGAGGGCAC TCCCAGGCCC CTGCCCCCAT 200

(2) INFORMATION FOR SEQ ID :5:

(i) SEQUENCE CHARACTERISTICS:
(A) LENGTH: 213 base pairs
(B) TYPE: nucleic acid
(C) STRANDEDNESS: double
(D) TOPOLOGY: linear

(xi) SEQUENCE DESCRIPTION: SEQ ID :5:

ACAGAGCCAT TTGCATCCAC TGTGGTCAAC ATTTAGGAAG TTTTAAGCTA 50

AGATTTGCCA AATTGTAGCC TACTGGATTC CGGTTCTCTT GACATCTCTT 100

TCTAGTAGCC ATGTCTTGCA CTTCCCGAGT ATAAACGAAC TGAGATGCAA 150

ATTAAAAAAA GGGAGGATTT AAGAATAATG AAAAGAGAAA AATCAAGAAA 200

GCACAATCAC TAG 213

(2) INFORMATION FOR SEQ ID :6:

(i) SEQUENCE CHARACTERISTICS:
(A) LENGTH: 345 base pairs
(B) TYPE: nucleic acid
(C) STRANDEDNESS: double
(D) TOPOLOGY: linear

(xi) SEQUENCE DESCRIPTION: SEQ ID :6:

CGCAGGAGAA GGAACAGAGC CATTGTGCAT CCACTGTGGT CAACATTTAG 50

GAAGTTTTAA GCTAAGATTT GCCAAATTGT AGCCTACTGG ATTCCGGTTC 100

TCTTGACATC TCTTTCTAGT AGCCATGTCT TGCACTTCCC GAGTATAAAC 150

GAACTGAGAT GCAAATTAAA AAAAGGGAGG ATTTAGAATA ATGAAAAGAG 200

AAAAATCAAG AAAGCACAAT CACTAGTGTA GAGATAACAG AATTTCTGAA 250

TTCCCTGAAA CAATCTATAT AAATGCATGT GAAATAATAC ACCAGCATCT 300

GTGGCCCATA CGTCACATAT TAGGAACTGA TAACATAAGG TAAAC 345

(2) INFORMATION FOR SEQ ID :7:

(i) SEQUENCE CHARACTERISTICS:
(A) LENGTH: 159 base pairs
(B) TYPE: nucleic acid
(C) STRANDEDNESS: double
(D) TOPOLOGY: linear

(xi) SEQUENCE DESCRIPTION: SEQ ID :7:

TCCATTTGAC ATCGCATTTC CATAGAAATG GCCAAAGAAA GAAGGTCCTG 50

GGGTTTTTTA TAGAAAGCTC AAAAAGTTCA ACCTTTGATG CTATCCCCCA 100

GCCCAATACA AAATATATAG AAAAAGCGAT TATTACAATA ACGCTTCAAT 150

TTCTTTTCC 159

(2) INFORMATION FOR SEQ ID :8:

(i) SEQUENCE CHARACTERISTICS:
(A) LENGTH: 124 base pairs
(B) TYPE: nucleic acid
(C) STRANDEDNESS: double
(D) TOPOLOGY: linear

(xi) SEQUENCE DESCRIPTION: SEQ ID :8:

ATATTTCAAT CGAACAAAAA GGAAACTTTT TTTGAACTTA TTGAGGCTCT 50

ACTTAAGTAC ATCGAAACCC TTAATGCTTC TGGGGCTGTG TTGATTTCCT 100

TGCCTGGCTG GGGGTTTGAT TCGC 124

(2) INFORMATION FOR SEQ ID :9:

(i) SEQUENCE CHARACTERISTICS:
(A) LENGTH: 259 base pairs
(B) TYPE: nucleic acid
(C) STRANDEDNESS: double
(D) TOPOLOGY: linear

(xi) SEQUENCE DESCRIPTION: SEQ ID :9:

CCGATACAAA TGTACGGAAT GTGTGAGTCC CTCTGGGAGC CCAACATGGA 50

TCCGGATCAC CTGTTTGAAA CCATCTCCCA AGCCATGCTG AATGCTGTGG 100

ACCGGGATGC AGTGTCAGGC ATGGGAGTCA TTGTCCACAT CATCGAGAAG 150

GACAAAATCA CCACCAGGAC ACTGAAGGCC CGAATGGACT AACCCTGTTC 200

CCAGAGCCCA CTATATATTA TTTCTACTTC ATCTATATAT TGCAAAAATA 250

GAAAATAGA 259

(2) INFORMATION FOR SEQ ID :10:

(i) SEQUENCE CHARACTERISTICS:

(A) LENGTH: 216 base pairs

(B) TYPE: nucleic acid

(C) STRANDEDNESS: double

(D) TOPOLOGY: linear

(xi) SEQUENCE DESCRIPTION: SEQ ID :10:

GTCACCAAGA CCGAGGCCAA ACTGGGCACC TTCCCCAGGG CCCTCAAGAA 50

GCTCCTGCAC ACAATAAACG CGCCCTCGTT CTTTAGCAAG TCTGCTCCCT 100

CGAGGCCACA GCAAGCCGGC TACGGAGCCC CCGTTCTGTT TTGAGCCGAA 150

GACTACTTTA TTGGATGCGG TGAAAGGCCT CAGCTCTGAC ACTCTGATCA 200

CTGTGACAAG GGGCCC 216

(2) INFORMATION FOR SEQ ID :11:

(i) SEQUENCE CHARACTERISTICS:

(A) LENGTH: 205 base pairs
(B) TYPE: nucleic acid
(C) STRANDEDNESS: double
(D) TOPOLOGY: linear

(xi) SEQUENCE DESCRIPTION: SEQ ID :11:

CACACTTCTT AAGATACATC AAGTACTAGT GATCTTTGCT AGCAGTTATG 50

CCTGTTTCGT GTTACAGATT TGGCCATATA TTGACTAAAC AGCCCCTGTA 100

AAGTTGAAAG AAAAAGTTTA TAACAGTGAA CTTCTGAGGT TTACGTTACT 150

GCAGGCTTTG TTGAGAAGAG ATTGTTACAG TGTGATTTAT GGATGATCAG 200

GGATG 205

(2) INFORMATION FOR SEQ ID :12:

(i) SEQUENCE CHARACTERISTICS:
(A) LENGTH: 267 base pairs
(B) TYPE: nucleic acid
(C) STRANDEDNESS: double
(D) TOPOLOGY: linear

(xi) SEQUENCE DESCRIPTION: SEQ ID :12:

TGCATCTGAA GAGTTGATGA TCGAGACTAC AGCTGCACTA TCAGACTGTC 50

AGGCAGCTCC CTCAGAAACA GCTTCAACAG ACTGGCTGCT GAGCAGACAT 100

CACCGTCCTT CCGAGCTCCA CGGCGACTCC ACTCTCGAAC TTCAGTCGAA 150

GTTGTTCCAC CACCTTCACG TTACCATTCA CCCTAAAAGA CCTTCTTGGG 200

TAAGTCCATG CTGCGTCAAA TATTCCACTA TATTCCACAC TACTGCTGGA 250

TATGCCATTC TCGGTGA 267

(2) INFORMATION FOR SEQ ID :13:

(i) SEQUENCE CHARACTERISTICS:
(A) LENGTH: 116 base pairs
(B) TYPE: nucleic acid
(C) STRANDEDNESS: double
(D) TOPOLOGY: linear

(xi) SEQUENCE DESCRIPTION: SEQ ID :13:

GCCTCATCAG GTTTGCCCAG ATGCTGGAGA AGGTGTGCGT GGAGACGGTG 50

GAGAGTGGAG CCATGACCAA GGACCTGGCG GGCTGCATTC ACGGCCTCAG 100

CAATGTGAAG CTGAAC 116

(2) INFORMATION FOR SEQ ID :14:

(i) SEQUENCE CHARACTERISTICS:
(A) LENGTH: 296 base pairs
(B) TYPE: nucleic acid
(C) STRANDEDNESS: double
(D) TOPOLOGY: linear

(xi) SEQUENCE DESCRIPTION: SEQ ID :14:

TTCGAACTTA CCAGCATCAT GTTTGGTCTC TTCGTATGGT CAAACTTCAA 50

CTTTATAAAA ATAGTGAATA CCACTTATTG AGTGCTTAGA GTGTACCAGG 100

CATGGTGCTA AATACTCTCT ATCCATTATC TCATTAAATC ACATGACACT 150

ATGAGAAATG TACTATTCTT ATACCCACGT TGCCCAGGGT CATACATCTA 200

AGGGGTGCAA GGACCAGGCT TTGATTTCAA ATTATAATCT AATGCTCACT 250

CTCCAGGCCT GATGTCACGC AGCTCCTCAT TCTTATACTT AACATA 296

(2) INFORMATION FOR SEQ ID :15:

(i) SEQUENCE CHARACTERISTICS:
(A) LENGTH: 123 base pairs
(B) TYPE: nucleic acid
(C) STRANDEDNESS: double
(D) TOPOLOGY: linear

(xi) SEQUENCE DESCRIPTION: SEQ ID :15:

CGTCAGTGTG CTACTTCACA TCATTAGCGA GGCCCAGAAA CTTGAACAGG 50

AAGTCCGGCA CTACCAACAT GCCGCCACTC ATACAACTCA ACTCTTCCTC 100

CAAACTCGAT TCAAAGAGCA ATA 123

(2) INFORMATION FOR SEQ ID :16:

(i) SEQUENCE CHARACTERISTICS:
(A) LENGTH: 262 base pairs
(B) TYPE: nucleic acid
(C) STRANDEDNESS: double
(D) TOPOLOGY: linear

(xi) SEQUENCE DESCRIPTION: SEQ ID :16:

CAACACATTA GTGCATCAAT ATGAATTACT TGTTTAAAAA ATCAAATGCT 50

GGCATTGTCA GAAAAATTTA ACAGGTTTAT TTATAATTAT CATAAAGTTG 100

ACGCTGAAAC TTGTTCACTG AAACATTTTA ACTTGCATTA ATGCTTTACG 150

TCTCCGCATT TATATTAAAA ATTCACACAC AAATGAAATG GAAAAACTGC 200

CAATACCTGA TTTCTGTCCC TATTTTTCAC TCGCAATCAT ATACTTAGTA 250

CTTTTGACTC TA 262

(2) INFORMATION FOR SEQ ID :17:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :17:

TAGCCAAATT CTAGGGCATC CAGATGCATT TGAGCCAGTC GTAATCCAGG 50

AAATGTCAAA AAAGCCCCAA TGAATGAACA GAAAATAGCC AGGAAAAATT 100

TGAAAGTAAG TTTTGAAACA GGACTCGTGG AGATTCTAAA CCTTGCATTT 150

TCAAGAAACG TGCATCAGC 169

(2) INFORMATION FOR SEQ ID :18:

(i) SEQUENCE CHARACTERISTICS:
(A) LENGTH: 221 base pairs
(B) TYPE: nucleic acid
(C) STRANDEDNESS: double
(D) TOPOLOGY: linear

(xi) SEQUENCE DESCRIPTION: SEQ ID :18:

AGGGGCACCA TTACCATCCA TCTGACATCG CATTTCCATA GAAATGGCCA 50

AAGAAAGAAG GTCCTGGTAG GTTTTTCATA GAAAGACTCA AAAAGTTCAA 100

CCTTTGATGC TATGCCCCAG CCCAATACAA AACTACACAG AACAAAGCAA 150

TTATTAAAAT ACTGGCTTCG GTTTCTTTTT TTCCTTTGCA AAGTTTCCTA 200

CATATATGTC TTTTACAGTA T 221

(2) INFORMATION FOR SEQ ID :19:

(i) SEQUENCE CHARACTERISTICS:
(A) LENGTH: 135 base pairs
(B) TYPE: nucleic acid
(C) STRANDEDNESS: double

(D) TOPOLOGY: linear

(xi) SEQUENCE DESCRIPTION: SEQ ID :19:

TAGGCTCTGT GACGGCATAG TTTTCAGTAG CTTTATCACA ATATTCACAA 50

TGGAGAATTA TATGACATGG TAGCAGAAAT AGGCCCTTTT ATGTGTTGCT 100

TCTATTTTAC CTGAAATTGT AGATATAGGG TAATC 135

(2) INFORMATION FOR SEQ ID :20:

(i) SEQUENCE CHARACTERISTICS:
(A) LENGTH: 314 base pairs
(B) TYPE: nucleic acid
(C) STRANDEDNESS: double
(D) TOPOLOGY: linear

(xi) SEQUENCE DESCRIPTION: SEQ ID :20:

GTTATCTATC TATCTTGCAG TTTACCTATC TGATCTGATC TCTGTAATTA 50

TAGTTCTGTC ATTTAAAATA TACTATTTAA ATCTAATTTT TACATTTCAA 100

AAATTATCTT CAGTAGTAAC TAAGTATATT TTCTGTGGAT TCTGAGAATG 150

TTATTTTTCA GAATGTGAGA GTACATATGT ACATTTATAA TCTTGTGACT 200

TTAAAGTCTG TTTTCAGATA CAGTATGTAA ATACTTGTAA AAAAAATTGT 250

ATAATTTTGT GATAATGTAG TTTCCCAAAA CACATTTAGA AAGCATTATG 300

TTATTAGTAA ATGA 314

(2) INFORMATION FOR SEQ ID :21:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :21:

TCTTCGCATC ACGCAGTACA GATACTCTTT CTGTACTTGC TTAATCTGCT 50

TTTTGGCATC AGTCAGTTCT CTTTCAGGTC AGCATAATCT TCTTCCTTCC 100

TCTGAAGATC TGCTTTCAGA TTCTGGGTAC GAGCAGAGCT TACAGAGAGT 150

TCCTCTTTCA ATATTTCTGT TTCTTGCC 178

(2) INFORMATION FOR SEQ ID :22:

(i) SEQUENCE CHARACTERISTICS:
(A) LENGTH: 188 base pairs
(B) TYPE: nucleic acid
(C) STRANDEDNESS: double
(D) TOPOLOGY: linear

(xi) SEQUENCE DESCRIPTION: SEQ ID :22:

ACATCTGATA TCCACCTCCA TGACAGACTG ATCACACCTC CTTCTATACT 50

ACTTCTGTTC TTTGTAAATA CTTAGTATTT TCCAAGGGAG TGTGAGAGAA 100

GAAATGCTAG GTTCATGAAG GGTCTCTAGG TTAAACTTTC TTTCTTTTTT 150

TTTCTTAAAA CAACACACTT ATTATCTTAC AAATCTGT 188

(2) INFORMATION FOR SEQ ID :23:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :23:

TTCCANCTAA ACATCCAATA TNTCNTTTAN TGCTTTTATA TTTTTNAAAT 50

GTTAAAACCC CTATACCACC TTTTGGGAAT GTTTTAAATT CTCCAATTTT 100

TCGTTATATA GGATCAACCA ACTAAGAAAA GATTTTATCA ATTGAATTGA 150

GG 152

(2) INFORMATION FOR SEQ ID :24:

(i) SEQUENCE CHARACTERISTICS:
(A) LENGTH: 83 base pairs
(B) TYPE: nucleic acid
(C) STRANDEDNESS: double

(D) TOPOLOGY: linear

(xi) SEQUENCE DESCRIPTION: SEQ ID :24:

ACAATACAGA GGACAAAGAC CCCTCACAGA ATGCTTTTCA ACCAACTTCA 50

ACTTTTGCAC ACTTTTTCAA CGGTCCCACC ACA 83

(2) INFORMATION FOR SEQ ID :25:

(i) SEQUENCE CHARACTERISTICS:
(A) LENGTH: 176 base pairs
(B) TYPE: nucleic acid
(C) STRANDEDNESS: double
(D) TOPOLOGY: linear

(xi) SEQUENCE DESCRIPTION: SEQ ID :25:

AAATGTCCAT TGCTGTATTG CGTGACCGTG GTCCTTGCCT GTCAAATNCA 50

ACAATGACCA AATGATGATG CGCCCTTAAT ACCAGGATGA GACCAAACCT 100

ACACATCTCC CAAGTGCCGA ACAAAAACCT GAACAAAAAC CATNTGCACC 150

CTACATCTGG CTGACATTTA CATTTT 176

(2) INFORMATION FOR SEQ ID :26:

(i) SEQUENCE CHARACTERISTICS:
(A) LENGTH: 232 base pairs
(B) TYPE: nucleic acid

(C) STRANDEDNESS: double
(D) TOPOLOGY: linear

(xi) SEQUENCE DESCRIPTION: SEQ ID :26:

AAATGTATGA TCAGAAAATA GGTACGCTTT TAAAATATTT GAACAGAAAA 50

GCTACAAATA AATNGAGCAA TGCTTTTAAA ATCATCTTTG TTTTATAGAC 100

TTTTTCTCGC ATGAATTACA TTTTACAATT TCATNNGGCA TATTCGCACT 150

TTAAGTACTG ACGAAGAAGA CTAAAACAAT CATTTTTTAA CAATATTTAA 200

AAGGATCATA TAGTCGACTT TTAAAACANC CC 232

(2) INFORMATION FOR SEQ ID :27:

(i) SEQUENCE CHARACTERISTICS:
(A) LENGTH: 192 base pairs
(B) TYPE: nucleic acid
(C) STRANDEDNESS: double
(D) TOPOLOGY: linear

(xi) SEQUENCE DESCRIPTION: SEQ ID :27:

GAGAAAGTCC TTGCCATTCT AAAAGCCACC CCACTTCTCT AAGGAGAATG 50

GCCCAATCTT CCCAAGTCCA CACAGGAGGG AAACATTGTT TGCGTAAATA 100

CGCAATGCAA AATTTNNTAT CTTGGCTTAA TACNNCGACG TTTTATTTCG 150

AATGATGAGC CTTCTGCCCC CCCTTCCCCT TTNNNCTCCC CC 192

(2) INFORMATION FOR SEQ ID :28:

(i) SEQUENCE CHARACTERISTICS:
(A) LENGTH: 201 base pairs
(B) TYPE: nucleic acid
(C) STRANDEDNESS: double
(D) TOPOLOGY: linear

(xi) SEQUENCE DESCRIPTION: SEQ ID :28:

TAACACTTTA ANCATCTCTC CAAGCCAAGG AGGCTATGAC ANTGTAGCTT 50

TTATACTGTC CCCATCGGCC ACAATAACAA ACTTTTAACC CTCATAAAAT 100

GAATGAAATC ATCTTTCTAA GAATCTATAC CTACAATTCT CTGAACTAAT 150

CAATGAAAAA GATGATCAAT TCTGACTAAC AAAGATATAT CGATTCCATT 200

T 201

(2) INFORMATION FOR SEQ ID :29:

(i) SEQUENCE CHARACTERISTICS:
(A) LENGTH: 275 base pairs
(B) TYPE: nucleic acid
(C) STRANDEDNESS: double
(D) TOPOLOGY: linear

(xi) SEQUENCE DESCRIPTION: SEQ ID :29:

AACCTAAGGC AGTTGACCCC ACCTTCCAAC ATGTTTTCAC TTTATTGGCC 50

CCTCCCTACA TTCGGGTTAG GTTCCATTTG ATTTGCACAA TAATGACTTT 100

ATTTCTCTTT GATCAGGATT TGGCACATAA AATCCTTTTA TCATAGAACT 150

AACTATTTTA ATTACATATA ATGTAACTAA TGGAGAGATT TATAGAGAAT 200

TTTGTTTTTT TGTCATATAC TCCATTTCGA AGACAGATAT GATAGAACTA 250

GAAATTAAGT TGCATTTCTG CAAGT 275

(2) INFORMATION FOR SEQ ID :30:

(i) SEQUENCE CHARACTERISTICS:
(A) LENGTH: 122 base pairs
(B) TYPE: nucleic acid
(C) STRANDEDNESS: double
(D) TOPOLOGY: linear

(xi) SEQUENCE DESCRIPTION: SEQ ID :30:

GTAAATNTAC AAAGATAACG TCGCAATTTT CTTAGATTTA AATCAAAGAC 50

ATTCATCAAC AAGATTTCGA ATGGAATATT CCAGAAATTT CTGAGCCATC 100

TGATCACAAC AACCGTCTTT GA 122

(2) INFORMATION FOR SEQ ID :31:

(i) SEQUENCE CHARACTERISTICS:
(A) LENGTH: 197 base pairs

(B) TYPE: nucleic acid
(C) STRANDEDNESS: double
(D) TOPOLOGY: linear

(xi) SEQUENCE DESCRIPTION: SEQ ID :31:

GGATTTGNAG CTTGAAGTTC AGAGCTGGTT TACCCAAAAA GGGAGCCAAT 50

AGAGATCTTC CCAATGAACC TCAATACACG ATCGTAATAC TCGCACAATG 100

AAATGTTAAG TATGATTCTA GACTTCACTG ACTATCACAA TGATATTTTC 150

TCGATCGCAC TAGTGCACAA CAAAACACGA TGAGTGCAAT GTGAAAC 197

(2) INFORMATION FOR SEQ ID :32:

(i) SEQUENCE CHARACTERISTICS:
(A) LENGTH: 97 base pairs
(B) TYPE: nucleic acid
(C) STRANDEDNESS: double
(D) TOPOLOGY: linear

(xi) SEQUENCE DESCRIPTION: SEQ ID :32:

ACAAAAGTCT GAAGAAATCA CCAACNACAA GACGATTAGA AAATATGTTG 50

TTGGGGTCAC AACTAAAAAG TCCCTGATCT ACATTGNNTT TCNACTC 97

(2) INFORMATION FOR SEQ ID :33:

(i) SEQUENCE CHARACTERISTICS:

(A) LENGTH: 260 base pairs
(B) TYPE: nucleic acid
(C) STRANDEDNESS: double
(D) TOPOLOGY: linear

(xi) SEQUENCE DESCRIPTION: SEQ ID :33:

CAAATAAAAC AANTNTTGTA AAGCTACAAT AGTTATATAC CAAAGCAATA 50

CCTATTACAT GCTTTACACA ATCCCATGAA AAAATAATTT AATAGCTCCT 100

AATCCCTGAT GCAAGGCACT TCAAAGCACC CGCACAAAAC TCCATGAAAC 150

AACATACAAT ACATCATTTA AATAACATAA ACGACTTTCA CACACTTGAC 200

CTAGGAAAAA ATAAAATCCA TACAACCACA GCTAAAAACA TGTTAAGATT 250

CACAATAAGA 260

(2) INFORMATION FOR SEQ ID :34:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :34:

GGACNTGCGC NNNANNNANA GCCANTGAAC NCAGCCACCA NTGCAAGAAG 50

ACTGCCTCTT NCCAGGCAAG ATTTTACTGG AGCAACANAA CCGGAGGTGT 100

GATCCAAAAT ACCTTCCTTN CCAAGCCCGG GGTNNNNGAT AAGGTGTGGA 150

NTTNGGTTAA AGACAAGG 168

(2) INFORMATION FOR SEQ ID :35:

(i) SEQUENCE CHARACTERISTICS:
(A) LENGTH: 173 base pairs
(B) TYPE: nucleic acid
(C) STRANDEDNESS: double
(D) TOPOLOGY: linear

(xi) SEQUENCE DESCRIPTION: SEQ ID :35:

CTCGNACACT GTGGAGAGCC TGCGNNNNNN NNGGTNTACT CAGGGGGACG 50

AAAAGGAGNN GAANAAGTGA CACNGCNGNT AGCAGAGNGC ACAGAGCTGT 100

GCTNNNGTGG TCCCTTAGNA GCCGAGNAGG TGGGCGCGAG GTGAANAAGG 150

TGCNNGTGCG AGAGTGCGTG ATT 173

(2) INFORMATION FOR SEQ ID :36:

(i) SEQUENCE CHARACTERISTICS:
(A) LENGTH: 134 base pairs
(B) TYPE: nucleic acid
(C) STRANDEDNESS: double
(D) TOPOLOGY: linear

(xi) SEQUENCE DESCRIPTION: SEQ ID :36:

AGCAAGTNNN NNNNNNCATC CTGAAAGTCA AAATGGAATT TGTGTTTATA 50

CAACTAATAA TGATTTTTAT TTGCTCAGTA CAGACTNATT TACAATGAAA 100

GTTTTGCTAA CCTTGGTAAG CTTGTTTACC GTTT 134

(2) INFORMATION FOR SEQ ID :37:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :37:

TGGCGCCTGT CCGAGAGCAA GTNGNNNNNN NNCCTGAAAG TCAAAATGGA 50

ATTTGTGTTT ATACAACTAA TAATGACCTT TTATTTGCTC AGTACAGACN 100

GATTTACAAT GAAAGTTTTG CTAACCTTGG TAAGCTTGTT AACCGTTTAC 150

ATGACTTCTT 160

(2) INFORMATION FOR SEQ ID :38:

(i) SEQUENCE CHARACTERISTICS:
(A) LENGTH: 140 base pairs
(B) TYPE: nucleic acid
(C) STRANDEDNESS: double

(D) TOPOLOGY: linear

(xi) SEQUENCE DESCRIPTION: SEQ ID :38:

CTAGTTTAAT GAATCTGAGG GGCTACTATA AACAATCCCA CCCTCACACG 50

ATTTTTTACC TTCNTTACTT NGCCCTTCAT TAGGCAACCC TAGCAGCACT 100

CCACCTCTAT TCTCGCACTG TCCAAGAGGC CCACCTAATC 140

(2) INFORMATION FOR SEQ ID :39:

(i) SEQUENCE CHARACTERISTICS:
(A) LENGTH: 203 base pairs
(B) TYPE: nucleic acid
(C) STRANDEDNESS: double
(D) TOPOLOGY: linear

(xi) SEQUENCE DESCRIPTION: SEQ ID :39:

AGCAAGTATC ANNNNNNNNN ATACATTTGA ATTCAAGTTG TTTTTTGTCA 50

AATTGTACAG TGTGTCAATT GATCTTCAAG CTGNNGGTGC CTAGAAATGG 100

GNCGGTGTCT GTAGCCCTGG CATGTGCACA CGGAGCATTT GCCACCACCG 150

CAAGCAAAAA GTCTGGGNGA AGTTCACCAA NGNCAAGAAN NATTANGGGA 200

AAA 203

(2) INFORMATION FOR SEQ ID :40:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :40:

GAAAAGCNNN NNNNNNNGGC TTAAAGAACA ATATGCTGAG ATGGAGAAGG 50

ACCTAGCGAA ANTNNNAACC TTTTAAGAAC TTGAANNACA ACAATCACAA 100

ACTAATGAGA AGATGTTCAC CTCTCTCCTG AAAACTATGC CCACCAGACC 150

GTTTAGCCTC TGCTCAAGCT 170

(2) INFORMATION FOR SEQ ID :41:

(i) SEQUENCE CHARACTERISTICS:
(A) LENGTH: 270 base pairs
(B) TYPE: nucleic acid
(C) STRANDEDNESS: double
(D) TOPOLOGY: linear

(xi) SEQUENCE DESCRIPTION: SEQ ID :41:

GCTGCATGTT TCCTTGNATT TGAGCTTGAA AGTTCAGAGC TGTTTACCCA 50

AAAAGGGAGC CAATAGAGAT CTTCCCAATG AACCTCAAAC ACGTCGTAAT 100

ACTCGCACAA TGAAATGTCA AGTATGATTC TAGACTTCAC TGACTCATCA 150

CAATGATATT TTCTCGATCG CACTAGGCAC AACAAAATAC GATGAGTGCA 200

ATGTGAAACA TCTACAAAGT AAATCACACA CTGTTTTTTT AAATNCATAG 250

AAATTTGATT TGTAATAAAA 270

(2) INFORMATION FOR SEQ ID :42:

(i) SEQUENCE CHARACTERISTICS:
(A) LENGTH: 245 base pairs
(B) TYPE: nucleic acid
(C) STRANDEDNESS: double
(D) TOPOLOGY: linear

(xi) SEQUENCE DESCRIPTION: SEQ ID :42:

AGAGCTGCAT GTTTCCTTGA TTTGNAGCTT GAAGTTCAGA GCTGTTTACC 50

CAAAAAGGGA GCCAATAGAG ATCTTCCCAA CGAACCTCAA TAACGATCGT 100

AATACTCGAC AATGAAATGT TAAGTATGAT TCTAGACTTC ACTGACTATC 150

ACAATGATAT TTTCTCGGAA TCGCACTAGT GCACAACAAA ATAGATGAGT 200

GCAATGTGAA ACAATCTCAA AGTAAATCAT ATACTTGTTT TCTTA 245

(2) INFORMATION FOR SEQ ID :43:

(i) SEQUENCE CHARACTERISTICS:
(A) LENGTH: 124 base pairs
(B) TYPE: nucleic acid

(C) STRANDEDNESS: double
(D) TOPOLOGY: linear

(xi) SEQUENCE DESCRIPTION: SEQ ID :43:

TACACTGNGA AAATGTACAA AGAAAGTATC CCCAAATNAT TTACAAAGCC 50

TAAATGTCCT TGATACACAT ACACGGNAGT ATGCAGACAA CAAAGATTAA 100

ATGAAGACAC TTTACACTTT TCGG 124

(2) INFORMATION FOR SEQ ID :44:

(i) SEQUENCE CHARACTERISTICS:
(A) LENGTH: 144 base pairs
(B) TYPE: nucleic acid
(C) STRANDEDNESS: double
(D) TOPOLOGY: linear

(xi) SEQUENCE DESCRIPTION: SEQ ID :44:

GAGTTCTCAC GAATACACAG GTTCAGAGAG ATGAGANGGA ANAACATAAG 50

GCAAATTCCT AACANNCGCT AATATAGGAG GCCGCTCGAT AGGATTTTAA 100

AAAAATAAAA ACAATCTTAA TAGTGGGACA AGGCCATCAG AAAA 144

(2) INFORMATION FOR SEQ ID :45:

(i) SEQUENCE CHARACTERISTICS:
(A) LENGTH: 177 base pairs

(B) TYPE: nucleic acid
(C) STRANDEDNESS: double
(D) TOPOLOGY: linear

(xi) SEQUENCE DESCRIPTION: SEQ ID :45:

ACTGATTCNN NNTGAAAATA CCCCCTTTCT CCATTAGTGG CATGCTCATT 50

CAGCTCTTAT CTTTATATTC CAGTAAGTTA TTTTGCTCTC ACTGTTTTAA 100

CAAAAAAAAA AACAACAACA TAAAAATCCT TGCAAACCAT GTCAATTGGA 150

GAAATTTAAT GTTTTTCATA ACATGAA 177

(2) INFORMATION FOR SEQ ID :46:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :46:

ACTGATTCTG CGAAAATACC CCCTTTTTAT TAGTGGCATG CTCATTCACT 50

TTATCTTTAT ATTCAAATAA GTTATTTCGC TTTCACTGTT TTAACAAAAA 100

AAAAAAAAAA AAAAAAATAC AGCCCTCCCC AATCGAAGAT TTCAACTTTT 150

TAATTCACAC GGAAAAACCA AGACAATTTC ACAACTTCTG GACACAACCA 200

TCAACACAGG ACATTTTTTT TACAGGCAAA TCACTTAAAA CAAAAAAGAT 250

CCCAGA 256

(2) INFORMATION FOR SEQ ID :47:

(i) SEQUENCE CHARACTERISTICS:
(A) LENGTH: 155 base pairs
(B) TYPE: nucleic acid
(C) STRANDEDNESS: double
(D) TOPOLOGY: linear

(xi) SEQUENCE DESCRIPTION: SEQ ID :47:

GAGAAAAGTC NNNNNNNNNA GGTTAATCTA ACTTTTCTTG CTTATTTCAG 50

CTATGATCTG AAAGGATGGA AGACACAAAA TGTATGNNTA AGGTATTTTT 100

AACAAAGATA CATGGGTAAA TTAACAGCAG TAATGTAAAA AAGACTGAGG 150

AGCAA 155

(2) INFORMATION FOR SEQ ID :48:

(i) SEQUENCE CHARACTERISTICS:
(A) LENGTH: 68 base pairs
(B) TYPE: nucleic acid
(C) STRANDEDNESS: double
(D) TOPOLOGY: linear

(xi) SEQUENCE DESCRIPTION: SEQ ID :48:

GCTTATTTTA CTATATCTAA AGGATAAAGC ACAAAATGAT GAATAAGATA 50

TTTNNACAAA GATACACG 68

(2) INFORMATION FOR SEQ ID :49:

(i) SEQUENCE CHARACTERISTICS:
(A) LENGTH: 244 base pairs
(B) TYPE: nucleic acid
(C) STRANDEDNESS: double
(D) TOPOLOGY: linear

(xi) SEQUENCE DESCRIPTION: SEQ ID :49:

CCTGAAAGCA AAGCCATNNA AAGCTTAGAG ACAAGCACNT GAGATGCAGG 50

GGCCTAGCTA AATTTNGAAC CTGNTGGAAC TTGAACCACA ACGATAAAAA 100

ATTACAGAAG AGTTCACCTC TTTCTGAAAA CTATCCACAG ACCGTTTACC 150

TCTGCTTCAA GCTANCAATA TATCAATGGC ACTCTCATAN CAGAAGAAAG 200

AAGTTCCTAC TAGCTCCTGA TTATATTNAA GAAGATGCCC ATGG 244

(2) INFORMATION FOR SEQ ID :50:

(i) SEQUENCE CHARACTERISTICS:
(A) LENGTH: 81 base pairs
(B) TYPE: nucleic acid
(C) STRANDEDNESS: double
(D) TOPOLOGY: linear

(xi) SEQUENCE DESCRIPTION: SEQ ID :50:

ACTCGNNCAG CGATAGTCGG AGCTCACCAA CAAAAACNCT NNNNCAGAAA 50

GGANAAAGNG CCGCCCTACG TGGTACACAC A 81

(2) INFORMATION FOR SEQ ID :51:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :51:

GTACTTACTG CACTATGAAA AGCAATAGAT CGTCCATAAG TTATAACCAA 50

AATCTCTCCC TCAGGAATAT ATTCCATACT ACTAACAGAC ATATTAAAAT 100

TTAGAGATTT CACTTCTGTC ATAGTAGCAT GATCCCAAAG TCGAACAGTT 150

TTGTCATCAG CAGAAAGAAT CTGTTTATCT CACTGCACCA CAGAGCTTTT 200

TTATACCAGA AGNATGACCA CTG 223

(2) INFORMATION FOR SEQ ID :52:

(i) SEQUENCE CHARACTERISTICS:
(A) LENGTH: 49 base pairs

(B) TYPE: nucleic acid
(C) STRANDEDNESS: double
(D) TOPOLOGY: linear

(xi) SEQUENCE DESCRIPTION: SEQ ID :52:

TATGGTTTNT TGTAAAAAAG CTCANNNANA AAGGGANNGG CTTAAGAGA 49

(2) INFORMATION FOR SEQ ID :53:

(i) SEQUENCE CHARACTERISTICS:
(A) LENGTH: 213 base pairs
(B) TYPE: nucleic acid
(C) STRANDEDNESS: double
(D) TOPOLOGY: linear

(xi) SEQUENCE DESCRIPTION: SEQ ID :53:

CAACGTCAGA GCACACNNNN NNCTCAGNGC ANAGGCATGA CAGGAGCTCA 50

TANCNATAAG CCATATAANN NTGTTACCTG TATAGAATGA TGAATTATCT 100

TTCTAGAGTC TATACCTACA GTTCTCTGAG CTAATCAATG GAAAAGATGA 150

TCAATCTGAC TAACAAGAGN AATTGATTCA TTTTCTTCCA CNCCCCTTCA 200

TTCAATAATC AAA 213

(2) INFORMATION FOR SEQ ID :54:

(i) SEQUENCE CHARACTERISTICS:

(A) LENGTH: 166 base pairs
(B) TYPE: nucleic acid
(C) STRANDEDNESS: double
(D) TOPOLOGY: linear

(xi) SEQUENCE DESCRIPTION: SEQ ID :54:

GCGTGTTGTG NGGAGTGGNN GTCTNGTCNT CCTGTTAAGG TTTTTGTGTG 50

CGTNNTTGCG TANGGGGNGG GTTTNGGCAG GTGTTGCCGG TAGCNAAACN 100

GTTGGCCCCA TNGCCNGNAT TGNNNCCCCN CNNGGGAANG GGGGGGGNGA 150

CCNNAGNGGG AAAAAA 166

(2) INFORMATION FOR SEQ ID :55:

(i) SEQUENCE CHARACTERISTICS:
(A) LENGTH: 75 base pairs
(B) TYPE: nucleic acid
(C) STRANDEDNESS: double
(D) TOPOLOGY: linear

(xi) SEQUENCE DESCRIPTION: SEQ ID :55:

ACCTCATAGC GAACTCGTCN TATAGANNNN ANNTGAGTCG AGCTCGATGT 50

NGNCGTTGTN GCTGCCAAGC GACAT 75

(2) INFORMATION FOR SEQ ID :56:

(i) SEQUENCE CHARACTERISTICS:
(A) LENGTH: 181 base pairs
(B) TYPE: nucleic acid
(C) STRANDEDNESS: double
(D) TOPOLOGY: linear

(xi) SEQUENCE DESCRIPTION: SEQ ID :56:

AATNNNNNCC TATTTTGTAA TTTTTTTGAA AAAAGTTCAA TGTTCAGTTT 50

TCCTTAGTTT TTACCTTGTT TTCTCTATAG GTCATGATTT CTGTGAAGCA 100

AAAAGATGCC TTTTACCATG AATTCTTGAG TTTACATCAA TAATATTGTA 150

TATTAAGGGG ATCAGAAGTA GGAAGGAAAA A 181

(2) INFORMATION FOR SEQ ID :57:

(i) SEQUENCE CHARACTERISTICS:
(A) LENGTH: 130 base pairs
(B) TYPE: nucleic acid
(C) STRANDEDNESS: double
(D) TOPOLOGY: linear

(xi) SEQUENCE DESCRIPTION: SEQ ID :57:

TCGAAGAGAC CTCAATCACC GTTTTTCAGG ATGTTTGATC ACAATACGAA 50

GATGACGNNA TCCAATTTCA GAACACCACA GGGCACTGGC ACACAGAGGG 100

GATTATTACA GAACCACTGA GATGACATTT 130

(2) INFORMATION FOR SEQ ID :58:

(i) SEQUENCE CHARACTERISTICS:
(A) LENGTH: 157 base pairs
(B) TYPE: nucleic acid
(C) STRANDEDNESS: double
(D) TOPOLOGY: linear

(xi) SEQUENCE DESCRIPTION: SEQ ID :58:

GTNATTTTGA AGGTCTCACA AATNTAAAGA CTTATTGTAG CCCATGAACA 50

CATTGACAAG TACAAAAATT ACAAAAATAT GCAGAAATAT TGAATAACTA 100

GAACACAAGC CACTGTTTCA ACTCCAGAAA AAAGAAAGGC TTTACTTTTT 150

CCATGAA 157

(2) INFORMATION FOR SEQ ID :59:

(i) SEQUENCE CHARACTERISTICS:
(A) LENGTH: 252 base pairs
(B) TYPE: nucleic acid
(C) STRANDEDNESS: double
(D) TOPOLOGY: linear

(xi) SEQUENCE DESCRIPTION: SEQ ID :59:

GAGGTGAAGT TGTTCTTTAT TGAATTGCAT TATNAATGNA TAGGCTGTGC 50

CTTTTTGNAC TCTCTATGAG TTCATGAATT TAACCAATAC GNCCACAAAT 100

GCTGCTGCTG TCACAGAGAG ATGCCGATAA AGGACACCCA CCACCAATTT 150

TTGAACAGGG AGGGGAGAAT CAACTCTGAA TGTGATGCAG TGACCAGGAG 200

AGAGGACCAT GTTAACAACA CCACACAAAT GCAAATGACT NGTTCTNAAA 250

CA 252

(2) INFORMATION FOR SEQ ID :60:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :60:

GACCTCGTTT TTCATCCTAT TATGGCGTTT ANCAGGCTAG ACAGAAACAC 50

GCCTTAACTT TANTTGCCAC AAATCTTAAT ATTTTCTCCA CTAATATTAG 100

AAAGGAAGCA ACAAATAATG TCGCTTTTCA CCTGACGTCT GGTTCAACTT 150

TCCGCCCAGC CTATTCCTGN GTCTTCCTCC TGCCTTTCTA ATGTCCCA 198

(2) INFORMATION FOR SEQ ID :61:

(i) SEQUENCE CHARACTERISTICS:
(A) LENGTH: 229 base pairs
(B) TYPE: nucleic acid

(C) STRANDEDNESS: double

(D) TOPOLOGY: linear

(xi) SEQUENCE DESCRIPTION: SEQ ID :61:

GAGGTGGCAT TATGTGAGAC AGCATTTGGT TAGGGAGTGC CAAGCATTCT 50

ACAGCATTTG ATGGTGGAAA TAGTCATGCT TTTTATTTCT GCTCTCTAGG 100

AATGTAAGGT GCACAGCAGG TCAGGGTACT GCTGTGTGAG ACAAAAGGTC 150

CAGGTAGAGG CAATTCCCCA GATGCAGGCA GGGCAGGTGC TCACTGGGCA 200

GAGTGCTTCT CATACACCTT CAGGAACCC 229

(2) INFORMATION FOR SEQ ID :62:

(i) SEQUENCE CHARACTERISTICS:

(A) LENGTH: 181 base pairs

(B) TYPE: nucleic acid

(C) STRANDEDNESS: double

(D) TOPOLOGY: linear

(xi) SEQUENCE DESCRIPTION: SEQ ID :62:

CATCCTGGGC CCTGGCCGAT GTGCATATCA ACANAAAAAA GGGACTGAAG 50

AACATCGGCC ATATCATCCA CACAAGCCAA ATCTCACACC TTTACTTTAA 100

ACCGCTTAAT GAATTTCATG ACCTTGAGGG CTAAAGATCG TTCTTCGGGC 150

AAGAGCTTTT GGACTGTTTT TAGAACAGAA T 181

(2) INFORMATION FOR SEQ ID :63:

(i) SEQUENCE CHARACTERISTICS:
(A) LENGTH: 180 base pairs
(B) TYPE: nucleic acid
(C) STRANDEDNESS: double
(D) TOPOLOGY: linear

(xi) SEQUENCE DESCRIPTION: SEQ ID :63:

GTTGATTGTG AATCATCCCT CTAACATATA ATCAANANTA TGAGTAGAGA 50

ATTTGGCAGA AACAAGAAAA GGACATGGGA TAACTTTTAG ATTTAAAGAG 100

GCAGGCTTGG AACACAAACT GGTATTCTGC TGACACACTG CTGCATATCA 150

TAAGGCTACT CCACAAGACC ATTAGAAGTC 180

(2) INFORMATION FOR SEQ ID :64:

(i) SEQUENCE CHARACTERISTICS:
(A) LENGTH: 120 base pairs
(B) TYPE: nucleic acid
(C) STRANDEDNESS: double
(D) TOPOLOGY: linear

(xi) SEQUENCE DESCRIPTION: SEQ ID :64:

ACAATGTTCC AACATATATA TGGGGAGGGG AGAACANTTA TCTGTATAAC 50

AGGGAACTGT GATTATTTAA AAATANGCNA GAACTTATTT CANCTGTGCT 100

TTAGAAANAA NTGTATACGG 120

(2) INFORMATION FOR SEQ ID :65:

(i) SEQUENCE CHARACTERISTICS:
(A) LENGTH: 70 base pairs
(B) TYPE: nucleic acid
(C) STRANDEDNESS: double
(D) TOPOLOGY: linear

(xi) SEQUENCE DESCRIPTION: SEQ ID :65:

CACATCGCTA TAATCCTTTC TGAGGACTTA AAACTTTATN CCACTTACCT 50

TTATGACTTT TAACAAGCCT 70

(2) INFORMATION FOR SEQ ID :66:

(i) SEQUENCE CHARACTERISTICS:
(A) LENGTH: 240 base pairs
(B) TYPE: nucleic acid
(C) STRANDEDNESS: double
(D) TOPOLOGY: linear

(xi) SEQUENCE DESCRIPTION: SEQ ID :66:

TTTCGAGCAA AATGTTTACA TTTACATGGA AATACACACT AAAACAGAAT 50

ATTTTCCTAA TCATGAAACT TCGCCAAAGC AAAATACAAA CTTCCAACGG 100

GAGGTCCACT CAACTAACAA CAATGATCCC CAAGCAGGGC ACCAAGAAAC 150

CTGGGGGACC CTTTNCAAAA AACCTCCTTT CAAGAGACCC TAATACTCTN 200

TCCACACACC CACACGATTT AGGAACTTGG ACATGTTCCT 240

(2) INFORMATION FOR SEQ ID :67:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :67:

GGAAGCACTA CATTTCATCC AAAGCTGGGT TGAGTTATTT TTGAACACTT 50

TACGATATGC TTAGGTAGGC TTTTAACTTG CTCCTCCAAA CAATATCTNT 100

TGGGAAAACA AGCCCTGTGG AGAGATCCTT CCATCAAGTC GCTTCAATTT 150

AACCTATTTC TAGAGGACTA GACATGCAGA ATCGTCAACT ACAGGGAATG 200

AAAAGTTCAA AAAGTAGATC CTACAAGATG AACGAGTACT TTTCTAAACA 250

TAAG 254

(2) INFORMATION FOR SEQ ID :68:

(i) SEQUENCE CHARACTERISTICS:

(A) LENGTH: 192 base pairs
(B) TYPE: nucleic acid
(C) STRANDEDNESS: double
(D) TOPOLOGY: linear

(xi) SEQUENCE DESCRIPTION: SEQ ID :68:

AGGCACCAAA GAAACACCAA GCAATAAAGT GAAAGACTAA CCAAGATTTG 50

ACATTGTATG NTTACTGTAT TCTTTAAGAA ACAACTACAA AAAGAAAATG 100

TCAACAAATN NNNACAACTG AGAACCTGGG AATTCCCGCA CGGAAGACAA 150

GAGATAACCT CTCCAATTTA ACACCGCTAG GNTTCTATNN TA 192

(2) INFORMATION FOR SEQ ID :69:

(i) SEQUENCE CHARACTERISTICS:
(A) LENGTH: 112 base pairs
(B) TYPE: nucleic acid
(C) STRANDEDNESS: double
(D) TOPOLOGY: linear

(xi) SEQUENCE DESCRIPTION: SEQ ID :69:

AGCAGCGNNN NNNNTNNAAA CAAAAGACAG GAGCAGAGAG GCCTGAGAGC 50

AGGAGGCGAA TTCGATCTCT CCTCACAAAC AGCCCAGGAA AATATACACC 100

CCGGGGGAAG CC 112

(2) INFORMATION FOR SEQ ID :70:

(i) SEQUENCE CHARACTERISTICS:
(A) LENGTH: 232 base pairs
(B) TYPE: nucleic acid
(C) STRANDEDNESS: double
(D) TOPOLOGY: linear

(xi) SEQUENCE DESCRIPTION: SEQ ID :70:

AAGGGTCTCC AATTTAATCC TTGGGTTGTT TTACCACTTC TTTCGTAAAT 50

TTATCAAGAT TTCTTTCGCA CAAATACTCT AGCGCCTCAC AACAAACCTG 100

ACCTTGCGCA GGAAGTCGAC CATCAGCACC CCCTTTACAA CATCGTTCAT 150

ATCACAATTG AGAAGATGAT GAATGAAGAT TCGCTTCCAA GTTCCAAGGG 200

CAGATTTATT CCTTTAACTG ACATTTCCAT GA 232

(2) INFORMATION FOR SEQ ID :71:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :71:

CNTGGATCCG NCCTTGTTAC GNCCAGGACG NCTGGACCGC AAAATGAATT 50

TTCACTTTTC GACCACCGCC AGAAGAGATG ATTTTCTCAT CATNAACAAG 100

GAACCTTTGA GGAGATCGAC TGAAAGACTA GCGNCCCNGT CAGATAAGAT 150

TTAGGGCTGA 160

(2) INFORMATION FOR SEQ ID :72:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :72:

AAGGAAGACT GGTTTGCCAT CCGAGATCAT TAAAAANGNC TGACCCTAAC 50

AATACGTACA AAAATATAAA ACGCAAATAA AAAATACAAA CAGATTCCTT 100

CTTAAAGTAC TTTTAAGAAA AAAAGCAGGN CCTTGGAAGT TTCGATTCTT 150

TTTTCCTCCC GTCGCAAATT CTATGTTTGG AT 182

(2) INFORMATION FOR SEQ ID :73:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :73:

CAAGAGGCAG CTGCCCCTCC CACCTCGGAG GNCTGAGAGG GNCTGTGNCG 50

ATGAGCTGGA CGAGCACAGC ACTAAAAAGG CTTGCCCTNG CACAATAACA 100

CTGAGAGGAT GATGAGAACA CNCTTGAAAT GCTTCATNCA CATGGGCAGG 150

ANAGGCTGCA CAATGAAA 168

(2) INFORMATION FOR SEQ ID :74:

(i) SEQUENCE CHARACTERISTICS:
(A) LENGTH: 221 base pairs
(B) TYPE: nucleic acid
(C) STRANDEDNESS: double
(D) TOPOLOGY: linear

(xi) SEQUENCE DESCRIPTION: SEQ ID :74:

AACCTTTATT CATCCTCCTN TCCAAGACCT ATGAGAAGGT TCCAGGCCCC 50

AGGAACACAG GCTTCTTGGC CCCAGATGCA CCTCCCTGCA CCCCGGGGTT 100

GTATACCACA CCCCGGGCCC CTAATCCCAG GCCCCGAGAT AGGAAAGCCA 150

ACTAGTTCTT TNTNTGTGAT TCAGTAGGCC TGACCTATAG NTGGAATGTC 200

NCTNTCCCTN NAATAAATTN C 221

(2) INFORMATION FOR SEQ ID :75:

(i) SEQUENCE CHARACTERISTICS:
(A) LENGTH: 127 base pairs
(B) TYPE: nucleic acid
(C) STRANDEDNESS: double
(D) TOPOLOGY: linear

(xi) SEQUENCE DESCRIPTION: SEQ ID :75:

AGACTGNTTG GGTCATCCGA GATCATTAAA AATGNCTGAC CCTAACAATA 50

GGCACAAAAA TAAAAACGAA ATAAAAAACC TCCTTANNTT CGAAGTATCT 100

TAGAAAAAAA CAGGGCCTTG AGTTCTG 127

(2) INFORMATION FOR SEQ ID :76:

(i) SEQUENCE CHARACTERISTICS:
(A) LENGTH: 180 base pairs
(B) TYPE: nucleic acid
(C) STRANDEDNESS: double
(D) TOPOLOGY: linear

(xi) SEQUENCE DESCRIPTION: SEQ ID :76:

AAAAGAACAG CAGTTTCACA TTTCACATAT TTGAAAAACA TTTCAAAACC 50

CTCTAATAAG TATTTAATGA AAATAAATTT ATCGAAGAGA AACAATGACC 100

ACAAAATTAA TACTACCAAA TCATTACTGA GACTCTTGCA TTACAATATT 150

TGGAGAGTAG GTGAAGAAAA TNTAGACCGA 180

(2) INFORMATION FOR SEQ ID :77:

(i) SEQUENCE CHARACTERISTICS:
(A) LENGTH: 142 base pairs
(B) TYPE: nucleic acid
(C) STRANDEDNESS: double
(D) TOPOLOGY: linear

(xi) SEQUENCE DESCRIPTION: SEQ ID :77:

GATTAANNNN NNNGCACCCN NNATTACTGG CACAGCTGGT GAATATTTTC 50

GTGGACTTTT GACTAGTGCA CCTGCGTGCG GGAAAACANT GATAAAACTG 100

TCACTTTAGC CNCNAACTAC AAGACCNGTT AGACTAGAGA GC 142

(2) INFORMATION FOR SEQ ID :78:

(i) SEQUENCE CHARACTERISTICS:
(A) LENGTH: 124 base pairs
(B) TYPE: nucleic acid
(C) STRANDEDNESS: double
(D) TOPOLOGY: linear

(xi) SEQUENCE DESCRIPTION: SEQ ID :78:

GGCCCTGAGA GCAGGACGGC GAATTCGATC TCCTCCTTCA CAAACAGCCC 50

ANGGAAANTA CACCCCGGGG AANNCCCCNC NCNNTTAGAA CCNNCAGGNT 100

CTGNCCCCCC CNNNGNCCCC CCCG 124

(2) INFORMATION FOR SEQ ID :79:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :79:

AGGTATAGTG TTCCCTAATC ATGTACTTGT GAAGCACCCG GANTTTTTCA 50

TATAGTCTAA AAGCTAGAAG AACAAGAGTG TATTTCGTGG GTGGATGTAT 100

NGTCACTTGC TGAANNNNTT GAAATACCAT TATCCCCCTT GCTAACNCCT 150

TTAAGNAAAN GCCNTTTTAA G 171

(2) INFORMATION FOR SEQ ID :80:

(i) SEQUENCE CHARACTERISTICS:
(A) LENGTH: 98 base pairs
(B) TYPE: nucleic acid
(C) STRANDEDNESS: double
(D) TOPOLOGY: linear

(xi) SEQUENCE DESCRIPTION: SEQ ID :80:

CTGAACAGTG TGGTCGAGCA TTCCAAGTCC GTGAAGGTGC AGGAGCGGTA 50

CGACAGTGCC GTGNGGGCAC CATGGCACCT NGACCACGGG CNCCTAAG 98

(2) INFORMATION FOR SEQ ID :81:

(i) SEQUENCE CHARACTERISTICS:
(A) LENGTH: 108 base pairs
(B) TYPE: nucleic acid
(C) STRANDEDNESS: double
(D) TOPOLOGY: linear

(xi) SEQUENCE DESCRIPTION: SEQ ID :81:

CCTCAACAAG TGCGAGACCT GGGCGTCCAA CCTGCCTACA ACCACAGCCG 50

CGAAAGAAGA AGACCTGCTG CTTCAATACA ACACGGGAGG ACCTGGCCAT 100

CAACATCA 108

(2) INFORMATION FOR SEQ ID :82:

(i) SEQUENCE CHARACTERISTICS:
(A) LENGTH: 180 base pairs
(B) TYPE: nucleic acid
(C) STRANDEDNESS: double
(D) TOPOLOGY: linear

(xi) SEQUENCE DESCRIPTION: SEQ ID :82:

ACACATNACC CACNGCCAGT ATATCTCGAA CAAAGTCTCA GACGATACTC 50

CCTGAAATTG TAAAGCTTAA TACAGGTTNT GGAAATCATT TAACACCCGA 100

GAATGTCCCA TCACAGTCTT CCGTCAAAAT TTAGCCTCAC AACAACAACA 150

ACGCCTACGA AATTCTAAAT TCAGAAGGAA 180

(2) INFORMATION FOR SEQ ID :83:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :83:

GGCTTCTGAT CGCCATCCTT GAACAGGGCA AAGGTGGCCT CACCAGCCTT 50

CGATGCAGCT GCCATGCGCG CCAGAATCAG CGAAGGCATA CCCTTACGTC 100

GTTGGACGCA TGTTTAGATG CCTTTGAGAC CGCCCAGAGA AGTCCTTGTC 150

CTTCTTAATA AACACCTCCT CGCCAACTGC GACGACCACA ATCACCGTA 199

(2) INFORMATION FOR SEQ ID :84:

(i) SEQUENCE CHARACTERISTICS:
(A) LENGTH: 214 base pairs
(B) TYPE: nucleic acid
(C) STRANDEDNESS: double
(D) TOPOLOGY: linear

(xi) SEQUENCE DESCRIPTION: SEQ ID :84:

CTTCGGTAGT GCCGCCGTGG TGCCACACAC CGTTGAGGTT GGAGTGGGCA 50

CAGGCATGGT ACCACCAGCC TCCCCGCTGG TACAGGGCAC AGTTACCTGA 100

GGGGAGAGAG AGAGTCCATG TCCTCTCACC AGAATAAAAG CCTCTACCTG 150

CACCTCACAG TGCAAGGCTT TTGCCAGGCA TCCCCTGGCC CCTCCCATTT 200

NACNGAATAC AACC 214

(2) INFORMATION FOR SEQ ID :85:

(i) SEQUENCE CHARACTERISTICS:
(A) LENGTH: 80 base pairs
(B) TYPE: nucleic acid
(C) STRANDEDNESS: double
(D) TOPOLOGY: linear

(xi) SEQUENCE DESCRIPTION: SEQ ID :85:

CCTGGGCAAG CTGAATGTGA AGTTGACCAG GCTAACTGAG AAGCAAGCCC 50

AGTACCTGGG CATGTCCTGT GATGGCCCCT 80

(2) INFORMATION FOR SEQ ID :86:

(i) SEQUENCE CHARACTERISTICS:
(A) LENGTH: 210 base pairs
(B) TYPE: nucleic acid

(C) STRANDEDNESS: double
(D) TOPOLOGY: linear

(xi) SEQUENCE DESCRIPTION: SEQ ID :86:

GGGGATAGCT GGCTCATCCT CGGAAAACAG ACCCACATCT CTATTCTTGC 50

CCTGAAATAC GCGCTTTTCA CTTGCGTGCT CAGAGCTGCC GTCTGAAGGT 100

CCACACGGCA TTGACGGGAC ACAGAAATGT GACTGTTACC GGATAACACT 150

GATTAGTCAG TTTTCATTTA TAAAAAAGCA TGACAGTTTA TTACTCTGTT 200

TCTTTTAATG 210

(2) INFORMATION FOR SEQ ID :87:

(i) SEQUENCE CHARACTERISTICS:
(A) LENGTH: 102 base pairs
(B) TYPE: nucleic acid
(C) STRANDEDNESS: double
(D) TOPOLOGY: linear

(xi) SEQUENCE DESCRIPTION: SEQ ID :87:

CACAAAAAGC ATGATCAGGG CTAGCCTCAA TACAGGGAGA AATCATGGAT 50

ATTTAAAAAT ACTTTTTTTG ATTCAGATTC CGGTATGACT GAAGANGCAA 100

CA 102

(2) INFORMATION FOR SEQ ID :88:

(i) SEQUENCE CHARACTERISTICS:
(A) LENGTH: 275 base pairs
(B) TYPE: nucleic acid
(C) STRANDEDNESS: double
(D) TOPOLOGY: linear

(xi) SEQUENCE DESCRIPTION: SEQ ID :88:

GATAGGCGCA TGCATACTAC GGCTAAGGAG AAACAATGTT CCTACATATT 50

ACGGGCAGTG AGAACATTAT CTGTATAACA GGAACTGTGA TTATTTAAAA 100

ATAGCAGAAC TTATCNGTCT GTGCTTTAGA AATAACTGTA TACAGTGTTA 150

TAAGTTGAAA AGAACTCAAA ATAACTAATA CAAATAAGAA CCTACGTATT 200

AGAATTCAAA AAAGCTGCTT TCTGTGAAGT CAATCAGCTA TATTAAAAAA 250

TGACACAAAT TCAAAACACG ATCAT 275

(2) INFORMATION FOR SEQ ID :89:

(i) SEQUENCE CHARACTERISTICS:
(A) LENGTH: 263 base pairs
(B) TYPE: nucleic acid
(C) STRANDEDNESS: double
(D) TOPOLOGY: linear

(xi) SEQUENCE DESCRIPTION: SEQ ID :89:

GCCTGATGGT ATATACTGTT TTGCAATTGC ATACAACTGT GCATTACAAA 50

TTAATAGTAA TTATGGTTTG GNNGTAAAAT CGAGTTTCAG AATAAAATNA 100

AAAACAATAA AATCCAAAGA ACGATGTAAA CAAAAAAGCT TTTGTTTTGT 150

TACAAAGTAT ATTAAGGATT TTCTGCTAAG ATTCAGTTTA AGAGTTTTCC 200

TCGTGAAAAC TAAGTAGAAA CACAATGCCA ACAGCTGGCC AGTAATCAGT 250

GCTGTGTACT CCA 263

(2) INFORMATION FOR SEQ ID :90:

(i) SEQUENCE CHARACTERISTICS:
(A) LENGTH: 108 base pairs
(B) TYPE: nucleic acid
(C) STRANDEDNESS: double
(D) TOPOLOGY: linear

(xi) SEQUENCE DESCRIPTION: SEQ ID :90:

GCGTCCTGCC CAACATCCAG GCCGTACTGC TGCCCAAGAA GACGGAGAGC 50

CACCACAAGG CCAAGGGCAA GTGAGGCCGC CCGCCGCCCC CGAGGGACCC 100

CTTTGAGA 108

(2) INFORMATION FOR SEQ ID :91:

(i) SEQUENCE CHARACTERISTICS:
(A) LENGTH: 206 base pairs

(B) TYPE: nucleic acid
(C) STRANDEDNESS: double
(D) TOPOLOGY: linear

(xi) SEQUENCE DESCRIPTION: SEQ ID :91:

GCCTTGGTGG AGGGCATGAG GCTATGATGA CTATTGGCTA TAATATGGAT 50

ATGGCTTTGG CTGATAGATT TGAAGAGACC TCAATTACGT TTTTCAGGAA 100

TGTTTGATCA TAGATACAGA GATGGTGGTC CAGTTTTCAG AGCACCACAG 150

GGCACTGTGT ACACATGAGG GGTTACCTTA CAGAGCCACT GAGAATATAT 200

TAATAA 206

(2) INFORMATION FOR SEQ ID :92:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :92:

CCTTGGTGGA GGGCATGAGG CTATGATGAC TATTGGCTAT AATATGGATA 50

TGGCTTTGGC TGATAGATTT GAAGAGACCT CAATTACGTT TTTCAGGAAT 100

GTTTGATCAT AGATACAGAG ATGGTGGTCC AGTTTTCAGA GCACCACAGG 150

GCACTGTGTA CACATGAGGG GTTACCTTAC AGAGCCACTG AGAATATATT 200

AATAAANNNG 210

(2) INFORMATION FOR SEQ ID :93:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :93:

AGGACTTCAT CCCAGACTCA CTTGTTCTGT TACAGAAACT AACCTAAAAG 50

GCTGGAAATT AAAGGATACA ACCTAAGAGG TTATAACAGC AGACTGGTAA 100

AACATGGCGA AAGGAGCTCT CTCTTTCCCC GCAGTCTACC AAGCTCCTGT 150

GCATTTTCAC CACATAGATC TGCTAGCTTA CAAATGATG 189

(2) INFORMATION FOR SEQ ID :94:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :94:

GAAGTGACAG GNAAGCTACA GGTGTATAAC AAATTTGTAA ACTAACCAAG 50

CACAATGTGG CAGGGCCTAG CTGCTACAAA GAAGACAATT TAACAAATAC 100

TCAACGCATG ACAAAAAACT CAGGACTGCA TTTGCACTAA TCGATAACGN 150

GTCATTTAAT 160

(2) INFORMATION FOR SEQ ID :95:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :95:

TGCTTTATAC ATNATNAAAG GTAGGCACTT CATAAAATTT GCATTTTGGT 50

AAAAGGCAAC AATTTGATGT CAGTATCTTA ATTGTGTCAT TAACTTTTTT 100

AAGAGAACAG ATTATCAAAA TTTTACGAAG AAGAAAAAAA NTATAGTTTT 150

TAAGGAAACT ACAGAAGGGA T 171

(2) INFORMATION FOR SEQ ID :96:

(i) SEQUENCE CHARACTERISTICS:
(A) LENGTH: 131 base pairs
(B) TYPE: nucleic acid

(C) STRANDEDNESS: double
(D) TOPOLOGY: linear

(xi) SEQUENCE DESCRIPTION: SEQ ID :96:

GTGGATAAGA AGATGTGTAC TTGGGAACAA GGCTAAAGCC ATTGGCTGAT 50

TTCCCCAACC TTTTTATTCG CGAAGAAACT CCAGTTGTTA ACTTTTTGAG 100

AGTTTTTTTT GGCAAAAGAA CTNCATTTAN C 131

(2) INFORMATION FOR SEQ ID :97:

(i) SEQUENCE CHARACTERISTICS:
(A) LENGTH: 279 base pairs
(B) TYPE: nucleic acid
(C) STRANDEDNESS: double
(D) TOPOLOGY: linear

(xi) SEQUENCE DESCRIPTION: SEQ ID :97:

GTCCTAATAT TTCTGTNCAG AAATTGTCAA CTTGACTTGA GAGTTGAGAA 50

GTAAAGTTAA GGCACTGACA ATATATCAGA ATGGAAGTCC TTAAGAGCAA 100

CTAACAGGTT CTTGATCAGA CTGACTATCT TTTCTTAAGT TCATAATATN 150

TTACTGTCCA GCCAACTATA CTTGGATCAA AATATCCTTC ATGAAGGGCC 200

ATAATGTATT GATGATCTGC TGTAACTTTG AGAAGCTTCC TGAAGCTCNT 250

TTTGAATAAA TTTATNGAAC TTATGAAGA 279

(2) INFORMATION FOR SEQ ID :98:

(i) SEQUENCE CHARACTERISTICS:
(A) LENGTH: 266 base pairs
(B) TYPE: nucleic acid
(C) STRANDEDNESS: double
(D) TOPOLOGY: linear

(xi) SEQUENCE DESCRIPTION: SEQ ID :98:

GTGAGTCTTT CTTCAACTAG GGGAATGTTT CCAGGGCACG CCAGGCCTCA 50

CTCACGCAGG CCTCCGCGAC AACTGTTCAG CACTGACTGA GGATGAAGTG 100

AAATCCTGAA AGCTGAGAGC CAGCGCCCTC ACACGAGGGC TGGGACGTAA 150

CAAAAGCCCA TCAAGAGTTT TGCCCAGGGC TTTCTTGAGC CTTGAAGCAT 200

GACGAGACCA GGACCCTTTA GGATTAAGCA AGTTTTATGC GGTCTNAAAA 250

AACTCCAGGG CCTCCA 266

(2) INFORMATION FOR SEQ ID :99:

(i) SEQUENCE CHARACTERISTICS:
(A) LENGTH: 206 base pairs
(B) TYPE: nucleic acid
(C) STRANDEDNESS: double
(D) TOPOLOGY: linear

(xi) SEQUENCE DESCRIPTION: SEQ ID :99:

GGGAACATTT ATTGACATGA AAAGGTGTTT CAGATATACT GATATGTCAA 50

ACAAATAGTA AAACAACATA GAGTAATGAT TCATTTTGGT AAAAAATATA 100

TATGTATATA TAGAAAAAAA TTCTGCAGGA CATATGCTAA ATTGGTAACA 150

GTGCTTACCC CTGGGAAGGG GGTATACGAT GTTGATTTAC TCTTTGGGTA 200

CGTATT 206

(2) INFORMATION FOR SEQ ID :100:

(i) SEQUENCE CHARACTERISTICS:
(A) LENGTH: 73 base pairs
(B) TYPE: nucleic acid
(C) STRANDEDNESS: double
(D) TOPOLOGY: linear

(xi) SEQUENCE DESCRIPTION: SEQ ID :100:

CGCAACTNTC NCTAAACATC CAGTTTTCTA ATNTAATAAA AAATGGCAGT 50

AATTATCCTC ACCTCTCAGG GAA 73

(2) INFORMATION FOR SEQ ID :101:

(i) SEQUENCE CHARACTERISTICS:
(A) LENGTH: 108 base pairs
(B) TYPE: nucleic acid
(C) STRANDEDNESS: double

(D) TOPOLOGY: linear

(xi) SEQUENCE DESCRIPTION: SEQ ID :101:

GAGCACAATC ATGNGCAAGC GCAACAGCAC ACCTTTATCC GAGTGAGACC 50

ACTATGCAGC NGAACAGAGA CTTCTTATCT CTTCTTCTTG ATACTTGAAT 100

ACTGCCCC 108

(2) INFORMATION FOR SEQ ID :102:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :102:

GAATTATCAA ACCATCNTTG CTGACATTAA TTTTCCAGCT TTGATCTTAN 50

NTCTNGCTTT AGTCCTCATA CAATGACTGT GTTTTTCTCA AACGATNTAT 100

CGTATAGGNA TCCTTCTAAG CAATCCTGCA CCCACAAAAA AGCTGCATCT 150

TCAATATAAC ANAAAAAGGN ATTTTGCAAA AAGTACAAGT TTTATGTCTN 200

CTGTTAACTG 210

(2) INFORMATION FOR SEQ ID :103:

(i) SEQUENCE CHARACTERISTICS:
(A) LENGTH: 76 base pairs
(B) TYPE: nucleic acid
(C) STRANDEDNESS: double
(D) TOPOLOGY: linear

(xi) SEQUENCE DESCRIPTION: SEQ ID :103:

CGTAGTTCGA TTCGAGTGGT CTATACAATC ACACCAAGCT TGATGTTGAT 50

GTCACCAAAA TTTCTTTCCA AAAAAA 76

(2) INFORMATION FOR SEQ ID :104:

(i) SEQUENCE CHARACTERISTICS:
(A) LENGTH: 167 base pairs
(B) TYPE: nucleic acid
(C) STRANDEDNESS: double
(D) TOPOLOGY: linear

(xi) SEQUENCE DESCRIPTION: SEQ ID :104:

CTTATGATTA ACTAAGCAAA TCTTTCATAG AAAGATATTA TCAAAGCTGA 50

AGAAATGCAA ACTTNAACAA AGTGCCGTGA GATTCCGGAA AAACCCTTAA 100

CCGATTGAAT GGTTTTTTAA GAATAAAAAA GAAGTCTGAT ACTGAACTAC 150

AAGTCGCAAG GAACATC 167

(2) INFORMATION FOR SEQ ID :105:

(i) SEQUENCE CHARACTERISTICS:
(A) LENGTH: 122 base pairs
(B) TYPE: nucleic acid
(C) STRANDEDNESS: double
(D) TOPOLOGY: linear

(xi) SEQUENCE DESCRIPTION: SEQ ID :105:

TATCTGCATA TCTAAACATA GAAAAGCACA ATAAAAACAC ATATCATAAC 50

CTCACGGGAC CACCATCATA CAGCAATTTG TCATGATCAA AAGAAACATC 100

ATTAGTCGTG CCATAACTGT AT 122

(2) INFORMATION FOR SEQ ID :106:

(i) SEQUENCE CHARACTERISTICS:
(A) LENGTH: 255 base pairs
(B) TYPE: nucleic acid
(C) STRANDEDNESS: double
(D) TOPOLOGY: linear

(xi) SEQUENCE DESCRIPTION: SEQ ID :106:

GAATTCTTCA CTCCAGATTT CCAATGCCAA GATACATTGA TACTGAACAT 50

GGAGGCAACC AGGCCCGTTT CCTCCTTTCA AAGTCAACCC TCACAGACTC 100

ATAATAATAT GCATGCCTGG GGGCAGGAGT CTGGAGCACC TATTCTNACA 150

GATGATGTTA TTTACAAGTG TTTATGGATC ACTTGAAGAA ACTTGCTGTG 200

TCCAGTGCTG CTTGAAGTGC TAATAATGTT AAAGACACTT AAGAAGATGA 250

AATAA 255

(2) INFORMATION FOR SEQ ID :107:

(i) SEQUENCE CHARACTERISTICS:
(A) LENGTH: 227 base pairs
(B) TYPE: nucleic acid
(C) STRANDEDNESS: double
(D) TOPOLOGY: linear

(xi) SEQUENCE DESCRIPTION: SEQ ID :107:

GGACGTGAAT TGGTGGAATA TTTACAAAGA AAACTGTTTT CTCAAAACAC 50

TGTTCATTGG TTGCAAGAAT GAATACTGAC TTCAGAACTC AAACAATGGA 100

AGAACTTGCA TTTTTATGGA ACTCAGTATT AAAAGAAAAT ATAATGTGAT 150

AGCACTTTGC AGATATGTCT AGACTGTGAT CTGAAGCATC GTAGTTTCCT 200

ATACCAAGAN ACANTTATGT GGTAAAT 227

(2) INFORMATION FOR SEQ ID :108:

(i) SEQUENCE CHARACTERISTICS:
(A) LENGTH: 162 base pairs
(B) TYPE: nucleic acid
(C) STRANDEDNESS: double

(D) TOPOLOGY: linear

(xi) SEQUENCE DESCRIPTION: SEQ ID :108:

GAAAAATNGC ATGAAAGAAG ACTCTTNNNN NGCCATACCA TGGTACAATA 50

ATCATNAAAA NACAANAACA AAAACAAACA CATAAAACCA CTCACATATA 100

CATGTAGATA CAACAACNAT ATAATATCAA TAAAAAAAAA ATAGNAAAAA 150

AAAAAAATAA CA 162

(2) INFORMATION FOR SEQ ID :109:

(i) SEQUENCE CHARACTERISTICS:
(A) LENGTH: 154 base pairs
(B) TYPE: nucleic acid
(C) STRANDEDNESS: double
(D) TOPOLOGY: linear

(xi) SEQUENCE DESCRIPTION: SEQ ID :109:

AATTAAAAAT ATTAAACAAA ACTACCACTC CTCCTTATTA AAGCCCATAA 50

AAATAAAAAA CGAAACCCGA GAACCAAAAT GAACGAAAAT CTTTCGCTTC 100

ATTCATCGTC CCCACAATNC CAGGCCTACC CCCCATACTG ATCATTCATT 150

GTTT 154

(2) INFORMATION FOR SEQ ID :110:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :110:

ATGTGCCAAG TAAAAAATCA ATTNGTNGCC TTTTTCCATT NCGCGGACAC 50

CCATAGGCAC CAAAAAAAGG TGCTAATAAG TAACATGTTT TAAGATGCAG 100

AATAAGCTAT GGAAACAAGG AATGCTCCAA GTGTCCCAGT CTTTCTCCTT 150

GCACTCCTTG TTAATAACAA TACACTATAT CA 182

(2) INFORMATION FOR SEQ ID :111:

(i) SEQUENCE CHARACTERISTICS:
(A) LENGTH: 94 base pairs
(B) TYPE: nucleic acid
(C) STRANDEDNESS: double
(D) TOPOLOGY: linear

(xi) SEQUENCE DESCRIPTION: SEQ ID :111:

GAAGGTGAGA ATAGGGTAGG GGAAACAGTA GGACAGGAAG TATTCACGTA 50

CNTCAAAACC AATGGTAGAA CATCACATTT CAAACTGCAA ACCA 94

(2) INFORMATION FOR SEQ ID :112:

(i) SEQUENCE CHARACTERISTICS:
(A) LENGTH: 92 base pairs
(B) TYPE: nucleic acid
(C) STRANDEDNESS: double
(D) TOPOLOGY: linear

(xi) SEQUENCE DESCRIPTION: SEQ ID :112:

TAGGGCAGTG AAACCTAATC TGCCTGATGC TATAANTGAN TGAATTACAT 50

GNTCATTNGT TAAATTTTGT TCTAAACCCA TTAGGAAATT GT 92

(2) INFORMATION FOR SEQ ID :113:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :113:

GATTGTTTTT GCATTGCGTG TGTCACACAG CAGCACAAAG ACAATATATG 50

TAAGCGTNNA TACACCAGAT TTGACACAAG AGATAGCGAA CACCACAAAG 100

ATTAGGACAG ACCGCGTATA GTAGCTCTGA GGAACTCCAA GAATCTAGAG 150

GG 152

(2) INFORMATION FOR SEQ ID :114:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :114:

TTGGCACTGA CATCCAGGAC AACAAATGCA GCTGGCTGGT GGTTTCAGTG 50

TCTGCAACGG GCCACTCCAG AACAGTACCA ATCCTGAAGG AAAATACNTG 100

GCAGAAGGAG GCTGAGAAAG TGGCTCCGTG AAGGCNCTAT AGAGGGCTGA 150

TCTGCCAGCA TGTCTTCAAT ATGAGGAAGG CA 182

(2) INFORMATION FOR SEQ ID :115:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :115:

GGCACTGACA TCCAGGACAA CAAATGCAGC TGGCTGGTGG TTTCAGTGTC 50

TGCAACGGGC CACTCCAGAA CAGTACCAAT CCTGAAGGAA AATACNTGGC 100

AGAAGGAGGC TGAGAAAGTG GCTCCGTGAA GGCNCTATAG AGGGCTGATC 150

TGCCAGCATG TCTTCAATAT GAGGAAGGCA NT 182

(2) INFORMATION FOR SEQ ID :116:

(i) SEQUENCE CHARACTERISTICS:

(A) LENGTH: 227 base pairs

(B) TYPE: nucleic acid

(C) STRANDEDNESS: double

(D) TOPOLOGY: linear

(xi) SEQUENCE DESCRIPTION: SEQ ID :116:

GAGGTTCTTG AATATGTATT TTTTACTGAA AAAATCATTC ATAAANTAAC 50

ATACAAAAAT GTACAAACAC ATGAGTAAAT AATGTAATGA CAAAGGACTA 100

TTTTCGGAAA AGTGTTTTTT AAAACANNCT AGATTTCAGT GCAAAAATGT 150

ACCCCTGGCA CCTCTTAAAA CGTAAGAGCA AGCTCAAAAA CACGTAGTGA 200

TGGAAATAAG CTAGCTACGC TCAATGC 227

(2) INFORMATION FOR SEQ ID :117:

(i) SEQUENCE CHARACTERISTICS:

(A) LENGTH: 172 base pairs

(B) TYPE: nucleic acid

(C) STRANDEDNESS: double

(D) TOPOLOGY: linear

(xi) SEQUENCE DESCRIPTION: SEQ ID :117:

CGAGAGATTG GTAATGAGGA AGCAATTTGG AGGGGNGGAA GCTACAANGA 50

NNNNGGGAAT TACAACAATC AGTCTTCAAA TTTTGGACCC ATGAAGGGAG 100

GAAATTTTGG AGGCAGAAGC TCTGGCCCCT ATGGCGGTGG AGGCCAATAC 150

TTGCAAAACC ACGAAACCAG GT 172

(2) INFORMATION FOR SEQ ID :118:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :118:

AATGATGGAA GCAATTTTGG AGGTGGTGGA AGCTACAATG ATTTTGGAAT 50

TACAACAATA GTCTTCAAAT NNGGACCCAT GAAGGGAGGA AATTTTGGAG 100

GCAGAAGCTC TGCCCCTATG NCGTGGAGGC CAATACTTTG CAAAACCACG 150

AAACCAAGGT GGCTATGGCG GTTCCAGCAG CAGCAGTAGC TATGGCAGTG 200

GCAGAAGATT 210

(2) INFORMATION FOR SEQ ID :119:

(i) SEQUENCE CHARACTERISTICS:
(A) LENGTH: 95 base pairs
(B) TYPE: nucleic acid
(C) STRANDEDNESS: double
(D) TOPOLOGY: linear

(xi) SEQUENCE DESCRIPTION: SEQ ID :119:

GGTAAACACA AAGAGTTTCT GATAGTGTCT GCACAACAGC AAACCAACAT 50

TTGGTGAGGA ATTAGCAATT TCTTGCCAAA GAAAATTGAT TCTGC 95

(2) INFORMATION FOR SEQ ID :120:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :120:

GGAGTATTTN AANNTTTCAA ACTTTATTAC TTAATGAAAC AGTTTCTATA 50

TACTGCTTCC AATATACTTT AATCCTTTTT TTCTCGTTAA ATTTTTTTTG 100

TTGTTCTTCA GTTGAGCTGA GATACTTTTA ATTACTTTTT ATTAACTGCT 150

TCCAGAAACC GTAACAGG 168

(2) INFORMATION FOR SEQ ID :121:

(i) SEQUENCE CHARACTERISTICS:
(A) LENGTH: 231 base pairs
(B) TYPE: nucleic acid
(C) STRANDEDNESS: double
(D) TOPOLOGY: linear

(xi) SEQUENCE DESCRIPTION: SEQ ID :121:

GGAGTATTTA TCTTTCAAAC TCCNTACTNA GTGAAACAGT TTCTATACCA 50

CTGCTTCCAA TTACTANCTN TTTTNTCNGT TAAATTTTCN NCTGTTTTTC 100

AGTTGAGCTG AGATACTTTT AATATNNNGT NACTGCTTCC AGAAACCGTA 150

ACAGGTGCAG GAATAATTGA TGATATCCAA GTAGAGGCTG ATGNCAGCTA 200

ATACATACTT CGGTGACNTT ATGCATCATG A 231

(2) INFORMATION FOR SEQ ID :122:

(i) SEQUENCE CHARACTERISTICS:
(A) LENGTH: 174 base pairs
(B) TYPE: nucleic acid
(C) STRANDEDNESS: double
(D) TOPOLOGY: linear

(xi) SEQUENCE DESCRIPTION: SEQ ID :122:

TCTATGGCAT GAATGTTTGC AACCAACNNN NNGGAAAGCC TTAAAGGAAT 50

AGCTGTTCAC ATAGGAGACC GTGACAATGC TGTACGCAAT GCTGCACTAG 100

ACACCATTGT ACGGTGTACA ATGTCATGGG ATCAGGTGTT CAAACTGATT 150

GGAATCTTTC TGAAAAGGAT ATGA 174

(2) INFORMATION FOR SEQ ID :123:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :123:

CTNNACAGGA GAAGNAAGTA ATGATCATTC CCCAAAAATG TTCTGTTATC 50

AACTGNNTTT ATAAAAAATC GATTGTGGGT AGAAGCAGAG AAAAGGCACT 100

TAGTAAAGAT ACTACATGAN GAAAAANNTC TGCCCTTTGA ATTCTTANGA 150

AACATNNNTNG NNGAATCAAT 170

(2) INFORMATION FOR SEQ ID :124:

(i) SEQUENCE CHARACTERISTICS:
(A) LENGTH: 157 base pairs
(B) TYPE: nucleic acid
(C) STRANDEDNESS: double
(D) TOPOLOGY: linear

(xi) SEQUENCE DESCRIPTION: SEQ ID :124:

AAGTAATGAT CATTCCCCAA AAATGTTCTG TTATCAACTG NNTTTATAAA 50

AAATCGATTG TGGGTAGAAG CAGAGAAAAG GCACTTAGTA AAGATACTAC 100

ATGANGAAAA ANNTCTGCCC TTTGAATTCT TANGAAACAT NNTNGNNGAA 150

TCAATNT 157

(2) INFORMATION FOR SEQ ID :125:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :125:

CAACTTGAAA TACATTATGA TGTCTGATAT GATTAAATAT CATTGAGNAT 50

CTTGCAAACA AAAAAAGCAA AAAATTAAAT CTCCATATCA ATCTTAAATT 100

CTTGGCATAT TTACTTCTGG TAAATATTAC TTCTGGTCCT TATTCTATAT 150

GTGTTATTGA AATTGTGTT 169

(2) INFORMATION FOR SEQ ID :126:

(i) SEQUENCE CHARACTERISTICS:
(A) LENGTH: 90 base pairs
(B) TYPE: nucleic acid
(C) STRANDEDNESS: double
(D) TOPOLOGY: linear

(xi) SEQUENCE DESCRIPTION: SEQ ID :126:

GTTTNGTTNN NNNNTGTTCC ACCTTTTGTT GAATTTTAGT TGTTAGGCTG 50

AACCTCCGAG CAGTTTNAGG ACTTGCCTGA GTTTTTCTTC 90

(2) INFORMATION FOR SEQ ID :127:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :127:

TTTAGTTTAC TTCTGTTCCA CCTTTTATTG AAATATTAGT TGTTAGGCTG 50

AAAGCCTCCA GTTAAGAACT TGCTGAGTTT TTTTGTTCAG CAACTTGACA 100

TTTACTATGC GCATTATATA NCTCAATTAT GTCTGTTTTT TATGCTAAGT 150

AGGAAAACCA 160

(2) INFORMATION FOR SEQ ID :128:

(i) SEQUENCE CHARACTERISTICS:
(A) LENGTH: 150 base pairs
(B) TYPE: nucleic acid
(C) STRANDEDNESS: double
(D) TOPOLOGY: linear

(xi) SEQUENCE DESCRIPTION: SEQ ID :128:

GCCCACAACT TACATCCTCA TTATTGGCGC CTACAAACTC AACTACGAAC 50

ACACTCACAG TCGCATCATA ATCTTTTGAG GACTTCAAAC TTACTCGGCT 100

ACCGCTTTTT GATGACTTCT AGCAAGCCTC GCTAACCTCC CTTACCCCCC 150

(2) INFORMATION FOR SEQ ID :129:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :129:

AGAGAGACAG ACACCATGGA GCCACAGGGG CAAGAGGNNG NTTTCCGAAG 50

CAGGGGANNG GCTATCACTC GGACGGACCN NNGCTCAACG AGTCCCACGA 100

GAACACACCA GAAATTTGTC ATTGCACTCA ACCAAAATCG ATATCAGCAA 150

TGAAAAACCC AAAACAGTTA CGANGCTAAT CC 182

(2) INFORMATION FOR SEQ ID :130:

(i) SEQUENCE CHARACTERISTICS:
(A) LENGTH: 219 base pairs
(B) TYPE: nucleic acid
(C) STRANDEDNESS: double
(D) TOPOLOGY: linear

(xi) SEQUENCE DESCRIPTION: SEQ ID :130:

GGACCATTCT GATCATCCTC ACTGGACGCC ACAGGGGCAA GAGGGTGGTT 50

TTCCTGAAGC AGCTGGCTAG TGGCTTATTA CTGTGACTGG ACCTCTGGTC 100

TCAATCGAGT TCCTCTACGA AGAACACACC AGAAATTTGT CATTGCCACT 150

TCAACCAAAA TCGATATCAG CAATGTAAAA ATCCCAAAAC ATNTTACCGA 200

TGCTTACTTC AAGAAGAAG 219

(2) INFORMATION FOR SEQ ID :131:

(i) SEQUENCE CHARACTERISTICS:
(A) LENGTH: 181 base pairs
(B) TYPE: nucleic acid
(C) STRANDEDNESS: double
(D) TOPOLOGY: linear

(xi) SEQUENCE DESCRIPTION: SEQ ID :131:

AATAATTCAT CCACTTATGG AGGAGGAGGA GAATGTGGAA GAGGTAAAAA 50

GCTGGGCACA AGTTCATATG CCTATGAGTC AGTAAAGACT GAAGTAATGT 100

CCTATGTTGA GCTGGTTATT TTGATATATG ATAATAATTA TCTTTGTAGT 150

AGAACATCGT TAACGGAATC ACAGATATAT C 181

(2) INFORMATION FOR SEQ ID :132:

(i) SEQUENCE CHARACTERISTICS:
(A) LENGTH: 188 base pairs
(B) TYPE: nucleic acid
(C) STRANDEDNESS: double
(D) TOPOLOGY: linear

(xi) SEQUENCE DESCRIPTION: SEQ ID :132:

GACCAGCAGC TGTGGACATT ATGAGAAAGT GAAACCTGAG ATCCNCTGTT 50

GATTGAGAAA TGCAACACCT CAAAATGNGG GACAGCTCCT GATTACCAGG 100

TAGAAGATGN AAACAACTGG GGTGTCCATT CAGGAGGAAA CAATTGCAAG 150

CTAAGAACTG TTGAGAGTGA AGCTGCATTT TCTGNACC 188

(2) INFORMATION FOR SEQ ID :133:

(i) SEQUENCE CHARACTERISTICS:
(A) LENGTH: 190 base pairs
(B) TYPE: nucleic acid
(C) STRANDEDNESS: double

(D) TOPOLOGY: linear

(xi) SEQUENCE DESCRIPTION: SEQ ID :133:

GAGGTTGGGT CGTTGCATCC ATCATCAGGA GTTGACTTGT TCTGAGCAAC 50

TGAACAGAAC ACACCGCGAT GCTCTCGACA CTCCGCTCCT GGACTTCAGT 100

CACGAGTGAG TTGATGGTGA CTTGACCTGA GAGATTTCAC AGAAGCTCTG 150

TGACTTGGTT GTGGAAGAAA TCTGAACTGT TCAAGTTAAC 190

(2) INFORMATION FOR SEQ ID :134:

(i) SEQUENCE CHARACTERISTICS:
(A) LENGTH: 235 base pairs
(B) TYPE: nucleic acid
(C) STRANDEDNESS: double
(D) TOPOLOGY: linear

(xi) SEQUENCE DESCRIPTION: SEQ ID :134:

ACATTACGAT CACTGATAGT TGGTGTGCGA CTGAAACCCA CATTNTGGTC 50

AATCTCCTCG TAGAGCTTCT TCTTCACCTG AGGATTGGCA GCAGGAAGGC 100

CAGGGTCCAT TTAACTAAGA GGTGGTGATC TCCACGCCAG CCCCAAAGAT 150

GTCCCCTATA TGGTGAGAAT GNGTCATCTG AAAGCAGCTC TGAGTCTTGA 200

TCTGGGCCAG CATTGCCATT ATTGAGTTTA TCTAG 235

(2) INFORMATION FOR SEQ ID :135:

(i) SEQUENCE CHARACTERISTICS:
(A) LENGTH: 147 base pairs
(B) TYPE: nucleic acid
(C) STRANDEDNESS: double
(D) TOPOLOGY: linear

(xi) SEQUENCE DESCRIPTION: SEQ ID :135:

GAGAAACAGT ACCATCCANG ACACGATGTG GTGGAGGTTG ACNCCGCTGT 50

ACCCCAGAGG AGCNCCACCT GTCCAAGATG CAGCAGAACA CTACAAAATC 100

AACTATGTTT TTTAGCAGAT GCAGGACTAG ACCCCCACGC AGCTCTG 147

(2) INFORMATION FOR SEQ ID :136:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :136:

GAAAAATGNG TTGANCCATT CATCCACAAA TTGACTTGCC TGAACAACCA 50

CCAAACAATA CACTAATGNT TCACACNTTT NCTTTTACTT GNACNTTAAG 100

NTCCCANTGA GTCACGGTGA CTTACCCTAA ACATCTCAAN NGTNNTCTGA 150

CTNAGAATGC GGAGGAGATC T 171

(2) INFORMATION FOR SEQ ID :137:

(i) SEQUENCE CHARACTERISTICS:
(A) LENGTH: 159 base pairs
(B) TYPE: nucleic acid
(C) STRANDEDNESS: double
(D) TOPOLOGY: linear

(xi) SEQUENCE DESCRIPTION: SEQ ID :137:

ACATATATGT GGTAGGATAG AGAGATGGNN NNNGTGTATG ACATAGGTGT 50

TTCTCGTGTG ATGAGGGTTT ATGTGTTATG TGGGGTGAGT GAGCCCATTG 100

TGTTGTGGTA ATATGTGAGG AGTATAGGCT GTGACTAGTA TGTTGAGTCT 150

GTAAGTAGG 159

(2) INFORMATION FOR SEQ ID :138:

(i) SEQUENCE CHARACTERISTICS:
(A) LENGTH: 179 base pairs
(B) TYPE: nucleic acid
(C) STRANDEDNESS: double
(D) TOPOLOGY: linear

(xi) SEQUENCE DESCRIPTION: SEQ ID :138:

CCCACGACTT ACATCCTATN ACNCTTCGCC TAGCAAACTA AAACTACGAC 50

NCACTCACAA TCGCTCATAA TCTCTTNAGG ACTTCAAACT NTCTCTNTGA 100

NCCTTTTGAT GACTTCTACA AGCCTCGCTA CCTCGCCTTA CCCCNTGTNC 150

TNCGGGAGAA CTCTCTGTGC TGTACCAGT 179

(2) INFORMATION FOR SEQ ID :139:

(i) SEQUENCE CHARACTERISTICS:

(A) LENGTH: 177 base pairs

(B) TYPE: nucleic acid

(C) STRANDEDNESS: double

(D) TOPOLOGY: linear

(xi) SEQUENCE DESCRIPTION: SEQ ID :139:

GACCTGGGAC GTAAATGATG AGACGGGTAC TTTGGCGGAC ATGAAGGAAC 50

TGGCATATGG AACCTTGGCT GTGAAGCTGC AGACTATAAG ACAGCATGAG 100

ACGACAATTC TGCTACTGCA ATGATGACAT CGTTTCAGAC CACAAAAAGA 150

AAGGCGATGA CCAGAGCCGC AAGGCNG 177

(2) INFORMATION FOR SEQ ID :140:

(i) SEQUENCE CHARACTERISTICS:

(A) LENGTH: 72 base pairs

(B) TYPE: nucleic acid

(C) STRANDEDNESS: double

(D) TOPOLOGY: linear

(xi) SEQUENCE DESCRIPTION: SEQ ID :140:

GTTTACAACA TTTACATCCT ATGAACTCAT GGATTATAAA ACATTTGTGA 50

CTTATACTGT CTNTGTCAGT TA 72

(2) INFORMATION FOR SEQ ID :141:

(i) SEQUENCE CHARACTERISTICS:
(A) LENGTH: 62 base pairs
(B) TYPE: nucleic acid
(C) STRANDEDNESS: double
(D) TOPOLOGY: linear

(xi) SEQUENCE DESCRIPTION: SEQ ID :141:

GTNGGCTGAA ATGAAANAAT AAAACCAAGA AACGAATTTA AGTATTNGTT 50

TTAGTACGNA AA 62

(2) INFORMATION FOR SEQ ID :142:

(i) SEQUENCE CHARACTERISTICS:
(A) LENGTH: 127 base pairs
(B) TYPE: nucleic acid
(C) STRANDEDNESS: double
(D) TOPOLOGY: linear

(xi) SEQUENCE DESCRIPTION: SEQ ID :142:

ACCAGTNNNT GATTGGTAAA TGGGAAATAT AATTGATTCT GATCACTCTT 50

GGTCAGCTTC TCTTTCTTTA TCTTTCTTTC TCCTTTTTTA AGAAAACGAG 100

TTAAGTTTAA CAGTTTTGCA TTACAGG 127

(2) INFORMATION FOR SEQ ID :143:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :143:

AATATAAAAG ACAGCAGTTT CACATTTCAC ATATTTGAAA AACATTTCAA 50

AACCCTCTAA TAAGTATTTA ATGAAAATAA ATTTATCGAA GAGAAACAAT 100

GACCACAAAA TTAATACTAC CAAATCATTA CTGAGACTTT TTGCATTACA 150

ATATTTGGAG AGTAGGTGAA GAAAATATAG AACAGAACAT GNACATTT 198

(2) INFORMATION FOR SEQ ID :144:

(i) SEQUENCE CHARACTERISTICS:
(A) LENGTH: 174 base pairs
(B) TYPE: nucleic acid
(C) STRANDEDNESS: double

(D) TOPOLOGY: linear

(xi) SEQUENCE DESCRIPTION: SEQ ID :144:

GTTTCTCTNT ACGTCATCCA CCTTGACATG ATGGGTCAGA AACAAATGGA 50

AATCCAGAGN CAAGTCCTCC AGGGTTGCAC CAGGGNNTAC CTAAAGCTTG 100

TTGCCTTTTC TTGTGCTGTT TATGCGTGTA GAGCACTCAA GAAAGTTCTG 150

AAACTGCTTT GTATCTGCTT TGNA 174

(2) INFORMATION FOR SEQ ID :145:

(i) SEQUENCE CHARACTERISTICS:
(A) LENGTH: 156 base pairs
(B) TYPE: nucleic acid
(C) STRANDEDNESS: double
(D) TOPOLOGY: linear

(xi) SEQUENCE DESCRIPTION: SEQ ID :145:

GAGAAATAGT ACTTTAAAAT AAAACTAACA TGGTTTGATC AGCTTGAAAT 50

AAGATTCATA AAATGTACCT TTTTTGATTG TTTTGTTCTN GAGTTTTCGA 100

TTGAGANCAT TTGGTAAAGA TAAAGAGGTT TCCTGGGTGG CAAAAAATTA 150

TTTTGG 156

(2) INFORMATION FOR SEQ ID :146:

(i) SEQUENCE CHARACTERISTICS:
(A) LENGTH: 151 base pairs
(B) TYPE: nucleic acid
(C) STRANDEDNESS: double
(D) TOPOLOGY: linear

(xi) SEQUENCE DESCRIPTION: SEQ ID :146:

AAGATTCNNN NNTCCATTGA ATGTTACCTG TGCCAGAATT AGAAAAGGGG 50

GTTGGAAATT GGCTGTTTTG TTAAAATATA TCTTTTAGTG TGCTTTAAAG 100

TAGATAGTAT ACTTTACATT TATAAAAAAA ATCAAATTTT GTTCTTTAAT 150

T 151

(2) INFORMATION FOR SEQ ID :147:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :147:

CTTTATTTTT CTTATACAGA TTCAGAGAAG TAAAANNCAG TACCAAACTC 50

CAGGTAANNT GGTTTGATCT GATCGATTTG GCTGCATACT TTCGGTACGT 100

ATAACATTCT AAACTTAAAA TAGAAATTTT TATATTACAA AACGAGGAAG 150

TAAAATTTTA AAAGTTAAAG TACTAGC 177

(2) INFORMATION FOR SEQ ID :148:

(i) SEQUENCE CHARACTERISTICS:
(A) LENGTH: 148 base pairs
(B) TYPE: nucleic acid
(C) STRANDEDNESS: double
(D) TOPOLOGY: linear

(xi) SEQUENCE DESCRIPTION: SEQ ID :148:

GATTCNNNNN TCCATTGAAT GTTACCTGTG CCAGAATTAG AAAAGGGGGT 50

TGGAAATTGG CTGTTTTGTT AAAATATATC TTTTAGTGTG CTTTAAAGTA 100

GATAGTATAC TTTACATTTA TAAAAAAAAT CAAATTTTGT TCTTTAAT 148

(2) INFORMATION FOR SEQ ID :149:

(i) SEQUENCE CHARACTERISTICS:
(A) LENGTH: 204 base pairs
(B) TYPE: nucleic acid
(C) STRANDEDNESS: double
(D) TOPOLOGY: linear

(xi) SEQUENCE DESCRIPTION: SEQ ID :149:

AGATTCNNNN NTGGNATTGA ATGTTACCTG TGCCAGAATT AGAAAAGGGG 50

GTTGGAAATT GGCTGTTTTG TTAAAATATA TCTTTTAGTG TGCTTTAAAG 100

TAGATAGTAT ACTTTACATT TATAAAAAAA ATCAAATTTT GTTCTTTATT 150

TTGTGTGTGC CTGTGATGTT TTTCTAGAGT GAATTATAGT ATTGACGTGA 200

ATCC 204

(2) INFORMATION FOR SEQ ID :150:

(i) SEQUENCE CHARACTERISTICS:
(A) LENGTH: 93 base pairs
(B) TYPE: nucleic acid
(C) STRANDEDNESS: double
(D) TOPOLOGY: linear

(xi) SEQUENCE DESCRIPTION: SEQ ID :150:

AACATCGAGG TCGTAAACCC TATTGTTGAT ATGGATCTCT ATGAATAGGA 50

TTGCGCTGTT ATCTCTAGGG AACCTCACCG TTGGCAAGTT ATT 93

(2) INFORMATION FOR SEQ ID :151:

(i) SEQUENCE CHARACTERISTICS:
(A) LENGTH: 151 base pairs
(B) TYPE: nucleic acid
(C) STRANDEDNESS: double
(D) TOPOLOGY: linear

(xi) SEQUENCE DESCRIPTION: SEQ ID :151:

AATCGAGAGA AAAAATGATG ACACTGTAGC AATATCGTCG GANTCCACCT 50

ACTTTGGGAT CAGCCTATCC ATCCGTGTCT TCCTATTTAA ATCGTCTATC 100

CTCTATCCTT CCCCTGTCTT TTTNTGAAAA GGAAAAAAAC CAGGAAGGTG 150

T 151

(2) INFORMATION FOR SEQ ID :152:

(i) SEQUENCE CHARACTERISTICS:
(A) LENGTH: 109 base pairs
(B) TYPE: nucleic acid
(C) STRANDEDNESS: double
(D) TOPOLOGY: linear

(xi) SEQUENCE DESCRIPTION: SEQ ID :152:

TCTGAGAGGA ATACTNNTAA GTGCAATGAA TATTGCAAAT TTTCCCCCCT 50

CTAAGTAATT CCCGATATTA GCAAANCANN NANATTAATG TCCCAGTGAA 100

TGTAGCCTC 109

(2) INFORMATION FOR SEQ ID :153:

(i) SEQUENCE CHARACTERISTICS:
(A) LENGTH: 136 base pairs
(B) TYPE: nucleic acid
(C) STRANDEDNESS: double
(D) TOPOLOGY: linear

(xi) SEQUENCE DESCRIPTION: SEQ ID :153:

AAAATTATTT TCACAGTCCC CCCCAACTCT CATTGCGTCG TTAAAGTCCC 50

TCCAATCCTT TTTTAGTTGT GAAAAAATAA GGGGCCTTTA AAGGAGGAGG 100

AGGAAAAGGG GAAAAAAACC CATAATGGGC CTAAAA 136

(2) INFORMATION FOR SEQ ID :154:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :154:

CATCTTCATC ACCATCACAA TACTCATCAT CACCACCCTT CATNCACTAT 50

CATCTTCTAT GACTGCAAAC TTCTTATCTT TCTCTTCATT ATAGAAAGTT 100

TCAAGATGAG TATACGCATC TATCATTCGA ATTGTGTCAT TAATTTGTAG 150

GGCCTCATTG 160

(2) INFORMATION FOR SEQ ID :155:

(i) SEQUENCE CHARACTERISTICS:
(A) LENGTH: 191 base pairs

(B) TYPE: nucleic acid
(C) STRANDEDNESS: double
(D) TOPOLOGY: linear

(xi) SEQUENCE DESCRIPTION: SEQ ID :155:

ACCAGTNNNN NNNGGTCAAA TAGGGAAATA TAATTCGATC TCTCGAATCA 50

ACTCTCTGGG TCAGCTTTCT NCTTNTCTTC TATCTTTNCT TNTCTCCTTT 100

TTTTAAGAAA AACGAGTTAA GTCTTAACAG TTCTCGCATT ACAGGCTTGT 150

GACTTCATGC TTACTGTAAA GTGGAAGTTG AGATATTTTA A 191

(2) INFORMATION FOR SEQ ID :156:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :156:

CAACTGAACG CTTTGGTCAG GCTGCTACAA TGGAAGGAAT TGNGGGCNAN 50

TTGGTGGAAC TCCTCCTGCN NTCAACCGTG CAGCTCCTGG AGCTGAATTT 100

GCCCCAAACA AACGTCGCCG ATACTAATAA GTTGCAGTG 139

(2) INFORMATION FOR SEQ ID :157:

(i) SEQUENCE CHARACTERISTICS:
(A) LENGTH: 172 base pairs
(B) TYPE: nucleic acid
(C) STRANDEDNESS: double
(D) TOPOLOGY: linear

(xi) SEQUENCE DESCRIPTION: SEQ ID :157:

GGTACAGAGC ACTCTGTACC AACACACAGA ATTTACTGTT CTGCAAATGA 50

CCAATACTAA AAATTTATAA AGATGTGCAC AATTNNNNGC AGGCAATCTT 100

TCTTTTGTTT ACAAGATACA ACATTTAACA GTTATTAAAT GTAATCCTGA 150

AGCACCCGCA AATTTACCTT TG 172

(2) INFORMATION FOR SEQ ID :158:

(i) SEQUENCE CHARACTERISTICS:
(A) LENGTH: 93 base pairs
(B) TYPE: nucleic acid
(C) STRANDEDNESS: double
(D) TOPOLOGY: linear

(xi) SEQUENCE DESCRIPTION: SEQ ID :158:

GGGTGGCTGT TNNGTATATC TCGTTAGTAA ATGTACATGC TCTTCAGGTT 50

CTAGGGCTCC TGTTAGGGGA GGGAGAAATG TTGGAAGNGG GGG 93

(2) INFORMATION FOR SEQ ID :159:

(i) SEQUENCE CHARACTERISTICS:
(A) LENGTH: 102 base pairs
(B) TYPE: nucleic acid
(C) STRANDEDNESS: double
(D) TOPOLOGY: linear

(xi) SEQUENCE DESCRIPTION: SEQ ID :159:

GNATTTTTTT ATTGATATAT CATAGTTGTA CAAACATTTG GGAGTNCANG 50

TNGATACTTT GATACTATCG TGTNNNNNGG ATAATCACCA AATTGGATTC 100

CA 102

(2) INFORMATION FOR SEQ ID :160:

(i) SEQUENCE CHARACTERISTICS:
(A) LENGTH: 205 base pairs
(B) TYPE: nucleic acid
(C) STRANDEDNESS: double
(D) TOPOLOGY: linear

(xi) SEQUENCE DESCRIPTION: SEQ ID :160:

GCTTTTNNNN NNNNNNNNNC AGGTTTATAT TTACAATAAT TATCTTCCTA 50

TAGAAGCAAT AANNCNAGTA TTCTCCAGTA ACAACACANN NNNATATTCT 100

ACTCATCAGA GTTGGGAAAA ATAGGAATAA AGCAGATTCC ATACAGAAAT 150

ACCGTACTCT GCATATGTAC AAATAAATTC AATATATTAA ATCATTTTGA 200

GCGGA 205

(2) INFORMATION FOR SEQ ID :161:

(i) SEQUENCE CHARACTERISTICS:
(A) LENGTH: 150 base pairs
(B) TYPE: nucleic acid
(C) STRANDEDNESS: double
(D) TOPOLOGY: linear

(xi) SEQUENCE DESCRIPTION: SEQ ID :161:

AACTTTTTTA ATGGGTCTCA AAATCTCTGA GACAAAATTA CTNNGNNNAA 50

AGTTGTTTTC CATATAAAAA ANNNNNNTGA TTTTTAAAAA ACTAANNAAC 100

TTAAAAACNT GCCACACGCA AAAAAAGAAA CCAAAAGTGG GNCACCAAAA 150

(2) INFORMATION FOR SEQ ID :162:

(i) SEQUENCE CHARACTERISTICS:
(A) LENGTH: 77 base pairs
(B) TYPE: nucleic acid
(C) STRANDEDNESS: double
(D) TOPOLOGY: linear

(xi) SEQUENCE DESCRIPTION: SEQ ID :162:

TATTCAAAAG AAAAACATGG GTAAAAATGA TAGTGTTAAA TCTTGGCTCT 50

GTGTACATAG ATAGATACCT GTTACAG 77

(2) INFORMATION FOR SEQ ID :163:

(i) SEQUENCE CHARACTERISTICS:

(A) LENGTH: 182 base pairs

(B) TYPE: nucleic acid

(C) STRANDEDNESS: double

(D) TOPOLOGY: linear

(xi) SEQUENCE DESCRIPTION: SEQ ID :163:

GAAAATTATT TTCACATCCC CCCCAACTTC TTGCTCTTAA TCCTCATCTT 50

TTAGTTGAAG AATAAGGCTT AAGAGAGAGA AAGGAAAAAC CATAATGGCT 100

AACTTAGCAG CACAACACGG TTCTTTTATC AAGGCGTNAT CATCATTTCT 150

CAAACTGACA TGCTACAGAA ATGTCTTCCA AA 182

(2) INFORMATION FOR SEQ ID :164:

(i) SEQUENCE CHARACTERISTICS:

(A) LENGTH: 182 base pairs

(B) TYPE: nucleic acid

(C) STRANDEDNESS: double

(D) TOPOLOGY: linear

(xi) SEQUENCE DESCRIPTION: SEQ ID :164:

GAGGGAGCAA AGAGAGCCAT AAAGGCTCGT GCTGGGAAAG AAAGCTGTTA 50

TGCTTAGACT TCTCTAGGTG AACTCAGAGT CTTCAAAGAG GAAATGTTAC 100

AAATTGTCAC CCGCCAGCTT TCTGGCCAGT AAGCAGAATG CCAGGTTGCT 150

CAGATTCACA GACATTTGCA AAACAGAAGA TG 182

(2) INFORMATION FOR SEQ ID :165:

(i) SEQUENCE CHARACTERISTICS:
(A) LENGTH: 119 base pairs
(B) TYPE: nucleic acid
(C) STRANDEDNESS: double
(D) TOPOLOGY: linear

(xi) SEQUENCE DESCRIPTION: SEQ ID :165:

GTATCTCCCT CTACATCCCT CCCGAAATAC TAGGAATACT TATTCTATAT 50

GAGACATATA TACCACCCAA GTTTTAACAC CATATCCCAT CGGCTGTTAG 100

TGTATATAAA AAGAAATAA 119

(2) INFORMATION FOR SEQ ID :166:

(i) SEQUENCE CHARACTERISTICS:
(A) LENGTH: 193 base pairs
(B) TYPE: nucleic acid
(C) STRANDEDNESS: double
(D) TOPOLOGY: linear

(xi) SEQUENCE DESCRIPTION: SEQ ID :166:

GTACAAAGCC ACTCATCCTC GTGTGCCTAT CACGTTTTCC AAACACATAG 50

GATCCCATCT CAGGAGCAGG ACCAGTGTTT AGCTAGATTA AACTTCGCTG 100

GTGATCTTGT TGATGCATAT AAAGTAATCT GGCATATATG GTTAAATTCA 150

AGATGTTATG GCAGAAGTGA CTTGTTTTGC TCAACAAGCA TTG 193

(2) INFORMATION FOR SEQ ID :167:

(i) SEQUENCE CHARACTERISTICS:
(A) LENGTH: 185 base pairs
(B) TYPE: nucleic acid
(C) STRANDEDNESS: double
(D) TOPOLOGY: linear

(xi) SEQUENCE DESCRIPTION: SEQ ID :167:

GTTCTGACTN AGAACTGAGC ACATAGCATT GACGCGGTAC CCTTGGAGAG 50

GGTGTGCTAG GAGGAGTGCT TGGCGAATTT GGACACGTAC TAATGTCTCT 100

GAGCCAGTCT GAATCTCTGT GAAGATGCCC CAGTGGAGGT GGCTGAAGAT 150

TAAATGGACA GTTTATAAAG TGTTCTGGAG GCCGA 185

(2) INFORMATION FOR SEQ ID :168:

(i) SEQUENCE CHARACTERISTICS:

(A) LENGTH: 163 base pairs
(B) TYPE: nucleic acid
(C) STRANDEDNESS: double
(D) TOPOLOGY: linear

(xi) SEQUENCE DESCRIPTION: SEQ ID :168:

GGACAACACA GCACCCATCC ACTACCCATT CAGAATTTAT ATAGAATATG 50

TACCCATGAT TATCTAGGTG AATCAATGCA ACAGTAGCTC TGATGTCCAG 100

ATTTCCTAGT CTATTATTTT GTGTACAGAT CCTCTAACCA CTTAGAAATA 150

ATTTTTAAAA ATA 163

(2) INFORMATION FOR SEQ ID :169:

(i) SEQUENCE CHARACTERISTICS:
(A) LENGTH: 202 base pairs
(B) TYPE: nucleic acid
(C) STRANDEDNESS: double
(D) TOPOLOGY: linear

(xi) SEQUENCE DESCRIPTION: SEQ ID :169:

AGACAAATTC NNNNTNNNNN NTGCCTGATA ATTTCAGATG CCACCGTATA 50

GCAAAGGGTG AACATGTTTT CAACCCTTTA ACTTTTTACG GTGTTTGAAG 100

ACCAGCTACT CCTTAATATT TATCAATGGA TTAAGAAGTT TAAGATTTTG 150

CAGATTTACA ATTTGGGTTT TTGTCTGGAG TTGCTTCGGT TTGAAGCCCC 200

CT 202

(2) INFORMATION FOR SEQ ID :170:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :170:

TCAGGGAACC AAAAAACTGG CTTGCTTGGC ACCCAGGGAC AGTAGCTGTT 50

TGGCTCTCCA CCCAATTAAA AAAACAAATC CCTGCCCTTT CCCCCACCCC 100

ACTAGCTAAG CAAGAGCAGA GCTCTGATGA AGAGCCAGTG CCGGGTGCCT 150

GGTGCCCAGG GCTGTAAA 168

(2) INFORMATION FOR SEQ ID :171:

(i) SEQUENCE CHARACTERISTICS:
(A) LENGTH: 144 base pairs
(B) TYPE: nucleic acid
(C) STRANDEDNESS: double
(D) TOPOLOGY: linear

(xi) SEQUENCE DESCRIPTION: SEQ ID :171:

AGAAGTCAAC TAAAGCTTCA ATGCATCAAT TTATAANGCC TCANAGATCA 50

GCAATTTATG ACACTTACAT TTACAGTCGA CCTTTACTAA CCAGGCAAAC 100

TTCCCGAAAT GATCAGGACT GATTCATCTC CTGAAATTNN CNGT 144

(2) INFORMATION FOR SEQ ID :172:

(i) SEQUENCE CHARACTERISTICS:
(A) LENGTH: 91 base pairs
(B) TYPE: nucleic acid
(C) STRANDEDNESS: double
(D) TOPOLOGY: linear

(xi) SEQUENCE DESCRIPTION: SEQ ID :172:

ACCCACAGNN NNNACCTAGA GGCCCAGCGC CCAGAGAGGC ACGTAGAAAT 50

GGGGACAGCA CGTTTATAGA CCACCAGAAA TTGAAGAGGA A 91

(2) INFORMATION FOR SEQ ID :173:

(i) SEQUENCE CHARACTERISTICS:
(A) LENGTH: 204 base pairs
(B) TYPE: nucleic acid
(C) STRANDEDNESS: double
(D) TOPOLOGY: linear

(xi) SEQUENCE DESCRIPTION: SEQ ID :173:

GATTTTTTAA TGGGTNGCCT CTTTTAGCTT GGAATATTAC GTTTACTTTA 50

ATCCAAGTCT AGGCCTTTTA AAGGGTCCTT AAAATTAAAG TTCAGAATGT 100

GAATCCCTTT GACATCTATT ACAGGTTATA GGACCTTTTT GGTGTGATTA 150

CGGTTTTCAA TACGATTGTA TAAATGAAGT TAACTTGGCA GAAGTTAAAA 200

TGGA 204

(2) INFORMATION FOR SEQ ID :174:

(i) SEQUENCE CHARACTERISTICS:
(A) LENGTH: 241 base pairs
(B) TYPE: nucleic acid
(C) STRANDEDNESS: double
(D) TOPOLOGY: linear

(xi) SEQUENCE DESCRIPTION: SEQ ID :174:

GTAAATTTCA CTACATCTTT TNNTNTGACT TTCATGCATT TCTCATACAT 50

TNNTNNCNGA TGCTTGACTT TATTGCTTCC TAGCAATAAT CTGCATTTAA 100

ACGAAAGGCG GTTCAATTCA TCAACTTGAA ATGACTATTT ATTTTTNAGG 150

ATTTTTTAGG GGAAGAGTAC CCATTTCGTT TATAAAAACA GATGACAAAT 200

TTCTTAAAGA AACAGAAGCA CAATACTTTC GAAATACAAC G 241

(2) INFORMATION FOR SEQ ID :175:

(i) SEQUENCE CHARACTERISTICS:

(A) LENGTH: 211 base pairs
(B) TYPE: nucleic acid
(C) STRANDEDNESS: double
(D) TOPOLOGY: linear

(xi) SEQUENCE DESCRIPTION: SEQ ID :175:

GGTGGGCTCN NNNANNANNG TTCTACACTG TGGTATAAAC TTGTCGTGGT 50

TCTCGTGATA GAAAACAGAC ACTGACCTTG GGTATAGTGG GCCTATAAAT 100

AACAAACCCT TGGGTACTCC TGAATAGGGG CAGGGCCCCC TGGGCCTCCC 150

TTACAGGTTT CTTCAGGGAA ATGGTCCCTG GGATAATTCT TTAGGGCCCT 200

TTGGCCCTTT T 211

(2) INFORMATION FOR SEQ ID :176:

(i) SEQUENCE CHARACTERISTICS:
(A) LENGTH: 252 base pairs
(B) TYPE: nucleic acid
(C) STRANDEDNESS: double
(D) TOPOLOGY: linear

(xi) SEQUENCE DESCRIPTION: SEQ ID :176:

AAAATAAGAA TAAACATCCA TAATAGTNAT AGATANATCC NTTTATTTAG 50

NACATCAGCA ACGACTGCTT CACACCAAAT CCCTTCCTCT GNGAATGATN 100

TATATAACTC TAGTACTGCT TTCCATGAAG AACCTTGCAA ATTGATTGAA 150

AGTCCAGGAT NNCTCGCTAA TCCTCCACCA TAAGATTTCT GACCTATGAT 200

AACTCCAAGG TGGCAAACAG AGAAATCAAC TTCCTTATAA ANAAATCCNA 250

AT 252

(2) INFORMATION FOR SEQ ID :177:

(i) SEQUENCE CHARACTERISTICS:
(A) LENGTH: 279 base pairs
(B) TYPE: nucleic acid
(C) STRANDEDNESS: double
(D) TOPOLOGY: linear

(xi) SEQUENCE DESCRIPTION: SEQ ID :177:

GCGGGCGACT TTCCATCCCT CGAACCAAGG CATGTTAGCA CTTGNCTCCA 50

GCATGTTGTC ACCATTCCAA CCAGAAATAG CACAAATGCT ACTGCGCGAG 100

TTGCAGCCAA TCTTCTCAAA GCAAACCGAC TTCCTAACAA CTNTCTACAT 150

CTGGCTCGCT GCAGGCGACT CAATGAAATC CATCTTTAAC ACCACAATCA 200

TTGTTTNACA CCCAGTGTGC AAGCCAGGAG GGCATGTTCT GAGTCTNTCC 250

ATCTGAAGAT ACCAGCTTCA AATACTAAT 279

(2) INFORMATION FOR SEQ ID :178:

(i) SEQUENCE CHARACTERISTICS:

(A) LENGTH: 157 base pairs
(B) TYPE: nucleic acid
(C) STRANDEDNESS: double
(D) TOPOLOGY: linear

(xi) SEQUENCE DESCRIPTION: SEQ ID :178:

TATGCCCTGA AATGAAACCT CTAAGTTTGA CAAAATACCC ACAAAAACAG 50

ACCTTCCTNG TTAACACTTT ATAAGAAGGT GTGACATTTG GTGGGTGGTC 100

GTTCTCAATT TATAAAANAA TAAATGACTT TAAAGGAGAA ATAAATTTAT 150

GTCAGGA 157

(2) INFORMATION FOR SEQ ID :179:

(i) SEQUENCE CHARACTERISTICS:
(A) LENGTH: 39 base pairs
(B) TYPE: nucleic acid
(C) STRANDEDNESS: double
(D) TOPOLOGY: linear

(xi) SEQUENCE DESCRIPTION: SEQ ID :179:

GATAATGCAA CTTTTGACAG GAAAGCGCGA TTTTACTAT 39

(2) INFORMATION FOR SEQ ID :180:

(i) SEQUENCE CHARACTERISTICS:
(A) LENGTH: 157 base pairs

(B) TYPE: nucleic acid
(C) STRANDEDNESS: double
(D) TOPOLOGY: linear

(xi) SEQUENCE DESCRIPTION: SEQ ID :180:

GCAAACTCAA ACTACGGACG CACTCACAAT CGCTCATAAT CCTCTCTAAG 50

GACTTAAACT CTACTACACT AATACTTTTT GATGACTTCT AACAAGCCTC 100

GCTAACCTCC CTTACCCCCC ACTATTAACA CGGGAGAACT CTCTGTCTAG 150

TACCACA 157

(2) INFORMATION FOR SEQ ID :181:

(i) SEQUENCE CHARACTERISTICS:
(A) LENGTH: 195 base pairs
(B) TYPE: nucleic acid
(C) STRANDEDNESS: double
(D) TOPOLOGY: linear

(xi) SEQUENCE DESCRIPTION: SEQ ID :181:

CCATCCAGGC CAAATAAGCN CCGGCTATGC CCNTGTATTG GATTGCCACA 50

CGNCTCACAT TGCATGCAAT TTGCTGAGCT GAAGGAAAAG ATTGATCNCC 100

ATTCTGGTAA AAACTGGAAG ATGACCCTAA ATTTTGAAGT TGATGATGTG 150

CCATGTGATA TGGTTCTGAC AAACCCATGT GCGTTGAGAG CTTTT 195

(2) INFORMATION FOR SEQ ID :182:

(i) SEQUENCE CHARACTERISTICS:
(A) LENGTH: 146 base pairs
(B) TYPE: nucleic acid
(C) STRANDEDNESS: double
(D) TOPOLOGY: linear

(xi) SEQUENCE DESCRIPTION: SEQ ID :182:

CATGGTCTTA ACCAGTGTCA GATGGAATCA GTGGATAAAT CCCCAGGTTT 50

GTTTGTCCTT CAAATGGGAC AATTTGAGGA ATGCTTTAGG CAGAGGACTC 100

AGATGACAGA GCGCCAACCA CCCACAATAG AAACCTGCTC ATCACA 146

(2) INFORMATION FOR SEQ ID :183:

(i) SEQUENCE CHARACTERISTICS:
(A) LENGTH: 50 base pairs
(B) TYPE: nucleic acid
(C) STRANDEDNESS: double
(D) TOPOLOGY: linear

(xi) SEQUENCE DESCRIPTION: SEQ ID :183:

TAGAGGAATA GGGNNNGNGA CGCCCCNAGT TGTAGGGACG GACGGAGGAC 50

(2) INFORMATION FOR SEQ ID :184:

(i) SEQUENCE CHARACTERISTICS:
(A) LENGTH: 248 base pairs
(B) TYPE: nucleic acid
(C) STRANDEDNESS: double
(D) TOPOLOGY: linear

(xi) SEQUENCE DESCRIPTION: SEQ ID :184:

ACGNTTACGG TCACTGATAG TTGATGTGCN NCTGAAACCC ACATCCGATC 50

AATCTCCTCA TAGAGCTTCT TCTTCACCTG AGGATTGCGC AACAGGAAGG 100

CCAGGATCCA TTTACCACAG AGGCGGGATC TCCACGCCAA CCCCAAAGAT 150

GTCCCCTATG ACGACGAGAA TGTGATTATC TGAAAGCAAC TTGATCTTGA 200

TCTGGGCCAG CATGCCTCAT CTGATTCATC TCGCTTCCAT CAATGNGT 248

(2) INFORMATION FOR SEQ ID :185:

(i) SEQUENCE CHARACTERISTICS:
(A) LENGTH: 113 base pairs
(B) TYPE: nucleic acid
(C) STRANDEDNESS: double
(D) TOPOLOGY: linear

(xi) SEQUENCE DESCRIPTION: SEQ ID :185:

AATGATGAGT AAAAGATTCA ACAACTCACA GCCCTGGGGG CACTCAGGCT 50

ACTGCTAAGG CCTGAGAGTT TTGCAAAAAT GCGCAGAGAA ACACCCTTTG 100

AACGTGGCTT TCT 113

(2) INFORMATION FOR SEQ ID :186:

(i) SEQUENCE CHARACTERISTICS:
(A) LENGTH: 148 base pairs
(B) TYPE: nucleic acid
(C) STRANDEDNESS: double
(D) TOPOLOGY: linear

(xi) SEQUENCE DESCRIPTION: SEQ ID :186:

GGGCCCCCTG ATCAATTCTT TGGATGCTTT TCAAATTTCC CAGGATCCCG 50

ATGTCGTCAT ACACTCCGAA CATGACCCTT TTTTCTTCCA ACGATCAACC 100

ACTNCGNGGG ACGGGAGAGT GAGCCTTATA CCGATCAATC TGCACACC 148

(2) INFORMATION FOR SEQ ID :187:

(i) SEQUENCE CHARACTERISTICS:
(A) LENGTH: 248 base pairs
(B) TYPE: nucleic acid
(C) STRANDEDNESS: double
(D) TOPOLOGY: linear

(xi) SEQUENCE DESCRIPTION: SEQ ID :187:

TCCTTACTGT AAGAGCCATT TAAACGATTA AAATCCCACT NGCCATACCG 50

TAAGATGATA GGACCAACCA TACCTACCGA TCAAAAATTT ATCAATCCAA 100

GCCAACTACA CTCCCACTGC TAAAAAGATG AAAGGACCAA TCAAAGATTT 150

AATTAAACTA AAGGGAAAGA ATCAGAGACA GAGAATGAAG AAAGAAATTC 200

TAAGTTGCGA CGGACAAACC AGAACAGACA ATGAAGCCTT TCAACTGC 248

(2) INFORMATION FOR SEQ ID :188:

(i) SEQUENCE CHARACTERISTICS:
(A) LENGTH: 146 base pairs
(B) TYPE: nucleic acid
(C) STRANDEDNESS: double
(D) TOPOLOGY: linear

(xi) SEQUENCE DESCRIPTION: SEQ ID :188:

TATGAAGAAG TGCAGCACTG GCCAACACCA GGGTTTACTG AATCATTTCA 50

GTTTAATACA TAAGTGTCCA ATAATAATGT CAACCCTCCC TCGCCACAGC 100

CAATAATTTG TCCTCACTGA GTTGGCAACA AGTGACTGCT GTGACT 146

(2) INFORMATION FOR SEQ ID :189:

(i) SEQUENCE CHARACTERISTICS:
(A) LENGTH: 81 base pairs
(B) TYPE: nucleic acid
(C) STRANDEDNESS: double
(D) TOPOLOGY: linear

(xi) SEQUENCE DESCRIPTION: SEQ ID :189:

ATTTACCACA AGGGACGATT TCCACACCAA CCCCAAAAAT GTCCCCTATG 50

ACGACGAAAT GTGTATTTGA AACAGCTCTG A 81

(2) INFORMATION FOR SEQ ID :190:

(i) SEQUENCE CHARACTERISTICS:
(A) LENGTH: 136 base pairs
(B) TYPE: nucleic acid
(C) STRANDEDNESS: double
(D) TOPOLOGY: linear

(xi) SEQUENCE DESCRIPTION: SEQ ID :190:

AAGTACAGCA TCCTGCTGCA AAAATGATTG TAATGNCTTC TCATATGCAA 50

GAGCAAGAAT TGAAGATGCA CAAACTTCGT TCTGAATTTG TGAGCTTCCT 100

GGATCAACTG AAGAACTTCT GAGGATTGAC CTGTCA 136

(2) INFORMATION FOR SEQ ID :191:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :191:

AGGATTTAAG ATGGGGACAG ACTGGTGAAA ATGCGGCTGA CTGGAAGGAA 50

ATGGGGCATA CGAGTAATAT GTACATATCA AACAATCAAT TGCCTCCTGA 100

AATCAAAAAA TCAAATGGTG AAATGGAGCC TCCTTGATAG TTTAGGCCAA 150

CA 152

(2) INFORMATION FOR SEQ ID :192:

(i) SEQUENCE CHARACTERISTICS:
(A) LENGTH: 65 base pairs
(B) TYPE: nucleic acid
(C) STRANDEDNESS: double
(D) TOPOLOGY: linear

(xi) SEQUENCE DESCRIPTION: SEQ ID :192:

TAAGCGAGGN NGTCTTTGAT TGCGTAGTAA GGTAAAGACG ATTTTATAGA 50

ATNAAGGTGA TTCCT 65

(2) INFORMATION FOR SEQ ID :193:

(i) SEQUENCE CHARACTERISTICS:
(A) LENGTH: 249 base pairs
(B) TYPE: nucleic acid
(C) STRANDEDNESS: double
(D) TOPOLOGY: linear

(xi) SEQUENCE DESCRIPTION: SEQ ID :193:

AGGACCCAAA CTACCTTATT GCATTTGAAG TTTTACTTAT NCTATTATAA 50

TCTAAGAGCC CACCCAACAA GGCACTACAC ATAGATGCTC ACACTCTATA 100

GGCTGCCTGA TCCTGGACCA CCTGGGGCCC TGATTATGAT CTCCACGGGG 150

CTGTCAATGA CTAGGGAAAG CTTTTTAAGA CCCAGCGATC ATGCAATGGC 200

TCAACCATGG CGAATCAAAG TTAGCATAAT GTCCCAGCAA ACAATGTTA 249

(2) INFORMATION FOR SEQ ID :194:

(i) SEQUENCE CHARACTERISTICS:
(A) LENGTH: 194 base pairs
(B) TYPE: nucleic acid
(C) STRANDEDNESS: double
(D) TOPOLOGY: linear

(xi) SEQUENCE DESCRIPTION: SEQ ID :194:

GGAAAGGGTG CCTCATCCCA GCAACCTATC CTTGTGGGNG ATGATCACTG 50

TGCTGCTTGC NNCTCATGGC AGAGCATTCA TGCCACGATT TAGGTGAATC 100

GCTGCATATG TGACTGTCAT GAGATCCTAC TAGATGATCC TGACTAGAAT 150

GATAATTAAA AGTATTTACT TCGAAGCACC ATTTGAATGN TCAT 194

(2) INFORMATION FOR SEQ ID :195:

(i) SEQUENCE CHARACTERISTICS:
(A) LENGTH: 121 base pairs
(B) TYPE: nucleic acid
(C) STRANDEDNESS: double
(D) TOPOLOGY: linear

(xi) SEQUENCE DESCRIPTION: SEQ ID :195:

GTGATTTTAG TTTAAGGATA AGAAGCCACT ATATCAACGT CGGGGGGGTA 50

TTTAAGTCAC ACACATAGTT AACAACNCNC GTNGCGTGCA ATAAATACCA 100

CATCCTTTNA TATGNNCNGN A 121

(2) INFORMATION FOR SEQ ID :196:

(i) SEQUENCE CHARACTERISTICS:
(A) LENGTH: 175 base pairs
(B) TYPE: nucleic acid
(C) STRANDEDNESS: double
(D) TOPOLOGY: linear

(xi) SEQUENCE DESCRIPTION: SEQ ID :196:

GGGCTTCNNN NNNNNNCATG TGCACTTAGA ACCGTTACTA ACCGAAACAC 50

CATTTGCTTG TCAACAATGT ACCCTTGACA GCAGGGAGAA ACTTCTTTAT 100

AGTCTCTGCT TCAGACAAGA TTTACNGCTT TCTCCAAGGC CAGAGGCAAA 150

TTGTGACCAC AAGTCTTGTT TCTTG 175

(2) INFORMATION FOR SEQ ID :197:

(i) SEQUENCE CHARACTERISTICS:
(A) LENGTH: 273 base pairs
(B) TYPE: nucleic acid
(C) STRANDEDNESS: double
(D) TOPOLOGY: linear

(xi) SEQUENCE DESCRIPTION: SEQ ID :197:

ACTGGCAACT TCTATGTACC TGCAGAACCC AAATTGACAT TTGTCATCAG 50

AATCAGAGGT ATCAATGAAA TGAGCCCAAA GGTTCGAAAG CTCGCAACTT 100

CTTCACCTTC ATCAAATCTT CAACGACCTT CGCAACTCAA CAAGCTTCTT 150

ACAGTGAGGA TTGCAGAGCC TATATAGCTG ATACCCCAAT CTGAATCATA 200

AATGACTAAT CTACAAGCNT GTTATGCAAA ATAAATAAGA AACGACTTGC 250

TTACAGATGC NTTTTAATTG TGG 273

(2) INFORMATION FOR SEQ ID :198:

(i) SEQUENCE CHARACTERISTICS:
(A) LENGTH: 56 base pairs
(B) TYPE: nucleic acid
(C) STRANDEDNESS: double
(D) TOPOLOGY: linear

(xi) SEQUENCE DESCRIPTION: SEQ ID :198:

GATTCCCCAA TAAGCAGACA CCTTGAACCA GCCTGGGGTG AGCGAAAGAT 50

GNTATA 56

(2) INFORMATION FOR SEQ ID :199:

(i) SEQUENCE CHARACTERISTICS:
(A) LENGTH: 132 base pairs
(B) TYPE: nucleic acid
(C) STRANDEDNESS: double
(D) TOPOLOGY: linear

(xi) SEQUENCE DESCRIPTION: SEQ ID :199:

GGACGCTGNN NNNNNNATCC TGCAATGCAC AGCACAGACC CCACCACAGG 50

GGTTTTATCC AGCCCAAATG TCAACAGTGT CAAGTTTAAG CAACTCTTAC 100

CGAGTGGGAC TCAATTCCCN AGTTGTATGG AA 132

(2) INFORMATION FOR SEQ ID :200:

(i) SEQUENCE CHARACTERISTICS:
(A) LENGTH: 284 base pairs
(B) TYPE: nucleic acid
(C) STRANDEDNESS: double
(D) TOPOLOGY: linear

(xi) SEQUENCE DESCRIPTION: SEQ ID :200:

AAACTTTTTN GCACTTACAC AGACGAGACT TCACTGCNTG AGGATCATAC 50

GACATTTCAA TCGNACACAA ANTTAAAAAA TAAAACAAAT TTTAAAAAAC 100

CATNTTGAAT TTCCTTAAAA TTATTCCAAT ACTTTCCAAC TTAAAATTCA 150

GAACAAATCC TCCTAGAGAC TATCAATACC AATATCTTCA CATTGCTCAG 200

CTGNTACATA CGNCCCACCA GTTCACAACT AATGACACAA CACTACATGN 250

TCAAATCTTA TCTNNNATAG CACAGTAACA AAGT 284

(2) INFORMATION FOR SEQ ID :201:

(i) SEQUENCE CHARACTERISTICS:

(A) LENGTH: 106 base pairs

(B) TYPE: nucleic acid

(C) STRANDEDNESS: double

(D) TOPOLOGY: linear

(xi) SEQUENCE DESCRIPTION: SEQ ID :201:

CCTGACACCA ATTTCGCCCA CATGTATGCG GGAAGAGGCC TGAGACTAGA 50

AGTCGTTGCC CTGTCCATCT CCCGGCCACA GGCTTCATTC CCAGATTTNT 100

CTTGNT 106

(2) INFORMATION FOR SEQ ID :202:

(i) SEQUENCE CHARACTERISTICS:

(A) LENGTH: 270 base pairs

(B) TYPE: nucleic acid
(C) STRANDEDNESS: double
(D) TOPOLOGY: linear

(xi) SEQUENCE DESCRIPTION: SEQ ID :202:

CTTTGGAAAA CAAACATTTN TTATTACTGA AATAGCAAAA ATCATTCTAC 50

ACTCCTCCTA AGCATGTTCA ATTAGCATAC ATTCCAACAA TGCATGAAAA 100

AATTNCTAGC CAGAGGCATT TAAGTGATTT CTTCCTAAGT GTTTGCTAAT 150

TCAATGCCAA GAACTATGAT GTTTATCNTT CTGATGGACA AATCAAGAAA 200

CAAAACAGAT ATAATACCAA GGGTAAAGCT GATATGACCC ACAACATTGT 250

CATTACTCTA ACTGTTAATC 270

(2) INFORMATION FOR SEQ ID :203:

(i) SEQUENCE CHARACTERISTICS:
(A) LENGTH: 173 base pairs
(B) TYPE: nucleic acid
(C) STRANDEDNESS: double
(D) TOPOLOGY: linear

(xi) SEQUENCE DESCRIPTION: SEQ ID :203:

AACTAAGGCA CATTGCCCTT TTTGACCTTT CTNNNGNACT ATTGAAATCA 50

AGCTTATTGA TTAGGTGATA TTTTTATAAC AATTGAAAGG GCAATATCAA 100

ATAATGACAT ATGAGAATTT TTTATTACAT ATTAAAACTG ATTTTTACTT 150

TACAAAANNG NAATTTGCAA TTA 173

(2) INFORMATION FOR SEQ ID :204:

(i) SEQUENCE CHARACTERISTICS:
(A) LENGTH: 164 base pairs
(B) TYPE: nucleic acid
(C) STRANDEDNESS: double
(D) TOPOLOGY: linear

(xi) SEQUENCE DESCRIPTION: SEQ ID :204:

CTGCTTCACC ATCCTGGCGT CTACCAGCCT GGTGNGGCTG GGTACTGTGN 50

ATCGTCTTCC TCAACAAATG CGAGACCTGC CAACCTGCAC TACACCACAT 100

CCGCNCAAGA AGAAGAACCT GCTGCTTCAT ACAACACGGG GAGGCCCTGT 150

CATTAACATT ANTT 164

(2) INFORMATION FOR SEQ ID :205:

(i) SEQUENCE CHARACTERISTICS:
(A) LENGTH: 99 base pairs
(B) TYPE: nucleic acid
(C) STRANDEDNESS: double
(D) TOPOLOGY: linear

(xi) SEQUENCE DESCRIPTION: SEQ ID :205:

GACCGCCCAN NNNCATCCAA AACTTCTAGG CACAATCTAT ACTGCTGCTG 50

AAGAAATTGA AGCAGTTGGG GGAAAGGCCT TGCCATTGAN TTTTGATNT 99

(2) INFORMATION FOR SEQ ID :206:

(i) SEQUENCE CHARACTERISTICS:
(A) LENGTH: 118 base pairs
(B) TYPE: nucleic acid
(C) STRANDEDNESS: double
(D) TOPOLOGY: linear

(xi) SEQUENCE DESCRIPTION: SEQ ID :206:

GTACCTTGGG NNNNNNANNG GGAATGAGGT TCTACCACTC TGGAAAATTC 50

ATGCCTGTCA NTNTAANTNC AGGTGCCAGT TNNCNNTAGG TCGCCAAAGT 100

TGGGGTTAGN TGTTCNAA 118

(2) INFORMATION FOR SEQ ID :207:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :207:

CACACTANCG ACCAACACTT AAAAGNCTNC TCACACAAGN ATCTTTTTAA 50

TAAAAATACC TCTTTCNTAA CTCCACTTTA ACTCCCTAAA ACCCATGTCG 100

AAGCCCCCAT CCTGGTCAAT AGTACTTGCC CAGTACTTTT AAAACTAGGC 150

GCTATGCATA ATACCCTCAC 170

(2) INFORMATION FOR SEQ ID :208:

(i) SEQUENCE CHARACTERISTICS:
(A) LENGTH: 129 base pairs
(B) TYPE: nucleic acid
(C) STRANDEDNESS: double
(D) TOPOLOGY: linear

(xi) SEQUENCE DESCRIPTION: SEQ ID :208:

TTCAATGAAA TGCTTGTATA CAATGATATA ACAAAGAAAC CCTAAGACAA 50

CGAGAACTTC AACTAAGTGC ACTCATGCAG AATCTCTGCG GGGAGAATTT 100

TTTCTCGGGG AAGTAACCCT GCCTTTGAA 129

(2) INFORMATION FOR SEQ ID :209:

(i) SEQUENCE CHARACTERISTICS:
(A) LENGTH: 190 base pairs
(B) TYPE: nucleic acid
(C) STRANDEDNESS: double
(D) TOPOLOGY: linear

(xi) SEQUENCE DESCRIPTION: SEQ ID :209:

CTTGGCGTCA TTTTCTGTAC TTCTGACAGT GCCCTCAGAG TCTGCAGGTG 50

GATCCTTTTT TGCATGGCAT TAATTATATG AGCAGCCTCC TTCTGACAAT 100

CCAAATTTTG GCTCCAGAGT CATTTCTGAA NNTCTACACT TANGGNCTTN 150

AGCNTGCTCA TTCAAGGTNA AGGGGAGTTT TNAAAAATAT 190

(2) INFORMATION FOR SEQ ID :210:

(i) SEQUENCE CHARACTERISTICS:
(A) LENGTH: 129 base pairs
(B) TYPE: nucleic acid
(C) STRANDEDNESS: double
(D) TOPOLOGY: linear

(xi) SEQUENCE DESCRIPTION: SEQ ID :210:

TCATTGAGTC ATCCTTTTTG CCTGCTGCTG TAAGGTTTTT TTTCTTCTAG 50

TAACTGTATG ATCCAGAGCG ACCCAGCAAG GACTCAATCG ATCACCAACT 100

GATGCAGAAC TGTTTCATAT CTAGAAATG 129

(2) INFORMATION FOR SEQ ID :211:

(i) SEQUENCE CHARACTERISTICS:
(A) LENGTH: 152 base pairs
(B) TYPE: nucleic acid

(C) STRANDEDNESS: double
(D) TOPOLOGY: linear

(xi) SEQUENCE DESCRIPTION: SEQ ID :211:

CTGTAAGGTT TTCTTTCTTC TAAGTAACTG TATGATCCAG AGCGACCCAG 50

CAAGGAATCA TATCGATCAC CAGCTGATGT AGAACTGGTT TCANATCTAG 100

AAATGGAANC NNNGNGTTTN TTCCTTAATG GACCCCCCCN GGGGCNGAAT 150

GG 152

(2) INFORMATION FOR SEQ ID :212:

(i) SEQUENCE CHARACTERISTICS:
(A) LENGTH: 186 base pairs
(B) TYPE: nucleic acid
(C) STRANDEDNESS: double
(D) TOPOLOGY: linear

(xi) SEQUENCE DESCRIPTION: SEQ ID :212:

CTTAACCCTT TGGAAGGNTT GACTGTGTGA CCCGCAGCAA ATAATTCATG 50

TCGAAAGATG AAAACAACTA AGTTCATAAC CCCCTGCCCG CCATTGACCT 100

CCCTTTNAAA ANCGAGACCA AGACTCCATC ACTGGTTTCG AATTTACATC 150

NAACTGCTAA GATTGATACA TTNCAAGTCT GCAAAT 186

(2) INFORMATION FOR SEQ ID :213:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :213:

CTTAAATGCC TGTTGTGATA TCTTCTTTAA ACCTGGAGAG ATTGAATCAA 50

CCTTTCTCTA AAATTCCTTT CCTTTGCCTC CTCCTCTAAC TTTTCCTCCT 100

TTCNCGCTTT TCCTCAGGCT TTGNTTTTCC TCATGCTTTG CTTCACTCTA 150

TT 152

(2) INFORMATION FOR SEQ ID :214:

(i) SEQUENCE CHARACTERISTICS:
(A) LENGTH: 290 base pairs
(B) TYPE: nucleic acid
(C) STRANDEDNESS: double
(D) TOPOLOGY: linear

(xi) SEQUENCE DESCRIPTION: SEQ ID :214:

TCTTCAGGAG AAGGGGCACC ACTGCTTTAA AAAACAATAC TCCNTTATAG 50

ACTTGAACAT TTGCAGACAT TATGATCTTG CTTCCAACTC CCACCGTATG 100

TCCAGCAAAC TCTCGCATGT GGCCACTAGG AGGAATGCGC AAGAATGTTC 150

ATATTACATA TTTATAACAT TAATAACTGG AAAAAGTGAA ATGCATGTCT 200

GTTACAGGAA AATAGGCGAA TAATCAGATA TATATATCTA NNNCCGGGAT 250

ATTATTCAAT AGTGGAAATG ATGACTACAG CTATACCTCA 290

(2) INFORMATION FOR SEQ ID :215:

(i) SEQUENCE CHARACTERISTICS:
(A) LENGTH: 273 base pairs
(B) TYPE: nucleic acid
(C) STRANDEDNESS: double
(D) TOPOLOGY: linear

(xi) SEQUENCE DESCRIPTION: SEQ ID :215:

GTTTCTTCCA GTACATCCAA GTTTAAAATT ATTAGCGAAA TGGTCCATGT 50

TTTTTCAATT ACTGCTGACA CGATTCTAAG CTAAGTGAAG GGGAAGATCT 100

GAGAGCATGC TGTTTGGACT GTTGATGCAT ATTCATGATG TAACAGGTCC 150

TGGGCCTCAC TTTACCCCAT TCGTAAAATG GGGATAATGT CACCTGCCTC 200

TTACCTACCT CAGAGGGATT TGCGAAGCAA ACTGTTAATC TTCGAAAACG 250

ACCATTTACT TTTAGGATAT CAA 273

(2) INFORMATION FOR SEQ ID :216:

(i) SEQUENCE CHARACTERISTICS:
(A) LENGTH: 118 base pairs
(B) TYPE: nucleic acid
(C) STRANDEDNESS: double
(D) TOPOLOGY: linear

(xi) SEQUENCE DESCRIPTION: SEQ ID :216:

ATCTACGGCT AGGGAGAAAC AATGTTCCTA CATATTATGG GTAGTGAGAA 50

CATTATCTGT ATAACAGGGA ACTGTGATTA TTTAAAATTA TGCAGAACTT 100

ATTTCATCTG TGCTTTAG 118

(2) INFORMATION FOR SEQ ID :217:

(i) SEQUENCE CHARACTERISTICS:
(A) LENGTH: 197 base pairs
(B) TYPE: nucleic acid
(C) STRANDEDNESS: double
(D) TOPOLOGY: linear

(xi) SEQUENCE DESCRIPTION: SEQ ID :217:

GAGGCTGGTG GCGAGGGAGT TGTGGAGGAT AACAAGAAGA AAACAAGTCT 50

ATCACTAATG ACTTATTTTA CTTAGTTTCC ATTCACGAAA CCCTTTTAAA 100

TACAAGGCAA CATTTTCACA GCTGAAAAAT TACAACTAAA NGNNNTGATT 150

TACCACCAAA AGCAATAGAT GTAGTTATGT ATAATCTATA GATAATA 197

(2) INFORMATION FOR SEQ ID :218:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :218:

CTCGCGAGCA CTCGTCCGAG AGGTCCCATA CNNNNNNNCC CAAGCCCCTC 50

AAGGGCCTTT GCCAATCTNG TCATTTTATG CCAAGTCCTC TAAAACGCAC 100

TCAGGGGTAT CTACATCGCA CTTGTACAGA ATATCAAGAT CTTATCCTCC 150

TATTTTAGGC TNCNAGGTCA AAATAAA 177

(2) INFORMATION FOR SEQ ID :219:

(i) SEQUENCE CHARACTERISTICS:
(A) LENGTH: 118 base pairs
(B) TYPE: nucleic acid
(C) STRANDEDNESS: double
(D) TOPOLOGY: linear

(xi) SEQUENCE DESCRIPTION: SEQ ID :219:

GAAATGAAAA AGAGAGCATT ATTGGAAGAA TGAAAAATAC ATCTCAGAAA 50

GAAACCTANT AGTTCAACAA ATTAAAAGAA AGAAAGAAAA AAAGCAAAAG 100

TNGGTNTCAG GGCTGGAC 118

(2) INFORMATION FOR SEQ ID :220:

(i) SEQUENCE CHARACTERISTICS:
(A) LENGTH: 233 base pairs
(B) TYPE: nucleic acid
(C) STRANDEDNESS: double
(D) TOPOLOGY: linear

(xi) SEQUENCE DESCRIPTION: SEQ ID :220:

GAGAGCCCAG CACGTCATCC CCTGATCTGA GTCTACTGAA CACCTGTTTT 50

ATGGACTACA CTGTCTTTTC CTTTGAATCC CCACTTCTCC TGGAACTGTA 100

CTTGGACCAC CAGGAACATC GTAAGACACA ACCCAATACA CTCACCGCAT 150

TCAGACAACT GTCCAGACAC TGCCCTGACA CCACAGGGNC CCCTTTACAN 200

NGGTTGGNGG AAATATNNTT TAATCTCAGG CCA 233

(2) INFORMATION FOR SEQ ID :221:

(i) SEQUENCE CHARACTERISTICS:
(A) LENGTH: 235 base pairs
(B) TYPE: nucleic acid
(C) STRANDEDNESS: double
(D) TOPOLOGY: linear

(xi) SEQUENCE DESCRIPTION: SEQ ID :221:

GCACACAGAT ACCATCCCAC CTTGCTTTNT GACAGGCCAG CCACACAATA 50

ACCCTTTCCC TACTCACTAA AGCATCCCTA GGACACCAAC AATGAGGACA 100

GGCAGACTTA CCCCCGCCAT CTAGAGAGAA TGTCGTTATT ACCCATAAAA 150

CTCGACCACC CCCATATNCA CTNTTGGGTA AAAACAAACG CTTAAACCTG 200

TGAGCCTGCC ATTCCTTTTT ACGTGTTAAT CAATT 235

(2) INFORMATION FOR SEQ ID :222:

(i) SEQUENCE CHARACTERISTICS:
(A) LENGTH: 101 base pairs
(B) TYPE: nucleic acid
(C) STRANDEDNESS: double
(D) TOPOLOGY: linear

(xi) SEQUENCE DESCRIPTION: SEQ ID :222:

GCCAATNNNN NGGCGCGAGG GNNAGAGAGA ATGGCAAACA GGGACCCGGG 50

CCTTAGGAAT TGANTGAGGA CTTAAATTTC CCCNGAGGGA GAGNAGTGGA 100

G 101

(2) INFORMATION FOR SEQ ID :223:

(i) SEQUENCE CHARACTERISTICS:
(A) LENGTH: 271 base pairs

(B) TYPE: nucleic acid
(C) STRANDEDNESS: double
(D) TOPOLOGY: linear

(xi) SEQUENCE DESCRIPTION: SEQ ID :223:

ACGGTGACTT TCCATCCCTT GAACCAAGGC ATGTTAGCCT TGGCTCCAGC 50

ATGTCGTCAC CATTCCAACC AGAAATTGNC ACAAATGCTA CTGGTCGGGT 100

TGACCAATTT TCTTAATGAA GTGCTGACTT CCTTAACAAT TTNTTATATT 150

TNTTCGACTG TAGGGCGCTC ATGAATCCAT TTCGTTAACA CCGACAATTA 200

ATTGTTTCAC ACCCAGTGTG CAAGCCAGAA GGGCATGCTC TGGGTCTNCC 250

CATTCTTGAG ATACCAGCTT C 271

(2) INFORMATION FOR SEQ ID :224:

(i) SEQUENCE CHARACTERISTICS:
(A) LENGTH: 101 base pairs
(B) TYPE: nucleic acid
(C) STRANDEDNESS: double
(D) TOPOLOGY: linear

(xi) SEQUENCE DESCRIPTION: SEQ ID :224:

AGTATTATTT ACTNGGTCAT CTGGGAACCT TAATGTGATT TATTTTGACA 50

ATTACTGTGG CACATGTTTA ATCTGCAGCT CCTGGCGACT ACTGTGCTTA 100

T 101

(2) INFORMATION FOR SEQ ID :225:

(i) SEQUENCE CHARACTERISTICS:
(A) LENGTH: 141 base pairs
(B) TYPE: nucleic acid
(C) STRANDEDNESS: double
(D) TOPOLOGY: linear

(xi) SEQUENCE DESCRIPTION: SEQ ID :225:

TTCATTCCCT CCAGTGCNCG NNCATGCGAC ATATACAGGN NNTGTACCGT 50

AGGCGCTANT GTGTGGTACT CTGCCACGNN ANACCNCNNC TCGTCTTGAA 100

GACCCTGTTA ANTTTGGTGA AAATAACTTT CCANATTTCA A 141

(2) INFORMATION FOR SEQ ID :226:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :226:

ATTTTCTTAT ACTCCTCCCA CAGATGAGTT CACAAATACA AAAANTGGTG 50

TACATTTATA CTCAAGNACA AATCTCCAAC AGCCAAGTAA TTATAGTTTG 100

TTCTGTTATG TGCAAAGTAG ATTATTTCAT ATTTACTTGG TATGGAAAGC 150

AGAGTACAGG CTCAATGGAC AATAATCATT AAACACACAT TATNTTTAAG 200

AAAANGCTGT TNNAAAAA 218

(2) INFORMATION FOR SEQ ID :227:

(i) SEQUENCE CHARACTERISTICS:
(A) LENGTH: 209 base pairs
(B) TYPE: nucleic acid
(C) STRANDEDNESS: double
(D) TOPOLOGY: linear

(xi) SEQUENCE DESCRIPTION: SEQ ID :227:

GAAAACTTTA TTTGTCCACA CCAGGATTAC CGAACAGAAN NACNNGGTGG 50

TGAGAAGTTG GTATTNATAG CACCTTATTT ACATGATGGA CTTGAGGAGG 100

CAGTTAATCC TATGGTTGTG TATCACAACC TTTTATTAGC AATGCCATCT 150

TCGTCTTGCC TCCNCCCTAC TTGAATATCC CTTACGGTCA ACANCCCNCG 200

GGGTTGGGC 209

(2) INFORMATION FOR SEQ ID :228:

(i) SEQUENCE CHARACTERISTICS:
(A) LENGTH: 179 base pairs
(B) TYPE: nucleic acid

(C) STRANDEDNESS: double
(D) TOPOLOGY: linear

(xi) SEQUENCE DESCRIPTION: SEQ ID :228:

TTTNNCAANC CAAATGGTTA TTTATTCTAA AACTGGAAGC TACTTTGCCT 50

ANCATTTTCG CCAGAATGGT GTAATGNNNA CAGGGGAGGA AAAAAGTTAC 100

AGATGTAAAC AATGACACAG TTACATTTTT TTTTTAAATG GTAAAACCCC 150

TTTTTACTGG NCNTTCCAGA ANCTTACAG 179

(2) INFORMATION FOR SEQ ID :229:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :229:

AGTTTAATCT CANNNNNNNA TGTCACAAGT TATTGTAGCA GTGAAACAAT 50

GAGGGCATAC ACTATATNGA AAAAAAAACC TCCTCCCTNA TTCTCACGCC 100

AACCACAGGC TTCTGCCCTG CAAAGATCAC CAATGTCAAT AGTTTGGTAA 150

TACACCATCA TAAAGGNTCC TAAATTCATC TCTA 184

(2) INFORMATION FOR SEQ ID :230:

(i) SEQUENCE CHARACTERISTICS:
(A) LENGTH: 140 base pairs
(B) TYPE: nucleic acid
(C) STRANDEDNESS: double
(D) TOPOLOGY: linear

(xi) SEQUENCE DESCRIPTION: SEQ ID :230:

GAGACAGATG TANNNAAAGT TCAGAATACA ACAGTTAAGA CTCAGTTTTC 50

TTTTTAGGTT TAGAATTTGA GAGCAAGTAT TGNTATGGTG AGCTGTTTTA 100

GTGCAAACAT TGTTGAGTAT GTTGTCAAAC GTCTAAAAAA 140

(2) INFORMATION FOR SEQ ID :231:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :231:

CGGAGACTTG TCCAGAGAGT TGTCTCTTNT NNGTTGGGGG CCGTCCCGCT 50

CCTAAGGCAG GAAGATGGTG GCNNNTNNNG ACGAAAAAGT CGCTGGNGNN 100

NATNAANTNT AGGCTCNAAN TNNTTATGAA AANTGGGAAG TAANTNNTCG 150

GGGGTAAAAG NAANATNNGA ANATGGAT 178

(2) INFORMATION FOR SEQ ID :232:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :232:

GAGCCAACGC CACNNNNNAG NTGAACCACA CTCACGAAAA AAACNCNTAC 50

CGTCGTCNTA ATACNNANTC TTCCCATACA AAAATCGTCC NTNTAAATNT 100

NNTAAACCAA TTCACAGCCC ACAGAACNAA TCAGTAATTT TATANCTTCN 150

NCGAAACCAC ACTTATCCCC ACCTTGGTCT ATTCATNACC CGGATNGAGG 200

GCAACCANGG 210

(2) INFORMATION FOR SEQ ID :233:

(i) SEQUENCE CHARACTERISTICS:
(A) LENGTH: 247 base pairs
(B) TYPE: nucleic acid
(C) STRANDEDNESS: double
(D) TOPOLOGY: linear

(xi) SEQUENCE DESCRIPTION: SEQ ID :233:

ACTGTGCGAG TAGCTTNAAA ANNNNNNNNN NNACTCAGTT TNATTTATAC 50

AAAAAGAAAG GGGTGTGNAG TCCATGGTGT TGTACAGTNT NTAATTAGNC 100

CANNACGAGA AAANANATNN NNNTNNNAAA NNNTGAATTA TGGGGTNAGG 150

ACTNTCTNAC NTTCAAACNA NTATTNCACG TAAAAAACAT CACAGTGCGA 200

AGAAAGNNAN CNCANNTAGA GCANGAAGAC ATCAAAAGCC AGCCGGG 247

(2) INFORMATION FOR SEQ ID :234:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :234:

AAACACCAAA NAAANNNNNC NAGCAANAAA GTGGAAGACT AACCAAGATT 50

GTGGACATTG GAATGTTTAC TGTTATTCTG TGTAAGAAAA CAACTNACAA 100

AAAAGAAAAA TGGNCAACAA AATTGTTTCC CGGCNAGGCT GNAGGAAACC 150

NGGGGGAAAN TGCCNGGGC 169

(2) INFORMATION FOR SEQ ID :235:

(i) SEQUENCE CHARACTERISTICS:

(A) LENGTH: 51 base pairs
(B) TYPE: nucleic acid
(C) STRANDEDNESS: double
(D) TOPOLOGY: linear

(xi) SEQUENCE DESCRIPTION: SEQ ID :235:

GCANCACANA AAGGAGACGN NANAGCAACG CAGAGATAGC CATCCAGATA 50

G 51

(2) INFORMATION FOR SEQ ID :236:

(i) SEQUENCE CHARACTERISTICS:
(A) LENGTH: 101 base pairs
(B) TYPE: nucleic acid
(C) STRANDEDNESS: double
(D) TOPOLOGY: linear

(xi) SEQUENCE DESCRIPTION: SEQ ID :236:

CAAGTGTAAA TGCTTGCACC TCTCTNCCTC CCCGANTGAA CTCTCTGATC 50

TCAAACTTTT TTAGGAAAGC CAGATTAAAA GCAGACGTAC CTAAATNCAA 100

A 101

(2) INFORMATION FOR SEQ ID :237:

(i) SEQUENCE CHARACTERISTICS:
(A) LENGTH: 156 base pairs

(B) TYPE: nucleic acid
(C) STRANDEDNESS: double
(D) TOPOLOGY: linear

(xi) SEQUENCE DESCRIPTION: SEQ ID :237:

CATTATAAAA CAGCCTAACT TCCCTTATGC CATATGATTG CCTTAAAAAG 50

ACCAGATCTC AAGGAAAAGA TCATCAAAGA GCAGAGATCT TGAAGCGGCA 100

CAGTTTTCCA GCAGTTTTCG TATTTNTTTT TATTTACGAA TGCCATACTC 150

TGTTTT 156

(2) INFORMATION FOR SEQ ID :238:

(i) SEQUENCE CHARACTERISTICS:
(A) LENGTH: 148 base pairs
(B) TYPE: nucleic acid
(C) STRANDEDNESS: double
(D) TOPOLOGY: linear

(xi) SEQUENCE DESCRIPTION: SEQ ID :238:

GCAGNCTAAT TGTGAATCTA AGAAACTACT CATAGACATC CCACCCTAAT 50

GATTTTACCT NNAACNTTTG TCCTTCATCA TAGAACCCTA GCAACATCCA 100

CCTCCTGTAG CACGAAACGA ATCAAACAAC CCCCTGGATA ACCTCTCA 148

(2) INFORMATION FOR SEQ ID :239:

(i) SEQUENCE CHARACTERISTICS:
(A) LENGTH: 258 base pairs
(B) TYPE: nucleic acid
(C) STRANDEDNESS: double
(D) TOPOLOGY: linear

(xi) SEQUENCE DESCRIPTION: SEQ ID :239:

GAGTTTTAAC TTAATCACCT CTTTAAAAGA CCTGTCTCCA AATACAGTTA 50

AATTTGAGGT ATTGAGGGTT AGGACTTCAA CATGTGAGTT TGGGAAGGGA 100

AGCACAAAAT CAGCCCCTAC CATGGTATAT TTATCATTGA TACATTACTA 150

TCAACTAAGC TCAAGATTTT ATTCAGATTT GACTAGTTTT TCCACTAAGG 200

CCCTTTTTCT TTTCTAGGNT CCCACAGAGG ATACATTACA TTTACTTACA 250

TCTTCTCT 258

(2) INFORMATION FOR SEQ ID :240:

(i) SEQUENCE CHARACTERISTICS:
(A) LENGTH: 377 base pairs
(B) TYPE: nucleic acid
(C) STRANDEDNESS: double
(D) TOPOLOGY: linear

(xi) SEQUENCE DESCRIPTION: SEQ ID :240:

GAGCATTATT TGATGCAGAA GTTGAAAAAC AATAGACTCA AGAAAGAAAA 50

CAAACCAGTG ATTCCCCTTC CTCAGATACT GGGACTAACA GCTTCACCTG 100

GTGTTGGAGG GGCCACGGAG CAAGCCAAAG CTGAAGAACA CATTTTAAAA 150

CTATGTGCCA ATCTTGATGC ATTTACTATT AAAACTGTTA AAGAAAACCT 200

TGATCAACTG AAAAACCAAA TACAGGAGCC ATGCAAGAAG TTTGCCATTG 250

CAGNTGCAAC CAGNGGAGNT CCTTTNAAGN GNAACTTCTN GNATAATNNC 300

AAGGGTNAAC NTNTTTNNAA ANNNGCCNAA NCNNGATTTT GNACNCCCTT 350

TNNCATTGGC ATTNANTGAA AAAAGTT 377

(2) INFORMATION FOR SEQ ID :241:

(i) SEQUENCE CHARACTERISTICS:

(A) LENGTH: 114 base pairs

(B) TYPE: nucleic acid

(C) STRANDEDNESS: double

(D) TOPOLOGY: linear

(xi) SEQUENCE DESCRIPTION: SEQ ID :241:

GGNGCACTGN TCCGAGAGCT TTTTTTNCTG AAGAATAGCA TCTTTAATGA 50

GTGTNCTAAT CCTTGTCATC TGAAGTTTTG AAATATATTT CCCAGGGTCA 100

GAACAATACA GAGA 114

(2) INFORMATION FOR SEQ ID :242:

(i) SEQUENCE CHARACTERISTICS:
(A) LENGTH: 122 base pairs
(B) TYPE: nucleic acid
(C) STRANDEDNESS: double
(D) TOPOLOGY: linear

(xi) SEQUENCE DESCRIPTION: SEQ ID :242:

CTCAAAATNC TGTGACAAAT TTNNNNGGTC AAGTTGTTNN CCATTAAAAA 50

GTACCTGATT TTCAAAAACC TAATAACCTT AAAACCNCCC CACGNAAAAA 100

AAAAAANCNA AAGNGGGCCC CC 122

(2) INFORMATION FOR SEQ ID :243:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :243:

GAGAATGGGA AGCCTCATTT TGGGGACAAG AACCTGTACA AGGATTTGTG 50

ATGAACTTTT CCAATGGGGA AATTATAGAC ATCTTCAAGC CAGTGCGCAC 100

NTATGATATG CCTCATGATA TTNTTGCATC TGAAGATGGG ACTNTGTACA 150

TTGGNGATGC TCATCCAAAC C 171

(2) INFORMATION FOR SEQ ID :244:

(i) SEQUENCE CHARACTERISTICS:
(A) LENGTH: 235 base pairs
(B) TYPE: nucleic acid
(C) STRANDEDNESS: double
(D) TOPOLOGY: linear

(xi) SEQUENCE DESCRIPTION: SEQ ID :244:

AACTTTACTG TCAGATAATA AAATTAGGGC TTTCTCTTAA AGGGCTTCTT 50

TAAGAGAAAT ACAGAGTGTT TGGTATNTGA GAGAAAAAAA GTTAAAACAG 100

GACTTTCAAC TTAATCCAGA CTTCCTAACA GTGTTTACAT GTGAGGGAAA 150

CTCCTTTAAG TAATGCGTAG TGTTTTATTT TTACCATCAT TGGNGACAAA 200

AAAAACAAAA ACATAAACAT CTNANGTGAA ATATA 235

(2) INFORMATION FOR SEQ ID :245:

(i) SEQUENCE CHARACTERISTICS:
(A) LENGTH: 211 base pairs
(B) TYPE: nucleic acid
(C) STRANDEDNESS: double
(D) TOPOLOGY: linear

(xi) SEQUENCE DESCRIPTION: SEQ ID :245:

GGCCTAGCCT GCCATACCCT TACGAGCAGG CTCAGTGATT AGACTTTGAG 50

TCTAAGTTAA AAACGCCCTG CCCCCTTCTC GCAGGCCACC TACACCGTNN 100

TTTTATCGAT TTGATAAAAC CACCAGCCTA CTCATCAAGN NGCACCCTGC 150

NTNTACNTCT AACCNTAACA TNACNGCGGC CACCTACTCA TGCCCTANTG 200

CAGCNCACCC T 211

(2) INFORMATION FOR SEQ ID :246:

(i) SEQUENCE CHARACTERISTICS:

(A) LENGTH: 194 base pairs

(B) TYPE: nucleic acid

(C) STRANDEDNESS: double

(D) TOPOLOGY: linear

(xi) SEQUENCE DESCRIPTION: SEQ ID :246:

GGCTGAGAAT CCTTGAGCTT ACCATTTGAT ATTTCTATAT TATTTAAGAA 50

AAGTCAAAAG ATTTTGAAAA CAGCAATAGA AGTAAGATCC TTTAAGCTCT 100

ATTTGCAGCC CCTAAGAAAA GTGATGAGGA CACTGTGCAT GCCCATATGT 150

GAACATGGTG GTACCTTAGG NATTTCCCTT TNTCNATGAA TATA 194

(2) INFORMATION FOR SEQ ID :247:

(i) SEQUENCE CHARACTERISTICS:

(A) LENGTH: 249 base pairs
(B) TYPE: nucleic acid
(C) STRANDEDNESS: double
(D) TOPOLOGY: linear

(xi) SEQUENCE DESCRIPTION: SEQ ID :247:

GCCCCCTCAA GGGCATCCTG GGCTACACTG AGCACCAGGT GGTCTCCTCT 50

GACTTCAACA GCGACACCCA CTCCTCCACC TGACGCTGGG GCTGGCATTG 100

CCCTCAACGA CCACTTTGTC AAGCTCATTT CCTGGTATGA CAACGAATTT 150

GCTACAGCAA CAGGGTGTGG ACCTCATGGC CCACATGGCC TCCAAGGTAA 200

GCCCCTGGAC CACCAGCCCC AGCAAGGCAC AAGAGGAAGG AGAGACCCT 249

(2) INFORMATION FOR SEQ ID :248:

(i) SEQUENCE CHARACTERISTICS:
(A) LENGTH: 248 base pairs
(B) TYPE: nucleic acid
(C) STRANDEDNESS: double
(D) TOPOLOGY: linear

(xi) SEQUENCE DESCRIPTION: SEQ ID :248:

GCCCCCTCAA GGGCATCCTG GGCTACACTG AGCACCAGGT GGTCTCCTCT 50

GACTTCAACA GCGACACCCA CTCCTCCACC TGACGCTGGG GCTGGCATTG 100

CCCTCAACGA CCACTTTGTC AAGCTCATTT CCTGGTATGA CAACGAATTT 150

GCTACAGCAA CAGGGTGTGG ACCTCATGGC CCACATGGCC TCCAAGGTAA 200

GCCCCTGGAC CACCAGCCCC AGCAAGGCAC AAGAGGAAGG AGAGACCC 248

(2) INFORMATION FOR SEQ ID :249:

(i) SEQUENCE CHARACTERISTICS:
(A) LENGTH: 82 base pairs
(B) TYPE: nucleic acid
(C) STRANDEDNESS: double
(D) TOPOLOGY: linear

(xi) SEQUENCE DESCRIPTION: SEQ ID :249:

GGAGGGGACC GCAGCATCCA GCCCTCTAAG GCCGGGCAGC GGTCGCGTTG 50

GGGCAGAGCG CAGCGCAAGC AGGCTCAGTG TA 82

(2) INFORMATION FOR SEQ ID :250:

(i) SEQUENCE CHARACTERISTICS:
(A) LENGTH: 125 base pairs
(B) TYPE: nucleic acid
(C) STRANDEDNESS: double
(D) TOPOLOGY: linear

(xi) SEQUENCE DESCRIPTION: SEQ ID :250:

GGGGCCCTCA GGACATCCAC GTGAGCGTCT GCCCAGCTGC ACTGATATTG 50

TNTTGCAAAT CCAGATTTGT TGNCATTACT GATGGGCGCG TGAAACCAGN 100

GAGAGATGCA CAAGATTTAC AGGCC 125

(2) INFORMATION FOR SEQ ID :251:

(i) SEQUENCE CHARACTERISTICS:
(A) LENGTH: 130 base pairs
(B) TYPE: nucleic acid
(C) STRANDEDNESS: double
(D) TOPOLOGY: linear

(xi) SEQUENCE DESCRIPTION: SEQ ID :251:

GGCCCAGAGG TCCTTTACTC TTACGGNACA CCTTAGCCAC ATTCACAGGG 50

AATGNTCCAG CACTCAGGCT CCTTCCCATN GGTTTTCAAA AGCGCGTTTT 100

TCTGGGGGAG CGGCCGCCTT TAGTCGACCC 130

(2) INFORMATION FOR SEQ ID :252:

(i) SEQUENCE CHARACTERISTICS:
(A) LENGTH: 216 base pairs
(B) TYPE: nucleic acid
(C) STRANDEDNESS: double
(D) TOPOLOGY: linear

(xi) SEQUENCE DESCRIPTION: SEQ ID :252:

GAGAAGGCTG GAGAAAAACC ATCCACACAT AAACAATNGN ATTTACTCNA 50

AAATNAAGTA CAGGTTTCAG GTATTTAAAA TAAATAAAGA AAAATCTCGT 100

TTCCTTTGGC ATCTTTAGAA AATAAACTAA GCAATAAAAG AGGTGATTGT 150

ATAAAGACAT GCGTAAGCAA ACATATGGGG AAAACCAGCA ACTTGGTTTT 200

ATGNGATAAT ATCAGC 216

(2) INFORMATION FOR SEQ ID :253:

(i) SEQUENCE CHARACTERISTICS:
(A) LENGTH: 249 base pairs
(B) TYPE: nucleic acid
(C) STRANDEDNESS: double
(D) TOPOLOGY: linear

(xi) SEQUENCE DESCRIPTION: SEQ ID :253:

GGAGAAGGAG GCTGATTGCG TACATCCAGC AGTTACAATT TTTAAAAATT 50

ACANTNNNNC NNTTNGATTN TTAATNTANG TAATTTCCTT CCAAAGAAGN 100

TTCACATGTA ATAAGTAGAA ATTCTGTATA GGAAAAAAGC ATTAAAAATA 150

TANATACNGC TTCATNCGTT GGGAACCATT AAAAGTAATA TAATNAGCTT 200

TTTTCAGAAG GATCTTTTGT AGCAGTGNTT ATGAATGNAC CCGCAAAAT 249

(2) INFORMATION FOR SEQ ID :254:

(i) SEQUENCE CHARACTERISTICS:

(A) LENGTH: 166 base pairs
(B) TYPE: nucleic acid
(C) STRANDEDNESS: double
(D) TOPOLOGY: linear

(xi) SEQUENCE DESCRIPTION: SEQ ID :254:

GACCCCATTC TATATTATNC GTNNNGCGAT TTTTTAGCCA CCCTGAAGTT 50

ATATTTNTAT ACCNAGGCTT CGAATAATCT CATCNGACTN ACCACCCTNG 100

GAAAAAAAGA ACCGTTTGAT ACATAGGNAT GNTNAGCTTG ATATCAATNG 150

CTCCCTGGGN TTCTTG 166

(2) INFORMATION FOR SEQ ID :255:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :255:

CCAGACCAAC CGCCTGCAGG AGGCTCTGAA CCCTCTTCAA GAGCATCTGG 50

AACAACAGAT GGCTGCGCAC CATCTCTGTG ATCCTGTTCC TCAACAAGCA 100

AGATTTGCTC GCTGAGAAAG TCCTTGTGGG AAATCGAAGT TGAGGACTAC 150

TTTCAGAATT TGCTCGCTAC ACTACTTTGA GGATGCTACT CCCGAGCCCC 200

CTTCTNTTGT NACAGACAGC AGA 223

(2) INFORMATION FOR SEQ ID :256:

(i) SEQUENCE CHARACTERISTICS:
(A) LENGTH: 292 base pairs
(B) TYPE: nucleic acid
(C) STRANDEDNESS: double
(D) TOPOLOGY: linear

(xi) SEQUENCE DESCRIPTION: SEQ ID :256:

GGAGAAGAAG GAGCAGGAGG TGATGCTACT GACTCAAGTC AAACAGCTCT 50

TGATAATAAA GCTTCATTGC TCCATTCAAT GCCTACTCAC TCCTCTCCGC 100

TCTCGAGACT ATAATCCATA TAACTATTAA GATAGCATCA GTCCCTTCAA 150

CAAGTCTGCC CTCAAGGAAG CCATGTTTGA TGATGATGCT GACCAGTTTC 200

CTGACGATCT TTCCCTAGAT CATTCTGACC TGTTGTAGAG TTGTTGAAGG 250

AGCTGTCTGA CCATAATGAG CTGTAGAAGA AAGAAAAATT GC 292

(2) INFORMATION FOR SEQ ID :257:

(i) SEQUENCE CHARACTERISTICS:
(A) LENGTH: 238 base pairs
(B) TYPE: nucleic acid
(C) STRANDEDNESS: double
(D) TOPOLOGY: linear

(xi) SEQUENCE DESCRIPTION: SEQ ID :257:

AAGGAGCAGG AGGTGATGCT ACTGACTCAA GTCAAACAGC TCTTGATAAT 50

AAAGCTTCAT TGCTCCATTC AATGCCTACT CACTCCTCTC CGCTCTCGAG 100

ACTATAATCC ATATAACTAT TAAGATAGCA TCAGTCCCTT CAACAAGTCT 150

GCCCTCAAGG AAGCCATGTT TGATGATGAT GCTGACCAGT TTCCTGACGA 200

TCTTTCCCTA GATCATTCTG ACCTGTTGTA GAGTTGTT 238

(2) INFORMATION FOR SEQ ID :258:

(i) SEQUENCE CHARACTERISTICS:
(A) LENGTH: 137 base pairs
(B) TYPE: nucleic acid
(C) STRANDEDNESS: double
(D) TOPOLOGY: linear

(xi) SEQUENCE DESCRIPTION: SEQ ID :258:

GGAGAGAAAA GTTCCTGAGT GACAGAGAAA AAGAACAAAA AGCTGCAGAA 50

GGCNTCAGCA GAGGCCACTG CTGGCCCTGA GGCTGCACCA AGTGACGAAG 100

AACCGGCTCC AAGCATTCGT CACAGCACTA ATTTAAA 137

(2) INFORMATION FOR SEQ ID :259:

(i) SEQUENCE CHARACTERISTICS:
(A) LENGTH: 241 base pairs
(B) TYPE: nucleic acid
(C) STRANDEDNESS: double
(D) TOPOLOGY: linear

(xi) SEQUENCE DESCRIPTION: SEQ ID :259:

GCGCGACTTT TAAGGGATTT GCNGTGATGC CTGTTGACCC AGTGCCTTCC 50

TAGCCGGGAA GGGGCTCGGC TGGAGTGNNA AGGCTCAGAA AAATTTCGCG 100

AAGAAAAAAA CCTATGAGGT AATAATAGGA TTATTCCGTA TCGAAGGCCT 150

TTTTGGACAG GTGGGTGCGG TGACCTTGGT ATGTATTTTT CGTGTTACAT 200

CGCGCCATCA TTGGATATGT TAGTGTGTNG GTTAGTAGGT C 241

(2) INFORMATION FOR SEQ ID :260:

(i) SEQUENCE CHARACTERISTICS:
(A) LENGTH: 248 base pairs
(B) TYPE: nucleic acid
(C) STRANDEDNESS: double
(D) TOPOLOGY: linear

(xi) SEQUENCE DESCRIPTION: SEQ ID :260:

GCGGACTTTT AAGGGATTTG CNGTGATGCC TGTTGACCCA GTGCCTTCCT 50

AGCCGGGGAA GGGGCTCGGC TGGAGTGNNA AAGGCTCAGA AAAATTTGCG 100

AAGAAAAAAA CCTATGAGGT AATAATAGGA TTATTCCGTA TCGAAGGCCT 150

TTTTGGACAG GTGGGGCGGT GACCTTGGTA TGTATTTTTC GTGTTACATC 200

GCGCCATATT GGTATATGTT AGTGTGTTGG TTAGTAGGTC TCGTATGA 248

(2) INFORMATION FOR SEQ ID :261:

(i) SEQUENCE CHARACTERISTICS:

(A) LENGTH: 239 base pairs

(B) TYPE: nucleic acid

(C) STRANDEDNESS: double

(D) TOPOLOGY: linear

(xi) SEQUENCE DESCRIPTION: SEQ ID :261:

GGATCTTTCA GGTGATGAAA TAGTTCTGTA TCTTGATTTT GGCGAATCTA 50

CACANGTGAT GAAGTAACGT GATAAAATGA CATAGACCTG TATGCCTACT 100

ACAATACAGT ATCAAAACCA GGGATTGATT CCTGGTTCTT TTCCAAATCC 150

ACTTCCCAAA CTTATGGTAA GGTAATATTA AAAAGGCACC AAAGAGCCCT 200

GATCCCTGGA TAAACAGGAT CATTTCAAAG NNGTTTATA 239

(2) INFORMATION FOR SEQ ID :262:

(i) SEQUENCE CHARACTERISTICS:

(A) LENGTH: 143 base pairs

(B) TYPE: nucleic acid

(C) STRANDEDNESS: double

(D) TOPOLOGY: linear

(xi) SEQUENCE DESCRIPTION: SEQ ID :262:

GCCGGGTGAT GGCCAGGAAG GTGCCCTCTG TTTTTTGTAA AACAGCCATT 50

GGCCTTTGTC ATTGAGTCAC CACCTGCAGG GCTTGGGAGT GAGCGTTGGG 100

TAGGNTCAGG CCCCCAGAAC CGCCTGGGTA CTCACCGCTA GCA 143

(2) INFORMATION FOR SEQ ID :263:

(i) SEQUENCE CHARACTERISTICS:
(A) LENGTH: 246 base pairs
(B) TYPE: nucleic acid
(C) STRANDEDNESS: double
(D) TOPOLOGY: linear

(xi) SEQUENCE DESCRIPTION: SEQ ID :263:

GGCGCTCAGC CTCCCCAAGG ACAAGCTCCT CCCCCTGTAA TACCTCCTCC 50

TAACAGCCGG ATATGGATGG CAAGTTACCA AACACAGTGA GCCGGGACTC 100

TAAAAAAAAA TAGCAATCCA GATAGGCTTC GATTTCCCGT GACACTCTGA 150

AGACATGAAA GTAGACATCG AAAATGAAAA TANTTATNNA AATGAAATGT 200

TTGGAACCTT TAGCACAGAT TTGTTTGGGA AGACACGGTC TTTTAG 246

(2) INFORMATION FOR SEQ ID :264:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :264:

GGCTCCATTA AGGACCAAAT TNATGCTACC ACTAAACAAA AANNTATAGT 50

CTGTGTTAAA TCGTATGCTT TTTAAAGGTA TTTAAAGATT CAACTAGCTT 100

TAAAGAGGCT GAGCAGCTCA GGAAGCCTGT AATGTGACAT AACTCTTTGG 150

ACCTGATCTT GATGTTCTGC TGTTGTNAGT CTTGAAGAGC GTATNTGAT 199

(2) INFORMATION FOR SEQ ID :265:

(i) SEQUENCE CHARACTERISTICS:
(A) LENGTH: 245 base pairs
(B) TYPE: nucleic acid
(C) STRANDEDNESS: double
(D) TOPOLOGY: linear

(xi) SEQUENCE DESCRIPTION: SEQ ID :265:

GGTTTCAATC GTCCATCCAA GATACAAGAG AACGCATTGC CACTGATGCT 50

TGCTGAGCCC CCACAGAACT TAATCGCCCA ATCTCAGTCT GGTACTGGTA 100

AAACAGCTGC CTTCGTGCTG GCCATGCTTA GCCAAGTAGA ACCTGCAAAC 150

AATATCCCGT GTCTTCTCTN TCCCAACGTA TGAGCTGCCC TCCAAACAGG 200

AAAAGTGATT GAACAAATGG CAAATNTTAC CCTGAACTGA AGCTG 245

(2) INFORMATION FOR SEQ ID :266:

(i) SEQUENCE CHARACTERISTICS:
(A) LENGTH: 121 base pairs
(B) TYPE: nucleic acid
(C) STRANDEDNESS: double
(D) TOPOLOGY: linear

(xi) SEQUENCE DESCRIPTION: SEQ ID :266:

AGGAAAAGAT GGGAATATGT TTCTTTCCTT TGAGAATTCA CAAAANGGGG 50

TCAAAAACAA AGCAATGCTG AAAGCGAACA TCCATTTNGC CTGCAATTCA 100

AGGCGAAAAT CCAAAGGCAT C 121

(2) INFORMATION FOR SEQ ID :267:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :267:

GGGTCGCTCT CCCCCCCTCT TTCTTCGGGT TGTGTGCGTC TCCGCTTTCG 50

TGATGTGAGG AACTCTGGGG TGGGCGACGG GTCCAACTCG CGTTGTCATC 100

TCCCAGGTTG GTACACCCCC CCCCCGTTTC CCCAGCCACA CTCCACGGCC 150

AGGGTGGAGG CAGATGTCT 169

(2) INFORMATION FOR SEQ ID :268:

(i) SEQUENCE CHARACTERISTICS:
(A) LENGTH: 200 base pairs
(B) TYPE: nucleic acid
(C) STRANDEDNESS: double
(D) TOPOLOGY: linear

(xi) SEQUENCE DESCRIPTION: SEQ ID :268:

TCGCAGGAGA GGAATTACAT GCTCATCCTA GAAGGGGGGG CTGACTGCAG 50

GTGTTGCTGG GAAGCCTCTC CAGGCCTGGA GCTGGAGTAC CCGTCCTCAG 100

CACTGCCAGC AGAAAAAGTT GTGATTCAAG GAAAGCACAT TGAATGCATT 150

ATAGCAATCC CAGACCTAAG TTCGAAGTTG CTTTGTAACA AGTGCTGCCT 200

(2) INFORMATION FOR SEQ ID :269:

(i) SEQUENCE CHARACTERISTICS:
(A) LENGTH: 163 base pairs
(B) TYPE: nucleic acid
(C) STRANDEDNESS: double

(D) TOPOLOGY: linear

(xi) SEQUENCE DESCRIPTION: SEQ ID :269:

AGGGGGGCGC AAGAGAGAAG AACTTCACAC TTCTTTATTG CTCAGCCTAG 50

ATAGCAGCAG CTGGGAATAC GTAGGACAAA CAGGACGTCG AACAAATTAC 100

TCCACTATAT TAATATTCAC TACACCACTT ATTCTTTCTT GAATTGTAAA 150

CACTAAGTGT AGT 163

(2) INFORMATION FOR SEQ ID :270:

(i) SEQUENCE CHARACTERISTICS:

(A) LENGTH: 115 base pairs

(B) TYPE: nucleic acid

(C) STRANDEDNESS: double

(D) TOPOLOGY: linear

(xi) SEQUENCE DESCRIPTION: SEQ ID :270:

TCACACTTCT TATTGCTCAG CCTAGATAGC AGCAGCTGGG GAATACGTAG 50

GACAAACAGG ACGTCGAACA AATTACTCCA CTATATTAAA TTCACTCACA 100

CCACTTATTC TTTCT 115

(2) INFORMATION FOR SEQ ID :271:

(i) SEQUENCE CHARACTERISTICS:

(A) LENGTH: 178 base pairs
(B) TYPE: nucleic acid
(C) STRANDEDNESS: double
(D) TOPOLOGY: linear

(xi) SEQUENCE DESCRIPTION: SEQ ID :271:

CCCGAGACCT GCGANAATGG TGCTGTGACG ACGAATAGCC ACGCATTATA 50

GGGTNTTNTG TTATGGGGGA CACTCTACTA CGGGATGCGT ATGATGNGGN 100

NCCATTATNG NAGTGGGCAT TGGGGGGAAA CAGAGCACCT GATGCTTTAC 150

TGCAGAAATN CCTATGTGAC TCTTATAA 178

(2) INFORMATION FOR SEQ ID :272:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :272:

CCCGAGACCT GCGANAATGG TGCTGTGACG ACGAATAGCC ACGCATTATA 50

GGGTNTTNTG TTATGGGGGA CACTCTACTA CGGGATGCGT ATGATGNGGN 100

NCCATTATNG NAGTGGGCAT TGGGGGGAAA CAGAGCACCT GATGCTTTAC 150

TGCAGAAATN CCTATGTGAC TCTTATAA 178

(2) INFORMATION FOR SEQ ID :273:

(i) SEQUENCE CHARACTERISTICS:
(A) LENGTH: 135 base pairs
(B) TYPE: nucleic acid
(C) STRANDEDNESS: double
(D) TOPOLOGY: linear

(xi) SEQUENCE DESCRIPTION: SEQ ID :273:

CTCTAGTAAA AATGTTTGAG GAAACAAAAA TGGGGAAGAA GATCAGAACA 50

AAAANATTGT TAACACTGAC CGTCCTCATG CAGGTAGGCT ACTACCAACG 100

CTGGTTGTTA CTCCAGGAAA ATCGAGGTGA ACATC 135

(2) INFORMATION FOR SEQ ID :274:

(i) SEQUENCE CHARACTERISTICS:
(A) LENGTH: 231 base pairs
(B) TYPE: nucleic acid
(C) STRANDEDNESS: double
(D) TOPOLOGY: linear

(xi) SEQUENCE DESCRIPTION: SEQ ID :274:

TGTCGAGGAG AAGAAACCAC TTGATAACAC CCCGCGACAT CGTGGGGCTG 50

CTTGTCACAN GAAAGCACCA TGTTCGCAAT GGATTGCTGC AGGTGCTGCC 100

GTTGCCTCCT CAGGGTCTGC TGAAAGTCAT CTTCTAGGGT CTGAACGACA 150

TAACGCAGGA AAAGGACTCG CCCAGGCAGA GTTTGTCCCT CTTCCTTCAT 200

GACATAGGTG AGCAGTTTCC AGTCCCACTC C 231

(2) INFORMATION FOR SEQ ID :275:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :275:

TCGCAGGAGA AGAAACCTNG TTGCTCCACA ATGCAACCAC ACTGATTTTC 50

TCTTTTCTCT NNAGTTNTCC TTGTCTGTAA CAGGAATGTC CCTTACTATA 100

GCAGGCGGAC ACGGCCATGG GTCAAGCACC CTGCTTCTGG AACTTGNNNG 150

NCGTNCCCAC CATTGATTGA 170

(2) INFORMATION FOR SEQ ID :276:

(i) SEQUENCE CHARACTERISTICS:
(A) LENGTH: 315 base pairs
(B) TYPE: nucleic acid
(C) STRANDEDNESS: double
(D) TOPOLOGY: linear

(xi) SEQUENCE DESCRIPTION: SEQ ID :276:

ATATGAAGGA GGAAATGGCT CTCACCTTCG TGAATACCAA GACCTGCTCA 50

ATGTTAAGAT GGCCCTTGAC ATGAGATTGC CACCTACAGG AAGCTGCTGG 100

AAGGCGAGGA GAGCAGGATT TCTCTGCCTC TTCCAAACTT TTNTCTGAAC 150

CTGAGGGAAA CTAATCTGGA TTCACTCCCT CTGGTTGATA CCCACTCAAA 200

AAGGACACTT CTGATTAAGA CGGTTGAAAC TAGAGATGGA CAGGTTATCA 250

ACGAAACTTC TCAGCATTAC GATGACCTTG AATGAAAATN GTACACACTT 300

AGCGTAGCAT ATTNA 315

(2) INFORMATION FOR SEQ ID :277:

(i) SEQUENCE CHARACTERISTICS:
(A) LENGTH: 209 base pairs
(B) TYPE: nucleic acid
(C) STRANDEDNESS: double
(D) TOPOLOGY: linear

(xi) SEQUENCE DESCRIPTION: SEQ ID :277:

ACGTTCTGCG CCTTCCTAGG AGAGTCTTAC AGTGTTGAGA TTTCACAAGC 50

AATGCGAGTG TAAAATACCA GCTCTACAAG AAGCTAGGCT CTGTGACGGC 100

ATAGTTTTCA GTAGCTTTAT CACAATGAAA CGAGAATTAT ATGACATGGT 150

AGCAGAAATA GGCCCTTTCG TGNGCTGTTC TATTTNCTCG GATNGTAGAT 200

ATAGTAATC 209

(2) INFORMATION FOR SEQ ID :278:

(i) SEQUENCE CHARACTERISTICS:
(A) LENGTH: 107 base pairs
(B) TYPE: nucleic acid
(C) STRANDEDNESS: double
(D) TOPOLOGY: linear

(xi) SEQUENCE DESCRIPTION: SEQ ID :278:

AAGACATCCT AAGCTATGTT GAGGAGGGAG AAGATCTGAG ACTCAAGTTC 50

TGCTGTTAAC CATGAGGTGA TTTAGTAGCT AAGTACGCCT TAGCCTTTTA 100

GAGTCTT 107

(2) INFORMATION FOR SEQ ID :279:

(i) SEQUENCE CHARACTERISTICS:
(A) LENGTH: 276 base pairs
(B) TYPE: nucleic acid
(C) STRANDEDNESS: double
(D) TOPOLOGY: linear

(xi) SEQUENCE DESCRIPTION: SEQ ID :279:

ATGAAGAGAA AACCATCCTC CCATATGAAA ATATTTGCAG TAGGAGAACA 50

CAGTGCAATA GGCTCCAAAA ATGGCTTTTA AGACCTTTGG NGGGGCAGTT 100

ACTACTGCTT TAAAAGCCAG GTTAAAGTAT ACTCTAAGCA AAGATGACCG 150

TAGAGCAGCT AGCTTCCTTT TCTATAANNA TAGGGAAAGC TCTCTCCATC 200

GTCCATCAAA TCAGCTCTAG AAGGTTTTTC TTTCCCCNCT ATAAGTGCAC 250

AAAGGGGAAA CACTGATTTC AAGCTT 276

(2) INFORMATION FOR SEQ ID :280:

(i) SEQUENCE CHARACTERISTICS:
(A) LENGTH: 185 base pairs
(B) TYPE: nucleic acid
(C) STRANDEDNESS: double
(D) TOPOLOGY: linear

(xi) SEQUENCE DESCRIPTION: SEQ ID :280:

AGGAACAGGA TAGGTGTATG CATACTACGG CTAAGGAGAA ACAAGTCCTA 50

CATACCAGGG TAGTGAGAAC ATTACCGCAT AACAGGGAAC TGTGATTATT 100

TAAAAAACGC AGAACTTATT TTATCCGTGC TTTAGAAATA ACTGTATACA 150

GTGTTATAAG TTGAAAAGAA CTCAAAACAA CCAAT 185

(2) INFORMATION FOR SEQ ID :281:

(i) SEQUENCE CHARACTERISTICS:

(A) LENGTH: 186 base pairs
(B) TYPE: nucleic acid
(C) STRANDEDNESS: double
(D) TOPOLOGY: linear

(xi) SEQUENCE DESCRIPTION: SEQ ID :281:

AGGAACAGGA TAGGTGTATG CATACTACGG CTAAGGAGAA ACAAGTCCTA 50

CATACCAGGG TAGTGAGAAC ATTACCGCAT AACAGGGAAC TGTGATTATT 100

TAAAAAACGC AGAACTTATT TTATCCGTGC TTTAGAAATA ACTGTATACA 150

GTGTTATAAG TTGAAAAGAA CTCAAAACAA CCAATA 186

(2) INFORMATION FOR SEQ ID :282:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :282:

TGTGGGCTCA GTAATGTCCC CGCTGATGAC AATTTCGAGA GTCCATGTTC 50

TATAGAAACC TTGAGGTCGG CCAGCCGTGT CTTGGCCAAT GAGATGTAGT 100

TGTGAGGAGG GGGCGTACTT GGGGACCAGG GGTGGGTGGA GATGTGCCCT 150

GTAGGCACAG GGAGACTCAA AAGCACGAGT TNTGAAAGCG TAAATGGG 198

(2) INFORMATION FOR SEQ ID :283:

(i) SEQUENCE CHARACTERISTICS:
(A) LENGTH: 222 base pairs
(B) TYPE: nucleic acid
(C) STRANDEDNESS: double
(D) TOPOLOGY: linear

(xi) SEQUENCE DESCRIPTION: SEQ ID :283:

AGAAAAAGGT GAAGCGAAGC CCAAGGACAC CATGCTAAGG GCAAAAGTAA 50

GAGACAGTCT CAGAACTGAG AGAATCGTGT CTTCTGCTTT TTGAAGTAGA 100

CTGTCACACT CAGGCAGCCT GTCAATGCTG AATGTTAGGA CTTCTGTCTC 150

CGCTGGAGAC ACGCCTGGGC AAGTCAGCGT TTAGTGTTTG ACAGCTTTCT 200

CAGCTCCCTG ACTCCGTTTA CC 222

(2) INFORMATION FOR SEQ ID :284:

(i) SEQUENCE CHARACTERISTICS:
(A) LENGTH: 162 base pairs
(B) TYPE: nucleic acid
(C) STRANDEDNESS: double
(D) TOPOLOGY: linear

(xi) SEQUENCE DESCRIPTION: SEQ ID :284:

ATGGATCAAA CTACCTCTAT AATGAAGACT GTTCTCAAAA ACGCGAGGNA 50

ATGTGNGACG ACACTGACCT ATCAGACAAG AGGGCATGCC CCCCTGGCCA 100

CCTTTGNCGC TGTTTNTGCA ACGTTCGCAG TGNTACTCTG CGTGAACCGG 150

TAGACTGCTT GG 162

(2) INFORMATION FOR SEQ ID :285:

(i) SEQUENCE CHARACTERISTICS:
(A) LENGTH: 67 base pairs
(B) TYPE: nucleic acid
(C) STRANDEDNESS: double
(D) TOPOLOGY: linear

(xi) SEQUENCE DESCRIPTION: SEQ ID :285:

GGATCCANGC AAAGCCCACT CCTCCAGGGT GAAGTTTTTC TCCCGCGACA 50

GACAGCAGAC TCGAGCC 67

(2) INFORMATION FOR SEQ ID :286:

(i) SEQUENCE CHARACTERISTICS:
(A) LENGTH: 153 base pairs
(B) TYPE: nucleic acid
(C) STRANDEDNESS: double
(D) TOPOLOGY: linear

(xi) SEQUENCE DESCRIPTION: SEQ ID :286:

GGTCGGATCA GGTCACCCGG CAGCAGCAGC TCTCGAGAGC TGAGGCACAA 50

GGCAGGGGCC CGGCTGTACA CCTGCAGGAC CCAGGAGAGC CTGTTGCAGT 100

TCTTGTCCGA AGCGCCGAGA TAGCCACTCA GTGTCCCTGC AGCAGGAGCA 150

GAA 153

(2) INFORMATION FOR SEQ ID :287:

(i) SEQUENCE CHARACTERISTICS:
(A) LENGTH: 293 base pairs
(B) TYPE: nucleic acid
(C) STRANDEDNESS: double
(D) TOPOLOGY: linear

(xi) SEQUENCE DESCRIPTION: SEQ ID :287:

TACTACGGCT AAGGAGAAAC AATGTTCCTA CATATCACGG GTAGTGAGAA 50

CATATCTGCA TAACAGGGAA CTGTGATATT TAAAAACAGC AGAACTTATT 100

CCATCGTGCT TTAGAAATAA CTGTATACAG TGTTATAAGT TGAAAAGAAC 150

TCAAAATAAC TGATATAAAT ACATCTATGT ATTAGAATTT AAAAAAGCTG 200

CTTTCTGTGA AGTCAATCAG CTATATTAAA AATGACACAA ATCCAAAACC 250

GATGCATGCC ATATANAAGG GACATTGNAA GTCCGCTCGC TGC 293

(2) INFORMATION FOR SEQ ID :288:

(i) SEQUENCE CHARACTERISTICS:
(A) LENGTH: 114 base pairs
(B) TYPE: nucleic acid
(C) STRANDEDNESS: double
(D) TOPOLOGY: linear

(xi) SEQUENCE DESCRIPTION: SEQ ID :288:

AGGGTAAATG TACGTTGTTG AGTCACAGGC CTGCCAGACC TCTACTACCT 50

CATTGTCCCC TCCGGTGACC AGTTCTGCCG TCACTGTCAG GAGAATGCCC 100

GTGTTGAATC ACTG 114

(2) INFORMATION FOR SEQ ID :289:

(i) SEQUENCE CHARACTERISTICS:
(A) LENGTH: 290 base pairs
(B) TYPE: nucleic acid
(C) STRANDEDNESS: double
(D) TOPOLOGY: linear

(xi) SEQUENCE DESCRIPTION: SEQ ID :289:

ATGAACCAGT GCCCGAGGTA CCCGGGTGTG GACCAGAGAA GTTTGCTGGA 50

AAGGACGTTG CCAGGACTTA CACGTTTACA GATCCAGCAA CTGCTCTNCC 100

CAGTGCCACA ACCATGGGGC GCCAACCACA AGCAGGAGTG CCACTGCCAC 150

GCGGGCTGGG TCCCACCCNA CTGCGCGAAG CTGCTGACTG AGGNGCACGC 200

AGCGTCCGGG AGCCTCCCTG TCCTTGTGTG GGGTATGGNG CTCTGGCGTT 250

GNGCTGGTTA CCCTGTAGGA GTTANTGNCT ACCGGAAGCT 290

(2) INFORMATION FOR SEQ ID :290:

(i) SEQUENCE CHARACTERISTICS:
(A) LENGTH: 179 base pairs
(B) TYPE: nucleic acid
(C) STRANDEDNESS: double
(D) TOPOLOGY: linear

(xi) SEQUENCE DESCRIPTION: SEQ ID :290:

AACCGCTCTC CACACCGCTC CCACGAGCTC CAAGCTTAAA CGTCCAGATN 50

NACTTTGTTG CTTTGCTGAT TTTAACAGCT TGATTCTAAG CNCTTACTAG 100

TATCATNTGT GGCAGGACTT GNTCCATATC AGTGTTACTT TTGCTACTGT 150

TTTGTAGAAC GATGTACATG AATGAGCCT 179

(2) INFORMATION FOR SEQ ID :291:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :291:

TCTTTCATTT TGNCTGCCAA TCATTGTCAG AAATTTAGGG AAGTCAATTG 50

TGCCATTACC ATCGGCATCT ACTTATTAAT TATGTCCTGT AACTCTGCTT 100

CTGTGGGATT CTGCCCAAGA GATCTCATTA CAGTTCCCAA TTCTTTGTTG 150

TTATAGTACC ATCACCATTT CGTTAAATAG TGAAAGAGCT TTTTGAATC 199

(2) INFORMATION FOR SEQ ID :292:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :292:

GGAAGACCAT TCTGATCATC CTCACTGACG CCACAGGCAA GAGGGTGGTT 50

TTCCTGAAGC AGCTGGCTGT GGCTTATACT CGTGACTGGA CCTCTGGNCT 100

CAATTGAGTT CCTCAACGAA GACCACACCA GAAATTGTCA TTGCCACCTC 150

AACCGAANNG ATATTACAAT GTAAAAA 177

(2) INFORMATION FOR SEQ ID :293:

(i) SEQUENCE CHARACTERISTICS:
(A) LENGTH: 295 base pairs

(B) TYPE: nucleic acid
(C) STRANDEDNESS: double
(D) TOPOLOGY: linear

(xi) SEQUENCE DESCRIPTION: SEQ ID :293:

ATTGGTTTTC CTCATCCACT GAGAATGCTG CTGGTTACTG CAAACGTTCA 50

CCAACCANAG CTTTGGTCCA TACAGCTTTC TTCTAGATTN GGAGACTCTC 100

AAGGACAGCA GGNGCTTTAA AATCCCTGGA CTGTGTTGAG AGGGCTTCTT 150

TAGGCTTTTC ATGATGTGAA TAGCCAGTCA TGAACTTTGN GTCTGTTTCT 200

TTTAGGCTCT TTTTGCAGCA GCAGAGCCAT GCTATNGAAG GAGTGAGAAC 250

CTATGCGAGN GACCCNGTGN TTGNACTTGC CAGGGAGCTT GGCGT 295

(2) INFORMATION FOR SEQ ID :294:

(i) SEQUENCE CHARACTERISTICS:
(A) LENGTH: 78 base pairs
(B) TYPE: nucleic acid
(C) STRANDEDNESS: double
(D) TOPOLOGY: linear

(xi) SEQUENCE DESCRIPTION: SEQ ID :294:

AATGATCCTT TTTTCGCTCT TACCTATAGT ACAAGTCCAT GATACTACTG 50

CATATTTTAC CATTTTGNAA ACTGTGAG 78

(2) INFORMATION FOR SEQ ID :295:

(i) SEQUENCE CHARACTERISTICS:
(A) LENGTH: 163 base pairs
(B) TYPE: nucleic acid
(C) STRANDEDNESS: double
(D) TOPOLOGY: linear

(xi) SEQUENCE DESCRIPTION: SEQ ID :295:

ATTCTGTACC TGTTCTGAAC CTCGCCATAA GGGACTTGCA GCTTCGATTT 50

GCTAACCTGA AATTCTGCTG CTGCCATGGA ACAAGCCTGG GCTAGCTTGG 100

GGGAGGAGGA GAGACCATGT GGAGTAGAGC CAAGCTCTGG ACATTTGAGA 150

GAGCCCGGTA ATA 163

(2) INFORMATION FOR SEQ ID :296:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :296:

AGCTTAGAGC GGAACGGGTT CCACGTGCGG TACTCCTGCT TCACGCCGCC 50

CTCGGTCACC GTGACGCGCC TCTCGCCGTA CACAGACTGG CCCGGCACCA 100

TGTTAGCGTG ACCAGCGCGT CCTCCGCCCC GCGTNAGATG AAGAGGCCCT 150

CGTGCCGGTG CGCTCCACCG ACACCACCAT GGCCCCTTC 189

(2) INFORMATION FOR SEQ ID :297:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :297:

GGAAACCAGC CCCTCACATC CTCCCTGAAC TTCCTGTCCC CACTCACACA 50

AGTGGTCCGG TGTCACCCTG CAGTTGGGTA TAGTCATAGG TACCATTGAT 100

GACGCCTTCT ACCTCGGCCA TGTAGGCCTT CCAGTCAACC TCTGTGTCTG 150

GAAGAAGACA AGATGATCTG GTTACTTTTG AGTCTAGAAC TTGTCTGCC 199

(2) INFORMATION FOR SEQ ID :298:

(i) SEQUENCE CHARACTERISTICS:
(A) LENGTH: 181 base pairs
(B) TYPE: nucleic acid
(C) STRANDEDNESS: double
(D) TOPOLOGY: linear

(xi) SEQUENCE DESCRIPTION: SEQ ID :298:

AAAAGATGAT AAATCCACCC TCNTGCTCTT AAAATCCCAT AAACGNTAGG 50

CTCTGGAGAA ACAAGTTGTT CTGTCGAGCC CTTGCCATCA ACACACTAAG 100

CAATCATAGT CCCCCAGAGC ACNAGATACT GCTAATGACC ATTANATNTT 150

GTATCATCAT GCTGCCTCCT GCATTTGAAT T 181

(2) INFORMATION FOR SEQ ID :299:

(i) SEQUENCE CHARACTERISTICS:
(A) LENGTH: 314 base pairs
(B) TYPE: nucleic acid
(C) STRANDEDNESS: double
(D) TOPOLOGY: linear

(xi) SEQUENCE DESCRIPTION: SEQ ID :299:

AACATTGTTT ATTCATCCAG CAGTGTTGCT CAGCTCCTAC CTCTGTGCCA 50

GGGCAGCATT TTCATATCCA AGATCAATTC CCTTTTTAGC ACAGCCTGGG 100

GAGGGGGTCA TTGTTCTCCT CGTCCATCAG GGATCTCAGA GGCTCAGAGA 150

CTGCAAGCTG CTTGCCCAAG TCACACAGCT AGTGAAGACC AGAGCAGTTT 200

CATCTGGTTG TGACTCTAGC TCAGTGCTCT TCCACTACTT ATATNCGCCT 250

TGGTGCCACC AAAAGTGCTC CCCAAAAGGA AGGAGAATGG GATTTTTTCC 300

GAGGCATGTA CATT 314

(2) INFORMATION FOR SEQ ID :300:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :300:

AAGAGTCCTG AAGGCATATC CAAGCCTGTT GTTCCTGCAG ACCTCATTAC 50

CACGCCAACA GAGAAGGCTG GACTGCTGCC CACATGCTGC TTCCAAAGGT 100

TTTAAGAACT GCCTAGAAAT CTCGTGTAGG CACGAAGGGC TTGAGCCAGA 150

AAGGAGAGAC AAGTGCAA 168

(2) INFORMATION FOR SEQ ID :301:

(i) SEQUENCE CHARACTERISTICS:
(A) LENGTH: 142 base pairs
(B) TYPE: nucleic acid
(C) STRANDEDNESS: double
(D) TOPOLOGY: linear

(xi) SEQUENCE DESCRIPTION: SEQ ID :301:

ACCCCACATG CCCAGATGTC CACGNGCTTG CNATACGCCT CTTTGCAAAG 50

GACCTCAGGG GACAGGTACC TGGTGTGCCA GCGAAACCAA ACCATGCCTG 100

CTGGTTCCCC TGACCTCGAT AGCTAGGCCA AGTTTGCCAG CT 142

(2) INFORMATION FOR SEQ ID :302:

(i) SEQUENCE CHARACTERISTICS:
(A) LENGTH: 197 base pairs
(B) TYPE: nucleic acid
(C) STRANDEDNESS: double
(D) TOPOLOGY: linear

(xi) SEQUENCE DESCRIPTION: SEQ ID :302:

AGAATTCGTA ACTCATCCTA GAGTGGGCAC ATTTTAGACA TAGCAGGCGT 50

GATGACCAAC AAAGACTGAA GTTCCCTATC TACGGAAAGG CATGACTGGG 100

AGGCCCACAA GGACTCTCAT TGAGTTCTTA CTTCGTTTCA GTCAAGACAA 150

TGCTTAGTTC AGATACTCAA AAATGTCTTC ACTCTGTCTT AAATTGG 197

(2) INFORMATION FOR SEQ ID :303:

(i) SEQUENCE CHARACTERISTICS:
(A) LENGTH: 236 base pairs
(B) TYPE: nucleic acid
(C) STRANDEDNESS: double
(D) TOPOLOGY: linear

(xi) SEQUENCE DESCRIPTION: SEQ ID :303:

AGAATTGCAA CTCATCCTAA GTGGGCACAT TTAGACATAG CAGGCATGAT 50

GACCAACAAA GATGAAGTTC CCTATCTACG AAAAGGCATG ACTGGGAGGC 100

CCACAAGACT TTCATCGAGT TCTTACTTCT TTCAATCAAG ACAATGCTTA 150

TTCAGATACT CAAAAACGTT TCACTCTGTC TTAAATGAAC AATTGAATTT 200

AAAAGTTTTT GAATAAATGA TGAAAATTTT TTAACT 236

(2) INFORMATION FOR SEQ ID :304:

(i) SEQUENCE CHARACTERISTICS:
(A) LENGTH: 220 base pairs
(B) TYPE: nucleic acid
(C) STRANDEDNESS: double
(D) TOPOLOGY: linear

(xi) SEQUENCE DESCRIPTION: SEQ ID :304:

ATGTCATTCT CCTCATCCTC CGCATCTCCA CTGTCGTGCA CAAGGACCAC 50

CATGTTTCCT TTAGTTCCCA GCACACGGGG CTCTGCAGTA GTGAATGAAG 100

TCTAGCACAG CCACCGCCCC CATGCCCAGG CTCAGGAGCA CACTGAGGTC 150

GTCCACCAAC ACACCGGGTA CGTCCACCGA GCCTCTCCAC TGCTCTGGCT 200

TTAGGCCTCC CGTACAAACT 220

(2) INFORMATION FOR SEQ ID :305:

(i) SEQUENCE CHARACTERISTICS:
(A) LENGTH: 145 base pairs
(B) TYPE: nucleic acid
(C) STRANDEDNESS: double
(D) TOPOLOGY: linear

(xi) SEQUENCE DESCRIPTION: SEQ ID :305:

AGAAGAAAGG ACACCATTAC CATCCATATT GACATCGCAT TTCCATAGAA 50

ATGCCAAAGA AAGAAGGTCC TGGGGTTTTT TATAGAAGCT CAAAAAGNTC 100

AACCTTCGAT GCTATCCCCC AGCCCAATAC AAAATCAGAA AAAGC 145

(2) INFORMATION FOR SEQ ID :306:

(i) SEQUENCE CHARACTERISTICS:
(A) LENGTH: 120 base pairs
(B) TYPE: nucleic acid
(C) STRANDEDNESS: double
(D) TOPOLOGY: linear

(xi) SEQUENCE DESCRIPTION: SEQ ID :306:

AGTCAAGGCA TTATGGTTTT TAATCTGAAA CTTAGAGAAC CCTTTAATAT 50

TNGCTTTTAC TGGCGTACAT ATGAGTGGAA TATAAACTGT ACACACNNNG 100

NGNTGATATA AACAGATNNA 120

(2) INFORMATION FOR SEQ ID :307:

(i) SEQUENCE CHARACTERISTICS:
(A) LENGTH: 120 base pairs
(B) TYPE: nucleic acid
(C) STRANDEDNESS: double
(D) TOPOLOGY: linear

(xi) SEQUENCE DESCRIPTION: SEQ ID :307:

AGTCAAGGCA TTATGGTTTT TAATCTGAAA CTTAGAGAAC CCTTTAATAT 50

TNGCTTTTAC TGGCGTACAT ATGAGTGGAA TATAAACTGT ACACACNNNG 100

NGNTGATATA AACAGATNNA 120

(2) INFORMATION FOR SEQ ID :308:

(i) SEQUENCE CHARACTERISTICS:
(A) LENGTH: 247 base pairs
(B) TYPE: nucleic acid
(C) STRANDEDNESS: double
(D) TOPOLOGY: linear

(xi) SEQUENCE DESCRIPTION: SEQ ID :308:

ACTGCACCCA CCCCAAGGCC ATGGCAGGGT ATGGAGATGT CATTTATCAT 50

AAGATGGACA GATAAGCTGG ACCAATAATT AAGATTCCAG CAGAGGGTGA 100

GGACACCCAG ATGCGCCAGG ACTGTAGGAA ATCACAATGA TGGCAACGTC 150

TTGCCTTCCT GGGGACAGGG AGCCCTATTC AAANANAGTC ACATCTGAGG 200

AGCCGGGGGT TATAACATCA AGTCTGTCCT TGACCTCACA AAGCCAG 247

(2) INFORMATION FOR SEQ ID :309:

(i) SEQUENCE CHARACTERISTICS:
(A) LENGTH: 107 base pairs
(B) TYPE: nucleic acid
(C) STRANDEDNESS: double
(D) TOPOLOGY: linear

(xi) SEQUENCE DESCRIPTION: SEQ ID :309:

AGGTAAGGTT GTCACGTGGA ACAACTGATA AAGGTCAGCT ATATATGTAG 50

AGCTATATAT GTGAGTCACA AGGTGTGTGA CATACGTGTA TACGTATAAT 100

ATGCGTT 107

(2) INFORMATION FOR SEQ ID :310:

(i) SEQUENCE CHARACTERISTICS:
(A) LENGTH: 114 base pairs
(B) TYPE: nucleic acid
(C) STRANDEDNESS: double
(D) TOPOLOGY: linear

(xi) SEQUENCE DESCRIPTION: SEQ ID :310:

GGAAGACCTC GCTGGACATC CTGCAGGGCC ACATGGTGGC TCTGCTTGTG 50

ATTCTGGGGT CCACCCTCTC CAGTCAAGAG AACCCCCGTC GGGTTTAATG 100

TATCAGGAAA GAGA 114

(2) INFORMATION FOR SEQ ID :311:

(i) SEQUENCE CHARACTERISTICS:

(A) LENGTH: 237 base pairs

(B) TYPE: nucleic acid

(C) STRANDEDNESS: double

(D) TOPOLOGY: linear

(xi) SEQUENCE DESCRIPTION: SEQ ID :311:

ATGGCAAATA GGAAGAAGCT CAGTATCCTC CTCCCACCAT AACCCCACTC 50

TCCACTGCCT CCTGGACCAT AGTTTCCTCC ACTATACGGT CCCCCCATGT 100

TCCTGCTACC ACCAAAGTTT CCACTCTTTA TCGAACCGTA TTAGAAGGTC 150

GCTGGTTATA ATTTCCAAAA TATGTAATTT CCACTTCCAA ATCCTTTATA 200

GTTGTCATAA CCACCTCCGT AGCCCCCACC CTGTTGC 237

(2) INFORMATION FOR SEQ ID :312:

(i) SEQUENCE CHARACTERISTICS:

(A) LENGTH: 147 base pairs

(B) TYPE: nucleic acid

(C) STRANDEDNESS: double

(D) TOPOLOGY: linear

(xi) SEQUENCE DESCRIPTION: SEQ ID :312:

TNNTCCACAA CAGNGGGACT ACTGAAGACT AGAGAACGCC TCTGTGNGAG 50

TGGTGCAGAC AAAGACCTCA CTAAAGTGNG CTTAACAGAG TACTAGAGGA 100

GAGAACTTGG CAATAGCAAG TACAGACAAC TATGTGAGAA ATACTGC 147

(2) INFORMATION FOR SEQ ID :313:

(i) SEQUENCE CHARACTERISTICS:
(A) LENGTH: 151 base pairs
(B) TYPE: nucleic acid
(C) STRANDEDNESS: double
(D) TOPOLOGY: linear

(xi) SEQUENCE DESCRIPTION: SEQ ID :313:

CACAACAGNG GGACTACTGA AGACTAGAGA ACGCCTCTGT GNGAGTGGTG 50

CAGACAAAGA CCTCACTAAA GTGNGCTTAA CAGAGTACTA GAGGAGAGAA 100

CTTGGCAATA GCAAGTACAG ACAACTATGT GAGAAATACT GCTCCCAAAG 150

G 151

(2) INFORMATION FOR SEQ ID :314:

(i) SEQUENCE CHARACTERISTICS:
(A) LENGTH: 287 base pairs

(B) TYPE: nucleic acid
(C) STRANDEDNESS: double
(D) TOPOLOGY: linear

(xi) SEQUENCE DESCRIPTION: SEQ ID :314:

GAAGGTTGTA CGTGGACACT ATAAAGGTCA GCAAATTGGC AAAGTAGTCC 50

AGGTTTACAG GAAGAAATAT GTTATCTACA TCGAACGGGT GCAGCGGGAA 100

AAGGCTAATG GCACAACTGT CCACGTAGGC ATTCACCCCA GCAAGGTGGT 150

TATCACTAGG CTAAAACTGG ACAAAGACCG CAAAAAGATC CTCGAACGGA 200

AAGCCAAATC TCGCCAAGTA GGAAAGGAAA AGGGCAAATA CAAGGAAGAA 250

ACCATTGAGA AGATGAGGAA TAAGTAATTN ATATANA 287

(2) INFORMATION FOR SEQ ID :315:

(i) SEQUENCE CHARACTERISTICS:
(A) LENGTH: 183 base pairs
(B) TYPE: nucleic acid
(C) STRANDEDNESS: double
(D) TOPOLOGY: linear

(xi) SEQUENCE DESCRIPTION: SEQ ID :315:

GGAATCAAAC GNCTCTATAA TGAAGATAAT GTTCAGAAAA CGTGGGTTCT 50

GTGGTGACAC TGATTTATCA AGACAAGAGG GACATGCTTC CCCTTGTCCA 100

CCTTTGCAGC CTGTTTCTGT CATGTAGTTT CAACAAGTGC TACCTTGAGT 150

GTAAACTAAG GTAGACTACT CTGNGAATAA GAA 183

(2) INFORMATION FOR SEQ ID :316:

(i) SEQUENCE CHARACTERISTICS:
(A) LENGTH: 135 base pairs
(B) TYPE: nucleic acid
(C) STRANDEDNESS: double
(D) TOPOLOGY: linear

(xi) SEQUENCE DESCRIPTION: SEQ ID :316:

GGAATGAATC AAACGNCTCT ATAATGAAGA TAATGTTCAG AAAACGTGGG 50

TTCTGTGCGT GCACTGATTT ATCAAGACAA GAGGGACATG CTTCCCCTTG 100

CCACCTTTGC AGCCTGTTTC TGTCATGTAG TTTCA 135

(2) INFORMATION FOR SEQ ID :317:

(i) SEQUENCE CHARACTERISTICS:
(A) LENGTH: 187 base pairs
(B) TYPE: nucleic acid
(C) STRANDEDNESS: double
(D) TOPOLOGY: linear

(xi) SEQUENCE DESCRIPTION: SEQ ID :317:

CGTTTGAAAT TCATCCCAAC TGTAGGCTGA GTGACCTGAA GTTGACAGAC 50

TGCCGAAGTC CAAAAGCTTC AGCATTTCCT TAGTGTCAGG ATCTACTTCA 100

ATAATNNTGA TCCAAGGCTG AGACCTCAGA AACATAATGC TCTCCTTTCC 150

CTATNTTTTC TGCGGCTTGA TGGAGATACC TTTACTG 187

(2) INFORMATION FOR SEQ ID :318:

(i) SEQUENCE CHARACTERISTICS:
(A) LENGTH: 268 base pairs
(B) TYPE: nucleic acid
(C) STRANDEDNESS: double
(D) TOPOLOGY: linear

(xi) SEQUENCE DESCRIPTION: SEQ ID :318:

TCCCAGGAGA AGGAACTTTG AAATTCATCC CAACGTAGGC TGAGTGACCT 50

GAAGATTGAC AGACTGCCGA AGTCCAAAAG CTTCAGCATT TCCTTAGTGT 100

CAGGATCTAC TTCAATAATN NTGATCCAAG GCTGAGACCT CAGAAACATA 150

ATGCTCTCCT CCCTTNCTTT TCTGCGCTTG ATGGAGATAC CTTTCACTGT 200

GCCTCTCTGA ATCGTTTCAT CAGATGCTGA CGTAACCTGC TATTTGTTGN 250

AGCTTTCNGT TGNNNTAA 268

(2) INFORMATION FOR SEQ ID :319:

(i) SEQUENCE CHARACTERISTICS:

(A) LENGTH: 138 base pairs
(B) TYPE: nucleic acid
(C) STRANDEDNESS: double
(D) TOPOLOGY: linear

(xi) SEQUENCE DESCRIPTION: SEQ ID :319:

ACTGCACCCA CCCCAAGGCC ATGCAGGGTA TGGAGATGTC ATTTATTATA 50

AGATGAACAG ATAAGCTGGA CCAATAATTT ACAGATTCCA CAGAGGGTGA 100

GGACACCCAG ATGCGCCAGG ACTGTAGGAA ATCACAAT 138

(2) INFORMATION FOR SEQ ID :320:

(i) SEQUENCE CHARACTERISTICS:
(A) LENGTH: 118 base pairs
(B) TYPE: nucleic acid
(C) STRANDEDNESS: double
(D) TOPOLOGY: linear

(xi) SEQUENCE DESCRIPTION: SEQ ID :320:

AAATGATGAT AAATTCATCC TCTTCTGCTC TTAAAATTTC ATAAACCTCA 50

GGCTCTGGAG AAACAAGTTG TTCTGTTGGG CCCTTGCCAT CAACACACTT 100

GTAATCATAC TTCCCCCA 118

(2) INFORMATION FOR SEQ ID :321:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :321:

AGAACAGTTG AAGGTCTGAG GTGTTGAGAG GCAAATGGGG TCTCTGGGTG 50

GATCCTGGTC CTGTCAGCAG GCCTGGACTT GTTCAGGATG GACTGGTGGC 100

GTTATAAAGG GCAGATCAGG GTGCCTGTCC CTTGGCATGT CCCTTAAGAT 150

TTCCTTTTCC 160

(2) INFORMATION FOR SEQ ID :322:

(i) SEQUENCE CHARACTERISTICS:
(A) LENGTH: 281 base pairs
(B) TYPE: nucleic acid
(C) STRANDEDNESS: double
(D) TOPOLOGY: linear

(xi) SEQUENCE DESCRIPTION: SEQ ID :322:

AGGAGGGAGT TAATCCAAAC CACACAATGA AGTCTTCAAA CCACCNCCCG 50

AACAGGGCTG CTGATTGTTC CTTTCACTTT TGNGGTGACC TTGAGCTCCC 100

TTAAAAAAAA AACTTGGAGA ATCACAACTG GCAATGCACC GCAGTTCTCG 150

AACTACACAA GCATAGTCTG ACTAAGTCAC ATGTGTTTCC ATATCAACTT 200

GTTTGACAGG GCGACCTACT GCAAAGCAGG CTCAGTTACC CCACCAGTCA 250

ACCCCCTGGG AGTATAATNN TCTCCATANA A 281

(2) INFORMATION FOR SEQ ID :323:

(i) SEQUENCE CHARACTERISTICS:
(A) LENGTH: 99 base pairs
(B) TYPE: nucleic acid
(C) STRANDEDNESS: double
(D) TOPOLOGY: linear

(xi) SEQUENCE DESCRIPTION: SEQ ID :323:

GGAATTTTCT CAAAGAAAAC GCAATAGCCA ATTGGAACCT ACTNTANCGC 50

ANTTTATNNN GTCTTCTTTA ANNTAGAGTG ACTTACTGAT TTATTATTG 99

(2) INFORMATION FOR SEQ ID :324:

(i) SEQUENCE CHARACTERISTICS:
(A) LENGTH: 167 base pairs
(B) TYPE: nucleic acid
(C) STRANDEDNESS: double
(D) TOPOLOGY: linear

(xi) SEQUENCE DESCRIPTION: SEQ ID :324:

GGAATTTTCT CAAAGAAAAC GCAATAGCCA ATTGGAACCT ACTNTANCGC 50

ANTTTATNNN GTCTTCTTTA ANNTAGAGTG ACTTACTGAT TTATTATTGA 100

CACNNGTCCN NNGCNCNAGN TTACCATCCT TATGTGAATA CTNCAAGGGA 150

TTGCCCGCGT TTTTAGG 167

(2) INFORMATION FOR SEQ ID :325:

(i) SEQUENCE CHARACTERISTICS:

(A) LENGTH: 160 base pairs

(B) TYPE: nucleic acid

(C) STRANDEDNESS: double

(D) TOPOLOGY: linear

(xi) SEQUENCE DESCRIPTION: SEQ ID :325:

TGATCCAACT CTTCCTGTGT GAAAAGAAGA ATGATGGCAG GAAGAACATA 50

AAGACTTTAA AACTCCTAGC CGGGGTTTGT CGGACTCTTT GNCAGTAGTG 100

ATTTAGCCAA CCAGGCAACA GAGATACGAT CTAATCTCCT CGCCCCTCTT 150

TCGGGTCGCG 160

(2) INFORMATION FOR SEQ ID :326:

(i) SEQUENCE CHARACTERISTICS:

(A) LENGTH: 124 base pairs

(B) TYPE: nucleic acid

(C) STRANDEDNESS: double

(D) TOPOLOGY: linear

(xi) SEQUENCE DESCRIPTION: SEQ ID :326:

GGAAGGGGTG TTGTTTGATA GACATTATCT GTGGCTGGGT CTTTCTGGCT 50

GATGAGAGAC ATGTAGATTA TGTGAAGCAA GTGGCTAGAA GATGAGAGAG 100

AACATGAGAG AGCAGAGTGC TCTC 124

(2) INFORMATION FOR SEQ ID :327:

(i) SEQUENCE CHARACTERISTICS:
(A) LENGTH: 253 base pairs
(B) TYPE: nucleic acid
(C) STRANDEDNESS: double
(D) TOPOLOGY: linear

(xi) SEQUENCE DESCRIPTION: SEQ ID :327:

AGCCTAGTTT GAACATCCTC ATCCCNGTGT TAGACCGGAT CCGATATACA 50

GAGTCTTAAG GAAATCGCAT CAACGTGCCT GAGCAGTCGG CTGTGACTCT 100

CGACAATGTA ACTCTGCAAA TCGATGGAGT CCTTTACCTG CCATCATGGA 150

CCCTTACAAG GCAAGCTACG ACGCGAGGAC CCTGATATGC CGTCACCCAG 200

CTAGCTCAAA CAACCACGAG ATCAGAGCTC AGCAAACTCT CTCTGGACAA 250

AGT 253

(2) INFORMATION FOR SEQ ID :328:

(i) SEQUENCE CHARACTERISTICS:
(A) LENGTH: 200 base pairs
(B) TYPE: nucleic acid
(C) STRANDEDNESS: double
(D) TOPOLOGY: linear

(xi) SEQUENCE DESCRIPTION: SEQ ID :328:

GAACCTCTGG GCTCTCATCC TCTCCTATTG ACAGTGAAGC CCATGGCAAA 50

TAGAAGAAGC TCAGTATCGG CTCCTCCCAC CATAACCGCG TTNAAATCGC 100

CTCCTGGACC ATAGTTTCCT CCACCATAAG TCCCCCCATG CTCCTGCTAC 150

CACCGAAGTT CCCACTCCTG NGCGAACCGT AGTTAGAAGA TCGCTGTTAT 200

(2) INFORMATION FOR SEQ ID :329:

(i) SEQUENCE CHARACTERISTICS:
(A) LENGTH: 280 base pairs
(B) TYPE: nucleic acid
(C) STRANDEDNESS: double
(D) TOPOLOGY: linear

(xi) SEQUENCE DESCRIPTION: SEQ ID :329:

TGACCTGATC ATCCTGAAAA ACTTTATGGG GGAGAAAGGT CAGCAGCTTC 50

TCTTTCTTTT NTCGAAAATN ATAAAACTGC GTATTCTACT TTATATTTAA 100

TGTAAGGAAG AAAATATACA AGCCCATATT TACATCGTAT TTCTATTAAG 150

AGCAACAATA GTTCATATGT TCATGTTTGC TACTATCACA ATNCAACATA 200

TGAACACAGA TCAGCTCTAT ACCATGAATA CTGCTGGAAG TGATGGTTTA 250

GGATTACCAA CTCACTGCTG CCATGACCGA 280

(2) INFORMATION FOR SEQ ID :330:

(i) SEQUENCE CHARACTERISTICS:
(A) LENGTH: 268 base pairs
(B) TYPE: nucleic acid
(C) STRANDEDNESS: double
(D) TOPOLOGY: linear

(xi) SEQUENCE DESCRIPTION: SEQ ID :330:

CCTGAAAAAC TTTATGGGGG AGAAAGGTCA GCAGCTTCTC TTTCTTTTNT 50

CGAAAATNAT AAAACTGCGT ATTCTACTTT ATATTTAATG TAAGGAAGAA 100

AATATACAAG CCCATATTTA CATCGTATTT CTATTAAGAG CAACAATAGT 150

TCATATGTTC ATGTTTGCTA CTATCACAAT NCAACATATG AACACAGATC 200

AGCTCTATAC CATGAATACT GCTGGAAGTG ATGGTTTAGG ATTACCAACT 250

CACTGCTGCC ATGACCGA 268

(2) INFORMATION FOR SEQ ID :331:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :331:

TCGCAGGAGA AGGAACCTAT ACACCCTTTA TAGAGCTTTA AATCGACTGT 50

AGAGTTTTGT GGTCTTCCAG TCCCAAATGT TTAATTTTCC ATTTGCATTN 100

TGAAATCACA TAACTCATGT CTGAAAAGTC CACCTGACAA GCATACCTGC 150

TACCATATGC CCTTAAAAAT 170

(2) INFORMATION FOR SEQ ID :332:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :332:

TCGCAGGAGA AGGAACCTAT ACACCCTTTA TAGAGCTTTA AATCGACTGT 50

AGAGTTTTGT GGTCTTCCAG TCCCAAATGT TTAATTTTCC ATTTGCATTN 100

TGAAATCACA TAACTCATGT CTGAAAAGTC CACCTGACAA GCATACCTGC 150

TACCATATGC CCTTAAAAAT NNGGNNNNNG NNNNGGNTGA ANGGTTCTGT 200

GTTCCAAAAA TNTAAGATTT GTT 223

(2) INFORMATION FOR SEQ ID :333:

(i) SEQUENCE CHARACTERISTICS:
(A) LENGTH: 132 base pairs
(B) TYPE: nucleic acid
(C) STRANDEDNESS: double
(D) TOPOLOGY: linear

(xi) SEQUENCE DESCRIPTION: SEQ ID :333:

AGACAATTGG CGGCATCCTC GTAGGCTTCA CTAACTCCCA CAAGTTCTTC 50

TGGTTTCATT TCGGTTATTT TTTGCAGCCA ATTCTCTCCA TGTTTGGCAG 100

TCACAAGGCT CATGTGCTTC ACCAAGGCAC TC 132

(2) INFORMATION FOR SEQ ID :334:

(i) SEQUENCE CHARACTERISTICS:
(A) LENGTH: 235 base pairs
(B) TYPE: nucleic acid
(C) STRANDEDNESS: double
(D) TOPOLOGY: linear

(xi) SEQUENCE DESCRIPTION: SEQ ID :334:

AAGTTGTTCA TGGNAGGCAT TTATCCTNTC AATAATNCAA ACGAGGCTTC 50

TGGAATAACC AGTGCCCCAT TCCATCAGAG TCTTTGCGCG ACTAAAGCCT 100

CCATNTTTGC CAATTTCAAT TGTTTGGGAT TCTAGCACTC CTTACCNGCA 150

GTAATGCCCT TGCTGCAGAC AACAACACCT GGACTGNGAG ATGGACCAAT 200

TCTCAATGGC AATCCAGGGA AAGAGTGATC CTTCT 235

(2) INFORMATION FOR SEQ ID :335:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :335:

ATGCCCGCAC CATCCGCTAC CCCGATCCCC TCATCAAGGT GAATGATACC 50

ATTCAGATTG ATTTAGAGAC TGGCAAGATT ACTGATTTCA TCAAGTTCGA 100

CACTGGTAAC CTGTGTATGG TGACTGAGGT GCTAACCTAG GAAGAATTGG 150

GNGATCACCA ACAGGAGAG 169

(2) INFORMATION FOR SEQ ID :336:

(i) SEQUENCE CHARACTERISTICS:
(A) LENGTH: 193 base pairs
(B) TYPE: nucleic acid

(C) STRANDEDNESS: double
(D) TOPOLOGY: linear

(xi) SEQUENCE DESCRIPTION: SEQ ID :336:

ATGCCCGCAC CATCCGCTAC CCCGATCCCC TCATCAAGGT GAATGATACC 50

ATTCAGATTG ATTTAGAGAC TGGCAAGATT ACTGATTTCA TCAAGTTCGA 100

CACTGGTAAC CTGTGTATGG TGACTGAGGT GCTAACCTAG GAAGAATTGG 150

GNGATCACCA ACAGGAGAGG CACNCTGNAN TCTTTTGGCN NNG 193

(2) INFORMATION FOR SEQ ID :337:

(i) SEQUENCE CHARACTERISTICS:
(A) LENGTH: 307 base pairs
(B) TYPE: nucleic acid
(C) STRANDEDNESS: double
(D) TOPOLOGY: linear

(xi) SEQUENCE DESCRIPTION: SEQ ID :337:

CAAAAGTCAT CCACAAGTTC TTTGTCTAGG ACTTCTAGCT GCTCAGACCC 50

TCAGGGTCTT TGGATTGTTA CCAAAGTCTG TCAAACAGAC CAGTAGTTTA 100

ATACCTGTAC AGAAAAATGT TTCTATTATG CTTCTAGTAT CTAGAAATTG 150

CTTGCTACAG CATGGAGGTG GTTCTGCCTT TCCCTGGCTC CTCACACTCT 200

CATCTGCAGG ATTCCCAGCT TTGCTCAGTC TTCATGCCCA CCAGAGGCAA 250

AAAGGCAAAC TAAAACTGTC ATGCAGTGGT AGGTTTGAAA TNAGCTGCTG 300

CATCATG 307

(2) INFORMATION FOR SEQ ID :338:

(i) SEQUENCE CHARACTERISTICS:
(A) LENGTH: 305 base pairs
(B) TYPE: nucleic acid
(C) STRANDEDNESS: double
(D) TOPOLOGY: linear

(xi) SEQUENCE DESCRIPTION: SEQ ID :338:

AAAAGTCATC CACAAGTTCT TTGTCTAGGA CTTCTAGCTG CTCAGACCCT 50

CAGGGTCTTT GGATTGTTAC CAAAGTCTGT CAAACAGACC AGTAGTTTAA 100

TACCTGTACA GAAAAATGTT TCTATTATGC TTCTAGTATC TAGAAATTGC 150

TTGCTACAGC ATGGAGGTGG TTCTGCCTTT CCCTGGCTCC TCACACTCTC 200

ATCTGCAGGA TTCCCAGCTT TGCTCAGTCT TCATGCCCAC CAGAGGCAAA 250

AAGGCAAACT AAAACTGTCA TGCAGTGGTA GGTTTGAAAT NAGCTGCTGC 300

ATCAT 305

(2) INFORMATION FOR SEQ ID :339:

(i) SEQUENCE CHARACTERISTICS:

(A) LENGTH: 101 base pairs

(B) TYPE: nucleic acid

(C) STRANDEDNESS: double

(D) TOPOLOGY: linear

(xi) SEQUENCE DESCRIPTION: SEQ ID :339:

AATATAAAGA GGAAATGGCT CNTCACCTTC ATAATACCAA GACCTGCTCA 50

ATTTAAGATG GCCCTTGACA TTGAAATCGT ACCTACAGAA AGCTTGNAAG 100

G 101

(2) INFORMATION FOR SEQ ID :340:

(i) SEQUENCE CHARACTERISTICS:

(A) LENGTH: 113 base pairs

(B) TYPE: nucleic acid

(C) STRANDEDNESS: double

(D) TOPOLOGY: linear

(xi) SEQUENCE DESCRIPTION: SEQ ID :340:

GTCGCAGGAG AAAAAAGTCG TCACGTCATG CGAGTGCGCA GGGGGCGTGG 50

AGAGTAGCAG TAGAGCAACA AAGTACATAT AGGACAGTGC AGCGAGGACA 100

AGGACTTCCC GCA 113

(2) INFORMATION FOR SEQ ID :341:

(i) SEQUENCE CHARACTERISTICS:
(A) LENGTH: 209 base pairs
(B) TYPE: nucleic acid
(C) STRANDEDNESS: double
(D) TOPOLOGY: linear

(xi) SEQUENCE DESCRIPTION: SEQ ID :341:

ACACAAATAA CTACATNTAC GCAATATAAT NTTTAAAAAT CCAAAGCAAT 50

ATAAAAGAGC AGAGCTAGGA CTGAACAGAA CATTTTGGTG TATAACCGGC 100

AGCTCAAAAT CGCCAGCTGA TTGGAGTAAA ACTGATTCTA AGCGTATTAA 150

ATACGATTGA TTGTTTCCAT CACTAAGGGT GCCTATGAGT TTCTGAACCA 200

TTTCTAGGG 209

(2) INFORMATION FOR SEQ ID :342:

(i) SEQUENCE CHARACTERISTICS:
(A) LENGTH: 252 base pairs
(B) TYPE: nucleic acid
(C) STRANDEDNESS: double
(D) TOPOLOGY: linear

(xi) SEQUENCE DESCRIPTION: SEQ ID :342:

GATCAANCAA AGCACAGGGA AAAGAGGCAA AATGATCACA GAAATCCGTT 50

TTTTAGACAG GTGTGACTTA CACTAACGAA AAAGAAAAAC ACATGAATAA 100

GCTTTTAATC TTCATTTTTN NNTTTTGTNA TGGTAGGCTG AGATGCTTTT 150

AAATGTGATG TTATAAGCCT AAGGCAGCTT GACTGCAGCA ACAAGTTTTT 200

TACCCTCCTT GGCAAAGCAG GTCTCCTTAT GTAGCCTTGC AGCACTTCTC 250

TA 252

(2) INFORMATION FOR SEQ ID :343:

(i) SEQUENCE CHARACTERISTICS:
(A) LENGTH: 258 base pairs
(B) TYPE: nucleic acid
(C) STRANDEDNESS: double
(D) TOPOLOGY: linear

(xi) SEQUENCE DESCRIPTION: SEQ ID :343:

GATCAANCAA AGCACAGGGA AAAGAGGCAA AATGATCACA GAAATCCGTT 50

TTTTAGACAG GTGTGACTTA CACTAACGAA AAAGAAAAAC ACATGAATAA 100

GCTTTTAATC TTCATTTTTN NNTTTTGTNA TGGTAGGCTG AGATGCTTTT 150

AAATGTGATG TTATAAGCCT AAGGCAGCTT GACTGCAGCA ACAAGTTTTT 200

TACCCTCCTT GGCAAAGCAG GTCTCCTTAT GTAGCCTTGC AGCACTTCTC 250

TACAAAAC 258

(2) INFORMATION FOR SEQ ID :344:

(i) SEQUENCE CHARACTERISTICS:
(A) LENGTH: 227 base pairs
(B) TYPE: nucleic acid
(C) STRANDEDNESS: double
(D) TOPOLOGY: linear

(xi) SEQUENCE DESCRIPTION: SEQ ID :344:

GAAGGAACCA GTGACCAGTC ATCCCCAGAG ATAGATGAAG ACCGGATCCC 50

CAACCCACAT TCAAGTCCAC TTTGCAATGT CTCCACGGCA ACGGAAGAAG 100

ATGACAAGGA TCACACCCAC AATGAAAGAG CTCCAGATGA TGGTTGAACA 150

TCACCTGGGG CAACAGCAGC AAGGAGAGGA ACCTGAGGGG CCGCTGAGAG 200

CACAGGACCC AGGAGTCCGC CCACCTG 227

(2) INFORMATION FOR SEQ ID :345:

(i) SEQUENCE CHARACTERISTICS:
(A) LENGTH: 188 base pairs
(B) TYPE: nucleic acid
(C) STRANDEDNESS: double
(D) TOPOLOGY: linear

(xi) SEQUENCE DESCRIPTION: SEQ ID :345:

AAATAAAAAA TAAGGTCCAA GCCAATGCCT AACAGATAAT AAGAGCTCAG 50

TAAATGTTGA TTGAATACTA ACAAAGTAGT GAAAGCAGAC GACACAGTAC 100

CTGGCACACT ACTAAACTGT AAATGTTTTC AAATCTGAAT CTGTAGAATT 150

CTGTAAGGTT TTATGTAATA TGANGTCATT AGCTATCA 188

(2) INFORMATION FOR SEQ ID :346:

(i) SEQUENCE CHARACTERISTICS:
(A) LENGTH: 262 base pairs
(B) TYPE: nucleic acid
(C) STRANDEDNESS: double
(D) TOPOLOGY: linear

(xi) SEQUENCE DESCRIPTION: SEQ ID :346:

AAATAAAAAA TAAGGTCCAA GCCAATGCCT AACAGATAAT AAGAGCTCAG 50

TAAATGTTGA TTGAATACTA ACAAAGTAGT GAAAGCAGAC GACACAGTAC 100

CTGGCACACT ACTAAACTGT AAATGTTTTC AAATCTGAAT CTGTAGAATT 150

CTGTAAGGTT TTATGTAATA TGANGTCATT AGCTATCATG NCTCTGGATT 200

NNCTNNNAGG TTTTAAGATG GAGCATCTGN GNATGTCAGC CCGTCCTATC 250

TAGAAGTGNA AA 262

(2) INFORMATION FOR SEQ ID :347:

(i) SEQUENCE CHARACTERISTICS:
(A) LENGTH: 159 base pairs
(B) TYPE: nucleic acid
(C) STRANDEDNESS: double

(D) TOPOLOGY: linear

(xi) SEQUENCE DESCRIPTION: SEQ ID :347:

CTCTGTTTTC CAAACGCCCA TGTGTGCTAT ACTACAACTC TTCTCGAGTC 50

TGATCAATTT GCAGTAGACC ATTTTAGTTC TTACGACGTT AATAACAAAC 100

ACTTCAACAT CANTGCTCCA ATCTGAAGTT CTTGTTGCAT TGTTAAAAGA 150

AATNTCTAA 159

(2) INFORMATION FOR SEQ ID :348:

(i) SEQUENCE CHARACTERISTICS:
(A) LENGTH: 283 base pairs
(B) TYPE: nucleic acid
(C) STRANDEDNESS: double
(D) TOPOLOGY: linear

(xi) SEQUENCE DESCRIPTION: SEQ ID :348:

TCGCAGGAGA AGGAAGCTGC ATGCACTTGA AAGGCATGGC CTGTCTCCTC 50

ACTGAGTGCA AGGTCCATCG ACCAAAGCCC TGTTCTGATC AATAACATCT 100

ACAATCGCAC CAATTTTCCG GCATGAGGTC CAAAGGAGAC ATAGGCCACC 150

CGGCCAACCT CCACGAAGCG CCTGAACACC ATGCGCTTCC CGGGACCCTC 200

TGGGAGCCGT TCGCNCCTCA GCTTCCCAGC GAGGTGGAGG TCGAGCGGCA 250

TGGNCGACGC CCCGGAGCCC ACAACAGTAA GAC 283

(2) INFORMATION FOR SEQ ID :349:

(i) SEQUENCE CHARACTERISTICS:

(A) LENGTH: 169 base pairs

(B) TYPE: nucleic acid

(C) STRANDEDNESS: double

(D) TOPOLOGY: linear

(xi) SEQUENCE DESCRIPTION: SEQ ID :349:

CAGAAAGGAG GCATTGAGCA AAAACTAGGA AAATCTGAAT AAACNGTGGA 50

CCTCGCTTGG TTAATATATT TANACTGGTG ACTCATTTTA AAACTTTCCC 100

GGGCGCGATG GCTCATGTCT GCAATCCCAG CACTTGNGAG GCTGAGCGGG 150

TGGATCACCC AGGTCAGGA 169

(2) INFORMATION FOR SEQ ID :350:

(i) SEQUENCE CHARACTERISTICS:

(A) LENGTH: 175 base pairs

(B) TYPE: nucleic acid

(C) STRANDEDNESS: double

(D) TOPOLOGY: linear

(xi) SEQUENCE DESCRIPTION: SEQ ID :350:

GAAGNAACCA GTACCAGTCA TCCCCAGAGA TAGATGAAGA CCGGATCCCC 50

AACCCACANT TAAGTCCACT TTGCAATGTC TCCACGGCAA CGAAGAAGAT 100

GACAAGGATC ACACCCACAA TGAAAGAGCT CCAGATGATG GNGAACATCA 150

CTTGGCAACA NCACAGGAAG GGACC 175

(2) INFORMATION FOR SEQ ID :351:

(i) SEQUENCE CHARACTERISTICS:

(A) LENGTH: 206 base pairs

(B) TYPE: nucleic acid

(C) STRANDEDNESS: double

(D) TOPOLOGY: linear

(xi) SEQUENCE DESCRIPTION: SEQ ID :351:

AAATAAAAAA TAAGNACCCA ACCAATGCTA CAGATAATAA GAGCTCAGTA 50

AATGTTGATT GAATCACTAA CAAAGATAAA AGCAGACGAC ACAGTACCTG 100

GCACACTACT AACTGTAAAT GTTTTAAAAT TGAATCTGTA AAATTTGTAA 150

GGTTTTATGA ATATAATATT ATTAACTATT ATGTCTCTGA ATTTTTNNNN 200

NAGGCT 206

(2) INFORMATION FOR SEQ ID :352:

(i) SEQUENCE CHARACTERISTICS:

(A) LENGTH: 76 base pairs

(B) TYPE: nucleic acid

(C) STRANDEDNESS: double
(D) TOPOLOGY: linear

(xi) SEQUENCE DESCRIPTION: SEQ ID :352:

AGACATACTG TAGTGTCTAA ATAATATTTG TCNGAAGATA ACAATTATGG 50

GACTTTAAAG CCGACAGTGA AATTAT 76

(2) INFORMATION FOR SEQ ID :353:

(i) SEQUENCE CHARACTERISTICS:
(A) LENGTH: 245 base pairs
(B) TYPE: nucleic acid
(C) STRANDEDNESS: double
(D) TOPOLOGY: linear

(xi) SEQUENCE DESCRIPTION: SEQ ID :353:

ACATTTGGCC CTCAGACTGT AATTTCCATA CTACTNTGAC TGATACTAGA 50

TGACCTGGCT GCCTAGGGGC TGTGCTGGTC TGATTTTTTG TGAGACAAAA 100

ACCACTCTAA ACCTCCTGGT GCACTGAGGC TGTACACACC GNCAGAACAG 150

GGCACTGCGT TTAAAAGTTT CTGACCAAGT GGTGACAACA GAGGNCAAAC 200

GTAAGGCTGT CTGGATAAGT TGAACCTNGC TGANCGGTAG CACCA 245

(2) INFORMATION FOR SEQ ID :354:

(i) SEQUENCE CHARACTERISTICS:
(A) LENGTH: 179 base pairs
(B) TYPE: nucleic acid
(C) STRANDEDNESS: double
(D) TOPOLOGY: linear

(xi) SEQUENCE DESCRIPTION: SEQ ID :354:

GGAATCTGTG CCACACAGCT GCACACGACT ACTGGCCAAA GGACAGCCTC 50

AGAACATCAT GAGCTGGCGT GACCTCATGA TGNNCCTCAN TGCTGTGGGG 100

CTTCTGCTNG AGGCCAGCAG AGGCTCGTAT GACTACCTGT NAGACCTGCT 150

CTTTGGGTTG AGACTTTTCA GNGACAACT 179

(2) INFORMATION FOR SEQ ID :355:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :355:

AAGGAACGNA TGACTTTCCA TCCCTTGAAC CAAGGCATGT TAGCACTTGC 50

TCCAGCATGT TGTCACCATT TCAACAGAAA TCGCACAAAT GCTACTGTGC 100

AAGGTGCAGC CAATTTTNTT GTAAGTGTGA TTTCTTACG 139

(2) INFORMATION FOR SEQ ID :356:

(i) SEQUENCE CHARACTERISTICS:
(A) LENGTH: 100 base pairs
(B) TYPE: nucleic acid
(C) STRANDEDNESS: double
(D) TOPOLOGY: linear

(xi) SEQUENCE DESCRIPTION: SEQ ID :356:

TCCAGGAGAA GGAAAACTTC CACTTATAAC TGTNTCAGCC TGACTATAAT 50

GAAGAGACTA GCAACACCTC CTGAACACAA GCCTANTGAG CCCAGTCTTT 100

(2) INFORMATION FOR SEQ ID :357:

(i) SEQUENCE CHARACTERISTICS:
(A) LENGTH: 137 base pairs
(B) TYPE: nucleic acid
(C) STRANDEDNESS: double
(D) TOPOLOGY: linear

(xi) SEQUENCE DESCRIPTION: SEQ ID :357:

AGAAGGAAAC ACCAAAGAAA CATCCAAGCA ATAAAGTGGA AGACTAACCA 50

AGATTTGGAC ATTGGAATGT TTACTGTTAT TCTTTAAGAA ACAACTACAA 100

AAAGAAAATG TCAACAAATT TTTCCTAGCT AACTGAG 137

(2) INFORMATION FOR SEQ ID :358:

(i) SEQUENCE CHARACTERISTICS:
(A) LENGTH: 228 base pairs
(B) TYPE: nucleic acid
(C) STRANDEDNESS: double
(D) TOPOLOGY: linear

(xi) SEQUENCE DESCRIPTION: SEQ ID :358:

AAGGAACAAG TGGGTCATTC TCACTAAGGC TGCCTGTCAC TGCAGTCTTG 50

ATCAGCTTGT CAATTTGATA CTTCAGCTTT TGGTCCAAGG GACGAAGCTT 100

TTCCAAAACC GTTCGAATCT CTACCAGTCT CAAAACTGCA TCATGTCCCT 150

GAAGAGATCC TCCTGAGGCT TTGTCCAGAA TGAGGTGGGT CAAATNNANG 200

GGNACATGAG CAGCAGCTGN TCTTTAAC 228

(2) INFORMATION FOR SEQ ID :359:

(i) SEQUENCE CHARACTERISTICS:
(A) LENGTH: 248 base pairs
(B) TYPE: nucleic acid
(C) STRANDEDNESS: double
(D) TOPOLOGY: linear

(xi) SEQUENCE DESCRIPTION: SEQ ID :359:

CAGGAGCAAG GAGGCCATTT TGCTGAGCTC TCACAGCTGC TAGAGATGCT 50

CAATGACTCC CAATACAACC CAAAGAGACA AACGCTTGTT TTTTCTGCCA 100

CACTCACCCT GGTGCATCAG CTCCTGCTCG AATCCTTCAT AAGAAGCACA 150

CCAAGAAAAT GAATAAAACA GCCAAACTCG ACCTTCTTAT GCAGAAAATT 200

GGCATGAGGG GCAAGCCCAA GGTCATTGAC CTCACAAGGA ATGAGGCC 248

(2) INFORMATION FOR SEQ ID :360:

(i) SEQUENCE CHARACTERISTICS:

(A) LENGTH: 253 base pairs

(B) TYPE: nucleic acid

(C) STRANDEDNESS: double

(D) TOPOLOGY: linear

(xi) SEQUENCE DESCRIPTION: SEQ ID :360:

AGGCACCTGT GGAGCCATCC TCCTTGTTTT AATCGTCCTG CTGCTGCTGC 50

CGTTCCGTGT CGCGTCGCCC ACATGACCCT GAGGTCGCAA TGATGAGTCC 100

TCTCTGGTCA GACACCGCTG GAAATGAATA CCAGGCCTGA CCTCAAGCAA 150

CCATGAACTA GCTATTAAGA AATACANNGG NAGGGCGGCA GCCGGATCGT 200

GNNGGCGTTT NTCTGNGCCG CCCGTCTCAA TCTNTGTTCT GCTTCCAGAT 250

GCC 253

(2) INFORMATION FOR SEQ ID :361:

(i) SEQUENCE CHARACTERISTICS:
(A) LENGTH: 154 base pairs
(B) TYPE: nucleic acid
(C) STRANDEDNESS: double
(D) TOPOLOGY: linear

(xi) SEQUENCE DESCRIPTION: SEQ ID :361:

GAGAACCACG GGCTCCATCC TGGCCTCCTG TNCACCTTCG AACAGATGCG 50

GATTAGCAAG CAGGAGCACA CGAATCAAGC CCCTCCAGCC ACCAAATTTT 100

CTAAACNGGC TNGGCNATGT CGTAGTTNGT TGTCAGTNGA TCGGTAGAGA 150

TAAT 154

(2) INFORMATION FOR SEQ ID :362:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :362:

GAGAACCACG GGCTCCATCC TGGCCTCCTG TNCACCTTCG AACAGATGCG 50

GATTAGCAAG CAGGAGCACA CGAATCAAGC CCCTCCAGCC ACCAAATTTT 100

CTAAACNGGC TNGGCNATGT CGTAGTTNGT TGTCAGTNGA TCGGTAGAGA 150

TAATCCTGNC CACGCCCT 168

(2) INFORMATION FOR SEQ ID :363:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :363:

AAGGGATAAA AGGAAGCTTT GNCACCACTC TACCATCAAA NNGGAATTGA 50

ACATTCCCAT TAAGGCAGTA AAACAAAAGC CAATAGCAAA CATGNATTTC 100

ATTCAACCAT TGATAGATCT CTGCCGTTAT TCTTCAGTTT CTCTTCTCGT 150

CTCTCTATTT NTTTCTCTGG TTGTCGACCA GCTGACTNTG NCATCGTT 198

(2) INFORMATION FOR SEQ ID :364:

(i) SEQUENCE CHARACTERISTICS:
(A) LENGTH: 121 base pairs
(B) TYPE: nucleic acid
(C) STRANDEDNESS: double
(D) TOPOLOGY: linear

(xi) SEQUENCE DESCRIPTION: SEQ ID :364:

TTATACAAGT CAAACTTGGA AGGTCATAGT AAGCATACCT ATGCTGAGAG 50

AAAAGCATCA AATCCTCCGC GACACATCTA GTTCATCGTA ACAAAGCAAC 100

TCGTACACTT TCAAGTTTAA A 121

(2) INFORMATION FOR SEQ ID :365:

(i) SEQUENCE CHARACTERISTICS:

(A) LENGTH: 211 base pairs

(B) TYPE: nucleic acid

(C) STRANDEDNESS: double

(D) TOPOLOGY: linear

(xi) SEQUENCE DESCRIPTION: SEQ ID :365:

GGTAAGTTGT ATTTGGGCCA GAATTTTAAA GAGCACTATT TCGACATTAA 50

AATGTATTCT TCTCGTATTA ATGCCTACAT CTTCAGAGTT TTCAATGCTT 100

TCTAAAAGTT TCCTCTTTGG AAAGAAGAAA TCTGAAAGAC CTATCATGCC 150

GTTCTTCCTG GCGTCTATAT TTCCTTTAGA GAGGCAAGGT AGGATTCCGT 200

CTCCAAGGGA G 211

(2) INFORMATION FOR SEQ ID :366:

(i) SEQUENCE CHARACTERISTICS:

(A) LENGTH: 285 base pairs

(B) TYPE: nucleic acid

(C) STRANDEDNESS: double

(D) TOPOLOGY: linear

(xi) SEQUENCE DESCRIPTION: SEQ ID :366:

CGGCTACAGC ATCACATCCA CTAAATGCAC AGTTGTTGAA GTCACGCCCT 50

GCCAGACCTC TACTACCACA TTGTTCCCTC CGGTGACCAG TTTGTCCGTC 100

ACTGTCAGGA GAATGNCCCG TGTCGAATCA TCGATCTCCA TCCGAGCCAA 150

AAGTTTTCCA TATGCTGAAA TTACTGAATT GCCCTCGTTT ATAGTATATG 200

ATACAGTGGC CTTCGTTGGA GGCAGATATC AAGCATGGGG ATAAGCAGTT 250

TTCTGGTCCT TAAGGGCTCT CAGCAAATCT CCGTG 285

(2) INFORMATION FOR SEQ ID :367:

(i) SEQUENCE CHARACTERISTICS:

(A) LENGTH: 149 base pairs

(B) TYPE: nucleic acid

(C) STRANDEDNESS: double

(D) TOPOLOGY: linear

(xi) SEQUENCE DESCRIPTION: SEQ ID :367:

GACACTGAGG CAGCTACTGA GAAGGGCAGA ACATGTCTCA ATCCATAGAG 50

GTCTTGGCAG GCGAACCCAG AGAGACTTGC AGTACGTGAG AAGATGGAGA 100

ACCAAATAAA AGGACTGGAG TCCAGTTCAA ACAAGGAGGG AGTGGTAGT 149

(2) INFORMATION FOR SEQ ID :368:

(i) SEQUENCE CHARACTERISTICS:
(A) LENGTH: 137 base pairs
(B) TYPE: nucleic acid
(C) STRANDEDNESS: double
(D) TOPOLOGY: linear

(xi) SEQUENCE DESCRIPTION: SEQ ID :368:

GGTTCCCGCT CTCACAGCCA TTGCAGTACA TGAGCTCCAT AGAGACAGCG 50

CCGGGGCAAG TGAGAGCCGG ACGAGCACTG GCGACTTGTG CCTCGCTGAG 100

GCAATACTAT AATANGCAAG GAACTTTTGA GGCCGAG 137

(2) INFORMATION FOR SEQ ID :369:

(i) SEQUENCE CHARACTERISTICS:
(A) LENGTH: 192 base pairs
(B) TYPE: nucleic acid
(C) STRANDEDNESS: double
(D) TOPOLOGY: linear

(xi) SEQUENCE DESCRIPTION: SEQ ID :369:

GCCATCATAA GTTCAAAAGA AAAGAGAAAG TGTTATTTTT CTGTTAGTGA 50

CATGTAGTCC CTTTGTTCTA GTAGGAAAAA AGGTGCCTAG AGGTAGTATA 100

TAGAGTAAAT ATTGTTCCTT TGCCTACTCG TGCTTCCAAT GATTAAGGAA 150

ATGTTAAACA NNNGTNAAAG TCTGTTTTGT CAATGCGGGA GT 192

(2) INFORMATION FOR SEQ ID :370:

(i) SEQUENCE CHARACTERISTICS:
(A) LENGTH: 197 base pairs
(B) TYPE: nucleic acid
(C) STRANDEDNESS: double
(D) TOPOLOGY: linear

(xi) SEQUENCE DESCRIPTION: SEQ ID :370:

GAGACTTAAG TAAAACGGCT GCAGGCCAGA TTCGCCCACC AGCTCAGAGA 50

CCACCTTTAT CCATGCTTTG AAGTAGGACT CCTTCCGTCT TCAAAATNTT 100

GAAGACCCTA ACANGCTTTT ATGATGGGGG TCATATCTAT GGTCACGNAT 150

ATAGTAGAAA CCAAAAGAAT GTAAGTATTT GTNNATGATT TAAAAAT 197

(2) INFORMATION FOR SEQ ID :371:

(i) SEQUENCE CHARACTERISTICS:
(A) LENGTH: 114 base pairs
(B) TYPE: nucleic acid
(C) STRANDEDNESS: double
(D) TOPOLOGY: linear

(xi) SEQUENCE DESCRIPTION: SEQ ID :371:

GCATCTGNCA GCTGCTGCAG GCTGACAGCC TCCAGACCTG GACAGAATTC 50

ATACACTCCC GGAGCTGAAA AAACCGAACA TAAGGGAGTG CACAAGAGCC 100

GGGCTTTGGA GAGG 114

(2) INFORMATION FOR SEQ ID :372:

(i) SEQUENCE CHARACTERISTICS:

(A) LENGTH: 127 base pairs

(B) TYPE: nucleic acid

(C) STRANDEDNESS: double

(D) TOPOLOGY: linear

(xi) SEQUENCE DESCRIPTION: SEQ ID :372:

CTTTCTCTAC TCCATCCATT ATCTCCGGGA TTCCTCCACA TTCCCTTCAG 50

CTCCTAGTTG TTCCACCTTG GCTAACAATT TACCAATCTT TTTTTCTATG 100

AACACGTTCT GCCTTTGCTG CTACTTC 127

(2) INFORMATION FOR SEQ ID :373:

(i) SEQUENCE CHARACTERISTICS:

(A) LENGTH: 257 base pairs

(B) TYPE: nucleic acid

(C) STRANDEDNESS: double

(D) TOPOLOGY: linear

(xi) SEQUENCE DESCRIPTION: SEQ ID :373:

AAGCGCTCAA TTCGCATCCA CAATGCAACC ACGAACTGAT TTTCTCTTTC 50

TTTTTCAGTT CTCCTTGGTC TGTAACAGGA ATGCCCCTTA CTCAGTAGCA 100

GGCGGACACG GCCATGGGTC AAGACACCCT GCTTCATGGG GAAACCTTGT 150

TTGTCGTTCC CACCACTGAT TCGGACCACA TAACCTTATN NNATCCCGAG 200

CGTTAGCAGC AACTTTTGTG GCCATACGCT TCTCATAGAA AGTACGAAGT 250

TTGGCAT 257

(2) INFORMATION FOR SEQ ID :374:

(i) SEQUENCE CHARACTERISTICS:
(A) LENGTH: 180 base pairs
(B) TYPE: nucleic acid
(C) STRANDEDNESS: double
(D) TOPOLOGY: linear

(xi) SEQUENCE DESCRIPTION: SEQ ID :374:

GCATCATCAT TGACATCCCT ATCAATGTCT ACGCTGCTGG CAGAGGACTG 50

NCCATTTGGA GCTGAGATGT GTAAGCTGGT GCCTTTAATA CAGAAAGCCT 100

CCGTGGGAAT CACTGTCTGA GTCTTGTGTC TAGTATTGAC AGATATAGCT 150

TTGTTCTTGG AGTAGAATTA AGGAATTGCG 180

(2) INFORMATION FOR SEQ ID :375:

(i) SEQUENCE CHARACTERISTICS:
(A) LENGTH: 323 base pairs
(B) TYPE: nucleic acid
(C) STRANDEDNESS: double
(D) TOPOLOGY: linear

(xi) SEQUENCE DESCRIPTION: SEQ ID :375:

GGACCATTCT GATCATCCTC ACTGGACGCC ACAGGGGCAA GAGGGTGGTT 50

TTCCTGAAGC AGCTGGCTAG TGGCTTATTA CTGTGACTGG ACCTCTGGNC 100

TCAATCGAGT TCCTCTACGA AGAACACACC AGAAATTTGT CATTGCCACT 150

TCAACCAAAA TNGATATCAG CAATGTAAAA ATCCCAAAAC ATCTTACTGA 200

TGCTTACTTC AAGAAGAAGA AGCTGCGGAA GCCCAGACAC CAGGAAGGTG 250

AGATCTTCGA CACAGAAAAA GAGAAATATG AGATTACGGA GCAGCCAAGA 300

TTGATCAGAA AGCTGTGGAC TTA 323

(2) INFORMATION FOR SEQ ID :376:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :376:

GGGGAATCCC ACCTCACCCA TTGNGGTGAA ATGCTTTTTT CTAAGAGGTG 50

AAATCACTCG CTGGTGTTTA TTTCGGCACA ACCAGAAAAT AGTGNGGGAT 100

ATTGAATTAT GGGAGGCTCT GACTGTCTCG NGTGCAGCTT ACATTCACAG 150

ATGGGGGGTA GTTTTTATAT TCTATAAGCG AGCATATTAA ANGCAATATG 200

AGTCAGTCCT GCATTTATGT CTT 223

(2) INFORMATION FOR SEQ ID :377:

(i) SEQUENCE CHARACTERISTICS:
(A) LENGTH: 122 base pairs
(B) TYPE: nucleic acid
(C) STRANDEDNESS: double
(D) TOPOLOGY: linear

(xi) SEQUENCE DESCRIPTION: SEQ ID :377:

GGCTCAGGAT CCGGCATCCT TTATGCCTCC ACAGCACCTT GCTCTTTCCC 50

CAGCCAATCA CTTTAGATGC TGAATCGATT TTAAACANAT GTTTTGTCAT 100

ATGGCTAATC AAGAGCCAGG TA 122

(2) INFORMATION FOR SEQ ID :378:

(i) SEQUENCE CHARACTERISTICS:
(A) LENGTH: 195 base pairs
(B) TYPE: nucleic acid
(C) STRANDEDNESS: double

(D) TOPOLOGY: linear

(xi) SEQUENCE DESCRIPTION: SEQ ID :378:

GGCAGTGCGT GCTTCATCCC TTTATGAAGA ACAGAAAATT ATGACTACTC 50

TACAAGGTGG ATAATACTTC GGTACCTTGC TTGCCACAGC CCTGTTCCTC 100

AAAGCTGAAT TGATAAATTT CTCTTTGACT TCCAAGACCT AGCAGTTATA 150

AGGCGCCTTG AATAAAGTTT GTGCCTGAAA ATGTGGAGCA ATGCT 195

(2) INFORMATION FOR SEQ ID :379:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :379:

GTAGCCCTGA GGTCATCCTG CAAAGNGCGT ATCAAAAAAN ACGAAGTTAG 50

GGTGACAAAG TTTGACAGTG ATGTTTACAA GTCAAACTTG GAAGGTTATA 100

GTAAGCATAC CTANGCTGAG AGAAAAGCAT CAAANCTNNG GNACATANTN 150

GGTTTNTNGN AACAAAGCAA CTTGTAATTT AAGNTTAAAC NGAGCATCAT 200

ATANNNNNGG 210

(2) INFORMATION FOR SEQ ID :380:

(i) SEQUENCE CHARACTERISTICS:
(A) LENGTH: 238 base pairs
(B) TYPE: nucleic acid
(C) STRANDEDNESS: double
(D) TOPOLOGY: linear

(xi) SEQUENCE DESCRIPTION: SEQ ID :380:

CTGGCTCACC TGGACAACCT CAAGGGCACC TTTGCCACAC TGAGTGAGCT 50

GCACTGTGAC AAGCTGCACG TGGATCCTGA GAACTTCAGG CTCCTGGGCA 100

ACGTGCTGGT CTGTGTGCTG GCCCATCACT TTGGCAAAGA ATTCACCCCA 150

CCGTGCAGGC TGCCTATCAG AAAGTGGTGG CTGGTGTGGC TAATGCCCTG 200

GCCCACAAGT ATCACTAAGC TCGCTTTCTT GCTGTTCA 238

(2) INFORMATION FOR SEQ ID :381:

(i) SEQUENCE CHARACTERISTICS:
(A) LENGTH: 275 base pairs
(B) TYPE: nucleic acid
(C) STRANDEDNESS: double
(D) TOPOLOGY: linear

(xi) SEQUENCE DESCRIPTION: SEQ ID :381:

GTCCTATGGC ATGAATGTTT GCAACCAACC CAGGAAAAGC CTTAAAGGAA 50

ATAGCTGTTC ACATAGGAGA CCGTGACAAT GCTGTACGCA ATGCTGCACT 100

CAACACCAAG CAACGATGCA CAATGTACAT GAGGATCAGG TGTTCAAACT 150

GATTGGAAAT CTTTTTGAAA AGGATATGAG CATGCTCGAG GAGAGGATTA 200

AGCGTCAGCA AAGAGACCCT CTCTGCACCA ATAAAACAGG TGGAAGAAAA 250

CCTAGCNCGC ACAGAACATA AGCTC 275

(2) INFORMATION FOR SEQ ID :382:

(i) SEQUENCE CHARACTERISTICS:
(A) LENGTH: 96 base pairs
(B) TYPE: nucleic acid
(C) STRANDEDNESS: double
(D) TOPOLOGY: linear

(xi) SEQUENCE DESCRIPTION: SEQ ID :382:

AAGCAGAGTA CGCACGTCAG GCCCTTCCAC GCCCACCCCA ACACTTTAAA 50

CNTACTGGGC GATGGGGCCG TTTNGCTGGC AGTTCAAGAT AAAACA 96

(2) INFORMATION FOR SEQ ID :383:

(i) SEQUENCE CHARACTERISTICS:
(A) LENGTH: 196 base pairs
(B) TYPE: nucleic acid
(C) STRANDEDNESS: double
(D) TOPOLOGY: linear

(xi) SEQUENCE DESCRIPTION: SEQ ID :383:

GGACTTGATG GCTTCTTTCA AAGTCATAGA CTTGTGGTAA ACTTCTTGCA 50

AGGAGCTCTG GGCACCCTCT GAAGCAGAGC CAATTGCTCG AGCATCACAC 100

TGTACAAAGG TCCAGATGGG TTCATATGAA ACAGCTGGAG TCCTTTTCAT 150

CAATNCNAAT NGANNGGTTA CTCCAAAGGG ACGAGACATG CACCTG 196

(2) INFORMATION FOR SEQ ID :384:

(i) SEQUENCE CHARACTERISTICS:
(A) LENGTH: 206 base pairs
(B) TYPE: nucleic acid
(C) STRANDEDNESS: double
(D) TOPOLOGY: linear

(xi) SEQUENCE DESCRIPTION: SEQ ID :384:

AAGGAGGCCT TTGAGTCCCA TCCTCTGCCA CGCAGAGAGG CAGGCCTGGC 50

ATTTCAGCCT CACTTTGTGT AGGTCACTTT TCGGTCTGTG TTGTAGCTCA 100

CCAACCAACA GTCAAACCCC AGCGTGGTAG CACGAGGTCC AGAACCTCTG 150

CTTAGTGGGC CCGTATAATA AACGCAACCT GACCCAATTN NGGTTTCTTT 200

CCCCAT 206

(2) INFORMATION FOR SEQ ID :385:

(i) SEQUENCE CHARACTERISTICS:
(A) LENGTH: 134 base pairs
(B) TYPE: nucleic acid
(C) STRANDEDNESS: double
(D) TOPOLOGY: linear

(xi) SEQUENCE DESCRIPTION: SEQ ID :385:

GAATGCTTGG GATCATCCAG GTGNCTCTAT TTATAGGCAC CTGTGTTCAC 50

CAGCAGTCTC TCTATTAGAA TGCTTGGTGT GCCAATGTTT CTTGAATTAG 100

AAGCCTGGGG NCACCTAGGT GTTTCTATTA TAAA 134

(2) INFORMATION FOR SEQ ID :386:

(i) SEQUENCE CHARACTERISTICS:
(A) LENGTH: 161 base pairs
(B) TYPE: nucleic acid
(C) STRANDEDNESS: double
(D) TOPOLOGY: linear

(xi) SEQUENCE DESCRIPTION: SEQ ID :386:

CAAGGCCTTC CTGCACTGGT ACACGGCGAG GGCATGGACG AGAGGAGTTC 50

ACCGAGGCCG AGAGCAACAT GAACGACCTG GTGTCCGAGT ACCAGCAGTA 100

CCAGGACGCC ACGCCGACGA ACAAGGGGAG TTCNGAGGAG GAGGAGGCGA 150

GGACGAGGCG T                                                    161

(2) INFORMATION FOR SEQ ID :387:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :387:

GGTGAGCCTC CGCCATCCAG CATGACAAAC TGTGGCCGAC CGCAGCTGTG          50

CCGTGGCACG ATGGCGAGGA AGCCAGCCCC AAGGAAACAC TGAAAACACA          100

ACCAGTCAAT GCCGTGTGGT TTTGTTTGAA TATAAATNGC TGAAAGTGTT          150

GTTTTTTNAG GCAGTAATNT                                           170

(2) INFORMATION FOR SEQ ID :388:

(i) SEQUENCE CHARACTERISTICS:
(A) LENGTH: 250 base pairs
(B) TYPE: nucleic acid
(C) STRANDEDNESS: double
(D) TOPOLOGY: linear

(xi) SEQUENCE DESCRIPTION: SEQ ID :388:

TCCATCTGAC ATCGCATTTC CATAGAAATG GCCAAAGAAA GAAGGTCCTG 50

GGGTTTTTCA TAGAAAGCTC AAAAAGTTCA ACCTTTGATG CTATCCCCCA 100

GCCCAATACA AAATACACAG AAAAAGCAAT TATTAAAAAC GGCTTCGGTT 150

TCTTTTTTCT CTTTAATNNC CTACAATNGC TTTACATATT CGTGTGCAGC 200

ACCTACTTCT TTATGCCGTG AACTGAAATC TAAGATTTCA AACTGAAATC 250

(2) INFORMATION FOR SEQ ID :389:

(i) SEQUENCE CHARACTERISTICS:
(A) LENGTH: 268 base pairs
(B) TYPE: nucleic acid
(C) STRANDEDNESS: double
(D) TOPOLOGY: linear

(xi) SEQUENCE DESCRIPTION: SEQ ID :389:

GCCATCTCCC CGCCACCCNC TCGTCCCCTT CTTNTCNNGC CACCCTTCCT 50

CNCTCNTCCG TTTTNGCTCC ACTCCACTCC TCCNTTTTNA GTACCCTCCT 100

CNTTTATNTA GAGTTACTGA GAGCCGACCT GACGTCTCCA ACATNCCGTN 150

TCTTATATCT CATCNCGGTT NTNGANGAAT GNAGTNAGGG TTTCCGGGAG 200

AGACCNAACT TGCTCTAGCC CTTTCCAGCC GCTGTTGTTA AACTGACCTC 250

GTAGGGCNTG AGGGAGGT 268

(2) INFORMATION FOR SEQ ID :390:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :390:

GTCAGAGGCA GCCCATCCAG CACGTGCTAG GTGTTCCCAT ACGCACAGGA 50

GAGGCGAGCT AGCCAGCCAA GGCGGGCAGG CGGGGAGGCC CTCTAGCTGT 100

TTGCCTCACC TGTGGGGCCC CAGCAGGGAG GAGTCACCAG CCTAGAGGGG 150

CCAGGTATAC ACCTTANAGA GGATGAC 177

(2) INFORMATION FOR SEQ ID :391:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :391:

GCTCCACTTC CCACATAANA CNAGACCTCT CACAAAAGAG TTCACTGATA 50

TGTGAATGCA GCTTTCTCCC CATGGTAGCC AGGACAGGGT GCACATTAAG 100

GCAACCCCAA AAACACCTCT TNCTGNCTGC CTCTGCAAA 139

(2) INFORMATION FOR SEQ ID :392:

(i) SEQUENCE CHARACTERISTICS:
(A) LENGTH: 259 base pairs
(B) TYPE: nucleic acid
(C) STRANDEDNESS: double
(D) TOPOLOGY: linear

(xi) SEQUENCE DESCRIPTION: SEQ ID :392:

GAGGAGCTGC CCTCAAGGTC GTGCGTCTGA AGCCTACAAG AAAGTTTGCC 50

TATCTGGGGC GCCTGGCTCA CGAGGTTGGC TGGAAGTACC AGGCAGTGAC 100

AGCCACCCTG GAGGAGAAGA GGAAAGAGAA AGCCAAGATC CACTACCGGA 150

AGAAGAAGCA GCTCATGAGG CTACGGAAAC AGGCCGAGAA GAACGCGGAG 200

AAGAAAATTG ACAAATAATA GAGGTCCTCA AGCCCACGGA CTCCTGGTCT 250

GAGCCCAAT 259

(2) INFORMATION FOR SEQ ID :393:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :393:

AAAGATCATC AGTTTGGAAG GTACTGGTCC AATTTATCTA GGAAGTATCT 50

CTTGGAGTTT CAGAAATGCT AGCTTGGACA ACTGAAAAGT CACATCACAG 100

CTGGCATTCT GGGGGCTACC AAAACACCCC TCNNGGAGTA GAAGCTGCTG 150

GAAGGCAGGC CTGAGCCATT CACCACGGAC AGGAAGAGC 189

(2) INFORMATION FOR SEQ ID :394:

(i) SEQUENCE CHARACTERISTICS:
(A) LENGTH: 97 base pairs
(B) TYPE: nucleic acid
(C) STRANDEDNESS: double
(D) TOPOLOGY: linear

(xi) SEQUENCE DESCRIPTION: SEQ ID :394:

AGAAGGGGAG GTCGAAGAAA TGTGGCTGAT CATAAGAAAG GAGCAAAGAA 50

AGCTAGAATT GAAGAANNNN GAAAGAAACA GAGGACAAAA AAACAGA 97

(2) INFORMATION FOR SEQ ID :395:

(i) SEQUENCE CHARACTERISTICS:
(A) LENGTH: 244 base pairs
(B) TYPE: nucleic acid
(C) STRANDEDNESS: double
(D) TOPOLOGY: linear

(xi) SEQUENCE DESCRIPTION: SEQ ID :395:

AAGAGGGGCT AAGACCCAAG TCATCCTCCA CTTTCGTTTT GTTATATTTC 50

GCTTAGAATT GCCTCTTCTT CTCCACTTCA GAACTGCCTC AAAATTGACC 100

CCCTTGACTG ATTTATTGTC GTCAAAAGCA TGTTCCTCTA TCTTTTNNNN 150

TCAGACGATC CGCTGCCTTT CTACATCTGA GAATCTTGTC AAGCATGGAT 200

AAACTTGNTT TTATGTTGCA TATTTTNACG GCTTCAACTT GAGT 244

(2) INFORMATION FOR SEQ ID :396:

(i) SEQUENCE CHARACTERISTICS:
(A) LENGTH: 185 base pairs
(B) TYPE: nucleic acid
(C) STRANDEDNESS: double
(D) TOPOLOGY: linear

(xi) SEQUENCE DESCRIPTION: SEQ ID :396:

TAATATCGCC ATGTAATGGT ATACATTGTA AGGCTTATGT CACTAAAGAT 50

TTTTATTCTG ATCTTTCCAT AATAAAGGTC ATATGATACT GTATAGACAA 100

GCTTTGCAGT GAAGTATAGT AGCAATAATT TTCGTACCTG ATCAAGTTTA 150

TTGCAGCCTT TCTTTTCCGT TTCTTTTNTG AAGGG 185

(2) INFORMATION FOR SEQ ID :397:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :397:

GGACACATCC ACCCAGTGAA CTGGACTGTG GCCATGACCC AGGGTATCAG 50

CTCCAAACTC TGCCAGGGCT GAGAGACACA TGAAGGAAGA TGATGGGAGG 100

AAAAGCCCAG GAGAAGTCCA CCAGGGACCA GCCCAGCCTG TATACTTGCC 150

ACTTACCACC AGGACTCCTT GNTCTGCTCT GGCAAGAGAC TCTTGTCTGA 200

ACACTGCTTA TCTGACCC 218

(2) INFORMATION FOR SEQ ID :398:

(i) SEQUENCE CHARACTERISTICS:
(A) LENGTH: 250 base pairs
(B) TYPE: nucleic acid
(C) STRANDEDNESS: double
(D) TOPOLOGY: linear

(xi) SEQUENCE DESCRIPTION: SEQ ID :398:

GGAAGCAAAA AAACAAAACT AAGCTCGAAT TTGCTTCCAA ACCTGTAGAT 50

TGCATCTAAC CANGTGTCCC TATGCACCTC AGAGTACTGG AATACGAACC 100

CAGCGAGACC TTGTCCCCTC CCATTTTGCT GNACTTTTGT TGGTGAAAAT 150

GAGAATGAGT TNATCCCTAC NTACTTAGTT TAATGCATTT GACCCCAGAA 200

AACCCCAGTA CCTTTNNACA ATGACCCAAC CANTACCTAC CATCGGCCAG 250

(2) INFORMATION FOR SEQ ID :399:

(i) SEQUENCE CHARACTERISTICS:

(A) LENGTH: 278 base pairs

(B) TYPE: nucleic acid

(C) STRANDEDNESS: double

(D) TOPOLOGY: linear

(xi) SEQUENCE DESCRIPTION: SEQ ID :399:

TCACTTGATT CATCCGTCGC GGGATTTGGG AGCACTAGCA ACATAATCAA 50

CACACTCCTA CAATCTTAGG CTTCACATGT GCTGATGATG ATGAAACCAA 100

CTCTGCCCCA ATCATCTCCC CTTCTNTTAG GGTCTTACTA CATATCGCAA 150

CAGAAGATAA TATTGAGGTG AAGAGGGTAA CATGAAGTTT GGCACTACCC 200

TGAAGAACTG TAGGCATCTC TTGGAATGTG CTAAGGAACT TGATGTCCAA 250

ATAATGAGAT TAAATTTATG TTTCGAGT 278

(2) INFORMATION FOR SEQ ID :400:

(i) SEQUENCE CHARACTERISTICS:

(A) LENGTH: 207 base pairs
(B) TYPE: nucleic acid
(C) STRANDEDNESS: double
(D) TOPOLOGY: linear

(xi) SEQUENCE DESCRIPTION: SEQ ID :400:

GGCCAAACTC ACAGGAGCCT AGGCACAGAG CACTGAACAC TGGCTCTGCA 50

GCGGGAAGGA ATTAGAGCCT TCTGCTTTTG CACCTGCTTT GAGTTAGGAA 100

GCAAGCTCTC TTCCTTGCCA GACTTCCCTT TGGGGCAGGA CACTTTTTTA 150

TACNCTGAGG CAAGGCAAAC AGTCATAGAA CAGTTATTAA ACAATANAAA 200

ATGTGTT 207

(2) INFORMATION FOR SEQ ID :401:

(i) SEQUENCE CHARACTERISTICS:
(A) LENGTH: 217 base pairs
(B) TYPE: nucleic acid
(C) STRANDEDNESS: double
(D) TOPOLOGY: linear

(xi) SEQUENCE DESCRIPTION: SEQ ID :401:

GAGGCATGGC CACATTCCAA GAAATGGAAT AAGGAGTGGG ACAGCAGGCA 50

GGGGGCCAGC CTGGGGCCTG GGCCTTTTAA TCTAAGGACT GGGGAGAACC 100

AAGGGACCTT AGAGGTCCTC CAGTCCTCCC CATATNNAAA GAGGGAGAAC 150

AAGCCTGGAG AGGAGGCTAC CTTAAGACCC TAAGAAGAAT TTAGCAATCG 200

NTTCTCCAAA GATAGCC 217

(2) INFORMATION FOR SEQ ID :402:

(i) SEQUENCE CHARACTERISTICS:
(A) LENGTH: 225 base pairs
(B) TYPE: nucleic acid
(C) STRANDEDNESS: double
(D) TOPOLOGY: linear

(xi) SEQUENCE DESCRIPTION: SEQ ID :402:

TTTACAATAG ATTTCACCTT CANTTTAGTC AGAGTTGTTG ATTCAAGACT 50

CTTCCCACAC TCGCACATCG AAACATTTTT GTGCCAGGGC TTTCCAGCTC 100

CAATTATCTT CTCGACAGCA TATACAGAAT CCCCACATCT GGAACACTTC 150

TCAGCACCTC CATANNTTGA GCAAATTTAG AAGTGTTTGG ATTTGTTGTA 200

GGCCTGTGAG GCTGAACACT CTCTG 225

(2) INFORMATION FOR SEQ ID :403:

(i) SEQUENCE CHARACTERISTICS:
(A) LENGTH: 95 base pairs
(B) TYPE: nucleic acid
(C) STRANDEDNESS: double
(D) TOPOLOGY: linear

(xi) SEQUENCE DESCRIPTION: SEQ ID :403:

AGAAGGGGCA TACTCGCTGG AGACCTGGTG CCTCTATCCA CTGNACGCTG 50

ACTGNGGCTT CCACTGCGCC CGTGTACNTT ATCGAAGCTA CAGAT 95

(2) INFORMATION FOR SEQ ID :404:

(i) SEQUENCE CHARACTERISTICS:
(A) LENGTH: 239 base pairs
(B) TYPE: nucleic acid
(C) STRANDEDNESS: double
(D) TOPOLOGY: linear

(xi) SEQUENCE DESCRIPTION: SEQ ID :404:

AGGAAAAATT GACATTACTT GTTAGAAGAG CAACTCTGAA AGAAAACAAG 50

CAAATTCCAA AACAGAAAAG ATTCACAATN NAAAGGTGCA TACATTAAAG 100

GGCCACACTG TGTAATACTG TGCCAACTTA TGCGAGTCTC ATTGTTCAGG 150

ATGAAATGTG AGATTGTAGT TTGAATGCTA TAAGCAGGTT CCAAGATACC 200

CCAAATGACT GTAAGCCAGA CTNGAAGCAT GTCAAAAAG 239

(2) INFORMATION FOR SEQ ID :405:

(i) SEQUENCE CHARACTERISTICS:
(A) LENGTH: 189 base pairs

(B) TYPE: nucleic acid
(C) STRANDEDNESS: double
(D) TOPOLOGY: linear

(xi) SEQUENCE DESCRIPTION: SEQ ID :405:

GGCTATTATT CTAAGTGAAG TAACTCAGGA ATGGAAAACC AAACGTTGTA 50

TGTTCTCACT GATCATTGAG AGCTAAGCTA TGAGGACTCA AAGGCATAAG 100

AATGATACAA TAGACTTTGG GACTTGGGAG GAAGAGCGGG AGTGGGGCGA 150

GGGATAAAAG ACTACAAATA TGTGCAGTGT TTACTGCTC 189

(2) INFORMATION FOR SEQ ID :406:

(i) SEQUENCE CHARACTERISTICS:
(A) LENGTH: 75 base pairs
(B) TYPE: nucleic acid
(C) STRANDEDNESS: double
(D) TOPOLOGY: linear

(xi) SEQUENCE DESCRIPTION: SEQ ID :406:

CACCATCCTC CAAGTAAATC CCCCCTTAGG AAAGTAAGGG AAAAGACCCC 50

TTATAGCCCT GAGCTCCCCC TTGGA 75

(2) INFORMATION FOR SEQ ID :407:

(i) SEQUENCE CHARACTERISTICS:

(A) LENGTH: 190 base pairs
(B) TYPE: nucleic acid
(C) STRANDEDNESS: double
(D) TOPOLOGY: linear

(xi) SEQUENCE DESCRIPTION: SEQ ID :407:

TATTCATCCA GCAGTGTTGC TCAGCTCCTA CCTCTGTGCC AGGGCAGCAT 50

TTTCATATCT AAGATCAATT CCCTCTTTTA GCACAGCCTG GGGAGGGNGT 100

CATTGTTCTC CTCGTCCATC AGATCTCAGA GGCTCAGAGA CTGCAAGCTG 150

CTTGCCCAAG TCACACAGCT AGTGAAGCCA GAGCAGTTTC 190

(2) INFORMATION FOR SEQ ID :408:

(i) SEQUENCE CHARACTERISTICS:
(A) LENGTH: 194 base pairs
(B) TYPE: nucleic acid
(C) STRANDEDNESS: double
(D) TOPOLOGY: linear

(xi) SEQUENCE DESCRIPTION: SEQ ID :408:

GAAGACATGG CGCCCTAACA CTTCGAGACC TGCTGNTAAA TTAAAAGCTA 50

TTTTTCATTA AACCACCATT TCCTCCACCT ATTGGAGTCA AATATGAAAG 100

CTGTCGATGA AGCCTGNCTG GCTGCACAAG TTNGACTGNG TCTGAATAAG 150

CACTTTCATC ATGGACTAAG AATCCTTGGT GTGGNCNTGA TCTT 194

(2) INFORMATION FOR SEQ ID :409:

(i) SEQUENCE CHARACTERISTICS:

(A) LENGTH: 97 base pairs

(B) TYPE: nucleic acid

(C) STRANDEDNESS: double

(D) TOPOLOGY: linear

(xi) SEQUENCE DESCRIPTION: SEQ ID :409:

GCAGCACAAG AGTCATCCAT GGATACAGTT CCAATCATTA TAACCACTTC 50

TAAAACAAGA TCATCTTCTG CAGCACCTGG TTTTAGTTTA TCCTTGA 97

(2) INFORMATION FOR SEQ ID :410:

(i) SEQUENCE CHARACTERISTICS:

(A) LENGTH: 155 base pairs

(B) TYPE: nucleic acid

(C) STRANDEDNESS: double

(D) TOPOLOGY: linear

(xi) SEQUENCE DESCRIPTION: SEQ ID :410:

TTTGAGCTCC TGGACCCTTC TTTGCCTACA CTGGCCTTCC TCTCGGAGGG 50

ACAAGGAAGC TGGCCTCCCT TTACTCTACC NTTNNTTNTG GTCCAGGGCC 100

AGCTCTTCCG AGGCTCCAGC CTGCTTTTCG CCGGTGTCAT CAGATCATGC 150

TTTGC 155

(2) INFORMATION FOR SEQ ID :411:

(i) SEQUENCE CHARACTERISTICS:
(A) LENGTH: 249 base pairs
(B) TYPE: nucleic acid
(C) STRANDEDNESS: double
(D) TOPOLOGY: linear

(xi) SEQUENCE DESCRIPTION: SEQ ID :411:

AACGAAGNGT TCCTGAATAC CACAGCCTGC TGGGAAATGA CTAGGCGGTA 50

AGGTTCTGCC ATGCCTGTGA CCCACCATGG ACATACTGGA CCTTAATTCC 100

TCTGCTTCTG TGCTCCCTCC TGAGAACCCT TTATGAGCCT GATTCCTCAG 150

TCTCACCAGA ATTCTGGATC ACCCAAGAGG AAAAGACTGC CAGTTCTAGA 200

TTCCTCTATA GGGAGACCTG GATTGTTGAC CAGGTGAGAA GCCAATGGT 249

(2) INFORMATION FOR SEQ ID :412:

(i) SEQUENCE CHARACTERISTICS:
(A) LENGTH: 143 base pairs
(B) TYPE: nucleic acid
(C) STRANDEDNESS: double
(D) TOPOLOGY: linear

(xi) SEQUENCE DESCRIPTION: SEQ ID :412:

AGAAGGAGAC AAAACACCTC CTCTGTAATA GTACCTCGAA TGGATTCAGC 50

TTTACTCCTC TATAACTCAT CTTCACACCN GCATATTTAA ACAAACTAAC 100

AAAATGAAAT ACTAATAGTA AAAAGGCTGA CCCATGTGGC TTT 143

(2) INFORMATION FOR SEQ ID :413:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :413:

GGGTCCCCCC ATTCACGTAC TCCATCACAA AGTACAGGCG GTCCATGGTC 50

TGGAAGCAGG AGTGGAGCTG TCAGGAAGGG CGGCTTCCCG GGCAGGCCAA 100

CACCCACTTC TCCACCATAG TGCACTCCAC GTCATCATCT GATCACAACG 150

TCCTTCTTCA GGATCTTCAC AGCATAGAGC TCATCTGTGC CTTTCGTTCT 200

GAAAGCATGA CCTTGCCAAA GCTGCCTTTC CCCAGCACCA TTAGGAAGTT 250

AAAATC 256

(2) INFORMATION FOR SEQ ID :414:

(i) SEQUENCE CHARACTERISTICS:

(A) LENGTH: 87 base pairs
(B) TYPE: nucleic acid
(C) STRANDEDNESS: double
(D) TOPOLOGY: linear

(xi) SEQUENCE DESCRIPTION: SEQ ID :414:

TCGCAAAAGA AGNGTACCAT AACTCCTATT TCNAATGGCT GATTGTAGTA 50

GCACTGGAAG TTTATCAAAA CCCAGGTGTA AATATGC 87

(2) INFORMATION FOR SEQ ID :415:

(i) SEQUENCE CHARACTERISTICS:
(A) LENGTH: 247 base pairs
(B) TYPE: nucleic acid
(C) STRANDEDNESS: double
(D) TOPOLOGY: linear

(xi) SEQUENCE DESCRIPTION: SEQ ID :415:

AGAGGGGGAC CAATCTAACC ATCGCTCTTC CAGTCCTACC AGCACACTCT 50

TGCAGCGCCA TNCCACTCCC TGAGCTCAGG ATCCTAGCCC GCCCGCCCGC 100

GGATGCTAGT NNNCTCCACC CTNCCCACCT GTCATACCTG TGTANTACAC 150

ACCCACCCGT ATCACGCAGG ACATGAACCA GCACCACTAA GGCCCCGGNG 200

CAGTGTTCTG NNNTTCTTCT CTANCTGTCT GCCCTGAGTC CCGGACC 247

(2) INFORMATION FOR SEQ ID :416:

(i) SEQUENCE CHARACTERISTICS:
(A) LENGTH: 162 base pairs
(B) TYPE: nucleic acid
(C) STRANDEDNESS: double
(D) TOPOLOGY: linear

(xi) SEQUENCE DESCRIPTION: SEQ ID :416:

TCTAGCACAC AGCTGCGCTC ACAAAAACTG CGCGACTTGT TAGAACTAAT 50

TGAGTGGAGC CTGCAGGTGA GGGGAGGGAG GGGCTGCAGG TCAGGTAAGA 100

TCTGGAAGAC AACGCACACT TGAAGGGCAG GGGACTCTAA GCAGGGATTT 150

ACATTGAAAG GA 162

(2) INFORMATION FOR SEQ ID :417:

(i) SEQUENCE CHARACTERISTICS:
(A) LENGTH: 109 base pairs
(B) TYPE: nucleic acid
(C) STRANDEDNESS: double
(D) TOPOLOGY: linear

(xi) SEQUENCE DESCRIPTION: SEQ ID :417:

CAGAAGAAGG AGAACTCATG ATTCCAGAGA GCTTTGGGGC TTATTTTTAA 50

GTACTTAGCA AAATATTTGT TTTNCGTGAT TTAGCTTGTC ATTAAACNAA 100

GAGCTACTG 109

(2) INFORMATION FOR SEQ ID :418:

(i) SEQUENCE CHARACTERISTICS:
(A) LENGTH: 202 base pairs
(B) TYPE: nucleic acid
(C) STRANDEDNESS: double
(D) TOPOLOGY: linear

(xi) SEQUENCE DESCRIPTION: SEQ ID :418:

GGATGTAAAT TATATGTTGT TTAAATTTTT CCAGCATCTG AAAACCTTAT 50

CTGCTAGACA ATGCAAGATT CACACAGAGT TATCTGGGAT TCTGATTTTT 100

TAAATAGTAC ATATCATTAA ACCATTCNTT CTAAANGTAA GAAGAGCAGA 150

AAAAATCTTA TAAGATTATC AGATTTTCCT AATGACACAG AAATGNAAGA 200

AA 202

(2) INFORMATION FOR SEQ ID :419:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :419:

TAGCAAATGT GGATTACAAG CAAATACAAC AAGTCGTGCC CCTCCAACCC 50

ACCACAAACT TCTAAACGCC ACTCAGCAGA TGCATAGGAT TCGTTACACG 100

CAACGATTGA AATAAAAATA AAAGCTNCAA ATGCAATACC CANACTAGCC 150

TA 152

(2) INFORMATION FOR SEQ ID :420:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :420:

CAGCCCTACA CTCGCCCGCG CCATGGCCTC TGTCTCCGAG CTCGCCTGCA 50

TCTACTCGGC CCTCATTCTG CACACGATGA GGTGACAGTC ACGAAGGATA 100

AGATCAATGC CCTCATTAAA GCAGCCGGTG TAAATGTTGA GCCTTTTTGG 150

CCTGGCTTGT TTGCAAAGGC CCTGGCCAAC GTCAACATTG GGAGCCTCAT 200

CTGCAATGTA GGGGCCGG 218

(2) INFORMATION FOR SEQ ID :421:

(i) SEQUENCE CHARACTERISTICS:

(A) LENGTH: 237 base pairs
(B) TYPE: nucleic acid
(C) STRANDEDNESS: double
(D) TOPOLOGY: linear

(xi) SEQUENCE DESCRIPTION: SEQ ID :421:

GATGCCAATG TTTCATCCAC CGGCTGCACA GGCACAAACT CCCCCACCCA 50

GGACGACTGT GATGAGGTGG CCCTCCCTGT CAACCCTGGT CCCTGGAGTC 100

CCCAGCACCT GGGGCCCTGA CGGTCTCGAT GTCACAGGCG CTTACTGTGC 150

TGCTGGCTGT CCTATGCCAG CCTCACCCAT GTGGGGACCA CGNAAGGCAC 200

ACTCCCTCAC CCCGGTGCCG GGCCGTGCGA TCCCCCA 237

(2) INFORMATION FOR SEQ ID :422:

(i) SEQUENCE CHARACTERISTICS:
(A) LENGTH: 202 base pairs
(B) TYPE: nucleic acid
(C) STRANDEDNESS: double
(D) TOPOLOGY: linear

(xi) SEQUENCE DESCRIPTION: SEQ ID :422:

AGGNGTAATA AATACTGCAT CCTTTCCACA ACATAGCAGG AATCTTATAG 50

GGAAATCCAT ATAGTTTCTG AACATGTATT NATATACTAA GTCTATATTG 100

TTTCTTTACG AAGTGTAAAT AAGTGCTGCA CCATACTGTA AACAAAACTC 150

GAATATTGAC TAAATAAAAT CAAAAGTTCA TCTTGTAGTC ATGTCTTTCT 200

CC 202

(2) INFORMATION FOR SEQ ID :423:

(i) SEQUENCE CHARACTERISTICS:
(A) LENGTH: 216 base pairs
(B) TYPE: nucleic acid
(C) STRANDEDNESS: double
(D) TOPOLOGY: linear

(xi) SEQUENCE DESCRIPTION: SEQ ID :423:

GGGACACCAT TACCATCCAT TGACATCGCA TTTCCATAGA AATGCCAAAG 50

AAAGAAGTCC TGGGGTTTTT CATAGAAAGC TCAAAAAGTT CAACCTTTGA 100

TGCTATCCCC CAGCCCAATA CAAAATACAC AAAAAAGCAA TTATCAAAAT 150

ACTGACTTCG GTTTCTTTTT CTCTTCAAAT NCCTATAATN GCTTTACATA 200

TTCGTATCAG CACCTA 216

(2) INFORMATION FOR SEQ ID :424:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :424:

CGACGAAGGG AGTACTCGCG CTTTGTCTCC CTATCCACAC TTGAGCAAAT 50

GCTCGCTGGT CTACTGCCAA AACCCATTTC ACCAACCATA TGTCCCTCGC 100

CCATACCCCC TCTCCCTCTC AAACCCTGGG GACAAATTCA AGGACCCAGG 150

GGTGCCCTTT TAAAAAAA 168

(2) INFORMATION FOR SEQ ID :425:

(i) SEQUENCE CHARACTERISTICS:
(A) LENGTH: 258 base pairs
(B) TYPE: nucleic acid
(C) STRANDEDNESS: double
(D) TOPOLOGY: linear

(xi) SEQUENCE DESCRIPTION: SEQ ID :425:

TCGCAGGAGA AGGAGTCGCT TACTATGCAT CCAAAGGCTA CCGTGGTTAC 50

TCCTATATGA CATTCCAGAT TTTGATGACT AGCACCCACC CCACAGCTGA 100

GAGGAGTCAC AGTGGAACTT CCCAGCTTTA AGATATCTAG CAGAAACTAT 150

AGCTGAGGAC TAAGGAATTC TGCAGCTTGC AGATGTTTAA GAAAATAATG 200

GCCAGATTTT TTGGTCCTTC CCAAAGATGT TAAGTGAACC TACAGTTAGC 250

TAATTAGG 258

(2) INFORMATION FOR SEQ ID :426:

(i) SEQUENCE CHARACTERISTICS:
(A) LENGTH: 275 base pairs
(B) TYPE: nucleic acid
(C) STRANDEDNESS: double
(D) TOPOLOGY: linear

(xi) SEQUENCE DESCRIPTION: SEQ ID :426:

TCCACATCAT CTCCAAAGCC CATAGGCCGA GAAATGGCAT CTGAGATCTG 50

CTGGGCCACC TCCTGTTGTT CCGTATGTCA GTCATCAGTT CATCTACCTN 100

GTCAATGTCC ATGTCCTGGT AGGCCTTCTT CATGCTTTGN CAGCAAGCTC 150

CATGCACGAA GGACTTCTGC ATTGGTAGTG GCATTCTCAA TAGCCTCACG 200

CTGAAACTCC AGGGTGGATA ATGCCCCGTC AATTTGTGCC AGCTGCTGTT 250

CGAATNNTTT TCTTTCTCGT AAAGC 275

(2) INFORMATION FOR SEQ ID :427:

(i) SEQUENCE CHARACTERISTICS:
(A) LENGTH: 162 base pairs
(B) TYPE: nucleic acid
(C) STRANDEDNESS: double
(D) TOPOLOGY: linear

(xi) SEQUENCE DESCRIPTION: SEQ ID :427:

CTCTGTGCCT CGCTGAGGAA AAATAACTAA AATGGCAAAG AAGATCCTAA 50

GAAGCCGAGA GGCAAAATGT CATCATATGC ATTTTNTGGC AAACTGTCGG 100

GAGGAGCATA AGAAGAAGCA CCAGATGCTT AAGTCAACTN TAAGAGTTTT 150

CTAAGAGTGC TC 162

(2) INFORMATION FOR SEQ ID :428:

(i) SEQUENCE CHARACTERISTICS:
(A) LENGTH: 220 base pairs
(B) TYPE: nucleic acid
(C) STRANDEDNESS: double
(D) TOPOLOGY: linear

(xi) SEQUENCE DESCRIPTION: SEQ ID :428:

ATGCAGAAAA GGAGTCGTGA GCGTAACACC CTCGCTGCTG GTCATAAAAC 50

CCATAGTTCC TGAACTCATC AAAGAACTTA ACTTCCTGAC GCCAGATATC 100

TGCCATGCGC GAAGGGNTGA TACCACTCAG GATTATGAAT TTTGTTTAAA 150

GTCGNGTTTA CAATGATTTG NCCTGGACTG AAATTCANGC TGCCTTAAGG 200

TGCTGATGAT ATTGAGAAGT 220

(2) INFORMATION FOR SEQ ID :429:

(i) SEQUENCE CHARACTERISTICS:
(A) LENGTH: 185 base pairs
(B) TYPE: nucleic acid
(C) STRANDEDNESS: double
(D) TOPOLOGY: linear

(xi) SEQUENCE DESCRIPTION: SEQ ID :429:

GACTCATCTT GCCCAAGAAG AGCCAGGATA TGAGAAGATG GGAGAGGGAA 50

GGGGAGGTGT GAAACGTGCT GGTGCTTCCT GTCCTCATGG TGTGATGGCT 100

GCCCAGGCTG CTCCTTCGAG GCAGGACAGG ATTCCCATGG GAGATATGAG 150

GACCATGGTG ATTTCCAGGA CCAGTTATGT CCAGA 185

(2) INFORMATION FOR SEQ ID :430:

(i) SEQUENCE CHARACTERISTICS:
(A) LENGTH: 277 base pairs
(B) TYPE: nucleic acid
(C) STRANDEDNESS: double
(D) TOPOLOGY: linear

(xi) SEQUENCE DESCRIPTION: SEQ ID :430:

GGGATGCCCT GACCTGATGG CCCTTGACCA ACATGGTGCT GGAGGAGCAC 50

TACCTGGTCG TGGGAGTAGT GGTCATCGTG GACCCAGGGG TGATCCCTAT 100

CAACTCTTGG GGTGAGAAGC AGCGCATGCA CCTGCGGGAC GCTTCCTGGC 150

TGACCAGCTG GACCCCATCT AGTCGCCTAC AACAGCGAGC CAGCACACCG 200

GCCCAGGTGC CGGAGATGAA TGAGCCCCAG CAGTCCAAGG TGTGATGTGG 250

GAAGACACCG CAGAGCTCAC TTACCAA 277

(2) INFORMATION FOR SEQ ID :431:

(i) SEQUENCE CHARACTERISTICS:
(A) LENGTH: 125 base pairs
(B) TYPE: nucleic acid
(C) STRANDEDNESS: double
(D) TOPOLOGY: linear

(xi) SEQUENCE DESCRIPTION: SEQ ID :431:

AAAGTGCGCT TGGGCATCCT GCCAGCCTGC CATGTCTTCA TGCGCTGGTC 50

GAAGGCAGCC NGGACTATGG CCAGGAGGCG AATGTAGTCA CTCAGGAGCT 100

CAGCAAGGAG GAAGAAGTCA TTGTT 125

(2) INFORMATION FOR SEQ ID :432:

(i) SEQUENCE CHARACTERISTICS:
(A) LENGTH: 222 base pairs
(B) TYPE: nucleic acid
(C) STRANDEDNESS: double
(D) TOPOLOGY: linear

(xi) SEQUENCE DESCRIPTION: SEQ ID :432:

GCCTAACTGT CAAAGCACAG AAAAGTTAAC AAAGGGTGGG ACTCGCCATT 50

TCGAAATAGC ACATTTTTAG CAATAGGCTC TCTACACTAG AGAGCCAGTA 100

GACTGATATT CTTTAATGCC AGTTTCCTAG TTAATCGTAA AGATAGACAC 150

AATTCCCCCC TTTATAAAAG CTTCTGTCGT TTCACATAAT GACTTTAACT 200

ANAANGGAAA TGGGGCAGGA CA 222

(2) INFORMATION FOR SEQ ID :433:

(i) SEQUENCE CHARACTERISTICS:
(A) LENGTH: 213 base pairs
(B) TYPE: nucleic acid
(C) STRANDEDNESS: double
(D) TOPOLOGY: linear

(xi) SEQUENCE DESCRIPTION: SEQ ID :433:

CCATGNCGGC CCCCAGGTCC TCGATCTTTT CGAGCTGACT TCTCATGGCT 50

CGATTTCCTG GTGCTTGGTT TGGTAGCTTC AAGCTGGGAT TCGAGGGTCC 100

CCGACTNTTC CACCATACAG GACCCATCGA GGAGGCATGT TCTGGTCTNT 150

TGGATCCACT GGTGGAAGGG TTGCGCGCTG GNCAAACTGT TGGTNTAACT 200

NGNCGTTTCC TCT 213

(2) INFORMATION FOR SEQ ID :434:

(i) SEQUENCE CHARACTERISTICS:
(A) LENGTH: 221 base pairs
(B) TYPE: nucleic acid
(C) STRANDEDNESS: double
(D) TOPOLOGY: linear

(xi) SEQUENCE DESCRIPTION: SEQ ID :434:

CCATGNCGGC CCCCAGGTCC TCGATCTTTT CGAGCTGACT TCTCATGGCT 50

CGATTTCCTG GTGCTTGGTT TGGTAGCTTC AAGCTGGGAT TCGAGGGTCC 100

CCGACTNTTC CACCATACAG GACCCATCGA GGAGGCATGT TCTGGTCTNT 150

TGGATCCACT GGTGGAAGGG TTGCGCGCTG GNCAAACTGT TGGTNTAACT 200

NGNCGTTTCC TCTGCCGGTG T 221

(2) INFORMATION FOR SEQ ID :435:

(i) SEQUENCE CHARACTERISTICS:
(A) LENGTH: 70 base pairs
(B) TYPE: nucleic acid
(C) STRANDEDNESS: double
(D) TOPOLOGY: linear

(xi) SEQUENCE DESCRIPTION: SEQ ID :435:

ACACTTGATA CTATGCATCA AAGGACGTGG AGAACTAGAG CGGGCTACAT 50

TAGTATATTT TCGTTGTCAG 70

(2) INFORMATION FOR SEQ ID :436:

(i) SEQUENCE CHARACTERISTICS:
(A) LENGTH: 163 base pairs
(B) TYPE: nucleic acid
(C) STRANDEDNESS: double
(D) TOPOLOGY: linear

(xi) SEQUENCE DESCRIPTION: SEQ ID :436:

AGTACGCAAT GCTTTCTTAC AGTAAGCAAG AGCTCCTCTG TAATCCTTCT 50

CGTTGAAGGA AATGCAAGCT TTACCAAGAA GGGCTGGAAT ATTATTCGGA 100

GACTGATTGA GTACAAAATG AAACTGTGCG TCAGCTTGAT CCATTTTGTC 150

ACCCTCAAGT AGG 163

(2) INFORMATION FOR SEQ ID :437:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :437:

TGTGGCTGAT CATAAGAAAG GAGCAAAGAA AGCTAGAATT GAAGAAGATA 50

AGAAGAAACA GAGGACAAAA AAACAGACGT TAAGGAAGAA GATAAATCCA 100

AGGACAACAG TGGCGAAAAA ACAGATACCA AAGGAACCAA ATCAGAACAG 150

CTCAGCAACC CCTGAATTTG 170

(2) INFORMATION FOR SEQ ID :438:

(i) SEQUENCE CHARACTERISTICS:
(A) LENGTH: 187 base pairs
(B) TYPE: nucleic acid
(C) STRANDEDNESS: double
(D) TOPOLOGY: linear

(xi) SEQUENCE DESCRIPTION: SEQ ID :438:

AGAACTACAG AAACCTGGCC TCCCTGAGTT TATCCTATGG GGGGCACAGG 50

AAAGAGCCCT GGACCATAGA AACCAAGTAC GAGTAGCAAG AAAACCAAAA 100

GGGTGGGAAT GGATCAAAGG TGTGAAAACA GATCTGTCTC GTAACTGTGT 150

AATCAAGGAA CTAGCACCAC AACAGGAAGA TAACCCA 187

(2) INFORMATION FOR SEQ ID :439:

(i) SEQUENCE CHARACTERISTICS:
(A) LENGTH: 234 base pairs
(B) TYPE: nucleic acid
(C) STRANDEDNESS: double
(D) TOPOLOGY: linear

(xi) SEQUENCE DESCRIPTION: SEQ ID :439:

AAAAGCAACC AGGTTCGAGA AACAATAGTG TATGTGTTCC TATAGGTATG 50

AGTTATTCAN TGTAGATTCG TTTTTCTGCA ATCCTCTGCC GAAATGATNT 100

ACACTTACAA TGNNGAATGC CATAGGACAC TACAATCTGA ATCAAAACAG 150

TCGCGTCTGA AATGNTNGTG TGGTAGACCA CCCCCATTCA AGCCTTCATA 200

CTTCGTAAAA ATGCAAATGT TGAAGATCGN NTCT 234

(2) INFORMATION FOR SEQ ID :440:

(i) SEQUENCE CHARACTERISTICS:
(A) LENGTH: 203 base pairs
(B) TYPE: nucleic acid
(C) STRANDEDNESS: double
(D) TOPOLOGY: linear

(xi) SEQUENCE DESCRIPTION: SEQ ID :440:

GGACCAGGAA CCTAACTGAG ATAAGTTCAG CTTTCCAGTT GACACCAAGT 50

CATCTAGTCT TCCCGGAGTA GATATAGTTG AGGTACTCCA TTTCCCAAAA 100

CAGAGAGCTG ATCCCGGGCT GCAACACCTC CAATAGTCGA AGCTCCCTTA 150

ATNAAGGATA TCAATGTATT TCTTAAACGC TTGATGTCGT TCAAAGTCTG 200

TTC 203

(2) INFORMATION FOR SEQ ID :441:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :441:

GTGATGCACC TGACATCCCC TCACAGGCTC TTGGAGAACT GTAGTGCAAC 50

TCACTTAACC GCAATGCTGA GAGCAGAATT CTGGAGTATG ATCCAGGGGA 100

ACGTTTCCCC ACACCACCCG AGCTACTTTA CCAGCGATCA TGATTGTGAT 150

GGAATAGGCT TATTAAGTTA CACATTTAAA AAGTCATTAG AACATCTCGT 200

TCTTGCACAC TAGTGTAGAA AGGTCTTCCA AAGATAAAAG GTGTAGGCCT 250

GGTT 254

(2) INFORMATION FOR SEQ ID :442:

(i) SEQUENCE CHARACTERISTICS:
(A) LENGTH: 147 base pairs
(B) TYPE: nucleic acid
(C) STRANDEDNESS: double
(D) TOPOLOGY: linear

(xi) SEQUENCE DESCRIPTION: SEQ ID :442:

GGGGNGCTAG CAGTTGGGNG GTGTAGGAGG CATTGCTGAT GATCTTGAGG 50

CTGTTGTCAT ACTNTTCANG NNNCACACTC ATGACGAACA TGGGGCATAG 100

CAAGGGGCGG ANGANGACCG TTTNGNTTCC TTGAAANGAG CCCCAGC 147

(2) INFORMATION FOR SEQ ID :443:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :443:

GGGGNGCTAG CAGTTGGGNG GTGTAGGAGG CATTGCTGAT GATCTTGAGG 50

CTGTTGTCAT ACTNTTCANG NNNCACACTC ATGACGAACA TGGGGCATAG 100

CAAGGGGCGG ANGANGACCG TTTNGNTTCC TTGAAANGAG CCCCAGCTNC 150

TCCGNGNGAG ACGCAGTGGA CTNCGATGNT TAGCNCTAGT NNCCCGCT 198

(2) INFORMATION FOR SEQ ID :444:

(i) SEQUENCE CHARACTERISTICS:
(A) LENGTH: 208 base pairs
(B) TYPE: nucleic acid
(C) STRANDEDNESS: double
(D) TOPOLOGY: linear

(xi) SEQUENCE DESCRIPTION: SEQ ID :444:

ATACTACGCT AGGAGAACAA TGTTCCTACA TATTATGGGT AGTGAGACAT 50

TATCTGTATA CAGGGACTGT GATTATTTAA AATATGCAGA ACTTATTTCA 100

TCTGTGCTTT AGAAATAACT GTATACAGTG TTATAAGTGA AAGAACTCAA 150

AATAACTAAT ACCAAATATA CACCTATGTA TTAGAATTCA AAAAGCTGCT 200

TTCTGTGA 208

(2) INFORMATION FOR SEQ ID :445:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :445:

AGCACATTTC ACTACGGGNT ATGTGCATTC TGGGCTTAGT AATGTCAAAT 50

CACTCTTCTC TCCAGCTTGG CCATTCCTCA TTCCTTGCGG GCCTGCCCTG 100

TAGACCACAG GCTAATGGAA TGTCCCGGTC TGNGTCATCA NATTCTGTAA 150

CCTGNAGCCC CCGCTGAG 168

(2) INFORMATION FOR SEQ ID :446:

(i) SEQUENCE CHARACTERISTICS:
(A) LENGTH: 243 base pairs
(B) TYPE: nucleic acid
(C) STRANDEDNESS: double
(D) TOPOLOGY: linear

(xi) SEQUENCE DESCRIPTION: SEQ ID :446:

AGGCAGAAGG GACGCTACTG ACTTAAGCCA AACCCTGATT GGTGGATAAA 50

GTATTCAGTT GTTCCATTCA TGCCTATCCT TTNTACAGCT CTCGCGACTA 100

TAACAAACAA CTGATAAAAT AGCATCAGTC CCTCCACCAG GTTGCCCTCA 150

AGGAAGCCAT GTTTGTGACG ATCCGACCAG TCCCTGACGA TCTCTCTAGA 200

CCACCCTGAC CTCGCCGAGA GTGCTGAAGG AGCTATCTAA CCA 243

(2) INFORMATION FOR SEQ ID :447:

(i) SEQUENCE CHARACTERISTICS:
(A) LENGTH: 251 base pairs
(B) TYPE: nucleic acid
(C) STRANDEDNESS: double
(D) TOPOLOGY: linear

(xi) SEQUENCE DESCRIPTION: SEQ ID :447:

CAGTCAGGGT CACAGAACAG TATTCAAAAT GATTGCCCAC CTGTTTTAGA 50

AATCTAAAAT NNTAAGTAAC TAAGAGCAAA GTGCTATGTG GGTTTTAGAC 100

CATGACTGTT TGTTTGCTCT CCTGCCCTAC CACCAAGCAA AGCAGCAGGG 150

CTCCTGGGGG AGAGGGATTT CAACCCCCCT GATGGCAGGG GGTGCTCTGG 200

GGAGGAGAGA GGAGAGAACA GGCTGTTTTG GAAAATATCC AGCACTTTGA 250

C 251

(2) INFORMATION FOR SEQ ID :448:

(i) SEQUENCE CHARACTERISTICS:

(A) LENGTH: 253 base pairs

(B) TYPE: nucleic acid

(C) STRANDEDNESS: double

(D) TOPOLOGY: linear

(xi) SEQUENCE DESCRIPTION: SEQ ID :448:

ACACTCCAGA NCATCCCACT AGAAAAACAA TTNNGCAGGA ACGTGATGGC 50

AACAATCAGC AGCCAATATT CTCAAGAGTT CCTAATCACC AAAAACATAT 100

ACAATNTAGT CTAGAAAAAT AAGTCAATTT CATAAAATAA GTTTTTAGAT 150

CGAAAAGCAC CCCCTTCACA GGTACAGAGA TACTGAAAAA TAGTCCCTAA 200

AAATCTACTC ATAGTTTACG GAGAGAAAGG CATGCCATGT GAGTTACGGA 250

GTG 253

(2) INFORMATION FOR SEQ ID :449:

(i) SEQUENCE CHARACTERISTICS:
(A) LENGTH: 113 base pairs
(B) TYPE: nucleic acid
(C) STRANDEDNESS: double
(D) TOPOLOGY: linear

(xi) SEQUENCE DESCRIPTION: SEQ ID :449:

ATATAAAAAA GATCCGCATA ATAAACCAAA TCAGAAAATA ATACCTTGTA 50

ATACCTCTGT AAGAAGCAGA ATACACCATA TGTTATTCAC ATGTATAGGA 100

GTGATAAGAA AAT 113

(2) INFORMATION FOR SEQ ID :450:

(i) SEQUENCE CHARACTERISTICS:
(A) LENGTH: 211 base pairs
(B) TYPE: nucleic acid
(C) STRANDEDNESS: double
(D) TOPOLOGY: linear

(xi) SEQUENCE DESCRIPTION: SEQ ID :450:

TAGCATCCAG GGGAAGTTAA TCAATACGCG AAAAATACTG AAATTAAAGA 50

GACAAGTAGA AGAAAATGAA ATAATCAAGA GATTGAGAAA AGTTGCCAGA 100

AAGCTTGGAG AAAAACCAAG ATATGTAATT TTCGCAGAAG TCAAAGGTAG 150

AAACTATTTG AGATCAAAGT CCTATAAACA AAGTTAAATG ATTCCAAGAG 200

GTAAATAGGA G 211

(2) INFORMATION FOR SEQ ID :451:

(i) SEQUENCE CHARACTERISTICS:
(A) LENGTH: 194 base pairs
(B) TYPE: nucleic acid
(C) STRANDEDNESS: double
(D) TOPOLOGY: linear

(xi) SEQUENCE DESCRIPTION: SEQ ID :451:

CTCCATTAGC TTTATGGGAC ATACGACGCA TAAAGATACT NTCCCGTGCG 50

NATTTCACAC NTGNCAGAGC TATAAACCGG TGNATGATGT GATTTTNCTG 100

TAGAATGATA TGGCCTGATA TGGAGGCCTC TTAATNGGCT TTTTCAAGCA 150

GCAAAATGGT CTTGNGTGAG TCGTGCCGAG GCTNNNGATC AAAG 194

(2) INFORMATION FOR SEQ ID :452:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :452:

ACTTCAAGCT CAACCTATTC TCGTTCTCTT TGTTAGAGGT GTATTGAGGA 50

TAGCATATTG AGAATATATT CTCTGGTTCA AACCAAAGAT TTTGTGACAT 100

TAAAACTACT TGAATTTCTA CTTCATAATA GGAGTCAGTC ACTTCTGGGA 150

CTATAGTGAT GCTTGCAA 168

(2) INFORMATION FOR SEQ ID :453:

(i) SEQUENCE CHARACTERISTICS:
(A) LENGTH: 220 base pairs
(B) TYPE: nucleic acid
(C) STRANDEDNESS: double
(D) TOPOLOGY: linear

(xi) SEQUENCE DESCRIPTION: SEQ ID :453:

CGATTCTTGC CCTAGTCAGA GACTATAGCA GCCCCCAGAC GTGAACCAAT 50

TGGCCATCTC CAGAAAGTTT AACAAAACCC GACCAGGTTG CCCCCTTCAC 100

TTTCTTACAA CCTCTTCCCC TTCCCCAGGG GCTCTGCTCC TCACTCCAGA 150

TCATCCTTTA GTTTAGAGCT GCGCAGTGAA GTGGATATCA CTGAAGGAGA 200

TAGGACGCCA GACTACACTG 220

(2) INFORMATION FOR SEQ ID :454:

(i) SEQUENCE CHARACTERISTICS:

(A) LENGTH: 275 base pairs
(B) TYPE: nucleic acid
(C) STRANDEDNESS: double
(D) TOPOLOGY: linear

(xi) SEQUENCE DESCRIPTION: SEQ ID :454:

GCCATCCTTG ATGGAAAAGA AACTGAAGAG AAAAGACAGC CTGTGGAAGA 50

AGCTCAAAGG TTCTTTGAAG AAGAAGAGAG AAAATATGAC ATGATATCTT 100

TGCTTTTGAG TTCCTCACGC TCTCTGAATN TTATTGGTTG GACATTCCAT 150

ATGTAGCATT CTGCTTCAAT ATTATCTATT ATGTGTCTCT CTCTCTTCAA 200

ATANNTGCCT GTAGGTAAAA GCAAGCTCTG CATATCTGTA CCTCTNGAGA 250

TAGTTTTGCT TTGTCTTTAG CGGTT 275

(2) INFORMATION FOR SEQ ID :455:

(i) SEQUENCE CHARACTERISTICS:
(A) LENGTH: 270 base pairs
(B) TYPE: nucleic acid
(C) STRANDEDNESS: double
(D) TOPOLOGY: linear

(xi) SEQUENCE DESCRIPTION: SEQ ID :455:

TCGCAGAAGG AGGAACGGGG ACTTTCCATC CCCTGAACCA AGGCATGTTA 50

ACACTTGGCT CCAGCATGTT GTCATCATTC AACCAGAAAT CGCACAAATG 100

CTACTGTGCC GGGGTGNAGC CAATTTTCTT AAGTAAGTGC TGACTTCCTT 150

AACANNTATC TNTNTTNTGG CTGTAGGGTG GCTCAGTGGA ATCCATTTTG 200

TTAACACCGA CAATTAGTTG TTTCACACCC AGTGGTAAGC CAGAAGGGCA 250

TGCTCTNGGG TCTGCCCATT 270

(2) INFORMATION FOR SEQ ID :456:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :456:

TCGCAGGAGA AGGAACCTTG ATGATACCAT TATCCTCACT TAGATGATGC 50

ACGACCCCTG CGCTGGATAC GGCGACGGTT TCTCATTTCG CCTTTGCCAG 100

CTCTCATTCG CTGAGGGGCA TAGACCTTTT GATATCATCC AGGCTTTAGT 150

TTCTTAAGGA GCAAAACAGC TTCCTTGNNC TTCTTGTAGC CTTCAACTTA 200

TTTAAACTAC CAAGGAAGTT CAGGAACTTC CTCAANACGA TGACCTTTAG 250

ACAT 254

(2) INFORMATION FOR SEQ ID :457:

(i) SEQUENCE CHARACTERISTICS:
(A) LENGTH: 236 base pairs
(B) TYPE: nucleic acid
(C) STRANDEDNESS: double
(D) TOPOLOGY: linear

(xi) SEQUENCE DESCRIPTION: SEQ ID :457:

ACACTAACTG TTCCATCCGT TATATTTGCT GTGAGGAAAA TTAAGATTCC 50

TGTTGTATGG GCTGCACTGT TTCTGGAAGA CTACAGAAAA TCTAACATGG 100

TTGACACTTC CTGGTAGCCC TTCTGTACAT ACACACACAC AACCAAGAGA 150

GAAGACAGAG AGAAAATCCT GGTCCAAAAG ATCACATGAC CTTACTAGTG 200

TTTCCCCAAT GACTGTAATT TATAAACTAA AAATTG 236

(2) INFORMATION FOR SEQ ID :458:

(i) SEQUENCE CHARACTERISTICS:
(A) LENGTH: 108 base pairs
(B) TYPE: nucleic acid
(C) STRANDEDNESS: double
(D) TOPOLOGY: linear

(xi) SEQUENCE DESCRIPTION: SEQ ID :458:

GAGANNNNTT NNGGNAAATG NTTNCGCACT GNAGCTAAGA ANAGNNATGG 50

NNNTAGGGNG NNAGANGNCN TGAACAGAGA AAGCNTGAGG GCTCTGGGAC 100

GCTGGTAT 108

(2) INFORMATION FOR SEQ ID :459:

(i) SEQUENCE CHARACTERISTICS:
(A) LENGTH: 155 base pairs
(B) TYPE: nucleic acid
(C) STRANDEDNESS: double
(D) TOPOLOGY: linear

(xi) SEQUENCE DESCRIPTION: SEQ ID :459:

ACCCATGGCA AATAGGAAGA AGCTCAGTAT CGGCTCCTCC CACCATAACC 50

CCCACTTCTC CCGCCTCCTG GACCATAGTT TCCTCCACCA TACGGTCCCC 100

CCATGTTCCT GCTACCACCA AAGTTTCCAC TCTCCATTGG ACCGTAGTTA 150

GAGGT 155

(2) INFORMATION FOR SEQ ID :460:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :460:

TGCTATTTCC CTTTCATAAA GGCACACATT TGATCTTATC TTCTCTTACC 50

CAACACGCAG TGGCAGTGTG TATTTTCCTT CTCTTTTTTT GTTAAATATT 100

CTGGTTTGTG GAGGTTCACA GACATGTGTT AGTATATCCT TGCCTGCATG 150

TAGTTGTTCA TTACTAGAC 169

(2) INFORMATION FOR SEQ ID :461:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :461:

ACCNTGTTTA ATTANTGCGC GGGCTCGCCG TTTGCATACA ATGGCTTACT 50

CAGTGCTNNC AGGCTGTGAG TNAATAGAGN GTGTATGACT TAATAAGCAT 100

TTTATCAGCG TACCTTTTTC GCCATGCGCT ACCTGCTATT GATGAAGGCG 150

GCTTAGGGCA TCGAAAAACC TAAAAGTCGA GCTT 184

(2) INFORMATION FOR SEQ ID :462:

(i) SEQUENCE CHARACTERISTICS:
(A) LENGTH: 275 base pairs
(B) TYPE: nucleic acid
(C) STRANDEDNESS: double

(D) TOPOLOGY: linear

(xi) SEQUENCE DESCRIPTION: SEQ ID :462:

AGGAACCNTG TTTAATTANT GCGCGGGCTC GCCGTTTGCA TACAATGGCT 50

TACTCAGTGC TNNCAGGCTG TGAGTNAATA GAGNGTGTAT GACTTAATAA 100

GCATTTTATC AGCGTACCTT TTTCGCCATG CGCTACCTGC TATTGATGAA 150

GGCGGCTTAG GGCATCGAAA AACCTAAAAG TCGAGCTTNG NNGTGCCGGN 200

AACGGCTCTA NANTACTTCT CATTGTAACT AGAGTACCAT ATTCGGCCGT 250

NNACTGNGTT GTTGGCGACA GATGT 275

(2) INFORMATION FOR SEQ ID :463:

(i) SEQUENCE CHARACTERISTICS:
(A) LENGTH: 149 base pairs
(B) TYPE: nucleic acid
(C) STRANDEDNESS: double
(D) TOPOLOGY: linear

(xi) SEQUENCE DESCRIPTION: SEQ ID :463:

AATTCACTAT GCGTGGCCGC CACAGCTATN CTTGTCCTCC TGGATCCTGA 50

ACCTTGNAAG CTGCACTAAT GAGTTCAACG GGAGTGCTCT GGGCCCAGGT 100

GTCAGCTGTA GCAATGCCCC NGCTGCAACT GAAGGNGCCA GCAATGCTA 149

(2) INFORMATION FOR SEQ ID :464:

(i) SEQUENCE CHARACTERISTICS:
(A) LENGTH: 179 base pairs
(B) TYPE: nucleic acid
(C) STRANDEDNESS: double
(D) TOPOLOGY: linear

(xi) SEQUENCE DESCRIPTION: SEQ ID :464:

ATCATCAGCA GTGTTGCTCA GCTCCTACCT CTGTGCCAGG GCAGCATTTT 50

CATATCCAAG ATCAATTCCC TCTCTCAGCA CAGCCTGGGA GGGGGTCATT 100

GTTCTCCTCG TCCATCAGAT CTCAGAGGCT CAGAGACTGC AAGCTGCTTG 150

CCCAAGTCAC ACAGCTAGTG AAGACCAGA 179

(2) INFORMATION FOR SEQ ID :465:

(i) SEQUENCE CHARACTERISTICS:
(A) LENGTH: 156 base pairs
(B) TYPE: nucleic acid
(C) STRANDEDNESS: double
(D) TOPOLOGY: linear

(xi) SEQUENCE DESCRIPTION: SEQ ID :465:

CTACCTCTGT GCCAGGGCAG CATTTTCATA TCCAAGATCA ATTCCCTCTC 50

TCAGCACAGC CTGGGAGGGG GTCATTGTTC TCCTCGTCCA TCAGATCTCA 100

GAGGCTCAGA GACTGCAAGC TGCTTGCCCA AGTCACACAG CTAGTGAAGA 150

CCAGAG 156

(2) INFORMATION FOR SEQ ID :466:

(i) SEQUENCE CHARACTERISTICS:
(A) LENGTH: 186 base pairs
(B) TYPE: nucleic acid
(C) STRANDEDNESS: double
(D) TOPOLOGY: linear

(xi) SEQUENCE DESCRIPTION: SEQ ID :466:

ACATCCCTGG AAGGAGGGCC TGAGGGCCAG GGAGGGAACA AGGCAGGAGA 50

CTGCTGGTTC TGGTTTTGGC CACCTCACCC TTGGCCACGT CCCCTCCGGC 100

TAAGCCACAG CACAAAGCAG AGCCAGGCTC TGGAGGCCCA GGGCCTCACC 150

ACTCCCCTNT GTCCCCCCAG CAGGGGGACA AAACAG 186

(2) INFORMATION FOR SEQ ID :467:

(i) SEQUENCE CHARACTERISTICS:
(A) LENGTH: 87 base pairs
(B) TYPE: nucleic acid
(C) STRANDEDNESS: double
(D) TOPOLOGY: linear

(xi) SEQUENCE DESCRIPTION: SEQ ID :467:

TNNNGATGAN TATANAAGCA TCATNGACGG TATTTCCCNG TCTTGNANTT 50

TNATCGAGAN TTTANTCTAG TAANTATATT AATNTNT 87

(2) INFORMATION FOR SEQ ID :468:

(i) SEQUENCE CHARACTERISTICS:
(A) LENGTH: 187 base pairs
(B) TYPE: nucleic acid
(C) STRANDEDNESS: double
(D) TOPOLOGY: linear

(xi) SEQUENCE DESCRIPTION: SEQ ID :468:

ACTAGAAGTA CAGCATCCTG CTGCAAAAAT GATTGTAATG GCTTCTCATA 50

TGCAAGAGCA AGAAGCCGAG ATGACACAAA CTTTGCCTGG TATTTGTTGG 100

AGCTCTCCTG GAATCACTGA AGAACTTCTG AGGATCGGCC TGTCAGTTTT 150

AGAGGTCATA GAAGGTCATG AAATAGCCTG CAGAAAA 187

(2) INFORMATION FOR SEQ ID :469:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :469:

AGGGCACCAT TACCATCCAT CTGACATCGC ATTTCCATAG AAATGCCAAA 50

GAAAGAAGGT CCTGGGGTTT TTCATAGAAA GCTCAAAAAG TTCAACCTTT 100

GATGCTATCC CCCAGCCCAA TACAAAATAC ACAGAAAAAG CAATTATTAA 150

ACATCGGCTT CGTTTCTTTT TCNCCTTTGA ATNTTAATGT TTACATACTA 200

GTGTGCAGCA CCTACTTCTT NATCGCCGTG AACTGAAATC TAGATTTTAA 250

ACTGAA 256

(2) INFORMATION FOR SEQ ID :470:

(i) SEQUENCE CHARACTERISTICS:
(A) LENGTH: 109 base pairs
(B) TYPE: nucleic acid
(C) STRANDEDNESS: double
(D) TOPOLOGY: linear

(xi) SEQUENCE DESCRIPTION: SEQ ID :470:

CTCAAAACGA CAATTCTGTG CCTGGGGGAT CTGACCTGGT GAGGTAGCCT 50

GAAGTCTGAA TGGAGCCCAT AGTCGAAAAC AACCTAAGAA TCTCTCAGAA 100

GAGGGTTTG 109

(2) INFORMATION FOR SEQ ID :471:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :471:

GCGGCAGTAT ACAGAAGCCA TATCAGTTGG GAAAAACTTT ATCAATCATA 50

GAGCCTTTTG AAGAAAAATT TGCCAAGCGT GGTTTTTTGC TTGNAGACCT 100

ATATATATTA CCTACAGGCT GAGAAAGCTT TGNATTTCT 139

(2) INFORMATION FOR SEQ ID :472:

(i) SEQUENCE CHARACTERISTICS:
(A) LENGTH: 262 base pairs
(B) TYPE: nucleic acid
(C) STRANDEDNESS: double
(D) TOPOLOGY: linear

(xi) SEQUENCE DESCRIPTION: SEQ ID :472:

TCGCAGGAGA AGGAATGTTC CCAATAGGAA CGCCTGTAGA CTGTTCAGAA 50

GAAATGCCCA AATGAGCCAG ATGAGAAGGC TGAGGGCAGG GCTGCTTTTG 100

GCTCTGAGGA CTATAGATTT ATCCTCTAGG TGATGAGGGA CTATTAACGG 150

CTGGTGAGTC TGGAGAACTG AACAGTGGAA GCTCTATTTT AGATTCACGT 200

GGCAGTAGAG GATAGAGGTG TTTGGAAGCG GTGGGCAGTT GCAAGCTATA 250

TGGGAGACAT TT 262

(2) INFORMATION FOR SEQ ID :473:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :473:

ACAGAAGGAC TTTGTCTCTT TAGCTTGTTT AGCTCAATGA ACATTATCTC 50

GGCAAATGAC TCTGCTTTCT CGAAGGTCCT TCTCCGCTCC AGGTTTACTT 100

GCATCTCTCA TACTTNNTAC AGCCAACATG AACACTCTAT GTATTTTCTA 150

AGCTTTCNTC TGTTCAAGAA CTTTGAATTT AAAACGTCT 189

(2) INFORMATION FOR SEQ ID :474:

(i) SEQUENCE CHARACTERISTICS:
(A) LENGTH: 166 base pairs
(B) TYPE: nucleic acid
(C) STRANDEDNESS: double
(D) TOPOLOGY: linear

(xi) SEQUENCE DESCRIPTION: SEQ ID :474:

TNCGTGCCTA GCAAACTTAA ACTCGAACGC ACGTAATAGT GCTCATAATT 50

CTNTNAAGGA CTTTAAACTT TACTCNGTAT GCTNTNTTGA TGACTCTAGC 100

AGCCTCGCTA ACCTAGTTTA CCCCACTGTC CCCACCGGCG AACTNTTTGT 150

GTTAGTACGC GNGTTA 166

(2) INFORMATION FOR SEQ ID :475:

(i) SEQUENCE CHARACTERISTICS:
(A) LENGTH: 109 base pairs
(B) TYPE: nucleic acid
(C) STRANDEDNESS: double
(D) TOPOLOGY: linear

(xi) SEQUENCE DESCRIPTION: SEQ ID :475:

TTTTTTTTTA GTAACACTAA AGAGCTGTAA AGAACATTGA AGGTGGTCAT 50

TCCTTCAAAA CTGTGTTTTG ACCACACAAG GTGGGCATTA ACAAACAAAT 100

TCAACTTAA 109

(2) INFORMATION FOR SEQ ID :476:

(i) SEQUENCE CHARACTERISTICS:
(A) LENGTH: 131 base pairs
(B) TYPE: nucleic acid
(C) STRANDEDNESS: double

(D) TOPOLOGY: linear

(xi) SEQUENCE DESCRIPTION: SEQ ID :476:

TCTGGGCGGA AGGTGGTGCG GTGAAAGGTG CAGGGACAGA CTGGGTTAGA 50

GGCCACTCTT GGTCTTATCC TCCATGGCCA CAACAGAGGT GACAAATACA 100

CGGGTCACTC AGTTACGTTT AGCCACAGCC T 131

(2) INFORMATION FOR SEQ ID :477:

(i) SEQUENCE CHARACTERISTICS:
(A) LENGTH: 265 base pairs
(B) TYPE: nucleic acid
(C) STRANDEDNESS: double
(D) TOPOLOGY: linear

(xi) SEQUENCE DESCRIPTION: SEQ ID :477:

ACATGGATAG GTGTATGCAT ACTACGGCTA AGGAGAAACA ATGTTCCTAC 50

ATATATGGGT AGTGAGAACA TTATCTGTAT AACAGGGAAC TGTGATTATT 100

TAAAAATATG CAGAACTTAT TTCATCTGTG CTTTAGAAAT AACTGTATAC 150

AGTGTTATAA GTTGAAAAGA ACTCAAAATA ACTAATACAA ATATACACTA 200

CGTATTAGAA TTCAAAAAAG CTGCTTTCTG TGAAGTCAAT CAGCTATATT 250

AAAAAAGACA CAAAT 265

(2) INFORMATION FOR SEQ ID :478:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :478:

AATCTACCAA CCTTAAAGCT CCCGNGATAA AGCTGTATTT CCAAAAGACC 50

TGTNTTTATT NGNNGGNGTT NGCTTTCTTT GTCATCTAGA GCCTTGTTGT 100

ACATGCAATG GGTGGGAGAT AGTGGTACCT ACTGTTGNTT CTNTCTGTGT 150

NTTCATCATG GTGTTGTCTA GGTCTCCTGA GG 182

(2) INFORMATION FOR SEQ ID :479:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :479:

AAGCCGTCGG GAGCCGCCGC CGCCATCTGA GGGAGGTACC CTGGAAACCA 50

CCTTTCACGG TGGGGAAGTG CAGTCGCGGT GGGCAGCTCT GGGGCCACGA 100

AACGGGAGCC TCTAAATCTT GGTCGGGACT GCTCGCCTGG AGCCGCACTC 150

TTGAGTCCGA GGCCATCTT 169

(2) INFORMATION FOR SEQ ID :480:

(i) SEQUENCE CHARACTERISTICS:
(A) LENGTH: 238 base pairs
(B) TYPE: nucleic acid
(C) STRANDEDNESS: double
(D) TOPOLOGY: linear

(xi) SEQUENCE DESCRIPTION: SEQ ID :480:

AGATAACGCT CCCATCCCAG GCTAGAGACT CCATCTCTAA TTTNCNCAGT 50

CCGTCAATGA GAGGCAGGGA CTGAAATCCC AAGTTCTGTC TCACCGGATA 100

TTTTCCAGGA ATGCCTCTCT TCCAAACATC AGCGACATTT AACAGACCCT 150

GAGCAGCAAA CTTCTGCCCC AGAGGAAAGC AGAAAACCAA TTTATGTAAA 200

ATTAGAAGCG ATTTGCTTGA TCCATCACTN GCTTCCAC 238

(2) INFORMATION FOR SEQ ID :481:

(i) SEQUENCE CHARACTERISTICS:
(A) LENGTH: 201 base pairs
(B) TYPE: nucleic acid
(C) STRANDEDNESS: double
(D) TOPOLOGY: linear

(xi) SEQUENCE DESCRIPTION: SEQ ID :481:

TCGCAGGAGA AGAAATCAAC CCCTGCTTCT CAAATCCAGA CAGGCCACAC 50

TGGCTAGAAC TTCCACCCAG CAGTCCTGCT CCTGCCCGAA GTCTGCAAGC 100

AAGTGAACCA CATGTCGCTA TGAAAGCACA CAGACAACAG ATTAGGGCAG 150

ACCTGGCAAA GATATGCCTG TCTGCCATCT TGGCCCCTGT CTGAGGGAGG 200

C 201

(2) INFORMATION FOR SEQ ID :482:

(i) SEQUENCE CHARACTERISTICS:
(A) LENGTH: 162 base pairs
(B) TYPE: nucleic acid
(C) STRANDEDNESS: double
(D) TOPOLOGY: linear

(xi) SEQUENCE DESCRIPTION: SEQ ID :482:

CGGCTCAGNC TGTTGGCGCG AAGAGAGTCT AACCCAAAAT TGCAAAACTC 50

CCGTTGATTT CCAGGCCCTA CCACACGGCG ATGTCAACTT GTCCTCCAGA 100

CATGGACGAC TACCAAAGAT CCCAGCTACG AAGCATGGCC TTGCTTAGAA 150

ACNTTTTTAG AT 162

(2) INFORMATION FOR SEQ ID :483:

(i) SEQUENCE CHARACTERISTICS:
(A) LENGTH: 250 base pairs
(B) TYPE: nucleic acid
(C) STRANDEDNESS: double
(D) TOPOLOGY: linear

(xi) SEQUENCE DESCRIPTION: SEQ ID :483:

ATATGAAGGA GGAAATGCTC GTCACCTTCG TGAATACCAA GACCTGCTCA 50

ATGTTAAGAT GACCCTTGAC ATGAGATTGC CACCTACAGA AGCTGCTGGA 100

AGGCGAGGAG AGCAGGATTT CTCTGCCTCT TCAAACTCNT NCCTGAACCT 150

GAGGGAAACT TTGGATTCTC CCTCTGGTCG ATACCCACTC AAAAAGGACA 200

CTTTTGATTA GGACGGTTGA AACTAGAGAT GAACGGTTAT CAACGAAACT 250

(2) INFORMATION FOR SEQ ID :484:

(i) SEQUENCE CHARACTERISTICS:
(A) LENGTH: 132 base pairs
(B) TYPE: nucleic acid
(C) STRANDEDNESS: double
(D) TOPOLOGY: linear

(xi) SEQUENCE DESCRIPTION: SEQ ID :484:

AACATTATCT TGACAAACTG AAGAACACTT CAGTTAACAC TACCTCGAAG 50

AACCATCAAT GACTTGCTTT GAACAGACTA TAAAAGGCAT TCTCAAGGAG 100

ATTAGAATGT TAATGCCACT TTGATTAGAT CT 132

(2) INFORMATION FOR SEQ ID :485:

(i) SEQUENCE CHARACTERISTICS:
(A) LENGTH: 129 base pairs
(B) TYPE: nucleic acid
(C) STRANDEDNESS: double
(D) TOPOLOGY: linear

(xi) SEQUENCE DESCRIPTION: SEQ ID :485:

TGTGCGTGGA GACGGTGGAG AGTGGAGCCA TGACCAAGGA CCTGGCGGGC 50

CGCATTCACG GCCTCAGCAA TGTGAAGCTG AACGAGCACT TCTTGAACCC 100

ACGGACTTCT CGACACCATC AAGAGCGAC 129

(2) INFORMATION FOR SEQ ID :486:

(i) SEQUENCE CHARACTERISTICS:
(A) LENGTH: 172 base pairs
(B) TYPE: nucleic acid
(C) STRANDEDNESS: double
(D) TOPOLOGY: linear

(xi) SEQUENCE DESCRIPTION: SEQ ID :486:

TCGCAAGAGG AGACATTCTG ATCATCCTCA CTGGACGCCA CAGGGGCAAG 50

AGGGTGGTTT TCCTGAAGCA GCTGGCTAGT GGCTTATTAC TTGTGACTGG 100

ACCTCTGGTC TCAATCGAGT TCCTCTACGA AGAACACACC AGAAATTTGT 150

CATTGCACTT CAACCATATC GA 172

(2) INFORMATION FOR SEQ ID :487:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :487:

ATGCTGCACA CTATGTCTCA CAAACTAAAT GGATCCATTA AAAGTTATGA 50

TTTAAAAGGC GACCACCCCC AAAAGAAGTC ATAACACTCA AGGGTGTCAA 100

TATATACAAC TGTGTAAACA CAACCAATCT ACAACTATAT CAACACAACC 150

AGCACTCCTC TATGGGCACA GACACACACA CAAAATTGTC CTTGCTTTTC 200

TCAGATATAT 210

(2) INFORMATION FOR SEQ ID :488:

(i) SEQUENCE CHARACTERISTICS:
(A) LENGTH: 90 base pairs
(B) TYPE: nucleic acid

(C) STRANDEDNESS: double
(D) TOPOLOGY: linear

(xi) SEQUENCE DESCRIPTION: SEQ ID :488:

ACATGGATAG GCGTATGCAT ACTACACTAA GGAGAAACAA TGGTCTACAC 50

ANACGTAGTG AGAACATTAT CTGTATACGG GAACTGTGAT 90

(2) INFORMATION FOR SEQ ID :489:

(i) SEQUENCE CHARACTERISTICS:
(A) LENGTH: 99 base pairs
(B) TYPE: nucleic acid
(C) STRANDEDNESS: double
(D) TOPOLOGY: linear

(xi) SEQUENCE DESCRIPTION: SEQ ID :489:

ACCATGAGAC CTACATCCGA ATCTGACCCA GGCAAACATA CCGGGAGCCA 50

TACCGCACTA NCGGCTCTTC TCAAATCTCC TGGCCACNCA CCGAGNGCC 99

(2) INFORMATION FOR SEQ ID :490:

(i) SEQUENCE CHARACTERISTICS:
(A) LENGTH: 186 base pairs
(B) TYPE: nucleic acid
(C) STRANDEDNESS: double
(D) TOPOLOGY: linear

(xi) SEQUENCE DESCRIPTION: SEQ ID :490:

GGAAACCTGG AGGTGCGCAT CCTCGAGTGC GAAGAGAAGG TCTTCCCCAG 50

CCCCCTCTGG ACTCCATGCA CCAAGGTCAT GGCCAGGAGC TCTTGGCAGC 100

TCAGCCCTGC CGCCCCAGAG CATGTGGCGG CTGCTCTCTA CCAGCCGAGA 150

GCTTCGGAGA TGCAGCATCT GCGGCGAATG CCCCGA 186

(2) INFORMATION FOR SEQ ID :491:

(i) SEQUENCE CHARACTERISTICS:
(A) LENGTH: 263 base pairs
(B) TYPE: nucleic acid
(C) STRANDEDNESS: double
(D) TOPOLOGY: linear

(xi) SEQUENCE DESCRIPTION: SEQ ID :491:

AGCCAACTAA GTTCTCTCTT CGTGAAACAC AGGTCCATGA GTCGACACAA 50

ACACTAATGC AAGAACCATC ACGGAAAACC ACCGCAGCAG CTGAAACTTT 100

TATAGCCCAT AAAAGGACCA AACAAGTAAG CTGAATGACT GTGAAAATAT 150

GACCTTCCAG AGCGGCACAT AACAGGATAT CAAATCAGGC TGATGCTTAG 200

CAGGCTTCAA ATATNATGGT CAAATGACTG GATTACTTAN ATGAGGCAAC 250

TTCATATCGG AAA 263

(2) INFORMATION FOR SEQ ID :492:

(i) SEQUENCE CHARACTERISTICS:
(A) LENGTH: 159 base pairs
(B) TYPE: nucleic acid
(C) STRANDEDNESS: double
(D) TOPOLOGY: linear

(xi) SEQUENCE DESCRIPTION: SEQ ID :492:

AGAGTGGGGT TAGCTCTGCC TAGCGCTACA CAAGAAAACC TCCCTCCAGT 50

AAATNGGTGT GGGNGGTCCG CTTTTGGCCA TCATCGCACC CCCCCGGTCA 100

CTGGGCGTTG TTGCCGGGCA CTTGTTTNNC NGGCTGGGTG TGTACCGTAA 150

CCGTGGGTC 159

(2) INFORMATION FOR SEQ ID :493:

(i) SEQUENCE CHARACTERISTICS:
(A) LENGTH: 197 base pairs
(B) TYPE: nucleic acid
(C) STRANDEDNESS: double
(D) TOPOLOGY: linear

(xi) SEQUENCE DESCRIPTION: SEQ ID :493:

GGGCAGAGNA AGAACTGTTC CACCAGGTGA ACAGTCCTAC CTGCTTGGTA 50

CCATAGTCCC TCAATAAGAT TCAGAGGAAG AAGCTTATGA AACTGAAAAT 100

CAAATCAAGG TATCGGGAAG AATAATTTCC CCTCGATTCC ACAGGAGGGA 150

AGACCACACA ATATGTNGTG CTGGGGCTCC CCAAGGCCCT GCCACCT 197

(2) INFORMATION FOR SEQ ID :494:

(i) SEQUENCE CHARACTERISTICS:
(A) LENGTH: 188 base pairs
(B) TYPE: nucleic acid
(C) STRANDEDNESS: double
(D) TOPOLOGY: linear

(xi) SEQUENCE DESCRIPTION: SEQ ID :494:

GATGGGGAAG GGCATCCCAA CACAGCCTGT GGATCCTGGG GCATCTGGAA 50

GGGCGCACCA TCAGCAGCCT CACCAGCTGT GAGCCTGCTA TCGGGCCTGC 100

CCCTCCAATA AAAGTGTGAG AACTCCACTG TGTGCCCTGT CTTTGGGCAG 150

GGAGGGCTGC TGTGAGTGGA GTCTTGAGTN GGTGAGTG 188

(2) INFORMATION FOR SEQ ID :495:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :495:

ACATGACCAT CCGCATCCGC CTTTATTGAC AATGAGAAGA TGGAGTCCCG 50

GACGCATCTA TCCCTCTTTG GCCCTTACAG GTTTGCCACG AGAGTGAGAC 100

GCCTTCCTGG ACCAGGGGAG GGNGNGTTGG TNCTNTGNGC GTGNGGGTNT 150

GTGGGNGCTG CTGGGGAGG 169

(2) INFORMATION FOR SEQ ID :496:

(i) SEQUENCE CHARACTERISTICS:
(A) LENGTH: 172 base pairs
(B) TYPE: nucleic acid
(C) STRANDEDNESS: double
(D) TOPOLOGY: linear

(xi) SEQUENCE DESCRIPTION: SEQ ID :496:

CAGAGAGAAC GTTTCTATGG CTGCTGCTTC TAGGAGTCTC TCGCTCATAG 50

AAAAGGCACA CACTGAAAGA GGAAGCAGAT CCCATTGCTG TGGAAGTCCC 100

ATTGTTAGGA AGCTCTGCTT TTCTGGAGTT CAAATTCGCA TTCATGATGC 150

TTTAAACCGT CAAGCTGGGT GG 172

(2) INFORMATION FOR SEQ ID :497:

(i) SEQUENCE CHARACTERISTICS:
(A) LENGTH: 246 base pairs

(B) TYPE: nucleic acid
(C) STRANDEDNESS: double
(D) TOPOLOGY: linear

(xi) SEQUENCE DESCRIPTION: SEQ ID :497:

GAGAGTGGGG TTAGCTCTGC CTAGCGCTAC ACAAGAAAAC CTCCCTCCAG 50

TAAATNGGTG TGGGNGGTCC GCTTTTGGCC ATCATCGCAC CCCCCCGGTC 100

ACTGGGCGTT GTTGCCGGGC ACTTGTTTNN CNGGCTGGGT GTGTACCGTA 150

ACCGTGGGTC CTCTGACAAG TGCCTAACTC GGCCCACCCC TTAGGGTGTG 200

TNTCATCGAA GTGTAGNGAA TGGTGGAACG TTTGTTTGTN GTGTGC 246

(2) INFORMATION FOR SEQ ID :498:

(i) SEQUENCE CHARACTERISTICS:
(A) LENGTH: 215 base pairs
(B) TYPE: nucleic acid
(C) STRANDEDNESS: double
(D) TOPOLOGY: linear

(xi) SEQUENCE DESCRIPTION: SEQ ID :498:

GCCCCTTTAC CAGCAAGTCC TCTACTCAGA AAGAACTGAC CCACGCAAGT 50

CTGGGAGAGT GACTAGTTCA AATGTGCAGG GCTGAAGCTT CCAAACACAG 100

CCACTATTTT TGTTGTATAT CTTCATCTCA ATGGCGACAT GGCCACTGCC 150

CAAGGAACTT GTGGCAGGGA TCCCAAGGTG AGGCAGCAAC AGATGTCTGT 200

GAACATCGTG CGTTA 215

(2) INFORMATION FOR SEQ ID :499:

(i) SEQUENCE CHARACTERISTICS:
(A) LENGTH: 195 base pairs
(B) TYPE: nucleic acid
(C) STRANDEDNESS: double
(D) TOPOLOGY: linear

(xi) SEQUENCE DESCRIPTION: SEQ ID :499:

GATNGCCACA TCTCAACACT ATACNACTCG CTNTTCGAAT TCGCCGTNTT 50

AGAACCGCAA GAGACCTTGA TTTAGTCACG CGAGTTCGTC TTCCTGTTCC 100

ACANGAAAAT AAAGCTAGGG AGGTGATTTA TCTATCCGAG AAAAAAGCCG 150

GGGACTGGTG GAANNNGAAC AATGNTCTCT NTGTCGTACT ACAAT 195

(2) INFORMATION FOR SEQ ID :500:

(i) SEQUENCE CHARACTERISTICS:
(A) LENGTH: 260 base pairs
(B) TYPE: nucleic acid
(C) STRANDEDNESS: double
(D) TOPOLOGY: linear

(xi) SEQUENCE DESCRIPTION: SEQ ID :500:

GCGGCCTTGG GGGCACCGGC GTGTCCTGCC CAGTGGGATT AAAAAATAAT 50

GCTCCCCACA TGGCGGGCCT TTGAGGTTCC AGTAAAAATG CTTTCAACAA 100

ATGGGCAATG CTTGTGTGAT TCACAATCGT GGCATTTAAA GTGCACAAAG 150

TACAAAGGAA TTTATACAGA TTGGTTTACC GAAGTATAAT CTATAGGAGG 200

CGCGATGGCA AGTTGATAAA ATGTGACTTA TCTCCTAATA AGTATGGGGG 250

GTGGAGCTGT 260

(2) INFORMATION FOR SEQ ID :501:

(i) SEQUENCE CHARACTERISTICS:
(A) LENGTH: 268 base pairs
(B) TYPE: nucleic acid
(C) STRANDEDNESS: double
(D) TOPOLOGY: linear

(xi) SEQUENCE DESCRIPTION: SEQ ID :501:

AAAGGCATAG TAAAAATAAA ATCTACGTAA GTAACAATCT AATACTATAT 50

TAAATNCGTT GCTACAAAGT GTTTTGTTTC TCTAAAAAGT AGTTTTTGCA 100

TATCATTCGA CCTCTTCACC CATNTGCTGG CTTATTTGCT TTATATACAA 150

CAGTTAAAAT TTGTGCACTA AGCTGAGCTG CCTTCACAAT GTGGTTCAGA 200

CAAAATGCAC CCAAAGAACT ACATGTTAAG AGAGTTTATG TCCATGCTCA 250

ACCATGGCTT GCCCAAAT 268

(2) INFORMATION FOR SEQ ID :502:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :502:

AAAAACTCTA ATCCAGACTA CAGTTGTCGA GATTCAAGTC GTGAGTGCAG 50

GAGCGTACAC AGTGCCGTGC TGGCACATGC ACTGCACACG CTCTAGAGAC 100

GCTGACCTGG CTCTCGGAAA CGCAGGAGTC TTTCTGAGCC AGCTCAGAAA 150

CC 152

(2) INFORMATION FOR SEQ ID :503:

(i) SEQUENCE CHARACTERISTICS:
(A) LENGTH: 141 base pairs
(B) TYPE: nucleic acid
(C) STRANDEDNESS: double
(D) TOPOLOGY: linear

(xi) SEQUENCE DESCRIPTION: SEQ ID :503:

CCCTGACCCC TCCTCACCAC CGCGCTGCAC CTCAGGGTTA CAAGAAGAAC 50

TAGGAAATAA CGCCGGCCAC CNGACCCCTG GAGAGGGGCC GGCTAGAACA 100

NTTCTAAGAT CCNGCACAGC AGGTCCCGNA TGTNGAACCT T 141

(2) INFORMATION FOR SEQ ID :504:

(i) SEQUENCE CHARACTERISTICS:

(A) LENGTH: 107 base pairs

(B) TYPE: nucleic acid

(C) STRANDEDNESS: double

(D) TOPOLOGY: linear

(xi) SEQUENCE DESCRIPTION: SEQ ID :504:

CACACGACAC ACGACACGCA CGCAAACACG CCAGACGCGA CAGAGCGCGC 50

GCGGGAGCGG AGCAGCGGAA GCGCAGCAGC GCACACGAGA GATAAGGGCG 100

GCCGAGC 107

(2) INFORMATION FOR SEQ ID :505:

(i) SEQUENCE CHARACTERISTICS:

(A) LENGTH: 193 base pairs

(B) TYPE: nucleic acid

(C) STRANDEDNESS: double

(D) TOPOLOGY: linear

(xi) SEQUENCE DESCRIPTION: SEQ ID :505:

ACCCTCTTCT GATAAATTTG AGGGCCCGTT TGTCCTTGGA GACCTTCAGT 50

AACTCCATGG CGCGCCATCG TACGGGGCAA ANACACACCT CCCGAATCAT 100

GTCCCGCACG AACTTGGTGT GTTTGGTCAG ACGCCCGCGT TNGGCNTGTG 150

CTGGGCTTGC TCACGTTCTT GTCACTTTGT GGCCCTTGTT GAG 193

(2) INFORMATION FOR SEQ ID :506:

(i) SEQUENCE CHARACTERISTICS:
(A) LENGTH: 274 base pairs
(B) TYPE: nucleic acid
(C) STRANDEDNESS: double
(D) TOPOLOGY: linear

(xi) SEQUENCE DESCRIPTION: SEQ ID :506:

ACATGGATAG GCGTATGCAT ACTACGCTAA GGAGAAACAA TGTTCCTACA 50

TATTACGGGT AGTGAGAACA TTATCTGTAT AACAGGGAAC TGTGATTATT 100

TAAAAATAGC AGAACTTATT ANCTGTGCTT TAGAAATAAC TGTATACAGT 150

GTTATAAGTT GAAAAGAACT CAAAATAACT AATAAATATA ACCTATGTAT 200

TAGAATTAAA AAAGCTGCTT TCTGTGAAGT CAATCAGCTA TATTAAAAAA 250

TGACACAAAT CCAAACAAGA TGCA 274

(2) INFORMATION FOR SEQ ID :507:

(i) SEQUENCE CHARACTERISTICS:
(A) LENGTH: 281 base pairs
(B) TYPE: nucleic acid
(C) STRANDEDNESS: double
(D) TOPOLOGY: linear

(xi) SEQUENCE DESCRIPTION: SEQ ID :507:

CAGGAGAAGG AAGGTTGTAC GTGGACACTA TAAAGGTCAG CAAATTGCAA 50

AGTAGTCCAG GTTTACAGGA AGAAATATGT TATCTACATC GAACGGGGCA 100

GCGGGAAAAG GCTAATGGCA CAACTGTCCA CGTAGGCATT CACCCCAGCA 150

AGTGGTTATC ACTAGGCTAA AACTGACAAA GACCGCAAAA AAACCTTCAA 200

CNGAAAGCCA AATGTNCCAG CCGGAAAGGA AAGNGCATAC AAGGAAGAAA 250

CCATTGAGAA GATGCAGGAG TAAAGTATTA T 281

(2) INFORMATION FOR SEQ ID :508:

(i) SEQUENCE CHARACTERISTICS:
(A) LENGTH: 180 base pairs
(B) TYPE: nucleic acid
(C) STRANDEDNESS: double
(D) TOPOLOGY: linear

(xi) SEQUENCE DESCRIPTION: SEQ ID :508:

AGGGNTGCTA ANNTATTGGT GGGCAGGAGG CATCGCTGAT GATCTTGAGG 50

CTGTTGTCAT ACTTCTCATG GTTCACACCC ATGACGAACA TGAGGCATCA 100

GCAGAGGGGA CAGAGATGAT GACCCTTTCG CTCCCCCCTG CAAATGAGCC 150

CCAGCCTTCT CCATGGTGGT GAAGACGCCA 180

(2) INFORMATION FOR SEQ ID :509:

(i) SEQUENCE CHARACTERISTICS:
(A) LENGTH: 104 base pairs
(B) TYPE: nucleic acid
(C) STRANDEDNESS: double
(D) TOPOLOGY: linear

(xi) SEQUENCE DESCRIPTION: SEQ ID :509:

AAATGCAAAA CTCACCGTGC ACACTCCTAG ATCCCTGCCA CAAAGAAATC 50

TTTGAAAAAT GAAGTCTTCC TTTCGGACAA TATACCATTN GAGTTTCTCT 100

ATTT 104

(2) INFORMATION FOR SEQ ID :510:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :510:

GTNNATACAC AACTAAGTTC AAATAAAAAA ACCAAATNAA AAATNGGCAG 50

GGAAGCTAGA GCCAGAATCA GGAAAATCTG TTTCCTCGTC CCCAGACTCC 100

CGCCAAGCCT ACTCCACTAA CTAACANNGA CTCTATCAAG TTTCTATCAA 150

GACTTGCATC TGNATCTTGN A 171

(2) INFORMATION FOR SEQ ID :511:

(i) SEQUENCE CHARACTERISTICS:
(A) LENGTH: 255 base pairs
(B) TYPE: nucleic acid
(C) STRANDEDNESS: double
(D) TOPOLOGY: linear

(xi) SEQUENCE DESCRIPTION: SEQ ID :511:

ACTGTACCTA TCATCCTGAA AAACTTTATG GGGGAGAAAG GTCAGCAGCT 50

TCTCTTTCTT TTNATCGAAA ATAATAAAAC TGCGTATTCT ACTTTAACTA 100

AATGTAAGGA AGAAAATATA CAAGCCCATA TTTAATGTAT TTCTATNCGA 150

GCAACAATAG TTCATATGTT CATGTTTGCT ACTATCACAA TTCAACATAT 200

GAACACAGAT CAGCTCTATA CCATGAATAC TGCTGGAAGT GATGGTTTAG 250

GATTA 255

(2) INFORMATION FOR SEQ ID :512:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :512:

GAAAGATTGG ACATGATTGC GTTTATAAGA ATGAGAGTGT TAAATTGGAT 50

TTCTTGCTTT ATTTGTGACA TTTCAGTTTA TTAGAAATCA TGTTACCATT 100

AGAAAAATTG AAGTTTCCTA GTAACAAAGT AATTTGATTT GTGTAACTTG 150

ATAAAAGATT TACTGACTTA AGCTTTTGTT TTTTTTCATA AGCTGCTTTT 200

GAGCTTTGTC 210

(2) INFORMATION FOR SEQ ID :513:

(i) SEQUENCE CHARACTERISTICS:
(A) LENGTH: 222 base pairs
(B) TYPE: nucleic acid
(C) STRANDEDNESS: double
(D) TOPOLOGY: linear

(xi) SEQUENCE DESCRIPTION: SEQ ID :513:

CTGTACAATC ATCCTGCAGA AAATTGTTTT GGAGAATTCT TGGTAATTGA 50

AGACCAGCAG AGCACCCCTC CCCACCCGCC CCGTAAAAGT GCTTACAATG 100

AACAGGGATT CTTTTCTTTA CAAAAGACCC AAAGATACGT GGACAAAAAA 150

AGAAAAGCTT GAAGTCTCAA TGCCTAATGT GTGCACATAA AACAGGCACG 200

AAGAAACAAA CGTGTGCATC CT 222

(2) INFORMATION FOR SEQ ID :514:

(i) SEQUENCE CHARACTERISTICS:
(A) LENGTH: 240 base pairs
(B) TYPE: nucleic acid
(C) STRANDEDNESS: double
(D) TOPOLOGY: linear

(xi) SEQUENCE DESCRIPTION: SEQ ID :514:

GCAGGAGAAG GAAAAGACAG CAACTCATCC CAGAATTGCC NAATGAAGAT 50

GAGGAGAATC CCCTCAAAGG GATCTGTGTG CTTACAGTGG TGACAGTGAC 100

AATGAGGAGG ACTGATGAGA GACTCAAGAG TGAGGAAGAG AGCTAGCTGA 150

CTGNAAGAAA TGACCTGTCT NNTCTNCAGC GCCATTCCCN AACAGATGCC 200

CTAGTCAGGA CCACAACTCT TAAACCTCAT AGCNAAATAT 240

(2) INFORMATION FOR SEQ ID :515:

(i) SEQUENCE CHARACTERISTICS:
(A) LENGTH: 183 base pairs
(B) TYPE: nucleic acid

(C) STRANDEDNESS: double
(D) TOPOLOGY: linear

(xi) SEQUENCE DESCRIPTION: SEQ ID :515:

CAATGCCCAA TCTGAGTGTA TACACATCTT AGGAAAAATA ATCTAANGTA 50

ACTTTTGAGG GTGAGAGNGG AAATAAGAGA TCACATTTAT TCAAGACTGA 100

TCCCTATNAG GAAGGAGAGG CCCAGGCACA GATACCACAA AAGAGCACAG 150

TACCCAGCTG TCCTGGNATT GNTTGAGTGT AAG 183

(2) INFORMATION FOR SEQ ID :516:

(i) SEQUENCE CHARACTERISTICS:
(A) LENGTH: 215 base pairs
(B) TYPE: nucleic acid
(C) STRANDEDNESS: double
(D) TOPOLOGY: linear

(xi) SEQUENCE DESCRIPTION: SEQ ID :516:

TCGCAGCAGA AGGAAGTCGT TGACCTGGCA GCCAAGGGAA CACACAAACA 50

CACTCACACA CACATGCACT CACCTGCATA CACACACACT CATACACACG 100

AACACTCATA CACACANGCT TGTGCACACA TGTTCATGCA CATGCATGCA 150

CTCACACTCA TACAAACGCA CATTTAAACA CGTGTGNACA NTGTACTCAG 200

ACACACACAG GTGTG 215

(2) INFORMATION FOR SEQ ID :517:

(i) SEQUENCE CHARACTERISTICS:
(A) LENGTH: 181 base pairs
(B) TYPE: nucleic acid
(C) STRANDEDNESS: double
(D) TOPOLOGY: linear

(xi) SEQUENCE DESCRIPTION: SEQ ID :517:

TCGCAGAGAA GGAACAATCA GGGCCATGAC AGGAACCGCA AAGAACCAGA 50

GGGTATAGGC AGCAAAGAAG GGCACATAAA AAGGCTGCTT CTCAGGAAAG 100

TGTCGCAGTG AGACAAACAC ACATACAGAC CACACACAGA CCACCACCTC 150

AATCATGGGC CCTAGCCGNC CTNGTAATAC G 181

(2) INFORMATION FOR SEQ ID :518:

(i) SEQUENCE CHARACTERISTICS:
(A) LENGTH: 260 base pairs
(B) TYPE: nucleic acid
(C) STRANDEDNESS: double
(D) TOPOLOGY: linear

(xi) SEQUENCE DESCRIPTION: SEQ ID :518:

ATGTAACTCA ATCCATCCGC AAGAAAACCA AACCAACAGA AAAGGAAGCT 50

GAAGACATGG ACATCGCAAG CCACGCGGTA ATGCATACTT GGCACAGAGT 100

AGCCAATATA GAAGACGTGT GCCTCACACG GTTCACTTTG TTCATCAATA 150

AAAGAATATA AAAATCTCGT TCACCCAGTG GTAAGTGTAT TAAAATAGAT 200

CTGTATCATA CACACAGTTT CTCCCGGAGT CGTGAGAATG ACAGGAGGGA 250

CCTGGCAACT 260

(2) INFORMATION FOR SEQ ID :519:

(i) SEQUENCE CHARACTERISTICS:

(A) LENGTH: 115 base pairs

(B) TYPE: nucleic acid

(C) STRANDEDNESS: double

(D) TOPOLOGY: linear

(xi) SEQUENCE DESCRIPTION: SEQ ID :519:

AAGCTAATAC AATGGTCATT TCCAGACAAA TTTAAAGGAA ACACTAAGGC 50

TGCTTCAAAG ATTATCTGAT TCCTTTAAAA TATATGTCTA TATACACAGA 100

CATGCTCTTT TTTTA 115

(2) INFORMATION FOR SEQ ID :520:

(i) SEQUENCE CHARACTERISTICS:

(A) LENGTH: 175 base pairs

(B) TYPE: nucleic acid

(C) STRANDEDNESS: double

(D) TOPOLOGY: linear

(xi) SEQUENCE DESCRIPTION: SEQ ID :520:

CANGTGGCTT CAATTAAACA ATNAGGAGCC TCNNAACATC CTGTCGCAGA 50

AACTCCCAAT ATAAACGCCC CCANACACTA ACACAAAACA GCCTTATTAA 100

CCAGATAAGT TCTCCACTAC CACTCCTAGA TTTGATGTAA CCCTGAATNT 150

GACTNATAGN TNGACCCACC TGTGA 175

(2) INFORMATION FOR SEQ ID :521:

(i) SEQUENCE CHARACTERISTICS:
(A) LENGTH: 136 base pairs
(B) TYPE: nucleic acid
(C) STRANDEDNESS: double
(D) TOPOLOGY: linear

(xi) SEQUENCE DESCRIPTION: SEQ ID :521:

ATGGAATCAA ACAGCTCTAT AATGAAGATA ATGTCTCAGA AAATGTGGGT 50

TCTGTGTGTG GCACTGATTT ATCAAGACAA GAGGGACATG CTTCCCCTTG 100

TCCACCTTTG CAGCCTGTTT CTGTCATGTA GTTTCA 136

(2) INFORMATION FOR SEQ ID :522:

(i) SEQUENCE CHARACTERISTICS:

(A) LENGTH: 200 base pairs
(B) TYPE: nucleic acid
(C) STRANDEDNESS: double
(D) TOPOLOGY: linear

(xi) SEQUENCE DESCRIPTION: SEQ ID :522:

AATGGAGCAA TTCATCCAGT TCTTCTAGAA ACAGCTCAGA ATCAAAGCTG 50

GATATATTTT GTGTCTTCTG TGACTGTTCA TTCATGGAAG GAAGCAGACT 100

GCTTTGGGCA GAATTATTCT CCTGACTACT TGAGCTAGTA GACTAGGAAC 150

TATTCCATAA GAGGAAATCC TGTAAGTCTT AAATCCCCAC TGGAGAAAGC 200

(2) INFORMATION FOR SEQ ID :523:

(i) SEQUENCE CHARACTERISTICS:
(A) LENGTH: 204 base pairs
(B) TYPE: nucleic acid
(C) STRANDEDNESS: double
(D) TOPOLOGY: linear

(xi) SEQUENCE DESCRIPTION: SEQ ID :523:

AGTAAGGTTT TGCATCCTTT GATCAGCAGG TAACTGACGA ATTCTTGAGT 50

CGAAGATTAT ACCTTGATGA GCTTTGATGA GCTCTTGCAA ACGAATGAGA 100

CCAGTGCTGT CATCATACAC AACCATGACG TTTTCCACTT GAAAAACTGC 150

ACCAGGTCTA AAATGCACGC TGAGTGAAGA GAAGTCTGGG CAGAGACTGA 200

CATA 204

(2) INFORMATION FOR SEQ ID :524:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :524:

ATATCTGTCT CATCATCCCA AGGTTTCACA TCTAGTAAGA TGGAAGACTT 50

GGCAACAAGT GCAGGTTTTT TGGCTTTCTT TGATTCATAT TGTGCAAGAC 100

GTTCTTCCCT CAGCCTCTTT GCTTCTTCAC TTNNTCTAAN ATAATCCAAA 150

GAGGTCAATG TATCATCA 168

(2) INFORMATION FOR SEQ ID :525:

(i) SEQUENCE CHARACTERISTICS:
(A) LENGTH: 291 base pairs
(B) TYPE: nucleic acid
(C) STRANDEDNESS: double
(D) TOPOLOGY: linear

(xi) SEQUENCE DESCRIPTION: SEQ ID :525:

AAACTGTTCT TTAAAAGGCC TCTCCTGGTT ATTAAGCCAG GAAGAGAAGT 50

AGAATCTCGA ATCACCTAAN GGAAATGGTG ACACAGGTTG TCCTTTCTCA 100

GCCGTTGGTT TCCTTTCATC TCTGAAGGCC TGTAGTACCA TGAGGAAAAC 150

ATTTAATTTA GAGGGTGAAC CCAACAGTAG GAAGCTGAAA GCAGAAGTGT 200

TTATCTCCCT CTGCATTCAG ACCAGGCTCC TTAGTGCACT CATCAGACTA 250

TCGCTGCCCC TGCTGTCTGC TGTTGAGCCT TCACCACCAC T 291

(2) INFORMATION FOR SEQ ID :526:

(i) SEQUENCE CHARACTERISTICS:

(A) LENGTH: 207 base pairs

(B) TYPE: nucleic acid

(C) STRANDEDNESS: double

(D) TOPOLOGY: linear

(xi) SEQUENCE DESCRIPTION: SEQ ID :526:

CATCCGCGTG CTGGACCCCT TCACCATCAA GCCCCTGGAC AGAAAACTCA 50

TTCTCGACAG CGCTCGTGCC ACCAAGGGCA GGATCCTCAC CGTGGAGGAC 100

CATTATTATG AAGATGGCAT TGGTGAGGCT GTGTCCNNNN GTAGTGGGCG 150

AGCCTGGCAT CACTGTCACC CACCTGGCAG TTAACCGGGT ACCAAGAAGT 200

GGGAAGC 207

(2) INFORMATION FOR SEQ ID :527:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :527:

AGCATTCCGT AAGGACGGAC GTGTTCAAGG ACAACTTTGA TGAGATGGAC 50

AGGTCTAGGG AGGTTGTTCA GGAGCTCATT GATGAGTACC ATGCGGCCAC 100

CCAGCCAGAC TACATTTCCT GGGGCACCCA GGAGCAGTGA TTTCCCTCCC 150

CACTACTTCT TTNCTTAGAT GGTAACCACA GCCTCGACCA TGCCTGCTCC 200

CTCTGACCCA GCTTCACC 218

(2) INFORMATION FOR SEQ ID :528:

(i) SEQUENCE CHARACTERISTICS:
(A) LENGTH: 229 base pairs
(B) TYPE: nucleic acid
(C) STRANDEDNESS: double
(D) TOPOLOGY: linear

(xi) SEQUENCE DESCRIPTION: SEQ ID :528:

AACCTNATGA CTCTCCATCC CCTTGAACCA AACATCTAGC ACTCAGCTCC 50

AGCATATTTC ACCATTCAAC CCGAAATTCA CAAACGCTAC TTGTCGACTT 100

GTAACCAATT TACTCAGCAA GTGCTGACTC CTTAACGGAT CATCCCCATC 150

CTNCGCTGCA AGGTGACTCA CTAAAATCAT NTGTTAACAC CAACATTATT 200

TTTACACCCA GTGTGTAAGC CAGAAGGGC 229

(2) INFORMATION FOR SEQ ID :529:

(i) SEQUENCE CHARACTERISTICS:
(A) LENGTH: 117 base pairs
(B) TYPE: nucleic acid
(C) STRANDEDNESS: double
(D) TOPOLOGY: linear

(xi) SEQUENCE DESCRIPTION: SEQ ID :529:

ATATTATTCA TCATCCCAAG GTCACATCTA GTAAGATGGA AGACTTGGCA 50

ACAAGTGCAG GTTTTTGCTT TCTTTGATTC ATATCGTGCA AGACTTCTTC 100

CTTAGCCTCT TTGCTTC 117

(2) INFORMATION FOR SEQ ID :530:

(i) SEQUENCE CHARACTERISTICS:
(A) LENGTH: 179 base pairs
(B) TYPE: nucleic acid
(C) STRANDEDNESS: double
(D) TOPOLOGY: linear

(xi) SEQUENCE DESCRIPTION: SEQ ID :530:

TCGCAGCAGA AGGAAGCTGA TCCACCATCC GGACAACCCG AACCCAAGCT 50

GAAGACGAGA AATGATCCAG AAAGAATGTG CTGCAATCCG GTCATCTTTT 100

AGAGAAGAAG ACAATACACA CCAATGTCGA AATGTGGCAA AATTACTGTA 150

TATGCACATG CTGGTGCTAC CCTCTCACT 179

(2) INFORMATION FOR SEQ ID :531:

(i) SEQUENCE CHARACTERISTICS:
(A) LENGTH: 204 base pairs
(B) TYPE: nucleic acid
(C) STRANDEDNESS: double
(D) TOPOLOGY: linear

(xi) SEQUENCE DESCRIPTION: SEQ ID :531:

AAAGCATTCA AGTAAGAATA TGGCAATAAA AAACAAAAAT ATCTTCTCAG 50

CATTCAAAAC AAAACGCATA AGTCATTCCT AACTTNAGAG CTTTATAGCA 100

TTTTCCTAGA CAGGAAGGGA AAAAACAGTT AGCATTTAAA AGTCCGGAAA 150

GCTTTTTCGT TGNNTTAATT ACATCAACNN TCTGCCTTTG TCCAAATCCC 200

TTAT 204

(2) INFORMATION FOR SEQ ID :532:

(i) SEQUENCE CHARACTERISTICS:
(A) LENGTH: 183 base pairs
(B) TYPE: nucleic acid
(C) STRANDEDNESS: double
(D) TOPOLOGY: linear

(xi) SEQUENCE DESCRIPTION: SEQ ID :532:

AAAGGAGCTC AGAACTTCAG CTTGAGCAGG AAGAGGAGGA CGTGCCAGAC 50

CAGGAACAGA GCAGCAGCAT CGAGACCCCA TCAGAGGAGG CGGCCTCTCC 100

CCACAGCTGA GGGGCTGGGC TAGGGGTGGG TGGAGCCCTT TTAAAATACC 150

CTTTCTTCAA AACTTAGCTC TGAATGGAGA AAC 183

(2) INFORMATION FOR SEQ ID :533:

(i) SEQUENCE CHARACTERISTICS:
(A) LENGTH: 287 base pairs
(B) TYPE: nucleic acid
(C) STRANDEDNESS: double
(D) TOPOLOGY: linear

(xi) SEQUENCE DESCRIPTION: SEQ ID :533:

GAAACAAGTT CTCGCTCATC CCTGCATTTC TGCCAACTTC AGCTTGCAAT 50

ATTTATACTC CAGACTATTT TCATCAGACA AAAACCAGTA AGCAGGGTCC 100

TCTTTGAGAA GAGTTCTCTC TTTGGGAGAC AGGCTGCCTT CGATGACACG 150

TTTCACAAGC TGGTTGATGG TGCCCACTAC CCGTGATCTG CTCGCTGGGG 200

GACAGCATCA CTCAGACTAC TTGGAGCCTT GCCTGAATTT CAGGTTTCGT 250

AGGAGGAATA ATTTTCTCCT TCTNTNGTAT CGNCTCT 287

(2) INFORMATION FOR SEQ ID :534:

(i) SEQUENCE CHARACTERISTICS:

(A) LENGTH: 154 base pairs

(B) TYPE: nucleic acid

(C) STRANDEDNESS: double

(D) TOPOLOGY: linear

(xi) SEQUENCE DESCRIPTION: SEQ ID :534:

AGCCACTGCC CCTCTTGTCT ACGTATTCCC AAAATTAAAC TTTGATGCCT 50

GACTTTTTGC AGTCAGTTTT AAGTGAGCTC CCTGAGGTGC CAAGGCCATG 100

GTGTCCCCCT GCTGCGTCTG TTCGTCAGCT GAGTTCTTGT GAATCTNTGT 150

TTAG 154

(2) INFORMATION FOR SEQ ID :535:

(i) SEQUENCE CHARACTERISTICS:

(A) LENGTH: 212 base pairs

(B) TYPE: nucleic acid

(C) STRANDEDNESS: double

(D) TOPOLOGY: linear

(xi) SEQUENCE DESCRIPTION: SEQ ID :535:

GATTACCCTA TATCTACAAT TNGAGGTAAA ATAGAAGCAA CACATAAAAG 50

GGCCTATTTC TGCTACCATG TCATATAATT CTCCATAGTG AATATTGTGA 100

TAAAGCTACT GAAAACTATG CCTCACAGAG CCTAGCTTCT TGTAGAGCTG 150

GTATTTTACA ACTCGCATTG CTTGGAAATC TCAACACACG TAAGACTCTC 200

CTAGGAAGGC AC 212

(2) INFORMATION FOR SEQ ID :536:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :536:

GGAAGCTCAA TGTCCGGCAG GTCAATGCTT CNCGGACACG GATCATTTTT 50

ATCTGATTCC AGCCTGCTTG CAACCCTGGA ATCCTCTTGT TCCCTGCTGC 100

CTGCCCCTTG GGAAGGNACA GTGATGTCTT TAGGGGAAGG AGGAGCCCCT 150

NTCGGCAGTT GTCTTACT 168

(2) INFORMATION FOR SEQ ID :537:

(i) SEQUENCE CHARACTERISTICS:
(A) LENGTH: 147 base pairs
(B) TYPE: nucleic acid
(C) STRANDEDNESS: double
(D) TOPOLOGY: linear

(xi) SEQUENCE DESCRIPTION: SEQ ID :537:

CGGATCATTT TTATCTGATT CCAGCCTGCT TGCAACCCTG GAATCCTCTT 50

GTTCCCTGCT GCCTGCCCCT TGGGAAGGNA CAGTGATGTC TTTAGGGGAA 100

GGAGGAGCCC CTNTCGGCAG TTGTCTTACT AGGNNNNTAA TGAAGTA 147

(2) INFORMATION FOR SEQ ID :538:

(i) SEQUENCE CHARACTERISTICS:
(A) LENGTH: 250 base pairs
(B) TYPE: nucleic acid
(C) STRANDEDNESS: double
(D) TOPOLOGY: linear

(xi) SEQUENCE DESCRIPTION: SEQ ID :538:

GTGGAATCTC AATAATGACA CAAGGTACCA ACTGCCAGCA TTTCTGCGAG 50

GCAATCCTGC TCTTAATCTG CAAGATGGAC CCTTCTGCAG AGAGATTGCT 100

GTGGGTGATT CTAAGGACAG ATTGTTATAT ACGATGTGGG AGAGCAGATT 150

GCTGTTCCCC GCAATGATGA ATGGGCACGG TTTGGCCGAA CACTTGCAGA 200

AATNAATGTA AACCCNAACT GATGTAGAGA GGACGCAGCT GCCCNAATAC 250

(2) INFORMATION FOR SEQ ID :539:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :539:

AGAAGGTAAG GGAGTGGGAG GCAGGTGGGT GTTCTGGAGG GGGTATCCTT 50

GTGCTCTAAG GGTGCTATGT TCGATGCTGG TGTTTCGGGG ATGGTGAATG 100

CCCTTCTTTA ANNTTAGAGG GAAATCCAAA CCAATAGGCC CCAAGGTTGC 150

CAGTGGGATA GGGGTGTAAA AAAGTAAATT GGGC 184

(2) INFORMATION FOR SEQ ID :540:

(i) SEQUENCE CHARACTERISTICS:
(A) LENGTH: 154 base pairs
(B) TYPE: nucleic acid
(C) STRANDEDNESS: double
(D) TOPOLOGY: linear

(xi) SEQUENCE DESCRIPTION: SEQ ID :540:

AACTTATAGA AAAGTAAAGG AAACCCCAAC ATGCATGCAC TGCCTTGTGA 50

CCAGGGAAGT CACCCCACGG CTATGGGAAA TTAGCCCGAG GCTTANCTTT 100

CATCATCATG TCTCCCAGGG NGTGCTTGCA AAGAGATATT CCGCCAAGCC 150

AGAT 154

(2) INFORMATION FOR SEQ ID :541:

(i) SEQUENCE CHARACTERISTICS:
(A) LENGTH: 175 base pairs
(B) TYPE: nucleic acid
(C) STRANDEDNESS: double
(D) TOPOLOGY: linear

(xi) SEQUENCE DESCRIPTION: SEQ ID :541:

AATTGAATTC TTAAGAAGCT GTCAAATATG GCAGTCTTTT GATGTTAGTA 50

ATTTTGTTTT CTTCTGTGTT ATTGGTTCAA AGTACTGGCC TTTTCCTTCA 100

TTTCCAGTAA TTATTTTATA ACTATCACTT TTAATTGAGT GGAAATTAGA 150

TGATTTGGTT ATACTGTGAA ACAGC 175

(2) INFORMATION FOR SEQ ID :542:

(i) SEQUENCE CHARACTERISTICS:
(A) LENGTH: 221 base pairs
(B) TYPE: nucleic acid

(C) STRANDEDNESS: double
(D) TOPOLOGY: linear

(xi) SEQUENCE DESCRIPTION: SEQ ID :542:

AAGTACCTTT TCCTGCAGCT GCCCGTATGC CTGAGTGACT AAGGGGCAGT 50

CGTGAGAGGC AGAGTCCAAG ATCTCATTGG TCGTTTCCAG ACTGCCGTCC 100

AGCCGTGCTG CTTCATCAGG GCACACTCGC CGCCCTCCTG GGGCCAGGTT 150

GCACATGTAC AGGTACCCGT CGGCGCACCC ACCAACAACG CGGTCTTCTG 200

AATCGCGACT GGCNAACAGA T 221

(2) INFORMATION FOR SEQ ID :543:

(i) SEQUENCE CHARACTERISTICS:
(A) LENGTH: 186 base pairs
(B) TYPE: nucleic acid
(C) STRANDEDNESS: double
(D) TOPOLOGY: linear

(xi) SEQUENCE DESCRIPTION: SEQ ID :543:

AAGTAGATAG CTTGCATCCT GACACCGTGG CAAAGTTAAG AAAGTTGAAG 50

GAGAAACATA CCTTGAGAGG GGGTTTTCTT TAAAACTAGT GTTAGAAGCT 100

TAGGGATTTT TTTTTTTATT CCTTACTAAC TTTCACCCAG AACCGCTCTA 150

TTTGACTTGT GCCGACATTG CAAACTTTNT GACAGG 186

(2) INFORMATION FOR SEQ ID :544:

(i) SEQUENCE CHARACTERISTICS:
(A) LENGTH: 153 base pairs
(B) TYPE: nucleic acid
(C) STRANDEDNESS: double
(D) TOPOLOGY: linear

(xi) SEQUENCE DESCRIPTION: SEQ ID :544:

AGCATCCTTG CCACCTCCCC ACCCGGGAGT CAAGGGTCGT GGTTCTGCCT 50

TGAACAGGCC ACAGCCGTAG CTGTAGAGAG GCCAGTGGTA CATCAGCCCA 100

CCGACAGGAG GAGGAGCCCT GGCTTGAGGG AAGGGGAAGC CCAGGCCTGT 150

GCC 153

(2) INFORMATION FOR SEQ ID :545:

(i) SEQUENCE CHARACTERISTICS:
(A) LENGTH: 161 base pairs
(B) TYPE: nucleic acid
(C) STRANDEDNESS: double
(D) TOPOLOGY: linear

(xi) SEQUENCE DESCRIPTION: SEQ ID :545:

GATTCAGCTC CAGCATCCTT GCCACCTCCC CACCCGGGAG TCAAGGGTCG 50

TGGTTCTGCC TTGAACAGGC CACAGCCGTA GCTGTAGAGA GGCCAGTGGT 100

ACATCAGCCC ACCGACAGGA GGAGGAGCCC TGGCTTGAGG GAAGGGGAAG 150

CCCAGGCCTG T 161

(2) INFORMATION FOR SEQ ID :546:

(i) SEQUENCE CHARACTERISTICS:
(A) LENGTH: 188 base pairs
(B) TYPE: nucleic acid
(C) STRANDEDNESS: double
(D) TOPOLOGY: linear

(xi) SEQUENCE DESCRIPTION: SEQ ID :546:

AATAGCCCTG AGGTCATCCT GCAAAGTGCG TATCAAAAAA TACGAAGTTA 50

GGGTGACAAA GTTTGACAGT GATGTTATAC AAGTCAAACT TGGAAGGTCA 100

TAGTAAGCAT ACCTATGCTG AGAGAAAGCA TCAAATCCTT TGTGTACACA 150

TTTAGTTTTA TTGTAACAAA GCAACTTGTA CACTTTTA 188

(2) INFORMATION FOR SEQ ID :547:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :547:

ATNCCTTCTC CATCCANTTA GTTANCAGAA ACTAATCAAA AGAAAGTCTG 50

ACAACTGCAC TCCCCCTTGC ATGCCATTCT CTCAAGCCCA TAATCTTGGA 100

GTATCCACAA CGTGCGAAGG CCTACCCTTT GTGTGTACTC ATCTCACGTT 150

TACGTATTTT GTNGTTGAGG AGCTCCTCTA CAAATGTTGC GTATCTTCCG 200

AATCACTCAT TTAGAAAA 218

(2) INFORMATION FOR SEQ ID :548:

(i) SEQUENCE CHARACTERISTICS:
(A) LENGTH: 46 base pairs
(B) TYPE: nucleic acid
(C) STRANDEDNESS: double
(D) TOPOLOGY: linear

(xi) SEQUENCE DESCRIPTION: SEQ ID :548:

GGAGGAGACC ATCAGNCCCG TGAAGACCAC TCCTGACGTC TCGTGT 46

(2) INFORMATION FOR SEQ ID :549:

(i) SEQUENCE CHARACTERISTICS:
(A) LENGTH: 146 base pairs
(B) TYPE: nucleic acid
(C) STRANDEDNESS: double
(D) TOPOLOGY: linear

(xi) SEQUENCE DESCRIPTION: SEQ ID :549:

AGGGGGCTAA NGGTTGGGGG CAGGAGGCAT TGCTGATGAT CTTGAGGCTG 50

TTGTCATACT TTTCACGGTT CACACCCACG ACGACACGGG GACTCAGCAG 100

AGGGGCAAGA CACGACCTTT AGTTTCCCCC TTGCGATAAN CTTCNC 146

(2) INFORMATION FOR SEQ ID :550:

(i) SEQUENCE CHARACTERISTICS:
(A) LENGTH: 221 base pairs
(B) TYPE: nucleic acid
(C) STRANDEDNESS: double
(D) TOPOLOGY: linear

(xi) SEQUENCE DESCRIPTION: SEQ ID :550:

AAATATNGAN TATCCATCCC CTCAAGCATT TATCCTTTGT GTTACAAACA 50

ATCCAATTAT ACTCTTTCAG TTATTTTAAC ATGTACAATT AAATTATTAT 100

TGACTCTAGT CACCTTGTTG TGCGAGCAAG TACTAGGTCT TATTCATTCT 150

TTCTAACTAT TCCAGGCCCT TTTTAATCAA GAAGGCTCCC TAGACCAAAA 200

TTTTAAAAAG ACAATGCTAG G 221

(2) INFORMATION FOR SEQ ID :551:

(i) SEQUENCE CHARACTERISTICS:
(A) LENGTH: 116 base pairs
(B) TYPE: nucleic acid
(C) STRANDEDNESS: double
(D) TOPOLOGY: linear

(xi) SEQUENCE DESCRIPTION: SEQ ID :551:

AAACATCGTT TATTCATCCA GCAGTGTTGC TCAGCTCCTA CCTCTGTGCC 50

AGGGCAGCAT TTTCATATCC AAGATCAATT CCCTCTCTCA GCACAGCCTG 100

GGGAGGGGGT CATTGT 116

(2) INFORMATION FOR SEQ ID :552:

(i) SEQUENCE CHARACTERISTICS:
(A) LENGTH: 150 base pairs
(B) TYPE: nucleic acid
(C) STRANDEDNESS: double
(D) TOPOLOGY: linear

(xi) SEQUENCE DESCRIPTION: SEQ ID :552:

CTCATCCTCT CCTATTACAT AGTGAAGCCC ATGNCAAATA GGAAGAAGCT 50

CAGTATCGCT CCTCCCACCA TAACCCCCCT TAATGCCTCC TGAACCATAG 100

TTNCCTCCAC TATATATCCC CCCATGTTCC TGCTACCCCA AGTTTTCACT 150

(2) INFORMATION FOR SEQ ID :553:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :553:

ACTTATTGAA ATAGCAGACA TTTCGCTAGA CACTCCAATT AACCTGATAT 50

GAGGCGCTAA TCAATAAAAA AAGTTAAACA TTTGCATGAC TCTACTATGG 100

GAATAAATTA CCTGTTTAAT ACCTCGACTT TTTATAGAAA AATAATGATG 150

NCCAAGGTAA ACCAGGTAAC CCAGGCCTGT GAACTATAT 189

(2) INFORMATION FOR SEQ ID :554:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :554:

ACTTATTGAA ATAGCAGACA TTTCGCTAGA CACTCCAATT AACCTGATAT 50

GAGGCGCTAA TCAATAAAAA AAGTTAAACA TTTGCATGAC TCTACTATGG 100

GAATAAATTA CCTGTTTAAT ACCTCGACTT TTTATAGAAA AATAATGATG 150

NCCAAGGTAA ACCAGGTAAC CCAGGCCTGT GAACTATATG CCTGGAAC 198

(2) INFORMATION FOR SEQ ID :555:

(i) SEQUENCE CHARACTERISTICS:

(A) LENGTH: 97 base pairs

(B) TYPE: nucleic acid

(C) STRANDEDNESS: double

(D) TOPOLOGY: linear

(xi) SEQUENCE DESCRIPTION: SEQ ID :555:

CAAGCTGAGG AGCAAGGAGA GCCAGTCTGA GTCCCAAAAC TGAAGAACTT 50

GAGTCTGATG TTCGAGGGCA GGAAGCACCC AGCACAGGAG AAAGATG 97

(2) INFORMATION FOR SEQ ID :556:

(i) SEQUENCE CHARACTERISTICS:

(A) LENGTH: 269 base pairs

(B) TYPE: nucleic acid

(C) STRANDEDNESS: double

(D) TOPOLOGY: linear

(xi) SEQUENCE DESCRIPTION: SEQ ID :556:

TCTGCTGTCT GTCGCAGGAG AAGGAATGTC AGAAACTTCA TCCTCTTGTT 50

GGGGAATGCA CCCTCNTGAG TAGGCTGACC CATGAGGCTG TGGGAATTGA 100

GTCTTAGGAC ACAGAGACCA GGGTGTTGAA TTTTCTTCCC TGCCCCTAGG 150

CTGTTCAGGT CTTCCTGCAG CAGTCAGGGC TGCAAGCCCT GGAAAGGCAT 200

CAAAAGAGGC CCAGCTCCAG GATCGTGTGT ATGCTCTCCA GCAGACAGCT 250

CTTCAAGGGT GATCTTGTT 269

(2) INFORMATION FOR SEQ ID :557:

(i) SEQUENCE CHARACTERISTICS:

(A) LENGTH: 245 base pairs

(B) TYPE: nucleic acid

(C) STRANDEDNESS: double

(D) TOPOLOGY: linear

(xi) SEQUENCE DESCRIPTION: SEQ ID :557:

ACTCCCTCAA GGTCGTGCGT CTAAGCCTAC AAGAAAGTTT GCCTATCTGG 50

ACCCTGNCTC ACGAGGTTGG CTGGAAGTAC CAGGCAGTGA CAGCCACCCT 100

GGAGGAGAAG AGGAAAGAGA AAGCCAAGAT CCACTACCGG AAGAAGAAAC 150

AGCTCATGAG GCTACGAAAC AGGCCGAGAA GAACGTGGAG AAGAAAATTG 200

ACAAATACAA AGGTTCTCAA GACCCACNNA CTCCTAGNTT NAGCC 245

(2) INFORMATION FOR SEQ ID :558:

(i) SEQUENCE CHARACTERISTICS:

(A) LENGTH: 183 base pairs

(B) TYPE: nucleic acid
(C) STRANDEDNESS: double
(D) TOPOLOGY: linear

(xi) SEQUENCE DESCRIPTION: SEQ ID :558:

GAATTTAAAA AAAAAGAAAG AAAGAAAGAA AGGTTCCATC TTAGATTCTC 50

ACAACCTTCG TTCCGCAGTT CATTAATCCG ACTCTGATGC TAAGGTGACA 100

GTGTATGTAA GTAGATTTTT GTTTTCAGTG AAGGAGACCT GGGAAAAGAT 150

GGATTTCTCT CTGTATCTTA AGAGTTATCA GAT 183

(2) INFORMATION FOR SEQ ID :559:

(i) SEQUENCE CHARACTERISTICS:
(A) LENGTH: 116 base pairs
(B) TYPE: nucleic acid
(C) STRANDEDNESS: double
(D) TOPOLOGY: linear

(xi) SEQUENCE DESCRIPTION: SEQ ID :559:

GTCATCTTTG GAAAGTGAGA TAACCAAGGA AATAATCGAA GGAGTTAGGG 50

AGATGATTAC GTGATAATGA CTTTGGGCGC TTAACCTTTG ATCCCGGGTA 100

TGCTNAAGAA GCTGAC 116

(2) INFORMATION FOR SEQ ID :560:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :560:

CCTTGATGAT ACCATTATCC TCATTATAGA TGATGCACGG GCCCCTGCGC 50

TGGATACGGC GACGGTTTCT CATTTGCCTT TGTCAGCTCT CATTCGCTGA 100

GAGGCATAGA CCTTTTTGAT ATCATCAGGC TTTCCGTTTT TAGGAGCAAA 150

ACAGCTTCTT 160

(2) INFORMATION FOR SEQ ID :561:

(i) SEQUENCE CHARACTERISTICS:
(A) LENGTH: 101 base pairs
(B) TYPE: nucleic acid
(C) STRANDEDNESS: double
(D) TOPOLOGY: linear

(xi) SEQUENCE DESCRIPTION: SEQ ID :561:

GCAGTTGGCA GGTGCACTAT CCCAGATGGG CCACTAATAG AAAGTTCCGC 50

AAATGCACCC CGTTCCCCTG TGCGAGATCG TTTGAATNAG ACCAGAAACT 100

G 101

(2) INFORMATION FOR SEQ ID :562:

(i) SEQUENCE CHARACTERISTICS:
(A) LENGTH: 183 base pairs
(B) TYPE: nucleic acid
(C) STRANDEDNESS: double
(D) TOPOLOGY: linear

(xi) SEQUENCE DESCRIPTION: SEQ ID :562:

ATAAGTTATA GCAAATACAG TCTTCACAGA TTTGAGTAAC TTTATTCGAT 50

TTTATAGTGA TTTCTTAAGG CCTATATCCA ATGAAACCAT TTCCAAGCTC 100

TATGAGGAGT GGAATTTTAG ATGTCTATTA CATTNGTCTT TTAAAAGAAA 150

AATGCTTAAC NNCTAGAATG AGCAAGATTA CTT 183

(2) INFORMATION FOR SEQ ID :563:

(i) SEQUENCE CHARACTERISTICS:
(A) LENGTH: 187 base pairs
(B) TYPE: nucleic acid
(C) STRANDEDNESS: double
(D) TOPOLOGY: linear

(xi) SEQUENCE DESCRIPTION: SEQ ID :563:

AGAGACACGT GAAATTCATA TCTCAAANNC ACAGAGCTGA GACTTTGGGC 50

CTAAATACTG TACCACTGGT TCCCTGAACC AAGGAAGAAA AGTGTCGGTA 100

AAGGCCCGTT AAGACAAGAT GGCAAGGAAA AGCACCTTAA ACAATGGTAA 150

GATTTATGTT AGATCAGTGG TAAGAGTTTC TAGTGAC 187

(2) INFORMATION FOR SEQ ID :564:

(i) SEQUENCE CHARACTERISTICS:
(A) LENGTH: 213 base pairs
(B) TYPE: nucleic acid
(C) STRANDEDNESS: double
(D) TOPOLOGY: linear

(xi) SEQUENCE DESCRIPTION: SEQ ID :564:

GGGACCCCAA ATCACATCCA GAATACTAGA GTGAGGCAGC TGCAACATGA 50

CACAGAAAAA TGGAATCAGG ATTTAGGGGA ATTGGATTTC AGTGCCTATT 100

GAGACACGAT CTAGGAAGCC TACCACTTTG GCTGCTCACT GTATGCACAC 150

AACCCNANNA NAATNGATGA AAACAAGAAT GTACAGCATG CTCCTAACAC 200

AANGTGACTA TTC 213

(2) INFORMATION FOR SEQ ID :565:

(i) SEQUENCE CHARACTERISTICS:
(A) LENGTH: 167 base pairs
(B) TYPE: nucleic acid
(C) STRANDEDNESS: double

(D) TOPOLOGY: linear

(xi) SEQUENCE DESCRIPTION: SEQ ID :565:

TCCATCTGAC ATCGCATTTC CATAGAAATG GCCAAAGAAA GAAGGTCCTG 50

GGGTTTTTCA TAGAAAGCTC AAAAAGTTCA ACCTTGATGC TATCCCCCAG 100

CCCAATACAA AATACACAGA AAAAGCAATT ATTAAAATAC TGGCTTCGGT 150

TTCTTTTTTT CCTTTAA 167

(2) INFORMATION FOR SEQ ID :566:

(i) SEQUENCE CHARACTERISTICS:
(A) LENGTH: 128 base pairs
(B) TYPE: nucleic acid
(C) STRANDEDNESS: double
(D) TOPOLOGY: linear

(xi) SEQUENCE DESCRIPTION: SEQ ID :566:

GAAGATACAG AACCATCCGT GAAAATCATT TAGCACTGGA GACCTTCTTT 50

GTATTACTTC CTNGTTACTA NGACCTCTAA TTCAATGGGG CCCTGCTGGT 100

TTGTCGATGA ATTGAGCAAC TGAGAACG 128

(2) INFORMATION FOR SEQ ID :567:

(i) SEQUENCE CHARACTERISTICS:

(A) LENGTH: 202 base pairs
(B) TYPE: nucleic acid
(C) STRANDEDNESS: double
(D) TOPOLOGY: linear

(xi) SEQUENCE DESCRIPTION: SEQ ID :567:

GGAGGCTCGG GAGTAGCATC CTCAGGAGTA GTGTAGCGAG CAAATTTGGA 50

AAGTAGTCCT CAATCTTCGA TTTCCCAGCA AGGACTTTCT CAGCNAGCGA 100

TCTCGNTTGT TGAGGAACGG ATCAAGAGAT GNNNGTNTAG CTGTNNTGTT 150

GTTTAGATGT CTTGAAGAGG TTAGAGCCTC CTGTAGGCGG TTGGNNTGGG 200

NG 202

(2) INFORMATION FOR SEQ ID :568:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :568:

ATGTCATGAT GCCTAACTCA TACACTCTTT GCCTCAAATA TAATTACTAA 50

AAACAAATAT AGTATAAACA TTAAACAAAT GAACAATAAT CATCAATAGA 100

CGGGTTACTT TCAAGGAAGA GTTGTTTTGT GACAAATTCT ACTCTTGATC 150

TA 152

(2) INFORMATION FOR SEQ ID :569:

(i) SEQUENCE CHARACTERISTICS:
(A) LENGTH: 181 base pairs
(B) TYPE: nucleic acid
(C) STRANDEDNESS: double
(D) TOPOLOGY: linear

(xi) SEQUENCE DESCRIPTION: SEQ ID :569:

CAGGTCCGCG TGCTCTCCGC ACCACCCCAC TTCATTCCGG CCAAACCAAC 50

CGCACCCCTG AATTTCTCCG CAAATTTCCT GCCGGCAAGG TCCCAGCATT 100

TGAGGGGATG ATGGATTCTG TGTGTTTGAG AGCAACGCCA TTGCCTATTA 150

CGCGAGCAAT GAGGAGCTGC GGGGAAGTAC T 181

(2) INFORMATION FOR SEQ ID :570:

(i) SEQUENCE CHARACTERISTICS:
(A) LENGTH: 157 base pairs
(B) TYPE: nucleic acid
(C) STRANDEDNESS: double
(D) TOPOLOGY: linear

(xi) SEQUENCE DESCRIPTION: SEQ ID :570:

ATCCCTTGGG AGTTTATTCT CCTGGTAAGC TGTAATTGCA TATCCAGTTT 50

AACTGGACTG GGCTGTGTTG GGCGAGGATC NGCAGGGTTT TTTCCTCNNT 100

NGAAAGATGA AATAGATTNT TGAGCACTGG NTGCAGAGCC AAAATGCNTA 150

ATGCTTT 157

(2) INFORMATION FOR SEQ ID :571:

(i) SEQUENCE CHARACTERISTICS:

(A) LENGTH: 193 base pairs

(B) TYPE: nucleic acid

(C) STRANDEDNESS: double

(D) TOPOLOGY: linear

(xi) SEQUENCE DESCRIPTION: SEQ ID :571:

GGCACTAAAG CCTTTAATAA TACGAGATGA AATGCAACNG TGNNATGACA 50

AGTAAGTGAG CCTGACCTGG CATTGCCTCG CCTCACCGCT GGCTTTGACC 100

AGGGTATGAT CTTTAACTTT TCTGAGCTGA TTTGATCGTG GTCTTTACAC 150

ACAGGTGGTC GTTCCTGTTT GGACACTGTT TTATTTGTTT GAC 193

(2) INFORMATION FOR SEQ ID :572:

(i) SEQUENCE CHARACTERISTICS:

(A) LENGTH: 179 base pairs

(B) TYPE: nucleic acid

(C) STRANDEDNESS: double

(D) TOPOLOGY: linear

(xi) SEQUENCE DESCRIPTION: SEQ ID :572:

TCGCAGGAGA AAGAGGTTTT CAGAGGCCCT GAGGACATGG CTGCCCTTGA 50

GAAGGATTTG AGGAGGTTGG TGGAATTTGT TGAAGGAGAG GGCGAGGAAG 100

AAGGAGAGGA TACTAAAGTT AAAACGTCAC AAGGTGTGCT TTTAAGGGAG 150

CTTTCCTGTT TTAAACATGA AAGTGTGGC 179

(2) INFORMATION FOR SEQ ID :573:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :573:

AGGACCTCTA AGACATCCTT ATGACGACAG TTTTGTCCAA GGGGATATCC 50

ACAGAGTACC TTGTGGCATT AGGTGATTGT AGTCATACAC TTTAAAAAGA 100

TTTTATTTCT GATCTTTTGG CGATCTTCTT CTTGCCCATG NNGCTGTTAC 150

TTNGCNCGGG NAGCGGTTAA TTCTANCCGC TAGGTGTGC 189

(2) INFORMATION FOR SEQ ID :574:

(i) SEQUENCE CHARACTERISTICS:
(A) LENGTH: 262 base pairs
(B) TYPE: nucleic acid
(C) STRANDEDNESS: double
(D) TOPOLOGY: linear

(xi) SEQUENCE DESCRIPTION: SEQ ID :574:

GTGCCTTCTA GGTTTTGAAC TTCTATGCAT TAGCGCAGAT GTGGAATGCG 50

TAAAGGTGTT CATAGTTTGA CTGTTTCTAT GATGTTTTTT CAAAGAATCG 100

TCCTTTTTTG AACTATAATN CCCCNCGGTT ATTTTACCAT CACAGTTTAA 150

ATGTATATCT TTTACGTCTC TACTCAGACC ATATTTNAAA GGGGCGCCTC 200

ATTATGGGGC AGAGAACCTT TTAATAAGTC TCATTAAGAT CTGAATTTTG 250

GTTCTAAGCA TT 262

(2) INFORMATION FOR SEQ ID :575:

(i) SEQUENCE CHARACTERISTICS:
(A) LENGTH: 56 base pairs
(B) TYPE: nucleic acid
(C) STRANDEDNESS: double
(D) TOPOLOGY: linear

(xi) SEQUENCE DESCRIPTION: SEQ ID :575:

CCAAGGAACC ATCTGCGCCG CAAGCCAGAC CCCACAAGAC CTAGNTTGGT 50

CCTGAC 56

(2) INFORMATION FOR SEQ ID :576:

(i) SEQUENCE CHARACTERISTICS:

(A) LENGTH: 158 base pairs

(B) TYPE: nucleic acid

(C) STRANDEDNESS: double

(D) TOPOLOGY: linear

(xi) SEQUENCE DESCRIPTION: SEQ ID :576:

GCGAGTCTCA AAGAGTAGAG GAGCGTCTAC TATCTTTCAA CTCCGATCTT 50

CTGATGNCGG ACTTTACCGT GACAGCGAAG TGGTATTGTA CGTCCAGGCC 100

CGCCAGCCAC TGTCTTCATG CAGGAACCAC AGTGCCAGAT CCCCACAGCT 150

CGTATCTT 158

(2) INFORMATION FOR SEQ ID :577:

(i) SEQUENCE CHARACTERISTICS:

(A) LENGTH: 255 base pairs

(B) TYPE: nucleic acid

(C) STRANDEDNESS: double

(D) TOPOLOGY: linear

(xi) SEQUENCE DESCRIPTION: SEQ ID :577:

GGACCTTGAC CCACATCCAT GTTGAGGAAT GTCCTCTTGT CAAGGTCAGG 50

GAACAGCACC CACAGAGGGC CTCTGGGTCC CTCTCTGCTC AACTCCCTCT 100

CTCTCGGTTC CTGCGAGGCT CATAGGGTGC AGGGCCCAGC AGAAGGACTG 150

AGTCTTCCTC CTGGACTTCT GGTCCTGGTA GGCTGTGCTT CATGCTCTCC 200

TGTCACCTGT ACTGTAAGGA ACTATTATGA CAAACGCATA AAGAATATGA 250

CTTTG 255

(2) INFORMATION FOR SEQ ID :578:

(i) SEQUENCE CHARACTERISTICS:
(A) LENGTH: 270 base pairs
(B) TYPE: nucleic acid
(C) STRANDEDNESS: double
(D) TOPOLOGY: linear

(xi) SEQUENCE DESCRIPTION: SEQ ID :578:

GAGGAGTCCA TGCCATCCTT GATGGAAAAG AAACTGAAGA GAAAAGACAG 50

CCTGTGGAAG AAGCTCAAAG GTTCTTTGAA GAAGAAGAGA GAAAATATGA 100

CATGATATCT TTGCTTTTGA GTTCCTCACG CTCTCTGAAT TTATTAGTTG 150

GACAATTCCA TATGCAGCAT TCTGCTTCAA TATANCTCTT NNGGTCTCTC 200

TCTCTNNAAT ATTTGCCTGT AGGTAAAAGC AAGCTCTGCA TATCTGTACC 250

TCTTGAGATA GTTTTGTTTT 270

(2) INFORMATION FOR SEQ ID :579:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :579:

GAGTTTTCAG AGGCCCGTGA GGACATGGCT GCCCTTGAGA AGGATTATGA 50

GGAGGTTGGG TGGATCTGTT GAAGGAGAGG GNAGAAGAGG AGAGGAATGC 100

TAAAGTTAAA ACGTAATAAA GATGCTGCTC TTACGGAAG 139

(2) INFORMATION FOR SEQ ID :580:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :580:

GGCAGGAATA CATATAGTCA TCATTGCCAG ACTTAATATG AGAGGTGAAA 50

TGTTCGATCC AATTTATTTC TTGGATAAGT TTTTCTTTCC TATNCCTNTN 100

GTTTTGATAA TATAATAAAG AAGATGAGGG GCCCAAATCA TATAGAGCTC 150

CTGAGNGAGT TTTNGGAG 168

(2) INFORMATION FOR SEQ ID :581:

(i) SEQUENCE CHARACTERISTICS:
(A) LENGTH: 242 base pairs
(B) TYPE: nucleic acid
(C) STRANDEDNESS: double
(D) TOPOLOGY: linear

(xi) SEQUENCE DESCRIPTION: SEQ ID :581:

CGGAGGGCCC TGTTTGGGAA AAATAGGATT TTAAAAATAT GGTTCATTAA 50

TTTAGGTTTT CTAACATCTA CTTGGGGATG TAGCCTCCAG TGAGGTCAGT 100

TAAGTGGGAC AGAAACGGCA GAGGGAAGAG GTCTTTGCTT CCCCTGGGCC 150

CATTCTCCCT GGCTGCCAGC CCTTGAAGTC AGAACACCAT GGGAAAATTC 200

AGGAGTCGGC ACTGTAGCCG TCAAGTGGCG CTACTTTCCA CA 242

(2) INFORMATION FOR SEQ ID :582:

(i) SEQUENCE CHARACTERISTICS:
(A) LENGTH: 37 base pairs
(B) TYPE: nucleic acid
(C) STRANDEDNESS: double
(D) TOPOLOGY: linear

(xi) SEQUENCE DESCRIPTION: SEQ ID :582:

GCATTTTTCT TGTGTGCTGT TTATAATAGC AAAGCAG 37

(2) INFORMATION FOR SEQ ID :583:

(i) SEQUENCE CHARACTERISTICS:
(A) LENGTH: 179 base pairs
(B) TYPE: nucleic acid
(C) STRANDEDNESS: double
(D) TOPOLOGY: linear

(xi) SEQUENCE DESCRIPTION: SEQ ID :583:

GGAACAAAGA AAATGTACAG AGTTATATGC GCTTTTTTTT GGTATGGGGG 50

ACAAGAAACA CTTACCAACA AAAATATTTC AACAACCCCA AAATAACTTA 100

CTCACAAATA TGCAAAATTA TCTATGGCAT AGTATTTCGC ACTCGATGAC 150

ATTTAGAGAT AAAAAATCAA ATGGAGCTT 179

(2) INFORMATION FOR SEQ ID :584:

(i) SEQUENCE CHARACTERISTICS:
(A) LENGTH: 186 base pairs
(B) TYPE: nucleic acid
(C) STRANDEDNESS: double
(D) TOPOLOGY: linear

(xi) SEQUENCE DESCRIPTION: SEQ ID :584:

AACTGCCATG AAGTAACCTG AAGGAGGCGC TGACTGGAGG GATTGATTAC 50

AGGATCGGAA CACTCCACAC TCGCCATTCT CTGCATATAC CGGTTAGCGA 100

GGCGAGCCTG GCGCTCTTCT TCGCGCTGAG CTAAAGCTAC ACACAATGCT 150

TTGCGACCAC AATNCACCCT TCATTTCGTA ACTGCT 186

(2) INFORMATION FOR SEQ ID :585:

(i) SEQUENCE CHARACTERISTICS:
(A) LENGTH: 180 base pairs
(B) TYPE: nucleic acid
(C) STRANDEDNESS: double
(D) TOPOLOGY: linear

(xi) SEQUENCE DESCRIPTION: SEQ ID :585:

ACCCACGGTA CTTACATCCT ATGATATGGC CTGCAAACTA AACTACAAAC 50

GCACTCACAT CGCTATAATC CTTTTAAGGA CTTAAACTTT ACTCCATTAA 100

GACTTTTATG ACTTCTAACA ACCTCGCCAA CCTCCTCACC CCCCACTATA 150

CCTCGGAGAA CTTTCGCGTA ATAACCACGT 180

(2) INFORMATION FOR SEQ ID :586:

(i) SEQUENCE CHARACTERISTICS:
(A) LENGTH: 183 base pairs

(B) TYPE: nucleic acid
(C) STRANDEDNESS: double
(D) TOPOLOGY: linear

(xi) SEQUENCE DESCRIPTION: SEQ ID :586:

ACCGACATCA CGANNGACTT GGTCTTAGTT GAGCAATTTG GCTAANNNNN 50

NTNCTNNTTA GCACGTTCTG AGTCTGTGGG ATAGCTGCCA TGAAGTAACC 100

TGAAGGAGGT GCTGGCTGGT AGGGGTTGAT TACAGGGTTG GGAACACTCG 150

AGANTGGCAT CCTGCATATA CTGGTTAGTG AGG 183

(2) INFORMATION FOR SEQ ID :587:

(i) SEQUENCE CHARACTERISTICS:
(A) LENGTH: 280 base pairs
(B) TYPE: nucleic acid
(C) STRANDEDNESS: double
(D) TOPOLOGY: linear

(xi) SEQUENCE DESCRIPTION: SEQ ID :587:

AGGTCAAGTC TACAGCTGGA GACACCCACT TGGGNGNANG AAGATTTTGN 50

ACAACCGAAT NGTCAACCAT TTTAATTGCT GAGTTTAAGC GCACNTTAAA 100

GAAGGACATC AGNGAGAACA AGAGAGCTGT AAGACGCCTC CNTACTGCTT 150

GTGAACGTGC TAAGCGTACC CTCTCTTCCA GCACCCAGGN CAGTATTNAG 200

NTCGNTCTNT CTATGAAGGA ATCGACTCTN TACTCCATAC CNNNCCGATT 250

GAGACTGATG TGACNTTCCT GGGACTGNCA 280

(2) INFORMATION FOR SEQ ID :588:

(i) SEQUENCE CHARACTERISTICS:
(A) LENGTH: 371 base pairs
(B) TYPE: nucleic acid
(C) STRANDEDNESS: double
(D) TOPOLOGY: linear

(xi) SEQUENCE DESCRIPTION: SEQ ID :588:

GGTGAAGAAA CTCCAGATAT CAAGGAATTG GGAAATCCTG GGCCAAACCA 50

CCCCAAGATG ATTACACTGA AATGTAGTAT TAGTACTNCT GCCAGATCTC 100

TTTTTAACAT CATGTGCGTC TCTTGGGATC CAGCAAAAGT GTTAAGCCAC 150

AATGCCCTTG TGCCTTTTAA TATACCACAG TGCCAGTTAA ACTAATATTT 200

TNTTTTGTTG CTTTTTGGGG TATTTTCATT AGTATTTCAG CAAATCTCAT 250

GATAAAGGNC AAGGNCAAGA ACTNCAGAGN ACTGAGCAGA GAGGCTNGTG 300

ATGAAANGTG AAGGCTTCNA CTGACTTTAN GCAGTGGCAG TCANGNTACT 350

GNGNNGCANG CTTANCTATG A 371

(2) INFORMATION FOR SEQ ID :589:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :589:

GAGAGAGAAC ACTCCCCTCC ATCCCAGCAC TATGCACAGT TCACGGCTCA 50

TATGCAAAGT GGAAGACACG TGGGACAAGA GCAAAGCACA AGTGACACAT 100

GGTCCCTCTC TAACACCTCA GCACACCAAC CCTGACGCTC CCATCACAGA 150

TGCTGNTCAT TCTTNCACGG NCCCCTTTTA TAAT 184

(2) INFORMATION FOR SEQ ID :590:

(i) SEQUENCE CHARACTERISTICS:
(A) LENGTH: 243 base pairs
(B) TYPE: nucleic acid
(C) STRANDEDNESS: double
(D) TOPOLOGY: linear

(xi) SEQUENCE DESCRIPTION: SEQ ID :590:

GGGGGCCCGC CGTNCACNCC CCCACACTCT TGGTAGGCAA TGCTTGTCCC 50

NATATAGTTG NNGTCCTATC GAGTGACACT CTCGTTCATG GATANGGTGN 100

GTAAAACCCN TNGTNGCATC CNNATTGGGN GCANTGNGCC TNTCCCCTTN 150

AANGGTTTTT GCNTNCACTC GACCTNGGGA GGATTCAATG NACNNNCTNG 200

CANTGGNCAA GCNTTGNTTG CNNGNATTGA GAACCCNCCA ATT 243

(2) INFORMATION FOR SEQ ID :591:

(i) SEQUENCE CHARACTERISTICS:
(A) LENGTH: 247 base pairs
(B) TYPE: nucleic acid
(C) STRANDEDNESS: double
(D) TOPOLOGY: linear

(xi) SEQUENCE DESCRIPTION: SEQ ID :591:

ATTCGCCCNN TAAGTAAGTC GTNATTANAC GCGACGNCTA CTACTGAGAC 50

NCGCATGCGC TCTCTCTACA CTAAAGCTCG TCGCTNGNTN ACTTGCGNGN 100

NAAAAAAACC CCCTGGGNNC GCTTTTCACC CCCAAACTTT CAAATTCCGC 150

CCCTTTNGGC NANGCCCAAC CANNCCCCCC CCCTTTTTNC CGNCCCANNC 200

TTNGGNCNTA ANNATTNAGN CGGNANGNNN GGGCCCCCGG CCAACCC 247

(2) INFORMATION FOR SEQ ID :592:

(i) SEQUENCE CHARACTERISTICS:
(A) LENGTH: 425 base pairs
(B) TYPE: nucleic acid
(C) STRANDEDNESS: double
(D) TOPOLOGY: linear

(xi) SEQUENCE DESCRIPTION: SEQ ID :592:

GGAGGGGAGT AGAGGAAATT TTCATTCTGG AAAAAAATGC ATACTGTTTG 50

ATATATTACT CNTCATGCTT TCCACAGATA TTATACACAG ATATTATATT 100

CCANGGATTA CGTTGCAATG TCTTCAAAAA TAGANAATTC ATTTTATATT 150

TCTNGATGAA ATATAATAGT ANCTNNGCTA CCTTTGGGAA TGTGACAAAA 200

TACTATGATG ATTACAACTC ATTAAAGCAT AAATNTGCAT GATTTAACTN 250

CATGTTCCTT CTATGANCTN CGTGGNATAT AGGCATATTT ATTAATGCTA 300

TTTANGGCNT NNGTGCTTTG TAATGATTCG NCNTTAGGTG AAGGGNTACT 350

TTTNTNNTNC TTCNTAGTAG ATTNGNTTNN NTCTTTTTAA GAGGANTCNA 400

NTTTCATGNG TAANCATCAT CTTTT 425

(2) INFORMATION FOR SEQ ID :593:

(i) SEQUENCE CHARACTERISTICS:
(A) LENGTH: 258 base pairs
(B) TYPE: nucleic acid
(C) STRANDEDNESS: double
(D) TOPOLOGY: linear

(xi) SEQUENCE DESCRIPTION: SEQ ID :593:

CGCTGCATGC GTCAGCGCNA CGCGACGACA GCGCGCGCGA CGCGCGCGAC 50

GCACAAAANA AATGCATGCC AACACGAANA TATGTGCACA CAAACGCAAA 100

CGCGTGTGAA CACATGCGCG CNNCGCGCNC GCGATNCAAA GCTGAAATGT 150

GCNNGNCNGT CGTGNGCGNA AATGTGAAAT GAACAAACAA CAATGAATGA 200

ATGAATGTGA AAAAGAGNGN GNTTGAAAAT TNTANAGNNC CCCCCCNTNA 250

ANCAAAAG 258

(2) INFORMATION FOR SEQ ID :594:

(i) SEQUENCE CHARACTERISTICS:
(A) LENGTH: 215 base pairs
(B) TYPE: nucleic acid
(C) STRANDEDNESS: double
(D) TOPOLOGY: linear

(xi) SEQUENCE DESCRIPTION: SEQ ID :594:

GACCCTAACA ATATGTACAA AAATATAAAA TGTAAATAAA AAATACAAAC 50

AAATTTCCTT TTTAAAGTAC TTTTAAGAAA AAAAGCAGGG CCTTGGAAGT 100

TTTGGTTCTT TTTTCCTCCC CTGTTGCAAA TTCTCATGGT TTGGGTTGGG 150

TGGTGGAGAG CGCGTGTCAT CTGCGGGTGC CTGCCCACGT GGGCGGGCGG 200

CTCTCTACTC GAAGG 215

(2) INFORMATION FOR SEQ ID :595:

(i) SEQUENCE CHARACTERISTICS:
(A) LENGTH: 272 base pairs
(B) TYPE: nucleic acid
(C) STRANDEDNESS: double
(D) TOPOLOGY: linear

(xi) SEQUENCE DESCRIPTION: SEQ ID :595:

GGGGCTGGTT TGGTCATCCG AGATCATTAA AAATGGCTGA CCCTAACAAT 50

ATGTACAAAA ATATAAAATG TAAATAAAAA ATACAAACAA ATTTCCTTTT 100

TAAAGTACTT TTAAGAAAAA AAGCAGGGCC TTGGAAGTTT TGGTTCTTTT 150

TTCCTCCCCT GTTGCAAATT CTCATGGTTT GGGTTGGGTG GTGGAGAGCG 200

CGTGTCATCT GCGGGTGGCA CTGCCACGGT GGGCGGGCGG GCTCTCTACT 250

CGAAGGTGAC CACGTTTAGA TT 272

(2) INFORMATION FOR SEQ ID :596:

(i) SEQUENCE CHARACTERISTICS:
(A) LENGTH: 250 base pairs
(B) TYPE: nucleic acid
(C) STRANDEDNESS: double
(D) TOPOLOGY: linear

(xi) SEQUENCE DESCRIPTION: SEQ ID :596:

GACAAACTGT TGACACCCGG AGGCCTAAAC GAGGATTTCA GCTTCCATTA 50

TGCCCAACTC CAGTCCAACA TCATTGAGGC GATTAATGAG CTGCTAGTGG 100

AGCTGGAAGG GACAATGGAG AACATTGCAG CCCAGGCTCT GGAGCACATT 150

CACTCCAATG AGGTGATCAT GACCATTGGC TTCTCCCGAA CAGTAGAGGC 200

CTTCCTCAAA GAGGCTGCCC GAAAGAGGAA ATTCCATGTC ATTGTAGCAG 250

(2) INFORMATION FOR SEQ ID :597:

(i) SEQUENCE CHARACTERISTICS:
(A) LENGTH: 225 base pairs
(B) TYPE: nucleic acid
(C) STRANDEDNESS: double
(D) TOPOLOGY: linear

(xi) SEQUENCE DESCRIPTION: SEQ ID :597:

CTGCCAAATA CTTTCTTCAC CAACTCATGA GGAGAGGGAA CATGCTGAGA 50

AACTGATGAA GCTGCAGAAC CAACGAGGTG GCCGAATCTT CCTTCAGGAT 100

ATCAAGAAAC CAGACTGTGA TGACTGGGAG AGCGGGCTGA ATGCAATGAG 150

TGTGCATTAC ATTTGGAAAA GATGTGAATC AGTCACTACT GGAACTGCAC 200

AAACTGGCCA CTGACAAAAA TGACC 225

(2) INFORMATION FOR SEQ ID :598:

(i) SEQUENCE CHARACTERISTICS:
(A) LENGTH: 210 base pairs

(B) TYPE: nucleic acid
(C) STRANDEDNESS: double
(D) TOPOLOGY: linear

(xi) SEQUENCE DESCRIPTION: SEQ ID :598:

ACAAAACGCA GATATAAAAA AGTTACAAAG ATTTTTAGAT TTTCATTCAC 50

AAAAAAAGTC ATTCACATTT TACACTATAC ACGTTATGAT ATAATACAGG 100

AAAGTATTAT GTGCATTGTA AGAGAAGGAA AATAGAACTA CTAGATCACA 150

CGTGTTGTTC TGTGCTCTAA AATACCTAAA GGTGGATTCA TTTAATGCAA 200

CACCAGGGAC 210

(2) INFORMATION FOR SEQ ID :599:

(i) SEQUENCE CHARACTERISTICS:
(A) LENGTH: 116 base pairs
(B) TYPE: nucleic acid
(C) STRANDEDNESS: double
(D) TOPOLOGY: linear

(xi) SEQUENCE DESCRIPTION: SEQ ID :599:

AAAGGAGTTG AGTACTGTAA ACGCAGAGCT ACATAGAAAA AACGGGCTTC 50

AAAAATCTGC ACAGAGGTTT GCTTGAGAAT TTAGCTACAC AAATATGTGT 100

AGAAGTGAAA AAAAAA 116

(2) INFORMATION FOR SEQ ID :600:

(i) SEQUENCE CHARACTERISTICS:
(A) LENGTH: 107 base pairs
(B) TYPE: nucleic acid
(C) STRANDEDNESS: double
(D) TOPOLOGY: linear

(xi) SEQUENCE DESCRIPTION: SEQ ID :600:

ACAGTGTGTG CCCTGTCAGC TCCGCAAATT GGCAGTCACT ACGTTTGTGC 50

CCCCTGTAAC CTTGTGATCT TCACTGCCAC TAGCGATGAA GTCTTCATTA 100

TGGCCTC 107

(2) INFORMATION FOR SEQ ID :601:

(i) SEQUENCE CHARACTERISTICS:
(A) LENGTH: 173 base pairs
(B) TYPE: nucleic acid
(C) STRANDEDNESS: double
(D) TOPOLOGY: linear

(xi) SEQUENCE DESCRIPTION: SEQ ID :601:

ACTGCTAGTG AAATTCACAT GACTCACAAT TCCCTTTAGC CAATGTTACC 50

GAAGTCAGTG TCAAGAAAAC TTAACAGAAA AAAAAAAGCA CAGAGTGAGT 100

TCCTACCATA AAAATCCAGG CTGCCCTGTT TCCTAGCTCT AATATAAGCC 150

ATTTTCTTTC CTTAGTGTAC CAA 173

(2) INFORMATION FOR SEQ ID :602:

(i) SEQUENCE CHARACTERISTICS:

(A) LENGTH: 216 base pairs

(B) TYPE: nucleic acid

(C) STRANDEDNESS: double

(D) TOPOLOGY: linear

(xi) SEQUENCE DESCRIPTION: SEQ ID :602:

GGAAGAGAAC ACATACACGA GGACCAGTAC CTGATGAGGA CAAGAGAGAT 50

GGGAANNNGC TGTGGAATTC CTTTCGGCAC CCTGGATGTT AACCCCTGCT 100

CAGGAAAGGG TGCATCTGTC TTCATCATGC CTCTCTCTCC TCCTCCTCCA 150

GCCACCTCCC AAAGGCAGAG CTGCCGCAAC CTGCCTGGCC ATGTGGTGGC 200

AAGTACCCAG TAGGAG 216

(2) INFORMATION FOR SEQ ID :603:

(i) SEQUENCE CHARACTERISTICS:

(A) LENGTH: 213 base pairs

(B) TYPE: nucleic acid

(C) STRANDEDNESS: double

(D) TOPOLOGY: linear

(xi) SEQUENCE DESCRIPTION: SEQ ID :603:

GAAAAAACAA TCATGACAGC AACTCTCCTA ACCACAAAAA TCACATATGT 50

TATCTTTCTT TCAGGACTAA TAATTAATAT TTAAGAGGAA AGCACATCAA 100

TTTCTAGGGC CCTTCTTGGG GAAAGGTTCA TATAATTTAG CATACATACA 150

TATTCAGTGA ATGCATTCAT ATATTACTAT ATAAACACTC TCAGCTACAA 200

TGGTAATATA ATG 213

(2) INFORMATION FOR SEQ ID :604:

(i) SEQUENCE CHARACTERISTICS:
(A) LENGTH: 219 base pairs
(B) TYPE: nucleic acid
(C) STRANDEDNESS: double
(D) TOPOLOGY: linear

(xi) SEQUENCE DESCRIPTION: SEQ ID :604:

GACATCTGAT CATCTCACTG GACGCCACAG GGGCAAGAGG GTGGTTTTCC 50

TGAAGCAGCT GGCTAGTGGC TTATTACTTG TGACTGGACC TCTGGTCTCA 100

ATCGAGTTCC TCTACGAGAA CACACCAGAA ATTTGTCATT GCCACTTCAA 150

CAAAATCGAT ATCAGCAATG TAAAAATCCC AAACATCTTA CTGATGCTTA 200

CTTCAAGAAG AAGAGCTGC 219

(2) INFORMATION FOR SEQ ID :605:

(i) SEQUENCE CHARACTERISTICS:
(A) LENGTH: 104 base pairs
(B) TYPE: nucleic acid
(C) STRANDEDNESS: double
(D) TOPOLOGY: linear

(xi) SEQUENCE DESCRIPTION: SEQ ID :605:

AGCGAACACG AGCACAGCGA GCGCACAGAG AGCAGCAGGG AGGGCAGCCA 50

CCGCCGGGAC GCCGGCAGGG GCACGAGAAG GCAAGGAGCG GACACCCGNG 100

NNGA 104

(2) INFORMATION FOR SEQ ID :606:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :606:

AATAAAGCAT TCTCACAACC TTTGTTGGGT CAATGATTCC TTTTTACCAC 50

ANNTACAGAA TCTCCACCAT AGCATCATAA CCAACTCTGA GGAACTTCGC 100

ATAATTCTTA ACTACAAAGA TCCTTCAACA CCCGCATTCT TACAATGTCA 150

TCGCCGGAAT TTTGAGTGTT CTTTCAATAA CCTACANTA 189

(2) INFORMATION FOR SEQ ID :607:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :607:

GGAAAAGNTC ATTACATAAG CACCAAGNCA TTGATTATGA TCCACCGGAA 50

GAGCTCGTAT TTATCCTTTG CTTTNATNTG AGACCAAGCT AGCCCTGAGT 100

AATTTTANNT GGTTCCTAAA ACATATGGCT TATCGTACTC TAAGAAAAAT 150

GCCTTACGCA CATTCCTTTN T 171

(2) INFORMATION FOR SEQ ID :608:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :608:

GGAAAAGTNC GCATATAGCG TGCNATAATA CTACGNCTAA GGAGAAACAA 50

TTTCCTACAC ATAAAGTAGT GAGAACATCA TCCTATAACA GGGAACTGTG 100

ATTATTTAAA AAACGCAGAA CTTATTTATT TGGCTTTAGA AATAACTGCA 150

TACAATGTCA TAAGTCGAAA AGAACTCAAA ATAA 184

(2) INFORMATION FOR SEQ ID :609:

(i) SEQUENCE CHARACTERISTICS:

(A) LENGTH: 191 base pairs

(B) TYPE: nucleic acid

(C) STRANDEDNESS: double

(D) TOPOLOGY: linear

(xi) SEQUENCE DESCRIPTION: SEQ ID :609:

GGGGACAGCT NNNNNGTTGT TTTGGAGCCT GTTGACTTTG TATTCTCTGC 50

CTGTGATTTT CNNTTCTAAA TGAAACTCCA TGTNNNAACC AGGACGAAGN 100

TGAGAAGGAA AACGCCAAAT GCTTTGGTTA TTAGAGNTTA ATAGGNAAGC 150

TCTGTTACAC TAGGTGTAGA GTTCCAGAAT GTTCTTTTGT T 191

(2) INFORMATION FOR SEQ ID :610:

(i) SEQUENCE CHARACTERISTICS:

(A) LENGTH: 172 base pairs

(B) TYPE: nucleic acid

(C) STRANDEDNESS: double

(D) TOPOLOGY: linear

(xi) SEQUENCE DESCRIPTION: SEQ ID :610:

GCAACTGTGC AAACATCCAT CAGTGTGGAG GAATGGTAAA CTTGGTACAT 50

GCATGCANTN GNNNCATATT TTTGTGGTTA AGATNNTGAT GTATAGGCAT 100

GGAATGTTAT CAAAAGCACA TTAAGTGGTC AAAGCCAGAT ACAGAAGAGT 150

AGGTATGATT TTATAGGNAT AA 172

(2) INFORMATION FOR SEQ ID :611:

(i) SEQUENCE CHARACTERISTICS:
(A) LENGTH: 51 base pairs
(B) TYPE: nucleic acid
(C) STRANDEDNESS: double
(D) TOPOLOGY: linear

(xi) SEQUENCE DESCRIPTION: SEQ ID :611:

TCCACCTTAC AGACCTGATT TGGCTGCTTC TGACGTCTGT TTCCTAATCT 50

T 51

(2) INFORMATION FOR SEQ ID :612:

(i) SEQUENCE CHARACTERISTICS:
(A) LENGTH: 60 base pairs
(B) TYPE: nucleic acid
(C) STRANDEDNESS: double

(D) TOPOLOGY: linear

(xi) SEQUENCE DESCRIPTION: SEQ ID :612:

CGCCTGAAAC TTTGAGGATA AACTTTTTCA AAAAAATAAA ACAGTATCTC 50

TTAATCACTG 60

(2) INFORMATION FOR SEQ ID :613:

(i) SEQUENCE CHARACTERISTICS:
(A) LENGTH: 75 base pairs
(B) TYPE: nucleic acid
(C) STRANDEDNESS: double
(D) TOPOLOGY: linear

(xi) SEQUENCE DESCRIPTION: SEQ ID :613:

TTGAAGCTGG GAGGCAGAGG TTGCAGCGTG CAGAGTCGTG CCACTGCACT 50

CCTGGGCGCA CAGCGAGACT GTCTC 75

(2) INFORMATION FOR SEQ ID :614:

(i) SEQUENCE CHARACTERISTICS:
(A) LENGTH: 25 base pairs
(B) TYPE: nucleic acid
(C) STRANDEDNESS: double
(D) TOPOLOGY: linear

(xi) SEQUENCE DESCRIPTION: SEQ ID :614:

ACGGGATTCT CTTCTTCGGC CGCCA 25

(2) INFORMATION FOR SEQ ID :615:

(i) SEQUENCE CHARACTERISTICS:
(A) LENGTH: 72 base pairs
(B) TYPE: nucleic acid
(C) STRANDEDNESS: double
(D) TOPOLOGY: linear

(xi) SEQUENCE DESCRIPTION: SEQ ID :615:

GCAGTGTACT ATGTTCGCAT CTGTGAATAG CCACTGCACT CTAGCCTGGA 50

TAATATAGTG AGAACCCATC TC 72

(2) INFORMATION FOR SEQ ID :616:

(i) SEQUENCE CHARACTERISTICS:
(A) LENGTH: 137 base pairs
(B) TYPE: nucleic acid
(C) STRANDEDNESS: double
(D) TOPOLOGY: linear

(xi) SEQUENCE DESCRIPTION: SEQ ID :616:

TCTAACTGAT TTCAAAGCAA ACTCTCTCTT AATTAGGCTG CCTCTCCAGG 50

GGAAATTTAG TGGCAGGGTC CCAGTGAGCC TGTAAGAAGT GTTCTACTCA 100

CCAGAGTCAC TACTCCAGGT TGAGGACATG AGGCAGG 137

(2) INFORMATION FOR SEQ ID :617:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :617:

GCAGGGCAGT CTTGGTGTGC AGCCCCTCTC CTCTCTGTCC CCTGACACTC 50

CACAGTGTGC CTGCAACCCA AGTGGCCTTA TCCGTGCAGT GGTGGCAGTT 100

CAGAAATAAA GGGCCCATTT GAGGGATGAC CGCATTCAC 139

(2) INFORMATION FOR SEQ ID :618:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :618:

TCTTCTTACT ACACTGGAAG TCTGAACTGG GTGCCTGTTA CCGTCGAGGG 50

TTACTGGCAG ATCACCGTGG ACAGCATCAC CATGAACGGA GAGGCCATCG 100

CCTNCNCTGA GGNCNGCCAG GCCATTGTTG ACACCNNCAC CTCTCNNCTG 150

ANNNNCCCAA CCAGCCCCAT TNCCAACA 178

(2) INFORMATION FOR SEQ ID :619:

(i) SEQUENCE CHARACTERISTICS:
(A) LENGTH: 73 base pairs
(B) TYPE: nucleic acid
(C) STRANDEDNESS: double
(D) TOPOLOGY: linear

(xi) SEQUENCE DESCRIPTION: SEQ ID :619:

ACCACCCCTT CCTTTTCTTC ATCCAGCACA GCAAGACCAA CGGGATTCTC 50

TTCTGCGGCC GCCACCGCGT GGA 73

(2) INFORMATION FOR SEQ ID :620:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :620:

TGCCTTACAC ACTCAGGGAG ACCTCGGGTT GTACCTAGGC CTAGTGGACA 50

AACTTTGGTA GAGGGTTCGG TACGACTTAC GACACCTGGC CCTACGTCAT 100

AGTCCGTACC CTCAGTAACA GTGTAGTAGC TCTTCCTGT 139

(2) INFORMATION FOR SEQ ID :621:

(i) SEQUENCE CHARACTERISTICS:

(A) LENGTH: 123 base pairs

(B) TYPE: nucleic acid

(C) STRANDEDNESS: double

(D) TOPOLOGY: linear

(xi) SEQUENCE DESCRIPTION: SEQ ID :621:

TTCAAAAACA ACTTTATTCA TGACACATAT TAANNNAAAA CCCCACCCCT 50

GGAAATGAGC TAAAAAAATA AACAAAATCC ACCTCCCACC TCCCTGNNCC 100

CACTTCCTCC CATGCCCTCC AAA 123

(2) INFORMATION FOR SEQ ID :622:

(i) SEQUENCE CHARACTERISTICS:

(A) LENGTH: 173 base pairs

(B) TYPE: nucleic acid

(C) STRANDEDNESS: double

(D) TOPOLOGY: linear

(xi) SEQUENCE DESCRIPTION: SEQ ID :622:

CACCCAAGAC CATCCTTTAT TGTAGTATTA GTTCATGGTA ACTGCATGAA 50

AAAACATTTC NNGAGGAATT TTCAATTTCC AGCTTAAAGA ACNNNCCCAC 100

CAACATAACC AATTTATGAA ANNNAATTCA TTAAAAGGTA TAGAACCTCT 150

TGTNNNCATG ATGGCAAGGG ACA 173

(2) INFORMATION FOR SEQ ID :623:

(i) SEQUENCE CHARACTERISTICS:
(A) LENGTH: 112 base pairs
(B) TYPE: nucleic acid
(C) STRANDEDNESS: double
(D) TOPOLOGY: linear

(xi) SEQUENCE DESCRIPTION: SEQ ID :623:

TTTATTGTAT CATGAGGCAT TGAAACATCT GAATAAATCA ATGTCTGGGC 50

GGTGAAGGCA GCTGCTTTCT CCTTCACTTC TTTGGGTTAC TAGAGCAACT 100

TGTCAGTAGA TT 112

(2) INFORMATION FOR SEQ ID :624:

(i) SEQUENCE CHARACTERISTICS:
(A) LENGTH: 119 base pairs
(B) TYPE: nucleic acid
(C) STRANDEDNESS: double
(D) TOPOLOGY: linear

(xi) SEQUENCE DESCRIPTION: SEQ ID :624:

ACTCCTTCTG CCCCCGTTCT TCTAGTGAGA GGGGCGGACA AGGGGGCGGC 50

GAAAAGAGGA GAAAGGAGAG AAACAAGAGT CGAGGGGGAC AGGGGAGTCG 100

AGGTCTGCAT CCCCTCCCC 119

(2) INFORMATION FOR SEQ ID :625:

(i) SEQUENCE CHARACTERISTICS:
(A) LENGTH: 39 base pairs
(B) TYPE: nucleic acid
(C) STRANDEDNESS: double
(D) TOPOLOGY: linear

(xi) SEQUENCE DESCRIPTION: SEQ ID :625:

AACAGCACGG AGATTGCGTT TATATATCAG ACCAAGCTC 39

(2) INFORMATION FOR SEQ ID :626:

(i) SEQUENCE CHARACTERISTICS:
(A) LENGTH: 72 base pairs
(B) TYPE: nucleic acid
(C) STRANDEDNESS: double
(D) TOPOLOGY: linear

(xi) SEQUENCE DESCRIPTION: SEQ ID :626:

CCAGCGCCGA GGTTGTATAT TTCTAGGTGC AGGTATATGA TTGCCATATA 50

ATAAAAATCT GAAAACATCC CC 72

(2) INFORMATION FOR SEQ ID :627:

(i) SEQUENCE CHARACTERISTICS:

(A) LENGTH: 153 base pairs

(B) TYPE: nucleic acid

(C) STRANDEDNESS: double

(D) TOPOLOGY: linear

(xi) SEQUENCE DESCRIPTION: SEQ ID :627:

CCTCCGCCGG AGCTATCTGC ACTACATCCG CAAGTACAAC CGTCTTCGAG 50

AAGCGCCACA AGAACATGTC TGTACACCTG TCCCCCTGCT TCAGGGACGT 100

CCAGATCGGT GACATCGTCA CAGTGGGCGA GTGCGGCCTC TAGACAAGAC 150

AGT 153

(2) INFORMATION FOR SEQ ID :628:

(i) SEQUENCE CHARACTERISTICS:

(A) LENGTH: 108 base pairs

(B) TYPE: nucleic acid

(C) STRANDEDNESS: double

(D) TOPOLOGY: linear

(xi) SEQUENCE DESCRIPTION: SEQ ID :628:

AAAAGAAGTA GGTCGTGTCG TTCTGGTTGC CCTAAGAGAA GAAGANNNCG 50

GTGGCCACCT CGAGGTTAAG AGGGATATCA CTCAGCATAA TGTTAAGTGA 100

CCGGCAGC 108

(2) INFORMATION FOR SEQ ID :629:

(i) SEQUENCE CHARACTERISTICS:
(A) LENGTH: 95 base pairs
(B) TYPE: nucleic acid
(C) STRANDEDNESS: double
(D) TOPOLOGY: linear

(xi) SEQUENCE DESCRIPTION: SEQ ID :629:

TCGCACCACT AGAAANCACC GTGACTGAGA AGAATGATGT GACCTTCAGA 50

CTTGACCCNN GGACAATGNC AGCTCCCAAT GNCCGTCTAG TGGCA 95

(2) INFORMATION FOR SEQ ID :630:

(i) SEQUENCE CHARACTERISTICS:
(A) LENGTH: 139 base pairs
(B) TYPE: nucleic acid
(C) STRANDEDNESS: double
(D) TOPOLOGY: linear

(xi) SEQUENCE DESCRIPTION: SEQ ID :630:

CCAGCNCCGA AAAGCCAAGA CTTCATCAAC TACATAGGTC TTACCATTGA 50

CCTAAGATCA ATCTGAACTA TCTTAGCCCA GTCAGGGAGC TCTGCTTCCT 100

AGAAAGGCAT CTTTCGCCAG TGGATTCGCC TCAAGGTTG 139

(2) INFORMATION FOR SEQ ID :631:

(i) SEQUENCE CHARACTERISTICS:
(A) LENGTH: 117 base pairs
(B) TYPE: nucleic acid
(C) STRANDEDNESS: double
(D) TOPOLOGY: linear

(xi) SEQUENCE DESCRIPTION: SEQ ID :631:

CCACCGCCCC GAGCGAATGT AACCCGGCCT TGGACGACCC GACGCCGGAC 50

TACATGAACC TGCTGGGCAT GATCTTCAGC ATGTNCNNCC TCATGCTTAA 100

GCTGAAGTGG TGTGCTT 117

(2) INFORMATION FOR SEQ ID :632:

(i) SEQUENCE CHARACTERISTICS:
(A) LENGTH: 161 base pairs
(B) TYPE: nucleic acid
(C) STRANDEDNESS: double
(D) TOPOLOGY: linear

(xi) SEQUENCE DESCRIPTION: SEQ ID :632:

CCAGCNCCGA AAAGCCAAGA CTTCATGAAC TACATAGGTC TTACCATTGA 50

CCTAAGATCA ATCTGAACTA TCTTAGCCCA GTCAGGGAGC TCTGCTTCCT 100

AGAAAGGCAT CTTTCGCCAG TGGATTCGCC TCAAGGTTGA GGCCGCCATT 150

GGAAGATGAA A 161

(2) INFORMATION FOR SEQ ID :633:

(i) SEQUENCE CHARACTERISTICS:
(A) LENGTH: 97 base pairs
(B) TYPE: nucleic acid
(C) STRANDEDNESS: double
(D) TOPOLOGY: linear

(xi) SEQUENCE DESCRIPTION: SEQ ID :633:

CCTGCNCCGA CGATGCCCAG AATCCAGAAC TTTGTCTATC ACTCTCCCCA 50

ACAACCTAGA TGTGAAAACA GAATAAACTT CACCCAGAAA AACAAAC 97

(2) INFORMATION FOR SEQ ID :634:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :634:

CCAGCNCCGA AGCATTGGAT GCTTGACAAA CTAACGGGTG TATTTGCACC 50

TCGTCCATCG ACAGGTCCCC AGAAGCTGAG GGAATGTCTT TCCTCTGATC 100

TTCTTCCTCA GGAATAGACT CAAGTATGCG TTGACTGGAG ATGAGGTAAA 150

GAAGATATGT ATGCAACGTT CATTCAAA 178

(2) INFORMATION FOR SEQ ID :635:

(i) SEQUENCE CHARACTERISTICS:
(A) LENGTH: 90 base pairs
(B) TYPE: nucleic acid
(C) STRANDEDNESS: double
(D) TOPOLOGY: linear

(xi) SEQUENCE DESCRIPTION: SEQ ID :635:

CCTGCNCCGA TGCACCCGCC ATCCAGCCTG TCCTTTGGAC CACGCCACCC 50

CTCCAGCATG GTCACCGCCA TGGGTTAGAN CCCTGCTCGA 90

(2) INFORMATION FOR SEQ ID :636:

(i) SEQUENCE CHARACTERISTICS:
(A) LENGTH: 101 base pairs
(B) TYPE: nucleic acid
(C) STRANDEDNESS: double
(D) TOPOLOGY: linear

(xi) SEQUENCE DESCRIPTION: SEQ ID :636:

CAGGAGACAC AGACAATAGT CACTACATCA CAGCCTTGTT CTTTCCGAAG 50

GATAAAATGT CATTCAAGAA TGGGGTGAGG TGGTTAGAGG GACTAGGTAC 100

T 101

(2) INFORMATION FOR SEQ ID :637:

(i) SEQUENCE CHARACTERISTICS:
(A) LENGTH: 70 base pairs
(B) TYPE: nucleic acid
(C) STRANDEDNESS: double
(D) TOPOLOGY: linear

(xi) SEQUENCE DESCRIPTION: SEQ ID :637:

CCGAGCACGA GACCCTGATG CACATTCTAA AATAAAAGAA TGATGCACAT 50

TTTAATAAAG CACAGCACAA 70

(2) INFORMATION FOR SEQ ID :638:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID :638:

CCGAGCANAN TCTAACCCGG CCTTGGACGA CCCGACGCCG GACTACATGA 50

ACCTGCTGGG CATGATCTTC AGCATGTGCG GCCTCATGCT TAAGCTGAAG 100

TGGTGTGCTT GGGTCGCTGT CTACTGCTCC TTCATCAGCT TTGCCAACTC 150

TCGGAGCTCG 160

(2) INFORMATION FOR SEQ ID :639:

(i) SEQUENCE CHARACTERISTICS:
(A) LENGTH: 138 base pairs
(B) TYPE: nucleic acid
(C) STRANDEDNESS: double
(D) TOPOLOGY: linear

(xi) SEQUENCE DESCRIPTION: SEQ ID :639:

CCGAACAAAT GTACGGAATG TGTGAGTCCC TCTGGAGCCC AACATGGATC 50

CGGATCACCT GTTTGAAACC ATCTCCCAAG CCATGCTGAA TGCTGTGGGC 100

CGGGATGCAT GTGCAGGCAT GGAGTCATTG TCACATCA 138

(2) INFORMATION FOR SEQ ID :640:

(i) SEQUENCE CHARACTERISTICS:
(A) LENGTH: 80 base pairs
(B) TYPE: nucleic acid
(C) STRANDEDNESS: double

(D) TOPOLOGY: linear

(xi) SEQUENCE DESCRIPTION: SEQ ID :640:

CCGACCACCC CTTCCTTTTC TTCGTCCATC CAGCACAGCA AGACCAACGG 50

GATTCTCTTC TGCGGCCGCC ACCGCGTGGA 80

(2) INFORMATION FOR SEQ ID :641:

(i) SEQUENCE CHARACTERISTICS:
(A) LENGTH: 100 base pairs
(B) TYPE: nucleic acid
(C) STRANDEDNESS: double
(D) TOPOLOGY: linear

(xi) SEQUENCE DESCRIPTION: SEQ ID :641:

AACTAAACTG TTACCTTCCC TCGCTCCACA GAAGAAGACA GCCAGCTTCA 50

GGGGTCCCTG TTGCTGGCCA AGCCAGTGAG CCTGCTGGGA GGCTGGTCCA 100

(2) INFORMATION FOR SEQ ID :642:

(i) SEQUENCE CHARACTERISTICS:
(A) LENGTH: 77 base pairs
(B) TYPE: nucleic acid
(C) STRANDEDNESS: double
(D) TOPOLOGY: linear

(xi) SEQUENCE DESCRIPTION: SEQ ID :642:

AGAGGACAGA AAGGAACAGA ATGATCTTCC TACNCACAAC ACAAACGTCA 50

GTTAATGTTC CATCCATGCT GCTTAAA 77

(2) INFORMATION FOR SEQ ID :643:

(i) SEQUENCE CHARACTERISTICS:
(A) LENGTH: 127 base pairs
(B) TYPE: nucleic acid
(C) STRANDEDNESS: double
(D) TOPOLOGY: linear

(xi) SEQUENCE DESCRIPTION: SEQ ID :643:

CCAGCGCCGA GAGCAGCCCC AGTAGCAGCN CATGGCCGGG TCCAACGCCT 50

ACATCGACAA CCTTCATGGC GGACGGGACC TGTCAGGACN GGCCATCGTG 100

GGCTACAAGG ACTCGCCCTC CGTCTGG 127

(2) INFORMATION FOR SEQ ID :644:

(i) SEQUENCE CHARACTERISTICS:
(A) LENGTH: 116 base pairs
(B) TYPE: nucleic acid
(C) STRANDEDNESS: double
(D) TOPOLOGY: linear

(xi) SEQUENCE DESCRIPTION: SEQ ID :644:

GTAGCCAGAC CACAACACCG AGTTGTACCC AGATAGCTGG GATTGGAAGT 50

GAGGAGGTTT CTCACCCCAC AGATAACCCA AGACACAAAT GTGCAATTAA 100

AAGTTTATTT TAGACC 116

(2) INFORMATION FOR SEQ ID :645:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :645:

CCACCACCCG ATTCGTGACC AAGAAGGCTC TGTGCATTCG GGTTTTCCAG 50

GAGACTCAAA AGCCGAAGAA GCGAAGAAGA GCCTTAAAGG CTGCAGCAGC 100

CAGAAAAACA AGCAAAGGAG GAACCCAGAC AGCCCTGCAA AGCATACAAG 150

ACACTCAAGA CAGCAATTAA TCTGTCATCA TT 182

(2) INFORMATION FOR SEQ ID :646:

(i) SEQUENCE CHARACTERISTICS:
(A) LENGTH: 66 base pairs
(B) TYPE: nucleic acid
(C) STRANDEDNESS: double
(D) TOPOLOGY: linear

(xi) SEQUENCE DESCRIPTION: SEQ ID :646:

CCAGCGCCGA GGTTGTATAT TTCTAGGTGC AGGTATATGA TTGCCATATA 50

ATAAAAATCT GAAAAC 66

(2) INFORMATION FOR SEQ ID :647:

(i) SEQUENCE CHARACTERISTICS:
(A) LENGTH: 93 base pairs
(B) TYPE: nucleic acid
(C) STRANDEDNESS: double
(D) TOPOLOGY: linear

(xi) SEQUENCE DESCRIPTION: SEQ ID :647:

CAAGACAATA GTCACATCAT CGCAGCCTTG TTCTTTCCGA AGGATAAAAT 50

GTCATTCAAG AATGGGGTGA GGTGGTTAGA GGGAGTAGGT ACT 93

(2) INFORMATION FOR SEQ ID :648:

(i) SEQUENCE CHARACTERISTICS:
(A) LENGTH: 114 base pairs
(B) TYPE: nucleic acid
(C) STRANDEDNESS: double
(D) TOPOLOGY: linear

(xi) SEQUENCE DESCRIPTION: SEQ ID :648:

CCCCTTTTTG TCCCCACTGA GATGTATGAA GGTTTTGGTC TCCCTGGGAG 50

TGGGTGGAGG CAGCCAGGGC TTACCTGTAC ACTGACTTGA GACCAGTTGA 100

AAAGTGCACA CCTT 114

(2) INFORMATION FOR SEQ ID :649:

(i) SEQUENCE CHARACTERISTICS:

(A) LENGTH: 81 base pairs

(B) TYPE: nucleic acid

(C) STRANDEDNESS: double

(D) TOPOLOGY: linear

(xi) SEQUENCE DESCRIPTION: SEQ ID :649:

CTGCNCCGAC CACCCCTTCC TTTTCTTCAT CCAGCACAGC AAGACCAACG 50

GGATTCTCTT CTGCGGCCGC CACCGCGTGG A 81

(2) INFORMATION FOR SEQ ID :650:

(i) SEQUENCE CHARACTERISTICS:

(A) LENGTH: 159 base pairs

(B) TYPE: nucleic acid

(C) STRANDEDNESS: double

(D) TOPOLOGY: linear

(xi) SEQUENCE DESCRIPTION: SEQ ID :650:

AGGAGCNCCG ACAAAAGACA CATTGGACCT GTCAGCTCCT CTGTTTCACC 50

AAGCAGACAC AATAACCTTA CCAACAAAGC AGAGTAAGCC AAGTGCCTCT 100

GTGTGACACC ACCGCATNNT GATGACGCAT AATAAAAATA TAACTAATTT 150

AGACTAGAG 159

(2) INFORMATION FOR SEQ ID :651:

(i) SEQUENCE CHARACTERISTICS:
(A) LENGTH: 78 base pairs
(B) TYPE: nucleic acid
(C) STRANDEDNESS: double
(D) TOPOLOGY: linear

(xi) SEQUENCE DESCRIPTION: SEQ ID :651:

CCGACCACCC CTTCCTTTTC TTCATCCAGC ACAGCAAGAC CAACGGGATT 50

CTCTTCTGCG GCCGCCACCG CTGGAGCT 78

(2) INFORMATION FOR SEQ ID :652:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :652:

CCGACAGACG GTCATTGATT ACAACGGGGA ACGCACGCTG GATGGTTTTA 50

AGAAATTCCT GGAGAGCGGT GGCCAGGATG GGGCAGGGGA TGATGACGAT 100

CTCGAGGACC TGGAAGAAGC AGAGGAGCCA GACATGGAGG AAGACNATGA 150

TCAGAAAGCT GTGAAAGATG AACTGTAATA CGCAAAGCCA GACCCGGG 198

(2) INFORMATION FOR SEQ ID :653:

(i) SEQUENCE CHARACTERISTICS:

(A) LENGTH: 224 base pairs

(B) TYPE: nucleic acid

(C) STRANDEDNESS: double

(D) TOPOLOGY: linear

(xi) SEQUENCE DESCRIPTION: SEQ ID :653:

CCGAAAAGCC AAGACTTCAT GAACTACATA GGTCTTACCA TTGACCTAAG 50

ATCAATCTGA ACTATCTTAG CCCAGTCAGG GAGCTCTGCT TCCTAGAAAG 100

GCATCTTTCG CCAGTGGATT CGCCTCAAGG TTGAGGCCGC CATTGGAAGA 150

TGAAAAATTG CACTCCCTTG GTGTAGACAA TACCAGTTCC ATTGGTGTTG 200

TTGCTATAAT AACACTTTTC TTTT 224

(2) INFORMATION FOR SEQ ID :654:

(i) SEQUENCE CHARACTERISTICS:

(A) LENGTH: 226 base pairs
(B) TYPE: nucleic acid
(C) STRANDEDNESS: double
(D) TOPOLOGY: linear

(xi) SEQUENCE DESCRIPTION: SEQ ID :654:

CCGCCTCAGA CTCTGGACCA GCCACTGTCC CAGAAGCCAG GCCGGGCAGT 50

GGCCTTCTCC ACTCCCCTCT GACTTCTCCA AGGGGCTCAG TGGCCAGTGC 100

CCCCCAGGAG GCTCCACCCT CAACTCAACC CAAGCAAGAG GGACAGATGA 150

AAAACAAAAT CCAATCAGGG CGATAAATGG CGGGGGGTTT AATTTGGTTT 200

CTGAGCGCAT AAAGCTAAGG AGGGGT 226

(2) INFORMATION FOR SEQ ID :655:

(i) SEQUENCE CHARACTERISTICS:
(A) LENGTH: 167 base pairs
(B) TYPE: nucleic acid
(C) STRANDEDNESS: double
(D) TOPOLOGY: linear

(xi) SEQUENCE DESCRIPTION: SEQ ID :655:

TTAAAAAAAT TCCCCCCTTT AATTGACCAA AGTAAAGCCA TGACATTTCA 50

TTTGGTAACC TGTTTAGAAT TATAAAAATC ATTTCATTTG GCCCAGCCCA 100

TACGCCCAAG AGAAAACTTC CAGACTTTTC TGATGCCATC CAGTTTTGTT 150

CTTACAAAAT GCATATT 167

(2) INFORMATION FOR SEQ ID :656:

(i) SEQUENCE CHARACTERISTICS:
(A) LENGTH: 72 base pairs
(B) TYPE: nucleic acid
(C) STRANDEDNESS: double
(D) TOPOLOGY: linear

(xi) SEQUENCE DESCRIPTION: SEQ ID :656:

CCACCCCTTC CTTTTCTTCA TCCAGCACAG CAAGACCAAC GGGATTCTCT 50

TCTGCGGCCG CCACCGCGTG GA 72

(2) INFORMATION FOR SEQ ID :657:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :657:

AGAGGGTTTT CTATATGTAA TTCTTTTATT CTGTAAAAGG TAACAAAATA 50

TACAGAACAA AAAAACTTTC CCTTTTTAAA ACTAATGTTA CAAATCTGTA 100

TTATCACTTG TATATAAATA GTATATAGCT GATCATTAAT AAGGTGTATA 150

GTACAATGTA TTCTAAAACT GTTCCGCC 178

(2) INFORMATION FOR SEQ ID :658:

(i) SEQUENCE CHARACTERISTICS:
(A) LENGTH: 76 base pairs
(B) TYPE: nucleic acid
(C) STRANDEDNESS: double
(D) TOPOLOGY: linear

(xi) SEQUENCE DESCRIPTION: SEQ ID :658:

CCGACCACCC CTTCCTTTTC TTCATCCAGC ACAGCAAGAC CAACGGGATT 50

CTCTTCTGCG GCCGCCACCG CGTGGA 76

(2) INFORMATION FOR SEQ ID :659:

(i) SEQUENCE CHARACTERISTICS:
(A) LENGTH: 68 base pairs
(B) TYPE: nucleic acid
(C) STRANDEDNESS: double
(D) TOPOLOGY: linear

(xi) SEQUENCE DESCRIPTION: SEQ ID :659:

GCCAGCGCCG AGGTCGCATA TTTCTAGGCG CAGGTATATG ACTGCCATAT 50

AACAAAAACC NTGAAAAC 68

(2) INFORMATION FOR SEQ ID :660:

(i) SEQUENCE CHARACTERISTICS:
(A) LENGTH: 151 base pairs
(B) TYPE: nucleic acid
(C) STRANDEDNESS: double
(D) TOPOLOGY: linear

(xi) SEQUENCE DESCRIPTION: SEQ ID :660:

CCCAAACTTC TTTAAAAATC AAGTAATGTT TACAAGAAAG AATAAAATCT 50

TAATCCTTTT CACTTTTAAA GACAATCAGA TAAGATTACC CACTGCGATT 100

AAACACTGAT CAAACTCAGT TGTCCTTACG TTAGCATTAC TCTGTCATAG 150

C 151

(2) INFORMATION FOR SEQ ID :661:

(i) SEQUENCE CHARACTERISTICS:
(A) LENGTH: 245 base pairs
(B) TYPE: nucleic acid
(C) STRANDEDNESS: double
(D) TOPOLOGY: linear

(xi) SEQUENCE DESCRIPTION: SEQ ID :661:

AGGCACTGAC CCCTGCCACC CCTCACTGCA TTAACTTCAG CCACGTCTCC 50

TCCTTCCTCT GCGCTTCCAG TGATAAGGGT ACTGTCCATA TCTTTGCTCT 100

CAAGGATACC CGCCTCAACC GCCGCTCCGG CCTGGCTCGC GTGGGCAAGG 150

TGGGGCCTAT GATTGGGCAG TACGTGGACT CTCAGTGGAG CCTGGCGAGC 200

TTCACTGTGC CTGCTGAGTC AGCTTGCATC TGCGCCTTCG GTGCG 245

(2) INFORMATION FOR SEQ ID :662:

(i) SEQUENCE CHARACTERISTICS:
(A) LENGTH: 72 base pairs
(B) TYPE: nucleic acid
(C) STRANDEDNESS: double
(D) TOPOLOGY: linear

(xi) SEQUENCE DESCRIPTION: SEQ ID :662:

GCCAGCGCCG AGGTCGCATA TTTCTAGGCG CAGGTATATG ATTGCCATAT 50

AATAAAAACM TGAAAACACC CC 72

(2) INFORMATION FOR SEQ ID :663:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :663:

GCCTGCGCCG ACGATGACCA GAATCCAGAA CTTTGCCTAT CACTCTCCCC 50

AACAACCTAG ACGCGAAAAC AGAATAAACT TCACCCAG 88

(2) INFORMATION FOR SEQ ID :664:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :664:

GCCTGCGCCG ACAAACAAAA CCTGGAGGCC ATTCTGCACA GCCTGCCCGA 50

GAACTGTGCC AGCTGGCGGT GAGGGCTGCC CAGATCCCCG GCACACACTC 100

CCCCACCTGC TGTTTACATG ACCCAGGGGG CGCACACTAC CCCACAGGCG 150

CGCCCATACA GACATTCCCC GGAGCCGGCT GCTGCGAACT CGACCCCGTG 200

CGGATAGTCA CACTCCCTGC CGA 223

(2) INFORMATION FOR SEQ ID :665:

(i) SEQUENCE CHARACTERISTICS:
(A) LENGTH: 70 base pairs
(B) TYPE: nucleic acid
(C) STRANDEDNESS: double
(D) TOPOLOGY: linear

(xi) SEQUENCE DESCRIPTION: SEQ ID :665:

GCCAGCGCCG AGGTTGCATA TTTCTAGGCG CAGGTATATG ATTGCCATAT 50

AATAAAAATC TGAAAACACC 70

(2) INFORMATION FOR SEQ ID :666:

(i) SEQUENCE CHARACTERISTICS:
(A) LENGTH: 118 base pairs
(B) TYPE: nucleic acid
(C) STRANDEDNESS: double
(D) TOPOLOGY: linear

(xi) SEQUENCE DESCRIPTION: SEQ ID :666:

GCCAGCGCCG ACCGCCCCAA AAATACTCCC ACCGGGATCA GGGCGATTAA 50

TGAAGACTCA ACGGGGACTA AAAAGGGGCC CCAAAAAAAA CCAAACCACC 100

TTTCTACGTA CCGTATAG 118

(2) INFORMATION FOR SEQ ID :667:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :667:

GCCAGCGCCG ACGATGCCCA GAATCCAGGA CTTTGCCTAT CACTCTCCCC 50

AACAACCTAG ACGCGAAAAC AGAATAAACT TCACCCAG 88

(2) INFORMATION FOR SEQ ID :668:

(i) SEQUENCE CHARACTERISTICS:
(A) LENGTH: 59 base pairs
(B) TYPE: nucleic acid
(C) STRANDEDNESS: double
(D) TOPOLOGY: linear

(xi) SEQUENCE DESCRIPTION: SEQ ID :668:

GCCACCGCCG ACTCCAGGCA CTCACTCAAA CTTGATCTTC AACTCTGCAT 50

ACAAGCAGA 59

(2) INFORMATION FOR SEQ ID :669:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :669:

GCCTGCGCCG AACAAATATA CAGAATGCGC GAGTCCCTCT GGAAGCCCAA 50

CATGGATCCG GATCACCTGT TTGAAACCAT CTCCCAAGCC ATGCTGAATG 100

CTGCGGACCA GGATGCAGTG CCAGGCATGG GAGTCATTGC CCACATCACC 150

GAGAAGGACA AAATCACCAC CAGGACACTG AAGGCCCGAA TGGACTCGCC 200

CTGCTCCCAG AGCCCACTTT TTT 223

(2) INFORMATION FOR SEQ ID :670:

(i) SEQUENCE CHARACTERISTICS:
(A) LENGTH: 37 base pairs
(B) TYPE: nucleic acid
(C) STRANDEDNESS: double
(D) TOPOLOGY: linear

(xi) SEQUENCE DESCRIPTION: SEQ ID :670:

GCCAGCGCCG AAAACACACA CTTTGCTTCT TACCTGC 37

(2) INFORMATION FOR SEQ ID :671:

(i) SEQUENCE CHARACTERISTICS:
(A) LENGTH: 37 base pairs
(B) TYPE: nucleic acid
(C) STRANDEDNESS: double
(D) TOPOLOGY: linear

(xi) SEQUENCE DESCRIPTION: SEQ ID :671:

GCCAGCGCCG AAAACACACA CTCTGCTTCT TACCTGC 37

(2) INFORMATION FOR SEQ ID :672:

(i) SEQUENCE CHARACTERISTICS:
(A) LENGTH: 59 base pairs
(B) TYPE: nucleic acid
(C) STRANDEDNESS: double
(D) TOPOLOGY: linear

(xi) SEQUENCE DESCRIPTION: SEQ ID :672:

GCCACCGCCG ACTCCAGGCA CTCACTCAAA CTCGATCTTC AACTCTGCAT 50

ACAAGCAGA 59

(2) INFORMATION FOR SEQ ID :673:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :673:

GCCTGCGCCG ANGCATTCCC TTTGACCTGA GTCTGCAGCA GGTCCCTTTT 50

GCGCTTCCTT CCCCTCAGGT AGCCTCTCTC CCCCTGGGCC ACTCCCGGGG 100

GTGAGGGGGT TACCCCTTCC CAGTGTTTTT TATTTCCGTG GGGCTCACCC 150

CAAAGTATTA AAAGCAACTT TGCAATT 177

(2) INFORMATION FOR SEQ ID :674:

(i) SEQUENCE CHARACTERISTICS:
(A) LENGTH: 77 base pairs
(B) TYPE: nucleic acid
(C) STRANDEDNESS: double
(D) TOPOLOGY: linear

(xi) SEQUENCE DESCRIPTION: SEQ ID :674:

GCCTGCGCCG AGCACAAGAC AATGACGAAC ATTTTAAAAA AAAAGAATGA 50

CGCACATTTT AATAAAGCAC AGCACAA 77

(2) INFORMATION FOR SEQ ID :675:

(i) SEQUENCE CHARACTERISTICS:
(A) LENGTH: 176 base pairs
(B) TYPE: nucleic acid
(C) STRANDEDNESS: double
(D) TOPOLOGY: linear

(xi) SEQUENCE DESCRIPTION: SEQ ID :675:

GCCAGCGCCG ACACCCAGAG ACTACAGTAC TTAGGGGTTA CACACAACAG 50

CCGTAACTGG CGGCTATCTG TTCATAACAA ACAAACCATA GCATATTTAC 100

ACCGCATCAC ATCGAGTGAT TATAGAAATC CATACACACA CCGATTGCAT 150

AAAATCTTTT TTTAGGAAAA AAACAC 176

(2) INFORMATION FOR SEQ ID :676:

(i) SEQUENCE CHARACTERISTICS:

(A) LENGTH: 141 base pairs

(B) TYPE: nucleic acid

(C) STRANDEDNESS: double

(D) TOPOLOGY: linear

(xi) SEQUENCE DESCRIPTION: SEQ ID :676:

GCCAGCGCCG ACGCCCAGAG AATACAGTAC TTAGGGGTTA CACACAACAG 50

CCGTAACTGG CGGCTATCTG CTCATAACAA ACAAACCACA GCATATCTAT 100

ACCGTATCAC ATCGAGTGAT TATAGAAATC CACAGAGAGA C 141

(2) INFORMATION FOR SEQ ID :677:

(i) SEQUENCE CHARACTERISTICS:

(A) LENGTH: 365 base pairs

(B) TYPE: nucleic acid

(C) STRANDEDNESS: double

(D) TOPOLOGY: linear

(xi) SEQUENCE DESCRIPTION: SEQ ID :677:

CTCTGAACAG ACACGAAGCT GCCCCTCGTA CAGCCACTCG GGCGCTGACC 50

ACCAGGGAAG GCAAAGGCAT AGGACTTCCA CACAGTCCAG ACACTGCACG 100

CTGACAAGCG CCTCCGCGGC GGCTGCGAGC CGGACTCAGG CGGATCTTGA 150

CAGCCTTGCC CGCGAGTGCC CGGGGATAGA ACCCGTGCGC GTGGACCTAG 200

GTGACTGGGA GGCCACCAAG CAGGCACTGG GCAGCGTGGG CCCCGTGGAC 250

CTGCTGGAGA ACAACACCAC CGTCGCCCTG CCGCAGCCCT TCCAGGAGGT 300

CACCAAGGAG GCCTTCGACA GATCCTTTGA GGTGAGCTTG CGTGCGATCA 350

TCCAGGTGCT GTAGA 365

(2) INFORMATION FOR SEQ ID :678:

(i) SEQUENCE CHARACTERISTICS:
(A) LENGTH: 366 base pairs
(B) TYPE: nucleic acid
(C) STRANDEDNESS: double
(D) TOPOLOGY: linear

(xi) SEQUENCE DESCRIPTION: SEQ ID :678:

GCCTGCCCCG ACACGAAGAT GACCCTCGAC AGCCGCCGGG CGCTGATCAC 50

CAGGGAAGGC AAAGGCATAG GACTTCTGCA CGGTCCAGAC CCATGTACGC 100

GACGGGCGCC TNCGCGGTGG CTGCGAGCCG GATTCAGGCA GATCTTGACA 150

GCCTTGCCCG CGAGTGCCCG GGGATAGAAC CCGTGTGCGT GGACCTNGGG 200

TGACTGGGAG GCCACCAAGC AGGCACTGGG GTAGCATGGG CCCCGCGGAC 250

CTGCCGGAGA ACAATACCAC CGCCGCCTNT GCCGCAGTCT TTCCCGGAGG 300

TCACCAAGGA GGCCTNTTGA TAGATCTTTT GAGGTGAGCT TGCGTGCGGT 350

CATCTAGGTG CTGTAG 366

(2) INFORMATION FOR SEQ ID :679:

(i) SEQUENCE CHARACTERISTICS:
(A) LENGTH: 253 base pairs
(B) TYPE: nucleic acid
(C) STRANDEDNESS: double
(D) TOPOLOGY: linear

(xi) SEQUENCE DESCRIPTION: SEQ ID :679:

GCCTGCCCCG ACACGAAGAT GACCCTCGAC AGCCGCCGGG CGCTGATCAC 50

CAGGGAAGGC AAAGGCATAG GCCAGCACCG ATGAATTCCC TTTGACCTAA 100

GTCTGCAGCA GGCCCCTTTT GCGCTTCCTT CCCCTCAGGC AGCCTCTCTC 150

CCCCCGGGCC ACTCCCGGGG GCGAGGGGGT TACCCCTTTC CCAGTGCTTT 200

TTATTCCCGC GGGGCTCACC CCAAAGCATT AAAAGCAGCT TTGCAATTCC 250

TTG 253

(2) INFORMATION FOR SEQ ID :680:

(i) SEQUENCE CHARACTERISTICS:
(A) LENGTH: 294 base pairs
(B) TYPE: nucleic acid
(C) STRANDEDNESS: double
(D) TOPOLOGY: linear

(xi) SEQUENCE DESCRIPTION: SEQ ID :680:

GCCAGCGCCG AGGCTTCTCC TAGGGCTGCT CCTGGGCCTA GCTCTTACAG 50

GCTCGTCCCC CAGGCCTGCC CTTCTCCACT GCCCCCTCCC GTGCCTGGGC 100

CCACACACCC TTCAGGAAGG GGGAGCACTG AGAAGCACAG CACAGGGGCT 150

CAGCCTGGGA TCCGGTGACG GCCTAGGCAG AGGCTGGGCC AGGAGTCCCA 200

AAGGTCAGTG ACAGTTTCTC AGAAGGGGCC CAGCGTCCAC CTCTCTCCCA 250

GGACCAGACA CCCCTTCCAG GCTCCCCCAC CCCCCTACGG GCTC 294

(2) INFORMATION FOR SEQ ID :681:

(i) SEQUENCE CHARACTERISTICS:
(A) LENGTH: 268 base pairs
(B) TYPE: nucleic acid
(C) STRANDEDNESS: double
(D) TOPOLOGY: linear

(xi) SEQUENCE DESCRIPTION: SEQ ID :681:

CCGCCAAGTG ACACCAAAGC CCTGGTTGAC TTTGACAGCC CCGTGGGCGC 50

GCGGGAGGCC GGGCACTCTA GGGTCTACCT ACCAGTGCAA TCGTTTAGCG 100

CTTTTTCAGT GGGGCAGGGC AGGAAGCAGG CGGGACCAGG CAGCCAGTTC 150

TCAAAGGCTG CGGGGCCAAC TAGAGGCCAC AGCCCCTCAC CCCTAGACAC 200

TGCCAACCAG AACTGACACG CGACCTCCTG GGCGCTGACG CCATTAAAAC 250

CAACGTTGGC GCCCGGCG 268

(2) INFORMATION FOR SEQ ID :682:

(i) SEQUENCE CHARACTERISTICS:

(A) LENGTH: 354 base pairs

(B) TYPE: nucleic acid

(C) STRANDEDNESS: double

(D) TOPOLOGY: linear

(xi) SEQUENCE DESCRIPTION: SEQ ID :682:

GCCACCGCCG AGGAAAACCG TGCACTGTTA GCCATGATCA ACCCCACCGT 50

GCTCTTCGAC ATTGACATCG ACGGCTGAGC CCTTGACCTA GTCTCCTTTG 100

AGCTGTTTGC AGACAAGGTC CCAAAGACAG CAGAAAATTT TCGTGCTCTA 150

AGCACTGGAG AGAAAGGATT TGGTTATAAG GGTTCCTGCT TTCACAGAAT 200

TATTCCAGGG TTTATGCGCC AGGGTGGTGA CTTCACACGC CATAATGGCA 250

CTGGTGGCAA GTCCATCCAT GGGGAGAAAT TTGAAGACGA GAACTTCATC 300

CTAAAGCATA CGGGCCCTGG CATCTTGCCC ATGGCAAATG CTGATCCTGA 350

TACA 354

(2) INFORMATION FOR SEQ ID :683:

(i) SEQUENCE CHARACTERISTICS:

(A) LENGTH: 148 base pairs

(B) TYPE: nucleic acid

(C) STRANDEDNESS: double

(D) TOPOLOGY: linear

(xi) SEQUENCE DESCRIPTION: SEQ ID :683:

CCTCTCCGTG AGGACAGGGC AGTCTTGGTG CGCAGCCCCT CTCCTCTCTG 50

TCCCCTGACA CTCCACAGTG CGCCTGCAAC CCAGGCGGCC TTATCCGCGC 100

AGTGGCGGCA GTTCAGAAAT AAAGGGCCCA TTTGCGGGAT GCCGCATT 148

(2) INFORMATION FOR SEQ ID :684:

(i) SEQUENCE CHARACTERISTICS:

(A) LENGTH: 307 base pairs

(B) TYPE: nucleic acid

(C) STRANDEDNESS: double

(D) TOPOLOGY: linear

(xi) SEQUENCE DESCRIPTION: SEQ ID :684:

AGCACCGAGG CGCTCAAGGT CCTGGGGAAC CCCAAGAGCG ACGAGACGAA 50

CGCGAAGGCG CTGGACTTTG AGCACTTTCT GCCCATGCTG CAGACAGTGG 100

CCAAGAACAA GGACCAGGGC ACCTATGAGG ATTATGCCGA AGGACTTCGG 150

GCGCTTGACA AGGAAGGAAA TGGCACCGTC ATGGGCGCTG AAACCCGGCA 200

TGCTCTTGCC ACACTGGGCG AGAAGACGAC AGAGGAAGAA GCAGAGACGC 250

TGGCGGCAGG GCATGAGGAC AGCAATGGTT GCATCAACTA CGAAGCATTT 300

GTGAGGC 307

(2) INFORMATION FOR SEQ ID :685:

(i) SEQUENCE CHARACTERISTICS:

(A) LENGTH: 174 base pairs

(B) TYPE: nucleic acid

(C) STRANDEDNESS: double

(D) TOPOLOGY: linear

(xi) SEQUENCE DESCRIPTION: SEQ ID :685:

CCATCTCAAG GCGATCCATG GAAAGCTTCC TGGGGAACGT ATGCTAGCAG 50

AGCTTCTCCC CGTGAATCAC ATGCCGGAGA TCCCACTCTT AGCTGGCAAA 100

TGAATCCGAA TTGACACAGC AGCCCCATAA GCATCAGCCC TGTAGAGTGA 150

GGAGCCATCT CTAGCGGGCC CTTC 174

(2) INFORMATION FOR SEQ ID :686:

(i) SEQUENCE CHARACTERISTICS:
(A) LENGTH: 106 base pairs
(B) TYPE: nucleic acid
(C) STRANDEDNESS: double
(D) TOPOLOGY: linear

(xi) SEQUENCE DESCRIPTION: SEQ ID :686:

ACATGCCAAA GAAAGCGTGC ATATTAGCCT ATAGAACCAC AGTAATCACA 50

CTAGAGAAAT TCCACTGCTA CAATAAAATG TAATCGGAAG CATCTTTACT 100

TATAAA 106

(2) INFORMATION FOR SEQ ID :687:

(i) SEQUENCE CHARACTERISTICS:
(A) LENGTH: 97 base pairs
(B) TYPE: nucleic acid
(C) STRANDEDNESS: double
(D) TOPOLOGY: linear

(xi) SEQUENCE DESCRIPTION: SEQ ID :687:

CAGATTTTTT TCAATTCTCC ACCCGCCAAA AGGGGAAGGG CTTTTCCCCA 50

GAGAAAAGGA AAGGGGGGAA AAGGGGAAAA AACCCAACCC AAAACCA 97

(2) INFORMATION FOR SEQ ID :688:

(i) SEQUENCE CHARACTERISTICS:
(A) LENGTH: 172 base pairs
(B) TYPE: nucleic acid
(C) STRANDEDNESS: double
(D) TOPOLOGY: linear

(xi) SEQUENCE DESCRIPTION: SEQ ID :688:

GCCTCTCTCT GGGCAAGTTC ATGGCTTCTG TGAGCCCTTT NACTGACCTC 50

CAGACACTGT TAGGCTGGAG CCTCGGTAGC CGTTCCTCCT GCCCACTGGA 100

CCTTCCAACA GGCCCTCCTC CCCTCCTTGA ACCGGCCCTT CCTGGCCTTT 150

GAATAGAGTC TAAGCGAACG AC 172

(2) INFORMATION FOR SEQ ID :689:

(i) SEQUENCE CHARACTERISTICS:
(A) LENGTH: 200 base pairs
(B) TYPE: nucleic acid
(C) STRANDEDNESS: double
(D) TOPOLOGY: linear

(xi) SEQUENCE DESCRIPTION: SEQ ID :689:

GCCACCGCCG AGCTCACCCC TGCGGTGCAC GCCTTCCTTG GACAAGTTTT 50

TGGCTTCTGC GAGCCTTTTA CTGACCTCCA AACGCTGTTA AGCCGGAGCC 100

TCGGTAGCCG TTCCTCCTGC CCACTGGACN TCCCAACGGG CCCTCCTCCC 150

CTCCTTGAAC CAGCCCTTCC TGGCCTTTGA ATAAAGTTTA AGCGAGTAGC 200

(2) INFORMATION FOR SEQ ID :690:

(i) SEQUENCE CHARACTERISTICS:
(A) LENGTH: 81 base pairs
(B) TYPE: nucleic acid
(C) STRANDEDNESS: double
(D) TOPOLOGY: linear

(xi) SEQUENCE DESCRIPTION: SEQ ID :690:

GCCACCGCCG AGCAGCAGCT TACAGCCAGG GCAGCTTTGA ATGCGGCCCA 50

ACACAAATTC ACAAGTGNTC TCAAAAAACT C 81

(2) INFORMATION FOR SEQ ID :691:

(i) SEQUENCE CHARACTERISTICS:
(A) LENGTH: 217 base pairs
(B) TYPE: nucleic acid
(C) STRANDEDNESS: double
(D) TOPOLOGY: linear

(xi) SEQUENCE DESCRIPTION: SEQ ID :691:

TTCCTAACCG ATCGAATGAA GGATTCAAAA TTAACCACTC CAAGGGGGGA 50

TTGAAGGAAG AACCACTCTT AACGGACAAA AAGAAAGAAA GGGGAGGGAG 100

TAACAGGGAT ATGAGCTCTA GCCGCCCAAG CTAGCAATGG CAACCCTTCT 150

GGGTCCCCTT TCAGCATGCG GAAGCTTTTC TTCGACTTCA CTCCATAAAC 200

AGCTGACGCT CAAAAAG 217

(2) INFORMATION FOR SEQ ID :692:

(i) SEQUENCE CHARACTERISTICS:
(A) LENGTH: 72 base pairs
(B) TYPE: nucleic acid
(C) STRANDEDNESS: double
(D) TOPOLOGY: linear

(xi) SEQUENCE DESCRIPTION: SEQ ID :692:

CGGCCTTCTG GAAACCCATG AAAAAAAAAA GTTCCGCACC TCCAAGGGGA 50

GAAGAGTAAG AGACAGCTTT CA 72

(2) INFORMATION FOR SEQ ID :693:

(i) SEQUENCE CHARACTERISTICS:
(A) LENGTH: 329 base pairs
(B) TYPE: nucleic acid
(C) STRANDEDNESS: double
(D) TOPOLOGY: linear

(xi) SEQUENCE DESCRIPTION: SEQ ID :693:

TCTGCTGCCG CCCGCAAGCA GAGGGACTCG GAGATCATGC AGGGGAAGCA 50

GAAAAAGGCA AACAAGAAGA AGGAGGAACC CAAGCAGCTT TGCGGCTTCG 100

CGTCCAACCC TCTTGCCCTT CACCTACGCG CCTAGAGCCA GTCCCACCAC 150

GCTCGCGTTT CCTCCTGTAG CGCTCACAGG CCCCAGCACC GATGGCATTC 200

CCTTTGACCT AAGCCTACAG CAGGCCCCTT TTGTGCTTCC TTCCCCTCAG 250

GCAGCCTCTT TCCCCCTGGG CCACTCCCGG GGGTGAGGGG GTTGTCCTTC 300

CCGATGCTTT TTGTTACCGT GGGGTTTGC 329

(2) INFORMATION FOR SEQ ID :694:

(i) SEQUENCE CHARACTERISTICS:

(A) LENGTH: 296 base pairs

(B) TYPE: nucleic acid

(C) STRANDEDNESS: double

(D) TOPOLOGY: linear

(xi) SEQUENCE DESCRIPTION: SEQ ID :694:

TCTGCTGCCG CCCGCAAGCA GAGGGACTCG GAGATCATGG CCTGCGCCGA 50

ACAAATGTAC GGAATGCGTG AGTCCCTCTG GGAGGCCCAA CACGGATCCG 100

GATCACCTGT TTGAAACCAT CTCCCAAGCC ATGCTGAATG CTGCGGACCA 150

GGATGCAGTG TCAGGCATGG GAGTCATTGC CCACATCATC GAGAAGGACA 200

AAATCACCAC CAGGACACTG AAGGCCCGAA TGGACTAACC CTGTTCCCAG 250

AGCCCACTTT TTTTTTTTTT TTGGAAATAA AATAGCCTAT CTTTCG 296

(2) INFORMATION FOR SEQ ID :695:

(i) SEQUENCE CHARACTERISTICS:
(A) LENGTH: 211 base pairs
(B) TYPE: nucleic acid
(C) STRANDEDNESS: double
(D) TOPOLOGY: linear

(xi) SEQUENCE DESCRIPTION: SEQ ID :695:

GCCAGCACCG ACCTAGCCTA AGCCGTCTAG AACCACCTAA GCCCCTAAGG 50

AAACCTGTGG TCACACTGGG CCCTCCTCAG GAACTCTCCC TGCGCAGAGG 100

CGTGTCTTAG CACTAACCTC CTCCCTAGCC CCTTATTTGG TGGCAGAAGT 150

GGCCTCCACC CCTTCACCGT TTAAAAATAC TCCGTGGAGA AAAGAAAGCT 200

TNAAGGAGTA G 211

(2) INFORMATION FOR SEQ ID :696:

(i) SEQUENCE CHARACTERISTICS:
(A) LENGTH: 92 base pairs
(B) TYPE: nucleic acid
(C) STRANDEDNESS: double
(D) TOPOLOGY: linear

(xi) SEQUENCE DESCRIPTION: SEQ ID :696:

GCCTGCACCG ACAATGCCCA GAACTCCAGA ACTTCACCTA TCACGTATCC 50

CCAACAACAC AGACGGCGAA AACAAAACAA ACTTAAACCT AG 92

(2) INFORMATION FOR SEQ ID :697:

(i) SEQUENCE CHARACTERISTICS:
(A) LENGTH: 314 base pairs
(B) TYPE: nucleic acid
(C) STRANDEDNESS: double
(D) TOPOLOGY: linear

(xi) SEQUENCE DESCRIPTION: SEQ ID :697:

GCCAGCACCG AGGCCTAGGG TAGAGCAGAC TGTGGCTTTA CCTCGGTGTC 50

CTACCAGCAA GGGGTCCTAT CTACCACCAT CCTCTATGAG ATCCTGCTAG 100

GGAAGGCCAC CCTGCATGCT GCGTTGGTCA GCACCCTTGC GCTGATGGCC 150

ATAGTCAAGA GAAAGGATTT CTGAAGGCAG CCCTAGAAGC GGAGTTAGGA 200

GCTTCTAACC CGTCATGGTT TAAATACACA CCCTTTTTTG GACAGCGCTT 250

CTGAAGAGCT GCTCTTACCT CTCTGCATCC CAATAGATAT CCCCCTATGC 300

GCATGCGTAC CTGT 314

(2) INFORMATION FOR SEQ ID :698:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :698:

GCCACCGCCG AGTTCACCCC TGCAGTGCAC GCCTCCCTGG ACAAGTTCCT 50

GGCTTCTGTG AGCACCGTGC TGACCTCCAA ACACCGTTAA GCTGGAGCCT 100

CGGTAGCCGT TCCTCCTGCC CACTGGACTC CCAACAGGCC CTCCTCCCCT 150

CCTTGCACCG GCCCTTCCTG GTCTTTGAAT AAAGTCTAAG CGGGCAGC 198

(2) INFORMATION FOR SEQ ID :699:

(i) SEQUENCE CHARACTERISTICS:
(A) LENGTH: 230 base pairs
(B) TYPE: nucleic acid
(C) STRANDEDNESS: double
(D) TOPOLOGY: linear

(xi) SEQUENCE DESCRIPTION: SEQ ID :699:

GCCAGCACCG ACAGCAACAA AAATGTTCCC ACAGAGATCA GGATGACTTG 50

CTGAAGCTCA GTGGAGGCTA AAAAGAGGAC ACAAAAGTGA ACAGAATGAC 100

CTTCCTACGC ACAACACAAA CACCAGTTAA TGCTCCATCC ACGCTGCTTA 150

AAGAGCATTC CTGTCCTAGC AAAATGGGCA AGTCCCTCTA CCCCCCACCC 200

TTAGCCGGCA TGCTTACATT AATAGCTAGA 230

(2) INFORMATION FOR SEQ ID :700:

(i) SEQUENCE CHARACTERISTICS:
(A) LENGTH: 282 base pairs
(B) TYPE: nucleic acid
(C) STRANDEDNESS: double
(D) TOPOLOGY: linear

(xi) SEQUENCE DESCRIPTION: SEQ ID :700:

AGCAACAAAA ATGTTTCCAC GGAGATCAGG ATGACTTGCT GAAGCTCAGT 50

GGAGGCTCAA AAGAGGACAC AAAAGTGAAC AGAATGATCT TCCTACGCAC 100

AACACAAACA TCAGTTAATG CTCCATCCAC GCTGCTTAAA GAGCATTCCT 150

GTCCTAGCAA AATGGGCAAG TCCCTCTACC CCCCACCCTC ACTTGGCATG 200

CTTACATTAA TAGCTAAAGT CAATCCTGTA ATGAAATAAA GCAAATGGCA 250

GCTGTCTAGT AGCCTCCACT ACCGCAAATA TC 282

(2) INFORMATION FOR SEQ ID :701:

(i) SEQUENCE CHARACTERISTICS:
(A) LENGTH: 255 base pairs
(B) TYPE: nucleic acid
(C) STRANDEDNESS: double

(D) TOPOLOGY: linear

(xi) SEQUENCE DESCRIPTION: SEQ ID :701:

GCCTGCGCCG AACAAACGCA CGGAATGAGC GAGTCCCTCT GGGAGCCCAA 50

CATGGATCCG AATCACCTGT TTGAAACCAT CTCCCAAGCC ATGCTGAATG 100

CTGCGGACCA GGATGCAGTG CCAGGCATGG GAGTCATTGC CCACATCACC 150

GAGAAGGACA AAATCACCAC CAGGACACTG AAGGCCCGAA TGGACTAACC 200

CTGTTCCCAG AGCCCACTTT TCCCCTATTT TGGAAATAAA ATAGCCTGTC 250

TTTCG 255

(2) INFORMATION FOR SEQ ID :702:

(i) SEQUENCE CHARACTERISTICS:
(A) LENGTH: 89 base pairs
(B) TYPE: nucleic acid
(C) STRANDEDNESS: double
(D) TOPOLOGY: linear

(xi) SEQUENCE DESCRIPTION: SEQ ID :702:

GCCTGCGCCG AGCACAAGAC AATGATGAAC ATTCTAAAAA AAAAGAATGA 50

CGCACATTTT AATAAAGCAC AGCACAAACT GTTCTTTCC 89

(2) INFORMATION FOR SEQ ID :703:

(i) SEQUENCE CHARACTERISTICS:
(A) LENGTH: 96 base pairs
(B) TYPE: nucleic acid
(C) STRANDEDNESS: double
(D) TOPOLOGY: linear

(xi) SEQUENCE DESCRIPTION: SEQ ID :703:

GCCAGCGCCG AGATCCTGGC GGCCTACCGC CCCCCGCCGC ACCCCCGCTA 50

GCGCCCCACC CCGCGTCTAT CGCCCAATAA AGGCATCTTT GTCGGG 96

(2) INFORMATION FOR SEQ ID :704:

(i) SEQUENCE CHARACTERISTICS:
(A) LENGTH: 123 base pairs
(B) TYPE: nucleic acid
(C) STRANDEDNESS: double
(D) TOPOLOGY: linear

(xi) SEQUENCE DESCRIPTION: SEQ ID :704:

GCCTGCGCCG AACAAACATA CAGAACGCAA CGAGTCCCTC TGAAGGCCCA 50

ACACGGATCC GAATCACCTG GCCCGAAACC ACCTACCTAG CCATGATGAA 100

TGCTGAGGAC CCAGATGCAG TAC 123

(2) INFORMATION FOR SEQ ID :705:

(i) SEQUENCE CHARACTERISTICS:
(A) LENGTH: 262 base pairs
(B) TYPE: nucleic acid
(C) STRANDEDNESS: double
(D) TOPOLOGY: linear

(xi) SEQUENCE DESCRIPTION: SEQ ID :705:

GCTTGATGCC TGCGCCGAAC AAACATACAG AATGCGGCGA GTCCCTCTGG 50

ACGCCCAACA CGGATCCGGA TCACCTATCT GAAACCATCT CCCAAGCCAT 100

GCTGAATGCT GCGGACCAGG ATGCAGTGCC AGGCATGGGA GCCATTGCCC 150

ACATCACCGA GAAGGACAAA ATCACCACCA GGACACTGAA GGCCCGAATG 200

GACTAACCCT GTTCCCAGAG CCCACTTTTT TTCTTTTTCA GAAATAAAAC 250

AGCCTGTCTT TC 262

(2) INFORMATION FOR SEQ ID :706:

(i) SEQUENCE CHARACTERISTICS:
(A) LENGTH: 209 base pairs
(B) TYPE: nucleic acid
(C) STRANDEDNESS: double
(D) TOPOLOGY: linear

(xi) SEQUENCE DESCRIPTION: SEQ ID :706:

GCCAGCACCG ACCTAGCCTA AGCCGTCTAA AACCACCTGA GCCCCTGAGG 50

AAACCTGTGG TCACACTGGG CCCTCCTCAG GAACTCTCCC TGCGCAGAGG 100

CGTGTCTTAG CACTGCCCCC CTCCCTAGCC CCTTATTTGG CGGCGGAAGC 150

GGCCTCCACC CCTTCCCTGT TTGCAAACAC TCTGCGGAGA AAAGAGGACT 200

TCAGGGAGT 209

(2) INFORMATION FOR SEQ ID :707:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :707:

GCCAGCACCG AGATGACGAG CTTTCTGCTG CCACCCACAA GCAGAGGGAC 50

TCGGAGATCA CGCAGCAGAA GCAGAAAAAG GCAAACAAGA AGAAGGAGGA 100

ACCCAAGCAG CTTTGCGGCT TCACGCCCAA CCCTCTCGCC CTTCACCTGT 150

GAGCCTGGAG CCAGTCCCAC 170

(2) INFORMATION FOR SEQ ID :708:

(i) SEQUENCE CHARACTERISTICS:
(A) LENGTH: 160 base pairs
(B) TYPE: nucleic acid
(C) STRANDEDNESS: double

(D) TOPOLOGY: linear

(xi) SEQUENCE DESCRIPTION: SEQ ID :708:

GCCACCGTCG AGGATTCAGC AGCCTCCCCC TTGAGCCCCC TCACTTCCCG 50

ACGTTCCGTT TCCCCCTGCC CGCCTTTTTC CGCCACCACC GCCGCCGCCT 100

TCTGCAGGCC GTTTCCACCG AGGAAAAGGA ATCGTATTGT ATACCCGCTA 150

CCCAGAACCT 160

(2) INFORMATION FOR SEQ ID :709:

(i) SEQUENCE CHARACTERISTICS:
(A) LENGTH: 51 base pairs
(B) TYPE: nucleic acid
(C) STRANDEDNESS: double
(D) TOPOLOGY: linear

(xi) SEQUENCE DESCRIPTION: SEQ ID :709:

GCCAGCACCG AGCAACCTGG GTCCAAATAA AAACTAAACT GCAAACTCCT 50

G 51

(2) INFORMATION FOR SEQ ID :710:

(i) SEQUENCE CHARACTERISTICS:
(A) LENGTH: 64 base pairs
(B) TYPE: nucleic acid

(C) STRANDEDNESS: double
(D) TOPOLOGY: linear

(xi) SEQUENCE DESCRIPTION: SEQ ID :710:

GCCTGCGCCG ACCACCCCTT CCTTTTCTTC ATCCAGCACA GCAAGACCAA 50

CAGGATTCTC TTCT 64

(2) INFORMATION FOR SEQ ID :711:

(i) SEQUENCE CHARACTERISTICS:
(A) LENGTH: 104 base pairs
(B) TYPE: nucleic acid
(C) STRANDEDNESS: double
(D) TOPOLOGY: linear

(xi) SEQUENCE DESCRIPTION: SEQ ID :711:

CCAGCCAAAG ATTCCCAGGC TTTCTTGTCT CAGCAACTTT CCCATCTTCT 50

CTCTCTTGGA TGATGTTTGC CGTCAGCATT CACCAAATAA ACTTGCTCTC 100

TGGG 104

(2) INFORMATION FOR SEQ ID :712:

(i) SEQUENCE CHARACTERISTICS:
(A) LENGTH: 68 base pairs
(B) TYPE: nucleic acid

(C) STRANDEDNESS: double
(D) TOPOLOGY: linear

(xi) SEQUENCE DESCRIPTION: SEQ ID :712:

CTAGAAATAG ACCCACAATT TAGAGACAAT CTATACTAGA TTTATCTCCT 50

TTGTTTTTAG TTGAAGGC 68

(2) INFORMATION FOR SEQ ID :713:

(i) SEQUENCE CHARACTERISTICS:
(A) LENGTH: 127 base pairs
(B) TYPE: nucleic acid
(C) STRANDEDNESS: double
(D) TOPOLOGY: linear

(xi) SEQUENCE DESCRIPTION: SEQ ID :713:

AGCGAATGTA AGGGGGCCTT GGACGACCCG ACGCCGGACT ACATGAACCT 50

GCTGGGCATG ATCTTCAGCA TGTGCGGCCT CATGCTTAAG CTGAAGTGGT 100

GTGCTTGGGT CGCTGTCTAC TGCTCCT 127

(2) INFORMATION FOR SEQ ID :714:

(i) SEQUENCE CHARACTERISTICS:
(A) LENGTH: 90 base pairs
(B) TYPE: nucleic acid
(C) STRANDEDNESS: double

(D) TOPOLOGY: linear

(xi) SEQUENCE DESCRIPTION: SEQ ID :714:

CTTGGGAGAT AACAGTTTCC CCTCTCCCTC CCCCTGCAGA TTTCCAGCGC 50

CCGGACCAAG GCCAAGTAAG CCTCTACAAA CCTAGCATTT 90

(2) INFORMATION FOR SEQ ID :715:

(i) SEQUENCE CHARACTERISTICS:
(A) LENGTH: 69 base pairs
(B) TYPE: nucleic acid
(C) STRANDEDNESS: double
(D) TOPOLOGY: linear

(xi) SEQUENCE DESCRIPTION: SEQ ID :715:

CCAGCGCCGA GGTTGTATAT TTGTAGGTGC AGGCACACGA CCAGGACACA 50

ACAAAAATCT GAAAACATC 69

(2) INFORMATION FOR SEQ ID :716:

(i) SEQUENCE CHARACTERISTICS:
(A) LENGTH: 102 base pairs
(B) TYPE: nucleic acid
(C) STRANDEDNESS: double
(D) TOPOLOGY: linear

(xi) SEQUENCE DESCRIPTION: SEQ ID :716:

GCCGAGGAGA ACCCCCGCTC CCTGAGGAGG ACCTGTCCAA ACTCTTCAAA 50

CCACCACAGC CGCCTGCCAG GATGGACTCG CTGCTCATTG CAGGCCAGAT 100

AA 102

(2) INFORMATION FOR SEQ ID :717:

(i) SEQUENCE CHARACTERISTICS:
(A) LENGTH: 124 base pairs
(B) TYPE: nucleic acid
(C) STRANDEDNESS: double
(D) TOPOLOGY: linear

(xi) SEQUENCE DESCRIPTION: SEQ ID :717:

AGCGAATGTA ACCCGGCCTT GGACGACCCG ACGCCGGACT ACATGAACCT 50

GCTGGGCATG ATCTTCAGCA TGTGCGGCCT CATGCTTAAG CTGAAGTGGT 100

GTGCTTGGGA TACGCTGTCT ACAG 124

(2) INFORMATION FOR SEQ ID :718:

(i) SEQUENCE CHARACTERISTICS:
(A) LENGTH: 123 base pairs
(B) TYPE: nucleic acid
(C) STRANDEDNESS: double
(D) TOPOLOGY: linear

(xi) SEQUENCE DESCRIPTION: SEQ ID :718:

GGAAAACCGT GTACTATTAG CCATGGTCAA CCCCACCGTG TTCTTCGACA 50

TTGCCGTCGA CGGGAGCCCT TGGCGCGCGT CTCCTTTGAG CTGTTTGCAG 100

ACAAGGTCCC AAAGACAGCA GAA 123

(2) INFORMATION FOR SEQ ID :719:

(i) SEQUENCE CHARACTERISTICS:
(A) LENGTH: 54 base pairs
(B) TYPE: nucleic acid
(C) STRANDEDNESS: double
(D) TOPOLOGY: linear

(xi) SEQUENCE DESCRIPTION: SEQ ID :719:

ACCACCCCTT CCTTTTCTTC ATCCAGCACA GCAAGACCAA CGGGATTCTC 50

TTCT 54

(2) INFORMATION FOR SEQ ID :720:

(i) SEQUENCE CHARACTERISTICS:
(A) LENGTH: 117 base pairs
(B) TYPE: nucleic acid
(C) STRANDEDNESS: double
(D) TOPOLOGY: linear

(xi) SEQUENCE DESCRIPTION: SEQ ID :720:

GACCACAGGG CCTTCAATCC TTTTTTGTTT TCAACAGTCT TGCTGAATTA 50

AGCAGAAAGG GCCTTGAATC CTGGCCTGGA ATTTGGGCAG ATATAGCATT 100

AATAAAACTG TGCACTC 117

(2) INFORMATION FOR SEQ ID :721:

(i) SEQUENCE CHARACTERISTICS:
(A) LENGTH: 55 base pairs
(B) TYPE: nucleic acid
(C) STRANDEDNESS: double
(D) TOPOLOGY: linear

(xi) SEQUENCE DESCRIPTION: SEQ ID :721:

GACCACCCCT TCCTTTTCTT CATCCAGCAC AGCAAGACCA ACGGGATTCT 50

CTTCT 55

(2) INFORMATION FOR SEQ ID :722:

(i) SEQUENCE CHARACTERISTICS:
(A) LENGTH: 92 base pairs
(B) TYPE: nucleic acid
(C) STRANDEDNESS: double
(D) TOPOLOGY: linear

(xi) SEQUENCE DESCRIPTION: SEQ ID :722:

GACTTCACGA ACTACACAGG TCTTACCATT GACCTAAGAT CAATCTGAAC 50

ATTCTTAGCC CAGTCAGGGA GCTCTGCTTC CTAGAAAGGC AT 92

(2) INFORMATION FOR SEQ ID :723:

(i) SEQUENCE CHARACTERISTICS:

(A) LENGTH: 55 base pairs

(B) TYPE: nucleic acid

(C) STRANDEDNESS: double

(D) TOPOLOGY: linear

(xi) SEQUENCE DESCRIPTION: SEQ ID :723:

TATTTCTAGG TGCAGGTATA TGATTGCCAT ATAATAAAAA TCTGAAAACA 50

TCCCC 55

(2) INFORMATION FOR SEQ ID :724:

(i) SEQUENCE CHARACTERISTICS:

(A) LENGTH: 164 base pairs

(B) TYPE: nucleic acid

(C) STRANDEDNESS: double

(D) TOPOLOGY: linear

(xi) SEQUENCE DESCRIPTION: SEQ ID :724:

GTGTACTATT AGCCATGGTC AACCCCACCG TGTTCTTCGA CATTGCCGTC 50

GACGGGCGAG CCCTTGGCGC GCGTCCTTTG AGCTGTTTGC AGACAAGGTC 100

CCAAAGACAG CAGAAAAATT TTTCGTGCTC TGAGCACTGG AGAGAAAGGA 150

TTTGGTTATA AGGG 164

(2) INFORMATION FOR SEQ ID :725:

(i) SEQUENCE CHARACTERISTICS:
(A) LENGTH: 110 base pairs
(B) TYPE: nucleic acid
(C) STRANDEDNESS: double
(D) TOPOLOGY: linear

(xi) SEQUENCE DESCRIPTION: SEQ ID :725:

GTGAGAAGCT GCAGCGGTGA CCTGGAGGCT GCGATGTGGA GGTGGAGGAC 50

ACACCCTCAA CCGTTGCTCC TGTAGCTTCC GAGTCCTGGT GGTGTCGGCC 100

AAGTTCAGTA 110

(2) INFORMATION FOR SEQ ID :726:

(i) SEQUENCE CHARACTERISTICS:
(A) LENGTH: 163 base pairs
(B) TYPE: nucleic acid
(C) STRANDEDNESS: double
(D) TOPOLOGY: linear

(xi) SEQUENCE DESCRIPTION: SEQ ID :726:

CCGTGTACTA TTAGCCATGG TCAACCCCAC CGTGTTCTTC GACATTGCCG 50

TCGACGGGAG CCCTTGGCGC GCGTCTCCTT TGAGCTGTTT GCAGACAAGG 100

TCCCAAAGAC AGCAGAAAAT TTTCGTGCTC TGAGCACTGG AGAGAAAGGA 150

TTTGGTTATA AGG 163

(2) INFORMATION FOR SEQ ID :727:

(i) SEQUENCE CHARACTERISTICS:
(A) LENGTH: 105 base pairs
(B) TYPE: nucleic acid
(C) STRANDEDNESS: double
(D) TOPOLOGY: linear

(xi) SEQUENCE DESCRIPTION: SEQ ID :727:

GAGGGCGAGT TCGAGGAGGA GGCTGAGGAG GAGGTGGCCT AGAGCCTTCA 50

GTCACTGGGG AAAGCAGGGA AGCAGTGTGA ACTCTTTATT CACTCCCAGC 100

CTGTT 105

(2) INFORMATION FOR SEQ ID :728:

(i) SEQUENCE CHARACTERISTICS:
(A) LENGTH: 186 base pairs
(B) TYPE: nucleic acid
(C) STRANDEDNESS: double
(D) TOPOLOGY: linear

(xi) SEQUENCE DESCRIPTION: SEQ ID :728:

GCCAGCGCCG AAAAGCCAAG ACTTCATGAA CTACACAGGT CTTACCATTG 50

ACCTAAGATC AATCTAAACT ATCTTAGCCC AGTCAGGGAG CTCTATTCCT 100

AGGAAGGCAT CTTTCGCCAG TGGATTCGCC TCAAGGTCGA GGCCGCCACT 150

GGAAGACGAA AAATAGAACT CCCTTAGAGT AGACAA 186

(2) INFORMATION FOR SEQ ID :729:

(i) SEQUENCE CHARACTERISTICS:
(A) LENGTH: 167 base pairs
(B) TYPE: nucleic acid
(C) STRANDEDNESS: double
(D) TOPOLOGY: linear

(xi) SEQUENCE DESCRIPTION: SEQ ID :729:

GCCAGCGCCG AGGTTGCATA TTCTAGGCGC AGGTATATGA TTGCCATATA 50

ATAAAAACCT GAAAACATCC CACCCGGGAA AAAAAAAAAA AAAAAAAAAA 100

AAAAACACCC CCCCCCCACA AAAAACTCAA ATTCCCCTCC CAAAAAACCC 150

CCTCAAAATC AAAAAAC 167

(2) INFORMATION FOR SEQ ID :730:

(i) SEQUENCE CHARACTERISTICS:
(A) LENGTH: 64 base pairs
(B) TYPE: nucleic acid
(C) STRANDEDNESS: double
(D) TOPOLOGY: linear

(xi) SEQUENCE DESCRIPTION: SEQ ID :730:

GCCTGCGCCG ACCACCCCTT CCTTTTCTTC ATCCAGCACA GCAAGACCAA 50

CAGGATTCTC TTCT 64

(2) INFORMATION FOR SEQ ID :731:

(i) SEQUENCE CHARACTERISTICS:
(A) LENGTH: 96 base pairs
(B) TYPE: nucleic acid
(C) STRANDEDNESS: double
(D) TOPOLOGY: linear

(xi) SEQUENCE DESCRIPTION: SEQ ID :731:

GCCAGCGCCG AGACCCTGGC GGCCTACCGG CCCCCCGTGC ACCCCCGCTA 50

GCGCCCCACC CCGCGTCTAT CGCCCAATAA AGGCATCTTT GCCGGG 96

(2) INFORMATION FOR SEQ ID :732:

(i) SEQUENCE CHARACTERISTICS:
(A) LENGTH: 84 base pairs
(B) TYPE: nucleic acid

(C) STRANDEDNESS: double
(D) TOPOLOGY: linear

(xi) SEQUENCE DESCRIPTION: SEQ ID :732:

GCCTGCGCCG ACCACCCCTT CCTTTTCTTC ATCCAGCACA GCAAGACCAA 50

CAGGATTCTC TTCTGCAGCC GCCACCGCGT CGGA 84

(2) INFORMATION FOR SEQ ID :733:

(i) SEQUENCE CHARACTERISTICS:
(A) LENGTH: 96 base pairs
(B) TYPE: nucleic acid
(C) STRANDEDNESS: double
(D) TOPOLOGY: linear

(xi) SEQUENCE DESCRIPTION: SEQ ID :733:

GCCAGCGCCG AGATCCTGGC GGCCTACCGG CCCTCAGTGC ACCCCCGCTA 50

GCGCCCCACC CCGCGTCTAT CGCCCAATAA AGGCATCTTT GCCGGG 96

(2) INFORMATION FOR SEQ ID :734:

(i) SEQUENCE CHARACTERISTICS:
(A) LENGTH: 96 base pairs
(B) TYPE: nucleic acid
(C) STRANDEDNESS: double
(D) TOPOLOGY: linear

(xi) SEQUENCE DESCRIPTION: SEQ ID :734:

GCCAGCGCCG AGATCCTGGC GGCCTACCAG CCCTCCGTGC ACCCCCGCTA 50

GCGCCCCACC CCGCGTCTAT CGCCCAATAA AGGCATCTTT GCCGGG 96

(2) INFORMATION FOR SEQ ID :735:

(i) SEQUENCE CHARACTERISTICS:
(A) LENGTH: 97 base pairs
(B) TYPE: nucleic acid
(C) STRANDEDNESS: double
(D) TOPOLOGY: linear

(xi) SEQUENCE DESCRIPTION: SEQ ID :735:

GCCAGCGCCG AGATCCTGGC GGCCTACCAG CTCCTCAGTG CACCCCCACT 50

AGCACCCCAC CCCGCATCTA TCGCCCAATA AAGGCATCTT TGCCGGG 97

(2) INFORMATION FOR SEQ ID :736:

(i) SEQUENCE CHARACTERISTICS:
(A) LENGTH: 127 base pairs
(B) TYPE: nucleic acid
(C) STRANDEDNESS: double
(D) TOPOLOGY: linear

(xi) SEQUENCE DESCRIPTION: SEQ ID :736:

GCCAGCGCCG ACCACAGGGC CTTGAATCCT TTTTTGTTTT CAACAGTCTT 50

GCTGAATTAA GCAGAAAGGG CCTTGAATCC TGGCCTAGAA TTTGGGCAGA 100

TACAGCATTA ACAAAACCGC GCATCTC 127

(2) INFORMATION FOR SEQ ID :737:

(i) SEQUENCE CHARACTERISTICS:
(A) LENGTH: 64 base pairs
(B) TYPE: nucleic acid
(C) STRANDEDNESS: double
(D) TOPOLOGY: linear

(xi) SEQUENCE DESCRIPTION: SEQ ID :737:

GCCTGCGCCG ACCACCCCTT CCTTTTCTTC ATCCAGCACA GCAAGACCAA 50

CAGGATTCTC TTCT 64

(2) INFORMATION FOR SEQ ID :738:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :738:

GCCAGCGCCG ACAATGCCCA GAATCCAGAA CTTTGCCTAT CACTCTCCCC 50

AACAACCTAG ACGCGAAAAC AGAATAAACT TCACCCAG 88

(2) INFORMATION FOR SEQ ID :739:

(i) SEQUENCE CHARACTERISTICS:

(A) LENGTH: 95 base pairs

(B) TYPE: nucleic acid

(C) STRANDEDNESS: double

(D) TOPOLOGY: linear

(xi) SEQUENCE DESCRIPTION: SEQ ID :739:

GCCAGCGCCG AGATCCTGGC GGCCTACCGC CCCCCGTGCA CCCCCGCTAG 50

CGCCCCACCC CGCGTCTACC GCCCAATAAA GGCATCTTTG CCGGG 95

(2) INFORMATION FOR SEQ ID :740:

(i) SEQUENCE CHARACTERISTICS:

(A) LENGTH: 245 base pairs

(B) TYPE: nucleic acid

(C) STRANDEDNESS: double

(D) TOPOLOGY: linear

(xi) SEQUENCE DESCRIPTION: SEQ ID :740:

GCCACCCCGA AGACGTATCA AGGCCCTCGA ACAACCCCGA GAGCTGATGA 50

GATGAATGAG AAGATGCCGA ACTTTGCGCA CTCCATGCCC CTGCTGCAGA 100

CAGTAACCAA GAGCAAGGAC CAGGGCACCC ATGAGGATTA TGTCGAAGGA 150

CTTCGGGTGT TTGACAAGGA AGGAAATGGC ACCGTCATGG GTGCTGAAAT 200

CCGGCATGTT CTTGTCACAC TGGGTGAGAA GATGACAGAG GAAGA 245

(2) INFORMATION FOR SEQ ID :741:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :741:

GCCTGCGCCG ACGATGCCCA GAATCCAGAA CTTCGTCTAT CACTCTCCCC 50

AACAACCTAG ATGTGAAAAC AGAATAAACT TCACCCAG 88

(2) INFORMATION FOR SEQ ID :742:

(i) SEQUENCE CHARACTERISTICS:
(A) LENGTH: 242 base pairs
(B) TYPE: nucleic acid
(C) STRANDEDNESS: double
(D) TOPOLOGY: linear

(xi) SEQUENCE DESCRIPTION: SEQ ID :742:

GCCGGAGCCA AAGCAAGCCA GAAGACTAAC AGTACTAACT ACAAATATTC 50

GCACCTCGAT CGCAGTACCC AGGTTCTCAC GTAGCTGAAG NAATGTATTA 100

CTCTGATAGT CTTCATTCGG ATAGACTAAA GCGTGTGCTG ACTGGAGATG 150

AGGTAAAGAA GATATGTATG CAACGTCCAG TCAAAACGGA TGGCAAGGTT 200

CGAGTCATGT CATATACCTT GCTGGATTAA TGGATGTCAT TA 242

(2) INFORMATION FOR SEQ ID :743:

(i) SEQUENCE CHARACTERISTICS:
(A) LENGTH: 64 base pairs
(B) TYPE: nucleic acid
(C) STRANDEDNESS: double
(D) TOPOLOGY: linear

(xi) SEQUENCE DESCRIPTION: SEQ ID :743:

GCCTGCGCCG ACCACCCCTT CCTTTTCTTG ATCTAGCACA GCAAGACCAA 50

CGGGATTCTC TTCT 64

(2) INFORMATION FOR SEQ ID :744:

(i) SEQUENCE CHARACTERISTICS:
(A) LENGTH: 69 base pairs
(B) TYPE: nucleic acid
(C) STRANDEDNESS: double
(D) TOPOLOGY: linear

(xi) SEQUENCE DESCRIPTION: SEQ ID :744:

GCCAGCGCCG AGGTTGTATA TTTCTAGGTG CAGGTATATG ATTGCCATAT 50

AATAAAAATT TGAAAACAT 69

(2) INFORMATION FOR SEQ ID :745:

(i) SEQUENCE CHARACTERISTICS:
(A) LENGTH: 74 base pairs
(B) TYPE: nucleic acid
(C) STRANDEDNESS: double
(D) TOPOLOGY: linear

(xi) SEQUENCE DESCRIPTION: SEQ ID :745:

GCCAGCTCCG AGGTTGTATA TTTCCAGGTG CAGATATATG ATTGCCATAT 50

AATAAAAATC TGAAAACATC CCAC 74

(2) INFORMATION FOR SEQ ID :746:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :746:

GCCTGCGCCG ACGATGCCCA GAATCCAGAA CTTTGTCTAT CACTCTCCCC 50

AACAACCTAG ATGTGAAAAC AGGATAAACT TCACCCAG 88

(2) INFORMATION FOR SEQ ID :747:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :747:

GCCTGCGCCG ACGATGCCCA GAATCCAGAA CTTTGTCTAT CACTCTCCCC 50

AACAACCTAG ATGTGAAAAC AGAATAAACT TCACCCAG 88

(2) INFORMATION FOR SEQ ID :748:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :748:

GCCAGCACCG ATGAATTCCC TTTGACCCAA GTCTGCAGCA GGTCCCTTTT 50

GCGCTTCCTT CCCCTCAGGC AGCCTCTTTC CCCCTGGGCC ACTCCCGGGG 100

GCGAGGGGGC TACCCCTTTC CCAGGCTTTT TATTCCCGTG GGGCTCACCC 150

CAAAGCATTA AAAGCAGCTT TGCAATTC 178

(2) INFORMATION FOR SEQ ID :749:

(i) SEQUENCE CHARACTERISTICS:
(A) LENGTH: 235 base pairs
(B) TYPE: nucleic acid
(C) STRANDEDNESS: double
(D) TOPOLOGY: linear

(xi) SEQUENCE DESCRIPTION: SEQ ID :749:

GCCAGCACCG AAAAGCCAAG ACTTCATGAA CTACACAGGT CTTACCATTG 50

ACCTAAGATC AATCTGAACT ATCTTAGCCC AGTCAGGGAG CTCTATTCCT 100

AGAAAGGCAT CTTTCGCCAG TGGATTCGCC CCAAGGTTGA GGCCGCCATT 150

GGAAGACGAA AAATTGCACT CCCTTGGCGC AGACAAACAC CAGTTCCCAT 200

TGGCGCTGCT GCCTATAACA AACACTTTTT TTTTT 235

(2) INFORMATION FOR SEQ ID :750:

(i) SEQUENCE CHARACTERISTICS:
(A) LENGTH: 87 base pairs
(B) TYPE: nucleic acid
(C) STRANDEDNESS: double
(D) TOPOLOGY: linear

(xi) SEQUENCE DESCRIPTION: SEQ ID :750:

GCCTACGCCG AAAACCCCTT CCTTTTTTTC ATCCAGAAAA GCAAGAGAAA 50

AAGGATTCTC TTCTGCGGCC GCCAACGCGT CGGAGAT 87

(2) INFORMATION FOR SEQ ID :751:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :751:

GCCTCCGCCG ATTCGTGACC AAGAAGGCTC TATGCATTCA GGCTTTCCAG 50

GAGACTCAAA AGCTGAAGAA GCAAAGAAGA GCCTTAAAGG CTGCAGCAGC 100

AGTCCNAAAA ACAAGCAGAG CAGAGGAACC CAGACAGCCC TGCCAAAGCC 150

ATGCCAAAGA CACTCAAAGA 170

(2) INFORMATION FOR SEQ ID :752:

(i) SEQUENCE CHARACTERISTICS:
(A) LENGTH: 72 base pairs
(B) TYPE: nucleic acid
(C) STRANDEDNESS: double
(D) TOPOLOGY: linear

(xi) SEQUENCE DESCRIPTION: SEQ ID :752:

GCCAGCACCG AGGTTGCATA TTTCTAGGCG CAGGCATATG ATTGCCATAT 50

AATAAAAAAA TGAAAACACC CC 72

(2) INFORMATION FOR SEQ ID :753:

(i) SEQUENCE CHARACTERISTICS:
(A) LENGTH: 122 base pairs
(B) TYPE: nucleic acid
(C) STRANDEDNESS: double
(D) TOPOLOGY: linear

(xi) SEQUENCE DESCRIPTION: SEQ ID :753:

GCCAGCGCCG AGGACACCAA GAAGGTTTTG GACAGCGTGG GCATCGAGGC 50

GGACAACAAC CAGCTCAACA AGGCCATTAG TGAGCTGAAT GAAAAAAACA 100

TTGAAGACGT TATTGNCCAG GA 122

(2) INFORMATION FOR SEQ ID :754:

(i) SEQUENCE CHARACTERISTICS:
(A) LENGTH: 243 base pairs
(B) TYPE: nucleic acid
(C) STRANDEDNESS: double
(D) TOPOLOGY: linear

(xi) SEQUENCE DESCRIPTION: SEQ ID :754:

GCCACCGCCG ACTGAAACTG CACCGACCCC CCCTCGAGGA CCTGCTCTTA 50

GGTTCAGAAG CAAACCTCAC GTGCACACTG ACCGACCTAA GAGACGCCTC 100

AGGCGCCACC TTCACCTGGA CACCCTCAAG CGGGAAGAGC ACTGTTCAAG 150

GACCACCTAA GCATGACCTC TACGGCTGCT ACAGCGTGTC CAGTGCCCTG 200

CCGGGCTGCG CCGAGCCATG GAACCATGGG AAGACCTTCA CTT 243

(2) INFORMATION FOR SEQ ID :755:

(i) SEQUENCE CHARACTERISTICS:
(A) LENGTH: 71 base pairs
(B) TYPE: nucleic acid
(C) STRANDEDNESS: double
(D) TOPOLOGY: linear

(xi) SEQUENCE DESCRIPTION: SEQ ID :755:

GCCAGCGCCG AGGTTGCATA TTTCTAGGCG CAGGCATATG ATTGCCATAT 50

AACAAAAACC TGAAAGCATC A 71

(2) INFORMATION FOR SEQ ID :756:

(i) SEQUENCE CHARACTERISTICS:
(A) LENGTH: 243 base pairs
(B) TYPE: nucleic acid

(C) STRANDEDNESS: double
(D) TOPOLOGY: linear

(xi) SEQUENCE DESCRIPTION: SEQ ID :756:

GCCACCGCCG AGTGACGCCA AAGCCCTTGG TTGACTCTAA CAGCCCCGTG 50

GGCGCGCGGG AGGCCGGGCG CTCTAGGGTC TACCTATCAG CGCAATCGTT 100

TAGTGCTTTT TCAGTGGGGC GGGGCGGGAA GCAGGCGGGA CCAGGCAGCC 150

AGTTCTCAAA GGCTGCGGGG CCGACTAGAG GCCACAGCCC CTCACCCCTA 200

GACGTCGCCA ACCAGAACTG ACGCGCGACC TCCTGGGCGC TGA 243

(2) INFORMATION FOR SEQ ID :757:

(i) SEQUENCE CHARACTERISTICS:
(A) LENGTH: 64 base pairs
(B) TYPE: nucleic acid
(C) STRANDEDNESS: double
(D) TOPOLOGY: linear

(xi) SEQUENCE DESCRIPTION: SEQ ID :757:

GCCTGCGCCG ACCACCCCTT CCTTTTTTTT ATCCAGCACA GCAAGACCAA 50

CAGGATTCTC TTCC 64

(2) INFORMATION FOR SEQ ID :758:

(i) SEQUENCE CHARACTERISTICS:
(A) LENGTH: 243 base pairs
(B) TYPE: nucleic acid
(C) STRANDEDNESS: double
(D) TOPOLOGY: linear

(xi) SEQUENCE DESCRIPTION: SEQ ID :758:

GCCAGCACCA AGTGACGCCA AAGCCCCTAG TTGACTCTAA CAGCCCCGTG 50

GGCGCGCGGG AGGCCGAGCG CTCTAGGGTC TACCTATCAG CGCAATCGTT 100

TAGCGCTTTT TCAATGGGGC AGGGCAGGAA GCAGGCGGGA CCAGGCAGCC 150

AGTTCTCAAA GGCTGCGGGG CCGACTAGAG GCCACAGCCC CTCACCCCTA 200

GACGTCGACA ACCAGAACTG ACGTGCGACC TCCCGGGCGC CGA 243

(2) INFORMATION FOR SEQ ID :759:

(i) SEQUENCE CHARACTERISTICS:
(A) LENGTH: 245 base pairs
(B) TYPE: nucleic acid
(C) STRANDEDNESS: double
(D) TOPOLOGY: linear

(xi) SEQUENCE DESCRIPTION: SEQ ID :759:

GCCACACCAG ACTTTCGACC CCCCAACCCT CTGAGGAAGA TGGGGGCAAG 50

AAGATCACGC TCCCCGCCTG TTCCCCCGCC GCTTTTCTCC TCTCTTCTCT 100

CTTCGCTCTC AGCTCCCCCT GTCCCCTCAG CTCCAGACGT AGGGGAGGGG 150

TTGCCACAGA CTCCCTGCTT GAAGCCTGCC CTTGCCTAAG ATGCTGGTAA 200

TGGCCATGGT ACCCCCTTCC GGGCATCTAC TCTGGTTTTC GGCCA 245

(2) INFORMATION FOR SEQ ID :760:

(i) SEQUENCE CHARACTERISTICS:
(A) LENGTH: 68 base pairs
(B) TYPE: nucleic acid
(C) STRANDEDNESS: double
(D) TOPOLOGY: linear

(xi) SEQUENCE DESCRIPTION: SEQ ID :760:

GCCAGCGCCG AGGTCGCATA TTTCTAGGCG CAGGTATATG ACTGCCATAT 50

AACAAAAACC NTGAAAAC 68

(2) INFORMATION FOR SEQ ID :761:

(i) SEQUENCE CHARACTERISTICS:
(A) LENGTH: 71 base pairs
(B) TYPE: nucleic acid
(C) STRANDEDNESS: double
(D) TOPOLOGY: linear

(xi) SEQUENCE DESCRIPTION: SEQ ID :761:

GCCAGCGCCG AGGTCGCATA TTTCTAGGCG CAGGTATATG ATTGCCATAT 50

AATAAAAACT GAAAACACCC C 71

(2) INFORMATION FOR SEQ ID :762:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :762:

GCCTGCGCCG ACGATGACCA GAATCCAGAA CTTTGCCTAT CACTCTCCCC 50

AACAACCTAG ACGCGAAAAC AGAATAAACT TCACCCAG 88

(2) INFORMATION FOR SEQ ID :763:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :763:

GCCTGCGCCG ACAAACAAAA CCTGGAGGCC ATTCTGCACA GCCTGCCCGA 50

GAACTGTGCC AGCTGGCGGT GAGGGCTGCC CAGATCCCCG GCACACACTC 100

CCCCACCTGC TGTTTACATG ACCCAGGGGG CGCACACTAC CCCACAGGCG 150

CGCCCATACA GACATTCCCC GGAGCCGGCT GCTGCGAACT CGACCCCGTG 200

CGGATAGTCA CACTCCCTGC CGA 223

(2) INFORMATION FOR SEQ ID :764:

(i) SEQUENCE CHARACTERISTICS:
(A) LENGTH: 70 base pairs
(B) TYPE: nucleic acid
(C) STRANDEDNESS: double
(D) TOPOLOGY: linear

(xi) SEQUENCE DESCRIPTION: SEQ ID :764:

GCCAGCGCCG AGGTTGCATA TTTCTAGGCG CAGGTATATG ATTGCCATAT 50

AATAAAAATC TGAAAACACC 70

(2) INFORMATION FOR SEQ ID :765:

(i) SEQUENCE CHARACTERISTICS:
(A) LENGTH: 118 base pairs
(B) TYPE: nucleic acid
(C) STRANDEDNESS: double
(D) TOPOLOGY: linear

(xi) SEQUENCE DESCRIPTION: SEQ ID :765:

GCCAGCGCCG ACCGCCCCAA AAATACTCCC ACCGGGATCA GGGCGATTAA 50

TGAAGACTCA ACGGGGACTA AAAAGGGGCC CCAAAAAAAA CCAAACCACC 100

TTTCTACGTA CCGTATAG 118

(2) INFORMATION FOR SEQ ID :766:

(i) SEQUENCE CHARACTERISTICS:
(A) LENGTH: 88 base pairs
(B) TYPE: nucleic acid
(C) STRANDEDNESS: double
(D) TOPOLOGY: linear

(xi) SEQUENCE DESCRIPTION: SEQ ID :766:

GCCAGCGCCG ACGATGCCCA GAATCCAGGA CTTTGCCTAT CACTCTCCCC 50

AACAACCTAG ACGCGAAAAC AGAATAAACT TCACCCAG 88

(2) INFORMATION FOR SEQ ID :767:

(i) SEQUENCE CHARACTERISTICS:
(A) LENGTH: 91 base pairs
(B) TYPE: nucleic acid
(C) STRANDEDNESS: double
(D) TOPOLOGY: linear

(xi) SEQUENCE DESCRIPTION: SEQ ID :767:

GCCACCGCCG ACTCCAGGCA CTCACTCAAA CTTGATCTTC AACTCTGCAT 50

ACAAGCAGAA GCAATAAACC AATCTGATTT TCTTTTCAAT T 91

(2) INFORMATION FOR SEQ ID :768:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :768:

GCCTGCGCCG AACAAATATA CAGAATGCGC GAGTCCCTCT GGAAGCCCAA 50

CATGGATCCG GATCACCTGT TTGAAACCAT CTCCCAAGCC ATGCTGAATG 100

CTGCGGACCA GGATGCAGTG CCAGGCATGG GAGTCATTGC CCACATCACC 150

GAGAAGGACA AAATCACCAC CAGGACACTG AAGGCCCGAA TGGACTCGCC 200

CTGCTCCCAG AGCCCACTTT TTT 223

(2) INFORMATION FOR SEQ ID :769:

(i) SEQUENCE CHARACTERISTICS:
(A) LENGTH: 37 base pairs
(B) TYPE: nucleic acid
(C) STRANDEDNESS: double
(D) TOPOLOGY: linear

(xi) SEQUENCE DESCRIPTION: SEQ ID :769:

GCCAGCGCCG AAAACACACA CTTTGCTTCT TACCTGC 37

(2) INFORMATION FOR SEQ ID :770:

(i) SEQUENCE CHARACTERISTICS:
(A) LENGTH: 37 base pairs
(B) TYPE: nucleic acid
(C) STRANDEDNESS: double
(D) TOPOLOGY: linear

(xi) SEQUENCE DESCRIPTION: SEQ ID :770:

GCCAGCGCCG AAAACACACA CTCTGCTTCT TACCTGC 37

(2) INFORMATION FOR SEQ ID :771:

(i) SEQUENCE CHARACTERISTICS:
(A) LENGTH: 91 base pairs
(B) TYPE: nucleic acid
(C) STRANDEDNESS: double
(D) TOPOLOGY: linear

(xi) SEQUENCE DESCRIPTION: SEQ ID :771:

GCCACCGCCG ACTCCAGGCA CTCACTCAAA CTCGATCTTC AACTCTGCAT 50

ACAAGCAGAA GCAATAAACC AATCTGATTT TCTTTTCAAT T 91

(2) INFORMATION FOR SEQ ID :772:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :772:

GCCTGCGCCG ANGCATTCCC TTTGACCTGA GTCTGCAGCA GGTCCCTTTT 50

GCGCTTCCTT CCCCTCAGGT AGCCTCTCTC CCCCTGGGCC ACTCCCGGGG 100

GTGAGGGGGT TACCCCTTCC CAGTGTTTTT TATTTCCGTG GGGCTCACCC 150

CAAAGTATTA AAAGCAACTT TGCAATT 177

(2) INFORMATION FOR SEQ ID :773:

(i) SEQUENCE CHARACTERISTICS:
(A) LENGTH: 151 base pairs
(B) TYPE: nucleic acid
(C) STRANDEDNESS: double
(D) TOPOLOGY: linear

(xi) SEQUENCE DESCRIPTION: SEQ ID :773:

AACATAAGAG GAGAAAGGAA GGGACATGAG GCATACCATT CCCCCCCCCA 50

GAATTAGAGG TAAAGGAATC CTAAAATAAA GAGCACAGCA GCAATCACAC 100

TCACAGGGTC CAGAGGCGTA TTCCTGGCCA TCTTCCTAGT ACTCGGTCCG 150

T 151

(2) INFORMATION FOR SEQ ID :774:

(i) SEQUENCE CHARACTERISTICS:
(A) LENGTH: 299 base pairs
(B) TYPE: nucleic acid
(C) STRANDEDNESS: double
(D) TOPOLOGY: linear

(xi) SEQUENCE DESCRIPTION: SEQ ID :774:

ATCTTAATGA ACATGGCATT TAAAATCCTG TAATTTCAAA CAGTGAACCA 50

CAATGCCGTA TGATCTAAAG GCTGCTGAAC CACAGCGTGG ATACACTTAA 100

CTGAGCTCCT CGCTGGGTCA AAGCACTCAT CTCCGAGTCT AAAGCTACAC 150

ACTATGGAGC ACACAACTCT GCCTCGCGCT GACACCAGAC AAACACGGCG 200

GGAGCTGAGG CGGACAGCTA CAGGACCACG AGCATAGACC ACGGCACCTG 250

AGACCATCTC TACGCAAGGA CTTAAGGAAG CAAATATAAT ATTAAAATA 299

(2) INFORMATION FOR SEQ ID :775:

(i) SEQUENCE CHARACTERISTICS:
(A) LENGTH: 185 base pairs
(B) TYPE: nucleic acid
(C) STRANDEDNESS: double

(D) TOPOLOGY: linear

(xi) SEQUENCE DESCRIPTION: SEQ ID :775:

TGAAGGAAAT GACTACCTTG CACTACATGA AAAGGATATC GTTGCCATTG 50

TGGCGTAATT TTCGTGCCGA GTGAATGTGG CGCAAACAAC TATATATCNA 100

AACCGTATAT TTAAAATGAA TTACTAGAGA GGGAATGTAA TCATGGCAAA 150

AGAAATTAAG TTTTAAGAAG ATGCTAGTGC TAAGC 185

(2) INFORMATION FOR SEQ ID :776:

(i) SEQUENCE CHARACTERISTICS:
(A) LENGTH: 255 base pairs
(B) TYPE: nucleic acid
(C) STRANDEDNESS: double
(D) TOPOLOGY: linear

(xi) SEQUENCE DESCRIPTION: SEQ ID :776:

ATCCAAGCCC ATACCCATCA ACAATGGGAC AGCGATTGGA TCACCAGCCA 50

TTTCACCACA CATGGCAACA AACTTACCTT CTTTAAGAGC GGCAGTAATC 100

ACATCGTCGA TCAAACGCAA AATCGATGGG TTATAAGGTC GATACAAATA 150

AGCAACCTTA TCATTACCAC GATCTGCAGC CATCGTATAA CCAATCAAAT 200

CGTCGGTACC AATCGAGAAG AAGTCCAATC CCTTAGCAAA CTCATCAGCC 250

AACAT 255

(2) INFORMATION FOR SEQ ID :777:

(i) SEQUENCE CHARACTERISTICS:
(A) LENGTH: 239 base pairs
(B) TYPE: nucleic acid
(C) STRANDEDNESS: double
(D) TOPOLOGY: linear

(xi) SEQUENCE DESCRIPTION: SEQ ID :777:

AGATTCTGCA GTGTAATATG ACCAAATCAA ATCCGTTGTA TGATCGAATA 50

AGGCCGTCAT AAAGTAAGTT GAAAATTTCG TTCTTGGCTG AATCGTGTTA 100

AACCGTTGTA CACAACGTAG TAAAATTCAT AACCCATCTG CATACCAATC 150

GTCAGCCGTG ATGCATTTAC TCACTTTACG TCGATGAATG TCAAACATCG 200

CACCTTTTCA ATGTTTACTA AAAACATAGC ACGCAATAT 239

(2) INFORMATION FOR SEQ ID :778:

(i) SEQUENCE CHARACTERISTICS:
(A) LENGTH: 252 base pairs
(B) TYPE: nucleic acid
(C) STRANDEDNESS: double
(D) TOPOLOGY: linear

(xi) SEQUENCE DESCRIPTION: SEQ ID :778:

ACTGAACAAC GTAAGGTCTT GACTGACACT TACAAGTTGG ATGAATCTGA 50

AATAAAGTTG GTATTGTTTG AAGCAGCCCA ACAATTTCGA ACATGCTACA 100

AGACCGTCGT TTGACTGACA AGGCCTTGAA GTACATGACT GACAACAACG 150

TCGATCTTCG CCTAGGCGCC ATGGTCACTG GTGTTGACGA AAATGGCGTG 200

ATTTTTAAGG ATGACTCAAC TTCGCCAACT CGGTTCCTCA TTAGGACAAC 250

TA 252

(2) INFORMATION FOR SEQ ID :779:

(i) SEQUENCE CHARACTERISTICS:
(A) LENGTH: 265 base pairs
(B) TYPE: nucleic acid
(C) STRANDEDNESS: double
(D) TOPOLOGY: linear

(xi) SEQUENCE DESCRIPTION: SEQ ID :779:

TCGGATTTAT ACGGCTGTTA GTGCTATCGG CGTCTGGTAT GTGTTATATA 50

TCGCCTATCG TTTTTTGGCC AATGGGCGTT GGCTACGCGG GGCAATGATT 100

GCCATCGTTT TTGTTATCTT GACTTATTTC GTCATTTTAA ATATTATTTA 150

ACTATTTTAC AAATAAAGTG GTCAAATGGG ATATTTTGCC AAAAATAGAG 200

AAAGTGTTAG GCGGCCCACA GCTGGAGGAA GAGAAAGCGG CTGTGCATGA 250

AGTTATTGCA CCCGC 265

(2) INFORMATION FOR SEQ ID :780:

(i) SEQUENCE CHARACTERISTICS:
(A) LENGTH: 249 base pairs
(B) TYPE: nucleic acid
(C) STRANDEDNESS: double
(D) TOPOLOGY: linear

(xi) SEQUENCE DESCRIPTION: SEQ ID :780:

CTCAGCTGGA CCAAATTAGT TGAATTACTC ACCATCAATA AACTATAAAT 50

GGCGCCAATG ACCAACCCGC CAACGATTCC CGTTGTCAAA GAACCTAATA 100

ACAAGTAACC AACCGCAGCC CCAACAAGCC CAACTAGGTT TAAAAAAACC 150

AAGCAAAAGG CCAACTGTCC GTCGTTTATT GGATTGTATC TGCTCATATA 200

ACATTAAAAC TTAACCGTTG GCACTTCTTT CTCAGACCCG CCGATACTG 249

(2) INFORMATION FOR SEQ ID :781:

(i) SEQUENCE CHARACTERISTICS:
(A) LENGTH: 188 base pairs
(B) TYPE: nucleic acid
(C) STRANDEDNESS: double
(D) TOPOLOGY: linear

(xi) SEQUENCE DESCRIPTION: SEQ ID :781:

GAGAGAACCC ACCATAGTAC TGTCTCCTGC AGACAAGACC AACGGCAAGG 50

CCGCCTGGGG TAAGGTCGGC GCGCACCTGG CGAGTATGGT GCGGAGGCCC 100

TGGAGAGGAT GTTCCTGTCC TTCCCCACCA CCANNNACTA CTTCCCGCAC 150

TTCGACCTGA GCCACGGCTC TGCCCAGGTT AAGGGCCA 188

(2) INFORMATION FOR SEQ ID :782:

(i) SEQUENCE CHARACTERISTICS:

(A) LENGTH: 182 base pairs

(B) TYPE: nucleic acid

(C) STRANDEDNESS: double

(D) TOPOLOGY: linear

(xi) SEQUENCE DESCRIPTION: SEQ ID :782:

AATATTTATT TCATTTGTTT ACTACCACTT CATTTTATTT GTTTGCTGCT 50

GCCGTTTTAT TTATTTTTAC TGAAAGTGAG AGGGAACTTT TGTGGCCTCC 100

AACCTTTTTC TGTAGGCCGC CTTAAGCTTT CTAAATTTGG AACATCTAAC 150

AAGCTGAAGN GGAAAAGGGG GTTTCGCAAA AT 182

(2) INFORMATION FOR SEQ ID :783:

(i) SEQUENCE CHARACTERISTICS:

(A) LENGTH: 295 base pairs

(B) TYPE: nucleic acid

(C) STRANDEDNESS: double
(D) TOPOLOGY: linear

(xi) SEQUENCE DESCRIPTION: SEQ ID :783:

CACAGCCAGA GCTTCGTCCT CAGTGACCAC AGGGCTGAGC CAGGCTCAAC 50

CGGCTTCTGG CTCTTGTTTT GCAGAAGAAG AACTAGAAGC AAGGNGCTTT 100

CCTCCGGGTC CCAGAGCTGT TAGTGATGGA GCCAGGCCTG GGATCCAGCT 150

TTCCTGAGTT CCTGACCCCT GCTATTTTAT TAGTCAGCTC TCAGCACTTA 200

CCAGAGGAAC AGGCAGCCTT TTGGCTACTG CCTTCAGAGA AAGAGAGATG 250

AGAGAATTCA AATGCGTGTT GGTGTTCCTG TTAGTACAAG CAGCA 295

(2) INFORMATION FOR SEQ ID :784:

(i) SEQUENCE CHARACTERISTICS:
(A) LENGTH: 295 base pairs
(B) TYPE: nucleic acid
(C) STRANDEDNESS: double
(D) TOPOLOGY: linear

(xi) SEQUENCE DESCRIPTION: SEQ ID :784:

CAATAGCGAC TTCTGGTGTC AATTCGATAA TCGGCCAATG GATACTTCAC 50

GTGTGTTTCA TCGTCTGTAA TAACGGCGAA TGGTGTCACT TCAGAACCAG 100

TACCTGATGT TGTTGGAATG GCAACCATTT GTGTCAAACG CACTGGTAGA 150

ACTTCACGAT ACGCTTACGG ATGTCCATGA ACTTTTGTTC TATTTCCATG 200

AACAATTCCT TGATACCTTC TTCGTCTGAC AAAATACCTT CGTGACGTGT 250

TGAGTATTCG TACAAGAAGC GACCAATCTT ACCGGCATCA AGTGC 295

(2) INFORMATION FOR SEQ ID :785:

(i) SEQUENCE CHARACTERISTICS:
(A) LENGTH: 303 base pairs
(B) TYPE: nucleic acid
(C) STRANDEDNESS: double
(D) TOPOLOGY: linear

(xi) SEQUENCE DESCRIPTION: SEQ ID :785:

GTGTAAAGCG AGTAGCCCCC AAATCAGTCA TCTTGGCCTG AAGATGTAAA 50

CAAGGAGGTT TGCTTCCTGC AAGATGAGTA CTTCACTGAG TTTGGTTTTC 100

ATTTTTCCTA CACCGAAGCC ACCTCTGTCC ATCCAATGTT CATGTCTGTC 150

CGAGGCTGCG CAGGAGGTTA CGTCAAGATG TGGCCAATAA AGTCCAAAAA 200

GCGCTTTGAA TACTGTTCTG GGTTCACGGT GGAGATCTCC GCGCAGCCAT 250

GTTTAACAGT TTTTGCAGCA TGGGCAGCTT TCTTTTTTGC ATCATAATGA 300

GTA 303

(2) INFORMATION FOR SEQ ID :786:

(i) SEQUENCE CHARACTERISTICS:
(A) LENGTH: 255 base pairs
(B) TYPE: nucleic acid
(C) STRANDEDNESS: double
(D) TOPOLOGY: linear

(xi) SEQUENCE DESCRIPTION: SEQ ID :786:

TATGGCGCGG ATCCTAACGG CTGGAATGAA GATATGCCAA CGTACAGCCT 50

CTATGACCCA GCCATGGGAT CAGGCTCATT GCTTTTGACG ACTGCTTCAT 100

ACATGAAGAA TGATGGTGTT CGTGGGGCCA TTAAGTACTA AGCCAAGAAG 150

TTATCACGAC AACCTATAAC TTGGGCCGAA TTAACTTGAT GATGCACGGG 200

GTGGAGTATA ACGATATCAA TATCCATAAT GCAGATACCT TGAGTTCAGA 250

CTGGC 255

(2) INFORMATION FOR SEQ ID :787:

(i) SEQUENCE CHARACTERISTICS:
(A) LENGTH: 206 base pairs
(B) TYPE: nucleic acid
(C) STRANDEDNESS: double
(D) TOPOLOGY: linear

(xi) SEQUENCE DESCRIPTION: SEQ ID :787:

CATTGAAAAG TGCCTGAAAA ATGGTAAATT CTTAAATGTG TGTGAGATTG 50

TCAGAATCAA CAAAACTAGG TTGGTTAAAC ATATCTCTGG TACATCAAGG 100

GGCATGATAC AAACCAGTCT AAAGACTGTT TATAAAGGAG AGAGCTGGCG 150

ACTTATTTTT ATTTTTTTTT TTTTGGACAG ACTCCCTTTG TCCCCAGGCC 200

GGAGTG 206

(2) INFORMATION FOR SEQ ID :788:

(i) SEQUENCE CHARACTERISTICS:

(A) LENGTH: 260 base pairs

(B) TYPE: nucleic acid

(C) STRANDEDNESS: double

(D) TOPOLOGY: linear

(xi) SEQUENCE DESCRIPTION: SEQ ID :788:

ACAAATAGCG ACCTCCTGGA GAAAAATCGA AAACGGCCAA GTGGATACCT 50

CACGTGGTTC ACGTCTGAAC AACGGCGAAT GTGAGCTACC TCAGAACCAG 100

TACCCGAAGT TGCTGGAAGG CAACCACATT TGGTTCAAAC GAGGCGTGGA 150

AGAACCTCAC GACACGCTTA CGGAGTCCAT GAACGCTTGT TGCAATCCAG 200

GAACAATTCC GCGACACCCT TCGTCTGACA AAAAACCTTC GTGACGCGTC 250

GAGTATCCGC 260

(2) INFORMATION FOR SEQ ID :789:

(i) SEQUENCE CHARACTERISTICS:
(A) LENGTH: 208 base pairs
(B) TYPE: nucleic acid
(C) STRANDEDNESS: double
(D) TOPOLOGY: linear

(xi) SEQUENCE DESCRIPTION: SEQ ID :789:

AACCCACCAT GGTGCTGTCT CCTGCCGACA AGACCAACGT CAAGGCCGCC 50

TGGGGTAAGG TCGGCCTGCG CACTATGCGT GAGTATGATG CAGAGGCCCT 100

GGAGAGGATG TTCCTGTCCT TCCCCACCAC CAAGCCCTAC TTCCCGCACT 150

TCGACCTGAG CCACGGCTCT GCCGAGGTTA GGGGCCACGA CAAGAAGGTG 200

ATCGACGC 208

(2) INFORMATION FOR SEQ ID :790:

(i) SEQUENCE CHARACTERISTICS:
(A) LENGTH: 289 base pairs
(B) TYPE: nucleic acid
(C) STRANDEDNESS: double
(D) TOPOLOGY: linear

(xi) SEQUENCE DESCRIPTION: SEQ ID :790:

AAAAGAAGTA CGGTAAGGCA AATCCTGAAA TTAAGAGTTA CTATGTGCCT 50

GAAAATACAT TGTTCTACTC ATTTAGTGTG ATGGCAATGG GCGCCGGCGC 100

ACTCTTGCTA TCGACCATCG TCGCGCTTTG GATGAACCGT CGTAAGTCAC 150

AATTAATGTA GACACAACGT CGATTCTCGT GACTTATGGG GATCGCAACT 200

TTCGCGCCAT TCTCGATCAA CACAGCCGGT TGGTCAGTGA CTGAATTAGG 250

TCGTTATCCA TGGGTTGTTT ATGGCTTGAT GCTAATAGC 289

(2) INFORMATION FOR SEQ ID :791:

(i) SEQUENCE CHARACTERISTICS:

(A) LENGTH: 232 base pairs

(B) TYPE: nucleic acid

(C) STRANDEDNESS: double

(D) TOPOLOGY: linear

(xi) SEQUENCE DESCRIPTION: SEQ ID :791:

TTTCCTGTGG AAGTAGTTAA ATTGATAAAT TCCAAAAATT TGGCAATTAA 50

TTGCTCATTA TTAATGGTAG AGTTTCCATT AATGAAGTTG GAAAATACGG 100

TGAATCTTTC AATGAACTCA GTTAGACATT CGGAATGAGC TTCATACATT 150

GTCTTATTAT CTTTCTTCAA TTTATCCTCC AAATCTGGGA ATTCTGATAA 200

CCAAATTGAA GCACCTTATC TATGATCCGG GC 232

(2) INFORMATION FOR SEQ ID :792:

(i) SEQUENCE CHARACTERISTICS:

(A) LENGTH: 232 base pairs
(B) TYPE: nucleic acid
(C) STRANDEDNESS: double
(D) TOPOLOGY: linear

(xi) SEQUENCE DESCRIPTION: SEQ ID :792:

TTTCAACAAA CAGAAACCAA TTTCCAACCA GAGGGCGATT TCTCCTTGTC 50

TGGTAATATC GAACAAACTA TTTTTAAGAA CTTGATTTCT GGCAACATTA 100

AGAGCGCTGT GAAAAATTCT CTAGAGAATG ACTTACTAAC GGAGGCCATG 150

GCGATCGCAT TAGATTCAAA TAACGAAAGA TCAAAGGAAA GTGTCAAGAA 200

TGCCTATTTC GCGAAGTATG GATCTAAATA AC 232

(2) INFORMATION FOR SEQ ID :793:

(i) SEQUENCE CHARACTERISTICS:
(A) LENGTH: 292 base pairs
(B) TYPE: nucleic acid
(C) STRANDEDNESS: double
(D) TOPOLOGY: linear

(xi) SEQUENCE DESCRIPTION: SEQ ID :793:

GGGAACATAT CTTCACGGTA GTGANCCCAG TGACCAGATG TCTTATACAA 50

GTTCAAGTTA GACAAAACGG GTGTATACAC GTGTTGGTAA CCATTGGCCA 100

ATTCTTTGTC GGTGATGTAA CGTTCAACTT GACGGCGAAG CGATTGCCCC 150

ATTTGGTAAC CAAACTGGCA AACCTGAACC CACTTCTTGA CTCGTAAAGA 200

ACAAGTCCAT GTCACGACCA ATCGTCCGGT GGTCACGTTC TTTAGCTTCT 250

TCACGACGTG CAATTCTGCT TCAACGTCGG CTTGCTTCCA TT 292

(2) INFORMATION FOR SEQ ID :794:

(i) SEQUENCE CHARACTERISTICS:

(A) LENGTH: 288 base pairs

(B) TYPE: nucleic acid

(C) STRANDEDNESS: double

(D) TOPOLOGY: linear

(xi) SEQUENCE DESCRIPTION: SEQ ID :794:

CAGCAGGCAC AACACCATCG GTTGTCGCTG AAAGTATTTT TAAGTCTTTG 50

GCCAAGAAGT CAGTTTCAGC TAAGTTAGAC GGCACTTATG CGGGTATGCA 100

TGATGTCATT CCTGCATCCG ATGATTTTTA ATTAATTACA ACAACTGATG 150

ATGAAGCGTC GAATTCGTCG CGTCATTCTG CCTCACACTC ACAGGCACAA 200

GCCTTAAAGC ATTTGCCAAA GTTTGCTAAC ATGCACTTTG GCGCGGCAAC 250

CATTTATTGA AAATGGTTTC TACTATGATA CAAATAAC 288

(2) INFORMATION FOR SEQ ID :795:

(i) SEQUENCE CHARACTERISTICS:

(A) LENGTH: 258 base pairs
(B) TYPE: nucleic acid
(C) STRANDEDNESS: double
(D) TOPOLOGY: linear

(xi) SEQUENCE DESCRIPTION: SEQ ID :795:

GTGGCTATGT ATAAGCAGAT ATTATCTAGT AAATGTAAAG TGAACCCAAT 50

CGATAGACCC AAAAAGTGTT TTAACTGGAA AACACCTTAT GAGGTTTATT 100

TTGATGAAGT GTTGCAGTTG GTTTGATAAT TCAAGGCATT AAGGCAATAT 150

CTCAATCACC GCATTTTCCA CAACCGGCCA CAAAACAGCT CGTCTATTTC 200

GAGGTCAGTT ACGGCGTCCA GTCTTTGAGC AGATGATTTG CCCCGAGTGA 250

TCGTCGGC 258

(2) INFORMATION FOR SEQ ID :796:

(i) SEQUENCE CHARACTERISTICS:
(A) LENGTH: 270 base pairs
(B) TYPE: nucleic acid
(C) STRANDEDNESS: double
(D) TOPOLOGY: linear

(xi) SEQUENCE DESCRIPTION: SEQ ID :796:

TGCTTCAAAG CCTGGCTTAA CCCGCTTGCC CACCAACTTC AATTATACCT 50

GCCCCTTTGC CTAACATCTC ACCGACTCGG GCAACCATCG TATCAACACC 100

CGCGACTCGA TCCCCATCGT AAAATGATTC CGAACTAACA TTTAACACAC 150

AATAGATAAT ACCATCAGCA TAATGCAAGG TTTTACCATC CACGACCCAG 200

AGGCGATCAA TCGCGACTAA CCGTCGTTGT AATTCAGGAC TAGCGGTACG 250

CTCATGTAAC GCGACAATAT 270

(2) INFORMATION FOR SEQ ID :797:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :797:

AAATATCGCT GCGTGCAGCT GAGTTACTAG CTGTTGAAAC TTATCGTTAC 50

TTGTGTAGGT TGTCTGAAAC GGTCGCAGTC ACCGGTTAGC TTGATAAGTC 100

AGGTTTTCAG CAACTGTCAT CAAGCCATCA CGACCTATAT AAGCCACTGC 150

AATCGCACTT TACATCATTA AGACCCTTAC TAGAAGTAAC TGGCCATCCT 200

GCCACCGCTT 210

(2) INFORMATION FOR SEQ ID :798:

(i) SEQUENCE CHARACTERISTICS:

(A) LENGTH: 218 base pairs
(B) TYPE: nucleic acid
(C) STRANDEDNESS: double
(D) TOPOLOGY: linear

(xi) SEQUENCE DESCRIPTION: SEQ ID :798:

ATCATAAGCG CTGGAACTTG GGGCATTGTA CAGGGACAAC TTCGGCCTTT 50

GTTAGCCCCA AGAGTCTACA CTCTGCCAAT GGTGCGCTCC ATAGGAAAAA 100

CCACGGTCCA AGGCAAAAAC TACGAACCTC AGATAACCGC AAAGAGGATA 150

TCAACCAGAG GACGGAAATG TAAGCCTATT TTAGCCCAAA TAACGAGACA 200

AGTAGTTGAG CTAAATGC 218

(2) INFORMATION FOR SEQ ID :799:

(i) SEQUENCE CHARACTERISTICS:
(A) LENGTH: 176 base pairs
(B) TYPE: nucleic acid
(C) STRANDEDNESS: double
(D) TOPOLOGY: linear

(xi) SEQUENCE DESCRIPTION: SEQ ID :799:

CCTATCAGAA TCCAATGAGA ACTACAATGG TGTGCCCAGA CGTAGAGCTG 50

AGAATAGCAT TACCAAATGG AACAACGTGC TACAGTCAGG ATTAAAAAGA 100

ACAATACTAC AAACCAAGTA TATCAGGCTA TTATAGCAAA GGCTGCCATG 150

AACAATACAA CACTGAATTA CTCGGC 176

(2) INFORMATION FOR SEQ ID :800:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :800:

GCAATTGCTT AATTTGTTCT TCCTTCTTAG CATTTTGCTG AGAAGCCAAA 50

CGTTGCGCCA ATTCAGAAGA TTCACGCCAG AAGTCGTAGT TACCAACGAA 100

TGGTGTGATC TTACCAAAGT CAACATCCAA GATGTTGGTT GACACGGCGT 150

TTAGGAAGTG ACGGTCGTGG GATACCACAA TCACTAGGTT CGGGAAATCA 200

GCCAAGAAAT CTTCTAACCA GTTAATTGTC TGCACATCCA GACCGTTGGT 250

TCGA 254

(2) INFORMATION FOR SEQ ID :801:

(i) SEQUENCE CHARACTERISTICS:
(A) LENGTH: 203 base pairs
(B) TYPE: nucleic acid
(C) STRANDEDNESS: double
(D) TOPOLOGY: linear

(xi) SEQUENCE DESCRIPTION: SEQ ID :801:

ATCCAGGCCC ATACCCATCA ACCCTGAAAC AACGATTGAA TCACCAGCCA 50

TTTAACCACA CATTGCAACA AACTCACATT GGGTTCCGAG CGGCAGTAAT 100

AACACAGTCG ATCAAACGCA AAATAGATGG GCAATAAGGT CGATACAAAT 150

AGGCAACCTT ACAATTACCA CGATCTGTAG CTATCGTATA ACCAATGAAA 200

ATG 203

(2) INFORMATION FOR SEQ ID :802:

(i) SEQUENCE CHARACTERISTICS:
(A) LENGTH: 193 base pairs
(B) TYPE: nucleic acid
(C) STRANDEDNESS: double
(D) TOPOLOGY: linear

(xi) SEQUENCE DESCRIPTION: SEQ ID :802:

GACACAACTG TGTTCACTAG CAACCTCAAA CAGACACCAT GGTGCACCTG 50

ACTCCTGAGG AGAAGTCTGC CGTTACTGCC CTGTGGGCTA GGCGAACGTG 100

GATGAAGTCG ATGGCGAGGC CCTGGGCAGG CTGCTGGCGA TCTACCCTTG 150

GACCCAGAGG TTCTTTGAGT CCTTTGAGGA TCTGTCAACT TCC 193

(2) INFORMATION FOR SEQ ID :803:

(i) SEQUENCE CHARACTERISTICS:
(A) LENGTH: 290 base pairs
(B) TYPE: nucleic acid
(C) STRANDEDNESS: double
(D) TOPOLOGY: linear

(xi) SEQUENCE DESCRIPTION: SEQ ID :803:

AACAAAAGTT GAAAATTTTA AATGGTCTTT TTATGGTTTG CGTGACCTAA 50

AAAGACAAAG TTTATCCTTG CCTTACTGTA TAGAAATGCG TCGTATCCAC 100

AATAGCGTAC AGATTTTTCC GCATTAATCC GTGTTTATAT TAACAGATTC 150

GTTAAGTATC GTTTAAAAAG GGAGAGAGGG GATACCCTCT CTCTAGATAA 200

ATGGGTCATC ATTTAATCCC AAGAATGATG TCGATAAGTA CTTTTCTAAA 250

CGATAACAAG AACCCCAGTA GGTAGTATAG CAGTCTTTAA 290

(2) INFORMATION FOR SEQ ID :804:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :804:

AACTTGATGG TCGAGGCCAT CTCCTGGGCC GCCTGACGAT ACCGTGGCTA 50

AACAGGTACT GCTGGGCCGG AAGGTGGTGG TCGTACGCTG TGAAGGCATC 100

AACATTTCCG GCAATTTCTA CAGAAACAAG TTGAAGTACC TGACTTTCCT 150

CCGCAAGCGA ATGAACACCA ACCCTTCCCG AGGCCCCTAC CACTTCCGGG 200

CCCCCAGCCG CATATTCCGG CGGACCGTGC GAGGTATGCT GTCCCACAAA 250

ACAGGG 256

(2) INFORMATION FOR SEQ ID :805:

(i) SEQUENCE CHARACTERISTICS:
(A) LENGTH: 286 base pairs
(B) TYPE: nucleic acid
(C) STRANDEDNESS: double
(D) TOPOLOGY: linear

(xi) SEQUENCE DESCRIPTION: SEQ ID :805:

AGCAACCTCA AACAGACACC ATGGCGCACC TGACTCCTGA GGAGAAGTCT 50

GCCGTTACTG CCCCTGTGGG ACCAGACGAA CGCGGATGAA GTCGGCGGCG 100

AGGCCCTGGG CAGGCTGCTG ACCGATTTAC CCTTGGACCC AGAGATTCTT 150

TGAGTCCTTT GAGAATCTGT CCACTTCTGA TGCTGTTATG GGCAACCCTA 200

AGACGAAGGC TCATGGCAAG AAAGTGTTCG GTGCCTTTAG TGATGACCTG 250

GCTCACCTGG ACGACCTCAA GGGCACCTTT GCACAC 286

(2) INFORMATION FOR SEQ ID :806:

(i) SEQUENCE CHARACTERISTICS:
(A) LENGTH: 291 base pairs
(B) TYPE: nucleic acid
(C) STRANDEDNESS: double
(D) TOPOLOGY: linear

(xi) SEQUENCE DESCRIPTION: SEQ ID :806:

TTCTTCATCA GATTTTACAT CTGCCTGATT AGAATCTTCT ACACTGGCCT 50

CAGAAGATGA TTGTTCAAAA CTTTTTCTAA GTTGCTGTAA AAAAACTTCC 100

ACGGACAAAG TAAAATGCAG TTCTTTATCG TTTAGCCAGT GTACAACAAA 150

AGGTCCAATC TTCTCTTCAT TTTAATTCAG ACTCAGAGAT GTAATAGATG 200

GAAGAAGTGA AATGTCTGTG GCTGGGTTGA TGCTGGCTGC AATATGAAAG 250

TGGCAAAGTG CATTGCTATG CAGTGGAGTG TTACTGTGGA C 291

(2) INFORMATION FOR SEQ ID :807:

(i) SEQUENCE CHARACTERISTICS:
(A) LENGTH: 276 base pairs
(B) TYPE: nucleic acid
(C) STRANDEDNESS: double
(D) TOPOLOGY: linear

(xi) SEQUENCE DESCRIPTION: SEQ ID :807:

AGTTTAATCA GCTGCAATGA AAACAAACGT CTTTTATTAG GCAGAATCCA 50

GATGCTCAAG GCCCTTCATA ATATCCCTTA ATTTAGTAGT CGAACTTAGG 100

GAACAAAGGA ACCTTTAACA GAAATAGAAC AACAAGAAAG CGAACTTAGC 150

GATACTCGCG GGCCAGGGCA TTAGCCACAC CAACCACCAC TTTACGATAG 200

GCAACCTGCA CTGGAGGGGC GACTTCTCCG CCAAAGCGAC GGGCCAGCAC 250

ACAGACCAGC ACGTCGCCCA GGAGCC 276

(2) INFORMATION FOR SEQ ID :808:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :808:

GGCCTGCCAA CAGCATAGGG AAGAATCTCA TTGTTATACA TATTAAGAAT 50

CGATTCTAAA TAGACCTTTT TACCAGTCGC TGAAACTCTT AAATTAAGTA 100

ACATCGCTAG CTAATTTTCG TAATGGCCGA TTAATCTTGA AACGGCGGCG 150

AAGCTTGTTC TTCACACGTT TCCCTTCAGG TCCTTTGATG AATCATACTG 200

ACGTGATCGC TTATCGTA 218

(2) INFORMATION FOR SEQ ID :809:

(i) SEQUENCE CHARACTERISTICS:
(A) LENGTH: 276 base pairs
(B) TYPE: nucleic acid
(C) STRANDEDNESS: double
(D) TOPOLOGY: linear

(xi) SEQUENCE DESCRIPTION: SEQ ID :809:

AGCAACCCAG CGCTCAATGG TGCTAGTGAA CACAGTTGTG TCAGAAACAA 50

CCTCAAACAG ACACCATGGT GCACCTGACT CCTGAGGAGA AGTCTGCCGT 100

TACTGCCCTG TGGGCAAAGG CGAACGCGGA TGAAGTCGGC GGCGAGGCCC 150

TGGGCAGGCT GCTGGCGATC TACCCTCGGA CCCAGAGGTT CTTTGAGTCC 200

TTTGGGGATC TGTCCATTAC TGATGCCGTA TGGGCAACCC TAAGGCGAAG 250

ACTAATGGCA AGCAAGTGCT AGACGC 276

(2) INFORMATION FOR SEQ ID :810:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :810:

GACTCCACCT GAACGGGCCC CTTCTGAACC GCCTCTGTGG GAGCAGGCCC 50

CATCTGAGGA GGGTCCATCT GAGCAGATCC CTTCTCAACA GGTTCCTTCT 100

GAGGAGGCTT ACTGCTTTTC TTACTCGATT TATTTTTCAG AGTTTTCTTC 150

TTCGTACTTT TTTTAACGCA AGTATTTTGC TTTTTATTCT CCTCCATTT 199

(2) INFORMATION FOR SEQ ID :811:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :811:

AAACAGCACA ACCAATGACA GGATGCAAGA ACTGATAGGG CGCCGCAAAA 50

AAAAAGCGGG CCAAAGATGA AGTAAATAAA CGTAAGGGAG GTACCGAATC 100

TGGGGGGCTT GGCGTAGAGT AAGGGAGAGG AGCTTCAACT ATCTCAATAA 150

GGGCAAGAAA ACACAAACAA CTCCTTCATT CGTCCAAAAT GCTTCTGAAT 200

TGCACCACAA TTGACACACA CACACCTCAA AGCAGAATCC GCACTGACCT 250

AAGT 254

(2) INFORMATION FOR SEQ ID :812:

(i) SEQUENCE CHARACTERISTICS:
(A) LENGTH: 284 base pairs
(B) TYPE: nucleic acid
(C) STRANDEDNESS: double
(D) TOPOLOGY: linear

(xi) SEQUENCE DESCRIPTION: SEQ ID :812:

TTTTTTTTTT AGCAATGAAA ATAAAAGTTT TTTATCAGGC AGACTCCAGA 50

CGCTCAAGGC CTTTCACAAC ACCCCCCAAT TTAGCAATAG ACTTCAGGGA 100

CCAAAGGACC CTTTAACAGA AATAGAACAA CAAGAAAACA AACTCAAAGA 150

CACTCGAGGG CCAGGGCCTC ACCACACCAA CCCCCACTTC CAAACAGGCA 200

ACCTGCACCG GAGGGGAGAC TTTTTCGCCA AAGCGACGGG CCAGCACACA 250

GACCAACACA TCGCCCAGGA ACCTGAAGTT TTCA 284

(2) INFORMATION FOR SEQ ID :813:

(i) SEQUENCE CHARACTERISTICS:
(A) LENGTH: 279 base pairs
(B) TYPE: nucleic acid
(C) STRANDEDNESS: double
(D) TOPOLOGY: linear

(xi) SEQUENCE DESCRIPTION: SEQ ID :813:

ATACAACAGC AGAAACAGGT ATCCACGCAC AGTCCAGCAA CATTTCTTCT 50

GGTACAACGC AGCTTCAAAG TTCACATCAA ACGTTACTTT GTCATACCAA 100

GGCAAGGATG CTAACTCGAA GTCAATCACG CGAGTGATGT CATTCACAAC 150

CGGTCACGGT GCCGATGTGA CAATCAAGAC TGAAGGCGAT GACGAAGAAG 200

CAGCACTGCC CGCCGATAAC CGCAGCGATG CAAAAAAAAG AGCTAGACGA 250

CTGACGAAAC CTGAGAACCC CGAAGGACT 279

(2) INFORMATION FOR SEQ ID :814:

(i) SEQUENCE CHARACTERISTICS:
(A) LENGTH: 208 base pairs
(B) TYPE: nucleic acid
(C) STRANDEDNESS: double
(D) TOPOLOGY: linear

(xi) SEQUENCE DESCRIPTION: SEQ ID :814:

AACGTGCTGG TCTGTGTGCT GGCCCATCAC TTTGGCAAAG AATTCACCCC 50

ACCAGTGCAG GCTGCCTATC AGAAAGTGGC GGCTGGCGTG GCAACAGTGA 100

CTGAGAAGAC CAAAGAGCAA GTGACAAACG CCGAAGGAGC AGTGGCGACG 150

GGCGTGACAG CAGTAGCCCA GAAGACAGCG GAGGGAGCAG GGGGCATCGC 200

AGCAGCCA 208

(2) INFORMATION FOR SEQ ID :815:

(i) SEQUENCE CHARACTERISTICS:
(A) LENGTH: 192 base pairs
(B) TYPE: nucleic acid
(C) STRANDEDNESS: double
(D) TOPOLOGY: linear

(xi) SEQUENCE DESCRIPTION: SEQ ID :815:

AAACCACGCT CAGTCAGGTT CCGGCAGCAG CTGCAGCATC TCATCTTCTG 50

CGACTCTCGG TGCCCTCTCC TTCCGATTTC CGGAAACATG GCCTCCGATG 100

TGGCTGTCTC TGACGACGTC ATCAAGGTGT TCAACGACAT GAAGGCGCAC 150

AAGTCTTCAA CGCCAGAGGA GGTGAAGAGG CGCAAGCAGA TA 192

(2) INFORMATION FOR SEQ ID :816:

(i) SEQUENCE CHARACTERISTICS:
(A) LENGTH: 264 base pairs
(B) TYPE: nucleic acid
(C) STRANDEDNESS: double
(D) TOPOLOGY: linear

(xi) SEQUENCE DESCRIPTION: SEQ ID :816:

GAGAGAACCC ACCATGGAGC TGTCTCCTGC CGACAAGACC AACGTCAAGG 50

CCGCCTGGGG TAAGGTAGGC GCGCACAATG GCGAGTATGG TCGCAGAGGC 100

CCTGGAAAGG ATGCTCCTGT CCTTCCCCAC CATGTGAGAC CTACTCCACA 150

CACTTCGACC TGAGCCACGC CTCTGCCAGG TTAAGGGCCA CGGCAAGAAG 200

GTGCCCGACG CGCTGACCAA CGCCGTGCGC ACGTGGACGA CATGCCCAAC 250

GCGCTGTCCC CCTG 264

(2) INFORMATION FOR SEQ ID :817:

(i) SEQUENCE CHARACTERISTICS:

(A) LENGTH: 202 base pairs

(B) TYPE: nucleic acid

(C) STRANDEDNESS: double

(D) TOPOLOGY: linear

(xi) SEQUENCE DESCRIPTION: SEQ ID :817:

ACCCTACACA CATCCGTTCA ACTGTCCCAT CACCCACTTA CACTCCAAAA 50

AAAACCACTC AACAAGCAAA CAACCCATCC TGTTTTCATA ACGTTATCGT 100

AACAAAAAAG CCGACCATAA TCAACTTGAA TTTACTTCAC CACAAGGCAA 150

AGCGATTATT TAGATGAGGC ACTCGCACTA CTGCTTGAAG TGCTACTAGA 200

TG 202

(2) INFORMATION FOR SEQ ID :818:

(i) SEQUENCE CHARACTERISTICS:

(A) LENGTH: 179 base pairs

(B) TYPE: nucleic acid
(C) STRANDEDNESS: double
(D) TOPOLOGY: linear

(xi) SEQUENCE DESCRIPTION: SEQ ID :818:

AGTAGAAAAT AAGTTCAAAA TTTTAGAAAT ACTGGCTTTA TACTCGCCCA 50

TGTATTTACA TTCACAGAGA TCTTTATTTA TTTACAAGCG CTTTGAGTTA 100

CTGTATAGTT TTTTTTAACA AAAAAGCGTG GGGGGCTCCC TTTAGCATTC 150

CCCATAGGAT AGGTAATGAA CTTTTTGTT 179

(2) INFORMATION FOR SEQ ID :819:

(i) SEQUENCE CHARACTERISTICS:
(A) LENGTH: 260 base pairs
(B) TYPE: nucleic acid
(C) STRANDEDNESS: double
(D) TOPOLOGY: linear

(xi) SEQUENCE DESCRIPTION: SEQ ID :819:

TGTGCTCTTA TGCTAGATTT ACGGACAGAT TTTCTTCTGC CCTGTAAACT 50

AATAGCATAG GAGCATTTTT TAATACGATT CGATACAAAA AAGAATTTAA 100

GCAATCTCTC GCCGAGACGC ACAATCAAGA CCATTCATAT ACTGATCTAT 150

CCGCTGAATA CGAACCTTCA ATCGACTCAA TCCGTAACTG GATCAAGTTG 200

TACGCGGGTC CACGAAGTGA CAGACGAAAA ATGAACGCAA GCTGATGTAA 250

ACGCATCACA 260

(2) INFORMATION FOR SEQ ID :820:

(i) SEQUENCE CHARACTERISTICS:
(A) LENGTH: 226 base pairs
(B) TYPE: nucleic acid
(C) STRANDEDNESS: double
(D) TOPOLOGY: linear

(xi) SEQUENCE DESCRIPTION: SEQ ID :820:

ACTCGGCACT TTCTTAGAAT AGACAATTTG CAAAATGTCC ATCCCAGGTT 50

CATCCTTGTA GCCTAATGTT TCCGTCACCG TTGCCACTAG TTAACGTGGG 100

TGAACTTTAT CTGGGAAACG GGTAACATCC GCGACCACAA CTAGACCATC 150

ATTTGACGTA ATGCCATCAG CACTCACCAA AACTTGGCCA ACCAACAGCC 200

TTTCCGTAAC TGAGTTTGAT TTCTCC 226

(2) INFORMATION FOR SEQ ID :821:

(i) SEQUENCE CHARACTERISTICS:
(A) LENGTH: 197 base pairs
(B) TYPE: nucleic acid
(C) STRANDEDNESS: double
(D) TOPOLOGY: linear

(xi) SEQUENCE DESCRIPTION: SEQ ID :821:

CACCTACATA TAGATGCACG AAGTACTTGC TCACTTTTTT TGGTTGTAAA 50

TTTTCAAAGC GTTGAAGGCA GCATTCTTAG CGGCACGGAT GTCCTTATCA 100

GTGAATGAAT CAGGCTTTTC CATGGCCAAC CAACCAACGA TTCGCATCGC 150

GTGATTTCGA ATCATGTCAA GCAAAGCACC GGCTGTATCA TAGTAAC 197

(2) INFORMATION FOR SEQ ID :822:

(i) SEQUENCE CHARACTERISTICS:
(A) LENGTH: 304 base pairs
(B) TYPE: nucleic acid
(C) STRANDEDNESS: double
(D) TOPOLOGY: linear

(xi) SEQUENCE DESCRIPTION: SEQ ID :822:

GGACTGGGTG CAGTGGCTCA CACCTCACGT TACATTTTAT AGCTAGCAGG 50

GTAATGGGGA GTTATGGCAC TCAGGTCACA TTCTAGGGAA TGTTTATCGG 100

GCAATCTCAA TGGCACCGCA AGCTAAATGA CTTCCAGTGT TTCTCATCTT 150

TGCGCTTTCT TCATCGCAAC CTTTGCCCAA GACATCTGTT TTTTCTGGAT 200

CACCCTTGCA CAGCCACTGA GGAATGATCT TCTGAAAGTG AATCTTCAGT 250

AGACACATCG CCACACCATC TANGTCAGCC ATCACATGCC CAGGTCTNGA 300

CATG 304

(2) INFORMATION FOR SEQ ID :823:

(i) SEQUENCE CHARACTERISTICS:
(A) LENGTH: 197 base pairs
(B) TYPE: nucleic acid
(C) STRANDEDNESS: double
(D) TOPOLOGY: linear

(xi) SEQUENCE DESCRIPTION: SEQ ID :823:

GGCCCAGCAG ANGGAGGAAC ACTACCGAGG CTCCAGCTTA ACGGTATTTG 50

GAGGTCAGCA CGGTGCTCAC AGAAGCCAGG AACTTGCCCA GGGAGGCGTG 100

CACCAANGGG GCGAACTCNC GGGGAGGCGG GCGACCAGGG TCACCAGCAG 150

GCAGTGCTTA GGAGCTGGGA GCCGACCGAG CCCACCGAAC TCGCGCG 197

(2) INFORMATION FOR SEQ ID :824:

(i) SEQUENCE CHARACTERISTICS:
(A) LENGTH: 281 base pairs
(B) TYPE: nucleic acid
(C) STRANDEDNESS: double
(D) TOPOLOGY: linear

(xi) SEQUENCE DESCRIPTION: SEQ ID :824:

AAAAACAATG TCATTTTGTA CAGACAAAAT TTAGCAGACT CAAGCTTCCA 50

CAGTTAGTGC GAGAGCCTGT GGATGGCATA GTTACATAAG AGTCTGTTGT 100

GGATTCAAAT GTCACCTCTT CCAGGCAGTC TTTCCTGACT TACCCTGGCA 150

GCTTTGTGTT CCCACATTAC TAGTAATGTT TTTTTCCTTA ATAGCAATGA 200

TTTCACTGTA ATAATGTATT TATATGCCTC TCTCCCCCAG ACAGCAAGCG 250

TTTTCCCACA GGTCTCGACA CACAGAAGAT A 281

(2) INFORMATION FOR SEQ ID :825:

(i) SEQUENCE CHARACTERISTICS:
(A) LENGTH: 312 base pairs
(B) TYPE: nucleic acid
(C) STRANDEDNESS: double
(D) TOPOLOGY: linear

(xi) SEQUENCE DESCRIPTION: SEQ ID :825:

TATTAAATTT ACTAGAATGT TACAGTTACA AATTTGGTAA TGTTCTTTCT 50

GAAAAACAGC CTAATTTTGC AGCCTGAAAC TTGACTGAAA ATCTCAATAC 100

TTTTATTCAT GATAGAAAAA TAATTCCTGG CTTCATCTCA CAATTAACTA 150

ATAATTATGT TAATATAAAA TATAACTGTG CCCTTTCTTT TCAGTGATGA 200

TCAAAGTGAT TCTCCCAGGC CAAAAAATCA AATAAGAAGT TATATTTTAA 250

AAAGACATAA CAAGCCATTC TACCCAGTGG GCATCTTCAG TGTACTCCCT 300

CTACTAATTG GC 312

(2) INFORMATION FOR SEQ ID :826:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :826:

ACACCAAACA CGGGGAGTGG GGAGTAGAGG CTCTGGAGGT CAGGATGGCA 50

GGGCAGGGAG GGGAAGGAAG GAGTTGTTGG TCTCACAGTG TGCCTGCCAA 100

TCCCAAAGCC CTAGAGACCC CTTCACTGCA GCACCTGCCC CCGGGTCTCA 150

GGCAGCTTCA GGGCCAGAGA GCTGCCAAGG GCAAGCAAA 189

(2) INFORMATION FOR SEQ ID :827:

(i) SEQUENCE CHARACTERISTICS:
(A) LENGTH: 232 base pairs
(B) TYPE: nucleic acid
(C) STRANDEDNESS: double
(D) TOPOLOGY: linear

(xi) SEQUENCE DESCRIPTION: SEQ ID :827:

CTCTTAAGGG CTTGAAAATT TCTGTGGGAG TAGGACAGAG TGTAGAACGT 50

ACAGTAAAAG GCAATAAAAC AGTGGGAATA ATTTTACCAG CTATGAGTAA 100

AGGAGTAATG GGTATTTACT GCCTGTCATT AATCCACACA TATATCCATT 150

TTAGAGATTA TTTTGTTGCC TGGAAATCTG TCTCATCACA GAGTGTTAAT 200

ATACACCGGC GGGATATTCA AGAGTGGCTA AG 232

(2) INFORMATION FOR SEQ ID :828:

(i) SEQUENCE CHARACTERISTICS:

(A) LENGTH: 282 base pairs

(B) TYPE: nucleic acid

(C) STRANDEDNESS: double

(D) TOPOLOGY: linear

(xi) SEQUENCE DESCRIPTION: SEQ ID :828:

GGACATTGGA ACACTATACT CTATTATTGC GGTCGTGCCT AGCAGTCTCG 50

CGCATGTCTC CTCAGCGCGC ACAGTCTCTC TCAGAGAGCT CTTCTCTCTT 100

TATATGCGCA GACCCGCAGA CTGGGAGACC CAGACCCAGG GCAAACTCTC 150

TTCTTAGGTA TATCGCACCC ATCATATCTC ACACATCGTG TATGTCGTAT 200

CATCAGACCC CCATAGAGCA TATATGCTAA ATTAATGCTC TCTTTCATCA 250

GTAATTACCC CATATCATAA AATGCGGGCG GG 282

(2) INFORMATION FOR SEQ ID :829:

(i) SEQUENCE CHARACTERISTICS:
(A) LENGTH: 230 base pairs
(B) TYPE: nucleic acid
(C) STRANDEDNESS: double
(D) TOPOLOGY: linear

(xi) SEQUENCE DESCRIPTION: SEQ ID :829:

CACTACATCC GCAAGTACAA CCGCTTCGAG AAGCGCCACA AGAACATGTC 50

TGTACACCTG TCCCCCTGCT TCAGGGACGT CCAGATCGGT GACATCGTCA 100

AGTGGGCGAG TGCCAACCTC TGAGCAAGAC AGTGCGCTTC AACGTGCTCA 150

AGGTCACCAA GGCTGCCGGC ACCAAGAAGC AGTTCCAGAA GTTCTGAGGC 200

TGGACATCGG CCCGCTCCCC ACAATGAAAT 230

(2) INFORMATION FOR SEQ ID :830:

(i) SEQUENCE CHARACTERISTICS:
(A) LENGTH: 226 base pairs
(B) TYPE: nucleic acid
(C) STRANDEDNESS: double
(D) TOPOLOGY: linear

(xi) SEQUENCE DESCRIPTION: SEQ ID :830:

TTCCGCAAAT AAAAGAATTC ACTAAGGTAC CAAAACAGAA AATATACAGA 50

GATCAATGAC TTTCATATAC ATTAACAAAC AAAAGTTCAG AGATAAAATG 100

GAAGAGAAAT GCTGTTTTTA ACAGTATAAT TAAGATAAAA TATGAAGGTA 150

TAAACTTAAC AAGAAATGTT GCAAAACCAT TATGTGAAAA TTACAACACT 200

CCTGAAGACG CAGACACACC TAACAA 226

(2) INFORMATION FOR SEQ ID :831:

(i) SEQUENCE CHARACTERISTICS:
(A) LENGTH: 247 base pairs
(B) TYPE: nucleic acid
(C) STRANDEDNESS: double
(D) TOPOLOGY: linear

(xi) SEQUENCE DESCRIPTION: SEQ ID :831:

AGAAGTCCTT CCAAGAGTCT TGGGTATGCA ACAGCCATGG AGGCTGTGAC 50

CTTTTTCCTT CTTTTCTACA GCCTGCAGTT CATTTAAGGA TCACCGGAGA 100

TGACTCGTGC TCTAGTTCTT AAAATCAAAC AAGGATCTGC CAAATCCAAG 150

ACCCTGAATT TGGCCCAAAT TTGTAGAAAC ATTGCTTTTT ACCACCCGGT 200

GCACCAAAAA TACCTCCCAT TTCAAGGCAA CAACCGCTTT AATTGCT 247

(2) INFORMATION FOR SEQ ID :832:

(i) SEQUENCE CHARACTERISTICS:
(A) LENGTH: 112 base pairs

(B) TYPE: nucleic acid
(C) STRANDEDNESS: double
(D) TOPOLOGY: linear

(xi) SEQUENCE DESCRIPTION: SEQ ID :832:

CACCAGGCAG GGGATCCCGG AGGGAAGCCC TCTGCCAGGG ACATGGTGAG 50

GGCGTGGCCA TCACCCACGA AGGGAGCATA AATAACACTG GCAGGTGGGT 100

GGGCAGCAGG AG 112

(2) INFORMATION FOR SEQ ID :833:

(i) SEQUENCE CHARACTERISTICS:
(A) LENGTH: 173 base pairs
(B) TYPE: nucleic acid
(C) STRANDEDNESS: double
(D) TOPOLOGY: linear

(xi) SEQUENCE DESCRIPTION: SEQ ID :833:

AGCCATTCCC ACTAGAGGCC AAACCGCCTG CCCACAGAGA TTGACAGCCA 50

ATGTTCATCT CATAACTCTC CTCCCAGCAG TGCACCAGTA AACTCAGATG 100

CCTGAGTGCT TGTGGCCACC ACACAACAGA TGCGGCCTTC CTCTTCACTG 150

GCCCCTCGGC TGCTGCTGGG TCC 173

(2) INFORMATION FOR SEQ ID :834:

(i) SEQUENCE CHARACTERISTICS:
(A) LENGTH: 288 base pairs
(B) TYPE: nucleic acid
(C) STRANDEDNESS: double
(D) TOPOLOGY: linear

(xi) SEQUENCE DESCRIPTION: SEQ ID :834:

TGGCCCATGG GAGCCCTTAT GAGCAAGTTT CCAGGTCCTT ATGACACAAT 50

TCCATCATTC TTCGGGTACA TCCTTACTCT CTGGCACAGT AGAGATGTTC 100

CAGACTTATC TTATATTTTC ACTTCCCCAT ACCTGGAATC AATCACTTCT 150

CCGAGGATGC TTGATTCCTT TTAGTGAAGA ACAGTCTTTG GAAACCAACC 200

GTCTAGGGAC ATCAAGTATG TTTGCCGCTA TTGGAGTGTC ATTGCTCCTG 250

AACCTTCTAA GTGGACACAG CTAGGAAATG TATGTGCT 288

(2) INFORMATION FOR SEQ ID :835:

(i) SEQUENCE CHARACTERISTICS:
(A) LENGTH: 310 base pairs
(B) TYPE: nucleic acid
(C) STRANDEDNESS: double
(D) TOPOLOGY: linear

(xi) SEQUENCE DESCRIPTION: SEQ ID :835:

CTTTTCTATT TTCCTTAAGT GTCTGCCAGT CTGAGAAATA AAGGGACAGA 50

GTACAAAAGA GAGAAATTTT AAAGCTGGGT GTCCGGGGGA GACATCACAT 100

GTCGGCAGGT TCCGTGATGC CCCACAAGCC ACAAAACCAG CAAGTTTTTA 150

TTAGTGATTT TAAAAGGGGA GGGAGTGTAC GAATAGGGTG CGGGTCACAG 200

AGATAACGTG CTTCACAAGA TAATAGAATA TCACAAGGCA AATGGAGGCA 250

GAACGAGATC ACAGGACCAC AGAACTGGGA CCAAATAAAG ATTGCTAAGA 300

ACGTCTAGGG 310

(2) INFORMATION FOR SEQ ID :836:

(i) SEQUENCE CHARACTERISTICS:

(A) LENGTH: 177 base pairs

(B) TYPE: nucleic acid

(C) STRANDEDNESS: double

(D) TOPOLOGY: linear

(xi) SEQUENCE DESCRIPTION: SEQ ID :836:

TCAGTGGAGG AATTAAAACT GGTGGAGAAG GGGAGGTCAG GTTATCAGTT 50

GAAATGCCAA GGTTATCTCT AATCTTAGCT AGATTCTGCC AAAGTTCACA 100

GAGGTAATCA AAAACTTGAG CATGTATTTA AGGATCCATG ATTGAATTTA 150

CATCTCCCAA AATGCCTAGC ATTCTTC 177

(2) INFORMATION FOR SEQ ID :837:

(i) SEQUENCE CHARACTERISTICS:
(A) LENGTH: 135 base pairs
(B) TYPE: nucleic acid
(C) STRANDEDNESS: double
(D) TOPOLOGY: linear

(xi) SEQUENCE DESCRIPTION: SEQ ID :837:

CCGTTGCACT TGGTGTTGGC ATTCTGCAGG GCGGCACTCT CCCACTCTTC 50

CCGGCCTCGA GCCAACCTGA CGGCAGCCAG ATGGCAGTCG TAGTGCACAA 100

TGTTGAAGTG GGACACGGTG CTGTAGCCCT GCTGT 135

(2) INFORMATION FOR SEQ ID :838:

(i) SEQUENCE CHARACTERISTICS:
(A) LENGTH: 201 base pairs
(B) TYPE: nucleic acid
(C) STRANDEDNESS: double
(D) TOPOLOGY: linear

(xi) SEQUENCE DESCRIPTION: SEQ ID :838:

TCAGTGGAGG AATTAAAACT GGTGGAGAAG GGGAGGTCAG GTTATCAGTT 50

GAAATGCCAA GGTTATCTCT AATCTTAGCT AGATTCTGCC AAAGTTCACA 100

GAGGTAATCA AAAACTCGAG CATGTATTTC AGGATCCATG ATTGAATTTA 150

CATCTCCCAA AACGCCTAGC ATTCTTCGCC ACATTACAGT AGCAACATCA 200

G 201

(2) INFORMATION FOR SEQ ID :839:

(i) SEQUENCE CHARACTERISTICS:
(A) LENGTH: 181 base pairs
(B) TYPE: nucleic acid
(C) STRANDEDNESS: double
(D) TOPOLOGY: linear

(xi) SEQUENCE DESCRIPTION: SEQ ID :839:

TTCTTTGGAG TACATCCTTA CTTTCTGCAC AACAGATGAG AGATGTTCCA 50

GAACTTATAC TGTATCATAT ATTTTACCTC CCCCCCTATA CACCCTGAGA 100

TATACATATA CACTATCTCC GCTAGAGAGA TGTCTATGAC ATATCACTTT 150

CTGAGATGTA CACGAACGAG ATCTTTTGAG A 181

(2) INFORMATION FOR SEQ ID :840:

(i) SEQUENCE CHARACTERISTICS:
(A) LENGTH: 222 base pairs
(B) TYPE: nucleic acid
(C) STRANDEDNESS: double
(D) TOPOLOGY: linear

(xi) SEQUENCE DESCRIPTION: SEQ ID :840:

CATACAAATG ATTAGAAAGC AATACAGCCT TATTGCTGAT ATGGAGAAGG 50

TTTTAGTGAA CATTCAGAGG AGTTTGGAAG AAAGTCGCTG CACAATCTTC 100

ATGGATGACT TTGAGGGATT CAAGACTTCA GTGGAAGAAC TAACTGCATA 150

TGTGAGTAGA AATGGCAGGA GAACTAGAGT TCGAAGTAGG AGCTGGAAGA 200

TAATAACATG GGTTTAAAAA AC 222

(2) INFORMATION FOR SEQ ID :841:

(i) SEQUENCE CHARACTERISTICS:

(A) LENGTH: 293 base pairs

(B) TYPE: nucleic acid

(C) STRANDEDNESS: double

(D) TOPOLOGY: linear

(xi) SEQUENCE DESCRIPTION: SEQ ID :841:

CGCTCAGCTG GTCTATCCTG CTCTTCCTCT TGTCCTTGCT CTTGCTGCGG 50

CTCCTATCTA CCGGCTACCT GCTGCCCGCT TTGCTCCTGC TACCGGCTCC 100

GACTCTGGCG GAGGCTCTTC TCCTGGCTCC TGCCCCTGCT CACAACTCCC 150

TCGCTTCTCC TCCTCCACTC TCCTCTCCCG ACTCCTGCTC CGACTTTTGC 200

TCTTACTTTT ATGCCTGCTA GGACTCCGGC TCTTGGGTTT CCCGAACATT 250

GTCATTGTTT TGGAACTTCT CCTTCAGCTT GGTCTTTTGC CGG 293

(2) INFORMATION FOR SEQ ID :842:

(i) SEQUENCE CHARACTERISTICS:
(A) LENGTH: 239 base pairs
(B) TYPE: nucleic acid
(C) STRANDEDNESS: double
(D) TOPOLOGY: linear

(xi) SEQUENCE DESCRIPTION: SEQ ID :842:

ATGAGGAGGT GAGGAAAAGG AGAAGTGCAG CTACCATCGA GTGATTCTTC 50

TGGGATAGGT GTCTCATTTA AATCTCATAA TCATCTTTTT TGGCAGGTCA 100

GTCAACTTCA GGCTCACAGA TGACAGACAG TTGGCCCAAA GACACACAGG 150

AAATACATAA GTGACAACGG AATACAAGTC CATGAATTAA AAAACCATGC 200

TTTTCTCTCC TTGCCACACA GCTTTAGTTT GAAAAAAAA 239

(2) INFORMATION FOR SEQ ID :843:

(i) SEQUENCE CHARACTERISTICS:
(A) LENGTH: 154 base pairs
(B) TYPE: nucleic acid
(C) STRANDEDNESS: double
(D) TOPOLOGY: linear

(xi) SEQUENCE DESCRIPTION: SEQ ID :843:

TTCCGAATTA TTGAATCTTC TTCTGTAACA TCACAATCTT CCTGGTTTTC 50

AGAATAAACG CTCTTCTCGC TCGCCTCTCT CTGCACTCAC TCCCACCTCA 100

CTCACTCACT CTATAATAAA ATGTTTGCAC TCAATTTATA TAGTAGTGTT 150

TGTC 154

(2) INFORMATION FOR SEQ ID :844:

(i) SEQUENCE CHARACTERISTICS:
(A) LENGTH: 392 base pairs
(B) TYPE: nucleic acid
(C) STRANDEDNESS: double
(D) TOPOLOGY: linear

(xi) SEQUENCE DESCRIPTION: SEQ ID :844:

GTCTTTAATT GAGAAAGCTG GAGTGGAGAC CCGATCCCCT GGGAGCAGTG 50

CCAGGAGTTG GGTGGAGACT GAGTGGGGTT TGTGTGGGTG AGGGGGCATC 100

TACTCCTCTT GCAACAAGCC AGAAGTAGAA CAGCCTAAGG AAAAGTGACC 150

TGCCTTGGAG CCTTAGTCCC TCCCTTAGGG CCCCCTCAGC CTACCCTATC 200

CAAGTCTGAG GCTATGGAAG TCTCCCTCCT AGTTCACTAG CAGGTTCCCC 250

ATCTTTTCCA GGCTGCCCCT AGCACTCCAC GTTTTTCTGA AAAAATCTAG 300

ACAGGCCCTT TTTGGGTACC TAAAACCCAG CTGAGGTTGT GAGCTGTAAG 350

GTAAAGCAAG TTCTATCCAA TTAGAAGCTG TTGGGGGCGT AT 392

(2) INFORMATION FOR SEQ ID :845:

(i) SEQUENCE CHARACTERISTICS:
(A) LENGTH: 200 base pairs
(B) TYPE: nucleic acid
(C) STRANDEDNESS: double
(D) TOPOLOGY: linear

(xi) SEQUENCE DESCRIPTION: SEQ ID :845:

CAAAGAATAA ATAATTTTTC TATTCCTGAA AGTTAACTAA TTATTTATTA 50

GAAAGTCAGA AATATGTGGA AAGCAAAGGA ATATTTGAGA AAGTGATATG 100

AAATTAATAA GTGGTAAAAA ATTAATAAAA TTAATATTAG AGTTTCCTTT 150

GAGCTAATCC TTTATTTATT TATTTTTTTC CTTGAGACAA TGTCTTGCCA 200

(2) INFORMATION FOR SEQ ID :846:

(i) SEQUENCE CHARACTERISTICS:
(A) LENGTH: 311 base pairs
(B) TYPE: nucleic acid
(C) STRANDEDNESS: double
(D) TOPOLOGY: linear

(xi) SEQUENCE DESCRIPTION: SEQ ID :846:

TTAAAAATAC TCTGGAAAGA GCACTTCCAT CGTTCATTTA CATCATATTA 50

GAAATGGATT TCCGAGTCAG CACTGGCTTC CTCCAGAATA GCAGTCCTGG 100

AGGAGCGAGA TCTCCCCAAG CCTCCAGGAT TGCAGTCTGT TTTGTGCCTT 150

CACTCCCTAA TAGGTTTGCC TTATTTAAAG GACCCACCTT CAGAGCTGCC 200

AAGGACTTTC TCAGAGCAGC TCCTTGGTCC CTCCGACAGC CTGGGATGGC 250

ATTTATTCTG GGGCCTGGGT GTGGGGAGGT CCTCACCAGC CTAGGAGTAA 300

GAGGAGGTGG T 311

(2) INFORMATION FOR SEQ ID :847:

(i) SEQUENCE CHARACTERISTICS:
(A) LENGTH: 287 base pairs
(B) TYPE: nucleic acid
(C) STRANDEDNESS: double
(D) TOPOLOGY: linear

(xi) SEQUENCE DESCRIPTION: SEQ ID :847:

CAGATCAAGA CTGGTGCCCC TTGCCGATCT GAGCGCTTGG CCAAGTACAA 50

CTCAGCTCCT CAGAATTGAA GAGGAGCTGG GCAGCAAGGC TAAGTTTGCC 100

GGCAGGAACT TCAGAAACCC CTTGGCCGGG TAAGCTGTGG GCAGGCAAGC 150

CCTTCGGTCA CCTGTTGGCT ACACAGACCC CTCCCCTCGT GTCAGCTCAG 200

GCAGCTCGAG GCCCCCGACC AACACTTGCA GGGGTCCCTG CTAGTTAGCG 250

CCCCACCGCC GTGGAGTTCG TACCGCTTCC TTAGTCT 287

(2) INFORMATION FOR SEQ ID :848:

(i) SEQUENCE CHARACTERISTICS:
(A) LENGTH: 263 base pairs
(B) TYPE: nucleic acid
(C) STRANDEDNESS: double
(D) TOPOLOGY: linear

(xi) SEQUENCE DESCRIPTION: SEQ ID :848:

GATTACAGGT GTGAGCCACC GTGCCTGGCC TATTAATATG CTCTTAAAAC 50

TCATCCATAC GTTTTCATAG CAAGAAAGCT TATTTCTCCC AACTACCGAA 100

TAATATTCTA TCGTATGGCC GCACCTCTGT TTATCCATTT ACCTATCGAG 150

AGGCATCTTG ATTACTTCTA GCTTTGGATT ATTACAAATA AAACTATACA 200

AACACGCAAA CAGAGTTTCT CGTGTGAATA TAAACCCGCA AACCGGCTGG 250

AGGCATATNA CCA 263

(2) INFORMATION FOR SEQ ID :849:

(i) SEQUENCE CHARACTERISTICS:
(A) LENGTH: 279 base pairs
(B) TYPE: nucleic acid
(C) STRANDEDNESS: double
(D) TOPOLOGY: linear

(xi) SEQUENCE DESCRIPTION: SEQ ID :849:

TTGTCAGTCA GAATAGGATG TAAAAGACTA TTTCAAAGAG TTAAATTATT 50

TGTTAATATC AGAGATCAGC CACAGACAAG AAGTTTATGG ATGAGTGCAG 100

CAGTGGTCCA TTGGACATGT TAAATACTCG TGGATATCCA CAATTCGAAT 150

TGACATTAAA AACGAATGGA TACCCAACTC TGAATTCCAT ATCGTTTTTT 200

AATATCAAAA ACACAATTTT AACTACTGAT AAACCAGGCA ACCACCGCAA 250

GTTTATCGAA ATCCTGCCGC TACTAAACA 279

(2) INFORMATION FOR SEQ ID :850:

(i) SEQUENCE CHARACTERISTICS:
(A) LENGTH: 173 base pairs
(B) TYPE: nucleic acid
(C) STRANDEDNESS: double
(D) TOPOLOGY: linear

(xi) SEQUENCE DESCRIPTION: SEQ ID :850:

GCTGAAAGTT GAAGTGGAGA GCTTGAAACG AGAACTCCAG GACAAGAAAC 50

AGCATCTGGA TAAAACATGG GCTGATGTGG AGAATCTAAA CAGTCAGAAT 100

GAAGCTGAGC TCCGACGCCA GTTCGAGGAG CGACAGCAGG AGACGGAGCA 150

TGTTTATGAG CTCTCGACAG AAT 173

(2) INFORMATION FOR SEQ ID :851:

(i) SEQUENCE CHARACTERISTICS:
(A) LENGTH: 240 base pairs
(B) TYPE: nucleic acid
(C) STRANDEDNESS: double
(D) TOPOLOGY: linear

(xi) SEQUENCE DESCRIPTION: SEQ ID :851:

TCGAGAAAGT TTCAATGTGA TTAGATTAAA ATTAAAAGAA TCCATAAAAA 50

TGGAACAAAG AAGAAGAGGA ATGAAATTAC TTTTACTTTA AACAGCATTG 100

TTATCACATA AAACACGTAT CTTACAAATT CATGGGATAG CCCATAAATG 150

GGACTACAGC AACAATGGTA GGAGAGTCCA TCCTTCTTCA AAAGCAACCC 200

AGCAGGAATT TTCTGTTAAA AATATTTTTG CCGTAATACT 240

(2) INFORMATION FOR SEQ ID :852:

(i) SEQUENCE CHARACTERISTICS:
(A) LENGTH: 314 base pairs
(B) TYPE: nucleic acid
(C) STRANDEDNESS: double
(D) TOPOLOGY: linear

(xi) SEQUENCE DESCRIPTION: SEQ ID :852:

ACTGGACTGG TCAAGAACAA AGCTCATGGC AATAGTCTTT TGGAATGTTA 50

AAGGAATTTT TCTAGTTGGC TTTCTGGAGG GCCAAAGAAC AACAACATCT 100

GCTTATTATG AAAGTGTTTT GAGAAAGCCA AAGCTTTAGC AGAAAAACAC 150

CTGGGAAAGC TTCATTAGAG AGTCCTTCCC CATCACAACG CTTCTGCTTA 200

TTCCTCTTAT AAAACAAGGG CAATTTTGTG AGAGCTACTG ACGATTTTCC 250

CCTCGAAATT TACTATTTCT ACAGCTTATT AATCTCAAAT AGTAAGCATG 300

TCCTTGCCGT CACT 314

(2) INFORMATION FOR SEQ ID :853:

(i) SEQUENCE CHARACTERISTICS:

(A) LENGTH: 267 base pairs

(B) TYPE: nucleic acid

(C) STRANDEDNESS: double

(D) TOPOLOGY: linear

(xi) SEQUENCE DESCRIPTION: SEQ ID :853:

TCCTGAAATA CTCTCTACTG TCACTGCTTC AGTTTAATTT ATAGATAGCA 50

CCAGATATGA GTCCTTATTA GTTCTGATAA CTCTGAAATG GTTATTTGGT 100

ATTTCTTCAG TGACAAGCAA ACTATCCCCC CACATGCCTT TAATGGCCAG 150

TGTTTTCGAT TTGTATACCA ATAAGCAATC TAGGTAGAGG TAATCATATA 200

CTGATACGCT AACCTTTGAA ACATAATTTC CAATCTAGTA AGCTAAAATC 250

GCGCCAATAC TGCTTAA 267

(2) INFORMATION FOR SEQ ID :854:

(i) SEQUENCE CHARACTERISTICS:
(A) LENGTH: 186 base pairs
(B) TYPE: nucleic acid
(C) STRANDEDNESS: double
(D) TOPOLOGY: linear

(xi) SEQUENCE DESCRIPTION: SEQ ID :854:

GCAGTGGATC TTTCTTTTTT TCCAAAGGAA ATTTCATATA GAGTCCATTC 50

ATAGGAAACA GATAAAATGT GAACGGCTGC AACTGAGATG GGGGAGAGTG 100

GCTTGGAGCC CCCAGCCTCT TTGCTTTCTC TTATCCCTAT AGGATGGCCA 150

TTAGGTGAAG CAGTTTAGCT TGTTGGTTCA GACCTG 186

(2) INFORMATION FOR SEQ ID :855:

(i) SEQUENCE CHARACTERISTICS:
(A) LENGTH: 162 base pairs
(B) TYPE: nucleic acid
(C) STRANDEDNESS: double
(D) TOPOLOGY: linear

(xi) SEQUENCE DESCRIPTION: SEQ ID :855:

ATTTTGAAAA GAAACCTACA AAAATTGTAT TTCCATATTT CATAGTCAGC 50

CAAAAATATT GGTCAACTCA TGCTCTCTGC AAGGTTCTGC AAAAATTGCG 100

GAGTGGTATA GACAAGGCAC CATTGCAATA TATAATACTT TTTGGGTATT 150

GGCCCAAAAA TT 162

(2) INFORMATION FOR SEQ ID :856:

(i) SEQUENCE CHARACTERISTICS:
(A) LENGTH: 286 base pairs
(B) TYPE: nucleic acid
(C) STRANDEDNESS: double
(D) TOPOLOGY: linear

(xi) SEQUENCE DESCRIPTION: SEQ ID :856:

TCTGTGGCCA CAGATGCATA TTACAAGCCC ATCCAGAGCC ATTATGAAGA 50

GAAAGTGGCA ATTTTATTCT CAGCCTGAGA AATCCACACC AATCTAGTCT 100

GGGTTTCCAA GGATCTGCCG TTTAAGTCAC TTAGCCTCTA TCCTTTCCGG 150

GAACAACCTG CCCCTAGAGA GGGTGAACGA GAATCGTCCC CAGTGCCTCC 200

AACATAACAA ACCCAATCGC CCTGTTAACT TTAAATCTTC AACTCGAGAA 250

AAAGGCTCAT GACTACTTCT AACCATGCCC AAACCC 286

(2) INFORMATION FOR SEQ ID :857:

(i) SEQUENCE CHARACTERISTICS:
(A) LENGTH: 280 base pairs
(B) TYPE: nucleic acid
(C) STRANDEDNESS: double

(D) TOPOLOGY: linear

(xi) SEQUENCE DESCRIPTION: SEQ ID :857:

AACTATATGG TATTATTTCA CGCTGTACCC AGTCCATTGC TTGAACTTAC 50

GGGTACCTAA TGAAACGTGG AGGTCCGGAT GTATGAAAAT CTCCTCTTTT 100

CCCCTTTACT TACAGCCTCT GTAGGCAATA ATTATAGAGT AGTATAGATG 150

ATTTTTCTTT TTTATAAATC TGCCTATCTC CAGGAGGATG GGGCGCGCAC 200

TTTTAGAAAT GCATATAAAT GCTCTACGCT CCTTTTTTCT GTTACTTAAT 250

CGGCGCCAAG GCCTTTACAT GAATACTCAG 280

(2) INFORMATION FOR SEQ ID :858:

(i) SEQUENCE CHARACTERISTICS:
(A) LENGTH: 310 base pairs
(B) TYPE: nucleic acid
(C) STRANDEDNESS: double
(D) TOPOLOGY: linear

(xi) SEQUENCE DESCRIPTION: SEQ ID :858:

CACTGGTTTT CATGACTGAT TTCTTTATTT TTAGTAGTTC TGACATGTTG 50

GCCAGGCTGG TCTTGAACTC CCAGCCAACC TCAAAGTGCT GGGATTACAG 100

CTGTGAGCAC CAGCCCAACC TCGCCTCTTT AAAAAGAAAA AACACAAGTC 150

CACTCTGAAG TCAGCCTCTG TAACCTCCCC ACAAGAAAAC CGTTTTACAT 200

CAGTCACTAA CCAAACAACC AACAGTGCTT CAACACAGAA AGTAAAGCAT 250

TATACAGGGC TTGAACTGTC TTTTAAGCAA GCCCCAAATC CTTTGAAAGG 300

AGGCAGTAAC 310

(2) INFORMATION FOR SEQ ID :859:

(i) SEQUENCE CHARACTERISTICS:

(A) LENGTH: 158 base pairs

(B) TYPE: nucleic acid

(C) STRANDEDNESS: double

(D) TOPOLOGY: linear

(xi) SEQUENCE DESCRIPTION: SEQ ID :859:

TCTGTGTGGA GGGGAGGGAC CCAGTCTGGA CCCAGGTGTC ATCATCTCAG 50

CCACAGCAGG GCCCTCTCAG GCTGGGAAAC TTCTGCCAGA GCTGGCGAGT 100

CCTCTGCAGG TCAAGCCAGG GCTTGGACAC AACTACTTCA TCTATCGCGC 150

AGGAAGAG 158

(2) INFORMATION FOR SEQ ID :860:

(i) SEQUENCE CHARACTERISTICS:

(A) LENGTH: 263 base pairs

(B) TYPE: nucleic acid

(C) STRANDEDNESS: double

(D) TOPOLOGY: linear

(xi) SEQUENCE DESCRIPTION: SEQ ID :860:

TTTCTAATAA GAACATAAAA TAAAGGCTAA TTAAAAGAAG GTGACTGAGT 50

CCAGGAAGGC ACTAATCAAA GATGATATAC AGCCAGGTAA AAAAGAACAA 100

TTCACAGGCA CAAGACTATA TAACCATCGT TTTATTTTCA AACGTTATAC 150

AAAATATACT CTACCGAACT ATAATAGATC ATTAATAGTG GCACAATCTT 200

TAGATTCGGA ATAACTTGAA AATAAATCCC AACCACGTAA CTTACTAAAG 250

GAATAATGAG CCA 263

(2) INFORMATION FOR SEQ ID :861:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :861:

ATTTGTCTGA AAAATATATT TCTACACGAT CTGATAAAGT TCAGATAAGG 50

GGCATTCTAT TCCTAAGAAT GTCCTAAAAA TGGAAAACTG ATAAAAGATT 100

ATGCTTAGAA TTATACAGGT AAAAGACCTA CAACAATTGT TCTCTTGTTT 150

TGATCCCATA GGTCATTTGT TATTACTCTT CTTCAACAGA GTGATTTTCT 200

ATTATAAACT TTCTTAGGAG CCAATACAGA GTAGAAAGAA ATATTTTCAG 250

AAAG 254

(2) INFORMATION FOR SEQ ID :862:

(i) SEQUENCE CHARACTERISTICS:
(A) LENGTH: 337 base pairs
(B) TYPE: nucleic acid
(C) STRANDEDNESS: double
(D) TOPOLOGY: linear

(xi) SEQUENCE DESCRIPTION: SEQ ID :862:

GTTCCTCCTT TGTAAATTAT GAAATATTTA TAGTTTAGAC TGAGTAATAT 50

GACATGAAAC AACAAACCTG CACATTTCTA ATTTATAACA AATCCGTTTC 100

CTTAATGGGT GGAAGGAAAT CTGAGGACAA TTCTAAGGAG TCTTGTCTGC 150

TTTCAGTGCG ATCTTCTAGT CATACTGAAG ACAATACCTC TCCAGATTGA 200

TCTTTCCCCT TCTTATACTC TTCCCGATCA ACGTCAATCA CACGCACCAC 250

TCCAGGTTTT AGACTTAGGT CATCCGTCTC TACCTGACTT CTGTTGCCCT 300

TCACCTACAC GTGGCTCGCT TTAGTTTGCT GAGCAGC 337

(2) INFORMATION FOR SEQ ID :863:

(i) SEQUENCE CHARACTERISTICS:
(A) LENGTH: 169 base pairs
(B) TYPE: nucleic acid
(C) STRANDEDNESS: double
(D) TOPOLOGY: linear

(xi) SEQUENCE DESCRIPTION: SEQ ID :863:

TGGTGGGTTC GAGCCCAACT TCAGCGCTCT TCGAGAAGTG CGAGGCGAAC 50

GGTGCGGGGG CGCCTCTCCT CTCCACCTTC CTGCGGGAGG CCCTGCAAGC 100

TCTCAGCGAC GACGCCACCA TCTTCTGACC AACTTCAGGC TACTACCCCA 150

ACTTACAATG CCACCGCGA 169

(2) INFORMATION FOR SEQ ID :864:

(i) SEQUENCE CHARACTERISTICS:
(A) LENGTH: 286 base pairs
(B) TYPE: nucleic acid
(C) STRANDEDNESS: double
(D) TOPOLOGY: linear

(xi) SEQUENCE DESCRIPTION: SEQ ID :864:

ATTAAGGTAA AGGAAGACTT TCCATTGTTA AGTAGAAAGA GTGTCCTGCT 50

ATTACTACCA TTCACATCTA GTTTGTGTGA ACTAGGGTTT TCTATCTTAA 100

CCCTGTTAAA AACAAAGGAA AGAAATGGGC TGAATGGCGC ACCAGCTATG 150

CGGATAGCAT TATCTTCCTG TGTTCCAGAC TGGAATGAAT TTATGAACAA 200

GGCAAGCACA CCATTATAGT AAATAAAATC TTACCTAGTT TTCGTTTTTG 250

CATTTCTTAC TTCGCAGTAT TTCTCCCGCC AAGAGC 286

(2) INFORMATION FOR SEQ ID :865:

(i) SEQUENCE CHARACTERISTICS:

(A) LENGTH: 272 base pairs

(B) TYPE: nucleic acid

(C) STRANDEDNESS: double

(D) TOPOLOGY: linear

(xi) SEQUENCE DESCRIPTION: SEQ ID :865:

ATATAGTCCG CTAGGGATAG TGAAAAATTT GGGGCTTGGT TTAGATTGGA 50

GAGGTATATG GTGGGGTGTC TTATAAGGCT TAACTTTGGG AGGGCCCAGG 100

GCCTTGATTT CTGTCCCCCT TGCCTTGCGT GGCCTTTGAA TTGAAAGCTT 150

TGAATTAGGC CTTCATAGAT TTTAGTGTGC ATACAAATAA CACGATCTTT 200

TTATAAGGCA GATTATGATT CTGAAGGTTT AGTGTGGGAC CCAAGCTTTC 250

GCATTTCTGA TCTATAAGGT GA 272

(2) INFORMATION FOR SEQ ID :866:

(i) SEQUENCE CHARACTERISTICS:

(A) LENGTH: 296 base pairs

(B) TYPE: nucleic acid
(C) STRANDEDNESS: double
(D) TOPOLOGY: linear

(xi) SEQUENCE DESCRIPTION: SEQ ID :866:

ATGCAGGAGA AGAAGGATGC CAAACTAAGT CAAAAAAACG GAACGCAAAT 50

AAAAGGAATG AGCACGGTTA CAAAGTCACA GGATGAGTCC CTGGGATCTG 100

GGGCGGGAGA AGGGGTGAAT CAAGAATGAC TTGAGCTTGT TACTCCCTAG 150

CAGGCTGAGG GCGTGACACA GCAGCTCGAT GACAAAGAGG TCTATTATAG 200

TTTCTAACAC TACAACGCTA ACTTTTGGAA CGTATCTACT TCTAGCATGT 250

AGACAGATCT CTAATCGCCT GCCAGACGGT AAGCCGCAGG AATGCG 296

(2) INFORMATION FOR SEQ ID :867:

(i) SEQUENCE CHARACTERISTICS:
(A) LENGTH: 241 base pairs
(B) TYPE: nucleic acid
(C) STRANDEDNESS: double
(D) TOPOLOGY: linear

(xi) SEQUENCE DESCRIPTION: SEQ ID :867:

TATTTTATCT CAACATAATA AAAACGACTA TAAATCTTCC TAAGAGAATG 50

CTCTACTCCC AAGACTAATT TAAACTCGGG GATCGTCAGA GGGAGTGCCA 100

CTGTGACTTC TACGATGAAA GAACAAGGGA GAGAAGATCT TATCGACAAT 150

CATACAAAGC CATATATACG CTATTCCTCA ACTCACAGAG TTAATTAAAT 200

GTCACCAGGA TGGAAGAAAC CTTATAAGCC CCTATCTATC A 241

(2) INFORMATION FOR SEQ ID :868:

(i) SEQUENCE CHARACTERISTICS:
(A) LENGTH: 130 base pairs
(B) TYPE: nucleic acid
(C) STRANDEDNESS: double
(D) TOPOLOGY: linear

(xi) SEQUENCE DESCRIPTION: SEQ ID :868:

GGACGACTGC AAGCTGGAAC TACACACGCG CACTCGCTCA CACTACACAC 50

ACGCCACTCT CACACACTCT CACACACGCA CCCTCTCGCG CATGCTCTCT 100

CAAAGCCCAA TATAACCAAG GGGAAGGAAT 130

(2) INFORMATION FOR SEQ ID :869:

(i) SEQUENCE CHARACTERISTICS:
(A) LENGTH: 310 base pairs
(B) TYPE: nucleic acid
(C) STRANDEDNESS: double
(D) TOPOLOGY: linear

(xi) SEQUENCE DESCRIPTION: SEQ ID :869:

TTCAAATATT TATGTATGTT TGAACATTTT CATAATAAAA TGTTGAAAAA 50

CTAATGAGAA TGGCATAAAC AACATTTAAG CAATATATTT TGAAATTTAA 100

TTCAAATGGT CAAATTCCTG GAAAACACAA ACTCCCTTCA CTAACAGAAT 150

TGATAGAAAA TCTGAGTAGT TCACCATTGT TAAAGAAATG GAATGTGCCA 200

TTTAAAACCC TCCAATTGAA AATACTACAT ATAGTTACAA TAGGGAATTT 250

TCCCAAGCAC TTAAACAATA AACAATGCCC TCTTTATACA AACCTTTCCC 300

AGTAATAGAA 310

(2) INFORMATION FOR SEQ ID :870:

(i) SEQUENCE CHARACTERISTICS:

(A) LENGTH: 192 base pairs

(B) TYPE: nucleic acid

(C) STRANDEDNESS: double

(D) TOPOLOGY: linear

(xi) SEQUENCE DESCRIPTION: SEQ ID :870:

AAAGAGGCAA GTTCCTGGTG CAAAGGTGGC TCTGCAGCAT AATTTAGGCA 50

TTGGAGGAGC TGTGTGTTGT AACACTCTAC AAGATGGGTT TTCCGGAAGC 100

CGCCAGTTCT TTTAGAACTC ATCAAATTGA AGCTGTTCCA ACCAGCTCTG 150

CAAGTGATGG ATTTAAGGCA AATCTTGTTT TTAAGGAGAT TG 192

(2) INFORMATION FOR SEQ ID :871:

(i) SEQUENCE CHARACTERISTICS:
(A) LENGTH: 250 base pairs
(B) TYPE: nucleic acid
(C) STRANDEDNESS: double
(D) TOPOLOGY: linear

(xi) SEQUENCE DESCRIPTION: SEQ ID :871:

ACCATAGATG TGTTCCAGAG GCAAAAGAGA CACATTATCC TAGATGGCAG 50

AACATGCTTT CAAAACATAT AAAACGTCAA AGTTCCAGAT CTTTCTACAT 100

TTTAAATCCT GTCTGAGGAT GGCAGCTGAC TTTATGTAGC TGATAGACGA 150

CTAGAGTTTC ATCCAAATAC CTGACCACGA CTTCATGGAG ATTTGAATAA 200

TCTATCCGAT GAGATTTATA TNTAAACAAC TCAACTCCTG TCGAAACAAA 250

(2) INFORMATION FOR SEQ ID :872:

(i) SEQUENCE CHARACTERISTICS:
(A) LENGTH: 313 base pairs
(B) TYPE: nucleic acid
(C) STRANDEDNESS: double
(D) TOPOLOGY: linear

(xi) SEQUENCE DESCRIPTION: SEQ ID :872:

TCAGCTACGA ATTGCTTCCT TCTCACAGAA CTGTGACATT TATCCAGGGA 50

AGGATTTTGT ACAACCACCT ACCAAGATTT GCGTGGGCTG CCCCAGAGAT 100

ATACCCACCA ACAGCCCAGA GCTGGAGGAG ACACTGACTC ACACCATCAC 150

AAAGCTTAAT GCAGAGAATA ACGCAACTTT CTATTTCAAG ATTGACAATG 200

TGAAAAAAGC AAGAGTACAG GTGGTGGCTG GCAAGAAATA TTTTATTGAC 250

TTCGTGGCCA GGGAAACACA TGTTCCAAGG AAAGTAATGA AGAGTTGACC 300

GAAAGCTGTG AGA 313

(2) INFORMATION FOR SEQ ID :873:

(i) SEQUENCE CHARACTERISTICS:

(A) LENGTH: 270 base pairs

(B) TYPE: nucleic acid

(C) STRANDEDNESS: double

(D) TOPOLOGY: linear

(xi) SEQUENCE DESCRIPTION: SEQ ID :873:

TAGATGCTGA TGGCAATGAC ACAATTGACT TCCCTGAATT CCTGACAATG 50

ATGGTAAGAA AAATGAAAGA CACAGACAAT GAAGAAGAAA TTAGAGAAGC 100

ATTCCGTGCG TTCGATAAGG ATGACAATGA CTATATTAAC GCTGCAGAAC 150

TTAACCATAC AATGACAAAC CCCGAAGAGA AGTTAATAAA TGAAGAAGTT 200

GATGAAATGA TCAAAAACGT AGATNATTGA TGACGATGGT AAGGTAAACT 250

ATCCAGCGTT AGCACAAATG 270

(2) INFORMATION FOR SEQ ID :874:

(i) SEQUENCE CHARACTERISTICS:
(A) LENGTH: 229 base pairs
(B) TYPE: nucleic acid
(C) STRANDEDNESS: double
(D) TOPOLOGY: linear

(xi) SEQUENCE DESCRIPTION: SEQ ID :874:

TTGGGCCATG TCCCCATTTT ATCTCCAAAG CCAGCCATGG TGTATTTCTC 50

TCTGCTTTTC TTCCATATTT CCAACCCAGA AATGACTCCT GTTCCCATAT 100

GTGATTATTA TATTGCGCTA ACCCAAGTGA TTAAGGATAA TCTCACTACT 150

TAATGACAGC TGATTATTTC CATCTGCAAA CTTACTCAAG AATGCAATCC 200

AGACTAACAC GACAATAGGA CATCAAGCT 229

(2) INFORMATION FOR SEQ ID :875:

(i) SEQUENCE CHARACTERISTICS:
(A) LENGTH: 294 base pairs
(B) TYPE: nucleic acid
(C) STRANDEDNESS: double
(D) TOPOLOGY: linear

(xi) SEQUENCE DESCRIPTION: SEQ ID :875:

TTTGAACTTT CAGCCGAATA CATCTTTTTC CAAAGGAGTG AATTCAGGCC 50

CTTGTATCAC TGGCAGCAGG ACGTGACCAT GGAGAAGCTG TTGTGTTTCT 100

TGGTCTTGAC CAGCCTCTCT CATGCTTTTG GCCAGACAGA CATGTCGAGG 150

AAGGCTTTTG TGTTTCCCAA AGAGTCGGAT ACTTCCTATG TATCCCTCAA 200

AGCACCGTTA ACGAAGCCTC TCAAAGCCTT CACTGTGTGC CTCCACTTCT 250

ACACGGAACT GTCCTCGACC CGTGGGTAAC AGTATTTTCT GCGT 294

(2) INFORMATION FOR SEQ ID :876:

(i) SEQUENCE CHARACTERISTICS:
(A) LENGTH: 173 base pairs
(B) TYPE: nucleic acid
(C) STRANDEDNESS: double
(D) TOPOLOGY: linear

(xi) SEQUENCE DESCRIPTION: SEQ ID :876:

ATTTTTGGTA ACATACATCA AGTGGCACTA ATTACACAGT AACTATAAGG 50

TAACTAACAT GAAACCACAG AACTGTAACT CTGCCACAGC TGCATGAACT 100

CGGGTTGTCC TGACCGAGCC CATCCCCAAA AAACCGCCCA CCCCAGAGCT 150

ACGCCAACAA AAACCGTTAT TAA 173

(2) INFORMATION FOR SEQ ID :877:

(i) SEQUENCE CHARACTERISTICS:
(A) LENGTH: 143 base pairs
(B) TYPE: nucleic acid
(C) STRANDEDNESS: double
(D) TOPOLOGY: linear

(xi) SEQUENCE DESCRIPTION: SEQ ID :877:

TAATCAAAAG AGCTCTAAAT CTGTAATTTC TTTCTCCTTT AAAAAAATGT 50

CTACTTTGTT TTGGTCCTAG GCATTAGGTA ATATAACTGA TAATATACTG 100

AAACATATAA CGGAAGATGC AGATGATAAA ACTAATTTCG AAC 143

(2) INFORMATION FOR SEQ ID :878:

(i) SEQUENCE CHARACTERISTICS:
(A) LENGTH: 213 base pairs
(B) TYPE: nucleic acid
(C) STRANDEDNESS: double
(D) TOPOLOGY: linear

(xi) SEQUENCE DESCRIPTION: SEQ ID :878:

ACTGAATAAA CTGCTGATGT CCAGGTTCAG TGGTTCCTGC TGTGGGACTT 50

GCGTTTTCAC TAAGTGTGGC GTTAGATCCA TTAGTTCCCG AAGAGCCTCC 100

AGTGCTTCCT AATGCCCCCA AGCCAGGAGT AAACCCTGGA ATGAGGCCCA 150

GGGCTTCTAT TGCTAATGCC TGTAAACCTT GCTGAATCTA TAACAGAGTC 200

TATACCGCTC TCG 213

(2) INFORMATION FOR SEQ ID :879:

(i) SEQUENCE CHARACTERISTICS:
(A) LENGTH: 227 base pairs
(B) TYPE: nucleic acid
(C) STRANDEDNESS: double
(D) TOPOLOGY: linear

(xi) SEQUENCE DESCRIPTION: SEQ ID :879:

AAAGAAGGAA AAGAAAGAAA TAACACTTAA CAGTCCATAA CAAAGTGTTA 50

ACGAGATAGA CACATGCTTA TTCAAACCAC AGATATGATC CAGTTAATTC 100

CCTTCTTAGA ATGTGCCCAT AGTGCCTTAT TGCCTCATAA TAATGATAAT 150

AATAAGAGCA ACAATAATAA CGGCACAAAC TCCAGTCGAT ACTGACAAAC 200

TACCAGAGTA ACCGTCATCC CCTTGAG 227

(2) INFORMATION FOR SEQ ID :880:

(i) SEQUENCE CHARACTERISTICS:
(A) LENGTH: 221 base pairs
(B) TYPE: nucleic acid
(C) STRANDEDNESS: double
(D) TOPOLOGY: linear

(xi) SEQUENCE DESCRIPTION: SEQ ID :880:

ATAGACAAAG TCCTTCCCAC TAGAACTTAC ATTCCAGTGG GAAGAATTAG 50

AAGCCTCAGG AATTCCATTG CTTACTTTTA GTTGTTACTT CAAAAGTACT 100

TACATTTAAT CTGATTATTA ATTATTCGTC ATGAGCTTCA TTTTATTACA 150

TCCAGGGCAC AGTATGTGAA TTGTGTTTCG TTCCTTTAGG AAAAGGAAAA 200

ATAATCACTC TTTACAAGGT A 221

(2) INFORMATION FOR SEQ ID :881:

(i) SEQUENCE CHARACTERISTICS:
(A) LENGTH: 310 base pairs
(B) TYPE: nucleic acid
(C) STRANDEDNESS: double
(D) TOPOLOGY: linear

(xi) SEQUENCE DESCRIPTION: SEQ ID :881:

AATTGTGGTA TATTTCATTT AAAATCGATC AAAGACAGCA ACATACATTA 50

ATCAGAGGAA AGCATCAATT AAATGCAGAA ACAAGATTTA AACAAAGATT 100

CATATTAAAA CAATGATAAG TAATACTTAG AAACTGCATC CTAGAGACAC 150

ATCATTCGCA TTTTTAGAAA AACAGAAACA TATTAGTGTG AAAAGATGTT 200

AAAAAATGAA TATTAAACCG TCGAGCACAA CACACTACGC CGATAACAAT 250

ACACCACAAG CGCCGTAATC CTGCCGACAC TGACACACTA CAGGCGTCGT 300

GGCGATCCGA 310

(2) INFORMATION FOR SEQ ID :882:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :882:

GTTCACATCA GTGTGTAGGA TATGCTATCA CGTATGTGCA TGAGAAGAAT 50

GGAGAAAATG GATCTATGCG GATGTCTGTA CACACACACA CACACACATA 100

CACAACAAGC TTGCACATGT ATAGAGGATC CGAAATTCCA CCAATACTGA 150

CGAACTACAA ACGTAACAGC AGCAGGC 177

(2) INFORMATION FOR SEQ ID :883:

(i) SEQUENCE CHARACTERISTICS:
(A) LENGTH: 152 base pairs
(B) TYPE: nucleic acid
(C) STRANDEDNESS: double
(D) TOPOLOGY: linear

(xi) SEQUENCE DESCRIPTION: SEQ ID :883:

CAATTCTATT TTCAGAGTTG ACTGTACTCT TTTCCTCTGA AGATACACGT 50

ATAAACGCGA CCAACGAGGT CTCTCGTCCT AATAACAGGT TCAGGCATGA 100

ATCACCGTCT AACAGCTATT TAAACACCGC CTCTTCATAG AAGTAACGCC 150

GA 152

(2) INFORMATION FOR SEQ ID :884:

(i) SEQUENCE CHARACTERISTICS:

(A) LENGTH: 177 base pairs

(B) TYPE: nucleic acid

(C) STRANDEDNESS: double

(D) TOPOLOGY: linear

(xi) SEQUENCE DESCRIPTION: SEQ ID :884:

CCATAATATA AGTATTTATC ATGTATTCTG TGTCTGGAAT ACAATTCAAT 50

GACCTATTTC AGAAAAATCT TTTCAAGTAC TGGCTGAAAG AGTGAGGGAT 100

ACATTAGGAG ACTGCAAATA AACTGGCAAT CACAAGAACT TTTTCGGATA 150

AAATGAAATT GTGCCGAAAT GTATACA 177

(2) INFORMATION FOR SEQ ID :885:

(i) SEQUENCE CHARACTERISTICS:

(A) LENGTH: 241 base pairs

(B) TYPE: nucleic acid

(C) STRANDEDNESS: double

(D) TOPOLOGY: linear

(xi) SEQUENCE DESCRIPTION: SEQ ID :885:

CACAGCTGGA AGAGCGGAGA AATAGAATTG TCTTCAAACA ATATAAAAAT 50

TCATAAAGAA TAGAAAGACT ACTATGTGTG GGTCGGGAAA TTAACTAGAA 100

TTTTTTTATA TCCACACTCT AGTATGAAAT GAGTACTTAT AGAGTAGAGT 150

GTAACATATT TAGACATAGT ATATATATAT ACAAGTGTAA CAAATATATA 200

TATTAAATAT ATATATGTTT TATAGAAACA CAGAGCACAC A 241

(2) INFORMATION FOR SEQ ID :886:

(i) SEQUENCE CHARACTERISTICS:
(A) LENGTH: 190 base pairs
(B) TYPE: nucleic acid
(C) STRANDEDNESS: double
(D) TOPOLOGY: linear

(xi) SEQUENCE DESCRIPTION: SEQ ID :886:

GTAGCAACCG GCGCTCAATA AAATTCAACC AGCAAACTCG ACAAGTAACA 50

ACCATAAAAC CAGCCACACT AAGTCCAGCC ACCACTACTC AATAAAAATA 100

ACACGTATCT TCCACCGTAC AACCAATAGC AACTGCAGGC TACTGCAACG 150

CCATCCATAT ACCAAAAATG CTACTTACAA CACCACAACA 190

(2) INFORMATION FOR SEQ ID :887:

(i) SEQUENCE CHARACTERISTICS:
(A) LENGTH: 168 base pairs
(B) TYPE: nucleic acid
(C) STRANDEDNESS: double
(D) TOPOLOGY: linear

(xi) SEQUENCE DESCRIPTION: SEQ ID :887:

CACGCTTTCT CTCACACAGC ACTAGCGCTC TCTCACTATC ACTCTTCTCA 50

CAGAGCTGCG CGCGAGTCTC ACTACTATCA AATATATCTG TCTCTTCTCA 100

CTCTATAGCT CTCTCACAGA TATATATCTA CATATAGCGC TCTCATTATA 150

CTATAATATT ATACTCTC 168

(2) INFORMATION FOR SEQ ID :888:

(i) SEQUENCE CHARACTERISTICS:
(A) LENGTH: 174 base pairs
(B) TYPE: nucleic acid
(C) STRANDEDNESS: double
(D) TOPOLOGY: linear

(xi) SEQUENCE DESCRIPTION: SEQ ID :888:

ACTGGGCGCG CTGGTGCTGA CATCTTCAAG AGCATCTGTC ACTGAATATT 50

GCCGATCTCG CAACCGGTTC CAGTTAGACA GAACATTGTG ATATTCAAAC 100

CACTTTCTCG TAATTTCCAA TGGAGTTGTA AAGTTTAATG AGACCTCGAT 150

AATCATATTC TAGTCCACTG TAGC 174

(2) INFORMATION FOR SEQ ID :889:

(i) SEQUENCE CHARACTERISTICS:
(A) LENGTH: 327 base pairs
(B) TYPE: nucleic acid
(C) STRANDEDNESS: double
(D) TOPOLOGY: linear

(xi) SEQUENCE DESCRIPTION: SEQ ID :889:

ACACCACACT TAAGGAATGG GAGTTATATT TCACTTCCCT GAGGCCAGAA 50

TATATATATG TATGTATGTA TATGTGTGTG TGTGTCTATA TATATATATA 100

TATAGACACA CACGCACATT ATACACACAT ATATATACAC ACACATATAT 150

ATATGTGTGT GTACAATATA TAAAAAATTA TATGGGAGAA AAGAAAGGCA 200

AATCTCCCAT GGCAGAGAGG TATCCCAAAA AATTTTTTTG TGTGTAACAA 250

AATATGTTGT GTGTATATAT ATATGCACAC ATATTTATGT GTATATATGT 300

ATATATATAT CTGTATATAC AGGTATA 327

(2) INFORMATION FOR SEQ ID :890:

(i) SEQUENCE CHARACTERISTICS:

(A) LENGTH: 181 base pairs
(B) TYPE: nucleic acid
(C) STRANDEDNESS: double
(D) TOPOLOGY: linear

(xi) SEQUENCE DESCRIPTION: SEQ ID :890:

TTTTTTTCTT GTCTCTCCCG CAGCGCGCGC CTCTCGCGGC GCTATCTCTC 50

GCTCTCTCTC GCTCTCTCGA GCTCTCGAGA TATAGCCGAG ACACGAGAGC 100

TCGCTCTCTC CCGCGCGAAG ATCTCACCCC CGCGCGCTCG CGCGATATAT 150

ATCTCCTCGC GCGCGCGCCG AGCGCGCCCC T 181

(2) INFORMATION FOR SEQ ID :891:

(i) SEQUENCE CHARACTERISTICS:
(A) LENGTH: 207 base pairs
(B) TYPE: nucleic acid
(C) STRANDEDNESS: double
(D) TOPOLOGY: linear

(xi) SEQUENCE DESCRIPTION: SEQ ID :891:

CTCTTTAATA GCAAGCGAAT GGTAATTACA TGGTCGGATG AGGTCCTCAC 50

TCTCAGGGGA GGGAGGAGGG AGCAGAGGTG GACAGGGTGC AGTATAGGAT 100

TTACACTGTT TGAAGCATCT AACGAAGGGC AACAGTTTTT GGCAACCCAA 150

TTCACAGTTT TGTAATTTAC AAGAGATTTC TTTGAAAGGA AATAGGAAGG 200

CAAAGAA 207

(2) INFORMATION FOR SEQ ID :892:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :892:

CTATCACTTC AGGGAAACAA ACAACTAACA GCCATCAATT CAGAGGGAAG 50

TGATTTTACA GTAGAGTGAA CGAAACTTGG GAAGGAAAAC ATCCAAGAGG 100

CGTCTGTTTG ACGTGGGTAA CGTGGGGAAC GCATACTGTC TGGCAAGAAT 150

TCTACTAGGA CCACGGGAAA CAAAGCAGAT TAAAACACTC TCTACTCT 198

(2) INFORMATION FOR SEQ ID :893:

(i) SEQUENCE CHARACTERISTICS:
(A) LENGTH: 96 base pairs
(B) TYPE: nucleic acid
(C) STRANDEDNESS: double
(D) TOPOLOGY: linear

(xi) SEQUENCE DESCRIPTION: SEQ ID :893:

CTTTAGAGAA TGCCTTGTGG AAAAAAAAAA AAATGGGCCC CAATACTTTT 50

TACTGCCCTT TATCAAAATT GTTGTGCATG GACCGGGCCA AATAAG 96

(2) INFORMATION FOR SEQ ID :894:

(i) SEQUENCE CHARACTERISTICS:

(A) LENGTH: 325 base pairs

(B) TYPE: nucleic acid

(C) STRANDEDNESS: double

(D) TOPOLOGY: linear

(xi) SEQUENCE DESCRIPTION: SEQ ID :894:

AATTGTATAC CAACATATAA ATTAAAAGTG TTATGCAATG AGAAACAATA 50

ATGGAAACAG CATAATACTA CATACTATCG CGCGGGGTTG TCGGCGTGGT 100

GGGCGTGCGT GTAGAGAGAG AGCAAGGGCG TGTGCGTGAG TGGGCGTAAA 150

GAGTGAGCGT GGGAGGGTGT GGTCGGTGGA GGTGTGTGGA GAGGTGAGTG 200

TGCGAGAGGG GCGAGTGTAT GTGTGATAAG TATAGCGCGC AAGAGGCGAG 250

GACAAAATAT ATATATATAT AGATATAATA GATATGAGAG AACACTAAAC 300

AATAACCACT ACTCACATAG AATAT 325

(2) INFORMATION FOR SEQ ID :895:

(i) SEQUENCE CHARACTERISTICS:

(A) LENGTH: 266 base pairs

(B) TYPE: nucleic acid
(C) STRANDEDNESS: double
(D) TOPOLOGY: linear

(xi) SEQUENCE DESCRIPTION: SEQ ID :895:

ATAGTTGTAC ATTTTTATGG GGTGCATGTG ATATTTTGAT ATGTGCATAC 50

AATGTGTAGC AATCAAATCA GGGTAATTGG GATATTCATC ACCTCAAACA 100

TTTATCATTT ATTTGTGTTG GAAACATTCA AACCTTTTCT TCTAGCTATT 150

TATCCATTGT TGGATACTTA TATCAATTCT ATATCTTAGC TGTTGTGAAT 200

AGAGCTGCAA TAAATGTAGG AGTGCAGATA TCTCTTTGAT ATACTGATTT 250

TCTTTCTTTT GTTATA 266

(2) INFORMATION FOR SEQ ID :896:

(i) SEQUENCE CHARACTERISTICS:
(A) LENGTH: 197 base pairs
(B) TYPE: nucleic acid
(C) STRANDEDNESS: double
(D) TOPOLOGY: linear

(xi) SEQUENCE DESCRIPTION: SEQ ID :896:

AAGAAGAGTC TTCTGCACAA ACAAAACCAT AGTGTTGACA AATAGTCATG 50

GCCAATGGCA ATCTGATGGT CCAGCGGCCC TCGGATGACT CCTCTGCAAG 100

GAGCATCTTC AGGGTTCTAG GGAAGTCACA GGGGCAACAT ATTGGAAACT 150

AGCAGGCCCA GAAGACCGCC CCGCCCCCCA TGCCCTGGCG CAGGGCC 197

(2) INFORMATION FOR SEQ ID :897:

(i) SEQUENCE CHARACTERISTICS:
(A) LENGTH: 192 base pairs
(B) TYPE: nucleic acid
(C) STRANDEDNESS: double
(D) TOPOLOGY: linear

(xi) SEQUENCE DESCRIPTION: SEQ ID :897:

GATGGCTGGA GTAGATTAGG GCGTAGGTAG AAGTAGAGGT TAAGGAGGGT 50

GATGGTGGAC TATGATGGTG GGGATGATGA GCGCTATTGT TTTTTGTGAA 100

TATTTTTCTT CCGACTAACT CGCGCCCCAC TCTCTTGCGA GCACAAACAC 150

ACGCGCGCGT GTAAGCGGCG CGAGACACGC GCCCCTTCCC CT 192

(2) INFORMATION FOR SEQ ID :898:

(i) SEQUENCE CHARACTERISTICS:
(A) LENGTH: 224 base pairs
(B) TYPE: nucleic acid
(C) STRANDEDNESS: double
(D) TOPOLOGY: linear

(xi) SEQUENCE DESCRIPTION: SEQ ID :898:

TGGATGTTTT CATTCGCAAA TCATGAGAAA CTTAAGTGGG TTTTATGCAC 50

TTGATAGAGT TGGCAAAATT GAACTATGAA GTTAACTATT TAACTCAAGG 100

AATGGGCGGC AAACCCATCC CCTCGATTGA TAAAGAAGGG GAACATTTTT 150

ACATTAGAAC TGACACTGAA AACATAGCTC TTTTTCAGTC CACCCTGGTT 200

GCTCTAGTAG CCCACAGCCC AATC 224

(2) INFORMATION FOR SEQ ID :899:

(i) SEQUENCE CHARACTERISTICS:
(A) LENGTH: 362 base pairs
(B) TYPE: nucleic acid
(C) STRANDEDNESS: double
(D) TOPOLOGY: linear

(xi) SEQUENCE DESCRIPTION: SEQ ID :899:

GAGGTGATTT CTCAGAGTTT AGAGAATAGG ATGGGGAAAA TTATATTTAG 50

TGAGTTATAA CCAGAATTAC ATAAGACAGA TATGGAAATT TTATAAACAA 100

AATGCAAAAT ATTCTAATGT TTCAATGTTC TACATGAACA TATAGGGAAG 150

CATAGACAAT AGCCAAAAAT ATGTTCTGCA TTCATATACT AGTTCAAGTC 200

CGAGTCTGGC TACTTTCTAG GTAGTGTGCT TTTTGTCAAA TTATAAAGAT 250

ATATTCCCTT TGTTTTTTGA AAACGAGTGA GATGCTTAAA TAGAGTACAA 300

TTATCTCATT CAAAATGTAT GTTGTTTCCT CTCGAGAATT GTGAAGGTTC 350

TGAGATTTGA TT 362

(2) INFORMATION FOR SEQ ID :900:

(i) SEQUENCE CHARACTERISTICS:
(A) LENGTH: 310 base pairs
(B) TYPE: nucleic acid
(C) STRANDEDNESS: double
(D) TOPOLOGY: linear

(xi) SEQUENCE DESCRIPTION: SEQ ID :900:

GTATGTAGCC CAGTGGGTGT CTTCCCACAG GGTAGGTACT CAGTTTGCTC 50

TGGAGGGTGA CTCATACCTA AACAAGTGCA CATCTCCTTT CTCAGTAAAG 100

CCAAAGCAGG TTTCTACATT TGGAACAAAA GAGATCCTGA CCAGAGAGCT 150

ATCACTGGTG GTCCACTTGG GCCCTCCTTG ATGGGTGTGT TCACTTAGAA 200

AACCAAATTA CAGATCTGAA GGCTGCTGGG TAGGGACAGG ATTAGAACAA 250

AGGGAATGAG ATTGAATGTT ATTTAAGGGA TATTTCTGTC AAGTTTTGGT 300

TTCTCGATGA 310

(2) INFORMATION FOR SEQ ID :901:

(i) SEQUENCE CHARACTERISTICS:
(A) LENGTH: 185 base pairs

(B) TYPE: nucleic acid
(C) STRANDEDNESS: double
(D) TOPOLOGY: linear

(xi) SEQUENCE DESCRIPTION: SEQ ID :901:

TTCCCCCATG AGACCGTTAG TCTCTCTTTG CCTGGCTGAC TACCTGCATA 50

CAGTAGGCAC TCACTGCTGG AGTGAGGCAC TGACTCCTCC AAAGATTGCA 100

GGGGGCGGAG GAGGGAACCA CGAAGGCCTG GGAGGGGGCA TCTTTGGCCC 150

CCACTAACCA TCTCCCTATT TCTGCATCCT GGTGA 185

(2) INFORMATION FOR SEQ ID :902:

(i) SEQUENCE CHARACTERISTICS:
(A) LENGTH: 381 base pairs
(B) TYPE: nucleic acid
(C) STRANDEDNESS: double
(D) TOPOLOGY: linear

(xi) SEQUENCE DESCRIPTION: SEQ ID :902:

ACAAGCTGCA AGATCCTTCA CTTGAGGCTT TCAGCCTTAT TCTCCTCCTG 50

TCAAACAACT AAACTACTCC GATGTTTGAT GAAAATTAAA CTGCTACTCA 100

GGATACTGCA ATTACAAGGA GAGGGAATGA TCAGCCCAGG GAGGCTATTA 150

CGTGTGACCT TTGAGATGGA CCTGATCGCC CCTTTTACTT TTTAGACTAC 200

AAGTGCAGGG AGGTGGAGCT TATTTGCATT TGAACTCCTG TAAAGAGTAA 250

GAATATGGAA AGGATGAAGC CTCATTCATT CGGGCATATT AAAAAGAAAT 300

TGCCTTCAGA AACACTTTGC CTTTTAATAT GTGTAGCTAC AGTAAGTACC 350

AATGGGCTAA CTAATTGAAG CTAACATTTT A 381

(2) INFORMATION FOR SEQ ID :903:

(i) SEQUENCE CHARACTERISTICS:

(A) LENGTH: 240 base pairs

(B) TYPE: nucleic acid

(C) STRANDEDNESS: double

(D) TOPOLOGY: linear

(xi) SEQUENCE DESCRIPTION: SEQ ID :903:

CATCAAGGGT TTTTCCTGCT GCAATTCTTG TCAAAAACTG ACATATGTAT 50

ATCGTTCTCA ACTGGCAAGC TGTTAGACTG GATAGTCCAT GAATAATAGC 100

CTCTGCGCTG TTGCGGGTCC TGCGGAAGTC CTCGGAGCGG CCGTCGCGGA 150

AAGCTCGGCA AAGAGAGAGG CAGAGGAAAT CGAGCATCCA GCCGGCAGCC 200

ACTTTTTTTT TATCGGCACC AGGCCGCGTC CTCCTCCTCC 240

(2) INFORMATION FOR SEQ ID :904:

(i) SEQUENCE CHARACTERISTICS:

(A) LENGTH: 280 base pairs

(B) TYPE: nucleic acid
(C) STRANDEDNESS: double
(D) TOPOLOGY: linear

(xi) SEQUENCE DESCRIPTION: SEQ ID :904:

CCCTGGGCAG GCTGCTGGTG GTCTACCCTC GGACCCAGAG GTTCTTTGAG 50

TCCTTTTGGG GATCTGTCCA CTCCTGATGC TGTTATGGGC AACCCTAAGG 100

TGAAGGCCTC ATGGCAAGAA AGTGCTCGGT GGCCTTTAGT GATGGCCTGG 150

CTCACCTGGA CAACCTCGAA GGGCACCTTT GCCACACTGA GTGAGCTGCA 200

CTGTGACAAG CTGCACGTGG ATCCTGAGAA CTTCAGGCTC CTGGGCAACG 250

TGCTGGTCTG TGTGCTGGCC CATCACTTTG 280

(2) INFORMATION FOR SEQ ID :905:

(i) SEQUENCE CHARACTERISTICS:
(A) LENGTH: 225 base pairs
(B) TYPE: nucleic acid
(C) STRANDEDNESS: double
(D) TOPOLOGY: linear

(xi) SEQUENCE DESCRIPTION: SEQ ID :905:

GTTCTAGTGG TAACTGCTGC TTCTGGAAAA TATTTAGAGA AACCAACGGT 50

AAAAAAAAAA ATAATAATTA ATACCGTTGG TTTCTACATA CACTCTCAAT 100

ATTTGCACGA GTAAAGCGTA GCAAGTTTAA CACAACTTAT GTAAACTTGG 150

AAAATTTTCC GAAATTTTAT TGACTTTTCT CGGTCTCTCC TATCTTTATA 200

TACACATCTC TCATGCACAC ACGCG 225

(2) INFORMATION FOR SEQ ID :906:

(i) SEQUENCE CHARACTERISTICS:
(A) LENGTH: 161 base pairs
(B) TYPE: nucleic acid
(C) STRANDEDNESS: double
(D) TOPOLOGY: linear

(xi) SEQUENCE DESCRIPTION: SEQ ID :906:

GGTGCTGACA TCTTCAAGAG CATCTGTCAC TGAATATTGC CGATCTCGCA 50

ACCGGTTCCA GTTAGACAGA ACATTGTGAT ATTCAAACAC TTTCTCGTAA 100

TTTCCAATGG AGTTGTAAAG TTTAATGAGA CCTCGATAAT CATATTCTAG 150

TCCACTGTAG C 161

(2) INFORMATION FOR SEQ ID :907:

(i) SEQUENCE CHARACTERISTICS:
(A) LENGTH: 204 base pairs
(B) TYPE: nucleic acid
(C) STRANDEDNESS: double
(D) TOPOLOGY: linear

(xi) SEQUENCE DESCRIPTION: SEQ ID :907:

AGAGATATAT GCAATCTGGT AGTCCATTAA ATGAGAGTTA CACTTAAAAT 50

ATTTTAGTTC TTTTAAAGAA AAAGTCTCCA TGTGCTATTT GGGAAAACCT 100

TCATTGCCTA AAGACCCACT TGCATAATTA AGGCAGATGA TGATGATCTT 150

TATATATGCG CACACACACA CACACACGAC ACGACGACAC ACACACACAC 200

TCTA 204

(2) INFORMATION FOR SEQ ID :908:

(i) SEQUENCE CHARACTERISTICS:
(A) LENGTH: 316 base pairs
(B) TYPE: nucleic acid
(C) STRANDEDNESS: double
(D) TOPOLOGY: linear

(xi) SEQUENCE DESCRIPTION: SEQ ID :908:

AGTTTCTGCA TTTGGTCTGT GTGACTCTAT GGAGCCCAGG ACTTACAGAA 50

AAAGGTGTAA CTGGCTCTTA GGACTTTAAG CCACATTATG GTAGTAGACC 100

TGCCGTTTTA TTTGACATGG TGAAATAAAC TACCACTCCT TTGTGGTACC 150

ATATGGGCAG GACAGAGCTC TTCAACCCTG GCTAACAAGC CTGAAGAAGA 200

CCCTGTGAGG CCGCAGCAGC CATGGGAACT TCCCAAAGGA CAGAAATCCA 250

CATTGGAACG TAGCATCCAG ATAGGTGACA GAAATTCCTT CCACCATATC 300

CAAATGTGTG TGTTTT 316

(2) INFORMATION FOR SEQ ID :909:

(i) SEQUENCE CHARACTERISTICS:
(A) LENGTH: 307 base pairs
(B) TYPE: nucleic acid
(C) STRANDEDNESS: double
(D) TOPOLOGY: linear

(xi) SEQUENCE DESCRIPTION: SEQ ID :909:

CTAGTAACAT AGAAAATAAA CTTCAGTGGG AATCTCTGCT TCCCGCGTGA 50

GGTGTTTAAT TCTTGGCATT TTTGTATTTT AAAGATGTAG CAACTTGTTT 100

CAAGTTAGAG GAGATGGCAG GGTCAAAATT TTAGAAACTG GATCCCACCA 150

CCACTGTGTT ACTTCCTAAA CCTGCATACA AATGTTCTGC CAACATGTAA 200

TGTGCCAATA GAATTATACG GTGTGAACTG CATATCTCAG TATCTCCACG 250

GGAAAAAACT GTGGTTGGGG CATGGAGGGG GGAAAGGGAA ACTTTTTTAA 300

GCTATTT 307

(2) INFORMATION FOR SEQ ID :910:

(i) SEQUENCE CHARACTERISTICS:
(A) LENGTH: 172 base pairs

(B) TYPE: nucleic acid
(C) STRANDEDNESS: double
(D) TOPOLOGY: linear

(xi) SEQUENCE DESCRIPTION: SEQ ID :910:

TTTTTTTTTT TTTCCAGGCA CCTAACGATT TGTTTTGCTC AATCAAACGC 50

AGACAGGCGT CTCCGAAGTA CCACCACTGG GATATCCTCG GACCAGCGCT 100

TAAACCGAAT CCCCACAATC TCAAACTCAA CCAGGCCAAA GGGAACACAG 150

TGACACAACA ACAGGGTTCC AA 172

(2) INFORMATION FOR SEQ ID :911:

(i) SEQUENCE CHARACTERISTICS:
(A) LENGTH: 255 base pairs
(B) TYPE: nucleic acid
(C) STRANDEDNESS: double
(D) TOPOLOGY: linear

(xi) SEQUENCE DESCRIPTION: SEQ ID :911:

GCATAGAGAG ATATATACAC AGAGAGGACA TACATATATA TATATAGAGA 50

CACAGAGACA GACATATAGA GATATATATA TACACAGCAT AGAGATACAG 100

AGAGATAGAG AGAGATACAC ATACACACAT TCATCAACGA GAGAGAAGAG 150

AGGAAAGAGA GAGAGAGCGA GCACAGACAG AGATAGAGCA CACAGAGAGA 200

TCGCGCAGAT CTATACAGAG GGATATTCAC CACATTGTAT ATGGAAAGCG 250

CATCT 255

(2) INFORMATION FOR SEQ ID :912:

(i) SEQUENCE CHARACTERISTICS:
(A) LENGTH: 196 base pairs
(B) TYPE: nucleic acid
(C) STRANDEDNESS: double
(D) TOPOLOGY: linear

(xi) SEQUENCE DESCRIPTION: SEQ ID :912:

CAAGCGAATG GTAATTACAT GGTCGGATGA GGTCCTCACT CTCAGGGGAG 50

GGAGGAGGGA GCAGAGGTGG ACAGGGCGCA GTATAGGATT TACACTGTTT 100

GAAGCATCTA ACGAAGGGCA ACAGTTTTTG GCAACCCAAT TCACAGTTTT 150

GCAATTTACA AGAGATTTCT TTGAAAGAAA ATAGGAAGGC AAAGAA 196

(2) INFORMATION FOR SEQ ID :913:

(i) SEQUENCE CHARACTERISTICS:
(A) LENGTH: 203 base pairs
(B) TYPE: nucleic acid
(C) STRANDEDNESS: double
(D) TOPOLOGY: linear

(xi) SEQUENCE DESCRIPTION: SEQ ID :913:

ACTATCACTT AAGGGAAACA AACAACTAAC AGCCATCAAT CCAGAGGAAA 50

GCGATTTTAC AGTAGAGTGA ACGAAACTNG AGAAGGAAAA CATCCAAGAG 100

GCATCTGTTT GACGCGAGCA ACGCAAGGAA CACATACCGT CTGGCAAGAA 150

TCCCACTAGG GCCACGAGAA ACAAAGCAAA TTAAAACATT ATCCACTACA 200

CGA 203

(2) INFORMATION FOR SEQ ID :914:

(i) SEQUENCE CHARACTERISTICS:
(A) LENGTH: 262 base pairs
(B) TYPE: nucleic acid
(C) STRANDEDNESS: double
(D) TOPOLOGY: linear

(xi) SEQUENCE DESCRIPTION: SEQ ID :914:

CTGCCGAGCC ACCATGCTTG CCTGAAGCTT CGGCCGCGCC ACCCGGGCAA 50

GGGTCCTCTT TTCCTGGCAG CTGCTGTGGG TGGGGCCCAG ACACCAGCCT 100

AGCCTGGCTC TGCCCCGCAG AACGGTCTGT GTGCTGTTTG AAAATAAATC 150

TTAGTGTTCA AAACAAAATG AAACAAAAAA AAAATGATAA AAAAATCGGA 200

TTTCCGGTTA ACACTGTGAC TTCAATTGAA ACACCTTTTT GTAGTATCTG 250

GAGGTGGACA TT 262

(2) INFORMATION FOR SEQ ID :915:

(i) SEQUENCE CHARACTERISTICS:
(A) LENGTH: 191 base pairs
(B) TYPE: nucleic acid
(C) STRANDEDNESS: double
(D) TOPOLOGY: linear

(xi) SEQUENCE DESCRIPTION: SEQ ID :915:

TGGGTTCTAA TGATGTGCTT GAAGAGGAAG ATTTTGATCG CACCACTGAT 50

ACACGGAGTC TGTATTTTGC ATCCTAGAAT GATTAACCAG AAACAAAGAA 100

AGGAAAAAGG TAAAATCAAT AATAAGATCA GCAGCTCTGG GACACAGAAT 150

AAAGAATGAA ATAATTTGAA AAAAAAGGCA GGGCAGGCAA A 191

(2) INFORMATION FOR SEQ ID :916:

(i) SEQUENCE CHARACTERISTICS:
(A) LENGTH: 252 base pairs
(B) TYPE: nucleic acid
(C) STRANDEDNESS: double
(D) TOPOLOGY: linear

(xi) SEQUENCE DESCRIPTION: SEQ ID :916:

AGACTTGGGG GTTCCTGGTG TGTGAGGGGT CCTGGGATAG CTAGGGGTTT 50

TCCAGGAGTT GTCCTTTGTT GTTTGTCGTG AAGAATTATA CTTTGTGTGT 100

GTGTGGGTTG TCCCATGTGT ATGCGTGTAG TGTGACAGTA TATGACCCCC 150

AGTGTGTGCA TATGTTTGTG TGGTTGTCTC TGAGTGATAT CCACCTCTCT 200

CTCCCCCTCT GTCTCTCTGC AGAGATATGT GTGGCGTCAC TGTACTCTTG 250

TG 252

(2) INFORMATION FOR SEQ ID :917:

(i) SEQUENCE CHARACTERISTICS:
(A) LENGTH: 247 base pairs
(B) TYPE: nucleic acid
(C) STRANDEDNESS: double
(D) TOPOLOGY: linear

(xi) SEQUENCE DESCRIPTION: SEQ ID :917:

TAGACAGAGT GACAGTGAGA GAAAAAGAGA GGAAGGAAAA GGGAAAAGGA 50

GTAGGGAAGG ATGAGGGAGA TAGGAAAGCA CCTACAGGAG GCAGAGAGTT 100

TCCTGAAGGT CATGCGATCC TTTACCCTCA GAAACTTTCA GGCTGAAATC 150

ACCATCTCCG TTATCCCAAC AAACGCACTC TCCTACCTCT ACAAGCACTT 200

CAAGTAAAGC AGAAATCACA CCAATACTGA CGNGCTTCAA GCGCAAC 247

(2) INFORMATION FOR SEQ ID :918:

(i) SEQUENCE CHARACTERISTICS:

(A) LENGTH: 191 base pairs

(B) TYPE: nucleic acid

(C) STRANDEDNESS: double

(D) TOPOLOGY: linear

(xi) SEQUENCE DESCRIPTION: SEQ ID :918:

ATTTGGATTC AATTGCCTGT TGCACTTTTA CATTAAGTGT TGCTTAAATA 50

AACAAAAATA GAGCATAAAT TCAATATTCT ACTGTCTAAA CATTTTAAAG 100

CAATGGTTAT GCCATCATAA ACAAGTAAAA TGCACCTAAT TCGAGTCTTT 150

TGACACCTCT TGTTTTAAGT TTCCTGTATG ATAAAGTTCT T 191

(2) INFORMATION FOR SEQ ID :919:

(i) SEQUENCE CHARACTERISTICS:

(A) LENGTH: 205 base pairs

(B) TYPE: nucleic acid

(C) STRANDEDNESS: double

(D) TOPOLOGY: linear

(xi) SEQUENCE DESCRIPTION: SEQ ID :919:

CAAGAGTGAG ACTCCATCTC AACCAAAAAA AAAAAGAAAG AAAAAAAGAA 50

ACAACCTCCT CATTTTCAGA AGCGAACACA CCCCCGCCCC AAGATAGACG 100

ACAGAGACCG GACTAAGATT TAACCCAGGA ACTTTTCGCT TGGGCACCTT 150

CGAACAACAC ACAACCCGCA AAACCATACA CGCTCCCTTG CCCGAGGCCC 200

AACCC 205

(2) INFORMATION FOR SEQ ID :920:

(i) SEQUENCE CHARACTERISTICS:
(A) LENGTH: 323 base pairs
(B) TYPE: nucleic acid
(C) STRANDEDNESS: double
(D) TOPOLOGY: linear

(xi) SEQUENCE DESCRIPTION: SEQ ID :920:

GACAAAATTG GGACTCTAAG TATTGCGCTT TTTTTTCCTC TCCAACGACT 50

CGTTCCCTTT CAACAAATAG CACTTCAGTC CACTGTGTAC AGAAGTAATA 100

CATCTCCTAT CGTTAGTTGA ATAAATTCAC ACACCGCGTT TAAGAGATGA 150

AACCACGACT TCGTCAAGCC ACATTCGATT GCCATAAGAT TCCAAAGAAT 200

TCTATCCTCT TAAAGCGAAC CACATGTAAC CCGAAGGTCC CCACCTATCA 250

AGGTTAGAAC TGATATTCAA ATTATAGACT CATTCCACTA AGCAAGTAAG 300

CTCTAAGCTA CAGGTTGATC ATA 323

(2) INFORMATION FOR SEQ ID :921:

(i) SEQUENCE CHARACTERISTICS:
(A) LENGTH: 230 base pairs
(B) TYPE: nucleic acid

(C) STRANDEDNESS: double
(D) TOPOLOGY: linear

(xi) SEQUENCE DESCRIPTION: SEQ ID :921:

AACATTGACG ACGCTAAGGA TGCCATGATG CCATGAATGG GAAGTCTGCA 50

GATGGACGGC AGATCCGAGT AGACCAGGCA GGCAAGTCGT CAGACAACCG 100

ATCCCGTGGG TACCGTGGCG ACTCTGCTGG GGGTCGGGGC TTCTTCCGTG 150

GGGACCGAGG CCACGGCCGT GGACTCTCTA GAGGCGGCGG GGATCAAAAC 200

TATAGAAACA ACAAGTTAGA GTACAGACGT 230

(2) INFORMATION FOR SEQ ID :922:

(i) SEQUENCE CHARACTERISTICS:
(A) LENGTH: 239 base pairs
(B) TYPE: nucleic acid
(C) STRANDEDNESS: double
(D) TOPOLOGY: linear

(xi) SEQUENCE DESCRIPTION: SEQ ID :922:

TATTTTTCCT GATGTTCTCC CTTCCTCCAG TCCCCGACCT CCCACAGGCC 50

CCAGTGTGTG TTGTTCCCGT TCCTCGTGTC CGTGAGTTCT CATTGTTCAG 100

TGCCCATTCT GAGGGAAAAC ATGCCAGTGC TTTCGCGTTC TTCCTCGCTC 150

TCACCCACCT CTCAGCTTAT CGCGTCGCGC GCGCAGTATA TTCGTCTCTC 200

TCTCCAGTCT CTCTCGCGAC ACACATCTCT CTTTTGTAA 239

(2) INFORMATION FOR SEQ ID :923:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :923:

GTCAGTTCAG TTGTGAACAA AGCCCTTTGT GTACGTTTTT AAATTTTAGT 50

TCACACGATA ACAATGCTTT GCAAGTAGGT GTAATTATTT TACTCCCATT 100

TTATGGATGA GAAAACAACA GAGAGGTTGA TTGGCCCCAC CTTCAAATCC 150

TGAAACCCGT CCACTCAATA AATTTTTG 178

(2) INFORMATION FOR SEQ ID :924:

(i) SEQUENCE CHARACTERISTICS:
(A) LENGTH: 325 base pairs
(B) TYPE: nucleic acid
(C) STRANDEDNESS: double
(D) TOPOLOGY: linear

(xi) SEQUENCE DESCRIPTION: SEQ ID :924:

CAGCTGGTCC CCCACTAAAA GAAGGGGCAG ACCACCACTA GCCAAAAGAG 50

ATAGCAAGAC TAACATCTGG AAGAAGCGAG AGGAACGCCC ACTGATTCCC 100

AAAAAAAAAC TCCAATGGGG ATTGTGTGTC TGCTGTCTCG TGCTGTTTAT 150

TCTTGCTTCT TGTTGTAAAT TGCAGTACGA ACTTAAGAAA ATGAGACTGA 200

GCAATCTCAT GGTTCTTGGA CATGTCTCAA GCAGAGTAAA TGGTAATTCA 250

GTAATCAGAG AGAAAGATAC CAAGGAATGC TTTTTCTGGC CTATTCATTT 300

ATTTTTGGGG GATGAATTTA CAGTA 325

(2) INFORMATION FOR SEQ ID :925:

(i) SEQUENCE CHARACTERISTICS:

(A) LENGTH: 261 base pairs

(B) TYPE: nucleic acid

(C) STRANDEDNESS: double

(D) TOPOLOGY: linear

(xi) SEQUENCE DESCRIPTION: SEQ ID :925:

ATGGCTATGA CCTGGGACAT GGAAACAGTG ACCTCCGCGT TCTGGTCCCG 50

AGATCCTCGC ATCAGCGTCA TCGTGTGCAC CGGCTTGGGG GGCTGGAGTT 100

CCGGTTTTCT TTGTTTTTTC TCTTTATTCG TCCTTTCTCA AAGATGGGAT 150

ACTGATCAGA ATCGTTCTGT ATATGCTTGG GACTGGATGG AAAGACTTTG 200

AAGCAGCTGT GGGGGGCGGG AGGACACCGA CAACCAAACA GACGTGTTGG 250

TTCCAGTCCT G 261

(2) INFORMATION FOR SEQ ID :926:

(i) SEQUENCE CHARACTERISTICS:
(A) LENGTH: 173 base pairs
(B) TYPE: nucleic acid
(C) STRANDEDNESS: double
(D) TOPOLOGY: linear

(xi) SEQUENCE DESCRIPTION: SEQ ID :926:

GTCAGCCTGG GCAATCACAA CGACAACAAA AGGTACAGTC TCAAGAGTCC 50

AACTGGGTTC AAATCCTGGC TCTGTCACAT ACTAGCTGTG TGACCTTGGC 100

CAAGTTTCTT AACCTCTCTG TGTGTATAGT CCTTATCTGA AAATGGGGAT 150

AACCAACCAA GAGAGCTGAG AGA 173

(2) INFORMATION FOR SEQ ID :927:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :927:

AACACTCAGA CCGGACGGGA CGAGGGATTC CCCTGCCTTC GCTTCCCTCC 50

CTCGCTTGCT TTGTAGTTTT CCCGCACCAG CCCGCTGCCC TGCGCCATCC 100

TAGGGCCTCG AAGCCCGGGG AATCTGTCCC ATCCTGTAAC GGGCCGCCCC 150

CTTNGATTTT TCTGCCCGTG CCACCGGAGG TTGCTGCGAC GCGCACCGAC 200

TCAGATCATT ACTACTGAAC TCG 223

(2) INFORMATION FOR SEQ ID :928:

(i) SEQUENCE CHARACTERISTICS:

(A) LENGTH: 212 base pairs

(B) TYPE: nucleic acid

(C) STRANDEDNESS: double

(D) TOPOLOGY: linear

(xi) SEQUENCE DESCRIPTION: SEQ ID :928:

GTTATAATTA TTTACATGCT GCAACACGTC ACAACATAAA TTTACCAAAT 50

ATTTCATGGT GGGATGCTGT AGGTTTCAGA TAATAGTTTT AAGATAAAAA 100

CCACAGATTA AGTGAAGACA CCCACAACTT TAATGACTCT ACGACTCTCG 150

GTTGCTAAAT GCTAGAAGTC AAAAGGCAGT GTTTTTTCAG TCCTACCTGT 200

CATAACTTGC AT 212

(2) INFORMATION FOR SEQ ID :929:

(i) SEQUENCE CHARACTERISTICS:
(A) LENGTH: 233 base pairs
(B) TYPE: nucleic acid
(C) STRANDEDNESS: double
(D) TOPOLOGY: linear

(xi) SEQUENCE DESCRIPTION: SEQ ID :929:

TTAACATAAT ATTTTGAATA TATAGGGCTC ATAAGATATA TTATTAAATT 50

ATAAACAAAA TAATCAGGAA CTTAGTGTGG TGCCTAGTTT GATATATGAT 100

TACTTTTTGA AATGCACTAA ATTCCACAAA TAATGAAAGT ATTCTTTGTG 150

TATAATGTTA TGTTTGGTTA TTATGTATGG TCTTCGTATC CAAAGGTATG 200

ACATAACTTG AGTTTGTTTG CTTGGTATTT ATT 233

(2) INFORMATION FOR SEQ ID :930:

(i) SEQUENCE CHARACTERISTICS:
(A) LENGTH: 235 base pairs
(B) TYPE: nucleic acid
(C) STRANDEDNESS: double
(D) TOPOLOGY: linear

(xi) SEQUENCE DESCRIPTION: SEQ ID :930:

AGTCTGTAAC GATACACCCA TAGATGTGAA TTATGTATAT TCGAGAAGGA 50

GATCAGATGG CATCCAGAAG ATAGTTTAGA TTTCAGCACA TTCATGGTTC 100

AGATGCAAAA TCAGATCATC AAATAGATCC AGCAATTCAC AATCATCATA 150

GCCATATCGC GTCACGTCCG CCGCACAAAG ATCCGCACCA ATTATCTATC 200

TCCCACCCCC CCCAGGCATT CAGCACCCGC AAAAG 235

(2) INFORMATION FOR SEQ ID :931:

(i) SEQUENCE CHARACTERISTICS:

(A) LENGTH: 221 base pairs

(B) TYPE: nucleic acid

(C) STRANDEDNESS: double

(D) TOPOLOGY: linear

(xi) SEQUENCE DESCRIPTION: SEQ ID :931:

CTATCTAGAA TTACTTATTT CACTTGAAAT GTATGGTTTC AGGAAAATTT 50

TCAATTTAAC TTGAAGGGAT TATCTCTTAT TTTGCTTGGA ATAATGGCAT 100

CTCAGAAACA TGGGTTTACC TGTGATTTTT TTGTTTGGGT GAATGCTTAA 150

AAACAAAAAA AAAATTTACA TATGCATTTT ATGGATACAC ACACACACAC 200

ACACACACAC ACAAAAAACA C 221

(2) INFORMATION FOR SEQ ID :932:

(i) SEQUENCE CHARACTERISTICS:

(A) LENGTH: 333 base pairs

(B) TYPE: nucleic acid

(C) STRANDEDNESS: double

(D) TOPOLOGY: linear

(xi) SEQUENCE DESCRIPTION: SEQ ID :932:

TGCTCTATTG CTATCTCCTG ATATAGGTTA TATATATATT ATGTCTTGAG 50

CGTCATGTCT TGTGCTACTA CACCGTGTTA ATCCCAGCTA CTCAGGAGGC 100

TGGAGGATCG CTTGAGCCCA GGAGTTCTGG GCTGTAGTGC GCTATGCCGA 150

TCGGGTGTCC GCACTAAGTT CGGCATCAAT ATGGTGACCT CCCGGGAGCG 200

GGGGACCACC AGGTCTGCCT AAGGAGGGGG TGAACCGGCC CAGGTCGGAA 250

ACGGAGCAGG TCAAAACTCC CGTGCTGATC ATGTAGTGGG ATCGCAGCCT 300

GTGAATTAGC CACTGCACTC CAGCCCTGAG CAA 333

(2) INFORMATION FOR SEQ ID :933:

(i) SEQUENCE CHARACTERISTICS:
(A) LENGTH: 281 base pairs
(B) TYPE: nucleic acid
(C) STRANDEDNESS: double
(D) TOPOLOGY: linear

(xi) SEQUENCE DESCRIPTION: SEQ ID :933:

AGATTTATGG CTGCTTTCAC TTGGCTCCAT GTTCTGCTGA TAATTAAACA 50

GTTTGTGTGT CCTGTCTTTC CTCAAAGGAA CTTGTCAACC TTTGGAATTT 100

TGTTCATCTC AGAGGGACGT CTTAAGTACT CAGCTTTCTG AATGTTTAAG 150

AAAAAAATAA TACTTTTCAG AATTACCTAC CTCTTTCTCA TTTTTAGCCT 200

GGGATCAATT ATTCCCTTTC ACACTTTTTT ACATCCTAAG TAGAAATAAA 250

ACTCTTTATT CTATTTTTTT TCAGTTATTG G 281

(2) INFORMATION FOR SEQ ID :934:

(i) SEQUENCE CHARACTERISTICS:

(A) LENGTH: 266 base pairs

(B) TYPE: nucleic acid

(C) STRANDEDNESS: double

(D) TOPOLOGY: linear

(xi) SEQUENCE DESCRIPTION: SEQ ID :934:

AATCTTGTAT TATGTTAATA TTAAAATCTA ATAATAAGAT ACTGTAGAAG 50

TATTTTACAT TTTTGGGGTA TAGGCACAAA TATATACATT TAAATATTAG 100

TAATAGAATT GAACAATTAA CTATAAAATA TTATATCATA ATAAACTGTT 150

GTTTTATCCC AAGGGTAGAG GGACTCTAAG TAAAACTGTA ACAGAATCAA 200

AAGGTTACTG ATAGTTAGAA TGGGTTGAGA ACTATTTTAA CTTGGGAACT 250

TTAAATAAGT AAATCT 266

(2) INFORMATION FOR SEQ ID :935:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :935:

GGAAAACAAA TGTAAGGAGG GAATTCAAAT ACCTAGTTAA TCTATTAACT 50

ACTAATAACT CATTAAATAA ATTAATAATA TTAAATGATC TGTGTGTTCC 100

ATGCAAATAA CAGAATGATT TCCACTGAAT AGTCATGATG GTTTAAAATA 150

TTACTCTAAC ACCAATAACA ACACGTAA 178

(2) INFORMATION FOR SEQ ID :936:

(i) SEQUENCE CHARACTERISTICS:
(A) LENGTH: 224 base pairs
(B) TYPE: nucleic acid
(C) STRANDEDNESS: double
(D) TOPOLOGY: linear

(xi) SEQUENCE DESCRIPTION: SEQ ID :936:

ACTCAGCTCC GTGCGACCCA CATGGCCCCA GGCGGGGGGA CACCAGAGGC 50

TCCTCCATGA GCAGCAGGAG TGAGCGGAGG AATGTGCCCC ACAGCAACTT 100

TCCCAGCCAA TGCCACGATG GAGATGACAA CCCCAGATCT GGGGANACAG 150

AAACCACTCA GAACGGCACA GGGTAACTGG CCCCAAACGC TGAAAGTTAG 200

ACTTCACCCG AATTACATTT ACCA 224

(2) INFORMATION FOR SEQ ID :937:

(i) SEQUENCE CHARACTERISTICS:

(A) LENGTH: 270 base pairs

(B) TYPE: nucleic acid

(C) STRANDEDNESS: double

(D) TOPOLOGY: linear

(xi) SEQUENCE DESCRIPTION: SEQ ID :937:

ATTTTTTTTC CCAAGAATCC CCAACTCAAT AAAACCGTGA AACAATTGCT 50

GGGGATGGGT AGAGAAAACC CATTTATAGT ACTTTTCTGC CTTCTTGTGG 100

TTTTCGGCTT TGCGATCAGG GACTGAAGAT AGAAGGAAAA AAAAAAAAAA 150

AAAACAAACA CAAAAACATT TCAAAAGAGA AAGGAAAAAA CTTTTTCCAA 200

CCAAAACTCC TAAAAAACCC AAAAACAGAA CAACCAATTA AACCCGACCC 250

GACACAACTA CCAATGACTG 270

(2) INFORMATION FOR SEQ ID :938:

(i) SEQUENCE CHARACTERISTICS:

(A) LENGTH: 215 base pairs

(B) TYPE: nucleic acid

(C) STRANDEDNESS: double

(D) TOPOLOGY: linear

(xi) SEQUENCE DESCRIPTION: SEQ ID :938:

AGCCTTCTCC CTATTCGATA AAGATGGCGA TGGCACCATC ACAACAAAGG 50

AACTTGGAAC TGTCATGAGG TCACTGGGTC AGAACCCAAC AGAAGCTGAA 100

TTGCAGGATA TGATCAATGA AGTGAATGCT GACGACAATG GCACCATTGA 150

CTTCCACAAA TCTTCAACTA TGATGCCTAG AAAAAAGAAA GATACAGATA 200

CTAGAACTAA AGTCA 215

(2) INFORMATION FOR SEQ ID :939:

(i) SEQUENCE CHARACTERISTICS:
(A) LENGTH: 303 base pairs
(B) TYPE: nucleic acid
(C) STRANDEDNESS: double
(D) TOPOLOGY: linear

(xi) SEQUENCE DESCRIPTION: SEQ ID :939:

TGTCGTCCTT AATATTTCTN AGCCTTTTGT CATAGAGGTG GTCGGTTGGA 50

CCTCAAAGCC TCCATCTAAT TTTTTTTGTC TATGTTTCTA TATCTCTCTC 100

TTTTACGCTT CTTTCCCGGA CCGTCCCCTC CCTCTACAAT TATATTTACT 150

ACCTTCATGA TTGCTTTTTA TTATTTCTTT CACTCATCAT TATTGTTCAT 200

TTTTTAATTA ACAATTTTTT TCATTATTCT TTTTCTTAAT TTGTAACCCG 250

TTTCTAATTT TCTCAATTAT TGCGCTTTCT CAACCCCCCT CCTTCCTCAT 300

TAC 303

(2) INFORMATION FOR SEQ ID :940:

(i) SEQUENCE CHARACTERISTICS:

(A) LENGTH: 330 base pairs

(B) TYPE: nucleic acid

(C) STRANDEDNESS: double

(D) TOPOLOGY: linear

(xi) SEQUENCE DESCRIPTION: SEQ ID :940:

ACACTATTTT CAAAAATCCA AATAAAAGTG CAGTTTCAGA CTTTTTAAAA 50

AAACACCGTT GACCTGTCTT AGTTGTACAT TCAGAAAATG TAGCCTCGAG 100

CGTTTGCATG CAACACTGCA TATTTTTTCT AATCAGATTA ATATGAGTTT 150

TAATGTTTAG CATGAACTAC AGCTAAGGAT AAAAATTTTA AAGTAGCTTT 200

CATAGTCTAG ATTCCTGGTT TCATAATGCT TTATGTAGTA CATTTCAGTT 250

TGCTTTGCTT TGTGGAGACT TGCGCGTATT TTCTTTTGCT TGTTTGTTTA 300

TCGCCAGGCA GCGCTGTTCG AACTGTGAGA 330

(2) INFORMATION FOR SEQ ID :941:

(i) SEQUENCE CHARACTERISTICS:
(A) LENGTH: 107 base pairs
(B) TYPE: nucleic acid
(C) STRANDEDNESS: double
(D) TOPOLOGY: linear

(xi) SEQUENCE DESCRIPTION: SEQ ID :941:

TTTATTTCAG ATAATTTTAT GTAAACAAAT TAAGAGTTAT TCATTCAAAT 50

TTTTGGCAGT GTTAATCTGT AAATGATGAC TTGATGTACA GAAAATGCAT 100

TTTTGCT 107

(2) INFORMATION FOR SEQ ID :942:

(i) SEQUENCE CHARACTERISTICS:
(A) LENGTH: 126 base pairs
(B) TYPE: nucleic acid
(C) STRANDEDNESS: double
(D) TOPOLOGY: linear

(xi) SEQUENCE DESCRIPTION: SEQ ID :942:

CCACAGCATA CACACCTTGG AAACCAAGAA GACTCTTTGT GTGTGTATGT 50

GTGCGAGACT GACGCGTCTC TACGCGCTCT ATGGGGGGAG ATTTGGTTAT 100

TTCGGCGTTA CACCTCCGGT GTTCTC 126

(2) INFORMATION FOR SEQ ID :943:

(i) SEQUENCE CHARACTERISTICS:
(A) LENGTH: 155 base pairs
(B) TYPE: nucleic acid
(C) STRANDEDNESS: double
(D) TOPOLOGY: linear

(xi) SEQUENCE DESCRIPTION: SEQ ID :943:

ACTGAACAGC CGCTGGTTCC AGAACAGGCA CAGAAGGCTC CAGCGCTAGA 50

AACAACTGGC ACAGGAATGG TAACAATAGT CTGGCTCAGC CACAACAGGA 100

GGCTCCGGGA CAGTCACAAC CGAGAGCTTC CAGGACAGTT ACAGTCTGTA 150

AGCTC 155

(2) INFORMATION FOR SEQ ID :944:

(i) SEQUENCE CHARACTERISTICS:
(A) LENGTH: 287 base pairs
(B) TYPE: nucleic acid
(C) STRANDEDNESS: double
(D) TOPOLOGY: linear

(xi) SEQUENCE DESCRIPTION: SEQ ID :944:

CGTGAGGAGC AGGTACCATT TTTAGTAGTC ACACAATTCC ACCTCCTGTT 50

TTTACTACTG GCAACTTCTA CTTGAGAATA ATGTTCTGAA AATGGAGGTG 100

GGGGGGGGTT TGGAAGCAAA CACATTTGGG GTTTTTAACC AATTGTTAGG 150

TTCTTTTTAT TTAGGGTTGT GCAGGGAACT GTTGGGAGGT TTCTTTAGGT 200

GGGATGGGGG GACTGGGTGT TGGTTTTTGG GGGGTTTTTG GGACGGGTTT 250

TATTTTGTCC CTTAACTTGG TTTGTTGGGC AGGTGGG 287

(2) INFORMATION FOR SEQ ID :945:

(i) SEQUENCE CHARACTERISTICS:

(A) LENGTH: 149 base pairs

(B) TYPE: nucleic acid

(C) STRANDEDNESS: double

(D) TOPOLOGY: linear

(xi) SEQUENCE DESCRIPTION: SEQ ID :945:

ATCATTGATC AATGATATGG TTTGGATTAC TGCCCCGCCC AAATCTCTTG 50

TCCAATTATA ATCCCCAGCA TTGGAGGAGG GGCCCGGTGG GAGGTGATTG 100

GATCATGGGC ATGGACTTCC CCCTTGCTGT TTTCGTGATA ATGAATTCT 149

(2) INFORMATION FOR SEQ ID :946:

(i) SEQUENCE CHARACTERISTICS:

(A) LENGTH: 261 base pairs

(B) TYPE: nucleic acid

(C) STRANDEDNESS: double

(D) TOPOLOGY: linear

(xi) SEQUENCE DESCRIPTION: SEQ ID :946:

TTTAAACTGC AAATAGTCGT TACAAAAAGT TTTNNNNNTT CTTTTAAATA 50

AATTTCACAC AAAGAAAGAG AAATAGAAAG CGACGGTAGT GACCAGCAAG 100

AGGAATAATA ATTACATTCA TCTTAATGTG TGTGTGCCAG TTCTGTTTAC 150

ATTAACATTG GAAAACTCCA GACCTGGACA CCAGAACCTC AAATCTGTGA 200

GTGGAATGTC TTGAGATGGG CACGCTGGAA GTCAAAGGGT TTCTCTTTTT 250

TTTTTTTCCC C 261

(2) INFORMATION FOR SEQ ID :947:

(i) SEQUENCE CHARACTERISTICS:
(A) LENGTH: 264 base pairs
(B) TYPE: nucleic acid
(C) STRANDEDNESS: double
(D) TOPOLOGY: linear

(xi) SEQUENCE DESCRIPTION: SEQ ID :947:

CTTTAGGAAC TGTCAGCATG TTGTTGTTGA AGTGTGGAGT TGTAACTCTG 50

CGTGGACTAT GGACAGTCAA CAATATGTAC TTAAAAGTTG CACTATTGCA 100

AAACGGGTGT ATTATCCAGG TACTCGTACA CTATTTTTTT GTACTGCTGG 150

TCCTGTACCA GAAACATTTT CTTTTATTGT TACTTGCTTT TTACACTTTG 200

TTTAGCCACT TAAAATCTGC TTATGGCACA ATTTGCCTCA AAATCCATTC 250

CAAGTTGTAT ATTT 264

(2) INFORMATION FOR SEQ ID :948:

(i) SEQUENCE CHARACTERISTICS:
(A) LENGTH: 149 base pairs
(B) TYPE: nucleic acid
(C) STRANDEDNESS: double
(D) TOPOLOGY: linear

(xi) SEQUENCE DESCRIPTION: SEQ ID :948:

CCTCGACTTA CCACGGGTGA TCCCAGAATG GACTATCAGC GGTGTTTTGA 50

GCCAGGTTGA TTGAGCACTG GGCTCCAACT TATTGTTAAT GAGAAACGGC 100

CCAACTTGGA CCATGACTTT CCCATTTGCA GGTCTTAGAA TAATTTTTT 149

(2) INFORMATION FOR SEQ ID :949:

(i) SEQUENCE CHARACTERISTICS:
(A) LENGTH: 242 base pairs
(B) TYPE: nucleic acid
(C) STRANDEDNESS: double
(D) TOPOLOGY: linear

(xi) SEQUENCE DESCRIPTION: SEQ ID :949:

CGCTCAGGGT GGAAGTGGCC GGGCGCGTCG AATGTGCCGA GCTGGTGCGA 50

GTGGGCGAGC AGAGCGCCTT GAGGGTTGAG CATCTTCTGG ATCCAGCAGA 100

GGCCTCCCAG GAGGGGAGCC AGGTGGCTGA AGGCAAGCAC TTTNNNNAGG 150

TTCTAGTCCC CTTTTTAGAT CCTAGGGGAC TGGAGATTTG GCACCTTCCC 200

CCAAGGCTTG GCCTCGTGCC CTATAGGGTG CCCCCCTCTC TT 242

(2) INFORMATION FOR SEQ ID :950:

(i) SEQUENCE CHARACTERISTICS:

(A) LENGTH: 153 base pairs

(B) TYPE: nucleic acid

(C) STRANDEDNESS: double

(D) TOPOLOGY: linear

(xi) SEQUENCE DESCRIPTION: SEQ ID :950:

ACTCTGCCTG CACTATAAAA TGAACCAAGG AGGATCCAGT TTGCTGTCTG 50

CACTGACAGA TTTACAGACA GGAAAACAAA GCCTTACTTA CCAGGAATCC 100

AAAGTTTATA CATGAAAATT TAAAAAGGGA GTCTATGCAA AGCGGAGTTC 150

AGG 153

(2) INFORMATION FOR SEQ ID :951:

(i) SEQUENCE CHARACTERISTICS:

(A) LENGTH: 128 base pairs

(B) TYPE: nucleic acid

(C) STRANDEDNESS: double

(D) TOPOLOGY: linear

(xi) SEQUENCE DESCRIPTION: SEQ ID :951:

TGCGGGAGTC TCACTGGGGG GGGGACACCT GTGCAAACGC GTAACGCAGG 50

TTGTTCCTAA GGCGAGCTCA GGGAGGATAC AGAAAACTCT CCTGTGGTGC 100

TATGAAATGT GGCGTAAAAA GCATTCTG 128

(2) INFORMATION FOR SEQ ID :952:

(i) SEQUENCE CHARACTERISTICS:
(A) LENGTH: 213 base pairs
(B) TYPE: nucleic acid
(C) STRANDEDNESS: double
(D) TOPOLOGY: linear

(xi) SEQUENCE DESCRIPTION: SEQ ID :952:

GTTGGAAATT TCCCTCAAAT CACCTACCGA TTACCCTTGA TTTCCCTTTG 50

TTTTCAGTTT CTCAAAACGA ATGAAATAGA ATATAGCAGA ATGTTAACCC 100

ATATAAAAAT AAAGTGTACC CAAATATTGT AATGTATATT GCTGCTCTTC 150

TTCAAATTAA ATAAGGGTTT AAAACCACTT AATTGGTAAT ACAACATCTC 200

AATTGATACA AAT 213

(2) INFORMATION FOR SEQ ID :953:

(i) SEQUENCE CHARACTERISTICS:
(A) LENGTH: 236 base pairs
(B) TYPE: nucleic acid
(C) STRANDEDNESS: double
(D) TOPOLOGY: linear

(xi) SEQUENCE DESCRIPTION: SEQ ID :953:

GCAGCAGTTT CTTTGAAAAT TTGGACCAGA AGGTGCATAA CAAGTTGTTC 50

TGCAGAAGTT CTTATCTGAT ATTCTTAGGG AGCTATCCTG CATGTAATCT 100

TCATTATTTT TTTTCTCTAC CATCATGTAG GCATACTCAG TGTAGACTAC 150

CACAATCCTG GATACCTCTC TGCTTAGATT TACAATCTCT GCTAAGATTT 200

GCCACTGCAG AAAGTGTAGT ATTTTCACTA CATTAT 236

(2) INFORMATION FOR SEQ ID :954:

(i) SEQUENCE CHARACTERISTICS:
(A) LENGTH: 217 base pairs
(B) TYPE: nucleic acid
(C) STRANDEDNESS: double
(D) TOPOLOGY: linear

(xi) SEQUENCE DESCRIPTION: SEQ ID :954:

GCAGCAGTTT CTTTGAAAAT TTGGACCAGA AGGTGCATAA CAAGTTGTTC 50

TGCAGAAGTT CTTATCTGAT ATTCTTAGGG AGCTATCCTG CATGTAATCT 100

TCATTTTTTT TTTCTCTACC ATCATGTGAG GCATACCCAG TGTAGACAAC 150

CACAATCCTG GAAACCTCTC TGCTTAGATT TACAATCTCT GCAAAGATTT 200

GCCACTGCAG AAAGTGT 217

(2) INFORMATION FOR SEQ ID :955:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :955:

AGCAGCGACC GCGCTCACTG GCTTTTTGTT TCTGCTTGGG CCTTTTCTGT 50

TCAGTCCATG CTTGAACTAC TCCACCCCCT TAAATCCAAC GTAAAAACCA 100

GTCTTTTCTG TGCTTGTTCT CTGTGCGTTA ATGCTTTTTC TTATGCTTGC 150

TTATTGAAAT ACTGTATTTT CATTGTCCCC TGGCCAAAAC ATCTGAGTCG 200

TGAAACCATT TTAGATACTC TACTTTCTTA CTGTGTTACG TGGCATTTTA 250

TGCTTG 256

(2) INFORMATION FOR SEQ ID :956:

(i) SEQUENCE CHARACTERISTICS:

(A) LENGTH: 194 base pairs
(B) TYPE: nucleic acid
(C) STRANDEDNESS: double
(D) TOPOLOGY: linear

(xi) SEQUENCE DESCRIPTION: SEQ ID :956:

CAAGCAATTG AGAGACTTTT TTCCTGTTAT TTTTCTACCA TTTATTTCTC 50

ATTTCATTAT ATTATGGTCA GAGAATATAT TTTGAATGAT TTCATTTATT 100

AATTTTTAAA AATAACATTA AAAAATTTTT TAAAATGTGA ATATACCACA 150

ATACAGTATA AAGATTGTAC ATTCTGTTTT TGGACAGTTT TCTA 194

(2) INFORMATION FOR SEQ ID :957:

(i) SEQUENCE CHARACTERISTICS:
(A) LENGTH: 196 base pairs
(B) TYPE: nucleic acid
(C) STRANDEDNESS: double
(D) TOPOLOGY: linear

(xi) SEQUENCE DESCRIPTION: SEQ ID :957:

ATCTATTTTT CTTGGTCTCA TACTCAGTTT TTTATGTAGT CACTTAGTAA 50

ATAACCTAAA AAAACATGTT TTCTCCACAC TAATTTTAGG GTGAATTCCT 100

CATTTCGCTT TTCAGATCAT GGGGTGAGGG GGATGGTTCG TGTGTGTGAG 150

GAACTGAGGA ATCAGATGGA AAACAATGCC TCTGCTCCTT TGAGTA 196

(2) INFORMATION FOR SEQ ID :958:

(i) SEQUENCE CHARACTERISTICS:
(A) LENGTH: 105 base pairs
(B) TYPE: nucleic acid
(C) STRANDEDNESS: double
(D) TOPOLOGY: linear

(xi) SEQUENCE DESCRIPTION: SEQ ID :958:

GCGCTCAGCT GGAACTTCCG ATCTATTTTT TCTTGGTCTC ATACTCAGTT 50

TTTTTATGTA GTCACTTAGT AAAATAAACC TAAAAAAAAA CATTGATTTT 100

TTCTG 105

(2) INFORMATION FOR SEQ ID :959:

(i) SEQUENCE CHARACTERISTICS:
(A) LENGTH: 261 base pairs
(B) TYPE: nucleic acid
(C) STRANDEDNESS: double
(D) TOPOLOGY: linear

(xi) SEQUENCE DESCRIPTION: SEQ ID :959:

GACAGCGACC GACACTCAAT GGAATCCCGA TTCCATGGAA ATGGAAAATA 50

GCTCTATTTA TACAGCAAAT TCAAATACAG TAGCTTGCTA AGCAACTTCA 100

TAATTCATTA ACACTCGATG GCAGAGCAGA TAGTCACAAT TACTAACGAT 150

TATCATGATC TGCATTCTTG ACAAGTATAC TATGGCGAGA TAAATCAAAC 200

TCAGACGATG ATAAGCAAAC TCAAAAATGA AAATGATGAT GCCAACTAAG 250

CTTTTGTTAA A 261

(2) INFORMATION FOR SEQ ID :960:

(i) SEQUENCE CHARACTERISTICS:
(A) LENGTH: 188 base pairs
(B) TYPE: nucleic acid
(C) STRANDEDNESS: double
(D) TOPOLOGY: linear

(xi) SEQUENCE DESCRIPTION: SEQ ID :960:

CACAGTTTTG TTGACTAAAC TCTACAGAAG TCAGGTATTG GGAAGGAGAA 50

TCGTTTCTTC TTTAGAGGAG TAGGTTTTAA CCATGTTAAA TTTCCAAGAA 100

TAAATTTATT TATCACCTCT ACACAGCGCA GACCATTTCA GGAAAATAAA 150

TAATTTTCGA TTCAAGTACT ATACAAACTT AACGATGG 188

(2) INFORMATION FOR SEQ ID :961:

(i) SEQUENCE CHARACTERISTICS:
(A) LENGTH: 191 base pairs
(B) TYPE: nucleic acid
(C) STRANDEDNESS: double

(D) TOPOLOGY: linear

(xi) SEQUENCE DESCRIPTION: SEQ ID :961:

ACTTTCGCCT ACCCGGAGAG GCCCAGAAAA TTGACCAGAT GATGGAGGCC 50

TTCACCCAGC AATACTGCCT ATGCAACCCT GGAGCTTTTC AATCCACAGA 100

CACACGCTAT GCACTGTCCT TCGCCATCAT AACGCTCAAC ACCAATTTCT 150

ACAACCAAAA AGCCAGAGAC AAACCTGACC TAAAGCGCTC C 191

(2) INFORMATION FOR SEQ ID :962:

(i) SEQUENCE CHARACTERISTICS:
(A) LENGTH: 191 base pairs
(B) TYPE: nucleic acid
(C) STRANDEDNESS: double
(D) TOPOLOGY: linear

(xi) SEQUENCE DESCRIPTION: SEQ ID :962:

CCAAGGCAGA GTTTATTAGG TTTATGCTTT ATATTTATAT ATAAGGTTTT 50

ATTCATGTTA AAAACTTAAA ACCAACGGAT TATGCAAAGT GATAAGTGTA 100

TCAATGAATA TACAACTGAC TGGATTTTAA ATATACACAA ACCAGTATAC 150

CCCCAGTACT TAAATGAAGG CACTCAGTAT ACACCAAAAT T 191

(2) INFORMATION FOR SEQ ID :963:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :963:

AGCAGGCCAA AAGCCCAGGT GAGGCCCAGC AGGAACAGCA GCCCAATGCC 50

TCCTAGCACT CAAGATCCAC CATAGTCCAA GCAACTGCAG TTACACTTGA 100

GTACAAATAC GCTCCCGACA GCCTCATGCA GCGACACCCT AAAGGACACC 150

CGAACACCAT CAATAACCGA ATAGACTA 178

(2) INFORMATION FOR SEQ ID :964:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :964:

ACTTTGCAGT CCTCACATCA CAAATAGAGT AAAAGATTCC CAAAAGGAAG 50

GGGATCCTTT TGACTGCCAG ACGCGGGAAG GAAAGAATGA AATTAAAGAA 100

TATCCTTTTA AACACACACC TCGACACAAT TTTCCACTCT GCTAAGGGAT 150

CACAAAGACT AAGACGCGGG CAGACTCCCC ATAAGCCACG CTTATAACC 199

(2) INFORMATION FOR SEQ ID :965:

(i) SEQUENCE CHARACTERISTICS:

(A) LENGTH: 217 base pairs

(B) TYPE: nucleic acid

(C) STRANDEDNESS: double

(D) TOPOLOGY: linear

(xi) SEQUENCE DESCRIPTION: SEQ ID :965:

ATGGAGCAGG ACCTGCGATG TTCTGATGGA TTATACCCAC AGTGTATTTT 50

GGCACATCTG CCAAAAGCTA CAAACAACCC CTAATAATTA CACACTACAG 100

ATAGTGAGAA GCGTCCTACC CAGGAGTCCT GAATGTGATC TGAGTATGCT 150

CTAAGGCAGC CCCAGGAAAA GCAATCCAAT CCCTTTCTCC TCGCCTTTAA 200

ACCTGCAGGT TGGGGCT 217

(2) INFORMATION FOR SEQ ID :966:

(i) SEQUENCE CHARACTERISTICS:

(A) LENGTH: 175 base pairs

(B) TYPE: nucleic acid

(C) STRANDEDNESS: double

(D) TOPOLOGY: linear

(xi) SEQUENCE DESCRIPTION: SEQ ID :966:

ATTTGCGACC AAAATCATCT GGGTTTATAT TTGAAAAAAC CTTGTTTTCC 50

AATTTAAAAA TGCCAAGAAA GTTGTGAATA CCGTTCATTA TCACTAGGAT 100

CAAGAAAAAG CAAATGGTTT AAGTTTTTAC ATAGGAAATC TAAGAACTAC 150

TCATCAATAA AACGCAACAC TATCA 175

(2) INFORMATION FOR SEQ ID :967:

(i) SEQUENCE CHARACTERISTICS:
(A) LENGTH: 92 base pairs
(B) TYPE: nucleic acid
(C) STRANDEDNESS: double
(D) TOPOLOGY: linear

(xi) SEQUENCE DESCRIPTION: SEQ ID :967:

AGGGGTTCAG GCCTGGAGTC AGGGAAGAAG GGGAAAGGGG CAGATAGCTG 50

GGGGACAAGG AAAACCTGGC GCCCCCCACC GCGAAAAACA CA 92

(2) INFORMATION FOR SEQ ID :968:

(i) SEQUENCE CHARACTERISTICS:
(A) LENGTH: 208 base pairs
(B) TYPE: nucleic acid
(C) STRANDEDNESS: double
(D) TOPOLOGY: linear

(xi) SEQUENCE DESCRIPTION: SEQ ID :968:

GTAATATTAG GGACATGAAT GTTAGTACAG TAATTACCAC ACATGGAAAA 50

TATTGTTCAG CAGGAAAAGT AAAACTTTCA AAAAATTCCC TAAAGATCCT 100

ATTCAATAAA CAATTTTAGA TTTAAGGAAC CACTTACGCA AAACTCGAAC 150

AAATAACCGA AAACTCCACC TACCGCGCAA TACTCAAAAA CACAAAAATA 200

CTACTAAC 208

(2) INFORMATION FOR SEQ ID :969:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :969:

AGTGGTTGGT GTTTACTGGA ATCTTGTTTT TTTTTTTTCG GTTTTTTTGA 50

CTACTCGGGG GGTTTTCCTT TTTTACAGGA ACTAAATCAA CAAACTTTTT 100

CGATTCCTCA ACTTTAGATT TTTATTTTTT TTGATTACCA TTTACTTTTC 150

TTCATTTAAA ACTTTTAACG CCTCAGCCTT TTTCTTAGGC CAAGGCCTTC 200

CACCAAGGAT TTACTCCGGG ACGGATCTAA CTGGATTTTC CGAGGTGGGT 250

TTAAGA 256

(2) INFORMATION FOR SEQ ID :970:

(i) SEQUENCE CHARACTERISTICS:
(A) LENGTH: 240 base pairs
(B) TYPE: nucleic acid
(C) STRANDEDNESS: double
(D) TOPOLOGY: linear

(xi) SEQUENCE DESCRIPTION: SEQ ID :970:

AATCTATTTT CCTTGGTCTC ATACTAAGTT TTTTATGTAG TCACTTAGTA 50

AATAACCTAA CCCGCTTGTT TTTTCCACAC TAATACTAGG GCGAATTCCT 100

CATTTTATTT TTAAGATCAT GGGGCGAGGG AGATGATCGC GAGCACGAGG 150

ACCTAAGACA TCAGATGAAA AACAATGCCT CTATTCCTTC AAGTATAATA 200

AATGATACCC GAGAGTAAAG AGCTACACTT CGCACCTCTA 240

(2) INFORMATION FOR SEQ ID :971:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :971:

ATCTATTTTT CTTGGTCTCA TACTCAGTTT TTTATGTAGT CACTTAGTAA 50

ATAACCTAAT CCGCTTGTTT TCTCCACACT AATCCGAGGG TGAATTCCTA 100

ATCTAGCTTT TCAGATCATG GGACGAGGGA AATGACCGCG TGCGCGAGGC 150

CCTAAGGAAT AACATAGAAA ACACCGCCTC TGCT 184

(2) INFORMATION FOR SEQ ID :972:

(i) SEQUENCE CHARACTERISTICS:
(A) LENGTH: 234 base pairs
(B) TYPE: nucleic acid
(C) STRANDEDNESS: double
(D) TOPOLOGY: linear

(xi) SEQUENCE DESCRIPTION: SEQ ID :972:

GTGTTCTCTA AGGTGATACC TTTTAATTTT GAAAGACTAA ATAATTTTAA 50

TCGAGAATTT CCAGTCTTTC AGTCTGATCT ATTTAATTCA CTACTTGTTA 100

CATAATCCAG TGAAAACTCT ACTTGTTGAA ATTATGACAT AAAGATCTTG 150

CAGCTTTATT TGAGTATTTG TTCTTTTGTG TAGTTTCCAT CTTTTAAAAT 200

ATTTAAAATA TTTTCAAGGA TAAAAGTATT ATCT 234

(2) INFORMATION FOR SEQ ID :973:

(i) SEQUENCE CHARACTERISTICS:

(A) LENGTH: 197 base pairs
(B) TYPE: nucleic acid
(C) STRANDEDNESS: double
(D) TOPOLOGY: linear

(xi) SEQUENCE DESCRIPTION: SEQ ID :973:

TTTTCATCTA AAGGAGAGAG GCAGGCTCAG CTCCTGAAGG TCGCAGAGCC 50

TCAGTAGTCC TCCTGAGTGC GTCTAACTAA CTACCATCAA AAAACAAACA 100

AAAAAAACAT CACAAAACTG AACTGCCGCC ATCACCACTA CACCAAGTAT 150

GTGATTGAAT TATTCAATGA TCTGTTTTCG CGGTAGTGAC CAACANC 197

(2) INFORMATION FOR SEQ ID :974:

(i) SEQUENCE CHARACTERISTICS:
(A) LENGTH: 244 base pairs
(B) TYPE: nucleic acid
(C) STRANDEDNESS: double
(D) TOPOLOGY: linear

(xi) SEQUENCE DESCRIPTION: SEQ ID :974:

AGCAGGAGAT TTTTTTAATA ATAAACANCA ATGGAGAGGT GGAAATAGAG 50

ATGGAAAAAC AGGATCAAAG GAGCAACATA TCCAGTGACT TAAAAAAATA 100

ATCACACGTG GCAAGAAGTT TTCTAAAGTA AACTGTATGT GGGGAGAAGC 150

ATACAGAAAA AAACGAAATA CATACACCCC CAGGTTTGAA AGAAAATAAT 200

TTTGAGTTGT ATCAATCTAT CGATATCAAA GAATGAATGA CCGC 244

(2) INFORMATION FOR SEQ ID :975:

(i) SEQUENCE CHARACTERISTICS:

(A) LENGTH: 330 base pairs

(B) TYPE: nucleic acid

(C) STRANDEDNESS: double

(D) TOPOLOGY: linear

(xi) SEQUENCE DESCRIPTION: SEQ ID :975:

TTTTTTTTTC TGAACACTTT GCTTCACACC TTCTCATATT CACGGTTATG 50

AATTCTCCTG ACTATATCTA TTGTAACCCA AAACAGCAGA CTGTATTTAG 100

AGACCCATTA GAGATTTCAT GTCTATATGC CCAGAGCCTG ATATAATGCC 150

ACCTTACTAT ACACATAATA TAGAATCTGA GGACTACGTT AACTGATACA 200

TGTTAAGTAT CCTGCAGAGA GCTGGCACAT TGAATGTGCT CAATAATCGT 250

TAGCTTTAAG TATCTACCTC AAAGGGCTAC TGTGTGACTC CAATGAGATA 300

ATGTGCAATG AAGGGTTTCG TGTAATATTG 330

(2) INFORMATION FOR SEQ ID :976:

(i) SEQUENCE CHARACTERISTICS:

(A) LENGTH: 200 base pairs

(B) TYPE: nucleic acid

(C) STRANDEDNESS: double
(D) TOPOLOGY: linear

(xi) SEQUENCE DESCRIPTION: SEQ ID :976:

TGGTGAAAGG AATAAGCAAC TCAAGGTAAA TAAAGAAGAA AGATGAAGAT 50

TCTTAGAATC GTAGAAAGAC TGCAGGGGGA AATAATTAAA GCAGCTTTAT 100

TCCTTACGGC ATTCATCATT CGTCGAGCTG GAAAGCACTG ACAACTTTGT 150

AATGAGTTTT TTGCGCTTAA CTCTGTCAGG TGATTTATAT GAATNACTTA 200

(2) INFORMATION FOR SEQ ID :977:

(i) SEQUENCE CHARACTERISTICS:
(A) LENGTH: 296 base pairs
(B) TYPE: nucleic acid
(C) STRANDEDNESS: double
(D) TOPOLOGY: linear

(xi) SEQUENCE DESCRIPTION: SEQ ID :977:

AAAGGACAAT TTAATATAAA TCTATGTTTG TGCATTTCTG ACAGGCCATT 50

ATTATCTGCT TTGACAAAGC CTTTCTGAAA CGCAGTGTAC AATGAATCTT 100

AATGATGTTA TGAAACGAGC TTTGCTCGGG GCTCTTGATT GGAGCTTCCG 150

GTATGTGATG ACGGTATGTC ATGTATGCAT GGATGTACTC AACTGTGTTT 200

AATACTCTGA ATTTTAATTA GAAAAAAATA CAATAGCAGC AAGGCCCTGG 250

TTTCTAAGCT GCATACTTTT ACTAACGCGG GACATGAGCA AATGCC 296

(2) INFORMATION FOR SEQ ID :978:

(i) SEQUENCE CHARACTERISTICS:
(A) LENGTH: 228 base pairs
(B) TYPE: nucleic acid
(C) STRANDEDNESS: double
(D) TOPOLOGY: linear

(xi) SEQUENCE DESCRIPTION: SEQ ID :978:

GAATCNCGCC TGACACATAG AATGCATAAA CGAGGGGGGT CCTTCTGCAG 50

ATACTCTAAT CACTACACAG CTTTCTCTAT AAAACTACCC ATAAGCCTTT 100

AACCTTTAGA GAAAAATGAA AAAGGTTAAT GCTTGGAAGC CGGGGGAGGA 150

CTGACCACTT CATAAGCCAG TACGTCTGAG CTGAGTATGC CCCCAGAATC 200

CAGCCACTAC TGATAATTCA CAACGGTA 228

(2) INFORMATION FOR SEQ ID :979:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :979:

CGTACAAAAG CGCTTTGGAG ATGCCAGAAA AAACCCTACG GGAGGGTTAT 50

CCCCCTGACG CTAGGCCATG ACACAACTAA ACTTCAATAC ACCAGGACTA 100

AGGCAAGTAT GAAGAAGCAA AATATCGTAA CGACGCACCC TCGAGATCTA 150

CCAGGCAAAA CCAGAACCTG A 171

(2) INFORMATION FOR SEQ ID :980:

(i) SEQUENCE CHARACTERISTICS:
(A) LENGTH: 241 base pairs
(B) TYPE: nucleic acid
(C) STRANDEDNESS: double
(D) TOPOLOGY: linear

(xi) SEQUENCE DESCRIPTION: SEQ ID :980:

TGATTCCCCA TTTCCCTGGA CAGGGCAGAG TCAAGCCGAG AAGGAGGAGC 50

CACAGGCCAG CTGTTAATGA GACACTAAAT AAAACGTTCA GTATTCATAC 100

CTGTGGAACA TGGGTGAACC TTTCTGGTAA CTCGATTTTT CACACCAATG 150

ACAGCAAATG AGAAAGTGAG AGAAGCTAGA TAAATGTTTT TAAAGCATTA 200

CTGCAGTGGA AAATAACTGC ACTCTCAGGA CACAGCACTG T 241

(2) INFORMATION FOR SEQ ID :981:

(i) SEQUENCE CHARACTERISTICS:
(A) LENGTH: 227 base pairs
(B) TYPE: nucleic acid
(C) STRANDEDNESS: double
(D) TOPOLOGY: linear

(xi) SEQUENCE DESCRIPTION: SEQ ID :981:

TCACTGCAAT CACGGTAGCT TCGCCCATTG CCTCTTCAAC CAGGCACATA 50

CATCGAGAAG TTGAACCTGC ACTTTATTTC ACACTGAACA GACTTACCCC 100

GACAACAACA CCTCCCCAGT GGGACAAACC TACCCCACCT CCCCACACTC 150

TACTCCCAGC TAACGTTCCC TATAATCATC ATGACCCATT AAACATTTGC 200

AAAACCACAC TTCAACCCAC AGGAAGA 227

(2) INFORMATION FOR SEQ ID :982:

(i) SEQUENCE CHARACTERISTICS:
(A) LENGTH: 205 base pairs
(B) TYPE: nucleic acid
(C) STRANDEDNESS: double
(D) TOPOLOGY: linear

(xi) SEQUENCE DESCRIPTION: SEQ ID :982:

CGTTGTCGCC ATGGTAATAC CGCTCAGTAC GGGAGGACCC ACAGGCTAAG 50

ACACTCGGAG CACGAGCTCG CCTGAGGGAC CAACGAGGCG AAGCCACAAT 100

CCGCGAACTC ATGACTGACC CCTTCTAAGT AAGAATCCCA CCCAGGCAAA 150

CCAATCCGAC AACCGAAATA GCGCAGGCAC CGCCAAATTC AAGGCGCCCC 200

AAAAG 205

(2) INFORMATION FOR SEQ ID :983:

(i) SEQUENCE CHARACTERISTICS:
(A) LENGTH: 204 base pairs
(B) TYPE: nucleic acid
(C) STRANDEDNESS: double
(D) TOPOLOGY: linear

(xi) SEQUENCE DESCRIPTION: SEQ ID :983:

AGCAACCGGC ACTCAAGTGG AATCCCGAAA AAGTGATTTA AAAATGATCT 50

TACCTGTACC AGAAAAGCAA AATTAAAGGA AACAAAATAA GAACCATAGT 100

CCCAAATGAC ATTTAACCGT ACATACAGCG ATAACATGTT CAAAATCCAA 150

CAAATAACGC AACTTCCAGA CGTAAATATC CGCCACTCGA TTCCCTCCCC 200

CCCT 204

(2) INFORMATION FOR SEQ ID :984:

(i) SEQUENCE CHARACTERISTICS:
(A) LENGTH: 168 base pairs
(B) TYPE: nucleic acid
(C) STRANDEDNESS: double

(D) TOPOLOGY: linear

(xi) SEQUENCE DESCRIPTION: SEQ ID :984:

ATTTGTATTA GAAAAGTAAA GTTGAAGGAA ATAAAATTTG CATTCTTGTT 50

TTAAGTGATC TTTAATTGTA TATATCGCGG TAGTATGTTC AAAATCCACT 100

AAGTACTGTG ACTTTTAGCT GCAAATCTTT GCTCTTTGCT TTTTTTTTTC 150

TTTCTCCCCC CTCCCCAG 168

(2) INFORMATION FOR SEQ ID :985:

(i) SEQUENCE CHARACTERISTICS:
(A) LENGTH: 350 base pairs
(B) TYPE: nucleic acid
(C) STRANDEDNESS: double
(D) TOPOLOGY: linear

(xi) SEQUENCE DESCRIPTION: SEQ ID :985:

CTCAGCTGGA ATGTCCGGTG CCTGCTAGGT GCATGGCCAC AGAAGCATGG 50

GCTGGGCCTG GGCACAGGAG GAGCAGCTGC TTTGGTCGGG GTGGAGACTT 100

TTTTTTTTTT TCTACCCACA GCCTATTCCA CTCCTCCCCA TCTCCAGGCG 150

CTGGGAGGGG GGCCCTCACC CCGTCACGCC TCGCTCCCTC CTGGCCCTCT 200

GGTCCAGCCC CTTACGCCTC CTCTCAGTCT ACTCAATTGT GACTGTCCCT 250

CCTGATGTAT TTTTTTTTCC TCCAACTCAA AAGGCAAAAA TAATCCTATT 300

TTTATTATCT TGCTACAACT GCAACTTTCT GCCCTATAAA TTTACATTCC 350

(2) INFORMATION FOR SEQ ID :986:

(i) SEQUENCE CHARACTERISTICS:
(A) LENGTH: 289 base pairs
(B) TYPE: nucleic acid
(C) STRANDEDNESS: double
(D) TOPOLOGY: linear

(xi) SEQUENCE DESCRIPTION: SEQ ID :986:

AGCGACCGCC ACTCACTGTG AGTTCCACTT AATTCAGGTG AGCCGCTCTT 50

CTGCACCTCC TCCTCACACC TCTCTACCTA TACTAACCAT TTTTGAAGGT 100

TCAACAATGC TCTGCAAATC TTTTTAAGAC CCGTTACTTC AACAACTCCC 150

AATACCTTCC TCCTCAACGG AACAACTTAA CCGTTACCTT TTAAACCCGA 200

CGAGCTACAA AGCAAGAGAT ACTTCGCGAG CCAAATTCTA CAAAGGACCA 250

AAAAAGAAAC CCATTACCTT ATAGTACTCA TGCAAAAAA 289

(2) INFORMATION FOR SEQ ID :987:

(i) SEQUENCE CHARACTERISTICS:
(A) LENGTH: 134 base pairs
(B) TYPE: nucleic acid
(C) STRANDEDNESS: double

(D) TOPOLOGY: linear

(xi) SEQUENCE DESCRIPTION: SEQ ID :987:

CACAATCATA GCATTTATTA CACGATTTAC ATCTGATCCT CACGCAATTT 50

TCTTCCTCAC CAATAAAGAA CTAATAGGAC AGCATATTAG AAGCAACATA 100

CATTCTTTTA TTCTTGCATC GCTATAAAGA AGTA 134

(2) INFORMATION FOR SEQ ID :988:

(i) SEQUENCE CHARACTERISTICS:
(A) LENGTH: 287 base pairs
(B) TYPE: nucleic acid
(C) STRANDEDNESS: double
(D) TOPOLOGY: linear

(xi) SEQUENCE DESCRIPTION: SEQ ID :988:

TTCTGAAATG AAGGTTTCGT CTTTAGCTAT TGACTGTAGG ATTTGTAATT 50

CAAATCATCA CAGCATCCTA AAGAAATACT GTGTGAATGG AATGCACACA 100

ATTCCTACAG AACACACAAA CTGATGTCCA AAAGGCACAG AGTAATGCTG 150

GTGGCTCTTT CTAGTCAGTT AAGAAACAAT AAAAAGTCTG CATTATTCTT 200

TCATAATTTA AATACTTAAG TAATCTCCAC TTTATTATTT TATAACAATG 250

ACTTCAAATT TACATTATTT TAAGTACCAT TGTAATC 287

(2) INFORMATION FOR SEQ ID :989:

(i) SEQUENCE CHARACTERISTICS:
(A) LENGTH: 177 base pairs
(B) TYPE: nucleic acid
(C) STRANDEDNESS: double
(D) TOPOLOGY: linear

(xi) SEQUENCE DESCRIPTION: SEQ ID :989:

AGCAACCAAC ACTCACTGGA ATCCCGCAAT CAAGAGCTAA AAGGACTTCT 50

TCCATCTCTC AGGGACACAC ATCCACCGAT AAGAATAAAA GAAATGCCTG 100

AATGACTCTC ACTGGCACTT TTAAACACAG CAAGCCCAAC ATATTTTCTC 150

CTTTTCATAA AGAAGCCTAT ACACCAT 177

(2) INFORMATION FOR SEQ ID :990:

(i) SEQUENCE CHARACTERISTICS:
(A) LENGTH: 158 base pairs
(B) TYPE: nucleic acid
(C) STRANDEDNESS: double
(D) TOPOLOGY: linear

(xi) SEQUENCE DESCRIPTION: SEQ ID :990:

TTCCTATACT ATGTCGGTTA AAAGCCTTCA TAGAATATAG GCACCAAAAA 50

TAAACTAACC CCACACCCTC ACCAACCAAA GGAGGCACAA TCCAACGAAA 100

CTGATGAAAA GTCGAGGTAA CGACTCTGCA GACCGTACTC ATTTCTAAAC 150

CAAAAGTC 158

(2) INFORMATION FOR SEQ ID :991:

(i) SEQUENCE CHARACTERISTICS:

(A) LENGTH: 267 base pairs

(B) TYPE: nucleic acid

(C) STRANDEDNESS: double

(D) TOPOLOGY: linear

(xi) SEQUENCE DESCRIPTION: SEQ ID :991:

TAATCCCTTT TAAGCAGAGA TACACATTCA CAGAGAGAGA ATGTTTTAAA 50

AAGACCCACA AGGGGAAGGG ACCAAGTAAG CACTTTTAAT TCATTTTGAT 100

TACACAAAAA TAAGGCAAAC TAAATGATTC AGAACAATTC AATTTAACTG 150

AAGCCTTCTA GAAAAATACT CAACAGGCTT CAACAAAAAG ACTTAGCGCA 200

CATAAAACAA TCACGAAGAT CACAATTTCA GCACAAATAT CTGAAGGAAT 250

ACACGCAAAA CATCATA 267

(2) INFORMATION FOR SEQ ID :992:

(i) SEQUENCE CHARACTERISTICS:

(A) LENGTH: 199 base pairs

(B) TYPE: nucleic acid

(C) STRANDEDNESS: double
(D) TOPOLOGY: linear

(xi) SEQUENCE DESCRIPTION: SEQ ID :992:

AGGATGTGTT CATGCTGGCT TTCAGGATGT CCCAAGGCAC GGCACAGTGA 50

TTGTCAAATT CCACTTTGCA TGGAAGACAG CACAGAACCC TGCTCTGCGT 100

CAGCTGGAGA GCTCCGGCCA GCACGGCCAC CAGCAGCATG ATCAGTGTGA 150

TGCAATACCA GAAGCCGTCC CACCATGATT TTAAGATGTG ATAAGATGA 199

(2) INFORMATION FOR SEQ ID :993:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :993:

TTTAAGGTGC TAAACTTGCA CCTCATGTCC ACTCAGTAAC AAGTATTGGG 50

ACGTAGAGCA CAGCCTCACT CAGCTCTGAA AGGTAATACA GCTTGCGAGG 100

AAGTGAGCCA GCAGTGGCCT TTGCAATTGT GGATCTTGAG CTTTGCTCTT 150

AGCAGATCTC AGGCGTAACC ATTCGCTAAC TGTACTGAAG ATGCGTCC 198

(2) INFORMATION FOR SEQ ID :994:

(i) SEQUENCE CHARACTERISTICS:
(A) LENGTH: 183 base pairs
(B) TYPE: nucleic acid
(C) STRANDEDNESS: double
(D) TOPOLOGY: linear

(xi) SEQUENCE DESCRIPTION: SEQ ID :994:

GCTATCTGCG GGGCCATTCG TAGGATGGGT GAGTCAGATG ATTCCATTCT 50

CCGATTGACC AAGGCCGATG GCATCGTCTC AAAGAACTTT TGACTGGAGA 100

GAATAACAGA CGTGGAATAT TTGTCATAAA TAAATAACGA AAACCTAAAA 150

AAAAAAAAAA ACAACAAACC CCAACAAACT CAA 183

(2) INFORMATION FOR SEQ ID :995:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :995:

ACCTCTGCAT TAACACTCCA GCACCTTTGG TTAGCCAATT TGGCCTTATT 50

TGTGTCTCCA CCATTGGATT ATCTGTCCAT CAAGGTCAGG AATGTTTTCG 100

GGTTACCCCA CTGTCCCCAA CTTCGAATAT GCTCACTGCC TGGAAAATGT 150

TTATCTGAAT ATAAGGCATC AAGCCAGAAC TTGCCCAAAA CTAAATCTA 199

(2) INFORMATION FOR SEQ ID :996:

(i) SEQUENCE CHARACTERISTICS:

(A) LENGTH: 177 base pairs

(B) TYPE: nucleic acid

(C) STRANDEDNESS: double

(D) TOPOLOGY: linear

(xi) SEQUENCE DESCRIPTION: SEQ ID :996:

GGTCTTGGCT GGTGGTTTAT GGCCCGGGCC TTCTTTCTAC GGGGGCCAGG 50

GTCCAATTTT CCACCTGGGG TTTAAAAGTT AAGGAGGACC AGCCTGGGAA 100

CCCCTCGAGT GGGGACGCCA TTTCTATTAC CAGGGCCCCC TTATATTTTA 150

AGGGGACACC ATATGGAGAT TTTATGC 177

(2) INFORMATION FOR SEQ ID :997:

(i) SEQUENCE CHARACTERISTICS:

(A) LENGTH: 229 base pairs

(B) TYPE: nucleic acid

(C) STRANDEDNESS: double

(D) TOPOLOGY: linear

(xi) SEQUENCE DESCRIPTION: SEQ ID :997:

GAGTTTCCTG TGCTGTTACA AATGTAACAT TCTCATATAC AACATTCATT 50

GAAGAGGCAC AATTCTTATT TTGAGGTGGC ACAGATTAGA CAAATTTTTC 100

AGTGAATAAT TCCTCTTTCA TTAGGAAATT TAACCACAAG CTGTATTTTC 150

GTAGAAAGTT GCTTGAAATT CTTTTCCTAA AAAAATTTAA TTTTTCTATC 200

TCAAAATGAC CGTATGCAAA CGAACATTA 229

(2) INFORMATION FOR SEQ ID :998:

(i) SEQUENCE CHARACTERISTICS:
(A) LENGTH: 233 base pairs
(B) TYPE: nucleic acid
(C) STRANDEDNESS: double
(D) TOPOLOGY: linear

(xi) SEQUENCE DESCRIPTION: SEQ ID :998:

TTGCAGTGTT TATGCCCGCC TTCTTTCACG GGCAGGTTCA ATTTCACTGG 50

TTTAAAAGTA AGAGACAGGC TGAACCCTTC GTGGAGCCAT TTCATTACAG 100

GTCCCTTAAT TTAAGGAACA AGTTGATTTA TGCTACCTTT TTGCACGGTT 150

TAGGGTACCG CGGCCGTTAA ACATGTGTTC ACTGGGCCAG GCGGTGCCTC 200

TTAATATCCG GGGATGTCCA GAAGGGGAGT TTT 233

(2) INFORMATION FOR SEQ ID :999:

(i) SEQUENCE CHARACTERISTICS:
(A) LENGTH: 216 base pairs
(B) TYPE: nucleic acid
(C) STRANDEDNESS: double
(D) TOPOLOGY: linear

(xi) SEQUENCE DESCRIPTION: SEQ ID :999:

TCAAAAACTC AATATAATAT GTAAATCAGG GGTGTCCAAT CTTTTGGCTT 50

CCCTAGGCCA TACTGGTGTT AGGCCACATT CAAAGCTATC CTAGGCCACA 100

TGTGGCTGGT GGGCCATGGG TTGGACAAGC TTGATGTAAA TAAAACCACC 150

AGTGGAAAGA GTTTGTTGTG AGTTACATTC AAATACAACA GGAGTAATAC 200

TATCATGATA TTAGTT 216

(2) INFORMATION FOR SEQ ID :1000:

(i) SEQUENCE CHARACTERISTICS:
(A) LENGTH: 280 base pairs
(B) TYPE: nucleic acid
(C) STRANDEDNESS: double
(D) TOPOLOGY: linear

(xi) SEQUENCE DESCRIPTION: SEQ ID :1000:

GACAAGACCT AGAAGCAACA ATGCCCAGTA GAAACAAACA CACCTACTGC 50

CTGGATCTTA GTTTTTAATT ATATTCCCCA ATAAAGAGAC CCAGGGCTCC 100

TTGTTGAAAT GGCTGATTCT AGGACTGGGG CAGGAAATAT GCAAGATGAA 150

CCTAGAATAT CTAATACTGC CAAAAATTAA GAAAGTGTTC TAAAAAAAAT 200

CCCCACGGAT AGGGTGTCAA AAGAGACATA GAAGCCATCT GAAGGAGTTC 250

CCAATGGCCA AAGATCAAAT AATTTTTCAA 280

(2) INFORMATION FOR SEQ ID :1001:

(i) SEQUENCE CHARACTERISTICS:

(A) LENGTH: 324 base pairs

(B) TYPE: nucleic acid

(C) STRANDEDNESS: double

(D) TOPOLOGY: linear

(xi) SEQUENCE DESCRIPTION: SEQ ID :1001:

TGCATGATAG GAGCGTCCTT TGTTCTCATG AGGTGACTCT TGGTGGGCTC 50

CTTATTTGGG GACTGGTCAC CAAAAATACC TAACTATGGT TGGAAGCTTA 100

GTGCTTTCAG CCCCATTCCC CATCCTCTGG AAAGGGGAGC AGAGCTGGAG 150

CTCGATCATG CCTGCGTGAC AAAGCCTCCA GAAAAATCCT TGAAAGACAG 200

GACATGGAGA GGCTGCTGGG TTGGCGAACA CAGCCATGTG CCGGGAGGAT 250

GGTGCACCCC AACTCCACAA GGACCCTTCC AGACCTCACC TGTGTATCTC 300

TTCATCTGGC TGTTCATTTG TATC 324

(2) INFORMATION FOR SEQ ID :1002:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :1002:

ATTTCCATTC ATAAGTCCTC ACAAACCCAC TTTTCCTCTT GCCTGAGTGC 50

CTCACATACC CTACTGCCTG AGATGGTTCC AGAGGCGGTC TCCTCTCTCC 100

CCCAACGAAA CACCAGGCAC ACACCTGCCC CCAGTCTTGC CTGCACTCTC 150

CTTTCCTCCC GCAGAGAGGC ATTCCTTTCT CCTCCTCTCA TCCTACACAC 200

ACTATAAGAG ACAACTGAAA TCTTGCCTCC TCCAGCAAGC TTCTTAATTC 250

ATTT 254

(2) INFORMATION FOR SEQ ID :1003:

(i) SEQUENCE CHARACTERISTICS:
(A) LENGTH: 265 base pairs
(B) TYPE: nucleic acid
(C) STRANDEDNESS: double
(D) TOPOLOGY: linear

(xi) SEQUENCE DESCRIPTION: SEQ ID :1003:

AAGGTTGCGC TGCCCATCCT CTCGCCCTTC GGAATTTACC CCTGGCCGCT 50

CAGGGTCTCG CTTCTAGAAG AAGATGATGG ATACTAGGGC GATCCTCCTA 100

TTGCCTTTAT ATTTAACAGG CCCGCCGAGG AGGGGCTCCC GCTGGTGCCC 150

ACATAACTCT ATTAGACCAT CTGAGGCGAC TTCCCCCACC CCCTCCAACG 200

CGCCTCACGC ACGACTCTAG CAAAAGACAG GTTCCGCGGT GGTCACGTCG 250

TTTGAGACTA TACTG 265

(2) INFORMATION FOR SEQ ID :1004:

(i) SEQUENCE CHARACTERISTICS:
(A) LENGTH: 263 base pairs
(B) TYPE: nucleic acid
(C) STRANDEDNESS: double
(D) TOPOLOGY: linear

(xi) SEQUENCE DESCRIPTION: SEQ ID :1004:

AAATATGCCC TGAGGATTAT CTGATGTCGA TGGTGTGGAA AAGAACTCCA 50

GCAGGCGACT TGGCATTCAA TCAATGTCCC CTGAATGCCA CAGAACCACT 100

AGCAGACGCT GCTCTCTCAG TCTTAATGGA GTGGCCTTCT GGGAACAGCC 150

GAGCTTTGAA AGATGCATAT CAAATGAGTA CAGACACTTG TAGCATTTAA 200

TCAAAGAGCA CCTTGCTAAG AGGCAGCGAA TGCTGGCAGG TGATGGAATG 250

CCCCAGGTGA CCA 263

(2) INFORMATION FOR SEQ ID :1005:

(i) SEQUENCE CHARACTERISTICS:
(A) LENGTH: 317 base pairs
(B) TYPE: nucleic acid
(C) STRANDEDNESS: double
(D) TOPOLOGY: linear

(xi) SEQUENCE DESCRIPTION: SEQ ID :1005:

CGAGACTGCA GTACTTCTAG AAAGTTGTGA ACTTCTAAGA AAGAGCCAGG 50

CTTCCATCTC ACTATTCCTT GATCATTATC TCTGAAGTCC CTACCTGCAC 100

TTCCCTGATT GTCCTGTAGC AACACCAGCA TGGTGGGAAT TGCAGGCAAA 150

GATTGTGAAA AATCCCTAAA GGGTCATTGC TCATGACGTT ATTTCGAATT 200

GTAAAATCGT GAGCCACTCA TATTCAAACT AAAAAAAGAA CAGAACTTTT 250

ATTCACGTTG TGAAGCTTAC TCCCTCGCCA TTTTACGGAT CAATGTCAAA 300

CGCTTGCATT CATAAAG 317

(2) INFORMATION FOR SEQ ID :1006:

(i) SEQUENCE CHARACTERISTICS:
(A) LENGTH: 287 base pairs
(B) TYPE: nucleic acid
(C) STRANDEDNESS: double

(D) TOPOLOGY: linear

(xi) SEQUENCE DESCRIPTION: SEQ ID :1006:

CGGAAGCATT ATCCTGTCAT CAGAAGATGG ACCTTTCCCT GGAATGCCTT 50

TTCCCTATCA TGTCTGTGTG GTAAATACCT ATTCATCCTT AACAACCTAG 100

CTGAGGTCTT ACCTCATCAG GATTTGATCC CCCAAAAAGA TTTGTCCCTC 150

TCTGAGCTAT CTTTATAACT AGTACATACT CCTACCAGCG GACTATGTTA 200

TAATAATGTG GCTATATGCC TGTGATCTCA GCGTGAGCTC TTTGAGGACA 250

GAGTCTTTGT CTAAATACTC AAAACCCTGT TTACCAT 287

(2) INFORMATION FOR SEQ ID :1007:

(i) SEQUENCE CHARACTERISTICS:
(A) LENGTH: 273 base pairs
(B) TYPE: nucleic acid
(C) STRANDEDNESS: double
(D) TOPOLOGY: linear

(xi) SEQUENCE DESCRIPTION: SEQ ID :1007:

GGGCACCAAA ATACACATCA GAGGAGACTG GCTTCCATGA GACGCTTCGA 50

CTGTCTCATC GGGGCACTTG TAATAAGCAT CTTGATGCCA CTGAATGCAA 100

TGCTGTATTC AAATAATAGC TTTCATCTTC ACTATAAAAC AATACAAACG 150

TAAACTTTGA AGCCCTTTGA AGGACCTAAC CAAACAACAC AAGGATGAAA 200

ATAGATCAGT ACAACTTTGA GACACATTAT TAGGTCAAAT CTGCAAAGCA 250

TATTCGGATT TTACCGTAAG GAA 273

(2) INFORMATION FOR SEQ ID :1008:

(i) SEQUENCE CHARACTERISTICS:
(A) LENGTH: 308 base pairs
(B) TYPE: nucleic acid
(C) STRANDEDNESS: double
(D) TOPOLOGY: linear

(xi) SEQUENCE DESCRIPTION: SEQ ID :1008:

TAGAGTGGGG GGCCAAAACC TCTGCAGTCC CCGGCAGTGA GCTATGGACT 50

TTCTTCCCCC TCACAAGGCT GGGGGCCTCC TGCTCTCGTC CCTGGCCCTC 100

CCTGCACAGG GCAAAGCCAG TCTGGGCTAT GGCACACAGA GTTCATGTTT 150

GCGCCCTCTC CCTGCCCCTC ACCCCAGAGG GTGAGGAGGA ATGAGGGGCA 200

TTGATGGTTA GGCCGGTTGT TGCCTCGAAC AGCTGGAGGG AAGCTGCAGG 250

GGTGATAAAA TCAAAGCGAT ATTGACACAC TACAAACATT ATAGAACCTG 300

CAAAAAAA 308

(2) INFORMATION FOR SEQ ID :1009:

(i) SEQUENCE CHARACTERISTICS:
(A) LENGTH: 187 base pairs
(B) TYPE: nucleic acid
(C) STRANDEDNESS: double
(D) TOPOLOGY: linear

(xi) SEQUENCE DESCRIPTION: SEQ ID :1009:

GAACCGCCTC TGTTTCCAGA GCAACTGGTC AAAACCTCTC CAAATACCGA 50

GCAGTTTTGA AAACTGGATT AGGAACCACT AATGAAAAAG AACAGACTCA 100

GGGACAAGTG TTTGGCGGGA ATGTGTGAAG AGGTAACTAA TGAGTGTCAC 150

TGAATCACAC TATTACACAG ATGAAACAAA GCCTTTA 187

(2) INFORMATION FOR SEQ ID :1010:

(i) SEQUENCE CHARACTERISTICS:
(A) LENGTH: 175 base pairs
(B) TYPE: nucleic acid
(C) STRANDEDNESS: double
(D) TOPOLOGY: linear

(xi) SEQUENCE DESCRIPTION: SEQ ID :1010:

AAGAAAAAGA TATTAAGATG GGCAAGTCCT GGTGGCAAGA CAAGCAGATG 50

ACATTACAGA CAATGGACAT CAAACTGCTG CCAAGCTAAT TTAGATTGTA 100

AAAACAGCTC CATAGTCAAT ACCCATGAGT GATCTTAAAT ACGCACAATT 150

AAGCTACTTC TCCTTGATAT TACCT 175

(2) INFORMATION FOR SEQ ID :1011:

(i) SEQUENCE CHARACTERISTICS:
(A) LENGTH: 208 base pairs
(B) TYPE: nucleic acid
(C) STRANDEDNESS: double
(D) TOPOLOGY: linear

(xi) SEQUENCE DESCRIPTION: SEQ ID :1011:

TGAAAAATAT TTGGAACTCT TTAGTACAGA ACAGTTTCAA TATGAGTTAA 50

GAATCATGCG ATGACTACCA AAAAGCTAAT GCGATCCCAG GCTGCGGTAA 100

CAGAAGCAGA GCAGCTCAGC TGGGGAACGT GATAGTGATA ACTACTTCGT 150

ACTAGGTTAG ACTGTACCGA GAGACCTATG TTCAGTTCTG GGAGTGAAAG 200

TATTTTAT 208

(2) INFORMATION FOR SEQ ID :1012:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :1012:

ATGATATCTA GACTACCAGT ATTTTTTGAA TGAGGCTTTC TTAGGAGTAT 50

GTTGATGCAC TCTCATCCTT TCCTCCCCGG ACTCCTACCA CAACCCTCTG 100

ACTGTGGAAT AGCATGGTTG TGTGTAAGGC TGGAGCACAG GCACAGTGAG 150

ATGAGGATGA GGGATTGAGG GATGCTATAA GCCA 184

(2) INFORMATION FOR SEQ ID :1013:

(i) SEQUENCE CHARACTERISTICS:
(A) LENGTH: 263 base pairs
(B) TYPE: nucleic acid
(C) STRANDEDNESS: double
(D) TOPOLOGY: linear

(xi) SEQUENCE DESCRIPTION: SEQ ID :1013:

CTTTGACATT TGATCAGACC AAACAGTGCT GTTTCCCGGG GAGGAAACAC 50

ATTTTAATAA ACACATCATC CCGCAGGCTC CCACCTTTAC CTCATGTTTC 100

ATACCTTGTT TATCAAATGA GCGACTCAAA ATGATTAAAA ATAATGCTGT 150

TCTTTAGTAG CAACTAAAAT GTGTCTTACC GTCATTTATA TCTTTGTCAC 200

CGGAAAGAAG CATTTTTGAT ACTTTACTGC GAGTCAAAAA TCAATACGCA 250

GAATGGCATT TGA 263

(2) INFORMATION FOR SEQ ID :1014:

(i) SEQUENCE CHARACTERISTICS:
(A) LENGTH: 229 base pairs
(B) TYPE: nucleic acid
(C) STRANDEDNESS: double
(D) TOPOLOGY: linear

(xi) SEQUENCE DESCRIPTION: SEQ ID :1014:

CAAATTAAGT GCCAGAATGA TGCAGCCCGC TCTCACCAGG AAGAACGACA 50

TGTGAGAAAA CTTATAGCAG ATGCAAAAAT GTCAACCCAC CATGTTATTT 100

AAAAAAAAAT ATGACCAGGA TACAAGGACA CTTCACCGTT TTTAACCCAC 150

ACAAGGTCAG GTAATGTTTA CCTTGAAACA CAATCGCATA TGACCCTTTA 200

TGCCACTCAC CTAGGCCTTT AATAATGAG 229

(2) INFORMATION FOR SEQ ID :1015:

(i) SEQUENCE CHARACTERISTICS:
(A) LENGTH: 246 base pairs
(B) TYPE: nucleic acid
(C) STRANDEDNESS: double
(D) TOPOLOGY: linear

(xi) SEQUENCE DESCRIPTION: SEQ ID :1015:

GAAAATAATG ATTCATATTA CCGGGTATTT CTTATCCAGT CTTTCATTCT 50

ATGTAAATTG TGTGTGTGTC TATATATGCG TGCATGTCTC TCTCTACATG 100

TATATGTGCA TATGTATGTA TATATATGCA TATGCACATA CATGCCACAC 150

ATGCACATAT ACACACACTC ATAGAGAACA AAATTATTCC GAATATTTCA 200

TGAGGTTTCT TATTATAAGG AGACAATATT GATGCAATCT AATTAA 246

(2) INFORMATION FOR SEQ ID :1016:

(i) SEQUENCE CHARACTERISTICS:

(A) LENGTH: 287 base pairs

(B) TYPE: nucleic acid

(C) STRANDEDNESS: double

(D) TOPOLOGY: linear

(xi) SEQUENCE DESCRIPTION: SEQ ID :1016:

TTCATAATCG CTGGCACTGA ACAAAGTTGC AGAATTCTTT GCCAGGTACT 50

TTAGGAAATC GTGAAGATAA TTGAGTAATA AAGCAAGGCT CTTCTCATCC 100

AGAGGTGTAT AAGCCAACAT CGCTCCAATT CGTACAAATA ATCTCAGGAG 150

ATGTGGCGCT CCATACACCT GGGACATGGG TGCATCGGGA TGATCTGCAA 200

GAATTTCAGC ATACTGTGGT CTCTCAAATT TATAGAGTAG CTGGGTACCC 250

AACATTACGT TGAAGTATTC TTTTATACTC GCCACAA 287

(2) INFORMATION FOR SEQ ID :1017:

(i) SEQUENCE CHARACTERISTICS:

(A) LENGTH: 299 base pairs
(B) TYPE: nucleic acid
(C) STRANDEDNESS: double
(D) TOPOLOGY: linear

(xi) SEQUENCE DESCRIPTION: SEQ ID :1017:

ATATGAGAAA GCAGAGCAAA ACAGATGATG CTCTAAATGC AACTGACACA 50

CTTGCTAAAG ACGTTAGGTG CTCCATAAAT GCTTGTTGAA ATGCTGTTTG 100

TCAACATTTG TTGTGAAAAA TTCATCTCTC CATCTAAGAA TCCTCATGAG 150

TTAACCACAA TCAGTTCTAC ATAATTTAAC AGAAAACATC TGGTGGCACT 200

TTTAAATGTT TAAGGACAGC ATTTCACAAA CTAAGCCCGC CCCCAGTCCC 250

ACTGCAGCAG GAGAAAATAA GGCTATGGAG AAAAGCAAGA TGAGTTATA 299

(2) INFORMATION FOR SEQ ID :1018:

(i) SEQUENCE CHARACTERISTICS:
(A) LENGTH: 274 base pairs
(B) TYPE: nucleic acid
(C) STRANDEDNESS: double
(D) TOPOLOGY: linear

(xi) SEQUENCE DESCRIPTION: SEQ ID :1018:

ATTACTCCTT CTCTCGAATG CTTCCAGAAG AGTAGCACTC ACCACCTCAT 50

GAGGAAAGGC ACTGCAACTT TGGTCAATGA TATCTGTTAG AAAGATCTTT 100

TCCTATATTT AGAAGAGTGA TTCTCATACC TGAGCATGTA TGAGAATCAG 150

TGGGTTTGAG GTGGGCCCAA GAATTTCCCT TTTTAACAAG TTCCCAGAAG 200

ATGCTGATGT GGCAGCTTCT AGCACAGTGC TTGAAAATCC ATGAGTTTTC 250

AAGAGGACAA TCTAAAGTGC TCCC 274

(2) INFORMATION FOR SEQ ID :1019:

(i) SEQUENCE CHARACTERISTICS:

(A) LENGTH: 232 base pairs

(B) TYPE: nucleic acid

(C) STRANDEDNESS: double

(D) TOPOLOGY: linear

(xi) SEQUENCE DESCRIPTION: SEQ ID :1019:

TTTCTTTGTC CTTGACAAAT ATTTATGGAA TGCCGGAAAT TATGGAAAAA 50

CAATTCCAAT GTTACGAAGT CCAAAGAACT GGAATGAGAA AACTCCTCAC 100

GGCACATTAT GCTGCATGGA ATGACAATCA TCCTCGGGGA ATTAGGGCAG 150

CATCTTCTAC CACTTTTAAA GGCTACCACG AGACGGGGTG GAAATGCCAA 200

AAATGGCACC TCATCTTTGA TCAAAGATTC AG 232

(2) INFORMATION FOR SEQ ID :1020:

(i) SEQUENCE CHARACTERISTICS:

(A) LENGTH: 133 base pairs
(B) TYPE: nucleic acid
(C) STRANDEDNESS: double
(D) TOPOLOGY: linear

(xi) SEQUENCE DESCRIPTION: SEQ ID :1020:

AACAGTCCAT GCTGATCTTA GTAACTATGA TGAAGATGGT GCTTGGCCTG 50

TTCTTCTTGA TGAATTTGTT GAGTGGCAAA AAGTCCGTCA GACATCATAG 100

CAAGAACTAT GTGAAGAAAA TGCAAACCTT TAC 133

(2) INFORMATION FOR SEQ ID :1021:

(i) SEQUENCE CHARACTERISTICS:
(A) LENGTH: 280 base pairs
(B) TYPE: nucleic acid
(C) STRANDEDNESS: double
(D) TOPOLOGY: linear

(xi) SEQUENCE DESCRIPTION: SEQ ID :1021:

TCCTAGAACT CTGCTTGGAC CAGAAGATGT GAATAAACTT AAGCTTATTT 50

ATTTAAAATC ACAAATGAGT TGCTCTAGAT TCTAAAAGGG TGAAACTTTG 100

ACTGTTGAAA GTTTAAGTAT TAGTAAACTT GAGTTACTTT TTCTTTCAAA 150

TTTCACTCCG CTTCCCTGCA TTTCGAAGCT GCTCTTTCTG GTCCTACCCA 200

CCACCCCACC AACAAGACTT GTGTTTGTTA ATAGAAATAA TTTATCAAGG 250

TATTGGGGAT CCATTGTCTA TATTTAAAAC 280

(2) INFORMATION FOR SEQ ID :1022:

(i) SEQUENCE CHARACTERISTICS:
(A) LENGTH: 304 base pairs
(B) TYPE: nucleic acid
(C) STRANDEDNESS: double
(D) TOPOLOGY: linear

(xi) SEQUENCE DESCRIPTION: SEQ ID :1022:

AAAATAAATA ACTTCTTAGA TTTTTGACTG AAAAGATCTG AGAATGTTCT 50

GCCAAACAGC CGACCAACTG GTGCAAAAGG TTAAGGCTGA CTTGACTTAG 100

CAACCTGCAG CACAACCAAA AACACTGATG CAGTTCAGAG CTCTTCAAAT 150

GCATACTTCA GTGTTACACA CACATCAATT AAATTAGGTT AAATTAGTTA 200

ATCCTCTAGA CAGTTTTCTT TTTGTTTTGC ATGCATCCCG TTCCATTTTC 250

ATTACGGGCA TCTATTCCTT GATCAATTAT GTGCTTTGCT TTTTAATCGG 300

TTTT 304

(2) INFORMATION FOR SEQ ID :1023:

(i) SEQUENCE CHARACTERISTICS:
(A) LENGTH: 237 base pairs
(B) TYPE: nucleic acid

(C) STRANDEDNESS: double
(D) TOPOLOGY: linear

(xi) SEQUENCE DESCRIPTION: SEQ ID :1023:

TTTCTTTGTC CTTGACAAAT ATTTATGGAA TGCCGGAAAT TATGGAAAAA 50

CAATTCCAAT GTTACGAAGT CCAAAGAACT GGAATGAGAA AACTCCTCAC 100

GGCACATTAT GCTGCATGGA ATGACAATCA TCCTCGGGGA ATTAGGGCAG 150

CATCTTCTAC CACTTTTAAA GGCTACCACG AGACGGGGTG GAAATGCCAA 200

AAATGGCACC TCATCTCTGA TCAAAGATTC AGAGCAT 237

(2) INFORMATION FOR SEQ ID :1024:

(i) SEQUENCE CHARACTERISTICS:
(A) LENGTH: 320 base pairs
(B) TYPE: nucleic acid
(C) STRANDEDNESS: double
(D) TOPOLOGY: linear

(xi) SEQUENCE DESCRIPTION: SEQ ID :1024:

GGCATGATTA TTAGAAACTA TTTAAGCTTT TTTCTTTGAA AAACAAGCTC 50

CTTTTACAGA ATATAAACAA CAGTAGTGCC TGTGGTTTAG CCCACCAATC 100

TTGATGACTA AAAGTAGCTG ATGCATTGTG CATATGATGC TTGAGATGGT 150

TTTTGCAAAA GCAGAAATCG CTGCAAGGTA ATCACAATAG ATAAAAGTGG 200

TATTTTAAAC CTTTGAAATA AATGGATGTA ACTGTACCTT GGTACAGCTT 250

TTCACTTGTT TAGTTTTTAA ACGTTAGTAT AAGATTAGGT AATTGAGGGT 300

TAGAGCCAAC AGGAATCTGC 320

(2) INFORMATION FOR SEQ ID :1025:

(i) SEQUENCE CHARACTERISTICS:
(A) LENGTH: 202 base pairs
(B) TYPE: nucleic acid
(C) STRANDEDNESS: double
(D) TOPOLOGY: linear

(xi) SEQUENCE DESCRIPTION: SEQ ID :1025:

TTCTGGCACC ACATTCAATA CTGATTCACA GGGATCTAAA ACATTCTAGT 50

TATTCAGTCA CTCTGTCCTG TGTGGCAGAT AGGACTTATG TATCTTCAGA 100

ATATTTGAGG AGTCATTCTC AGAGTCTCTC ATGAGTGCTC AACTTAACTG 150

TTCCACAAGT CTGTCTTCAT AGCTGCATGT TGCATCTTCC AGTCTCTGTT 200

CT 202

(2) INFORMATION FOR SEQ ID :1026:

(i) SEQUENCE CHARACTERISTICS:
(A) LENGTH: 243 base pairs
(B) TYPE: nucleic acid

(C) STRANDEDNESS: double
(D) TOPOLOGY: linear

(xi) SEQUENCE DESCRIPTION: SEQ ID :1026:

ATTGGTAGAC AATATCATAA GAGTTTTCTG ATAAAAGATG CTTTTTAACC 50

CTGACTCCCA GTCACAATTT TGGCATCTCA TCCATGGGAA AAAAATAGGA 100

AACTTATACA TTTCATAAAC TAAAGGTCAT CCAAACACTG CCAAAAACGT 150

TTTTATAAAT TGAGACCTAC TTACATCACA TCGGTTTTCC AAAGAGAATA 200

CACAGCGAAA GATGAGGCTC CACCCGGCCG GCACAGTCAT GAA 243

(2) INFORMATION FOR SEQ ID :1027:

(i) SEQUENCE CHARACTERISTICS:
(A) LENGTH: 185 base pairs
(B) TYPE: nucleic acid
(C) STRANDEDNESS: double
(D) TOPOLOGY: linear

(xi) SEQUENCE DESCRIPTION: SEQ ID :1027:

TATTTGCATT GAGGAAATAT TAATTTTCCA ATGCACAGTT GCCACATTTA 50

GTCCTGACTG TAGGAAACAC TGATTTTGTA AAGTTGCCTT TATTTGCTGT 100

TAACTGTTAA CTATGACAGA TATATTTAAG CCTTATAAAC CAATCTTAAA 150

CATAATAAAT CACACATTCA GTTTTAAAAA AAAAA 185

(2) INFORMATION FOR SEQ ID :1028:

(i) SEQUENCE CHARACTERISTICS:
(A) LENGTH: 312 base pairs
(B) TYPE: nucleic acid
(C) STRANDEDNESS: double
(D) TOPOLOGY: linear

(xi) SEQUENCE DESCRIPTION: SEQ ID :1028:

GCTTCTCTAT TCCTCCTGCC CGGGAGGAGT AGAGGAATTC TGGAGCCACC 50

TTAGTGGGCA ATGGGGGAGC TCCTGATGGT TCAGGAGCAG GAAATTTATG 100

AGCAGAGTGG GGTCCCAGGC CACAGGGAGA TGTCTATCCA CAAGGGATGG 150

TGGCCAAGGG TTGAATGGAA AGAGGCAGGC AGGGGGAGAG GGGCAGAGGG 200

TGTGGGTATT CCCAGGGCCT TGAGAGTGGA CATGGCCCCT TCTCCTCAGC 250

CTTCCATTAG CAAGGATGTC ACTGCTGCTC TTGCCTGATG ACAGCCAGAT 300

CTGTTCACAG TT 312

(2) INFORMATION FOR SEQ ID :1029:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :1029:

ATTATTTATA AATTTTAGTG AGCACCACCA AAGTTAGGAT TCAATTGAGT 50

TTGAATTCAA ATTCATTCAA AAGTTGCACA AATTTATTTC TTTTTTCCTG 100

AACATTCCTT TACTTCAGAT TGACAATTCA TTCTATTTAG AGCGCTATTT 150

TAAGAAACCT TGATGAATCG CCCTTGACTA AAGGAAACAA AGTAGAATTT 200

TATACAGATG GAAAAAGA 218

(2) INFORMATION FOR SEQ ID :1030:

(i) SEQUENCE CHARACTERISTICS:
(A) LENGTH: 124 base pairs
(B) TYPE: nucleic acid
(C) STRANDEDNESS: double
(D) TOPOLOGY: linear

(xi) SEQUENCE DESCRIPTION: SEQ ID :1030:

TTATTAGGGG GTTTAGAAAA TAAATTCGTA GGGTTTAAAC ATTGAATAAA 50

ACTACAAAAA AAGACACACA TTCAGGTAGC GAGGCTCTGG GGGTAACTCT 100

TTCTTAGTTC TTTGGACACA TCCG 124

(2) INFORMATION FOR SEQ ID :1031:

(i) SEQUENCE CHARACTERISTICS:
(A) LENGTH: 237 base pairs
(B) TYPE: nucleic acid
(C) STRANDEDNESS: double
(D) TOPOLOGY: linear

(xi) SEQUENCE DESCRIPTION: SEQ ID :1031:

TTTTGCCAAG TATATGTAGT GCAAATATTT TCTTTCAGTC TGTAGCTTGC 50

CTTTTTATTT NTTAATGGGT ATCTTTAAAA AAATGGAAGT TTTAAGTTTG 100

AATGGAATTC AGTTTGTTTT TAAAGCTTGT GCTTTTTGTG TTCAACTAAG 150

GGATATTAGC TTACCATTTT TCTCGGTTGA ATATTATGTG TTTATAGACA 200

AGAAATGGTC ACTAAAGCAA AATAACTGAA AATTATC 237

(2) INFORMATION FOR SEQ ID :1032:

(i) SEQUENCE CHARACTERISTICS:
(A) LENGTH: 147 base pairs
(B) TYPE: nucleic acid
(C) STRANDEDNESS: double
(D) TOPOLOGY: linear

(xi) SEQUENCE DESCRIPTION: SEQ ID :1032:

ACAGCAACCG GCACTCACTT GGAATCCCAG TGTTCCGAGT TCTAAGGCCA 50

CTGAAAACCA TCAAGCGCTT GCCAAGCTCA AGGCCATCTT CGACTGCGCA 100

GCGACTTCCT TGAAGACTGT TTTTAACATA CCAAAGGAGG ACAAGCT 147

(2) INFORMATION FOR SEQ ID :1033:

(i) SEQUENCE CHARACTERISTICS:
(A) LENGTH: 238 base pairs
(B) TYPE: nucleic acid
(C) STRANDEDNESS: double
(D) TOPOLOGY: linear

(xi) SEQUENCE DESCRIPTION: SEQ ID :1033:

AGGCCTGCCT ACCGTAGGTC TCTCAGAGCC TTTACTCTAG TCACTCTTTA 50

GAGGGGGGCA CTAGAAGCAA TATCTCCGAA ACTTCTTCGA CTGTCGAGAC 100

TTTTTACTCA TAATTCGCTT CAAGCCAACT ACGGAGGCGC ATTTACAAAA 150

CTGAAATACG AGCGACACCC CAGGAACGCA CGGAGACATG ACGCCCTTAA 200

TCCTCTGTCC CTACTCCCTC TCCTAGGACA CCGCATTA 238

(2) INFORMATION FOR SEQ ID :1034:

(i) SEQUENCE CHARACTERISTICS:
(A) LENGTH: 63 base pairs
(B) TYPE: nucleic acid
(C) STRANDEDNESS: double
(D) TOPOLOGY: linear

(xi) SEQUENCE DESCRIPTION: SEQ ID :1034:

TCCATGNCTC NTGGTCTCTG TGCNCTTGNC AGGGGCATGC CAGGGCCCTT 50

GGACTGTGCA GGG 63

(2) INFORMATION FOR SEQ ID :1035:

(i) SEQUENCE CHARACTERISTICS:
(A) LENGTH: 180 base pairs
(B) TYPE: nucleic acid
(C) STRANDEDNESS: double
(D) TOPOLOGY: linear

(xi) SEQUENCE DESCRIPTION: SEQ ID :1035:

CGACCGGCGC TCAATGGCGA AATTCCGAGC CAGAGCTATT GGTTGAATGA 50

GTAGCGCTGA TGGTTTAGAT AATAACTAGT ATGGGGATAA GGGTGAGCAG 100

GCGCGCCTTG AGGCAAGAAG CGGGTTAGAG CATCTCTACT TTAAAAGCCA 150

AAGCCTATAA CGGTAGCGTC TACATCACGA 180

(2) INFORMATION FOR SEQ ID :1036:

(i) SEQUENCE CHARACTERISTICS:
(A) LENGTH: 142 base pairs
(B) TYPE: nucleic acid
(C) STRANDEDNESS: double
(D) TOPOLOGY: linear

(xi) SEQUENCE DESCRIPTION: SEQ ID :1036:

CCAAATACGA TGACATCAAG GAGTCGATCC GTCAGCGTGA CTTGAATACA 50

CCACCGCCGA CGACCCGAAA GCCTGATGAG GAGAAGTCCA GAATCAGGAA 100

CAACGGCGAC ACTCGACTAA CCATTTTAAA CCAATTGGAA CC 142

(2) INFORMATION FOR SEQ ID :1037:

(i) SEQUENCE CHARACTERISTICS:
(A) LENGTH: 95 base pairs
(B) TYPE: nucleic acid
(C) STRANDEDNESS: double
(D) TOPOLOGY: linear

(xi) SEQUENCE DESCRIPTION: SEQ ID :1037:

CCACATGGAG TGACCTGGGC CTCTGCAAGA AGCGCCCGAA GCCTGGAGGC 50

TGATTGTCAA AGCAGGGGGC AGGACCCGGG GGCAGGAGAA ACCAG 95

(2) INFORMATION FOR SEQ ID :1038:

(i) SEQUENCE CHARACTERISTICS:
(A) LENGTH: 195 base pairs
(B) TYPE: nucleic acid
(C) STRANDEDNESS: double
(D) TOPOLOGY: linear

(xi) SEQUENCE DESCRIPTION: SEQ ID :1038:

ATTTTATTGT AGTAAGAATA ATACATAGAA ATAACATTAA ATAAATATGT 50

TTGACAAAAA CATACACACA TAGTACAGGT AGAACATATA ACTGGTTGAT 100

GCTAAATAAC AGATCCAGAT AATTTTCAAT TTGAATTAGC AGCAGGGAGT 150

CATTGAATGC TATGCCCTTA GAATGGCTTG CAGGCTTTCA CGAGT 195

(2) INFORMATION FOR SEQ ID :1039:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :1039:

GGTTGTCAAG AAAATGAAAG GTAAAGCTCA GAAGGGATAA GGGAAGACAT 50

TACAGAATTT ATCATCAAGC AGGAGGTTTT TTCCAGGTTC TAAGATGAGG 100

TTAAGTTTCT TAACAAAAGA ATAAACAACT GTGACTCTAT CTGTCGGGGG 150

AGGGTCTAGA CAGAAAAAAT GAATATACCC CAAAAGGCTG AAAAAAAAAA 200

CGAGACTTTG ATGGAGAC 218

(2) INFORMATION FOR SEQ ID :1040:

(i) SEQUENCE CHARACTERISTICS:

(A) LENGTH: 318 base pairs
(B) TYPE: nucleic acid
(C) STRANDEDNESS: double
(D) TOPOLOGY: linear

(xi) SEQUENCE DESCRIPTION: SEQ ID :1040:

TTTATTATTT TGAATGATTT AATGGTTTTC TACACAATTT ACATCACAAC 50

ATGTAAATTT TAGCAGTAAC ATCTGATTCT AACAGCACAT CATGCTATTC 100

CTTTCATAGA GCCTTCAGAG ATTCAATGCT AAACAAATTT CCTTAGTTGG 150

CATCAAGGCA CTGATCACTT TAGAGGCTTT TAAGAAATTA TTTAAAGATG 200

CAAATGCCTC TGAGTGAAGT GTACTATCCC ATCACTGAAG CCCACAGGAA 250

CAAGTCCTAC AATTTTAAAA AGGCTCGATG GAAAAATTTC TCAATCCTGA 300

AATCCCCTAG GGAAGGGG 318

(2) INFORMATION FOR SEQ ID :1041:

(i) SEQUENCE CHARACTERISTICS:
(A) LENGTH: 199 base pairs
(B) TYPE: nucleic acid
(C) STRANDEDNESS: double
(D) TOPOLOGY: linear

(xi) SEQUENCE DESCRIPTION: SEQ ID :1041:

ACGTACCGAG CCGTCGTCGC GGTGCTTGAG CTTGAAGGCG AGATAGCCGA 50

TGCCGCCGAG GCGGTGGACC GCATCACGGT GGCGCTGACG AACGCGAGAT 100

TCAGCAGCAG CGATGCCATC AATAGCAGCC TGATCGGACG CATGACCGAC 150

TCTCCCTGTG AGCGGAGGTT GGGGGGCCGA TCTCCCGCCG GTGCCGGGC 199

(2) INFORMATION FOR SEQ ID :1042:

(i) SEQUENCE CHARACTERISTICS:
(A) LENGTH: 300 base pairs
(B) TYPE: nucleic acid
(C) STRANDEDNESS: double
(D) TOPOLOGY: linear

(xi) SEQUENCE DESCRIPTION: SEQ ID :1042:

GCCTCTAACA AAACTAGGCT GTCCCGGACC ATGGTAATAG AATTGTTTAC 50

CTTTATACCA AGAAGGTTGG GAAAGCACCA AAATCTGCAT GTGGTGTGTG 100

CCCAGGCAGA CTTCGAGGGG TTCGTGCTGT AAGACCTAAA GTTCTTATAG 150

ATTGTCCAAA ACAAAGAAAC ATGTCAGCAG GGCCTATGGT GGTTCCATGT 200

GTGCTAAATG TGTTCGTGAC AGGATCAAGC GTGCTTTCCT TATCGAGGAG 250

CAGAAAATTC GTTGTGAAAG TGTTGAAGGC ACAAGGCACA ATGTCTGGAA 300

(2) INFORMATION FOR SEQ ID :1043:

(i) SEQUENCE CHARACTERISTICS:

(A) LENGTH: 170 base pairs
(B) TYPE: nucleic acid
(C) STRANDEDNESS: double
(D) TOPOLOGY: linear

(xi) SEQUENCE DESCRIPTION: SEQ ID :1043:

GAGACCAGTC TCAAGTGGGA GGGGCTGATG GTGGGAAGCC CTAGAAGAGA 50

GTCTGGGATG AAGCGGCCTC CTCCCTGTCT TGCCCTCCAA AATTGAGTCT 100

GGCCTGATTC CTTTGAGGAG CAAATTTTAC AATCATCCCT CACCCTAACA 150

CACGGTGAAA CTGGAAAACC 170

(2) INFORMATION FOR SEQ ID :1044:

(i) SEQUENCE CHARACTERISTICS:
(A) LENGTH: 260 base pairs
(B) TYPE: nucleic acid
(C) STRANDEDNESS: double
(D) TOPOLOGY: linear

(xi) SEQUENCE DESCRIPTION: SEQ ID :1044:

GGCCCCCCCT GGGGACTGCT GGTTTCCAGG GGCCACCCCC CCTCATCCAA 50

AAGAAGAATC AAGTTTTGTT TTCCTGTGGT TCCCTTGCTC TCCCCGGCCT 100

CACCTCGAGC TTCCACTTGG GCCCTTTCCC ATGCTTCTAC TTGAGCCCCG 150

CCTGCCTCAC CGTCTGCCTC ACCGCCACCT CCTGCTCAGA GTGAGTGCAT 200

GCATGCCAGA CAGATACACA TATACATACA CACACACACA CACACCCTAT 250

TTTTTTTTTT 260

(2) INFORMATION FOR SEQ ID :1045:

(i) SEQUENCE CHARACTERISTICS:
(A) LENGTH: 164 base pairs
(B) TYPE: nucleic acid
(C) STRANDEDNESS: double
(D) TOPOLOGY: linear

(xi) SEQUENCE DESCRIPTION: SEQ ID :1045:

AAATCTATAA TTTTAAGGGC TTAACCTGTG ACTTTAATAA GCTGGAACAG 50

TCCACTGAAT GGGTATAATG AATTGCAGTA TATACGTATG ATCGCTTTTT 100

AAGTGATTAT CTTTTCTTTC GTTAAGTCAT GTAAATTCAT AAATTTTTTG 150

GCACTGATGT GTTG 164

(2) INFORMATION FOR SEQ ID :1046:

(i) SEQUENCE CHARACTERISTICS:
(A) LENGTH: 130 base pairs
(B) TYPE: nucleic acid
(C) STRANDEDNESS: double
(D) TOPOLOGY: linear

(xi) SEQUENCE DESCRIPTION: SEQ ID :1046:

TCCCTCCTTT CCGAACTGGA GCCCCATCCT CTCCAGAGTA TCCAGGGCTT 50

CTTCACTCCC GGGTACCTGC CCTTCGGCCC CTTTTCACCA CAGCTGTGCT 100

ACTGTCAACC AGTCTTTGCT GCATAGGCAT 130

(2) INFORMATION FOR SEQ ID :1047:

(i) SEQUENCE CHARACTERISTICS:
(A) LENGTH: 285 base pairs
(B) TYPE: nucleic acid
(C) STRANDEDNESS: double
(D) TOPOLOGY: linear

(xi) SEQUENCE DESCRIPTION: SEQ ID :1047:

GCTTAACTGG AAATTGGAAG GGAAAAGAAG AAGAAACAAT GTAATGTAGC 50

AGTGGAAGCA AAATTCTCAC AATGAATAGC AGTCTTTCCA GCTTCTTTGA 100

CACGGATTTT CTCATTAGAA AAGACGACTC TCCTGAACTA GGGATGAGTG 150

TGAATCTCCA TTCGAGGAAG GGGAAGAAGA GCTCGGAGGG TGACAGCCTC 200

CCCCTGAGAG AGCCAGTTGC GTTCATGATT TTGATTCCTT CTTCCTCCTG 250

ATGAAGTATC TGCCCAGGAG CCCAGGAGCA GAATA 285

(2) INFORMATION FOR SEQ ID :1048:

(i) SEQUENCE CHARACTERISTICS:
(A) LENGTH: 233 base pairs
(B) TYPE: nucleic acid
(C) STRANDEDNESS: double
(D) TOPOLOGY: linear

(xi) SEQUENCE DESCRIPTION: SEQ ID :1048:

TAAAACTGAC CTAGAAGCTA TCAGCAACCA CAAAGAGACA GAAGAAAAGA 50

CTGCTTCTGA GGCTCTCTCA TGGAACCTAC TGATGATGGT AATACCACGC 100

CCAGAAATAA CGGAGTCGAT GATGATGGCA ATGATGACGG CGATGATGAC 150

GGCACTGATG ACCCCAGGCA CAGCGCGAGT GATGACTACT CAACACGAGC 200

CAGGTCTTTC TGAAGGCCGA GAGAGCTAAA TAA 233

(2) INFORMATION FOR SEQ ID :1049:

(i) SEQUENCE CHARACTERISTICS:
(A) LENGTH: 293 base pairs
(B) TYPE: nucleic acid
(C) STRANDEDNESS: double
(D) TOPOLOGY: linear

(xi) SEQUENCE DESCRIPTION: SEQ ID :1049:

ACTTTAATTT ATTTCGGATG CCGGAATTGT GCCCAGAGTT TCTCCTGAGC 50

TTGATTCCAT AGCTAGCAGC TTCAATCCTT CGCAGCTGCG GTGCGTTCTG 100

AACTCTGAGC TGTCCTTTCC TGACAGGCAC TTTCCATAGC ATCTGCCTGC 150

TTAATTCCTC ACGACTCAGA AATGTTAACG GCACTGTCGA GTGCTGTGAT 200

CATTTTGCTT TAACTATGTA AAGCTTTATG CCCTTCGAGA AGCTTATTTC 250

GAGATAGAGC TAGAGAAGAA AAGCAGCAAA ATTCGGTCGA TAC 293

(2) INFORMATION FOR SEQ ID :1050:

(i) SEQUENCE CHARACTERISTICS:

(A) LENGTH: 219 base pairs

(B) TYPE: nucleic acid

(C) STRANDEDNESS: double

(D) TOPOLOGY: linear

(xi) SEQUENCE DESCRIPTION: SEQ ID :1050:

AAAACTATAC TTTCATTCAA AAGAAGAGCT GAATTGATGC TTGAGTTACA 50

GCACAAATCT ATTATTAGTG AATGAAGTAT ATTCTTGGCA GATAAATACC 100

AGATCAAAAG TCCTGTTTCA GTAATTTGAT TAAACTGTAG AATACTAAAA 150

AATAAGTTAT TTCTACAGAA ATCTTTGAAG GGAGCAAAAA ATCAAATCAC 200

AGTATATACC TAATGGCTA 219

(2) INFORMATION FOR SEQ ID :1051:

(i) SEQUENCE CHARACTERISTICS:

(A) LENGTH: 70 base pairs

(B) TYPE: nucleic acid

(C) STRANDEDNESS: double

(D) TOPOLOGY: linear

(xi) SEQUENCE DESCRIPTION: SEQ ID :1051:

AGCGACCGGC GCTCAAGTGG AATTCCGTGC AGTGTTTGAT TTTTCGTTTT 50

TGCGATAGTT TACTGAGAAC 70

(2) INFORMATION FOR SEQ ID :1052:

(i) SEQUENCE CHARACTERISTICS:

(A) LENGTH: 204 base pairs

(B) TYPE: nucleic acid

(C) STRANDEDNESS: double

(D) TOPOLOGY: linear

(xi) SEQUENCE DESCRIPTION: SEQ ID :1052:

ATTCCTGACC TGTGATATCG GCCATTAGAG CCTTTCAAGA ATGGTACCTC 50

CTGCCGATGA TTTTTTTTAA AGCCTTAATG AAGAGAGTGT CCTCTGACTC 100

CTTCCAGAGA AGATAGGATG TGAGTGAGTA AGTTATGTAT AATAATTTTA 150

TCTCAAAATA ACCACGTCCT TGATACCATT CCCTGTATAC CAAGGAAGGG 200

TATT 204

(2) INFORMATION FOR SEQ ID :1053:

(i) SEQUENCE CHARACTERISTICS:
(A) LENGTH: 252 base pairs
(B) TYPE: nucleic acid
(C) STRANDEDNESS: double
(D) TOPOLOGY: linear

(xi) SEQUENCE DESCRIPTION: SEQ ID :1053:

ACTAAACACA GCACTGAGAA TTCTCCTCTT TTTACAAAAA TGACAGACTT 50

AAGAGAGGGC ATGGAACAGA TTAAGAGAAT TAACACGGAG AGGAGAGCAG 100

AGGTATAAAA GGTATCCAAG AAAAGTAAAA CATACAAAGG CAATTCAAAT 150

CAAAGTGAGC ACTGCCTATA TACAGACAGA AAGCGAGTCC TCTATGAAAC 200

CATACCCGAC AGAAAGTACC GGAATACCAC CGACACTGAT GTCTTCCAAA 250

CG 252

(2) INFORMATION FOR SEQ ID :1054:

(i) SEQUENCE CHARACTERISTICS:
(A) LENGTH: 212 base pairs
(B) TYPE: nucleic acid
(C) STRANDEDNESS: double
(D) TOPOLOGY: linear

(xi) SEQUENCE DESCRIPTION: SEQ ID :1054:

AAATCAGGAA ATTGTACATG TTAACAAAAT AGTATAACCA TGCATACATA 50

CATCTGCTTA TTAAACACTG AAGTAAAAGA ACTAATCCAG TTTGCAGTCT 100

TTAGAGGTGT TTAGTATTTC ACTCCGTTCT GATGGAAGCA CTTTGAATGC 150

TTGCATGGAG ACATAAAACT GAATATTTAA TTGACAATAG ATCATGCGCC 200

TGTATTTATA AA 212

(2) INFORMATION FOR SEQ ID :1055:

(i) SEQUENCE CHARACTERISTICS:

(A) LENGTH: 152 base pairs

(B) TYPE: nucleic acid

(C) STRANDEDNESS: double

(D) TOPOLOGY: linear

(xi) SEQUENCE DESCRIPTION: SEQ ID :1055:

ATAAACTGTG CTAACTAAAG TTTTTATTTC ATCAATATAA AAGCTGTAAA 50

AAAACTGAGG AGAAAGCAGT TGCCAAAAAG ATGAAAGAAA AACAACATAG 100

AAAACCTTTA AAACAGGCAA AAGTCAGTTC ATAATAAAGT AACTCATCTC 150

AC 152

(2) INFORMATION FOR SEQ ID :1056:

(i) SEQUENCE CHARACTERISTICS:

(A) LENGTH: 205 base pairs

(B) TYPE: nucleic acid

(C) STRANDEDNESS: double
(D) TOPOLOGY: linear

(xi) SEQUENCE DESCRIPTION: SEQ ID :1056:

AACAGATCCT CAGTTTCTCT GGAGACACCC CCAATTCTCG GGTGAGTGTT 50

GAAACCTGAC AACTGACTGT TAGCCTTGTC ATCATCCTCA TTGGAGATGG 100

AGATGTTGTA GTCGGAGCCT CATTCCTTCC GGGGCACTGT TTCTTCTAAT 150

GGCAACCATC AGAGCCTGTC TGGAAACAGC CTGCTGCCTT TGCTAGGGGG 200

GCAAA 205

(2) INFORMATION FOR SEQ ID :1057:

(i) SEQUENCE CHARACTERISTICS:
(A) LENGTH: 174 base pairs
(B) TYPE: nucleic acid
(C) STRANDEDNESS: double
(D) TOPOLOGY: linear

(xi) SEQUENCE DESCRIPTION: SEQ ID :1057:

GAGAGAAGAG CGCTTTTCTG GGCTCTTGGT TACTCCGTCA GAGACTTTGC 50

CCTTTGTCTG TTTAAATACA CTGGGAGCCA GAGCTGATTC CCCACCTGCT 100

GCTGTGGTTT TCCGCTTAAC ACAGGAGAGA TGAGTTGGTC TGGTATATTT 150

GATAGCAGGT TTTAAAATGA ATCC 174

(2) INFORMATION FOR SEQ ID :1058:

(i) SEQUENCE CHARACTERISTICS:
(A) LENGTH: 125 base pairs
(B) TYPE: nucleic acid
(C) STRANDEDNESS: double
(D) TOPOLOGY: linear

(xi) SEQUENCE DESCRIPTION: SEQ ID :1058:

TATTTGATGG TCCATAAGAC TTTGTCAAAT GTAAACCTAC AGTTTGATAA 50

GCTTTAAAAT ACCATGTTGA CAGCATTTTG AATTGTTTCA TAGACGTACT 100

TATTTAACTG ATGCGAACAT TCACA 125

(2) INFORMATION FOR SEQ ID :1059:

(i) SEQUENCE CHARACTERISTICS:
(A) LENGTH: 205 base pairs
(B) TYPE: nucleic acid
(C) STRANDEDNESS: double
(D) TOPOLOGY: linear

(xi) SEQUENCE DESCRIPTION: SEQ ID :1059:

TTATCTTACA ATTCTACTTC CCAGCATCCT GAAATTTTCA AATACTGTCT 50

TAGTATATTA AACTCTTTTG AAATAGATAA CAAAAGCACC ATGGGAGTCC 100

GTTGAAAACT TAGGTTTGTT CCTTCTAAAA TTTCTAAGTC CTTCATTATG 150

ACTATAAAGA AAAAAGGTTC ATCAATTCAA AAAAAAATCC TGATTCTTCA 200

AAATA 205

(2) INFORMATION FOR SEQ ID :1060:

(i) SEQUENCE CHARACTERISTICS:
(A) LENGTH: 249 base pairs
(B) TYPE: nucleic acid
(C) STRANDEDNESS: double
(D) TOPOLOGY: linear

(xi) SEQUENCE DESCRIPTION: SEQ ID :1060:

ATCTCACAGA TTCTTTTTCA CAGATTCATT CATGTTGAGT GAAAGAAGCC 50

AGATGCAAGA GTCCACACCA CATGATTCTG TTTCTATACA ACTTAATTTG 100

AAAACTAAGC AATACTTACA GAAGTGAGAA TAGTAGTTGT CTCAGGGTAG 150

GTGAGTGTCA TGAAGGGAAT CTTATACGTT TGCTAAAAAT GTTTTATAAT 200

ACTTGGATTT TGGGTCTATG GGCTGGCAGG GGAAGGGATA CATTGCAAA 249

(2) INFORMATION FOR SEQ ID :1061:

(i) SEQUENCE CHARACTERISTICS:
(A) LENGTH: 200 base pairs
(B) TYPE: nucleic acid
(C) STRANDEDNESS: double

(D) TOPOLOGY: linear

(xi) SEQUENCE DESCRIPTION: SEQ ID :1061:

TAGCACATTG CATCCCAAGA GCTGTAGTCA AAATCCTCAA AATCTGATGA 50

AAATGGCATA CTACACTAAG GAGGATTTTT TGGGCATTTT CCATTCATAT 100

GCAGAATCAG TGGTTGAAAA AGGAGAAAGA GAGATTTTCT TTACAAAAGC 150

TCCTGACCCC ATTAACATCT TATCTATAAA TCTGATATGT TCCTGACTCC 200

(2) INFORMATION FOR SEQ ID :1062:

(i) SEQUENCE CHARACTERISTICS:
(A) LENGTH: 200 base pairs
(B) TYPE: nucleic acid
(C) STRANDEDNESS: double
(D) TOPOLOGY: linear

(xi) SEQUENCE DESCRIPTION: SEQ ID :1062:

GCGCACAGCT GAGAATCTCC GGTCTTGTCT CACTTTGGAC TGGGACAGTG 50

GATGCCCATC TAAAAGTTAA GTGTCATTTC TTTTTAGATG TTTTACCTTT 100

ACAGCCATAG CTTGATTGCT CAGAGAAATA TGCAGAAGGC AGGATCAAAG 150

ACACACAGGA GTCCTTTCTT TTGAAATGGC CACGTGCCAT TGTCTTTCCT 200

(2) INFORMATION FOR SEQ ID :1063:

(i) SEQUENCE CHARACTERISTICS:
(A) LENGTH: 246 base pairs
(B) TYPE: nucleic acid
(C) STRANDEDNESS: double
(D) TOPOLOGY: linear

(xi) SEQUENCE DESCRIPTION: SEQ ID :1063:

TATCAAATCA TGAGTTGAAA GATTTTGACT ATTGAAAACC AAATTCTAGA 50

ACTTACTATC AGTATTCTTA TTTTCAAAGG AAATAATTTT CTAAATATTT 100

GATTTTCAGA ATCAGTTTTT TAATAGTAAA GTTAACATAC CATATAGATT 150

TTTTTTTACT TTTATATTCT ACTCTGAAGT TATTTTATGC TTTTCTTATC 200

AAATTCAAAT CTCAAAATCA CAGCTCTGAA TCTTAGAGTA TCATAA 246

(2) INFORMATION FOR SEQ ID :1064:

(i) SEQUENCE CHARACTERISTICS:
(A) LENGTH: 170 base pairs
(B) TYPE: nucleic acid
(C) STRANDEDNESS: double
(D) TOPOLOGY: linear

(xi) SEQUENCE DESCRIPTION: SEQ ID :1064:

CCCTCATGTG CCAGTTCGGT CTGCCTGCAG AGGCTGTGGA GGCCGCCAAC 50

AAGGGCGAGT GGGAAGCGTT TGCAAAGCCA TGCAGAACAA CGCGCAAGTT 100

TGAGCAGAAA GAGGGCGACA CGAAGGACAA GAAGGACGAA GAGGAGGACA 150

TGGAGCCTGG ACTGAGCCAC 170

(2) INFORMATION FOR SEQ ID :1065:

(i) SEQUENCE CHARACTERISTICS:
(A) LENGTH: 171 base pairs
(B) TYPE: nucleic acid
(C) STRANDEDNESS: double
(D) TOPOLOGY: linear

(xi) SEQUENCE DESCRIPTION: SEQ ID :1065:

CTCTCCCCCA CAGGTTCTCA GCAGTTACTA AGATGTCCCC TGATTTCATT 50

GACCTCTGTG TGTCTTCAGT CCTTGACCCT TTAAGGCTCC CTGGTGCCAG 100

AATGTCTGCA GCTGTAGGAT CAAAGACCCT TGGGGGAAAA ATCCATTCTC 150

AAAAGAGAGG AAGATGGGGT G 171

(2) INFORMATION FOR SEQ ID :1066:

(i) SEQUENCE CHARACTERISTICS:
(A) LENGTH: 201 base pairs
(B) TYPE: nucleic acid
(C) STRANDEDNESS: double
(D) TOPOLOGY: linear

(xi) SEQUENCE DESCRIPTION: SEQ ID :1066:

GAGTCTATAT CCCTTACTCA GTGCAGTTCT CAAAGTCCTT TGTATGTCGT 50

ACAGGATCAC ATCTGTACAT ATCACACTCT TGTGGGGTAA GCCCAGAAGC 100

TCATACACAG CTTCATGGGT TTACTTTCCC AAGCACTTCC TCCCTCCCTG 150

CAATATCTCC CCAGCACTTT CTGCTTCCTA TCAGCTTTCC CCTTTTTCAA 200

C 201

(2) INFORMATION FOR SEQ ID :1067:

(i) SEQUENCE CHARACTERISTICS:
(A) LENGTH: 151 base pairs
(B) TYPE: nucleic acid
(C) STRANDEDNESS: double
(D) TOPOLOGY: linear

(xi) SEQUENCE DESCRIPTION: SEQ ID :1067:

ATCAACCGGA GGTGAAATGA AATACCGTCA ACCTGTGCAT GAGTTTGTGT 50

GAACTGAATG TGTGTGCATA CACATAAGCA TGTGTGCGAG CGCAAATGTG 100

GCGTCTCCAC AGCATGCGCA ATCCATGAGT CTGTTTCTAA CGCGCGCGCG 150

C 151

(2) INFORMATION FOR SEQ ID :1068:

(i) SEQUENCE CHARACTERISTICS:
(A) LENGTH: 273 base pairs
(B) TYPE: nucleic acid
(C) STRANDEDNESS: double
(D) TOPOLOGY: linear

(xi) SEQUENCE DESCRIPTION: SEQ ID :1068:

ACTGTGTCAT GTCAGGTTAG ATCTGAATCT CTAGGAGAAT AGCTTTTTTG 50

ATCTGTTTGC TCCTTTGAGT CTCCCTTTTA CTTTACAACT AGAAAACCAA 100

TCGCATACTT CACTTCTATC GCTTCCAGAT ATGGCATATT TTTCTTAGGC 150

TACCGTTGAA ATTTAGCATA ATATTTCTCC CTTACCTTTA CGTCCTTTTA 200

AAAAAATAGG ACTATAGTAA GTCCTCCGTG TCGGTGGCAT GTTTTTGGAA 250

ACTGACTTTA AAGAAAACAT ACA 273

(2) INFORMATION FOR SEQ ID :1069:

(i) SEQUENCE CHARACTERISTICS:
(A) LENGTH: 189 base pairs
(B) TYPE: nucleic acid
(C) STRANDEDNESS: double
(D) TOPOLOGY: linear

(xi) SEQUENCE DESCRIPTION: SEQ ID :1069:

CAATCTTGCT TATAAAATAA GAACACCTTT CAATTAAGTG AGTGGGTCAT 50

TCCTGGTGCA ATTGTGATTT TTTTTTAGCC AAAATGAATG GCAAACTCTA 100

TTTAGAGCAA AGTAAGTATT AGAAAACCCT AGGAACTCTT AATCAACGTT 150

TATTACACTT TTATAAAGGC AAACTACGCG AAAGAGCCC 189

(2) INFORMATION FOR SEQ ID :1070:

(i) SEQUENCE CHARACTERISTICS:

(A) LENGTH: 280 base pairs

(B) TYPE: nucleic acid

(C) STRANDEDNESS: double

(D) TOPOLOGY: linear

(xi) SEQUENCE DESCRIPTION: SEQ ID :1070:

TTTATTCCAC TGGAGAAAGG GGATTGAAAA TCAGTTAGAA CCAATGTATT 50

TCTTGCCCCA CGGAACACTA TTCCTATAAG ACAGCTGAAA GAAGCTGCCG 100

TGAGGAGCTC AGCTCCAAAC ACAGGATCAG CACCTCGCAT AGGAATTCCC 150

ATGAATCACG ACTTCTCATC CCGTTTTATC AGAGTGCATA TACGTCCTAC 200

TTAAGGAAAA GTAAAACAGT CATTTACGAA AGAAAGTCAA TCTGTATCCT 250

AAGCATTTTA ATAAAAAGTC AGAAGGAATT 280

(2) INFORMATION FOR SEQ ID :1071:

(i) SEQUENCE CHARACTERISTICS:

(A) LENGTH: 193 base pairs

(B) TYPE: nucleic acid
(C) STRANDEDNESS: double
(D) TOPOLOGY: linear

(xi) SEQUENCE DESCRIPTION: SEQ ID :1071:

AGAACCGAAG CCTGCAGAGA GGCCATTCTG ACAGGCTGGG AAATGTAGCC 50

TACCGCCAGA AGCTAGAAGC AAGCACTTCC TCCTAACCGA GTTTTTGAAA 100

GACTAAGGGA AAGAGAATAG AGAAGGGATA TTCCCATCTA CCGGGGAAAA 150

GATGCGCAGT AACAAACCAT CTAGGGAGAC AGCCTTCTAA CAG 193

(2) INFORMATION FOR SEQ ID :1072:

(i) SEQUENCE CHARACTERISTICS:
(A) LENGTH: 175 base pairs
(B) TYPE: nucleic acid
(C) STRANDEDNESS: double
(D) TOPOLOGY: linear

(xi) SEQUENCE DESCRIPTION: SEQ ID :1072:

AAAGCACCTT CCCCTCGCTG AGCTCCAGGG GGCCCAGATA GGCCACGCGC 50

AGCTCCAGCA TCTTGGAGAG CTGCACGTCA TCTGCCGCGT CGTAATCCTC 100

CACTCTGCAG CCATAGGCCC CACTCTGGCC CCGGGTCACT CCCTCCAGGG 150

TCAAGTTCCC CTCGAGATTC ACATC 175

(2) INFORMATION FOR SEQ ID :1073:

(i) SEQUENCE CHARACTERISTICS:
(A) LENGTH: 339 base pairs
(B) TYPE: nucleic acid
(C) STRANDEDNESS: double
(D) TOPOLOGY: linear

(xi) SEQUENCE DESCRIPTION: SEQ ID :1073:

AGTGCCCTAC TCTTTTCCCA AGAAGGGTCA AAGCCTACAA TATCATCAGG 50

GGGCATGAAG CACATTAATT TGCAGTGGCT GCTTCATATG AGGAGGTATG 100

GTGGACAGGC TAATTTTTCC TTGAAAATGT GGCTTCTTCA ACTCCTTTCA 150

AATTTAGGAT GGAATACTTC CTGAAATAAA AACTGGGCTT TATGCAGGAT 200

TCTCTTTGAA AATTCTTGTA TGTCCAGAAC AAAAGATAAA ACTAATTGTA 250

TTCCTCACAT TCACAATCCC CATTGGTCTG AAGTCACGTG ACACAGAGCA 300

TCTATATAGC ACATAGTGTT TAAAGACTAA TGAATGCAA 339

(2) INFORMATION FOR SEQ ID :1074:

(i) SEQUENCE CHARACTERISTICS:
(A) LENGTH: 172 base pairs
(B) TYPE: nucleic acid
(C) STRANDEDNESS: double
(D) TOPOLOGY: linear

(xi) SEQUENCE DESCRIPTION: SEQ ID :1074:

ATTAATTATG CGGTCCTCCT CCTGGCAGCT GGACACCAGT TTGAATCTTC 50

CTTTGAGCTC CGGAAAGTTG GTAATTACAA CTTGATATTT CTACATGGAA 100

ATCAAGAAAC TCGGACCCAA CTTGGTGCAA AGACGGATCT CCGCCGATTC 150

TGACGGCTCT CCAGGTTTTG TC 172

(2) INFORMATION FOR SEQ ID :1075:

(i) SEQUENCE CHARACTERISTICS:
(A) LENGTH: 299 base pairs
(B) TYPE: nucleic acid
(C) STRANDEDNESS: double
(D) TOPOLOGY: linear

(xi) SEQUENCE DESCRIPTION: SEQ ID :1075:

AGAGTAGGAA GAGGGAGGGG AACAGGCATC TAAGATAGAC TTTCTCCATC 50

TGTTGGGAGC CTGGGCAAGT AGGAAAGAAG CCTTTTTCAT ACACTTCAGT 100

CGTATGACTG AGCTGCTTGG CAGGGCACTG GAAACGACGA ACTCGCGCAG 150

CCTGCAAATG AGACCACTCT CATTTCTCAG TTTCGATTTG ATTCACTCAC 200

TAGTAGTTAG GTAAATACGA GCTCTATGTG ACTCAAGGAA TGTCAGGCTG 250

GGGCAGGTGG CAAAAGCTAC AGTGATCGAA ATTCACGTTG CTACTGATG 299

(2) INFORMATION FOR SEQ ID :1076:

(i) SEQUENCE CHARACTERISTICS:

(A) LENGTH: 283 base pairs

(B) TYPE: nucleic acid

(C) STRANDEDNESS: double

(D) TOPOLOGY: linear

(xi) SEQUENCE DESCRIPTION: SEQ ID :1076:

GCCCTCATCG GCGATACTGG AGGTTCGCTT TCTCAACCCT GTGGGCTTCA 50

CCCCAGACAC GCCCTGAATC GTTTCATGTT CCAGCATGCC TGAGACAATC 100

ATCGACTGAA GGATGTTCTC TAACACCCGC ACGAGCTGCC GGCAGATCTG 150

AATGCCCAAG TCACTCAGCA CCTGCCGATA CTCAGCCAGG TCAAAATCGG 200

CGAGGTAGTG CTCATGCCTG GCGAGACGTG TTGTGCTTAA TGAAGCCCTA 250

TCATTCGAAG TATTGCTATG AGCAGTGCGA AAA 283

(2) INFORMATION FOR SEQ ID :1077:

(i) SEQUENCE CHARACTERISTICS:

(A) LENGTH: 297 base pairs

(B) TYPE: nucleic acid

(C) STRANDEDNESS: double

(D) TOPOLOGY: linear

(xi) SEQUENCE DESCRIPTION: SEQ ID :1077:

ACCAATCAAG TACACTAAAT TAGAATATTT TTAAAGTATG TAACATTCCC 50

AGTTTCAGCC ACAATTTAGC CAAGAATAAG ATAAAAACTC GAATAAGAAG 100

TAAGTAGCAT AAATCAGTAT TTAACCTAAA ATGACATATT AGAAACAGAA 150

GATATTACGT TATGCTCAGT AAATAATCAA GAGATGGCAT CGCGTAAGAA 200

GGAGCCCTAG ACTGAAAGTC AAGACATCTG AATTTCAGGC TGGAAAACTA 250

TCAGTATGAT CTAAGCCTCA GTTCTCTNGT CTGCAAAATG AAAGCAC 297

(2) INFORMATION FOR SEQ ID :1078:

(i) SEQUENCE CHARACTERISTICS:
(A) LENGTH: 291 base pairs
(B) TYPE: nucleic acid
(C) STRANDEDNESS: double
(D) TOPOLOGY: linear

(xi) SEQUENCE DESCRIPTION: SEQ ID :1078:

AATCTTTTAA TCAATCGAAA CCTTAATAAT TAGGACTGAT AATAGTGATC 50

AGTGATTGAA CCTTTATTAT TTACCAGCTA ATATAGTAGG CCCATAAATA 100

GGTTATATCA TTCGTAGTCA CAATAACCAC ATACATCACA CACCATGACG 150

TAGCGCTACT ACCCATACCT GCTAGCAGTC AAGGTTCAGA TAATCATAAC 200

ACGCCCAACA TCTCATTGAT AGTGAGTTAT CCAAACAGGA ACAATCTCAA 250

GTTTATATCT AAAGCCCATG TTCTTTTGCT TAATATTAGA G 291

(2) INFORMATION FOR SEQ ID :1079:

(i) SEQUENCE CHARACTERISTICS:
(A) LENGTH: 136 base pairs
(B) TYPE: nucleic acid
(C) STRANDEDNESS: double
(D) TOPOLOGY: linear

(xi) SEQUENCE DESCRIPTION: SEQ ID :1079:

AGTCACAGCC CCTATACTCC CTCTACACAT TAACCACAAC ATAAGCGGGG 50

CTCACTCACC CACCACATTA ACACCACGAA ACGGGTAATC TAACACGAGA 100

AAACACCCTA ACGTTCATAC ACCCCATACA CCATTA 136

(2) INFORMATION FOR SEQ ID :1080:

(i) SEQUENCE CHARACTERISTICS:
(A) LENGTH: 300 base pairs
(B) TYPE: nucleic acid
(C) STRANDEDNESS: double
(D) TOPOLOGY: linear

(xi) SEQUENCE DESCRIPTION: SEQ ID :1080:

CCAAGGTCGC CTGTGTGCTA GTCACGACCA TTAGGATCCG AAGAAGAGAT 50

GATGACTACC CTAAAAGTAA GGTCTGTGAA CAGCCTGTAA TGGGACGTGA 100

GCTGCGGTAT TAAAAAAATC AAGAATGGAA GCGTTCCTCA ATTGTCGTTG 150

AGAATCATAC CAACACGGCA GATGCAACCA TGAAAAACGC AGCTGCGTAT 200

CGCGCGTTTT TCATGGTTCG CATCTGCCGT GTATGCAATG ATTCTCAACA 250

CACATTGGAG TCACGCGGCC TGCGTAAGAG TATGAAGGTC TTCCTGTAAA 300

(2) INFORMATION FOR SEQ ID :1081:

(i) SEQUENCE CHARACTERISTICS:
(A) LENGTH: 219 base pairs
(B) TYPE: nucleic acid
(C) STRANDEDNESS: double
(D) TOPOLOGY: linear

(xi) SEQUENCE DESCRIPTION: SEQ ID :1081:

GTTAATGGTT TTTTCATACA TATGAGACTG AGCGGTCAAA GTTTTACTAA 50

AGTCTTTATG ATCTTTAATT AATACTCCAA GACATCCGAG TTTGGCAGAT 100

ATTGGATGAG ATTTGTTTTG CTCCTTACTT TCTTTGTATA CAGCCAAGCC 150

TGAAAATCTC TAGACATTTG CTGAAATTGA TGAGAGCATG CACAGGACGA 200

CTGAAGGTGC TGGACATGA 219

(2) INFORMATION FOR SEQ ID :1082:

(i) SEQUENCE CHARACTERISTICS:

(A) LENGTH: 360 base pairs
(B) TYPE: nucleic acid
(C) STRANDEDNESS: double
(D) TOPOLOGY: linear

(xi) SEQUENCE DESCRIPTION: SEQ ID :1082:

ATTTTGGGAA AGTGAGCATG AAGAAGGGAC TCAGAGCTGC CAGGTACCCG 50

AGTCTCAGGC TCTGACATCT CTTGGGGCCC CCTTCTGCCA CAACTTCTTG 100

CCACTTCACG GACTTGAATT ATGTCCCGCG CCTAAAAAAA AGATTGGCCT 150

GCAGCACAGG CGTGTATCCT CTCCAAAAAG CTGCTGCTCA TTTCTGGCCC 200

CATCTGCTGC TATCTTTGCC AGTCAGCCAT CACAGTTTGG CATCATCAGA 250

GTCTCAGCTA GAGGGAGCGT GTCATTTCTG CCTTAGTGGT TTGCCTGCGA 300

CCTGACCAGG GCAAGACCAG CTGGGATGTC AAGTGACATT TTGGGAACTT 350

GTCGGAAGTT 360

(2) INFORMATION FOR SEQ ID :1083:

(i) SEQUENCE CHARACTERISTICS:
(A) LENGTH: 250 base pairs
(B) TYPE: nucleic acid
(C) STRANDEDNESS: double
(D) TOPOLOGY: linear

(xi) SEQUENCE DESCRIPTION: SEQ ID :1083:

CATACATATG AGACTGAGCG GATCAAAGTT TTACTACAAG TCTTTATGAT 50

CTTTCAATTA AATACACTCC AAGACATCGA GTTTGGCAGA TATTGCGCCC 100

GCCGTTTTGC TCTTCTTATC TTTGTATCCA GCCAAGCCTG AAAAGTCTCT 150

AGACATTTGC TTGAAATTTG ATGAGAGCTT GCACAGGCCG ACTGAAGGAT 200

GCCGGACATG ATTCTCTGCT TTCTGGTTCA ACAAAAAAAA AAAGTTCCAG 250

(2) INFORMATION FOR SEQ ID :1084:

(i) SEQUENCE CHARACTERISTICS:
(A) LENGTH: 261 base pairs
(B) TYPE: nucleic acid
(C) STRANDEDNESS: double
(D) TOPOLOGY: linear

(xi) SEQUENCE DESCRIPTION: SEQ ID :1084:

TNCTAGATAC NTCCCTCTGT CTCACATTCA TAGTGTGTTC TTGTCTAGTG 50

TATGAAGTCT CACNCAAGGA AAGTGTNCAG GCAGAGTTTG GGGAGAGTGA 100

AAAAAGTGAA TGGCCTATAT NTGCTCTNTT GGGTGNTCCA NCCATTATTG 150

GGGTGAACCC TTTCAGCTTT TTCCTAAAAA TTTGAAAAGA NTTTTTTAAA 200

AAATTAAAAA GAGGATTTTT AAAAGGAATA TCTTGTTCAA AATTTNTTAA 250

GTTTTAATAG G 261

(2) INFORMATION FOR SEQ ID :1085:

(i) SEQUENCE CHARACTERISTICS:
(A) LENGTH: 260 base pairs
(B) TYPE: nucleic acid
(C) STRANDEDNESS: double
(D) TOPOLOGY: linear

(xi) SEQUENCE DESCRIPTION: SEQ ID :1085:

GCAATCCATA AGTGTCCTTA TCTACAAAGT GAAAAGTTGG ACAAGATATT 50

CTTCATGATC TTTTTCGATT TTAAAATGTC ATGCAATTTT AGAGAAAAGC 100

TGAAGGGTCA CCCAATATGG CGCGACACCA AAAGACAAAT ACGAGGACAA 150

TCACTTTTTC ACTTTCCGCA AACTATGCCT GGACACTTCC TTGGCGAGAC 200

TTAATACACC AGACAAGAAC ACACTATGAA TGCGAGACAG AGGGAGACAG 250

AGGCGAAATA 260

(2) INFORMATION FOR SEQ ID :1086:

(i) SEQUENCE CHARACTERISTICS:
(A) LENGTH: 213 base pairs
(B) TYPE: nucleic acid
(C) STRANDEDNESS: double
(D) TOPOLOGY: linear

(xi) SEQUENCE DESCRIPTION: SEQ ID :1086:

AAGACGTGGA TTTGCTGGAA GACACGGATT TCCTGGAAGA CCTGGATTTT 50

TCGGAAGCTA TGGATTTGAG GGAAGACAAG GATTTTCTGG AAGACATGGA 100

TAATCTGGAA GACATGACTT TGTCGGAAGA CGTGGACTTG CCGGAAGACA 150

CGAATTTCCT GGAAGACCCG GATTTTTCGG AAGCTATAGA TTTAAGGGAA 200

GACAAGACGG ACT 213

(2) INFORMATION FOR SEQ ID :1087:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :1087:

TCCCAGGCAC CCGCTGAATT TCTGAGGCCT TGCTTAAAGC TCAGAAGTGG 50

TTTAGGCATT TGGAAAATCT GGTTCACATC ATAAAGAACT TGATTTGAAA 100

TGTTTTCTAT AGAAACAAGT GCTAAGTGTA CCATATTATA CTCGACGTCG 150

ATCATTTCTC AGTCCTATTT CTCAGTTCTA TTATTTGAGA ACCTAGTCAG 200

TTCTTTAAGA TTATAACTGG TCCTACATCA AAATAATAGA AATTACGTTT 250

TTTTTT 256

(2) INFORMATION FOR SEQ ID :1088:

(i) SEQUENCE CHARACTERISTICS:
(A) LENGTH: 264 base pairs
(B) TYPE: nucleic acid
(C) STRANDEDNESS: double
(D) TOPOLOGY: linear

(xi) SEQUENCE DESCRIPTION: SEQ ID :1088:

TTGCTTAGAA CGGAGCTAGC AAGACAAAAA TACTTCAGTT GGCATCTCCC 50

TTAAAGCACA TCCCCAAACG CGGGTCCTGG CCCCAGACAG GGAGACCAGG 100

GCTCTGACAG TGACAGGTTC TTCCCTCTGA AAAAAGAATA GAAGAGGAGC 150

TCCTCCCTAA CCCACAGATT CCCAGGGCAG ACCCTGGGAG GAGGTGCTGA 200

AACACAGAAG AGAGTGTGTC TTCCCCAACC TCTACCAGAC AGTAGAGAAA 250

CTGAGGCGAG AGGC 264

(2) INFORMATION FOR SEQ ID :1089:

(i) SEQUENCE CHARACTERISTICS:
(A) LENGTH: 157 base pairs
(B) TYPE: nucleic acid
(C) STRANDEDNESS: double
(D) TOPOLOGY: linear

(xi) SEQUENCE DESCRIPTION: SEQ ID :1089:

GACCTGGAAT TTGTTTTCGT CTGTTCAGTA GACTCCGATT TCAGAATATG 50

TCGGAATTTC AGACTCCGGA ACTTTTGAGA ATGTCATTAC AGGAACTTTG 100

TTTACATACC GAGCTGTTAG CCCCAGTCAC TGGGTCCGAA ATTACGCCGA 150

TACCGAC 157

(2) INFORMATION FOR SEQ ID :1090:

(i) SEQUENCE CHARACTERISTICS:
(A) LENGTH: 366 base pairs
(B) TYPE: nucleic acid
(C) STRANDEDNESS: double
(D) TOPOLOGY: linear

(xi) SEQUENCE DESCRIPTION: SEQ ID :1090:

GCATATACCA AAAAAGGACA AACAGATAGA AGAGTCATCT CAGTGTGTAA 50

CGAGGAAAAG ATTTATCTCC CATCCTCTGA TCCTCCATAT GATATCTGAT 100

AGCGAAGATG ACAATAACCC CTTGACTCTT CACGAAAACT CGATGAGAGA 150

GAAACTTGAA AAGTCAGAAA ATAAGTTCAT ACCGCAATTA ACCTTTTGGT 200

CAAGTAATGG AAACTTTTGA CTACTAGTGT AAGTCAAAAG ACAAAGGACT 250

CCAGCAAGAT GAGAGATTTC TCTATTTTGT AACGAGTAGT CCACCGATCG 300

TCATCAAAGA GAGAGAATGA ACAACTACGA AAGTTTAAGG GAAAAAAAAA 350

AAAAAACATA GATGTT 366

(2) INFORMATION FOR SEQ ID :1091:

(i) SEQUENCE CHARACTERISTICS:
(A) LENGTH: 305 base pairs
(B) TYPE: nucleic acid
(C) STRANDEDNESS: double
(D) TOPOLOGY: linear

(xi) SEQUENCE DESCRIPTION: SEQ ID :1091:

GGTCTGAATC ATGAAAAAGC CCATAAGAGA GATACAGTGA CTCCAGTTTC 50

AATCATATTA CAGGAGATGA ATCTGTTGCT CTCCCATTTT GCTCTTGTCA 100

CACCATATGA AGACATCAAA AAACACTTAA GGATTCGAGA AAGAGAACAT 150

CTTTTGTTAA AGAAGAGAAT AAGATTTTGG AAGAAAAAGT AATAGCAAAT 200

TTGAAGAAGA AACAAGTTAC GTAGGGACGA GAACAAGTAA ATAAGGCCTA 250

TCATGCATAT CGAGAGGTTT ACATTGATAG AGATAATTTG AAGAGCAAAT 300

GGACA 305

(2) INFORMATION FOR SEQ ID :1092:

(i) SEQUENCE CHARACTERISTICS:
(A) LENGTH: 237 base pairs
(B) TYPE: nucleic acid
(C) STRANDEDNESS: double

(D) TOPOLOGY: linear

(xi) SEQUENCE DESCRIPTION: SEQ ID :1092:

AAATGGAAGT TGAACTGAGT GTGGTTTCCA GTACAGGGCA TCTGGCCACC 50

TTCACCTCAG TAAATACTGC TGATCGACTC TCTCTGGCTC TGGCTGTCCT 100

GCCAGAGACA GCCAAACACA GGAGACATGA CAAATTACTA TCATCTTTTG 150

CTTCTTTATT TTTATTATTA TTATTTTTCT TTTTGTGGAG AACAGCGTCT 200

TGCTGTATTG CCCAGCCTGA TCTCAAACTC CTGGGCG 237

(2) INFORMATION FOR SEQ ID :1093:

(i) SEQUENCE CHARACTERISTICS:
(A) LENGTH: 420 base pairs
(B) TYPE: nucleic acid
(C) STRANDEDNESS: double
(D) TOPOLOGY: linear

(xi) SEQUENCE DESCRIPTION: SEQ ID :1093:

AGAAATTTAT TGCCTCACAG TTCTGGAGCC TGGAAGTGCA ATATCAAGGT 50

GGAGACAGGG TTGGTTTCCT CTGGAGGCCA TAATGAAAGC ATCTGTTCCA 100

GGTCTCTGTC CTTGGCTTGT AGAAACACGC ATTCTTCTGG TGGATCCACA 150

CAGTCTTCTC CATGTGTATC CTTCCTTCAG TTTCCCTTAT AGGACACCAG 200

TGATGTCAGA TTAGGGATCG AACCCAACAA CCTCATTTGA AGTTACTCAC 250

CTGTTTGAAA ACCCTATGTC CAAATACAGT TATAGTGTAA GGAACTAGGA 300

CTTAGGGCTT ACAAATATGG AGTGGGGCAT CATCCAGACC ATACCATTTA 350

AATTGCAGGG TTTCTCTCCA ATGTGAGTTA CATACACACA TTAAGGTTTG 400

TGGGATTCAG AAAGGTATAC 420

(2) INFORMATION FOR SEQ ID :1094:

(i) SEQUENCE CHARACTERISTICS:
(A) LENGTH: 192 base pairs
(B) TYPE: nucleic acid
(C) STRANDEDNESS: double
(D) TOPOLOGY: linear

(xi) SEQUENCE DESCRIPTION: SEQ ID :1094:

GTTTCTTGGG TAACCTCCCC TCCTTAATCC TTCTGGCATA GGCAGACTCA 50

CAGCAGGTAC CCAGTAGAAG GTAAGGTGGC TGATCATTTT TCCATAGGAG 100

GTCTCCATGG CACACAGGGT AGAAAGTGCA TTCTGTGAAT TAGAGGCAGC 150

AGAGCATGGC AGCTAATGGA AACTGGCTCT GGGAGGTCAA AT 192

(2) INFORMATION FOR SEQ ID :1095:

(i) SEQUENCE CHARACTERISTICS:
(A) LENGTH: 228 base pairs

(B) TYPE: nucleic acid
(C) STRANDEDNESS: double
(D) TOPOLOGY: linear

(xi) SEQUENCE DESCRIPTION: SEQ ID :1095:

AGAGAGCTTA ATAAGACCTA ACTACAGGCA AACCTGACTG GAACGTGAGT 50

ATCTGTGGTC TGGTGTGGAA ATAGCTCTCT AGCCCCATCT CCCTCCTCCC 100

ATTGTCCACC CCCATCCTCC TTACCACGAA ACACATTTTT CACCAGCATT 150

GCCAAACCAC TTGGGATTCC TGCGCATGGC TTTGCTTAGT TTGCTCTTCT 200

GCTTCTTGGA TACCTCCTTC AGGAAATC 228

(2) INFORMATION FOR SEQ ID :1096:

(i) SEQUENCE CHARACTERISTICS:
(A) LENGTH: 198 base pairs
(B) TYPE: nucleic acid
(C) STRANDEDNESS: double
(D) TOPOLOGY: linear

(xi) SEQUENCE DESCRIPTION: SEQ ID :1096:

ATTTTTTATG GAAAAAGGGG GATAATGCAA AATAGCAAAA ATTGTAAACA 50

AAGTTAAAGA TTATCTTCAT CTAAACCTTA AGCTGACAAT TTAAAACACC 100

TTGTGCATAA TGCAGAAAAA AACTGTCCGT TTGACTTCAC TTTCATTCAC 150

ACTGCTCCCT ACGCATGAGA AAGACCAGAT GTCTGGTTTC AGAGTATT 198

(2) INFORMATION FOR SEQ ID :1097:

(i) SEQUENCE CHARACTERISTICS:
(A) LENGTH: 118 base pairs
(B) TYPE: nucleic acid
(C) STRANDEDNESS: double
(D) TOPOLOGY: linear

(xi) SEQUENCE DESCRIPTION: SEQ ID :1097:

TAGAGAGAAG TTGATATATG AAGATAGGGT GGACAGGACT GTGAAGGAGG 50

CTGAAGAAAA ACTGACTGAA GTGTCACAGT TTTTTTCTAC AAAACTGTGA 100

CACTTCAGTC AGTTTTTC 118

(2) INFORMATION FOR SEQ ID :1098:

(i) SEQUENCE CHARACTERISTICS:
(A) LENGTH: 138 base pairs
(B) TYPE: nucleic acid
(C) STRANDEDNESS: double
(D) TOPOLOGY: linear

(xi) SEQUENCE DESCRIPTION: SEQ ID :1098:

AAAACTGACT AAAGTCANTG TGTAATACGA AAACCGAACA CTCCAGCAGT 50

CCTATTTTAT NTNTTCCAAC CTAGNNCACC CAGGTGACGC NNGGGACTCG 100

TCNCAGGTGT TTNGNACTTG CCGCCCGACN GGCNAAGG 138

(2) INFORMATION FOR SEQ ID :1099:

(i) SEQUENCE CHARACTERISTICS:
(A) LENGTH: 151 base pairs
(B) TYPE: nucleic acid
(C) STRANDEDNESS: double
(D) TOPOLOGY: linear

(xi) SEQUENCE DESCRIPTION: SEQ ID :1099:

ACTCCACCAC TGGGNCCGCC ATGTTTTGGG TNGGNGCAAT GCGCTGTCTG 50

TGAGCGCCAN TCTAATTGTA TTNGGACTGC ACGGGTCCGT ACACTGACTG 100

ACACTGGGCT ACCTCGCCCG AAAATGNGAA CACCGCTCAA TATCGNTGCG 150

G 151

(2) INFORMATION FOR SEQ ID :1100:

(i) SEQUENCE CHARACTERISTICS:
(A) LENGTH: 201 base pairs
(B) TYPE: nucleic acid
(C) STRANDEDNESS: double
(D) TOPOLOGY: linear

(xi) SEQUENCE DESCRIPTION: SEQ ID :1100:

TAGCAATAGG CTATAACATA AGCCGAGGTA TAGGCTACAC TAGCTAGGTT 50

CGTGTAAGTA CACTCTATAT TGGCACAACA TAAAATTAAC TAGTGACGCA 100

TTTCTCAGAA TGCATCCCCA TCGTTAAGTG ACACAGACTG TCCCTCATAT 150

CACCGGAGGA CTGGNCCAGG ACCTCCTGGC GGCACCAAAA TCCACGAGCG 200

C 201

(2) INFORMATION FOR SEQ ID :1101:

(i) SEQUENCE CHARACTERISTICS:
(A) LENGTH: 212 base pairs
(B) TYPE: nucleic acid
(C) STRANDEDNESS: double
(D) TOPOLOGY: linear

(xi) SEQUENCE DESCRIPTION: SEQ ID :1101:

TAGCAATAGG CTATAACATA TACCGAGGTA TAGGCTACAC TAGCTAGGTT 50

CGTGTAAGTA CACTCTATAT TAGCACAACG ATAAAATTAA CTAGTGATGC 100

ATTTTTCAGA ATGCATCCCC ATCGTTAAGT GACACATGAC TGTCCCTCAG 150

TATCACTGGA GGACTGGGNC CAGGACCTGA CCTGGTGGTA CCAAAATCCA 200

TGAGGCNGNA AT 212

(2) INFORMATION FOR SEQ ID :1102:

(i) SEQUENCE CHARACTERISTICS:
(A) LENGTH: 218 base pairs
(B) TYPE: nucleic acid
(C) STRANDEDNESS: double
(D) TOPOLOGY: linear

(xi) SEQUENCE DESCRIPTION: SEQ ID :1102:

CGGTAGCAAT AGCTATAACA TATAGCCGAG GTATAGGCTA CACTAGCTAG 50

GTTCGTGTAA GCACACTCTA TACNNGCACA ACATAAAATN AACTAGTGAT 100

GCATTTCTCA GAATGCATCC CCATCGTNAA GNGACACATG ACTGTCCCTC 150

AGTATTACTG NAGGACTGGN CCAGGACCTC CCTGGGGTAC CAAAATCCAN 200

GAGTGGAAAT TCCANCAT 218

(2) INFORMATION FOR SEQ ID :1103:

(i) SEQUENCE CHARACTERISTICS:
(A) LENGTH: 207 base pairs
(B) TYPE: nucleic acid
(C) STRANDEDNESS: double
(D) TOPOLOGY: linear

(xi) SEQUENCE DESCRIPTION: SEQ ID :1103:

GTAGCAATAG GCTATAACAT ATACCGAGGT ATAGGCTACA CTAGCTAGGT 50

TCGTGTAAGT ACACTCTATA TTAGCACAAC GATAAAATTA ACTAGTGATG 100

CATTTTTCAG AATGCATCCC CATCGTTAAG TGACACATGA CTGTCCCTCA 150

GTATCACTGG AGGACTGGGN CCAGGACCTG ACCTGGTGGT ACCAAAATCC 200

ATGAGCG 207

(2) INFORMATION FOR SEQ ID :1104:

(i) SEQUENCE CHARACTERISTICS:
(A) LENGTH: 259 base pairs
(B) TYPE: nucleic acid
(C) STRANDEDNESS: double
(D) TOPOLOGY: linear

(xi) SEQUENCE DESCRIPTION: SEQ ID :1104:

GGCGTCAAGT GGCCCAATCT AACAGCTCCA GGGTAGCCAC AACCATCGTG 50

ATGGATTGCC ATTATTGTGT GCTAGTGGCT AGAGGTAGAC CCAATAGAAC 100

TCTGCCCCAA CAGGGCAACA GCCAAGCTCA ATTCTCCAAG CCCCCTGAAC 150

AGACCTTCCA CATCCAGGAG AAGCTGTTGT TGTCTAGAGC TACTTAGTGT 200

CAGAATCCAG GCCAGCTTGG CTGCTTGATG CGACTGGCTA TAGGATTNGN 250

GTGNAGGCC 259

(2) INFORMATION FOR SEQ ID :1105:

(i) SEQUENCE CHARACTERISTICS:
(A) LENGTH: 230 base pairs

(B) TYPE: nucleic acid
(C) STRANDEDNESS: double
(D) TOPOLOGY: linear

(xi) SEQUENCE DESCRIPTION: SEQ ID :1105:

ACATGACCCC TTGGACTGAA GGCGCTCAGT AGTAAAGGAG TGTCATGCAG 50

GTCAACACAT GTCGCACATG GACCACAAAG CCTGCCACCA GCAGGATGCA 100

CGGGGACTTC TGGGAGGGGG TGGACAGGAT ACTTATCTGT GACTGGAATG 150

CAGGCGAGAG GCGGAGAAGA GAGTGAAGGA TAACTCATAG AGGGGGCAGC 200

ATTTGTTTCG NGTTGAAAGA GGCAGAAAAT 230

(2) INFORMATION FOR SEQ ID :1106:

(i) SEQUENCE CHARACTERISTICS:
(A) LENGTH: 163 base pairs
(B) TYPE: nucleic acid
(C) STRANDEDNESS: double
(D) TOPOLOGY: linear

(xi) SEQUENCE DESCRIPTION: SEQ ID :1106:

ACATGNCCTG GACTGAAGGT GCTCAGTTAG TAAGGGAGTG TCATGCAGGT 50

CAACACACGT CGCACATGGA CCACAAATGC CTGCCACCAC AGGATGCACA 100

GGACTTTTGG GGGGAGTGGA CAGGTATTAT TGNGACTGGT GAGGTGAGAG 150

GTNAGAGGGG GCT 163

(2) INFORMATION FOR SEQ ID :1107:

(i) SEQUENCE CHARACTERISTICS:
(A) LENGTH: 182 base pairs
(B) TYPE: nucleic acid
(C) STRANDEDNESS: double
(D) TOPOLOGY: linear

(xi) SEQUENCE DESCRIPTION: SEQ ID :1107:

ACNTGATGAT TGCCATTATT GTGTGCTAGT GGCTAGAGGT AGACCCAATA 50

GAACTCTGCC CCAACAGGGC AACAGCCAAG CTCAATTCTC CAAGCCCCCT 100

GAACAGACCT TCCACATCCA GGAGAAGCTG TTGTTGTCTA GAGCTACTTA 150

GTGTCAAATC CAGGCCAGCT TGGCTGCTTG AT 182

(2) INFORMATION FOR SEQ ID :1108:

(i) SEQUENCE CHARACTERISTICS:
(A) LENGTH: 214 base pairs
(B) TYPE: nucleic acid
(C) STRANDEDNESS: double
(D) TOPOLOGY: linear

(xi) SEQUENCE DESCRIPTION: SEQ ID :1108:

CCCCTGGACT GAAGGCGCTC AGTAGTAAAG AGTGTCATGC AGGTCAACAC 50

ATGTCGCACA TGGACCACAA AGCCTGCCAC CAGCAGGATG CACGGGGACT 100

TCTGGGAGGG GAGTGGACAG GATACTATCT GTGACTGGAA TGCAGGCGAG 150

AGGCGGAGAA GAGAGTGAAG GATAACTCAT AGAGGGGGCA GCATTTGTTT 200

CGNGCCTTGA AAGA 214

(2) INFORMATION FOR SEQ ID :1109:

(i) SEQUENCE CHARACTERISTICS:

(A) LENGTH: 133 base pairs

(B) TYPE: nucleic acid

(C) STRANDEDNESS: double

(D) TOPOLOGY: linear

(xi) SEQUENCE DESCRIPTION: SEQ ID :1109:

ACATGGCCCC TGGACTGAAG GTGCTCAGTT AGTAAGGGAG TGTCATGCAG 50

GTCAACACAC GTCGCACATG GACCACAAAT GCCTGCCACC AGCAGGATGC 100

ACAGGACTTT TGGGGGGGGT GGACAGGTAT TAT 133

(2) INFORMATION FOR SEQ ID :1110:

(i) SEQUENCE CHARACTERISTICS:

(A) LENGTH: 156 base pairs

(B) TYPE: nucleic acid

(C) STRANDEDNESS: double

(D) TOPOLOGY: linear

(xi) SEQUENCE DESCRIPTION: SEQ ID :1110:

GCATGACCCC ATGGCACTGA AGGTGCTCAG GTTAGTAAGG GAGTGTCATG 50

CAGGTCAACA CATGTCGCAC ATGGACCACA AATGCCTGCC ACCAGCAGGA 100

TGCACGGGGG ACTTCTGGGA GGAGAGTGCA TAGGATACTT GTCTGTGACT 150

GGAATG 156

(2) INFORMATION FOR SEQ ID :1111:

(i) SEQUENCE CHARACTERISTICS:
(A) LENGTH: 282 base pairs
(B) TYPE: nucleic acid
(C) STRANDEDNESS: double
(D) TOPOLOGY: linear

(xi) SEQUENCE DESCRIPTION: SEQ ID :1111:

GGCGCTCANT GGATCCAATA CACCAGACTC CCGGGAGCGA AAACGCCCGC 50

AGGTGCCTCA AAGAACTTAA AACAGAACTG CCATTAGACC CCACAATCTC 100

ATNAAGGATG TTATAGATAT ATCCAAAAGA AAATAAATCA TTCTTCAAAA 150

AGACACATAC ACTAACACGT TCATGGAGCA CTATTCACAC AGCAAAGACT 200

CGATCAACTC ACACACTCAT GAATGCGGAT CGAAATAGAA GATGTGGTAT 250

GCATACACCA CGAAATACTA CGAGCCATAA AA 282

(2) INFORMATION FOR SEQ ID :1112:

(i) SEQUENCE CHARACTERISTICS:
(A) LENGTH: 181 base pairs
(B) TYPE: nucleic acid
(C) STRANDEDNESS: double
(D) TOPOLOGY: linear

(xi) SEQUENCE DESCRIPTION: SEQ ID :1112:

TAATTAGTTT AACCACTGTG GAAAGCGTTG TAGGGTCTCA AAGACTNAAA 50

ACAGAACTGC TTAGACCCCA CAATNTTANG AGCGGATGTT ATGATATATT 100

CAAAAGGAAA TAAANNGNCN TGTNAAAGAC CATACACNAT NGTCCATCGG 150

AGCCCGTATN CACGCAGCTT GTCATAACTA T 181

(2) INFORMATION FOR SEQ ID :1113:

(i) SEQUENCE CHARACTERISTICS:
(A) LENGTH: 278 base pairs
(B) TYPE: nucleic acid
(C) STRANDEDNESS: double
(D) TOPOLOGY: linear

(xi) SEQUENCE DESCRIPTION: SEQ ID :1113:

ATCCAATGAC ACCAGACTCC CGGGAGCGAA ACGCCCGCAG GTGCTCAAAG 50

AACTTAAAAC AGAACTGCCA TTAGACCCCA CAATCTCATN AAGGATGTTA 100

TAGATATATC CAAAAGAAAA TAAATCATTC TTCAAAAAGA CACATACACT 150

AACACGTTCA TGGAGCACTA TTCACACAGC AAAGACTCGA TCAACTCACA 200

CACTCATGAA TGCGGATCGA AATAGAAGAT GTGGTATGCA TACACCACGA 250

AATACTACGA GCCATAAAAG GCGAAATC 278

(2) INFORMATION FOR SEQ ID :1114:

(i) SEQUENCE CHARACTERISTICS:
(A) LENGTH: 125 base pairs
(B) TYPE: nucleic acid
(C) STRANDEDNESS: double
(D) TOPOLOGY: linear

(xi) SEQUENCE DESCRIPTION: SEQ ID :1114:

ACCTGCAAAC TTTTTCTGTA AAGACCAGAT AGTAAGTATT TTATGCTTTG 50

TGAGCCATAC AGTTTTTGTT GCAACTAGTC AACTCTCAAC TNACGTGGAC 100

AGCATGTAAA GAACGAGAGT GACTG 125

(2) INFORMATION FOR SEQ ID :1115:

(i) SEQUENCE CHARACTERISTICS:
(A) LENGTH: 157 base pairs
(B) TYPE: nucleic acid
(C) STRANDEDNESS: double

(D) TOPOLOGY: linear

(xi) SEQUENCE DESCRIPTION: SEQ ID :1115:

GTAGCGACCG GCGCTCATGG AATTCCGGGA CCTGCAAACT TTTTTGAAAG 50

ACCAGATAGT AAGTATTTTA TGCTTTGTGA GCCATACAGT TTTTGTGCAA 100

CTAGTCAACT CTCAACTCAC CGTGGACAGC ATGTAAATGG ATGGGAGTGG 150

CTGCCGG 157

(2) INFORMATION FOR SEQ ID :1116:

(i) SEQUENCE CHARACTERISTICS:
(A) LENGTH: 123 base pairs
(B) TYPE: nucleic acid
(C) STRANDEDNESS: double
(D) TOPOLOGY: linear

(xi) SEQUENCE DESCRIPTION: SEQ ID :1116:

CCTGCAAACT TTTTCTGTAA AGACCAGATA GTAAGTATTT TATGCTTTGT 50

GAGCCATACA GTTTTTGTTG CAACTAGTCA ACTCTCAACT NACGTGGACA 100

GCATGTAAAG AACGAGAGTG ACT 123

(2) INFORMATION FOR SEQ ID :1117:

(i) SEQUENCE CHARACTERISTICS:

(A) LENGTH: 125 base pairs
(B) TYPE: nucleic acid
(C) STRANDEDNESS: double
(D) TOPOLOGY: linear

(xi) SEQUENCE DESCRIPTION: SEQ ID :1117:

ACCTGCAAAC TTTTTTTGTA AAGACCAGAT AGTAAGTATT TTATGCTTTG 50

TGAGCCATAC AGTTTTTGTG CAACTAGTCA ACTCTCAACT CACCGTGGAC 100

AGCATGTAAA TGGATGGGAG TGGCT 125

(2) INFORMATION FOR SEQ ID :1118:

(i) SEQUENCE CHARACTERISTICS:
(A) LENGTH: 125 base pairs
(B) TYPE: nucleic acid
(C) STRANDEDNESS: double
(D) TOPOLOGY: linear

(xi) SEQUENCE DESCRIPTION: SEQ ID :1118:

ACCTGCAAAC TTTTTCTGTA AAGGACCAGA TAGTAAGTAT TTTATGCTTT 50

GTGAGCCATA CAGTTTTCGT TGCAACTAGT CAACTCTCAA CTTCACCGTG 100

GACAGCATGT AAATGGATGG GAGTG 125

(2) INFORMATION FOR SEQ ID :1119:

(i) SEQUENCE CHARACTERISTICS:
(A) LENGTH: 148 base pairs
(B) TYPE: nucleic acid
(C) STRANDEDNESS: double
(D) TOPOLOGY: linear

(xi) SEQUENCE DESCRIPTION: SEQ ID :1119:

ATTAAGCTTG ACACATCTGT GTTATCACGC ACTGAAGACA GGAAGCAGTT 50

CACTGAGTCA GCTGGCTTCC AAGCTTACAC AGAAGGCGAT AAGTCACTAT 100

CAAAGAGCCA ATGAGAATCT TCTTATAGAA TAACCTGGGC CCAAGTGA 148

(2) INFORMATION FOR SEQ ID :1120:

(i) SEQUENCE CHARACTERISTICS:
(A) LENGTH: 240 base pairs
(B) TYPE: nucleic acid
(C) STRANDEDNESS: double
(D) TOPOLOGY: linear

(xi) SEQUENCE DESCRIPTION: SEQ ID :1120:

AGAATAACAG AGCCAAAGGA CTAATAAAAT CAATGAATCN TGGTGAGACT 50

AATCAAGAAA AAATAGCACC AACAACAATG AGGAGAAAAT GGAAAAAGGG 100

CAGAGTATTT CAATGATTAC GAGAGGGCAA ACCAATCAAC GAATGATTCT 150

TAAGCCTTCA GTTTGCCCTG TAAGCAAACT GAAGACGTGC AAGTCATCCT 200

TTGCCCTGGG AGAGTTAACT TAACCCACAG GGACAACGAG 240

(2) INFORMATION FOR SEQ ID :1121:

(i) SEQUENCE CHARACTERISTICS:
(A) LENGTH: 254 base pairs
(B) TYPE: nucleic acid
(C) STRANDEDNESS: double
(D) TOPOLOGY: linear

(xi) SEQUENCE DESCRIPTION: SEQ ID :1121:

AAATGGGGAG AATCAACAGA GCCAAAGGAC TAATAAAATT AAGAATCCTG 50

GTGAGACTAA TAAGAAAAAA TACACCAACA ACAATCCGAG AAAATGGAAA 100

AAGGGCAGAG TATTTAATGA TACGAAGATG GGCAAACCAA TCAATGAATG 150

ATTTTTAAGC CTTCACGTCT GCCCTGTAAG CAAACTGAAG ACGTGCAAGT 200

CATCCTTCGG CCCTGGAGAG TTAACGTTNC CCAAGGGGCA ATGAGAAGGG 250

ACAG 254

(2) INFORMATION FOR SEQ ID :1122:

(i) SEQUENCE CHARACTERISTICS:
(A) LENGTH: 253 base pairs
(B) TYPE: nucleic acid
(C) STRANDEDNESS: double
(D) TOPOLOGY: linear

(xi) SEQUENCE DESCRIPTION: SEQ ID :1122:

AAACGAGGGG AGAATCAACA GAGCCAAAGG ACTAATAAAA TCAATGAATC 50

NTGGTGAGAC TAATCAAGAA AAAATAGCAC CAACAACAAT GAGGAGAAAA 100

TGGAAAAAGG GCAGAGTATT TCAATGATTA CGGAGAGGGC AAACCAATCA 150

ACGAATGATT CTTAAGCCTT CACGTTTGCC CTGTAAGCAA ACTGAAGACG 200

TGCAAGTCAT CCTTTGCCCT GGGAGAGTTA ACTTAACCCA CAGGGACAAC 250

GAG 253

(2) INFORMATION FOR SEQ ID :1123:

(i) SEQUENCE CHARACTERISTICS:
(A) LENGTH: 210 base pairs
(B) TYPE: nucleic acid
(C) STRANDEDNESS: double
(D) TOPOLOGY: linear

(xi) SEQUENCE DESCRIPTION: SEQ ID :1123:

AAATGGGGAG AATCAACAGA GCCAAAGGAC TAATAAAATT AATGAATCCT 50

GGTGAGACTA ATAAGAAAAA ATACACCAAC AACAATCCGA GAAAATGGAA 100

AAAGGGCAGA GTATTTAATG ATACGAAGAT GGGCAAACCA ATCAATGAAT 150

GATTTTTAAG CCTTCACGTC TGCCCTGTAA GCAAACTGAA GACGTGCAAG 200

TCATCCTTTG 210

(2) INFORMATION FOR SEQ ID :1124:

(i) SEQUENCE CHARACTERISTICS:
(A) LENGTH: 231 base pairs
(B) TYPE: nucleic acid
(C) STRANDEDNESS: double
(D) TOPOLOGY: linear

(xi) SEQUENCE DESCRIPTION: SEQ ID :1124:

AAATGGGGGG AGAATCAACA GAGCCAAAGG ACTAATAAAA TTAATGAATC 50

CTGGTGAGAC TAATCAAGAA AAAATAGCAC CAACAACAAT CAAGAGAAAA 100

TGGAAAAAGG GCAGAGTATT TAAATGATTA CGGAGACGGG CAAACCAATC 150

AACGAATGAT TCTTAAGCCT TCATGTTTGC CCTGTAAGCA AACTGAAGAC 200

GTGCAAGTCA TCCTTTGGAC CCTGGGAGAG T 231

(2) INFORMATION FOR SEQ ID :1125:

(i) SEQUENCE CHARACTERISTICS:
(A) LENGTH: 89 base pairs
(B) TYPE: nucleic acid
(C) STRANDEDNESS: double
(D) TOPOLOGY: linear

(xi) SEQUENCE DESCRIPTION: SEQ ID :1125:

AAGTCCCATC AGCAACCCGT TTTTTACCAG ATGTCACTCA AGAATGCGCC 50

CGTGGCCCTC CAGTTCCTGC GCACTAAGAG CGTCCCGCT 89

(2) INFORMATION FOR SEQ ID :1126:

(i) SEQUENCE CHARACTERISTICS:
(A) LENGTH: 205 base pairs
(B) TYPE: nucleic acid
(C) STRANDEDNESS: double
(D) TOPOLOGY: linear

(xi) SEQUENCE DESCRIPTION: SEQ ID :1126:

AAGAAACCCG AGACMCCAAC TAATCCCAGA ACAGCTAAAA CCAATAAAGA 50

ACCAAAACTT CACGACCACT TTCCTGAGGA CCCTGCTGAG TGCTCATGAC 100

ACCAATACTG ACAATTGTAG CGGTAATACA TACAATGATT TAATAAGCCT 150

ATGCTACTGG ACACATGCCC ACACAATAAT ANTCANRTGT NTYGTTAGGC 200

CTACT 205

(2) INFORMATION FOR SEQ ID :1127:

(i) SEQUENCE CHARACTERISTICS:
(A) LENGTH: 151 base pairs
(B) TYPE: nucleic acid
(C) STRANDEDNESS: double

(D) TOPOLOGY: linear

(xi) SEQUENCE DESCRIPTION: SEQ ID :1127:

GCAAGGCCTG GCCGACAACA CCCTTATTGC TAAAGTAAAT AATGNCGCGC 50

GGGGCCTGGA CCACCCTCTG GAAGAAGATT GTACCTTGGA GCTTCTCAAG 100

TCTGAGGATG AGGAAGCTCA GGCAGTGTAT TGGCGCTTTA GTATCACATA 150

A 151

(2) INFORMATION FOR SEQ ID :1128:

(i) SEQUENCE CHARACTERISTICS:
(A) LENGTH: 237 base pairs
(B) TYPE: nucleic acid
(C) STRANDEDNESS: double
(D) TOPOLOGY: linear

(xi) SEQUENCE DESCRIPTION: SEQ ID :1128:

GAGCACCCCA GCCCATAACA CAGAACTCGT CCCCCTACCC CCCTCAGCAA 50

GCCGGAGAGG CTAGCCCAAG TAATCATAAY AACAGCCGCC CGAGAGCAGC 100

CCCAGTAGCA GCCCCATGGC CGGGCGGAAC ACCTACATCG ACAACCTCAT 150

GACAGACGAG ACCTGCCAGG ACGCAGCCAT TGCGGGCTGC AAGGCCTCRC 200

CCTTCGTCTG GGCCGCCGTC CCCGGAGAAA CGCTCGT 237

(2) INFORMATION FOR SEQ ID :1129:

(i) SEQUENCE CHARACTERISTICS:
(A) LENGTH: 126 base pairs
(B) TYPE: nucleic acid
(C) STRANDEDNESS: double
(D) TOPOLOGY: linear

(xi) SEQUENCE DESCRIPTION: SEQ ID :1129:

GCCTCGTGGA AGTGACATAG CCTTTAAACC CTGCGTGGCA ATCCCTGACG 50

CACCACGTGA CGCCCAGGAA AGACAGGGCG ACCTGGAAGT CCAACTACTT 100

CCTTAAGATC ATCCAACTAT AGGATG 126

(2) INFORMATION FOR SEQ ID :1130:

(i) SEQUENCE CHARACTERISTICS:
(A) LENGTH: 246 base pairs
(B) TYPE: nucleic acid
(C) STRANDEDNESS: double
(D) TOPOLOGY: linear

(xi) SEQUENCE DESCRIPTION: SEQ ID :1130:

GCCATCTTCT GTAACAAAAT AAATAGTGTC AAGCACTAAA TATATGCATG 50

AATGCATCAG CAATAAGATG ACAATCAAAG AATGCTGTGA AGATTGGCAT 100

CAAGATGTTA GCAGCATACT GCAGGACTAG GCTTAGAAGC CTACCTGCAG 150

TTTATTTGCA AGGATTTTTC TTTCGRGGGC GGAGGGGAGA GAAAAGTAAA 200

TGTGCTAACT TCGGATACTT GCCCTTATAA AGAATTCCTT YGTATC 246

(2) INFORMATION FOR SEQ ID :1131:

(i) SEQUENCE CHARACTERISTICS:
(A) LENGTH: 273 base pairs
(B) TYPE: nucleic acid
(C) STRANDEDNESS: double
(D) TOPOLOGY: linear

(xi) SEQUENCE DESCRIPTION: SEQ ID :1131:

CGAGGATCCG GGTACCATGG CAAGTCTCAT GAGGCTATTT ACCGAATTTA 50

TCCTCTGTCT GACCATGATT TTTTCCCTCA AATACAACCA CTCCTCGACT 100

TTTCACCCTC AAAGTATAAA AAGTATGAAA NATAAACAAG CTCTTGCACT 150

GTACACTTAG AAGTGTACAA TTAAAGCATT ATAGAGCTAT CTACACACCG 200

ATAAATCCCA TCGAATCTTG AATAATCCAT CAATACGTAG AACGCAAGGG 250

TGCAGACAGA ACTAAAACCA ACT 273

(2) INFORMATION FOR SEQ ID :1132:

(i) SEQUENCE CHARACTERISTICS:
(A) LENGTH: 329 base pairs
(B) TYPE: nucleic acid

(C) STRANDEDNESS: double
(D) TOPOLOGY: linear

(xi) SEQUENCE DESCRIPTION: SEQ ID :1132:

GAGGATCCGG GTACCATGGT CTGTATATCT GTGCTTCCAC TTCTCGGAGG 50

TGGTAGAGGG GAGGCTGTCC TCTGGTCAGG AGAATCCTAT TCAGTGCTCC 100

CTTAGACATT CTTCCAGGCA GGATCAAACT CAAAGGAAAA GGAATTTGTG 150

AAGCAAACCA TGGCTTTGTC ACAGTAAAGT AATTGTCACT CTCAACCCAG 200

AATGTGTGAA GCGCTGCAGG GAGAAGCTTC TCTTCCAGGA GAGCAATANA 250

AGCCAATGTA TCTGACCCTT GCTTYGATGA GAGTTAATAA TAAGTATTAT 300

ATTTCTGTTT GTTAAAAAGT TCAGAATTT 329

(2) INFORMATION FOR SEQ ID :1133:

(i) SEQUENCE CHARACTERISTICS:
(A) LENGTH: 24 base pairs
(B) TYPE: nucleic acid
(C) STRANDEDNESS: double
(D) TOPOLOGY: linear

(xi) SEQUENCE DESCRIPTION: SEQ ID :1133:

CCCCGAGCTA GTTGTCCCGA CCTG 24

(2) INFORMATION FOR SEQ ID :1134:

(i) SEQUENCE CHARACTERISTICS:
(A) LENGTH: 297 base pairs
(B) TYPE: nucleic acid
(C) STRANDEDNESS: double
(D) TOPOLOGY: linear

(xi) SEQUENCE DESCRIPTION: SEQ ID :1134:

GAGGATCCAC AAACCATGGA ACACTCTCGC GGCTGCCACT CCCCCATGAC 50

CACCCTACAG GCCCTAACCC CAGCCCCTCA CGTTATCGTC CAGCCACAAA 100

TAGCTGCCGC CCTGAGATCT CCACCTCAGA CCCTCTCCCG AAGCCCACAG 150

CGGCCCCTGC CGGGCACCCC AGCTTCCTCG CAGGCACCAA AAGCGCAGTT 200

CCAGGAATCT CTACCCTAGA GTGATCCGGC GCCAGTTGCC AGTTAAGGGA 250

CCAAACCTCG CCAGAAGGCT TCTTTTCGGG TTGATCGCCA TCTATTY 297

(2) INFORMATION FOR SEQ ID :1135:

(i) SEQUENCE CHARACTERISTICS:
(A) LENGTH: 93 base pairs
(B) TYPE: nucleic acid
(C) STRANDEDNESS: double
(D) TOPOLOGY: linear

(xi) SEQUENCE DESCRIPTION: SEQ ID :1135:

GCCGCTTTAC TGCTCAGTTC GGAGCTACCG CTTCGAAAGC AACAAAAAAG 50

CNTTTGCAAA TGAAACGAGA TTGCTGAATT GCGTACACCG AGA 93

(2) INFORMATION FOR SEQ ID :1136:

(i) SEQUENCE CHARACTERISTICS:

(A) LENGTH: 334 base pairs

(B) TYPE: nucleic acid

(C) STRANDEDNESS: double

(D) TOPOLOGY: linear

(xi) SEQUENCE DESCRIPTION: SEQ ID :1136:

CGAGGATCCG GGTACCATGG AGCTTCACAC GGGTCTTCTT GGTCTCAGGG 50

TTGTGGGAGA TAACGGTGGC ATAGTTCCCT GATGCCCGGG CCAGCTTGCC 100

ACGGTCTCCA GGCTTCTCCT CCAGGCAGCA CACGATTGTA CCCTCAGGCA 150

TGGTGCCCAC AGGGAGCRCA TTGCCAATGT TGAGCTGGGC CTTCTTGCCG 200

CAATACACAA ACTGGCCCGT GNGAATGCCC TCGCGGGCAA TGAACAGTTC 250

CGCCCGCTTC TTAAACCAAG ACAGATCCCG GAAGGCCGCC TGGGCAAGGG 300

GCCCCTCRAA CCCGGGGCTA TACACGTCAA AATC 334

(2) INFORMATION FOR SEQ ID :1137:

(i) SEQUENCE CHARACTERISTICS:

(A) LENGTH: 199 base pairs

(B) TYPE: nucleic acid
(C) STRANDEDNESS: double
(D) TOPOLOGY: linear

(xi) SEQUENCE DESCRIPTION: SEQ ID :1137:

CGAGGATCCG CATACCATGG CCCCCATCTT ACGAGCCCTT ACTCCTCATG 50

ATGAAGAAAC TCCAAACCCT TTGACAGCTC CTCGGGGTNA AACCTGGNAY 100

AAAGCTGACT GACCAGGCTC CATGAAATCT CCCATCTCCA TRATGTACCT 150

CCTTGRTGCC ATCTCTATAT AAAGMGAACC AGTCCTAAAA TCAAACACT 199

(2) INFORMATION FOR SEQ ID :1138:

(i) SEQUENCE CHARACTERISTICS:
(A) LENGTH: 304 base pairs
(B) TYPE: nucleic acid
(C) STRANDEDNESS: double
(D) TOPOLOGY: linear

(xi) SEQUENCE DESCRIPTION: SEQ ID :1138:

CGAGGATCCA AATACCATGG CGCTCCCTCC TCCCGAGCGC CGCTCCGCGT 50

GGCACCCGGC TCGCTCCGAG TTTCAGGCTC GTGCTAAGCT AGCGCCGTCG 100

TCGTCTCCCT TCAGTCGCCA TCATGATTAT CTACCGGGAC CTCATCAGCC 150

ACGATGAGAT GTTCTCCAAC ATCTACAAGA TCCGGGAGAT CGCGGACGGG 200

TTGTGCCTGG AGGTGAAGGG GAAGATGATC AGTAGGACAG AAGGTAACAT 250

TGMTGACTTG CTCATTGGTG GAAATGCTTC CTCTGAAGGC CCCGAGGAAG 300

AAGG 304

(2) INFORMATION FOR SEQ ID :1139:

(i) SEQUENCE CHARACTERISTICS:

(A) LENGTH: 277 base pairs

(B) TYPE: nucleic acid

(C) STRANDEDNESS: double

(D) TOPOLOGY: linear

(xi) SEQUENCE DESCRIPTION: SEQ ID :1139:

GAGGATCCGG GTACCATGAG AAACTTTGAA GCCAGAGATT TTAAACAATC 50

AAGGCACTTG AAAACATTAA GTATATGTAC AAATGTGCAA GTAAAACAAA 100

CAGCTGTACC AACGAGTAAC AAAGAAACAG TAAATCTTCA TCTTAACAAC 150

CTTTAATAGT TATCTAAATG CAGAGTTTGT TTATGAAATG AACCAAAGCA 200

GTTTGTCATT TCTTACTATA AAATACCGAA AATAAAGTGC AAAACTTAGC 250

CACTACTGGC TAAAGAAACT AAGTAAA 277

(2) INFORMATION FOR SEQ ID :1140:

(i) SEQUENCE CHARACTERISTICS:

(A) LENGTH: 208 base pairs

(B) TYPE: nucleic acid
(C) STRANDEDNESS: double
(D) TOPOLOGY: linear

(xi) SEQUENCE DESCRIPTION: SEQ ID :1140:

CGAGATCCGG GTACCATGGC CTCCTACAGG TGCCGTGGAG CCACGCCCAA 50

AAGAGAGCTC CCTGAGAAAC TCGTTGATGC CTTGCTCACT GAAGGAGCCT 100

TTTAGCAGAG CAAATTTCAT CTTGCGTGCA TTGATGGCGG CCATGGCGGG 150

GTACCCAAAC CCTCCAATTC CCAACGCGGT CTCAAGTTCA GACTGGGCTC 200

CAGCTTCT 208

(2) INFORMATION FOR SEQ ID :1141:

(i) SEQUENCE CHARACTERISTICS:
(A) LENGTH: 274 base pairs
(B) TYPE: nucleic acid
(C) STRANDEDNESS: double
(D) TOPOLOGY: linear

(xi) SEQUENCE DESCRIPTION: SEQ ID :1141:

CGAGGATCCG AGTACCATGG CCTCGAACTG AGCATCAATA TATTCTACTG 50

CCAGCTTATA ACTGTCATCT TTATTCATAT GGTCTCCAAA TCCCACGATG 100

TCAACAATGG TTAACTTCAG CCGTACATTG CTTTCCTGAA GCTCATAACT 150

TCTGGCTTTT AACCGAACAC CTGGTTCATT GTGAGTAGCT GGGTCACTTT 200

TAAATTTGGT GTTGAACAAA GTGTCCATTA ACGTGGATTT GCCAATGCCT 250

GTCTCACCAA TACAAAGGAT GTTG 274

(2) INFORMATION FOR SEQ ID :1142:

(i) SEQUENCE CHARACTERISTICS:

(A) LENGTH: 226 base pairs

(B) TYPE: nucleic acid

(C) STRANDEDNESS: double

(D) TOPOLOGY: linear

(xi) SEQUENCE DESCRIPTION: SEQ ID :1142:

GCCTGCACAT TGACTGTGGG AAACTCGGAA ACAAGCTCAC ATCTCCCCGT 50

GGGAAACCTT CTAGCAACAG GATGAGTCTG CAGTGAACTG CAGTTGCCAC 100

CTTCCTCTAT GCGGAGGTCT TTGTTGTGTT GCTTCTCTGC ATTCCCTTCA 150

TTTCTCCTAA AAGATGGCAG AAGATTTTAA AGTCCCGGCT GATGGAGTTG 200

TTAGTGTCCT ATGGTAACAC CTTCTT 226

(2) INFORMATION FOR SEQ ID :1143:

(i) SEQUENCE CHARACTERISTICS:

(A) LENGTH: 168 base pairs

(B) TYPE: nucleic acid

(C) STRANDEDNESS: double

(D) TOPOLOGY: linear

(xi) SEQUENCE DESCRIPTION: SEQ ID :1143:

RGGRTCCRNG TGCCRTGGGG YNTNNCTAGR CRGCRGGYAA GGTCCACCRC 50

TGRCRCGNTG NCNGTGAGGR CRTGNRGGNC CRTGCGAGTG GGCTTACCGN 100

TAGGTTCGGG AATGACCTTG CYMACGNCNN YGACAGCTAA GTRGRNGCRG 150

GNANGRTGNN GTGGAGRG 168

(2) INFORMATION FOR SEQ ID :1144:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID :1144:

CGAGGATCCA AATACCATGG AGCTGACAAT GTCAGCTCAA TTTGAAGTAT 50

TCTTGAGGGA CCTTTTGCTC ATAGGCTTTA ATTCTGTCTG TGACTTTTGC 100

CAGGATTTGA GGAAATCGAC TGCCTTCACT CTTCCCTGAA ACTTTGAAGT 150

CCACATAAGG GCTTTCTCAT CAAATGGCAG AAATTATAAT TTATAAACCT 200

AAACAACCTT ATAGTGTTTT CGCTTTGAAT TGTATGAATT CTTAGAACTG 250

AGAAGT 256

(2) INFORMATION FOR SEQ ID :1145:

(i) SEQUENCE CHARACTERISTICS:
(A) LENGTH: 184 base pairs
(B) TYPE: nucleic acid
(C) STRANDEDNESS: double
(D) TOPOLOGY: linear

(xi) SEQUENCE DESCRIPTION: SEQ ID :1145:

AAACACAAGG CTAAAAGTCA CTAAAGGTGA CACAGTACCA ACCCCGGGAA 50

GGTTGAGTTC TGTGCTGCTA CCTTCTACTG ATGGCGAATA GACTATTCTG 100

TACCCTGTGA TGGGAGCCTG GGGTCTGCTC CAGCAAACAA CAATCGAGGT 150

GTAATCAACT TGGTCCACAG TCGGGTCAGG AGGG 184

(2) INFORMATION FOR SEQ ID :1146:

(i) SEQUENCE CHARACTERISTICS:
(A) LENGTH: 221 base pairs
(B) TYPE: nucleic acid
(C) STRANDEDNESS: double
(D) TOPOLOGY: linear

(xi) SEQUENCE DESCRIPTION: SEQ ID :1146:

CGAGGATCCG GGTACCATGG CCCAAAGAGT GAATGATGTA TCCCAGGGCG 50

CAGTCCACAA CTTGCGCAGT ACCTTCCCAG ATGACAGCCT CACTGGACCG 100

ATTTCCATCC ACGAAGATGA TGCTAAGAAG CACCATGAGC AGACCCAGCT 150

TGGGTGAGTC CTTAGTCGTT CCCAGTATGC CTGCATCAGT GGGCTCTAAG 200

GTGCTGAGAA GAATGCACAA G 221

(2) INFORMATION FOR SEQ ID :1147:

(i) SEQUENCE CHARACTERISTICS:

(A) LENGTH: 255 base pairs

(B) TYPE: nucleic acid

(C) STRANDEDNESS: double

(D) TOPOLOGY: linear

(xi) SEQUENCE DESCRIPTION: SEQ ID :1147:

CGAGGATCCA AATACCATGG GAGTCAAGCA TGCCTCGATA GCCACAGATT 50

TACAAGAATT ACCTACAGTT TGCTTCTTNG AATCGACATG CAAATCTGCT 100

TAAGGATTCT CACATTCAGG ACAGAGAACA AATTTTTTAA TGAATCCATC 150

CAACAGGTCT TGNAGTTTAT TCGCCTCATG AGATCCATTG ATAATGGAAC 200

RRTMATTCTT AACANCAAAC NGMGMCTGNN NTCCCRNCTT ATAACCMAAA 250

GATNT 255

(2) INFORMATION FOR SEQ ID :1148:

(i) SEQUENCE CHARACTERISTICS:
(A) LENGTH: 230 base pairs
(B) TYPE: nucleic acid
(C) STRANDEDNESS: double
(D) TOPOLOGY: linear

(xi) SEQUENCE DESCRIPTION: SEQ ID :1148:

CGAGGATCCA GGTACCATGG ACGATTCACA GCCCCTGGCC ACTGGCCATT 50

TCCAGTGGTA GGGAGCACAC TGRTCACATT CGAGACCCAC CACGTTGAGC 100

AGACAAAGGC AGCCTCCACT CTCCTCGTCA CTCAACATGT ACCTCCGTCC 150

CCCAGGATGT TGTAGTTATA GCAATAGCAG CCCTGCGGTT GACGTAGGTG 200

AGTCGGTGAC GCCCGATTTG GATCGGGTGT 230

(2) INFORMATION FOR SEQ ID :1149:

(i) SEQUENCE CHARACTERISTICS:
(A) LENGTH: 223 base pairs
(B) TYPE: nucleic acid
(C) STRANDEDNESS: double
(D) TOPOLOGY: linear

(xi) SEQUENCE DESCRIPTION: SEQ ID :1149:

GTGCCGTTGG TCCTGTGCGG TCACTTAACC AAGATGCCTG AGGAAACCCA 50

GACCCAAGAC CAACCAATGG AGGAGGAGGA GGTTGAGACG TTCTCCTTTC 100

AGGCAGAAAT CGCCCAGTTG ATGTCATTGA TCATCAATAT TACCNACTAG 150

AACAAAGAGA TCTTTCTGAG AGAGCTTATT CAAAATACAT TAAATGTATT 200

GGACAAAATT CGATATAAAA GCT 223

(2) INFORMATION FOR SEQ ID :1150:

(i) SEQUENCE CHARACTERISTICS:

(A) LENGTH: 272 base pairs

(B) TYPE: nucleic acid

(C) STRANDEDNESS: double

(D) TOPOLOGY: linear

(xi) SEQUENCE DESCRIPTION: SEQ ID :1150:

CGAGGATCCG AGCACCATGG AGAGACTATG ACGCTCAACA AGAATCACTC 50

GGAGGGCGAC GGAGTGATCG TCAATAACAC CGAGAACATC CTAATGCCCT 100

ATGATCACGT GGAACTAACA TTCAATGACA TGAAGAACGT GCCAGAAGCC 150

TTCAAAGAGA CCAAGAAAGG CGCTGTCTAC CTTACTTCTC ATTGAACCAT 200

CTTTATGTCC AAGAACAAGG ATGCGATGGG TATTTCGTGA NGCTATTTGA 250

NMTRAGGRMA GNCTGNAAGG TM 272

(2) INFORMATION FOR SEQ ID :1151:

(i) SEQUENCE CHARACTERISTICS:

(A) LENGTH: 140 base pairs
(B) TYPE: nucleic acid
(C) STRANDEDNESS: double
(D) TOPOLOGY: linear

(xi) SEQUENCE DESCRIPTION: SEQ ID :1151:

TGAGGATTTA TAGATTANAG CTAGAGTTCA CATTTTATGG ATTACAACCA 50

AAAAAAACCT GAAAAAGAAA AAAAACAAAA AAGCTCAAAA GCAATCACAA 100

GGATAGTTGA ATCCCTCTTT AAACCCCCAA AGCAACCCCC 140

(2) INFORMATION FOR SEQ ID :1152:

(i) SEQUENCE CHARACTERISTICS:
(A) LENGTH: 208 base pairs
(B) TYPE: nucleic acid
(C) STRANDEDNESS: double
(D) TOPOLOGY: linear

(xi) SEQUENCE DESCRIPTION: SEQ ID :1152:

AAGGATCCGG GTACCATGTA TTCCCCCGGT ATCAGCAGAG GCGTGTACGG 50

GCACTGCTTT AAAACTGGGA AGGAGGAAGA CGAGGCCAGG GAGCCGGAGG 100

GTCACCAAGG TAGATTTCCA GCAGCCTAGT CCAGCTGAAC GCTTTCCAGC 150

CTTGCTTTTT AGCAGCTTTG AGGAAAAGTA TAGTGATCCG GATGTGAAAC 200

TTTCATTG 208

(2) INFORMATION FOR SEQ ID :1153:

(i) SEQUENCE CHARACTERISTICS:
(A) LENGTH: 274 base pairs
(B) TYPE: nucleic acid
(C) STRANDEDNESS: double
(D) TOPOLOGY: linear

(xi) SEQUENCE DESCRIPTION: SEQ ID :1153:

CGAGGATCCA GGTACCATGG GGTGGATCCG CCGGGCATAG CCACCCTGGA 50

TGGTGGCCAT GGTGAGGCCG ATGAGGAAAA ACATCTTCCC CTGCTGTAGG 100

CTACTGAACT GGAAGCGCTG GTGTGTGAGG AAGCTCAACG TGCACTCCAG 150

GCCCAAGAAC AGGAAGAGGT AGAGGAAGTA GACTMGGCCC AGGCGGCCAG 200

GCTGATGAAT TGTCTCCAAA GGGTGAGACC TGGCCACGAG CCATANCCCA 250

GAMGCCNMAC AGGGCTRGGG ACTG 274

(2) INFORMATION FOR SEQ ID :1154:

(i) SEQUENCE CHARACTERISTICS:
(A) LENGTH: 267 base pairs
(B) TYPE: nucleic acid
(C) STRANDEDNESS: double
(D) TOPOLOGY: linear

(xi) SEQUENCE DESCRIPTION: SEQ ID :1154:

GAGGATCCAC CGACCATGGA ACACTCTCAA TGGCTGCAAC CACCCCGTGG 50

CCCCCCTACC AGCCCCAATC TCCAAACCCC GCACGTCGAT CATGACGCCA 100

CCAACAGCTG CAGCCCCTGA GATCTTCACC TTAGACCCTC TCCCGAAGCC 150

CGCAGCCGCC CCTGCGAACC CTCCAACTCG TTCACACGCG CCGAAAGCCT 200

ATTCCCAGGA CTCTCTGCCC CTACGTGACC GNCCCTAGCT GCTAGTCGAG 250

AATCCGAACT CCGCTCC 267

(2) INFORMATION FOR SEQ ID :1155:

(i) SEQUENCE CHARACTERISTICS:

(A) LENGTH: 231 base pairs

(B) TYPE: nucleic acid

(C) STRANDEDNESS: double

(D) TOPOLOGY: linear

(xi) SEQUENCE DESCRIPTION: SEQ ID :1155:

CTCAAACCCC GNTTCCCCCC TAATAAGAGC AACTAAAAAA CTTCTGATAA 50

CCACAATAAA AGCGTGCATT TGAGCACAAT TCAAAAGCCA ACCCCTTCGA 100

AGGNGCTTCT GATGNAAAAG GAGTTATGTT GAACGAAGCA GGAGTTAACG 150

CTACACAGTC AAATTGTGGC ATTAGGACGC GCGAGCGGGC TAGCTGCGGC 200

CGTCTANCAA GGCAACGGCC GCCTTTGAAT T 231

(2) INFORMATION FOR SEQ ID :1156:

(i) SEQUENCE CHARACTERISTICS:
(A) LENGTH: 128 base pairs
(B) TYPE: nucleic acid
(C) STRANDEDNESS: double
(D) TOPOLOGY: linear

(xi) SEQUENCE DESCRIPTION: SEQ ID :1156:

GCGCGAGCAC AGAGCCTCGC CTTTGCCGAT CCGCCGCCCA TCCACACCTG 50

CCGCCAGCTC ACCGTGTATG ATGATATCAC CNCGCTCGCC ACCAACRACG 100

GCTCYAACMT GTGCAAGGCC GCCTTCAA 128

(2) INFORMATION FOR SEQ ID :1157:

(i) SEQUENCE CHARACTERISTICS:
(A) LENGTH: 126 base pairs
(B) TYPE: nucleic acid
(C) STRANDEDNESS: double
(D) TOPOLOGY: linear

(xi) SEQUENCE DESCRIPTION: SEQ ID :1157:

GCCGCAACAC AACCCAGGGA GACTCAAGNC CACAGGGGCC CCCCGGCTCT 50

GAGGGATTCA CCGTCGCCTC CCGGTCCCCG AAGGCCCACA AGGGCAGAAA 100

GGTGAGCCTT ATGCACTGCC TAAAGA 126

(2) INFORMATION FOR SEQ ID :1158:

(i) SEQUENCE CHARACTERISTICS:
(A) LENGTH: 101 base pairs
(B) TYPE: nucleic acid
(C) STRANDEDNESS: double
(D) TOPOLOGY: linear

(xi) SEQUENCE DESCRIPTION: SEQ ID :1158:

AATAACCATA ACTGCAAAAA CCCAAMCAAA AAAAAAGGGG GAACAAAAAA 50

CCCCAAACCC CCAAAAAAAC CAGAAAAAAC CACAAAAAAA AACCCCTCCT 100

T 101

(2) INFORMATION FOR SEQ ID :1159:

(i) SEQUENCE CHARACTERISTICS:
(A) LENGTH: 172 base pairs
(B) TYPE: nucleic acid
(C) STRANDEDNESS: double
(D) TOPOLOGY: linear

(xi) SEQUENCE DESCRIPTION: SEQ ID :1159:

GAGGATCCGG GTACGGGCAG GGTGAGAGCC TGGGGTCCAC CGATAACCGG 50

GAGGGAGATG GCGTTCTTGA GCAGAGGGGA TGGGCCGTCC GGGAGCTCCC 100

CCCACACACG GTGGCGGTGC GGGTGAACTG GAAGGGGAGG TCGAAGGTGC 150

CATCTTCTTC AGGCCCCTCC AC 172

(2) INFORMATION FOR SEQ ID :1160:

(i) SEQUENCE CHARACTERISTICS:

(A) LENGTH: 235 base pairs

(B) TYPE: nucleic acid

(C) STRANDEDNESS: double

(D) TOPOLOGY: linear

(xi) SEQUENCE DESCRIPTION: SEQ ID :1160:

GAGGATCCAC ATACCATGGA ACAGCACCAC AAAAATAGCC ATTTTGATCA 50

TGGTTATTTC CCAGGACCAC ACGATTTGCC TACGTCACTG GAAGGCTATG 100

TGTCTGTTCT GAGCCTTCCC ACTCTCCTAA AGGGCAGATG AAGATCAGAG 150

CTTTGACCCT GTGATGCCAT TTTAATCAAC CCTGCTTGGT TTTAGAGGAT 200

TGCTCCCGTG GGTCACTTGA GGCAGGCTCC ACCTT 235

(2) INFORMATION FOR SEQ ID :1161:

(i) SEQUENCE CHARACTERISTICS:

(A) LENGTH: 284 base pairs

(B) TYPE: nucleic acid

(C) STRANDEDNESS: double

(D) TOPOLOGY: linear

(xi) SEQUENCE DESCRIPTION: SEQ ID :1161:

GAGGATCCAC CAACCATAGA AAGTAAATAC TGTATAAAAA GCTCCAGCTG 50

TTAGATACAA ATGCAAAACG CTTCCTAAAA AACGGCCTGA AAAAAAACTC 100

ACAGACAATG CCAAACTTTA TAGGTCTGTA TTTTCCTTTA TACAAAATGC 150

CCTCAAATTA AAAAGTAATT CCCATATAGC AATAAAGTCC ACATCTCTGG 200

ATTACCTATA ACCGGTATTA TGGGGTGTGT TTATACTGCC TAGAATGTTA 250

ACCCTCATAA AGCCTTAAAA GTACATTTGT GAAA 284

(2) INFORMATION FOR SEQ ID :1162:

(i) SEQUENCE CHARACTERISTICS:
(A) LENGTH: 287 base pairs
(B) TYPE: nucleic acid
(C) STRANDEDNESS: double
(D) TOPOLOGY: linear

(xi) SEQUENCE DESCRIPTION: SEQ ID :1162:

GCTCACATAA ATTTCTTCAC CGACCCTTTT CCTTCCAGCT TCCTTACTAT 50

AAAAAACCCC ACAATTAATA TGTTCATCAT CATCCATACT AACCAACCCC 100

GTCACCATCT CAATCAGCAG CAAGTCCTAC TCTCTGTGGG TGAAACCTTG 150

TTACCACCTC TAGGGGCAGA CCCCTTTTCA GATATATTCA AGAGTTTAAT 200

ATCCTCCCAC TATAGCGCTT CGACTTTGTA TCTTCCTCTA TGGCTACCAA 250

ATTCTGTCCG TTGATAAGTA CTGGCTCTAA ACCATGC 287

(2) INFORMATION FOR SEQ ID :1163:

(i) SEQUENCE CHARACTERISTICS:
(A) LENGTH: 122 base pairs
(B) TYPE: nucleic acid
(C) STRANDEDNESS: double
(D) TOPOLOGY: linear

(xi) SEQUENCE DESCRIPTION: SEQ ID :1163:

GAGGATCCAA AAACCATAAA ATTCATCATC CCCAGCAGGT GCNCTAGCTA 50

TACTTTATTA CAGCAAARCA CAACCACACG CTGAMCTANM TCGTATAGAT 100

AAACACCAAT CATGGGTCGG CC 122

(2) INFORMATION FOR SEQ ID :1164:

(i) SEQUENCE CHARACTERISTICS:
(A) LENGTH: 142 base pairs
(B) TYPE: nucleic acid
(C) STRANDEDNESS: double
(D) TOPOLOGY: linear

(xi) SEQUENCE DESCRIPTION: SEQ ID :1164:

GCGCGGGTCT GTCTCTTGCT TCAACAGTGT TTGGACGGAA CAGATCCGGG 50

GACTCTCTTC CAGCCTCCGA CCGCCCTCCG ATTNCCTCTC CACTTGCAAC 100

CTCCAAGACC ATCTTCTCGG CCATCTCCTG CTTCTGAAGC CT 142

(2) INFORMATION FOR SEQ ID :1165:

(i) SEQUENCE CHARACTERISTICS:
(A) LENGTH: 219 base pairs
(B) TYPE: nucleic acid
(C) STRANDEDNESS: double
(D) TOPOLOGY: linear

(xi) SEQUENCE DESCRIPTION: SEQ ID :1165:

TATTCCCCCG GTATCAGCAG AGGCGTGTAC GGGCACTGCT TTAAAACTGG 50

GAAGGAGGAA GACGAGGCCA GGGAGCCGGA GGGTCACCAA GGTAGATTTC 100

CAGCAGCCCT AGTCCAGCTG AACACTTTCC AGCCTTGCTT TTCAGCAGCT 150

TTGAGGAAAA GTATAGTGAT CCGTATGTGA AATTTTCATC GCACGTAGCG 200

GATGAGAATA GAGAACTCA 219

(2) INFORMATION FOR SEQ ID :1166:

(i) SEQUENCE CHARACTERISTICS:
(A) LENGTH: 202 base pairs
(B) TYPE: nucleic acid
(C) STRANDEDNESS: double

(D) TOPOLOGY: linear

(xi) SEQUENCE DESCRIPTION: SEQ ID :1166:

GAGGATCCAC CAACCATAGA AAGGAAACAA CACTTGGAGT GAACCCGACC 50

CAAGCCACTA CACCCCAGCC TAACCGACAG GTGCTAGACT AATNGTNAAA 100

AACAACCGGA AAATAGACCC GGACGAAGAT CAAAGNTTNT CAATCCAAAC 150

ATTTTAGGGG GACCAAGACC CMGGGATCAA AAACAAGGTC CACCACACCC 200

AA 202

(2) INFORMATION FOR SEQ ID :1167:

(i) SEQUENCE CHARACTERISTICS:
(A) LENGTH: 159 base pairs
(B) TYPE: nucleic acid
(C) STRANDEDNESS: double
(D) TOPOLOGY: linear

(xi) SEQUENCE DESCRIPTION: SEQ ID :1167:

GAGAAACCTA CCCAACCAAG AGGCGCCCTG CTTTGTAATG ACCTTTACGA 50

AGACACGTCT GATACCCAAC CTGACAGAGG AAACAACAGT AGTCTGAAAG 100

GGACAGAATG AGAGAGGGGG CTGGAGAAAG AAATGAATAA ACATGAATGC 150

ATCTGGAGA 159

(2) INFORMATION FOR SEQ ID :1168:

(i) SEQUENCE CHARACTERISTICS:
(A) LENGTH: 211 base pairs
(B) TYPE: nucleic acid
(C) STRANDEDNESS: double
(D) TOPOLOGY: linear

(xi) SEQUENCE DESCRIPTION: SEQ ID :1168:

CCCTAGACAA GCCACCTGAG GAGAGGCTCG GAGCCGGGCC CGGACCCCGG 50

CGATTGCCAC CGCTTCTCTC TAGTCTCACG AGGGGTTTCC CGCCTCGCAC 100

CCCCACCTCT GGACTTGCCT TTCCTTCTCT TCTCCGCGTG TGGAGGGAGC 150

CAGCGCTTAG GTCGGAGCGA GCCTGGGGCC ACCGCCGTGA AGACATCGCG 200

GGGACCGATT C 211

(2) INFORMATION FOR SEQ ID :1169:

(i) SEQUENCE CHARACTERISTICS:
(A) LENGTH: 211 base pairs
(B) TYPE: nucleic acid
(C) STRANDEDNESS: double
(D) TOPOLOGY: linear

(xi) SEQUENCE DESCRIPTION: SEQ ID :1169:

GGATTTCCGG TCCTGGCTTT CTGATATTTC TAAAATCGAC CTGGAATCAA 50

CCATTGACAT GTCCTGTGCT AAATATGAAT TCACTGATGC CCTGCTGTGC 100

CATGATGATG AGCTGGAAGG GCGCCGGATT GCCTTCATCC TGTACCTGGT 150

TCCTCCCTGG GACAGGAGCA TGGGTGGTAC CCTGGACCTG TACAGCATTG 200

ATGAACACTT T 211

(2) INFORMATION FOR SEQ ID :1170:

(i) SEQUENCE CHARACTERISTICS:

(A) LENGTH: 266 base pairs

(B) TYPE: nucleic acid

(C) STRANDEDNESS: double

(D) TOPOLOGY: linear

(xi) SEQUENCE DESCRIPTION: SEQ ID :1170:

GAGGATCCGC GCAACCATAG AACTCACAAC CCAGCCATAT ACCTCAGACA 50

CAATGGAATG GGCGGAGGTC GAGGTNGACA AACCCTGAGT AATGTTGGGC 100

ACTTCCGCAC CGGAGCTGTT CTTACCTTTG ATAAAGTGGA TGTTATTGCT 150

ATTAATGATC TCTTAACGGA CTTCAACTGT AAAATTNGCA TGATCTAGTC 200

CTATCCACCA ACGNCGAACA ATATGCTGTT GCCAMGATTG AGCACGAGCC 250

GTTTGGGCTC AACGGC 266

(2) INFORMATION FOR SEQ ID :1171:

(i) SEQUENCE CHARACTERISTICS:
(A) LENGTH: 167 base pairs
(B) TYPE: nucleic acid
(C) STRANDEDNESS: double
(D) TOPOLOGY: linear

(xi) SEQUENCE DESCRIPTION: SEQ ID :1171:

GGCACAACCC ACTTTGAACA ATCATGCTTC AGAAATCTGC CTGACCTTAG 50

CTGCTGCTGC TGCTCACTTT ATTATAGTAT AACTTCGGTA GGCATACTTG 100

GAGAACATAT CCCACATTAG GAATTGATTT AAGCCTGAGA GTTTGAGGGC 150

TTTAATCCTT TAAAACT 167

(2) INFORMATION FOR SEQ ID :1172:

(i) SEQUENCE CHARACTERISTICS:
(A) LENGTH: 232 base pairs
(B) TYPE: nucleic acid
(C) STRANDEDNESS: double
(D) TOPOLOGY: linear

(xi) SEQUENCE DESCRIPTION: SEQ ID :1172:

AATAAACCTC CCTATCACAG TGACCTACTA CCCGCGTGTG CTTATATAAC 50

TAATCCAGGA CAACCCACAA AAATTATAGC AACACACAAA CACACCGCTG 100

ACCATAACAT GTGCGTCTTT CAAAGATGCC TTATCAACCA GAGCGATGAT 150

TACTGAGGAT ACGCAACTCA TAAAACTCTA CTTAAAGCAA CAGGGCAGAC 200

GTGCGTCTGT GCCAGTCGTG AATGTGGTGA AC 232

(2) INFORMATION FOR SEQ ID :1173:

(i) SEQUENCE CHARACTERISTICS:

(A) LENGTH: 93 base pairs

(B) TYPE: nucleic acid

(C) STRANDEDNESS: double

(D) TOPOLOGY: linear

(xi) SEQUENCE DESCRIPTION: SEQ ID :1173:

GAGGATCCAC CAACCATAGC CCGAAAATGT GGTAAGGGAC CCTCATCTAT 50

CACACAACNC AGGTAAGAAG GCACCCAGCC CCATGGGCCA TAC 93

(2) INFORMATION FOR SEQ ID :1174:

(i) SEQUENCE CHARACTERISTICS:

(A) LENGTH: 200 base pairs

(B) TYPE: nucleic acid

(C) STRANDEDNESS: double

(D) TOPOLOGY: linear

(xi) SEQUENCE DESCRIPTION: SEQ ID :1174:

GCCTTTTTTT ACCCGCCGGA AGCTACAGCT TTTGCCCCCC CAAAAAAACC 50

CCACCCTTTT ACCCACCGCG GACCCAAAAA CAGCAAAAAC CAAGGACCTC 100

TCCCAACCCC AACCCCTCCT TTTTGGCCCT TCCTTCCCCC CCTCCCAGCC 150

CACCCCCAGA CACCTCAATC CCCCAAAAGG ATGCCCTAAA CCTCTCCTAA 200

(2) INFORMATION FOR SEQ ID :1175:

(i) SEQUENCE CHARACTERISTICS:
(A) LENGTH: 121 base pairs
(B) TYPE: nucleic acid
(C) STRANDEDNESS: double
(D) TOPOLOGY: linear

(xi) SEQUENCE DESCRIPTION: SEQ ID :1175:

GAGGATCCAA AAACCATGGC ATTCATCACG CCCAGCAGGT GTCCCAGCCA 50

TGACTTACCA TAGCAAAACA CAACCACACA CTAACCTGCA TCGCCTAGCT 100

TACTGATGAT GATGTCCTGG T 121

(2) INFORMATION FOR SEQ ID :1176:

(i) SEQUENCE CHARACTERISTICS:
(A) LENGTH: 26 base pairs
(B) TYPE: nucleic acid
(C) STRANDEDNESS: double
(D) TOPOLOGY: linear

(xi) SEQUENCE DESCRIPTION: SEQ ID :1176:

AACCAGACAC GCCGACCCGC TGAATC 26

(2) INFORMATION FOR SEQ ID :1177:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :1177:

GAATCGATCT TGACCTGGCT GCCAGGAATA TCCAGCTTGT TACCGACTGG 50

TAAAGAATAA GACCGCTGAT CATAGAGTGA AGCTCCAGGA ATCAGAGAGT 100

CTCATAGCAA ACCTCGAAAC TGAGGATGAG ATGGTTACAG ATAAAGCCTT 150

TCAGGATGGA TTMAAGNAMG CAGAGAGG 178

(2) INFORMATION FOR SEQ ID :1178:

(i) SEQUENCE CHARACTERISTICS:
(A) LENGTH: 144 base pairs
(B) TYPE: nucleic acid
(C) STRANDEDNESS: double
(D) TOPOLOGY: linear

(xi) SEQUENCE DESCRIPTION: SEQ ID :1178:

GGTTAAGTTT TTCATCAAAG ACTCAGGTAC CTATTATCGT TCCCTGGCGA 50

AACTGAGGAG AAAAGTTAAT CAACCAGGTT ACTCCCACAG TTTGCCCGTG 100

TGTTATGCAT CAGTTATACA GGTATCCCAC CAAGTTCAAG TCAA 144

(2) INFORMATION FOR SEQ ID :1179:

(i) SEQUENCE CHARACTERISTICS:

(A) LENGTH: 233 base pairs

(B) TYPE: nucleic acid

(C) STRANDEDNESS: double

(D) TOPOLOGY: linear

(xi) SEQUENCE DESCRIPTION: SEQ ID :1179:

GACCAGGGAG GAGGTTTAAT CAACTTGGAC CCCCTCCGGC CTAGCCGCCG 50

AGGAGGTGCA TTCGGGACAA CCACTAGGTC AGAACGCAGC CTCTCCAGAG 100

TCCTCAGGCT CGACAACGAT TATCCTGCTG CTATCACGCT TTGATTTACT 150

GATCTCGCTG AAAAGACAGA CGCTTTTAGA TACCGAGTCG ATAGGGGGTC 200

TGCGGTACTT TTCAGTAGAT AGGTGGTGCT TGT 233

(2) INFORMATION FOR SEQ ID :1180:

(i) SEQUENCE CHARACTERISTICS:

(A) LENGTH: 225 base pairs

(B) TYPE: nucleic acid

(C) STRANDEDNESS: double

(D) TOPOLOGY: linear

(xi) SEQUENCE DESCRIPTION: SEQ ID :1180:

CGAGGATCCG GGTACCATGG GCCGGATACA ACGAGCTATC ATTACTGCTC 50

CCATGGCCAA AACCAGCAGT CCCACAATCC CCGTGAAAGG GATGAGGTAA 100

TAGCCCAAGG GGAAGGTATT GTCTGGAACC AGAAGCGCCC GAGCCCCCTT 150

CTTGTAGRCA AAGAGGGCGC CCAGGTGCTT GGAGCTNCTN TCCCCAATGG 200

AGGTAGACGG GACCAAGATC TGCTG 225

(2) INFORMATION FOR SEQ ID :1181:

(i) SEQUENCE CHARACTERISTICS:
(A) LENGTH: 293 base pairs
(B) TYPE: nucleic acid
(C) STRANDEDNESS: double
(D) TOPOLOGY: linear

(xi) SEQUENCE DESCRIPTION: SEQ ID :1181:

TGAGGATTTA AAAATTCTGG AATATCTATG ATTGATCGTC AACTTTATTC 50

GATGAATAAG CTTGATGTGG CCTAGTTTTN NGNNNNNTGG NYATGGTNNA 100

TCNNNTTANT TTTTTGGTTG TTGTGNATAT TATNGAATAR AMGAATRGNG 150

TTTAGAGTTT GGAAGCGGCN RNGCGTARMG NNACTCYACG CTCGCNNCTN 200

TTGNNNNACA GMRGGNTCTC TNGGRTGAGT GGRTNCMGTT GGMGGNNTCN 250

NNNTAGCTGN NGNGAGRATC AGCTRGCTTN CTTTGNGCTN GCT 293

(2) INFORMATION FOR SEQ ID :1182:

(i) SEQUENCE CHARACTERISTICS:

(A) LENGTH: 252 base pairs

(B) TYPE: nucleic acid

(C) STRANDEDNESS: double

(D) TOPOLOGY: linear

(xi) SEQUENCE DESCRIPTION: SEQ ID :1182:

GAGGATCCGG GTACCATGGC TCTCTCTTTC TTTTTTTTCT TTTTCTTGGC 50

GGATGTGAGA GCTGCCTGAG ATTCAAGGTC ATCCGGCAGC TCAGTCCCCA 100

CCACCTCTGT CTCTGGCTCC ACTGTGGCAT CTTGCTGTTT TTCTTTCTCA 150

GTCTTCTCTT AGGGAGCTGC CAGAGCTGCC TGGACCTGAG AATTCATTCC 200

TTCTGGCTGT TGAGACCCCG TGGACTCCCC TGGATTCCAG AGNNTNATTT 250

NG 252

(2) INFORMATION FOR SEQ ID :1183:

(i) SEQUENCE CHARACTERISTICS:

(A) LENGTH: 202 base pairs

(B) TYPE: nucleic acid

(C) STRANDEDNESS: double

(D) TOPOLOGY: linear

(xi) SEQUENCE DESCRIPTION: SEQ ID :1183:

ACGAGGATCT GAATACTCTG GCCTGCAAGT AGAGGCTAAT TCGATGTGTG 50

AAACTGTAGA TTCGGCTTAA GGTAATAGTC ATTTATAGAC TATTCAATAG 100

AGTCAGACTG GTCGAGGCTG GTAAGTGTAG CGGAACGGTT GCTGACTGAT 150

ATTGTTAGAG TGAGGCTTGT ACTGGTGTGT ATCCGTAGGT GGTCTGCAGT 200

GT 202

(2) INFORMATION FOR SEQ ID :1184:

(i) SEQUENCE CHARACTERISTICS:

(A) LENGTH: 43 base pairs

(B) TYPE: nucleic acid

(C) STRANDEDNESS: double

(D) TOPOLOGY: linear

(xi) SEQUENCE DESCRIPTION: SEQ ID :1184:

CAGATCCTCA GCTTTCGTGG TTCACAATTT CTTCAGTCTC TTA 43

(2) INFORMATION FOR SEQ ID :1185:

(i) SEQUENCE CHARACTERISTICS:

(A) LENGTH: 48 base pairs

(B) TYPE: nucleic acid

(C) STRANDEDNESS: double
(D) TOPOLOGY: linear

(xi) SEQUENCE DESCRIPTION: SEQ ID :1185:

ACCGTCCTTC TGGTTCATCC TAGCAAAAAT CTCACCATCT TCTATCAC 48

(2) INFORMATION FOR SEQ ID :1186:

(i) SEQUENCE CHARACTERISTICS:
(A) LENGTH: 104 base pairs
(B) TYPE: nucleic acid
(C) STRANDEDNESS: double
(D) TOPOLOGY: linear

(xi) SEQUENCE DESCRIPTION: SEQ ID :1186:

ACATCATCCG AGTCCCCTCT ACAGTGTTCA TTGTGATCGT TGCATCCCCC 50

TGGTTTGGAA ATAAAATATA AACTGCCCGG CAAGAGATAA AATTGTATTT 100

TTTA 104

(2) INFORMATION FOR SEQ ID :1187:

(i) SEQUENCE CHARACTERISTICS:
(A) LENGTH: 76 base pairs
(B) TYPE: nucleic acid
(C) STRANDEDNESS: double
(D) TOPOLOGY: linear

(xi) SEQUENCE DESCRIPTION: SEQ ID :1187:

CTGGTTCTGT TTCTCGCAGG TGGTAGAGGG GAGGCTGTCC TCTGGTCAGG 50

AGAATCCTAT TCAGTGCTCC CTTAGA 76

(2) INFORMATION FOR SEQ ID :1188:

(i) SEQUENCE CHARACTERISTICS:
(A) LENGTH: 42 base pairs
(B) TYPE: nucleic acid
(C) STRANDEDNESS: double
(D) TOPOLOGY: linear

(xi) SEQUENCE DESCRIPTION: SEQ ID :1188:

AATAGGGCGC GATCAACTCT TAACTTTGAG GAGAACCAAC AA 42

(2) INFORMATION FOR SEQ ID :1189:

(i) SEQUENCE CHARACTERISTICS:
(A) LENGTH: 253 base pairs
(B) TYPE: nucleic acid
(C) STRANDEDNESS: double
(D) TOPOLOGY: linear

(xi) SEQUENCE DESCRIPTION: SEQ ID :1189:

GGCGGACGTG CGCGCCTTGT CTTCGCGGCA CCTGGGCCTG AGGTGCGTGC 50

CTCCCGGGCC CTCGCCAGCT CCAGATGCGT GAGGAGGACT TCAGAAACCC 100

GACTGAGAAG TGGAGCAACC CCCAGGAAGG GCCGGACCTG CCTAAATGCC 150

GCCAAGGCCT TTTATTTATG GCTAGTTTGC TCTCGTGAAA TACTAACATC 200

GTTTTAATGG CACTCATCAA GTACGCAAAT GATATGATTT AACCTCGCGC 250

AAT 253

(2) INFORMATION FOR SEQ ID :1190:

(i) SEQUENCE CHARACTERISTICS:

(A) LENGTH: 252 base pairs

(B) TYPE: nucleic acid

(C) STRANDEDNESS: double

(D) TOPOLOGY: linear

(xi) SEQUENCE DESCRIPTION: SEQ ID :1190:

GAGGATCCAC CAACCATAGA ACGGCCCTAC TAATGAACCA CACAAGCTAG 50

TTGCACCACT ATAGAAACAC AAAACTACAC ATCTATACCA ATAAAAATAA 100

CAACTACTCC AATTGCCCAT GTGGTATTGT CGCAGACTGA AATGAATTAT 150

GAATTTAGGT TGGTTACCAA TATCGGCATA AAATAAACTR TGTAAGGCTC 200

AMTATGTTGA CAGTAAGCTC TTGTCAGGTG TCTAATGAGG TAAAAGCATT 250

TT 252

(2) INFORMATION FOR SEQ ID :1191:

(i) SEQUENCE CHARACTERISTICS:
(A) LENGTH: 178 base pairs
(B) TYPE: nucleic acid
(C) STRANDEDNESS: double
(D) TOPOLOGY: linear

(xi) SEQUENCE DESCRIPTION: SEQ ID :1191:

GAGGATCCAA CAACCATAGA GCACATAAAA ACCGCCCAAC GATCTAACTA 50

ATATACAACG GCTAACCGGG CCATTCAAAA GCTCGCCCAG ATTAAATGCC 100

TGCCGANAGC AAGTACATGG GGAGGATTAC TACTTCCTGG TTGCCAACCA 150

CCCTTCCGGC TGCCCTTGTG TTGACTTG 178

(2) INFORMATION FOR SEQ ID :1192:

(i) SEQUENCE CHARACTERISTICS:
(A) LENGTH: 156 base pairs
(B) TYPE: nucleic acid
(C) STRANDEDNESS: double
(D) TOPOLOGY: linear

(xi) SEQUENCE DESCRIPTION: SEQ ID :1192:

GGATCGGCGG TAATCGGTGA GCTCGGTGTC GGGTAAGGGA CCCAGTCCAT 50

CGCCACCAAG CGCCAGGAAT GGGCAGCATA AGGGAAGGCT AAGGAGGACT 100

GCAACAGGTT AGGGCCCTGG AGATTGTATT TAGCAAGGGT ACCTGCGNNG 150

NGGGCA 156

(2) INFORMATION FOR SEQ ID :1193:

(i) SEQUENCE CHARACTERISTICS:
(A) LENGTH: 99 base pairs
(B) TYPE: nucleic acid
(C) STRANDEDNESS: double
(D) TOPOLOGY: linear

(xi) SEQUENCE DESCRIPTION: SEQ ID :1193:

ATTCTGAATC TTTAATGAAA ATCAGCCCAG AGCTTGTTGT AATACAGACA 50

TAACTGCAAG AGCATGCCCA CAGCTTAGAG AGGTTGTACC AAATTTAGA 99

(2) INFORMATION FOR SEQ ID NO: 1194

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID : 1194:

NNNNTCCTTC TCCTGCGACA GACA 24

(2) INFORMATION FOR SEQ ID NO: 1195

(i) SEQUENCE CHARACTERISTICS:

(A) LENGTH: 20 base pairs

(B) TYPE: nucleic acid

(C) STRANDEDNESS: single

(D) TOPOLOGY: linear

(xi) SEQUENCE DESCRIPTION: SEQ ID : 1195:

TGTCTGTCGC AGGAGAAGGA 20

(2) INFORMATION FOR SEQ ID NO: 1196

(i) SEQUENCE CHARACTERISTICS:

(A) LENGTH: 26 base pairs

(B) TYPE: nucleic acid

(C) STRANDEDNESS: single

(D) TOPOLOGY: linear

(xi) SEQUENCE DESCRIPTION: SEQ ID : 1196:

AANNTCTCGG ACAGTGCTCC GAGAAC 26

(2) INFORMATION FOR SEQ ID NO: 1197

(i) SEQUENCE CHARACTERISTICS:

(A) LENGTH: 26 base pairs

(B) TYPE: nucleic acid
(C) STRANDEDNESS: single
(D) TOPOLOGY: linear

(xi) SEQUENCE DESCRIPTION: SEQ ID : 1197:

TTNNTCTCGG ACAGTGCTCC GAGAAC 26

(2) INFORMATION FOR SEQ ID NO: 1198

(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID : 1198:

GTTCTCGGAG CACTGTCCGA GA 22

(2) INFORMATION FOR SEQ ID NO: 1199

(i) SEQUENCE CHARACTERISTICS:
(A) LENGTH: 23 base pairs
(B) TYPE: nucleic acid
(C) STRANDEDNESS: single
(D) TOPOLOGY: linear

(xi) SEQUENCE DESCRIPTION: SEQ ID : 1199:

GTTCTCGGAG CACTGTCCGA GAG 23

(2) INFORMATION FOR SEQ ID NO: 1200

(i) SEQUENCE CHARACTERISTICS:
(A) LENGTH: 23 base pairs
(B) TYPE: nucleic acid
(C) STRANDEDNESS: single
(D) TOPOLOGY: linear

(xi) SEQUENCE DESCRIPTION: SEQ ID : 1200:

GTTCTCGGAG CACTGTCCGA GAC 23

(2) INFORMATION FOR SEQ ID NO: 1201

(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID : 1201:

CTGTCTGTCG CAGGAGAAGG AA 22

(2) INFORMATION FOR SEQ ID NO: 1202

(i) SEQUENCE CHARACTERISTICS:
(A) LENGTH: 22 base pairs

(B) TYPE: nucleic acid
(C) STRANDEDNESS: single
(D) TOPOLOGY: linear

(xi) SEQUENCE DESCRIPTION: SEQ ID : 1202:

CTGTCTGTCG CAGGAGAAGG AG 22

(2) INFORMATION FOR SEQ ID NO: 1203

(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID : 1203:

AGCTCGGCTC GAGTCTG 17

(2) INFORMATION FOR SEQ ID NO: 1204

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID : 1204:

GCGACAGACA GCAGACTCGA GCCG 24

(2) INFORMATION FOR SEQ ID NO: 1205

(i) SEQUENCE CHARACTERISTICS:
(A) LENGTH: 14 base pairs
(B) TYPE: nucleic acid
(C) STRANDEDNESS: single
(D) TOPOLOGY: linear

(xi) SEQUENCE DESCRIPTION: SEQ ID : 1205:

GATCCGGCTC GAGT 14

(2) INFORMATION FOR SEQ ID NO: 1206

(i) SEQUENCE CHARACTERISTICS:
(A) LENGTH: 18 base pairs
(B) TYPE: nucleic acid
(C) STRANDEDNESS: single
(D) TOPOLOGY: linear

(xi) SEQUENCE DESCRIPTION: SEQ ID : 1206:

CCGAGAACAC TCGAGCCG 18

(2) INFORMATION FOR SEQ ID NO: 1207

(i) SEQUENCE CHARACTERISTICS:
(A) LENGTH: 17 base pairs

(B) TYPE: nucleic acid
(C) STRANDEDNESS: single
(D) TOPOLOGY: linear

(xi) SEQUENCE DESCRIPTION: SEQ ID : 1207:

GTAAAACGAC GGCCAGT 17

(2) INFORMATION FOR SEQ ID NO: 1208

(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID : 1208:

CGAGGTCGAC GGTATCG 17

(2) INFORMATION FOR SEQ ID NO: 1209

(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID : 1209:

CGAGGTCGAC GGTATCG 17

(2) INFORMATION FOR SEQ ID NO: 1210

(i) SEQUENCE CHARACTERISTICS:
(A) LENGTH: 56 base pairs
(B) TYPE: nucleic acid
(C) STRANDEDNESS: single
(D) TOPOLOGY: linear

(xi) SEQUENCE DESCRIPTION: SEQ ID : 1210:

TACGTTCGAC AAGCTTGAAT TCGCGGCCGC TTTTTTTTTT TTTTTTTTTT 50

TTTTTT 56

(2) INFORMATION FOR SEQ ID NO: 1211

(i) SEQUENCE CHARACTERISTICS:
(A) LENGTH: 11 base pairs
(B) TYPE: nucleic acid
(C) STRANDEDNESS: single
(D) TOPOLOGY: linear

(xi) SEQUENCE DESCRIPTION: SEQ ID : 1211:

GCCWSCGCCG A 11

(2) INFORMATION FOR SEQ ID NO: 1212

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID : 1212:

GGTGGCGACG ACTCCTGGAG CCCG 24

(2) INFORMATION FOR SEQ ID NO: 1213

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID : 1213:

TTGACACCAG ACCAACTGGT AATG 24

In the above SEQUENCE LISTINGS, some sequences are preferred because they fall into the category of sequences referred to hereinbefore which exhibit no more than 90% homology to a human sequence known per se. The preferred sequences in these terms are all of sequences SEQ ID Nos 1 to 1193, EXCEPT FOR SEQ ID Nos:
85, 117, 177, 197, 223, 248, 317, 354, 355, 483, 829, 1057, 594, 595, 597, 164, 427, 420, 58, 67, 374, 373, 501, 569, 188, 550,904, 932, 97, 89, 134, 433, 434, 357, 4, 6, 11, 336, 529, 544, 545, 549, 1037, 847, 870, 871, 872, 873, 875, 876, 579, 199, 524, 544, 513, 380, 276, 291, 615, 623, 627, 634, 635, 648, 652, 617, 619, 684, 697, 718, 720, 1127, 1145, 1148, 1164, 938, 587, 589, 588, 241, 243, 335, 61.

## Claims

1. A nucleic acid fragment encoding a gene product or portion thereof and comprising any one of:-
(a) a sequence selected from SEQ ID Nos 1 to 1193;
(b) an allelic variation of a sequence as defined in (a); or
(c) a sequence complementary to (a) or (b).

2. A nucleic acid sequence as set out in any one of SEQ ID Nos 1 to 1193, or a complement or allelic variation thereof.

3. A sequence as claimed in claim 2 and which exhibits no more than 90% homology to a human sequence known per se.

4. A nucleic acid fragment comprising a portion of a sequence as defined in claim 2 or claim 3 of sufficient size such that a probe of the same size and exhibiting complementarity to said portion can hybridize to said sequence as defined in claim 2 or claim 3.

5. A fragment as claimed in claim 4, wherein said portion is at least 15 bases in length.

6. A fragment as claimed in any one of claims 1, 4 or 5 and encoding at least a portion of a biologically active polypeptide.

7. A nucleic acid sequence as claimed in claim 2 or claim 3 and encoding at least a portion of a biologically active polypeptide.

8. A DNA construct comprising a fragment as defined in any one of claims 1, 4, 5 or 6 or a sequence as defined in any one of claims 2, 3 or 7, together with a control or regulatory sequence.

9. A construct as claimed in claim 8 which encodes a fusion protein comprising a known protein and the polypeptide encoded by said fragment or sequence.

10. A construct as claimed in claim 9, wherein the fusion protein encoded is a cleavable fusion protein having an endopeptidase recognition site positioned between codons corresponding to said known protein and said fragment or sequence.

11. The use of a fragment as defined in any one of claims 1, 4, 5 or 6 or a sequence as defined in any one of claims 2, 3 or 7 to produce a gene.

12. A DNA fragment comprising a gene obtainable by the use defined in claim 11.

13. An expression vector comprising a fragment as defined in any one of claims 1, 3, 5 or 6, a sequence as defined in any one of claims 2, 3 or 7, a DNA construct as defined in any one of claims 8 to 10, or a DNA fragment as claimed in claim 12, positioned such that that nucleic acid sequence which encodes the polypeptide corresponding to said fragment, sequence or DNA fragment is in operable reading frame with a control or regulatory sequence.

14. A vector as claimed in claim 13, wherein said vector control or regulatory sequence comprises a regulatable promoter.

15. Host cells which incorporate as a heterologous part of their expressible genetic information a fragment as defined in any one of claims 1, 3, 5 or 6, a sequence as defined in any one of claims 2, 3 or 7, or a DNA fragment as defined in claim 12.

16. A process for the production of a polypeptide comprising cultivating host cells as defined in claim 15.

17. An antibody directed against a polypeptide obtainable by the performance of a process as defined in claim 16.

18. An antibody as claimed in claim 17 and which is monoclonal.

19. A novel gene product or portion thereof encoded by a fragment as defined in any one of claims 1, 3, 5 or 6, or encoded by a sequence as defined in any one of claims 2, 3 or 7, or encoded by the gene comprised in a DNA fragment as defined in claim 12.
